# EUROPEAN PATENT APPLICATION

(11) **EP 2 660 241 A2**
(43) Date of publication of application: **06.11.2013**
(21) Application number: 13179069.3
(22) Date of filing: 16.12.2011
(51) Int. Cl.: C07D 401/08, C07D 403/08, C07D 405/08, C07D 413/08, C07D 471/04, C07J 43/00, C07J 61/00, C07J 63/00, C07J 73/00, A61P 5/26, A61P 5/42, A61P 5/46, A61P 35/00, A61K 31/56

(54) **C-17-HETEROARYL STEROIDAL COMPOUNDS AS INHIBITORS OF CYP11B, CYP17, AND/OR CYP21**

(30) Priority: 16.12.2010 US 423718 P; 25.05.2011 US 201161489887 P
(62) Divisional of application: 11808473.0
(71) Applicant: BioMarin Pharmaceutical Inc., Novato, CA 94949 (US)
(72) Inventor: Chu, Daniel, Santa Clara, CA 95138 (US); Wang, Bing, San Jose, CA 95138 (US); Ye, Tao, ung Hom, Kowloon, Hong Kong (HK)
(74) Representative: Alcock, David

(57) **Abstract**

Provided herein are inhibitors of CYP11B, CYP17, and/or CYP21 enzymes of Formula Z, IX, X, XI, XII, XIII, XIV, XV, XVI, or XVII. Also described herein are pharmaceutical compositions that include at least one compound described herein and the use of a compound or pharmaceutical composition described herein to treat androgen-dependent diseases, disorders and conditions.

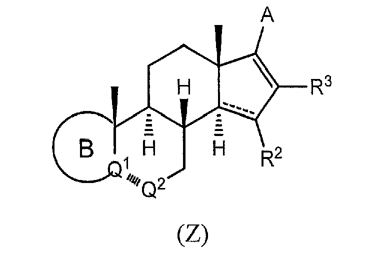

## Description

### FIELD OF THE INVENTION

Described herein are compounds, methods of making such compounds, pharmaceutical compositions and medicaments containing such compounds, and methods of using such compounds to treat androgen-dependent diseases or conditions.

### BACKGROUND OF THE INVENTION

The 17α-hydroxylase/C₁₇₋₂₀ lyase enzyme complex is essential for the biosynthesis of androgens. CYP17 is a bifunctional enzyme which possess both a C_{17,20}-lyase activity and a C17-hydroxylase activity. These two alternative enzymatic activities of CYP17 result in the formation of critically different intermediates in steroid biosynthesis and each activity appear to be differentially and developmentally regulated.

In the testes and adrenal glands, the last step in the biosynthesis of testosterone involves two key reactions, which act sequentially and are both catalyzed by a single enzyme, the cytochrome 6P450 monooxygenase 17α-hydroxylase/C_{17,20}-lyase (P450₁₇ or CYP17). CYP17 is a key enzyme in the biosynthesis of androgens, and converts the C₂₁ steroids (pregnenolone and progesterone) to the C₁₉ androgens, dehydroepiandrosterone (DHEA), androstenediol (A-diol), testosterone, and androstenedione in the testes and adrenals. Both DHEA and androstenedione lyase products are key intermediates in the synthesis of not only the androgens testosterone and dihydrotestosterone (DHT), but also the estrogens 17β-estradiol and estrone. Adrenal and ovarian estrogens are the main sources of estrogens in postmenopausal women. The C₁₇-hydroxylase activity of CYP17 catalyzes the conversion of the common intermediate progesterone to 17-hydroxyprogesterone, a precursor of cortisol. Thus, the C₁₇-hydroxylase activity promotes the formation of glucocorticoids while the C_{17,20}-lyase activity promotes the formation of sex hormones-particularly androgens including testosterone as well as estrogens.

Examples of CYP17-associated diseases and disorders include, but are not limited to, sex steroid hormone dependent cancers, such as androgen-dependent prostate cancer, which in some or any embodiments is treated by inhibiting CYP17-mediated androgen synthesis, and estrogen-dependent breast cancer or ovarian cancer, which in other embodiments is treated by inhibiting CYP17-mediated estrogen synthesis.

For example, adenocarcinoma of the prostate is a common disease that causes significant morbidity and mortality in the adult male population (see Han and Nelson, Expert Opin. Pharmacother. 2000, 1, 443-9). Prostate cancer is the most common malignancy and age-related cause of cancer death worldwide. Apart from lung cancer, prostate cancer is the most common form of cancer in men and the second leading cause of death in American men. During the period of 1992 to 1999, the average annual incidence of prostate cancer among African American men was 59% higher than among Caucasian men, and the average annual death rate was more than twice that of Caucasian men (American Cancer Society-Cancer Facts and Figures 2003). Hormonal therapy for prostate cancer is considered when a patient fails with initial curative therapy, such as radical prostatectomy or definitive radiation therapy, or if he is found with an advanced disease. Hormonal agents have been developed to exploit the fact that prostate cancer growth is dependent on androgen. Non-steroidal anti-androgens (NSAAs) block androgen at the cellular level. Castration is another, albeit drastic means of decreasing androgens levels in order to treat or prevent prostate cancer.

Androgens play an important role in the development, growth, and progression of prostate cancer. Two important androgens in this regard are testosterone and dihydrotestosterone (DHT). The testes synthesize about 90% of testosterone and the rest (10%) is synthesized by the adrenal glands. Testosterone is further converted to the more potent androgen DHT by the enzyme steroid 5α-reductase that is localized primarily in the prostate.

Since prostate cancer is typically androgen-dependent, the reduction of androgen production via surgical or pharmacological castration is the major treatment option for this indication. Androgen deprivation has been used as therapy for advanced and metastatic prostate cancer. Androgen ablation therapy has been shown to produce the most beneficial responses in multiple settings in prostate cancer patients. However, orchidectomy remains the standard treatment option for most prostate cancer patients.

Medical and surgical orchidectomy reduces or eliminates androgen production by the testes but does not affect androgen synthesis in the adrenal glands. Several studies have reported that orchidectomy therapy and treatment with anti-androgens to inhibit the action of adrenal androgens significantly prolongs the survival of prostate cancer patients. Further, it has been shown that testosterone and DHT occur in recurrent prostate cancer tissues at levels sufficient to activate androgen receptor. In addition, the use of microarray-based profiling of isogenic prostate cancer xenograft models showed that a modest increase in androgen receptor mRNA was the only change consistently associated with the development of resistance to anti-androgen therapy. Since CYP17 is implicated in the synthesis of key intermediates of androgens, the pharmacological inhibition of CYP17 is a promising treatment in that testicular, adrenal, and peripheral androgen biosynthesis would be reduced rather than only testicular androgen production. (Njar, V. et al., J. Med. Chem. 1998, 41, 902).

In addition to the use of CYP17 inhibitors in the treatment of prostate cancer, CYP17 inhibitors will find utility for the indication of breast cancer, more particularly, estrogen-dependent breast cancer. In post-menopausal patients with advanced breast cancer, treatment with high doses of ketoconazole resulted in suppression of both testosterone and estradiol levels, implicating CYP17 as a potential target for hormone therapy. (Harris, A. L. et al., Br. J. Cancer 1988,58,493).

In hormone-dependent breast cancer in postmenopausal women adrenal and ovarian androgens are the main precursors of the estrogens which stimulate the growth of the cancer. It has been shown that patients failing to respond to aromatase inhibitors show elevated levels of androgens in response to aromatase inhibitor treatment (see Harris et al., Bi. J. Cancer 1988, 58, 493-6). Accordingly, sequential blockade to inhibit androgen production as well as inhibit aromatase produces greater estrogen suppression and enhanced therapeutic effects in treating breast and other estrogen hormone-dependent forms of cancer. Furthermore, susceptibility to prostate cancer and breast cancer has been associated with particular polymorphic alleles of the CYP17 gene (see e.g. McKean-Cowdin, Cancer Res. 2001, 61, 848-9; Haiman et al., Cancer Epidmeiol. Biomarkers 2001,10,743-8; Huang et al., Cancer Res. 2001, 59, 4870-5).

Inhibitors of CYP17 have been previously described. For example, ketoconazole, an active imidazole fungicide has been used to reduce testosterone biosynthesis in the treatment of patients with advanced prostatic cancer. However, there are side-effects including liver damage, inhibition of several other cytochrome P₄₅₀ steroidogenic enzymes, and reduction of cortisol production.

Potent and selective inhibitors of CYP17 as potential prostate cancer treatments have been the subject of previous studies. Finasteride, a 5α-reductatse inhibitor, is an approved treatment for benign prostatic hyperplasia (BPH), although it is only effective with patients exhibiting minimal disease. While finasteride reduces serum DHT levels, it increases testosterone levels and may therefore be insufficient for prostate cancer treatment.

21α-hydroxylase (Cyp21) and 11β-hydroxylase (Cyp 11B1) are two enzymes that are critical for the synthesis of cortisol and overproduction of cortisol has been implicated in Cushing's syndrome. Cushing's syndrome refers to the manifestations induced by chronic exposure to excessive glucocorticoid and may result from various causes. It most commonly arises from iatrogenic causes, when glucocorticoids are given to treat inflammatory diseases. In contrast, spontaneous Cushing's syndrome may result from various causes that all have in common a chronic over-secretion of cortisol by the adrenals. Cushing's disease is the most common cause of spontaneous Cushing's syndrome, occurring in 60-70% of Cushing's patients. It results from the hyper-secretion of adrenocorticotropic hormone (ACTH) by a pituitary corticotroph adenoma. Besides Cushing's disease, ectopic ACTH syndrome is responsible for 5-10% of the cases of spontaneous Cushing's syndrome; it is caused by a variety of ACTH-secreting non-pituitary tumors. Finally, about 20-30% of spontaneous Cushing's syndromes are independent of ACTH and are caused by primary adrenocortical tumors.

The short- and long-term consequences of hypercortisolism dictate the necessity for the normalization of cortisol levels. Surgical removal of the pituitary adenoma still represents the first-line treatment, which may be followed by radiotherapy in cases of surgical failure. Drugs are an alternative as monotherapy, but may also be used in addition to radiotherapy or radiosurgery while awaiting their delayed effects, before surgery to reverse the metabolic consequences and poor healing of hyper-cortisolaemia, or in patients who cannot be submitted to surgical procedures because of comorbidities or who are unwilling to receive other types of treatment. Medical therapy is not considered the primary treatment in Cushing's disease because of the lack of highly effective and safe drugs that block the over-production of cortisol.

CYP21 catalyzes the reactions that convert progesterone to deoxycorticosterone and 17α-hydroxyprogesterone to 11-deoxycortisol. CYP11B1 converts deoxycorticosterone to corticosterone and 11-deoxycortisol to cortisol. Thus, blocking Cyp21 and/or Cyp11B1 represents logical therapeutic strategies for overcoming Cushing's syndrome and other types of hypercortisolism. Indeed, several drugs with activities of inhibiting Cyp11B1have been used clinically to treat Cushing's syndrome. These include metyrapone, ketoconazole, etomidate, and mitotane. While some clinical efficacy has been noted for these drugs in treating Cushing's syndrome, their effectiveness is hampered by their lack of potency in blocking Cyp11B1 activity. Furthermore, all these drugs have significant non-specific activities, interfering with other enzymes such as side-chain cleavage enzyme, 16α-hydroxylase, 17α-hydroxylase, and 3β-hydroxysteroid dehydrogenase, etc. These non-specific activities are believed to be responsible for the significant clinical toxicity these drugs cause.

No selective Cyp21 inhibitors have been reported for medical use. The fact that Cyp21-deficient mice are incapable of glucocorticoid and mineralocorticoid production suggests inhibiting Cyp21 could be an effective strategy to decrease cortisol synthesis and to alleviate the clinical manifestation of hypercortisolism, including Cushing's syndrome. Specific and potent inhibitors of Cyp11B1 may be useful for the treatment of Cushing's syndrome and other types of hypercortisolism.

### SUMMARY OF THE INVENTION

Provided herein are compounds, compositions and methods for inhibiting the CYP11B, CYP17, and/or CYP21 enzymes. Also described herein is the use of such compounds and compositions for the treatment of cancer and/or androgen-dependent diseases, disorders or conditions. The methods and compositions described herein are useful in inhibiting the C_{17,20}-lyase activity of CYP11B, CYP17, and/or CYP21 and thereby decreasing levels of androgen production and the associated growth of androgen-dependent cancers such as prostate cancer.

In one aspect, disclosed is a compound of Formula Z 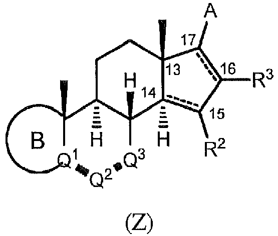 where 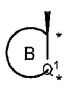 is according to one of the following formulas Z-I to Z-IX: 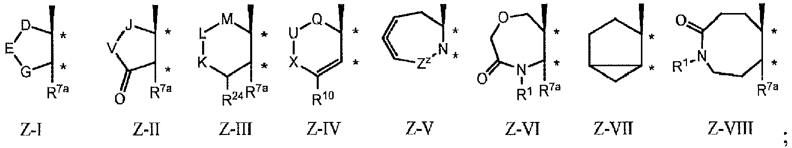 each 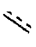 is independently a single bond or a double bond;
Q¹'''''Q²'''''Q³ is Q¹-Q²=Q³; where Q¹ is as depicted in formulas Z-I to Z-VIII above and R^{7a} is absent or is hydrogen or hydroxy; and Q² and Q³ are CH; or
Q¹'''''Q²'''''Q³ is Q¹=Q²-Q³; where Q¹ is as depicted in formulas Z-I to Z-VIII above and R^{7a} is absent; Q² is CH; and Q³ is CHR⁸ or C(O) where R⁸ is hydrogen; or
Q¹'''''Q²'''''Q³ is Q¹-Q²-Q³; where Q¹ is as depicted in formulas Z-I to Z-VIII above and R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7a}, when present, and R^{7b} together with the carbons to which they are attached form cycloalkyl or heterocycloalkyl;
b) R^{7b} and R^{7c} together with the carbon to which they are attached form C=CH₂, cycloalkyl, or carbonyl; or
c) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxy, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxy, hydroxyalkyl and alkylcarbonyloxy;
R³ is selected from the group consisting of hydrogen, halogen, alkyl, cycloalkyl, alkynyl, cyano, haloalkoxy, haloalkyl, hydroxy, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxy, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂^{R13}a, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
D is (CH₂)_{d} where d is an integer from 1 to 3;
E is CH₂, CR¹⁴R^{14a}, O, NR¹, N-COR¹, N-S(O)₀₋₂(akyl), or N-COOR¹;
G is CH(CH₃), C(CH₃)₂, or (CH₂)ₑ where e is an integer from 1 to 3,
provided that
1) when E is CH₂ and d + e is 3, then A is not unsubstituted furyl or unsubstituted thienyl,
2) when Q¹'''''Q²'''''Q³ is Q¹-Q²-Q³, E is CH₂, d + e is 3, and the bond between carbons 16 and 17 is a double bond, then a) A is not oxadiazolyl substituted with alkyl or phenyl and is not thiadiazolyl substituted with alkyl;
V is (CH₂), O, NR¹, N-COR¹ or N-COOR¹;
J is (CH₂)₁₋₃;
K is C(O), NR¹, N-C(O)R¹ or N-C(O)OR¹;
L is CH₂, C(O), NR¹, N-C(O)R¹ or N-C(O)OR¹;
M is (CH=CH) or (CH₂)_{g} where g is an integer 2 or 3,
provided that when g is 2, K is C(O) and L is NR¹, N-C(O)R¹ or N-C(O)OR¹; when g is 2, K is NR¹, N-C(O)R¹ or N-C(O)OR¹ and L is C(O); then the bond between carbons 14 and 15 is a double bond; and
provided that g cannot be 2 when K is NR¹, N-C(O)R¹ or N-C(O)OR¹ and L is or CH₂; R²⁴ is hydrogen or alkyl; or when R^{7a} is present, R²⁴ and R^{7a} together with the carbons to which
they are attached form cycloalkyl;
Q is (CH₂)ᵢ where i is an integer from 1 to 3;
U is (CH₂), CO, O, NR¹, N-COR¹ or N-COOR¹; or
Q and U together are CH=CH;
X is CR^{11a}R^{11b}, C=O, C=NOR⁹, O, NR¹, N-COR¹ or N-COOR¹
provided that i cannot be 1 when X is CO and U is CH₂;
provided that when X is CR^{11a}R^{11b}, R^{11b} is OR¹, then
a) the bond between carbons 16 and 17 is a double bond;
b) A is not unsubstituted benzimidazolyl, unsubstituted imidazolyl, or unsubstituted pyrazolyl; and
c) A is not imidazolyl, thiazolyl, or oxazolyl, where each are substituted with amino, alkylamino, or dialkylamino;
R⁹ is hydrogen, alkyl, or haloalkyl;
R¹⁰ is hydrogen, hydroxy, or alkyl;
R^{11a} is hydrogen or alkyl;
R^{11b} is hydrogen or -OR¹;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR¹⁵R^{15b}, -NR¹⁵C(O)OR^{15a}, or -NR¹⁵R^{15a};
provided that when R^{14a} is -NR¹⁵R^{15a}, then Q¹'''''Q²'''''Q³ is not Q¹-Q²-Q³ and A is monocyclic heteroaryl;
each R¹⁵ and R^{15b} is independently hydrogen, alkyl, or haloalkyl;
each R^{15a} are independently alkyl, haloalkyl, cycloalkyl, or heteroaryl where the heteroaryl is optionally substituted with one or two alkyl;
Z^{Z} is CO or (CH₂);
provided that when 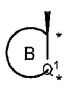 is according to formula Z-VII, and Q² is CH(OH), CH(OCH₃), or CH(OC(O)alkyl), then A is not unsusbstituted pyridinyl and A is not pyrazolyl optionally substituted with one R⁴;
or a single stereoisomer or tautomer or mixture thereof, optionally as a pharmaceutically acceptable salt or solvate thereof.

In another aspect, disclosed is a compound of Formula IX 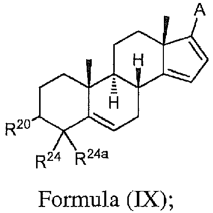 where
A is a heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²⁰ is arylcarbonyloxy, heterocycloalkylcarbonyloxy, heteroarylcarbonyloxy, -OC(O)NR²¹R^{21a}, or -OC(O)-alkylene-NR²¹R^{21a}; where the aryl, heterocycloalkyl, and heteroaryl are independently optionally substituted with one or two alkyl;
R²¹ is hydrogen or alkyl;
R^{21a} is hydrogen, alkyl, or heteroaryl;
R²⁴ and R^{24a} are independently hydrogen or alkyl; and
provided that when R²⁰ is phenylcarbonyloxy, then A is not unsubstituted pyridinyl or pyridinyl substituted with one alkyl; or
a single stereoisomer or tautomer or mixture thereof, optionally as a pharmaceutically acceptable salt or solvate thereof.

In another aspect, disclosed is a compound of Formula X 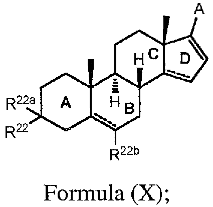 where
one 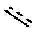 is a single bond and the other is a double bond or both are single bonds;
A is heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²² is halo or hydroxy; and R^{22a} is halo, alkyl, hydroxyalkyl, alkynyl, or cycloalkyl; or R²² and
R^{22a} together with the carbon to which they are attached form heterocycloalkyl; and
R^{22b} is hydrogen or alkyl; or
a single stereoisomer or tautomer or mixture thereof, optionally as a pharmaceutically acceptable salt or solvate thereof.

In another aspect, disclosed is a compound of Formula XI: 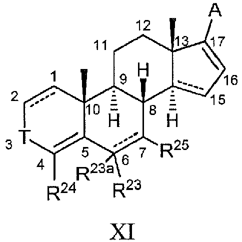 where
each 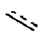 is independently a single or double bond;
T is C(O), C(=N-OH), or C(=N-O(alkyl));
A is a 5- or 6-membered heteroaryl optionally substituted with 1, 2, 3, or 4 R⁴; each R⁴ is independently halogen, cyano, hydroxy, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen, halo, alkyl, hydroxyalkyl, cycloalkyl, phenyl, or heteroaryl and R^{23a} is hydrogen or is absent when the bond between carbons 6 and 7 is a double bond; or R²³ and R^{23a} together with the carbon to which they are attached form C=O, C=CH₂ or cycloalkyl;
R²⁴ is hydrogen, hydroxy, or alkyl;
R²⁵ is hydrogen; or R²³ and R²⁵ together with the carbons to which they are attached form cycloalkyl; and
when all 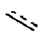 are single bonds, then
a) one of R²³, R²⁴, and R²⁵ is not hydrogen, or
b) R²³ and R²⁵ together with the carbons to which they are attached form cycloalkyl; and when A is unsubstituted pyridinyl or pyridinyl substituted with one alkyl, and R²⁴ and R²⁵ are hydrogen, then
a) R²³ is not halo, and
b) R²³ and R^{23a} do not form oxo; or
a single stereoisomer or tautomer or mixture thereof, optionally as a pharmaceutically acceptable salt or solvate thereof.

Another aspect of the invention is a compound of Formula XII 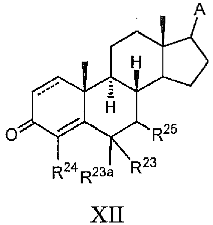 where 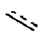 is a single bond or a double bond;
A is a heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl;
R²⁴ is hydrogen, hydroxy, or alkyl; and
R²⁵ is hydrogen; or
a single stereoisomer or tautomer or mixture thereof, optionally as a pharmaceutically acceptable salt or solvate thereof.

Another aspect of the invention is a compound of Formula XIII 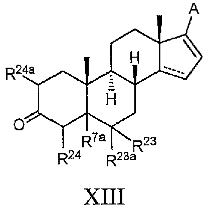 where
A is a heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms; 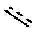 is a single bond and at least one of R²³ and R^{7a} is not hydrogen; or 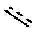 is a double bond;
R^{7a} is hydrogen or hydroxy;
R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl; and
R²⁴ and R^{24a} are independently hydrogen, hydroxy, or alkyl; or
a single stereoisomer or tautomer or mixture thereof, optionally as a pharmaceutically acceptable salt or solvate thereof.

Another aspect of the invention is a compound of Formula XIV 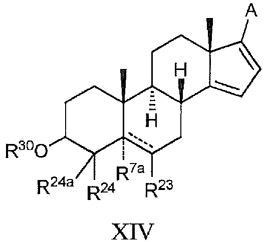 where 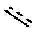 is a single bond or a double bond;
A is a heteroaryl optionally substituted with 1, 2, 3, or 4 R⁴;
provided that A is not unsubstituted benzimidazolyl; A is not furyl; and A is not pyridinyl optionally substituted with alkyl when both R²⁴ and R^{24a} are hydrogen;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen or alkyl and R^{7a} is hydrogen; or R^{7a} and R²³ together with the carbons to which they are attached form oxiranyl;
R²⁴ and R^{24a} are independently hydrogen or alkyl; and
R³⁰ is hydrogen or alkylcarbonyl where the alkyl is optionally substituted with one or two groups independently selected from hydroxy, amino, alkylamino, and dialkylamino;
provided that when R³⁰ is hydroxy, then A is not unsubstituted imidazolyl;
provided that when R³⁰ is hydroxy or alkylcarbonyloxy, then A is not unsubstituted pyridinyl or pyridinyl substituted with one alkyl; or
a single stereoisomer or tautomer or mixture thereof, optionally as a pharmaceutically acceptable salt or solvate thereof.

Another aspect of the invention is a compound of Formula XV 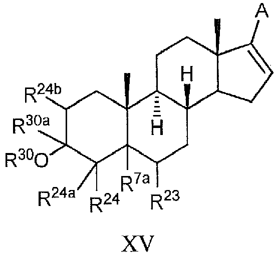 where
A is a heteroaryl optionally substituted with 1, 2, 3, or 4 R⁴;
provided that
A is not un substituted benzimidazolyl, unsubstituted benzotriazolyl, unsubstituted triazolyl, unsubstituted imidazolyl, unsubstituted pyrimidinyl, or unsubstituted pyrazinyl;
A is not furyl; and
A is not pyridinyl optionally substituted with alkyl when R³⁰ and both R²⁴ and R^{7a} are hydrogen;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen, halo, or alkyl; or R²³ and R^{7a} together with the carbons to which they are attached form cycloalkyl;
R^{7a} is hydrogen, alkyl, or hydroxy; and R²⁴, R^{24a}, and R^{24b} are independently hydrogen or alkyl; or R²⁴ and R^{7a} together with the carbon to which they are both attached form cycloalkyl, R^{24a} is hydrogen, and R^{24b} is hydrogen or alkyl; and
R³⁰ is hydrogen or alkyl and R^{30a} is hydrogen or R³⁰ and R^{30a} together with the carbon to which they are attached form oxiranyl or 2(3*H*)-oxo-dihydrofuranyl; or
a single stereoisomer or tautomer or mixture thereof, optionally as a pharmaceutically acceptable salt or solvate thereof.

Another aspect of the invention is a compound of Formula XVI 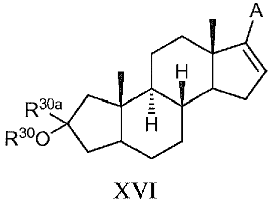 where
A is a heteroaryl optionally substituted with 1, 2, 3, or 4 R⁴; provided that A is not pyridinyl optionally substituted with alkyl;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R³⁰ is hydrogen, alkyl, or halo; and
R^{30a} is hydroxy or halo; or
a single stereoisomer or tautomer or mixture thereof, optionally as a pharmaceutically acceptable salt or solvate thereof.

Another aspect of the invention is a compound of Formula XVII 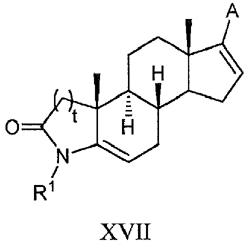 where
t is 1 or 3;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl;
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
each R¹³ is independently hydrogen or alkyl; and
each R^{13a} is independently hydrogen, alkyl, haloalkyl, alkoxyalkyl, cycloalkyl, aryl, arylalkyl, heteroaryl, or heteroarylalkyl; or
a single stereoisomer or tautomer or mixture thereof, optionally as a pharmaceutically acceptable salt or solvate thereof.

Another aspect is a pharmaceutical composition comprising a compound having a structure of Formula (Z), IX, X, XI, XII, XIII, XIV, XV, XVI, or XVII, or a single stereoisomer or tautomer or mixture thereof, optionally as a pharmaceutically acceptable salt or solvate thereof, and a pharmaceutically acceptable carrier, excipient or binder thereof. In some cases, the pharmaceutical composition is an oral dosage form.

In another aspect, disclosed is a method for treating cancer in a subject comprising administering to a subject in need thereof a therapeutically effective amount of a compound having the structure of Formula (Z), IX, X, XI, XII, XIII, XIV, XV, XVI, or XVII, or a single stereoisomer or tautomer or mixture thereof, optionally as a pharmaceutically acceptable salt or solvate thereof.

In another aspect, disclosed is a method for treating cancer in a subject comprising administering to a subject in need thereof a therapeutically effective amount of a compound having the structure of Formula (Z), IX, X, XI, XII, XIII, XIV, XV, XVI, or XVII, or a single stereoisomer or tautomer or mixture thereof, optionally as a pharmaceutically acceptable salt or solvate thereof in combination with an additional therapy selected from the group consisting of surgery, radiation therapy, chemotherapy, gene therapy, immunotherapy, and a combination thereof.

Also disclosed herein is a method of inhibiting CYP11B, CYP17, and/or CYP21 enzymes comprising contacting a compound having the structure of Formula (Z), IX, X, XI, XII, XIII, XIV, XV, XVI, or XVII, or a pharmaceutically acceptable salt or solvate thereof with a CYP11B, CYP17, and/or CYP21 enzyme.

Also described herein is a method of treating an androgen-dependent disorder in a subject comprising administering to a subject in need thereof a therapeutically effective amount of a compound having the structure of Formula ((Z), IX, X, XI, XII, XIII, XIV, XV, XVI, or XVII, or a pharmaceutically acceptable salt or solvate thereof.

Presented herein is a method of treating a proliferative disease comprising administering to a subject in need thereof a therapeutically effective amount of a compound having the structure of Formula (Z), IX, X, XI, XII, XIII, XIV, XV, XVI, or XVII, or a pharmaceutically acceptable salt or solvate thereof.

In another aspect, disclosed is a method of treating a disease associated with hypercortisolism comprising administering to a subject in need thereof a therapeutically effective amount of a compound having the structure of Formula (Z), IX, X, XI, XII, XIII, XIV, XV, XVI, or XVII, or a pharmaceutically acceptable salt or solvate thereof.

### DETAILED DESCRIPTION OF THE INVENTION

In certain embodiments, stereoisomers including enantiomers and diastereoisomers, and chemically protected forms of compounds having a structure represented by Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, X1, XIa, XIb, XIc, XId, XIe, XIf, XIg, XIh, XII, XIII, XIV, XIV-1, XV, XVI, or XVII are also provided.

In some aspects, compounds provided herein have the structure of Formula Z, I, IA, IB, IC, ID, IE, IF, IG, IH, D, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII or pharmaceutically acceptable salts, solvates, esters, acids and prodrugs thereof.

In some or any embodiments, isomers and chemically protected forms of compounds having a structure represented by Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII are also provided.

Provided herein are compounds having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII or pharmaceutically acceptable salts or solvates thereof, in the treatment of cancer, in the inhibition of CYP11B, CYP17, and/or CYP21, and/or in the treatment of androgen-dependent diseases.

The following paragraphs present a number of embodiments of compounds of the invention. In each instance the embodiment includes both the recited compounds as well as a single stereoisomer or mixture of stereoisomers thereof, as well as a pharmaceutically acceptable salt thereof.

The compound of any of the aspects of the invention or of any of the following embodiments is that where A is pyridin-3-yl optionally substituted at the 5-position with an R⁴ group. In another embodiment, the compound of any of the aspects of the invention or of any of the following embodiments is that where A is pyridin-3-yl optionally substituted at the 5-position with an R⁴ group where R⁴ is alkyl, halo, haloalkyl, or alkoxy. In another embodiment, the compound of any of the aspects of the invention or of any of the following embodiments is that where A is pyridin-3-yl optionally substituted at the 5-position with an R⁴ group where R⁴ is fluoro. When A is 3-fluoro-pyridin-5-yl, the compounds exhibit or are expected to exhibit better pk than the unsubstituted pyridin-3-yl.

Embodiment 1: In another embodiment, disclosed is a compound of Formula (Z) having the structure of Formula (I): 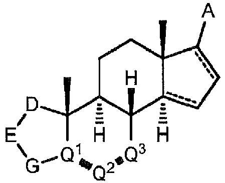 where Q¹'''''Q²'''''Q³ is -CH-CH=CH-, -C=CH-CH₂-, -CH-CH₂-CH₂-, -CH-CH(OH)-CH₂-, -CH-C(O)CH₂-, or ;
each 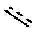 is independently a single bond or a double bond;
A is a heteroaryl optionally substituted with 1, 2, 3, or 4 R⁴;
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
D is (CH₂)_{d} where d is an integer from 1 to 3;
E is CH₂, CR¹⁴R^{14a}, O, NR¹, N-COR¹, N-S(O)₀₋₂(alkyl), or N-COOR¹;
G is CH(CH₃), C(CH₃)₂, or (CH₂)ₑ where e is an integer from 1 to 3,
provided that when E is CH₂ and d + e is 3, then A is not unsubstituted furyl, unsubstituted thienyl, oxadiazolyl substituted with alkyl or phenyl, or thiadiazolyl substituted with alkyl;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR^{15a}R^{15b}, -NR¹⁵C(O)OR^{15a}, or -NR¹⁵R^{15a};
provided that when R^{14a} is -NR¹⁵R^{15a}, then Q¹'''''Q²'''''Q³ is not -CH-CH₂-CH₂- and A is monocyclic heteroaryl;
each R¹⁵ and R^{15b} is independently hydrogen, alkyl, or haloalkyl; and
each R^{15a} are independently alkyl, haloalkyl, cycloalkyl, or heteroaryl where the heteroaryl is optionally substituted with one or two alkyl.

Embodiment 1a: The compound of Formula I is according to Formula IH 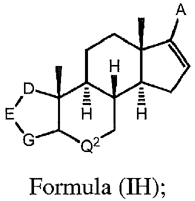 where
Q² is CH₂, CH(OH), or C(O);
D is (CH₂)_{d} where d is an integer from 1 to 3; E is CH₂, CR¹⁴R^{14a}, O, NR¹, N-COR¹, N-S(O)₀₋₂(alkyl), or N-COOR¹;
G is CH(CH₃), C(CH₃)₂, or (CH₂)ₑ where e is an integer from 1 to 3,
provided that when E is CH₂ and d + e is 3, then A is not unsubstituted furyl, unsubstituted thienyl, oxadiazolyl substituted with alkyl or phenyl, or thiadiazolyl substituted with alkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two hetero atoms;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR^{15a}R^{15b}, -NR¹⁵C(O)OR^{15a}, or -NR¹⁵R^{15a};
provided that when R^{14a} is -NR¹⁵R^{15a}, then Q¹'''''Q²'''''Q³ is not -CH-CH₂-CH₂- and A is monocyclic heteroaryl;
each R¹⁵ and R^{15b} is independently hydrogen, alkyl, or haloalkyl; and
each R^{15a} are independently alkyl, haloalkyl, cycloalkyl, or heteroaryl where the heteroaryl is optionally substituted with one or two alkyl.

Embodiment 1b: The compound of Formula IH is that where A is monocyclic heteroaryl optionally substituted with 1 or 2 R⁴; and R⁴ and all other groups are as defined in any of embodiments 1 and 1a. The compound of Formula IH is that where A is triazolyl, imidazolyl, or pyridinyl, each of which is optionally substituted with 1 or 2 R⁴; and R⁴ and all other groups are as defined in any of embodiments 1 and 1a. The compound of Formula IH is that where A is unsubstituted triazolyl and all other groups are as defined in any of embodiments 1 and 1a. The compound of Formula IH is that where A is unsubstituted imidazolyl and all other groups are as defined in any of embodiments 1 and 1a. The compound of Formula IH is that where A is pyridinyl optionally substituted with 1 or 2 R⁴; and R⁴ and all other groups are as defined in any of embodiments 1 and 1a. The compound of Formula IH is that where A is pyridinyl optionally substituted with 1 or 2 groups independently selected from alkyl, alkoxy, or halo; and R⁴ and all other groups are as defined in any of embodiments 1 and 1a. The compound of Formula IH is that where A is pyridinyl optionally substituted with 1 methyl, ethyl, methoxy, ethoxy, chloro, or fluoro; and all other groups are as defined in any of embodiments 1 and 1a.

Embodiment 1c: The compound of Formula IH is that where D is -CH₂CH₂- or -CH₂CH₂CH₂-; E is O; and G is -CH₂- or -CH₂CH₂-; and all other groups are as defined in any of embodiments 1, 1a, and 1b. The compound of Formula IH is that where D is -CH₂- and G is -CH₂CH₂- or D is -CH₂CH₂- and G is -CH₂- or D is -CH₂CH₂- and G is -CH₂CH₂- or D -CH₂CH₂CH₂- is and G is -CH₂-; E is O; and all other groups are as defined in any of embodiments 1, 1a, and 1b. In another embodiment, the compound of Formula IH is that where A is pyridinyl optionally substituted with one alkyl, alkoxy, or halo; and all other groups are as defined in any of embodiments 1 and 1a. In another embodiment, the compound of Formula IH is that where A is pyridinyl optionally substituted with one methyl, ethyl, methoxy, ethoxy, or fluoro; and all other groups are as defined in any of embodiments 1 and 1a.

Embodiment 1d: The compound of Formula IH is that where D is -CH₂CH₂- or -CH₂CH₂CH₂-; E is NR¹, N-C(O)R¹, N-S(O)₀₋₂(alkyl), or N-C(O)OR¹; G is -CH₂- or -CH₂CH₂-; and all other groups are as defined in any of embodiments 1, 1a, and 1b. In another embodiment, the compound of Formula IH is that where D is -CH₂CH₂- and G is -CH₂CH₂- or D is or -CH₂CH₂CH₂- and G is -CH₂- or D is or -CH₂CH₂- and G is -CH₂- or D is or -CH₂- and G is -CH₂CH₂-; E is NR¹, N-C(O)R¹, N-S(O)₀₋₂(alkyl), or N-C(O)OR¹; and all other groups are as defined in any of embodiments 1, 1a, and 1b. In another embodiment, the compound of Formula IH is that where R¹ is alkyl; and all other groups are as defined in embodiment 1d. In another embodiment, the compound of Formula IH is that where R¹ is methyl, ethyl, or propyl; and all other groups are as defined in embodiment 1d. In another embodiment, the compound of Formula IH is that where A is triazolyl, imidazolyl, or pyridinyl each of which is optionally substituted with one or two R⁴; and all other groups are as defined in any of embodiments 1 and 1a. In another embodiment, the compound of Formula IH is that where A is unsubstituted triazolyl, unsubstituted imidazolyl, or pyridinyl optionally substituted with one or two alkyl, alkoxy, or halo; and all other groups are as defined in any of embodiments 1 and 1a, In another embodiment, the compound of Formula IH is that where A is unsubstituted triazolyl, unsubstituted imidazolyl, or unsubstituted pyridinyl; and all other groups are as defined in any of embodiments 1 and 1a.

Embodiment 1e: The compound of Formula IH is that where D is -CH₂- or -CH₂CH₂-; E is CH₂ or CR¹⁴R^{14a}; G is -CH₂-, CH(CH₃), or -CH₂CH₂-; and all other groups are as defined in any of embodiments 1, 1a, and 1b. In another embodiment, the compound of Formula IH is that where -D-E-G- is -CH₂CH₂CH₂-, -CH₂CH₂CH₂CH₂-, or -CH₂CH₂CH₂CH₂CH₂-; and all other groups are as defined in any of embodiments 1, 1a, and 1b. In another embodiment, the compound of Formula IH is that where -D-E-G- is -CH₂CH₂CH₂-, -CH₂CH₂CH₂CH₂-, or -CH₂CH₂CH₂CH₂CH₂-; A is pyridinyl optionally substituted with one or two R⁴; and R⁴ and all other groups are as defined in any of embodiments 1 and 1a. In another embodiment, the compound of Formula IH is that where -D-E-G- is -CH₂CH₂CH₂-, -CH₂CH₂CH₂CH₂-, or -CH₂CH₂CH₂CH₂CH₂-; A is pyridinyl optionally substituted with one or two R⁴; and R⁴ is alkyl, alkoxy, or halo; and all other groups are as defined in any of embodiments 1 and 1a. In another embodiment, the compound of Formula IH is that where E is CR¹⁴R^{14a}; D is -CH₂- and G is -CH₂CH₂-, or D is -CH₂CH₂-and G is -CH₂- or CH(CH₃); and all other groups are as defined in any of embodiments 1, 1a, and 1b. In another embodiment, the compound of Formula IH is that where R¹⁴ is hydrogen; and all other groups are as defined in any of embodiments 1, 1a, 1b, and 1e. In another embodiment, the compound of Formula IH is that where R^{14a} is N₃; and all other groups are as defined in any of embodiments 1, 1a, 1b, and 1e. In another embodiment, the compound of Formula IH is that where R^{14a} is NR¹⁵C(O)R^{15a}; and all other groups are as defined in any of embodiments 1, 1a, 1b, and 1e. In another embodiment, the compound of Formula IH is that where R^{14a} is NR¹⁵C(O)NR^{15a}R^{15b}; and all other groups are as defined in any of embodiments 1, 1a, 1b, and 1e. In another embodiment, the compound of Formula IH is that where R^{14a} is NR¹⁵C(O)OR^{15a}; and all other groups are as defined in any of embodiments 1, 1a, 1b, and 1e. In another embodiment, the compound of Formula IH is that where R¹⁵ is hydrogen, alkyl, or haloalkyl and R^{15a} is alkyl, haloalkyl, or cycloalkyl; and all other groups are as defined in any of embodiments 1, 1a, 1b, and 1e. In another embodiment, the compound of Formula IH is that where R¹⁵ is hydrogen and R^{15a} is alkyl, haloalkyl, or cycloalkyl; and all other groups are as defined in any of embodiments 1, 1a, 1b, and 1e. In another embodiment, the compound of Formula IH is that where R¹⁵ is hydrogen and R^{15a} is methyl, ethyl, n-propyl, iso-propyl, t-butyl, trifluoromethyl, or cyclopropyl; and all other groups are as defined in any of embodiments 1, 1a, 1b, and 1e. In another embodiment, the compound of Formula IH is that where R¹⁵ is hydrogen and R^{15a} is cycloalkyl; and all other groups are as defined in any of embodiments 1, 1a, 1b, and 1e. In another embodiment, A is imidazolyl or pyridinyl each of which is optionally substituted with one or two groups independently selected from alkyl, alkoxy, and halo; and all other groups are as defined in any of embodiments 1, 1a, or 1e. In another embodiment, A is unsubstituted imidazolyl or pyridinyl optionally substituted with one or two groups independently selected from alkyl, alkoxy, and halo; and all other groups are as defined in any of embodiments 1, 1a, or 1e. In another embodiment, A is pyridinyl optionally substituted with one methyl, ethyl, methoxy, ethyoxy, chloro, or fluoro; and all other groups are as defined in any of embodiments 1, 1a, or 1e.

The compound of Formula IH where D is CH₂CH₂, E is CH₂ or N-C(O)R¹, and G is CH₂ is an inhibitor of CYP21 and/or CYP11B and/or CYP17.

Embodiment 1f: The compound of Formula I is according to Formula IJ 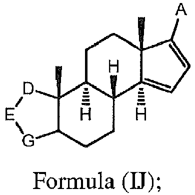 where
D is (CH₂)_{d} where d is an integer from 1 to 3;
E is CH₂, CR¹⁴R^{14a}, O, NR¹, N-COR¹, N-S(O)₀₋₂(alkyl), or N-COOR¹;
G is CH(CH₃), C(CH₃)₂, or (CH₂)ₑ where e is an integer from 1 to 3, provided that when E is CH₂ and d + e is 3, then A is not unsubstituted furyl, unsubstituted thienyl, oxadiazolyl substituted with alkyl or phenyl, or thiadiazolyl substituted with alkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR^{15a}R^{15b}, -NR¹⁵C(O)OR^{15a}, or -NR¹⁵R^{15a};
provided that when R^{14a} is NR¹⁵R^{15a}, then Q¹'''''Q²'''''Q³ is not -CH-CH₂-CH₂- and A is monocyclic heteroaryl;
each R¹⁵ and R^{15b} is independently hydrogen, alkyl, or haloalkyl; and
each R^{15a} are independently alkyl, haloalkyl, cycloalkyl, or heteroaryl where the heteroaryl is optionally substituted with one or two alkyl.

Embodiment 1g: The compound of Formula U is that where A is imidazolyl or pyridinyl each of which is optionally substituted with one or two R⁴ groups; and R⁴ and all other groups are as defined in embodiment 1f. In another embodiment, the compound of Formula U is that where A is unsubstituted imidazolyl or pyridinyl optionally substituted with one or two R⁴ groups; and R⁴ and all other groups are as defined in embodiment 1f. In another embodiment, the compound of Formula IJ is that where A is pyridinyl optionally substituted with one or two groups independently selected from alkyl, alkoxy, and halo; and all other groups are as defined in embodiment 1f. In another embodiment, the compound of Formula IJ is that where A is pyridinyl optionally substituted with methyl, methoxy, chloro, or fluoro; and all other groups are as defined in embodiment 1f.

Embodiment 1h: The compound of Formula IJ is that where D is -CH₂CH₂-; E is CR¹⁴R^{14a} or N-S(O)₂(alkyl); and G is -CH₂-; and all other groups are as defined in any of embodiments 1f and 1g. In another embodiment, the compound of Formula IJ is that where D is -CH₂CH₂-; E is N-S(O)₂(alkyl); and G is -CH₂- and all other groups are as defined in any of embodiments 1f and 1g. In another embodiment, the compound of Formula IJ is that where D is -CH₂CH₂-; E is CR¹⁴R^{14a}; and G is -CH₂-; and all other groups are as defined in any of embodiments 1f and 1g. In another embodiment, the compound of Formula IJ is that where R¹⁴ is hydrogen and R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(O)OR^{15a}, or -NR¹⁵R^{15a}; and all other groups are as defined in any of embodiments 1f, 1g, and 1h. In another embodiment, the compound of Formula IJ is that where R¹⁴ is hydrogen and R^{14a} is -NR15C(O)R^{15a}; and all other groups are as defined in any of embodiments 1f, 1g, and 1h. In another embodiment, the compound of Formula IJ is that where each R¹⁵ is hydrogen and each R^{15a} is alkyl, haloalkyl, cycloalkyl, or heteroaryl where the heteroaryl is optionally substituted with one or two alkyl; and all other groups are as defined in any of embodiments 1f, 1g, and 1h. In another embodiment, the compound of Formula U is that where each R¹⁵ is hydrogen and each R^{15a} is alkyl; and all other groups are as defined in any of embodiments 1f, 1g, and 1h.

Embodiment 1j: The compound of Formula I is according to Formula IK 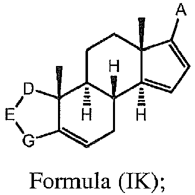 where
D is (CH₂)_{d} where d is an integer from 1 to 3;
E is CH₂, CR¹⁴R^{14a}, O, NR¹, N-COR¹, N-S(O)₀₋₂(alkyl), or N-COOR¹;
G is CH(CH₃), C(CH₃)₂, or (CH₂)ₑ where e is an integer from 1 to 3,
provided that when E is CH₂ and d + e is 3, then A is not unsubstituted furyl, unsubstituted thienyl, oxadiazolyl substituted with alkyl or phenyl, or thiadiazolyl substituted with alkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R¹⁵b, -NR¹⁵C(S)NR^{15a}R^{15b}, -NR¹⁵C(O)OR^{15a}, or -NR¹⁵R^{15a};
provided that when R^{14a} is -NR¹⁵R^{15a}, then A is monocyclic heteroaryl;
each R¹⁵ and R^{15b} is independently hydrogen, alkyl, or haloalkyl; and
each R^{15a} are independently alkyl, haloalkyl, cycloalkyl, or heteroaryl where the heteroaryl is optionally substituted with one or two alkyl.

Embodiment 1k: The compound of Formula IK is that where A is imidazolyl or pyridinyl each of which is optionally substituted with one or two R⁴ groups; and R⁴ and all other groups are as defined in embodiment 1j. In another embodiment, the compound of Formula IK is that where A is unsubstituted imidazolyl or pyridinyl optionally substituted with one or two R⁴ groups; and R⁴ and all other groups are as defined in embodiment 1j. In another embodiment, the compound of Formula IK is that where A is pyridinyl optionally substituted with one or two groups independently selected from alkyl, alkoxy, and halo; and all other groups are as defined in embodiment 1j. In another embodiment, the compound of Formula IK is that where A is pyridinyl optionally substituted with methyl, methoxy, chloro, or fluoro; and all other groups are as defined in embodiment 1j.

Embodiment 1m: The compound of Formula IK is that where D is -CH₂CH₂-; E is CR¹⁴R^{14a}; and G is -CH₂-; and all other groups are as defined in any of embodiments 1j and 1k. In another embodiment, the compound of Formula IK is that where R¹⁴ is hydrogen and R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(O)OR^{15a}, or -NR¹⁵R^{15a}; and all other groups are as defined in any of embodiments 1j, 1k, and 1m. In another embodiment, the compound of Formula IK is that where R¹⁴ is hydrogen and R^{14a} is -NR¹⁵C(O)R^{15a}; and all other groups are as defined in any of embodiments 1j, 1k, and 1m. In another embodiment, the compound of Formula IK is that where R¹⁵ is hydrogen and R^{15a} is alkyl or cycloalkyl; and all other groups are as defined in any of embodiments 1j, 1k, and 1m. In another embodiment, the compound of Formula IK is that where R¹⁵ is hydrogen and R^{15a} is methyl or cyclopropyl; and all other groups are as defined in any of embodiments 1j, 1k, and 1m.

The compound of Formula IK where E is C(H)(NHC(O)R¹) is an inhibitor of CYP21 and/or CYP 11 B and/or CYP17.

Embodiment In: The compound of Formula I is according to Formula IL 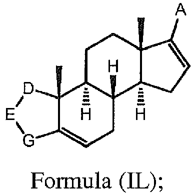 where
D is (CH₂)_{d} where d is an integer from 1 to 3;
E is CH₂, CR¹⁴R^{14a}, O, NR¹, N-COR¹, N-S(O)₀₋₂(alkyl), or N-COOR¹;
G is CH(CH₃), C(CH₃)₂, or (CH₂)ₑ where e is an integer from 1 to 3,
provided that when E is CH₂ and d + e is 3, then A is not unsubstituted furyl, unsubstituted thienyl, oxadiazolyl substituted with alkyl or phenyl, or thiadiazolyl substituted with alkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R¹⁴ is hydrogen or alkyl;
R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR^{15a}R^{15b}, -NR¹⁵C(O)OR^{15a}, or -NR¹⁵R15^{a};
provided that when R^{14a} is -NR¹⁵R^{15a}, then A is monocyclic heteroaryl;
each R¹⁵ and R^{15b} is independently hydrogen, alkyl, or haloalkyl; and
each R^{15a} are independently alkyl, haloalkyl, cycloalkyl, or heteroaryl where the heteroaryl is optionally substituted with one or two alkyl.

Embodiment 1p: The compound of Formula IL is that where A is imidazolyl or pyridinyl each of which is optionally substituted with one or two R⁴ groups; and R⁴ and all other groups are as defined in embodiment 1n. In another embodiment, the compound of Formula IL is that where A is unsubstituted imidazolyl or pyridinyl optionally substituted with one or two R⁴ groups; and R⁴ and all other groups are as defined in embodiment 1n. In another embodiment, the compound of Formula IL is that where A is pyridinyl optionally substituted with one or two groups independently selected from alkyl, haloalkyl, alkoxy, and halo; and all other groups are as defined in embodiment 1n. In another embodiment, the compound of Formula IL is that where A is pyridinyl optionally substituted with methyl, ethyl, difluoromethyl, trifluoromethyl, methoxy, or fluoro; and all other groups are as defined in embodiment 1n.

Embodiment 1q: The compound of Formula IL is that where D is -CH₂-; E is -CH₂- or CR¹⁴R^{14a}; G is -CH₂CH₂-; and all other groups are as defined in any of embodiments 1n and 1p. In another embodiment, the compound of Formula IL is that where D is -CH₂-; E is -CH₂-; G is -CH₂CH₂-; and all other groups are as defined in any of embodiments In and 1p. In another embodiment, the compound of Formula IL is that where D is -CH₂-; E is CR¹⁴R^{14a}; G is -CH₂CH₂-; and all other groups are as defined in any of embodiments In and 1p. In another embodiment, the compound of Formula IL is that where R¹⁴ is hydrogen and R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(O)OR^{15a}, or -NR¹⁵R^{15a}; and all other groups are as defined in any of embodiments In, 1p, and 1q. In another embodiment, the compound of Formula IL is that where R¹⁴ is hydrogen and R^{14a} is halo, -NR¹⁵S(O)₂R^{15a}, -NR¹⁵C(O)R^{15a}, -NR¹⁵C(O)NR^{15a}R^{15b}, or -NR¹⁵C(O)OR^{15a}; and all other groups are as defined in any of embodiments In, 1p, and 1q. In another embodiment, the compound of Formula IL is that where R¹⁴ is hydrogen and R^{14a} is -NR¹⁵S(O)₂R^{15a}, -NR¹⁵C(O)R^{15a}, -NR¹⁵C(O)NR^{15a}R^{15b}, or -NR¹⁵C(O)OR^{15a}; and all other groups are as defined in any of embodiments In, 1p, and 1q. In another embodiment, the compound of Formula IL is that where R¹⁵ and R¹⁵ are hydrogen and R^{15a} is alkyl, cycloalkyl, or heteroaryl; and all other groups are as defined in any of embodiments In, 1p, and 1q. In another embodiment, the compound of Formula IL is that where R¹⁵ and R¹⁵ are hydrogen and R^{15a} is methyl, ethyl, propyl, cyclopropyl, cyclobutyl, or thiazolyl; and all other groups are as defined in any of embodiments 1n, 1p, and 1q.

Embodiment 2: In some or any embodiments, disclosed is a compound of Formula (Z) having the structure of Formula (IA): 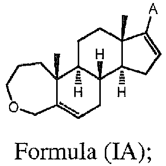 or a single stereoisomer or tautomer or mixture thereof, optionally as a pharmaceutically acceptable salt, solvate, or prodrug thereof, where A, R² and R³ are as defined in any of embodiments 1, 1a, 1b, 1c, In, and 1p.

Embodiment 3: In some or any embodiments, disclosed is a compound of Formula (Z) having the structure of Formula (IB): 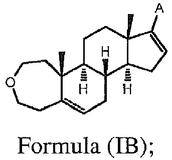 or a single stereoisomer or tautomer or mixture thereof, optionally as a pharmaceutically acceptable salt, solvate, or prodrug thereof, where A, R² and R³ are as defined in any of embodiments 1, 1a, 1b, 1c, In, and 1p.

Embodiment 4: In some or any embodiments, disclosed is a compound of Formula (Z) having the structure of Formula (IC): 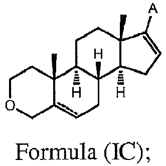 or a pharmaceutically acceptable salt, solvate, stereoisomer, tautomer or prodrug thereof, where A, R² and R³ are as defined in any of embodiments 1, 1a, 1b, 1c, 1n, and 1p.

Embodiment 5: In some or any embodiments, disclosed is a compound of Formula (Z) having the structure of Formula (ID): 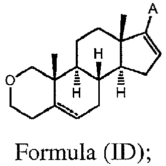 or a single stereoisomer or tautomer or mixture thereof, optionally as a pharmaceutically acceptable salt, solvate, or prodrug thereof, where A, R² and R³ are as defined in any of embodiments 1, 1a, 1b, 1c, In, and 1p.

Embodiment 6: In some or any embodiments, disclosed is a compound of Formula (Z) having the structure of Formula (IE): 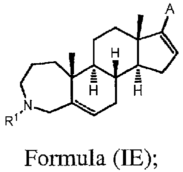 or a single stereoisomer or tautomer or mixture thereof, optionally as a pharmaceutically acceptable salt, solvate, or prodrug thereof, where A, R¹, R² and R³ are as defined in any of embodiments 1, 1a, 1b, 1d, In, and 1p. The compound of Formula IE is an inhibitor of CYP21 and/or CYP11B and/or CYP 17.

Embodiment 7: In some or any embodiments, disclosed is a compound of Formula (Z) having the structure of Formula (IF): 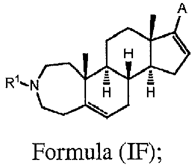 or a single stereoisomer or tautomer or mixture thereof, optionally as a pharmaceutically acceptable salt, solvate, or prodrug thereof, where A, R¹, R² and R³ are as defined in any of embodiments 1, 1a, 1b, 1d, In, and 1p. The compound of Formula IF is an inhibitor of CYP21 and/or CYP 11 B and/or CYP17.

Embodiment 8: In some or any embodiments, disclosed is a compound of Formula (Z) having the structure of Formula (IG): 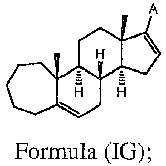 or a single stereoisomer or tautomer or mixture thereof, optionally as a pharmaceutically acceptable salt, solvate, or prodrug thereof, where A, R² and R³ are as defined in any of embodiments 1, 1a, 1b, 1e, In, 1p, and 1q.

Embodiment 8a: The compound of Formula I, IA, IB, IC, ID, IE, IF, IG, IH, U, IK, or IL is a CYP17 inhibitor and has an IC₅₀ of less than or equal to about 5 nM, in another example less than or equal to about 2 nM, in another example less than or equal to about 1 nM. In another embodiment, the compound of Formula I, IA, IB, IC, ID, IE, IF, IG, IH, U, IK, or IL has a T_{1/2} (min) in human liver microsomes of greater than or equal to about 80, in another example greater than or equal to about 90, in another example greater than or equal to about 100, in another example greater than or equal to about 120, in another example greater than or equal to about 140.

Embodiment 9: In another embodiment, disclosed is a compound of Formula (Z) having the structure of Formula (II): 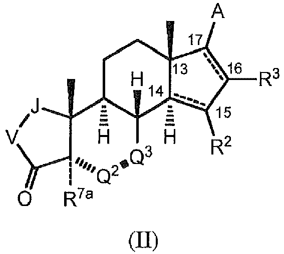 where:
each 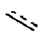 is independently a single bond or double bond;
C(R^{7a})'''''Q²'''''Q³ is C(R^{7a})-Q²=Q³ ; R^{7a} is hydrogen or hydroxy; Q² and Q³ are CH; or
C(R^{7a})'''''Q²'''''Q³ is C=Q²-Q³ ; R^{7a} is not present; Q² is CH; and Q³ is CHR⁸ or C(O) where R⁸ is hydrogen; or
C(R^{7a})'''''Q²'''''Q³ is C(R^{7a})-Q²-Q³ ; R^{7a} is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7a} and R^{7b} together with the carbons to which they are attached form cycloalkyl or heterocycloalkyl;
b) R^{7b} and R^{7c} together with the carbon to which they are attached form C=CH₂, cycloalkyl or carbonyl; or
c) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B};
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
V is (CH₂), O, NR¹, N-COR¹or N-COOR¹; and
J is (CH₂)₁₋₃.
In another embodiment, the Compound of Formula II is that where A is heteroaryl optionally substituted with one R⁴; and all other groups are as defined in embodiment 9. In another embodiment, the Compound of Formula II is that where A is imidazolyl, triazolyl, or pyridinyl optionally substituted with one R⁴; and all other groups are as defined in embodiment 9. In another embodiment, the Compound of Formula II is that where A is unsubstituted imidazolyl, unsubstituted triazolyl, or pyridinyl optionally substituted with one alkyl, alkoxy, halo, or haloalkyl; and all other groups are as defined in embodiment 9. In another embodiment, the Compound of Formula II is that where A is unsubstituted pyridinyl; and all other groups are as defined in embodiment 9.

Embodiment 9a: The compound of Formula II is that where R² and R³ are hydrogen; and all other groups are as defined in embodiment 9.

Embodiment 9b: The compound of Formula II is that where C(R^{7a})'''''Q²'''''Q³ is C(R7a)-Q²-Q³ ; R^{7a} is hydrogen; Q² is CH₂; Q³ is CH₂; and all other groups are as defined in any of embodiments 9 and 9a.

Embodiment 9c: The compound of Formula II is according to Formula IIE 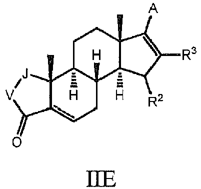 where A, R² and R³ are as defined in any of embodiments 9 and 9a. In another embodiment, 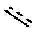 is a single bond; and all other groups are as defined in any of embodiments 9 and 9a.

Embodiment 10: The compound of Formula II is according to Formula (IIA): 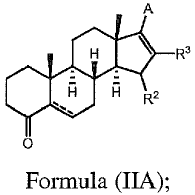 where A, R² and R³ are as defined in any of embodiments 9 and 9a. In another embodiment, 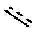 is a single bond; and all other groups are as defined in any of embodiments 9 and 9a.

Embodiment 11: The compound of Formula II is according to Formula (IIB): 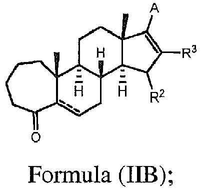 where A, R² and R³ are as defined in any of embodiments 9 and 9a. In another embodiment, 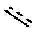 is a single bond; and all other groups are as defined in any of embodiments 9 and 9a.

Embodiment 12: The compound of Formula II is according to Formula (IIC): 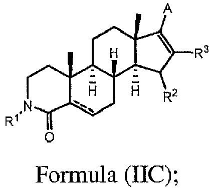 where A, R² and R³ are as defined in any of embodiments 9 and 9a. In another embodiment, 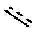 is a single bond; and all other groups are as defined in any of embodiments 9 and 9a.

Embodiment 13: The compound of Formula II is according to Formula (IID): 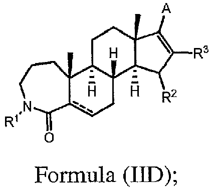 where A, R² and R³ are as defined in any of embodiments 9 and 9a. In another embodiment, 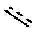 is a single bond; and all other groups are as defined in any of embodiments 9 and 9a.

In another embodiment, the compound of Formula II is a CYP17 inhibitor.

Embodiment 14: In some or any embodiments, disclosed is a compound of Formula (Z) having the structure of Formula (III): 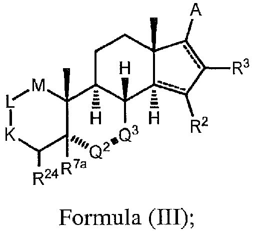 where:
each 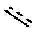 is independently a single bond or a double bond;
C(R^{7a})'''''Q²'''''Q³ is C(^{R7a})-Q²=Q³ ; where R^{7a} is absent or is hydrogen or hydroxy; and Q² and Q³ are CH; or
C(R^{7a})'''''Q²'''''Q³ is C(^{R7a})=Q²-Q³ ; R⁷_{,} is absent; Q² is CH; and Q³ is CHR⁸ or C(O) where R⁸ is hydrogen; or
C(R^{7a})'''''Q²'''''Q³ is C(R^{7a})-Q²-Q³ ; R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7a}, when present, and R^{7b} together with the carbons to which they are attached form cycloalkyl or heterocycloalkyl;
b) R^{7b} and R^{7c} together with the carbon to which they are attached form C=CH₂, cycloalkyl, or carbonyl; or
c) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;
K is C(O), NR¹, N-C(O)R¹ or N-C(O)OR¹;
L is CH₂, C(O), NR¹, N-C(O)R¹ or N-C(O)OR¹; and
M is (CH=CH) or (CH₂)_{g} where g is an integer 2 or 3,
R²⁴ is hydrogen or alkyl; or when R^{7a} is present, R²⁴ and R^{7a} together with the carbons to which they are attached form cycloalkyl;
provided that when g is 2, K is C(O) and L is NR¹, N-C(O)R¹or N-C(O)OR¹; when g is 2, K is NR¹, N-C(O)R¹or N-C(O)OR¹ and L is C(O); then the bond between carbons 14 and 15 is a double bond; and
provided that g cannot be 2 when K is NR¹, N-C(O)R¹or N-C(O)OR¹ and L is or CH₂.

Embodiment 14a: In another embodiment, the compound of Formula (III) is that where A is heteroaryl optionally substituted with one or two R⁴; R⁴ and all other groups are as defined in embodiment 14. In another embodiment, the compound of Formula (III) is that where A is imidazolyl, triazolyl, pyrazinyl, or pyridinyl, each of which is optionally substituted with one R⁴; R⁴ and all other groups are as defined in embodiment 14. In another embodiment, the compound of Formula (III) is that where A is unsubstituted imidazolyl, unsubstituted triazolyl, unsubstituted pyrazinyl, or pyridinyl optionally substituted with alkyl, haloalkyl, halo, or alkoxy; R⁴ and all other groups are as defined in embodiment 14. In another embodiment, the compound of Formula (III) is that where A is unsubstituted imidazolyl, unsubstituted triazolyl, unsubstituted pyrazinyl, or pyridinyl optionally substituted with alkyl or halo; R⁴ and all other groups are as defined in embodiment 14.

Embodiment 14b: In another embodiment, the compound of Formula (III) is that where R² and R³ are hydrogen; and all other groups are as defined in any of embodiments 14 and 14a.

Embodiment 14c: In another embodiment, the compound of Formula (III) is that where C(R^{7a})'''''Q²'''''Q³ is C=CH-CH₂ or CH-CH₂-CH₂; and all other groups are as defined in any of embodiments 14, 14a, and 14b.

Embodiment 14d: In another embodiment, the compound of Formula (III) is that where K is C(O), L is CH₂, and g is 2; and all other groups are as defined in any of embodiments 14, 14a, 14b, and 14c.

Embodiment 14e: In another embodiment, the compound of Formula (III) is that where K is C(O), L is N-R¹, and g is 2; and all other groups are as defined in any of embodiments 14, 14a, 14b, and 14c. In another embodiment, the compound of Formula (III) is that where K is N-R¹, L is C(O), and g is 2; and all other groups are as defined in any of embodiments 14, 14a, 14b, and 14c. In another embodiment, the compound of Formula (III) is that where R¹ is hydrogen or alkyl; and all other groups are as defined in any of embodiments 14, 14a, 14b, 14c, and 14e.

Embodiment 14e1: In another embodiment, the compound of Formula (III) is that where R²⁴ is hydrogen; and all other groups are as defined in any of embodiments 14, 14a, 14b, 14c, 14d, and 14e. In another embodiment, the compound of Formula (III) is that where R²⁴ is alkyl, in another example methyl; and all other groups are as defined in any of embodiments 14, 14a, 14b, 14c, 14d, and 14e. In another embodiment, the compound of Formula (III) is that where R²⁴ and R^{7a} together with the carbons to which they are attached form cycloalkyl, in another example cyclopropyl; and all other groups are as defined in any of embodiments 14, 14a, 14b, 14c, 14d, and 14e.

Embodiment 14f: In another embodiment, the compound of Formula (III) is according to Formula IIIE 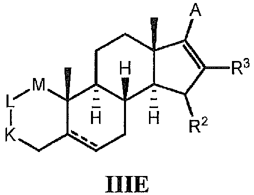 where all groups are as defined in any of embodiments 14, 14a, 14b, 14c, 14d, and 14e.

Embodiment 14g: In another embodiment, the compound of Formula (III) is according to Formula IIIF-1 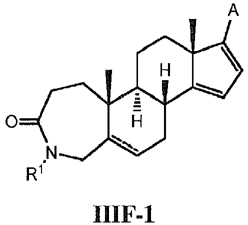 where all groups are as defined in any of embodiments 14, 14a, 14b, 14c, 14d, and 14e. In another embodiment, 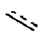 is a single bond; and all other groups are as defined in any of the embodiments 14, 14a, 14b, 14c, 14d, 14e, and 14f.

Embodiment 14g: In another embodiment, the compound of Formula (III) is according to Formula IIIF-2 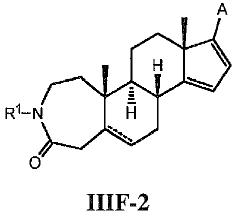 where all groups are as defined in any of embodiments 14, 14a, 14b, 14c, 14d, and 14e. In another embodiment, 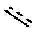 is a single bond; and all other groups are as defined in any of the embodiments 14, 14a, 14b, 14c, 14d, 14e, and 14f.

Embodiment 15: In another embodiment, the compound of Formula III is according to Formula (IIIA): 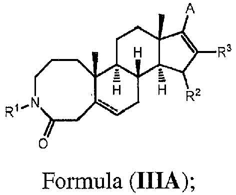 where all groups are as defined in any of embodiments 14, 14a, 14b, 14c, 14d, and 14e.

Embodiment 16: In another embodiment, the compound of Formula III is according to Formula (IIIB): 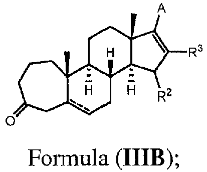 where all groups are as defined in any of embodiments 14, 14a, 14b, 14c, 14d, and 14e. In another embodiment, 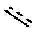 is a double bond; and all other groups are as defined in any of the embodiments 14, 14a, 14b, 14c, 14d, and 14e. In another embodiment, the compound of Formula IIIB is a CYP17 inhibitor.

Embodiment 17: In another embodiment, the compound of Formula (III) is according to Formula (IIIC): 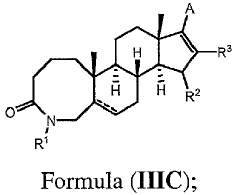 where all groups are as defined in any of embodiments 14, 14a, 14b, 14c, 14d, and 14e.

Embodiment 18: In another embodiment, the compound of Formula (III) is according to Formula (IIID): 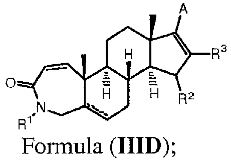 where all groups are as defined in any of embodiments 14, 14a, 14b, 14c, 14d, and 14e.

Embodiment 19: In some or any embodiments, disclosed is a compound of Formula (Z) having the structure of Formula (IV): 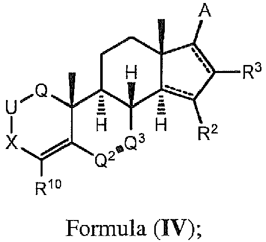 where:
each 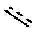 is independently a single bond or a double bond;
C'''''Q²'''''Q³ is C-Q²=Q³ ; Q² and Q³ are CH; or
C'''''Q²'''''Q³ is C-Q²-Q³ ; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
   a) R^{7b} and R^{7c} together with the carbon to which they are attached form C=CH₂, cycloalkyl, or carbonyl; or
   b) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
Q is (CH₂)ᵢ where i is an integer from 1 to 3;
U is (CH₂), CO, O, NR¹, N-COR¹or N-COOR¹; or
Q and U together are CH=CH;
X is CR^{11a}R^{11b}, C=O, C=NOR⁹, O, NR¹, N-COR¹or N-COOR¹; and
provided that i cannot be 1 when X is CO and U is CH₂;
provided that when X is CR^{11a}R^{11b}, R^{11b} is OR¹, then
   a) the bond between carbons 16 and 17 is a double bond;
   b) A is not unsubstituted benzimidazolyl, unsubstituted imidazolyl, or unsubstituted pyrazolyl; and
   c) A is not imidazolyl, thiazolyl, or oxazolyl, where each are substituted with amino, alkylamino, or dialkylamino.

Embodiment 19a: In another embodiment, the compound of Formula IV is that where A is heteroaryl optionally substituted with one or two R⁴; and R⁴ and all other groups are as defined in embodiment 19. In another embodiment, the compound of Formula IV is that where A is monocyclic heteroaryl optionally substituted with one or two R⁴; and R⁴ and all other groups are as defined in embodiment 19. In another embodiment, the compound of Formula IV is that where A is bicyclic heteroaryl optionally substituted with one or two R⁴; and R⁴ and all other groups are as defined in embodiment 19. In another embodiment, the compound of Formula IV is that where A is imidazolyl, triazolyl, tetrazolyl, pyridinyl, pyrimidinyl, pyrazinyl, pyridazinyl, or benzimidazolyl, each of which is optionally substituted with one or two R⁴; and R⁴ and all other groups are as defined in embodiment 19. In another embodiment, the compound of Formula IV is that where A is unsubstituted triazolyl, unsubstituted tetrazolyl, unsubstituted pyrimidinyl, or unsubstituted pyrazinyl, or A is pyridinyl or imidazolyl, each of which is optionally substituted with one or two R⁴; and R⁴ and all other groups are as defined in embodiment 19. In another embodiment, the compound of Formula IV is that where A is pyridinyl optionally substituted with one or two R⁴; and R⁴ and all other groups are as defined in embodiment 19.

Embodiment 19b: In another embodiment, the compound of Formula IV is that where R⁴, when present, is alkyl, alkoxy, halo, or haloalkyl; and all other groups are as defined in any of embodiments 19 and 19a. In another embodiment, the compound of Formula IV is that where R⁴, when present, is methyl, ethyl, methoxy, ehtyoxy, propoxy, fluoro, difluoromethyl, or trifluoromethyl; and all other groups are as defined in any of embodiments 19 and 19a.

Embodiment 19c: In another embodiment, the compound of Formula IV is that where C'''''Q²'''''Q³ is C-Q²-Q³ ; Q² is CH₂ or CR^{7b}R^{7c}; Q³ is CH₂ or CHR⁸; and all other groups are as defined in any of embodiments 19,19a, and 19b. In another embodiment, the compound of Formula IV is that where R⁸ is hydrogen; and all other groups are as defined in any of embodiments 19,19a, 19b, and 19c. In another embodiment, the compound of Formula IV is that where R^{7c} is hydrogen; and all other groups are as defined in any of embodiments 19,19a, 19b, and 19c. In another embodiment, the compound of Formula IV is that where R^{7b} and R⁸ together with the carbon to which they are attached form cycloalkyl; and all other groups are as defined in any of embodiments 19,19a, 19b, and 19c. In another embodiment, the compound of Formula IV is that where R^{7b} is hydrogen, alkyl, or halo; and all other groups are as defined in any of embodiments 19,19a, 19b, and 19c.

Embodiment 19d: In another embodiment, the compound of Formula IV is that where R¹, when present, is hydrogen or alkyl; and all other groups are as defined in any of embodiments 19,19a, 19b, and 19c.

Embodiment 19e: In another embodiment, the compound of Formula IV is that where R¹⁰ is hydrogen or alkyl; and all other groups are as defined in any of embodiments 19,19a, 19b, 19c, and 19d.

Embodiment 19f: In another embodiment, the compound of Formula IV is that where X is C^{R11a}R^{11b},N-R¹, or C(O); U is CH₂, N-R¹, or C(O); and Q is CH₂ or CH₂CH₂; and all other groups are as defined in any of embodiments 19,19a, 19b, 19c, 19d and 19e.

Embodiment 19g: In another embodiment, the compound of Fonnula IV is that where R² and R³ are hydrogen; and all other groups are as defined in any of embodiments 19,19a, 19b, 19c, 19d, 19e, and 19f.

Embodiment 19h: In another embodiment, the compound of Formula IV is according to Formula IVF 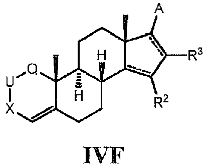 where all groups are as defined in any of embodiments 19,19a, 19b, 19d, 19f, and 19g.

Embodiment 19j: In another embodiment, the compound of Formula IV, IVA, IVB, IVC, IVD, IVE, or IVF is an inhibitor of CYP17 and has an IC₅₀ of less than or equal to about 5 nM, in another example less than or equal to about 2 nM, in another example less than or equal to about 1 nM. In another embodiment, the compound of Formula IV, IVA, IVB, IVC, IVD, IVE, or IVF has a T_{1/2} (min) in human liver microsomes of greater than or equal to about 80, in another example greater than or equal to about 90, in another example greater than or equal to about 100, in another example greater than or equal to about 120, in another example greater than or equal to about 140.

Embodiment 20: In some or any embodiments, disclosed is a compound of Formula (IV) having the structure of Formula (IVA): 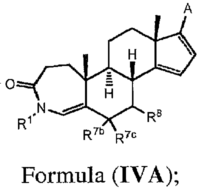 where all groups are as defined in any of embodiments 19,19a, 19b, 19c, and 19d. In another embodiment, the compound of Formula IVA is that where each 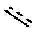 is a single bond; and all groups are as defined in any of embodiments 19,19a, 19b, 19c, and 19d. In another embodiment, the compound of Formula IVA is that where the 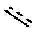 bond between carbons 16 and 17 is a double bond and the 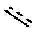 bond between carbons 14 and 15 is a single bond; and all groups are as defined in any of embodiments 19,19a, 19b, 19c, and 19d. In another embodiment, the compound of Formula IVA is that where each 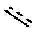 is a double bond; and all groups are as defined in any of embodiments 19,19a, 19b, 19c, and 19d. In another embodiment, the compound of Formula IVA is that where R⁸ is hydrogen or R⁸ and R^{7b} together with the carbon to which they are attached form cycloalkyl; and all groups are as defined in any of embodiments 19,19a, 19b, 19c, 19d, and 20. In another embodiment, the compound of Formula IVA is that where R⁸ and R^{7c} are hydrogen and R^{7b} is hydrogen, alkyl, or halo; and all groups are as defined in any of embodiments 19,19a, 19b, 19c, 19d, and 20.

Embodiment 21: In some or any embodiments, disclosed is a compound of Formula (IV) having the structure of Formula (IVB): 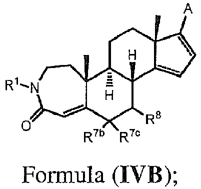 where all groups are as defined in any of embodiments 19,19a, 19b, 19c, and 19d. In another embodiment, the compound of Formula IVB is that where each 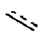 is a single bond; and all groups are as defined in any of embodiments 19,19a, 19b, 19c, and 19d. In another embodiment, the compound of Formula IVB is that where the 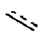 bond between carbons 16 and 17 is a double bond and the 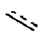 bond between carbons 14 and 15 is a single bond; and all groups are as defined in any of embodiments 19,19a, 19b, 19c, and 19d. In another embodiment, the compound of Formula IVB is that where each 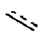 is a double bond; and all groups are as defined in any of embodiments 19,19a, 19b, 19c, and 19d. In another embodiment, the compound of Formula IVB is that where R⁸ is hydrogen or R⁸ and R^{7b} together with the carbon to which they are attached form cycloalkyl; and all groups are as defined in any of embodiments 19,19a, 19b, 19c, 19d, and 21. In another embodiment, the compound of Formula IVB is that where R⁸ and R^{7c} are hydrogen and R^{7b} is hydrogen, alkyl, or halo; and all groups are as defined in any of embodiments 19,19a, 19b, 19c, 19d, and 21. In another embodiment, the compound of Formula IVB is a CYP17 inhibitor.

Embodiment 22: In some or any embodiments, disclosed is a compound of Formula (IV) having the structure of Formula (IVC): 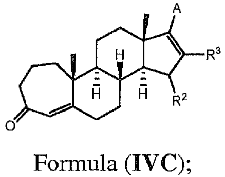 where all groups are as defined in any of embodiments 19,19a, 19b, and 19g.

Embodiment 23: In some or any embodiments, disclosed is a compound of Formula (IV) having the structure of Formula (IVD): 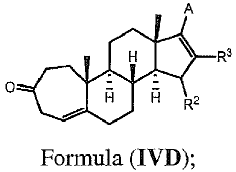 where all groups are as defined in any of embodiments 19,19a, 19b, and 19g.

Embodiment 23a: In some or any embodiments, disclosed is a compound of Formula (IV) having the structure of Formula (IVE): 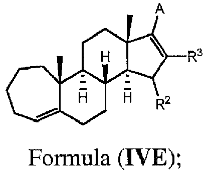 where all groups are as defined in any of embodiments 19,19a, 19b, and 19g.

Embodiment 24: In some or any embodiments, disclosed is a compound of Formula (IV) having the structure of Formula (IVG): 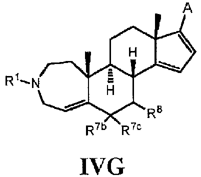 where all groups are as defined in any of embodiments 19,19a, 19b, 19c, and 19d. In another embodiment, the compound of formula IVG is that where R^{7b}, R^{7c}, and R⁸ are hydrogen; and all other groups are as defined in any of embodiments 19,19a, 19b, 19d, and 24. In another embodiment, the compound of formula IVG is that where A is pyridinyl optionally substituted with one alkyl or alkoxy; and all other groups are as defined in any of embodiments 19, 19d, and 24. In another embodiment, the compound of formula IVG is that where 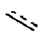 is a single bond; and all other groups are as defined in any of embodiments 19,19a, 19b, 19c, 19d, and 24. In another embodiment, the compound of Formula IVG is a CYP17 inhibitor.

Embodiment 24a: In some or any embodiments, disclosed is a compound of Formula (IV) having the structure of Formula (IVH): 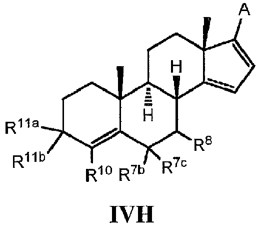 where all groups are as defined in any of embodiments 19,19a, 19b, 19c, and 19e. In another embodiment, the compound of Formula IVH is that where R^{11a} is hydrogen or alkyl and R^{11b} is hydroxy; and all other groups are as defined in and of embodiments 19,19a, 19b, 19c, and 19e. In another embodiment, the compound of Formula IVH is that where one of R¹⁰, R^{7b}, and R^{7c} is not hydrogen; and all other groups are as defined in and of embodiments 19,19a, 19b, 19c, 19e, and 24a. In another embodiment, the compound of Formula IVH is that where 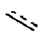 is a single bond; and all other groups are as defined in and of embodiments 19,19a, 19b, 19c, 19e, and 24a.

In another embodiment, the Compound of Formula IV where Q² is N-alkyl, U is (CH₂), Q is (CH₂)ᵢ where i is an integer from 1 to 3, and X is C(O) is a CYP17 and/or CYP11B inhibitor.

Embodiment 25: In some or any embodiments, disclosed is a compound of Formula (Z) having the structure of Formula (V): 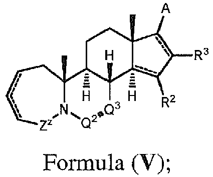 where:
Z^{Z} is CO or (CH₂);
each 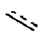 is independently a single bond or double bond;
N'''''Q²Q³ is N-Q²=Q³ ; Q² and Q³ are CH; or
N'''''Q²'''''Q³ is N-Q²-Q³ ; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
   a) R^{7b} and R^{7c} together with the carbon to which they are attached form C=CH₂, cycloalkyl or carbonyl; or
   b) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R13^{a}, -NR^{13a}C(O)R^{13a}, and -NR_{A}R_{B}; and
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms.
In another embodiment, the Compound of Formula V is that where A is heteroaryl optionally substituted with one R⁴; and R⁴ and all other groups are as defined in embodiment 25. In another embodiment, the Compound of Formula V is that where A is pyridinyl optionally substituted with one R⁴; and R⁴ and all other groups are as defined in embodiment 25. In another embodiment, the Compound of Formula V is that where A is pyridinyl optionally substituted with one alkyl, alkenyl, alkoxy, halo, or haloalkyl; and all other groups are as defined in embodiment 25. In another embodiment, the Compound of Formula V is that where A is pyridinyl optionally substituted with one methyl, ethyl, methoxy, ethoxy, n-propoxy, or propen-3-yl; and all other groups are as defined in embodiment 25.

Embodiment 25a: In another embodiment, the compound of Formula II is that where Z^{Z} is C(O) ; and all other groups are as defined in embodiment 25. In another embodiment, the compound of Formula II is that where Z^{Z} is CH₂ ; and all other groups are as defined in embodiment 25.

Embodiment 25b: In another embodiment, the compound of Formula II is that where R² and R³ are hydrogen; and all other groups are as defined in any of embodiments 25 and 25a.

Embodiment 25c: In another embodiment, the compound of Formula II is according to VE 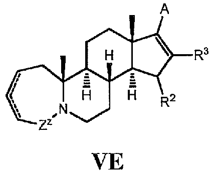 where A, R², R³, and Z^{Z} are as defined in any of embodiments 25, 25a, and 25b.

Embodiment 26: In some or any embodiments, the compound of Formula II is according to Formula (VA): 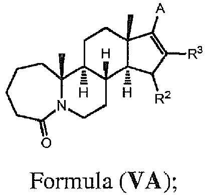 where A, R², R³, and Z^{Z} are as defined in any of embodiments 25, 25a, and 25b. In another embodiment, the compound of Formula VA is a CYP17 inhibitor.

Embodiment 27: In some or any embodiments, the compound of Formula II is according to Formula (VB): 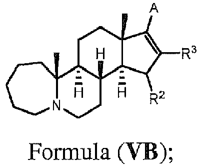 where A, R², R³, and Z^{Z} are as defined in any of embodiments 25, 25a, and 25b. In another embodiment, the compound of Formula VB is a CYP17 inhibitor.

Embodiment 28: In some or any embodiments, the compound of Formula II is according to Formula (VC): 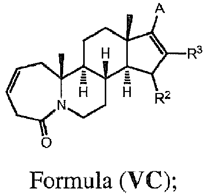 where A, R², R³, and Z^{Z} are as defined in any of embodiments 25, 25a, and 25b.

Embodiment 29: In some or any embodiments, the compound of Formula II is according to Formula (VD): 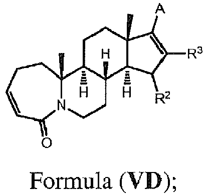 where A, R², R³, and Z^{Z} are as defined in any of embodiments 25, 25a, and 25b.

Embodiment 30: In some or any embodiments, disclosed is a compound of Formula (Z) having the structure of Formula (VI): 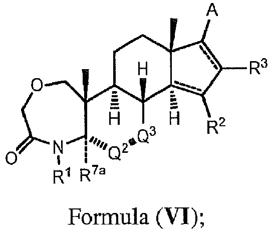 where:
each 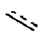 is independently a single bond or a double bond;
C(R^{7a})'''''Q²'''''Q³ is C(R^{7a})-Q²=Q³ ; where R^{7a} absent or is hydrogen or hydroxy; and Q² and Q³ are CH; or
C(R^{7a})'''''Q²'''''Q³ is C(R^{7a})=Q²-Q³ ; R^{7a} is absent; Q² is CH; and Q³ is CHR⁸ or C(O) where R⁸ is hydrogen; or
C(R^{7a})'''''Q²'''''Q³ is C(R^{7a})-Q²-Q³ ; R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
   a) R^{7a}, when present, and R^{7b} together with the carbons to which they are attached form cycloalkyl or heterocycloalkyl;
   b) R^{7b} and R^{7c} together with the carbon to which they are attached form C=CH₂, cycloalkyl, or carbonyl; or
   c) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms;

Embodiment 30a: In another embodiment, the compound of Formula (VI) is that where R² and R³ are hydrogen; and all other groups are as defined in embodiment 30.

Embodiment 30b: In another embodiment, the compound of Formula (VI) is that where R¹ is alkyl; and all other groups are as defined in any of embodiments 30 amd 30a.

Embodiment 30c: In another embodiment, the compound of Formula (VI) is that where A is heteroaryl optionally substituted with one R⁴; and R⁴ and all other groups are as defined in any of embodiments 30, 30a, and 30b. In another embodiment, the compound of Formula (VI) is that where A is pyridinyl optionally substituted with one R⁴; and R⁴ and all other groups are as defined in any of embodiments 30, 30a, and 30b. In another embodiment, the compound of Formula (VI) is that where A is pyridinyl optionally substituted with one alkyl, alkoxy, halo, or haloalkyl; and all other groups are as defined in any of embodiments 30, 30a, and 30b. In another embodiment, the compound of Formula (VI) is that where A is pyridinyl optionally substituted with one alkyl or alkoxy; and all other groups are as defined in any of embodiments 30, 30a, and 30b.

Embodiment 30d: In another embodiment, the compound of Formula (VI) is according to VIa 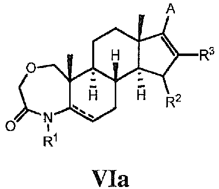 where all groups are as defined in any of embodiments 30, 30a, 30b, and 30c. In another embodiment, the compound of Formula VIa is that where 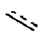 is a single bond; and all groups are as defined in any of embodiments 30, 30a, 30b, and 30c. In another embodiment, the compound of Formula VIa is that where
R¹ is alkyl, in another example methyl; and all groups are as defined in any of embodiments 30, 30a, 30b, and 30c.

Embodiment 31: In some embodiments, disclosed is a compound of Formula (Z) having the structure of Formula (VII): 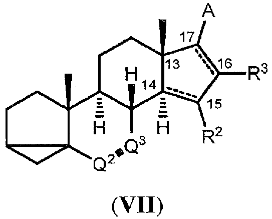 where
each 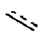 is independently a single bond or a double bond;
'''''Q²'''''Q³ is Q²=Q³ ; Q² and Q³ are CH; or
'''''Q²'''''Q³ is Q²-Q³ ; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7b} and R^{7c} together with the carbon to which they are attached form C=CH₂, cycloalkyl or carbonyl; or
b) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or
R_{A} and R_{B} taken together with the nitrogen atom form a 4 to 7 membered heterocyclic ring having one or two heteroatoms
provided that when 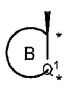 is according to formula Z-VII, Q² is CH(OH), CH(OCH₃), or CH(OC(O)alkyl), then A is not unsusbstituted pyridinyl and A is not pyrazolyl optionally substituted with one R⁴.

Embodiment 31a: In another embodiment, the compound of Formula VII is that where A is heteroaryl optionally substituted with one or two R⁴; and R⁴ and all other groups are as defined in embodiment 31. In another embodiment, the compound of Formula VII is that where A is heteroaryl optionally substituted with one alkyl, haloalkyl, halo, or alkoxy; and all other groups are as defined in embodiment 31. In another embodiment, the compound of Formula VII is that where A is imidazolyl, triazolyl, or pyridinyl, each of which is optionally substituted with one alkyl, haloalkyl, halo, or alkoxy; and all other groups are as defined in embodiment 31. In another embodiment, the compound of Formula VII is that where A is unsubstituted imidazolyl, unsubstituted triazolyl, or pyridinyl, optionally substituted with one alkyl, haloalkyl, halo, or alkoxy; and all other groups are as defined in embodiment 31. In another embodiment, the compound of Formula VII is that where A is unsubstituted imidazolyl, unsubstituted triazolyl, or unsubstituted pyridinyl; and all other groups are as defined in embodiment 31.

Embodiment 31b: In another embodiment, the compound of Formula VII is that where '''''Q²'''''Q³ is Q²-Q³; Q² is C(R^{7b})(R^{7c}); Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7b} and R^{7c} together with the carbon to which they are attached form C=CH₂, cycloalkyl or carbonyl; or
b) R⁸ and R^{7b} together with the carbons to which they are attached form cycloalkyl; and
all other groups are as defined in any of embodiments 31, 31a, and 31b. In another embodiment, the compound of Formula VII is that where '''''Q²'''''Q³ is Q²-Q³ and Q³ is CH₂; Q² is C(R^{7b})(R^{7c}) where R^{7c} is hydrogen and R^{7b} is hydroxy, alkoxy, alkylamino, or heteroarylamino or R^{7c} and R^{7b} together with the carbon to which they are attached form C(O); and all other groups are as defined in any of embodiments 31, 31a, and 31b. In another embodiment, the compound of Formula VII is that where '''''Q²'''''Q³ is Q²-Q³ and Q³ is CH₂; Q² is CH(OH), CH(OCH₃), C(NHCH₃), C(NHthiazolyl), or C(O); and all other groups are as defined in any of embodiments 31, 31a, and 31b.

Embodiment 31c: The compound of Formula (VIIa) is that where R² and R³ are hydrogen; and all other groups are as defined in any of embodiments 31, 31a, and 31b.

Embodiment 31d: In some embodiments, disclosed is a compound of Formula (VII) having the structure of Formula (VIIa): 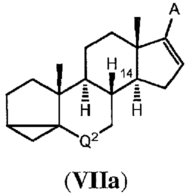 where A and Q² are as defined in any of embodiments 31, 31a, 31b, and 31c,

Embodiment 31e: In some embodiments, disclosed is a compound of Formula (VII) having the structure of Formula (VIIb): 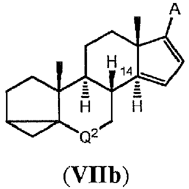 where A and Q² are as defined in any of embodiments 31, 31a, 31b, and 31c,

Embodiment 32: In some embodiments, disclosed is a compound of Formula (Z) having the structure of Formula (VIII): 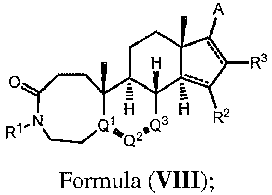 where:
each 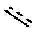 is independently a single bond or a double bond;
Q¹'''''Q²'''''Q³ is Q¹-Q²=Q³; where R^{7a} is absent or is hydrogen or hydroxy; and Q² and Q³ are CH; or
Q¹'''''Q²'''''Q³ is Q¹=Q²-Q³; R^{7a} is absent; Q² is CH; and Q³ is CHR⁸ or C(O) where R⁸ is hydrogen; or
Q¹'''''Q²'''''Q³ is Q¹-Q²-Q³; R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
   a) R^{7a}, when present, and R^{7b} together with the carbons to which they are attached form cycloalkyl or heterocycloalkyl;
   b) R^{7b} and R^{7c} together with the carbon to which they are attached form C=CH₂, cycloalkyl, or carbonyl; or
   c) R⁸ and R^{7b} together with the carbons to which they are attached form cycloalkyl;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B};
R² is selected from the group consisting of hydrogen, halogen, alkyl, cycloalkyl, cyano, nitro, oxo, alkoxy, alkoxyalkyl, haloalkoxy, haloalkoxyalkyl, hydroxyl, hydroxyalkyl and alkylcarbonyloxy;
R³ is selected from the group consisting of hydrogen, halogen, alkyl, cycloalkyl, alkynyl, cyano, haloalkoxy, haloalkyl, hydroxyl, hydroxyalkyl, nitro, -C(O)R_{A}, -NR_{A}R_{B}, and -C(O)NR_{A}R_{B}; and
each R⁴, when present, is independently selected from the group consisting of halogen, cyano, hydroxy, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B}; and
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms.

Embodiment 32a: In another embodiment, the compound of Formula VIII is that where R² and R³ are hydrogen; and all other groups are as defined in embodiment 32.

Embodiment 32b: In another embodiment, the compound of Formula VIII is that where R¹ is hydrogen and all other groups are as defined in any of embodiments 32 and 32a.

Embodiment 32c: In another embodiment, the compound of Formula VIII is that where Q¹'''''Q²'''''Q³ is CHCH₂CH₂; and all other groups are as defined in any of embodiments 32, 32a, and 32b.

Embodiment 32d: In some embodiments, disclosed is a compound of Formula (VIII) is according to Formula (VIIIa): 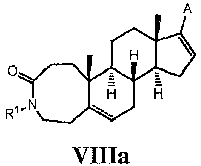 where all groups are as defined in any of embodiments 32, 32a, 32b, and 32c.

Embodiment 33: In another aspect, disclosed is a compound of Formula (IX): 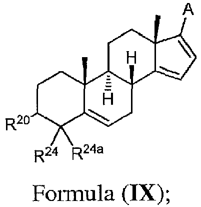 where
A is a heteroaryl optionally substituted with 1, 2, 3, or 4 R⁴; each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²⁰ is arylcarbonyloxy, heterocycloalkylcarbonyloxy, heteroarylcarbonyloxy, or -OC(O)NR²¹R^{21a}; where the aryl, heterocycloalkyl, and heteroaryl are independently optionally substituted with one or two alkyl;
R²¹ is hydrogen or alkyl;
R^{21a} is hydrogen, alkyl, or heteroaryl;
R²⁴ and R^{24a} are independently hydrogen or alkyl; and
provided that when R²⁰ is phenylcarbonyloxy, then A is not unsubstituted pyridinyl or pyridinyl substituted with one alkyl.
In another embodiment, the Compound of Formula IX is that where A is substituted with one R⁴ and the R⁴ is alkyl, halo, haloalkyl, or alkoxy; and all other groups are as defined in any of the embodiments of embodiment 33. In another embodiment, A is imidazolyl optionally substituted with one R⁴; and all other groups are as defined in any of the embodiments of embodiment 33. In another embodiment, A is pyridinyl substituted with one halo, haloalkyl, or alkoxy; and all other groups are as defined in any of the embodiments of embodiment 33. In another embodiment, A is pyridinyl substituted with one methoxy, fluoro, or difluoromethyl; and all other groups are as defined in any of the embodiments of embodiment 33. In another embodiment, A is pyridinyl substituted with one halo; and all other groups are as defined in any of the embodiments of embodiment 33. In another embodiment, R²⁰ is phenylcarbonyloxy, 2-methyl-phenylcarbonyloxy, 2-*tert*-butyl-phenylcarbonyloxy, 2,6-dimethyl-phenylcarbonyloxy, N-methyl-pyridinylcarbonyloxy, 6-methylpyridinylcarbonyloxy, or pyridinylaminocarbonyloxy; and all other groups are as defined in any of the embodiments of embodiment 33.

Embodiment 33a: In another embodiment, the Compound of Formula IX is according to IX1 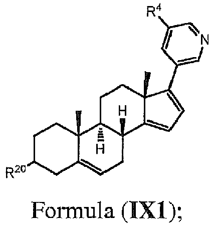 where R⁴ is halo, haloalkyl, or alkoxy; R²⁰ is arylcarbonyloxy, heterocycloalkylcarbonyloxy, heteroarylcarbonyloxy, or -OC(O)NR²¹R^{21a}; where the aryl, heterocycloalkyl, and heteroaryl are independently optionally substituted with one or two alkyl; R²¹ is hydrogen or alkyl; and R^{21a} is hydrogen, alkyl, or heteroaryl. In another embodiment, the Compound of Formula IX1 is that where R⁴ is halo.

Embodiment 33b: In another embodiment, the Compound of Formula IX is that where R²⁴ and R^{24a} are hydrogen; and all other groups are as defined in embodiment 33.

Embodiment 33c: In another embodiment, the Compound of Formula IX is that where R²⁴ and R^{24a} are alkyl; and all other groups are as define in embodiment 33.

Embodiment 34: In another aspect, disclosed is a compound of Formula (X): 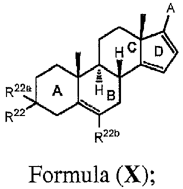 where
one 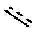 is a single bond and the other is a double bond or both are single bonds; A is a heteroaryl optionally substituted with 1, 2, 3, or 4 R⁴; each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²² is halo or hydroxy; and R^{22a} is halo, alkyl, hydroxyalkyl, alkynyl, or cycloalkyl; or R²² and R^{22a} together with the carbon to which they are attached form heterocycloalkyl; and
R^{22b} is hydrogen or alkyl.
In another embodiment, the Compound of Formula X is that where A is a monocyclic heteroaryl optionally substituted with 1, 2, 3, or 4 R⁴; and all other groups are as defined in any of the embodiments of embodiment 34. In another embodiment, the Compound of Formula X is that where A is substituted with one R⁴ and the R⁴ is alkyl, halo, haloalkyl, or alkoxy; and all other groups are as defined in any of the embodiments of embodiment 34. In another embodiment, A is imidazolyl optionally substituted with one R⁴; and all other groups are as defined in any of the embodiments of embodiment 34. In another embodiment, A is imidazol-1-yl optionally substituted with one R⁴; and all other groups are as defined in any of the embodiments of embodiment 34. In another embodiment, A is pyridinyl substituted with one halo, haloalkyl, or alkoxy; and all other groups are as defined in any of the embodiments of embodiment 34. In another embodiment, A is pyridinyl substituted with one halo; and all other groups are as defined in any of the embodiments of embodiment 34. In another embodiment, A is pyridinyl substituted with one chloro or fluoro; and all other groups are as defined in any of the embodiments of embodiment 34.

Embodiment 34a: In another embodiment, disclosed is a compound of Formula (X1): 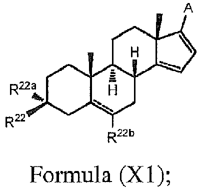 where A, R²², R^{22a}, and R^{22b} are as defined in any of the embodiments in embodiment 34.

Embodiment 34b: In another embodiment, disclosed is a compound of Formula (X2): 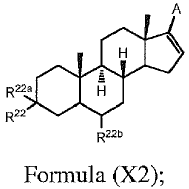 where A, R²², R^{22a}, and R^{22b} are as defined in any of the embodiments in embodiment 34. In another embodiment, the compound of Formula X2 is that where R²² and R^{22a} are halo; and all other groups are as defined in any of the embodiments of embodiment 34. In another embodiment, the compound of Formula X2 is that where R²² and R^{22a} are fluoro; and all other groups are as defined in any of the embodiments of embodiment 34. In another embodiment, the compound of Formula X2 is that where R²² is hydroxy and R^{22a} is alkyl, hydroxyalkyl, alkynyl, or cycloalkyl; and all other groups are as defined in any of the embodiments of embodiment 34. In another embodiment, the compound of Formula X2 is that where R²² is hydroxy and R^{22a} is methyl, ethyl, hydroxypropyl, ethinyl, or cyclopropyl; and all other groups are as defined in any of the embodiments of embodiment 34. In another embodiment, the compound of Formula X2 is that where R²² and R^{22a} together with the carbon to which they are attached form heterocycloalkyl; and all other groups are as defined in any of the embodiments of embodiment 34.

Embodiment 34c: In another embodiment, disclosed is a compound of Formula (X3): 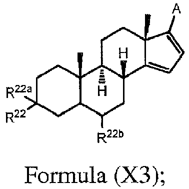 where A, R²², R^{22a}, and R^{22b} are as defined in any of the embodiments in embodiment 34. In another embodiment, the compound of Formula X3 is that where R²² and R^{22a} are halo; and all other groups are as defined in any of the embodiments of embodiment 34. In another embodiment, the compound of Formula X3 is that where R²² and R^{22a} are fluoro; and all other groups are as defined in any of the embodiments of embodiment 34. In another embodiment, the compound of Formula X3 is that where R²² is hydroxy and R^{22a} is alkyl; and all other groups are as defined in any of the embodiments of embodiment 34. In another embodiment, the compound of Formula X3 is that where R²² is hydroxy and R^{22a} is methyl or ethyl; and all other groups are as defined in any of the embodiments of embodiment 34.

Embodiment 34d: In another embodiment, disclosed is a compound of Formula (X4): 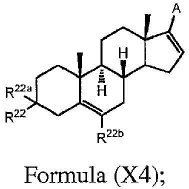 where A, R²², R^{12a}, and R^{22b} are as defined in any of the embodiments in embodiment 34. In another embodiment, the compound of Formula X4 is that where R²² and R^{22a} are halo; and all other groups are as defined in any of the embodiments of embodiment 34. In another embodiment, the compound of Formula X4 is that where R²² and R^{22a} are fluoro; and all other groups are as defined in any of the embodiments of embodiment 34. In another embodiment, the compound of Formula X4 is that where R²² is hydroxy and R^{22a} is alkyl; and all other groups are as defined in any of the embodiments of embodiment 34. In another embodiment, the compound of Formula X4 is that where R²² is hydroxy and R^{22a} is methyl or ethyl; and all other groups are as defined in any of the embodiments of embodiment 34.

Embodiment 34e: The compound of Formula X, X1, X2, X3, or X4 is an inhibitor of CYP17. The compound of Formula X, X1, X2, X3, or X4 is an inhibitor of CYP17 and has an IC₅₀ of less than or equal to about 5 nM, inanother example less than or equal to about 2 nM, in another example less than or equal to about 1 nM. The compound of Formula X, X1, X2, X3, or X4 is an inhibitor of CYP17 and demonstrates a percent inhibition of CYP17 at 1 nM of greater than or equal to about 75%, in another example greater than or equal to about 80%, in another example greater than or equal to about 85%, in another example greater than or equal to about 90%. In another embodiment, the compound of Formula X, X1, X2, X3, or X4 has a T_{1/2} (min) in human liver microsomes of greater than or equal to about 80, in another example greater than or equal to about 90, in another example greater than or equal to about 100, in another example greater than or equal to about 120, in another example greater than or equal to about 140, in another example greater than or equal to about 160, in another example greater than or equal to about 180, in another example greater than or equal to about 200.

Embodiment 35: In another aspect, disclosed is a compound of Formula XI: 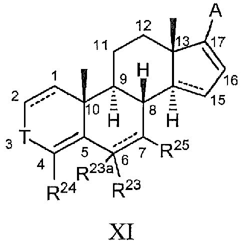 where
each 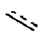 is independently a single or double bond;
T is C(O), C(=N-OH), or C(=N-O(alkyl));
A is a 5- or 6-membered heteroaryl optionally substituted with 1, 2, 3, or 4 R⁴; each R⁴ is independently halogen, cyano, hydroxy, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen, halo, alkyl, hydroxyalkyl, cycloalkyl, phenyl, or heteroaryl and R^{23a} is hydrogen or is absent when the bond between carbons 6 and 7 is a double bond; or R²³ and R^{23a} together with the carbon to which they are attached form C=O, C=CH₂ or cycloalkyl;
R²⁴ is hydrogen, hydroxy, or alkyl;
R²⁵ is hydrogen; or R²³ and R²⁵ together with the carbons to which they are attached form cycloalkyl; and
when all 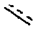 are single bonds, then one of R²³, R²⁴, and R²⁵ is not hydrogen, or R²³ and R²⁵
together with the carbons to which they are attached form cycloalkyl; and
when A is unsubstituted pyridinyl or pyridinyl substituted with one alkyl, and R²⁴ and R²⁵ are hydrogen, then
a) R²³ is not halo, and
b) R²³ and R^{23a} do not form oxo.
In another embodiment, the Compound of Formula XI is that where A is substituted with one R⁴ and the R⁴ is amino, halo, haloalkyl, or alkoxy; and all other groups are as defined in any of the embodiments of embodiment 35. In another embodiment, A is imidazolyl optionally substituted with one R⁴; and all other groups are as defined in any of the embodiments of embodiment 35. In another embodiment, A is imidazol-1-yl optionally substituted with one R⁴; and all other groups are as defined in any of the embodiments of embodiment 35. In another embodiment, A is unsubstituted pyridinyl; and all other groups are as defined in any of the embodiments of embodiment 35. In another embodiment, A is pyridinyl substituted with one amino, halo, haloalkyl, or alkoxy; and all other groups are as defined in any of the embodiments of embodiment 35. In another embodiment, A is pyridinyl substituted with one halo; and all other groups are as defined in any of the embodiments of embodiment 35. In another embodiment, A is pyridinyl substituted with one chloro or fluoro; and all other groups are as defined in any of the embodiments of embodiment 35. In another embodiment, A is pyridinyl substituted with one amino; and all other groups are as defined in any of the embodiments of embodiment 35. In another embodiment, A is pyridinyl substituted with one haloalkyl; and all other groups are as defined in any of the embodiments of embodiment 35. In another embodiment, A is pyridinyl substituted with one difluormethyl or trifluoromethyl; and all other groups are as defined in any of the embodiments of embodiment 35. In another embodiment, A is pyridinyl substituted with one alkoxy; and all other groups are as defined in any of the embodiments of embodiment 35. In another embodiment, A is pyridinyl substituted with one methoxy; and all other groups are as defined in any of the embodiments of embodiment 35. In another embodiment, A is pyridinyl substituted with one alkyl; and all other groups are as defined in any of the embodiments of embodiment 35. In another embodiment, A is pyridinyl substituted with one methyl; and all other groups are as defined in any of the embodiments of embodiment 35. In another embodiment, A is pyridinyl substituted with one or two groups independently selected from amino, alkyl, halo, haloalkyl, and alkoxy; and all other groups are as defined in any of the embodiments of embodiment 35. In another embodiment, A is pyridinyl substituted with two groups independently selected from amino, alkyl, halo, haloalkyl, and alkoxy; and all other groups are as defined in any of the embodiments of embodiment 35. In another embodiment, A is pyridinyl substituted with one alkyl and one halo; and all other groups are as defined in any of the embodiments of embodiment 35.

Embodiment 35a: In another embodiment of embodiment 35, the compound of Formula XI is according to Formula XIa 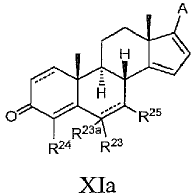 where all groups are as defined in embodiment 35. In another embodiment, the compound of Formula XIa is that where A is imidazolyl, pyridinyl, pyrimidinyl, or pyrazinyl each of which is optionally substituted with one or two R⁴ groups; and R⁴ and all other groups are as defined in embodiment 35. In another embodiment, the compound of Formula XIa is that where R²³ is hydrogen, alkyl, hydroxyalkyl, cycloalkyl, or phenyl or R²³ and R^{23a} together with the carbons to which they are attached form cycloalkyl or C=CH₂; R²⁴ is hydrogen or alkyl; R²⁵ is hydrogen; and all other groups are as defined in embodiment 35. In another embodiment, R²³, R²⁴, and R²⁵ are hydrogen; and all other groups are as defined in embodiment 35. In another embodiment, R²³ is hydrogen, R²⁴ is alkyl, and R²⁵ is hydrogen; and all other groups are as defined in embodiment 35. In another embodiment, R²⁴ is hydrogen, and R²⁵ is hydrogen, R²³ is alkyl, hydroxyalkyl, cycloalkyl, or phenyl; and all other groups are as defined in embodiment 35. In another embodiment, R²³ and R^{23a} together with the carbons to which they are attached form cycloalkyl or C=CH₂; and all other groups are as defined in embodiment 35. In another embodiment, R²³, R²⁴, and R²⁵ are hydrogen; and all other groups are as defined in embodiment 35. In another embodiment, R²⁴ and R²⁵ are hydrogen and R²³ is alkyl; and all other groups are as defined in embodiment 35. In another embodiment, R²⁴, and R²⁵ are hydrogen and R²³ and R^{23a} together with the carbon to which they are attached form C=CH₂; and all other groups are as defined in embodiment 35. In another embodiment, R²⁴ is hydroxy, R²³ and R²⁵ are hydrogen; and all other groups are as defined in embodiment 35.

Embodiment 35a1: In another embodiment of embodiment 35, the compound of Formula XI is according to Formula XIb 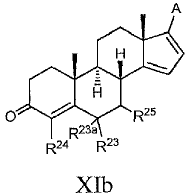 where all groups are as defined in embodiment 35. In another embodiment, the Compound of Formula XIb is that where R²³ is hydrogen, alkyl, hydroxyalkyl, cycloalkyl, or phenyl or R²³ and R^{23a} together with the carbons to which they are attached form cycloalkyl or C=CH₂; R²⁴ is hydrogen or alkyl; and R²⁵ is hydrogen; and all other groups are as defined in embodiment 35. In another embodiment, the Compound of Formula XIb is that where R²³, R²⁴, and R²⁵ are hydrogen; and all other groups are as defined in embodiment 35. In another embodiment, the Compound of Formula XIb is that where R²³ is hydrogen, R²⁴ is alkyl, and R²⁵ is hydrogen; and all other groups are as defined in embodiment 35. In another embodiment, the Compound of Formula XIb is that where R²⁴ is hydrogen, and R²⁵ is hydrogen, R²³ is alkyl, hydroxyalkyl, cycloalkyl, or phenyl; and all other groups are as defined in embodiment 35. In another embodiment, the Compound of Formula XIb is that where R²³ and R^{23a} together with the carbons to which they are attached form cycloalkyl or C=CH₂; and all other groups are as defined in embodiment 35.

Embodiment 35b: In another embodiment of embodiment 35, the compound of Formula XI is according to Formula XIc 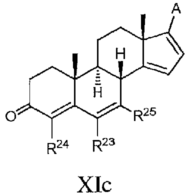 where all groups are as defined in embodiment 35. In another embodiment, the Compound of Formula XIc is that where R²³ is hydrogen; R²⁴ is hydrogen or hydroxy; and R²⁵ is hydrogen; and all other groups are as defined in embodiment 35. In another embodiment, the Compound of Formula XIc is that where R²³, R²⁴, and R²⁵ are hydrogen; and all other groups are as defined in embodiment 35. In another embodiment, the Compound of Formula XIc is that where R²⁴ is hydroxy, R²³ and R²⁵ are hydrogen; and all other groups are as defined in embodiment 35.

Embodiment 35c: In another embodiment of embodiment 35, the compound of Formula XI is according to Formula XId 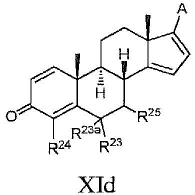 where all groups are as defined in embodiment 35. In another embodiment, the Compound of Formula XId is that where R²³ is hydrogen or alkyl or R²³ and R^{23a} together with the carbon to which they are attached form C=CH₂ or cycloalkyl; R²⁴ is hydrogen; and R²⁵ is hydrogen; and all other groups are as defined in embodiment 35. In another embodiment, R²³, R²⁴, and R²⁵ are hydrogen; and all other groups are as defined in embodiment 35. In another embodiment, R²⁴ and R²⁵ are hydrogen and R²³ is alkyl; and all other groups are as defined in embodiment 35. In another embodiment, R²⁴ and R²⁵ are hydrogen and R²³ and R^{23a} together with the carbon to which they are attached form C=CH₂ or cycloalkyl; and all other groups are as defined in embodiment 35. In another embodiment, R²⁴ and R²⁵ are hydrogen and R²³ and R^{23a} together with the carbon to which they are attached form C=CH₂ or cyclopropyl; and all other groups are as defined in embodiment 35.

Embodiment 35d: In another embodiment of embodiment 35, the compound of Formula XI is according to Formula XIe 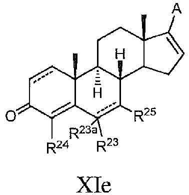 where all groups are as defined in embodiment 35. In another embodiment, the Compound of Formula XIe is that where R²³ is hydrogen, alkyl, halo, cycloalkyl, heteroaryl, or phenyl or R²³ and R^{23a} together with the carbon to which they are attached form C(O), C=CH₂ or cycloalkyl; R²⁴ is hydrogen, hydroxy, or alkyl; and R²⁵ is hydrogen; and all other groups are as defined in embodiment 35. In another embodiment, R²⁵ and R²³ together with the carbons to which they are attached form cycloalkyl and R²⁴ is hydrogen, hydroxy, or alkyl; and all other groups are as defined in embodiment 35. In another embodiment, R²³, R²⁴, and R²⁵ are hydrogen; and all other groups are as defined in embodiment 35. In another embodiment, R²⁴ and R²⁵ are hydrogen and R²³ is alkyl; and all other groups are as defined in embodiment 35. In another embodiment, R²⁴ and R²⁵ are hydrogen and R²³ is cycloalkyl; and all other groups are as defined in embodiment 35. In another embodiment, R²⁴ and R²⁵ are hydrogen and R²³ is heteroaryl; and all other groups are as defined in embodiment 35. In another embodiment, R²⁴ and R²⁵ are hydrogen and R²³ is phenyl; and all other groups are as defined in embodiment 35. In another embodiment, R²⁴ and R²⁵ are hydrogen and R²³ and R^{23a} together with the carbon to which they are attached form C(O), C=CH₂ or cycloalkyl; and all other groups are as defmed in embodiment 35. In another embodiment, R²⁴ and R²⁵ are hydrogen and R²³ is halo; and all other groups are as defined in embodiment 35. In another embodiment, R²³ and R²⁵ are hydrogen and R²⁴ is alkyl; and all other groups are as defined in embodiment 35. In another embodiment, R²⁴ is hydrogen and R²⁵ and R²³ together with the carbons to which they are attached form cycloalkyl; and all other groups are as defined in embodiment 35. In another embodiment, R²⁴ is hydroxy, and R²⁵ and R²³ are hydrogen; and all other groups are as defined in embodiment 35. In another embodiment of embodiment 35d, A is not pyrimidinyl or pyrazinyl when T is N=OH and the bond between carbons 14 and 15 is a single bond; and all other groups are defined in embodiment 35. In another embodiment of embodiment 35d, when A is unsubstituted imidazolyl or unsubstituted pyrazolyl then
a) one of R²³, R²⁴, and R²⁵ is not hydrogen, or
b) R²³ and R²⁵ together with the carbons to which they are attached form cycloalkyl. and all other groups are defined in embodiment 35.

Embodiment 35e: In another embodiment of embodiment 35, the compound of Formula XI is according to Formula XIf 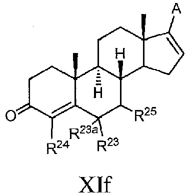 where all groups are as defined in embodiment 35. In another embodiment, the Compound of Formula XIf is that where R²³ is hydrogen, halo, alkyl, cycloalkyl, heteroaryl, or phenyl or R²³ and R^{23a} together with the carbon to which they are attached form C(O), C=CH₂ or cycloalkyl; R²⁴ is hydrogen; and R²⁵ is hydrogen; or R²⁴ is hydrogen and R²⁵ and R²³ together with the carbons to which they are attached form cycloalkyl; and all other groups are as defined in embodiment 35. In another embodiment, R²³, R²⁴, and R²⁵ are hydrogen; and all other groups are as defined in embodiment 35. In another embodiment, R²⁴ and R²⁵ are hydrogen and R²³ is alkyl; and all other groups are as defined in embodiment 35. In another embodiment, R²⁴ and R²⁵ are hydrogen and R²³ is cycloalkyl; and all other groups are as defined in embodiment 35. In another embodiment, R²⁴ and R²⁵ are hydrogen and R²³ is heteroaryl; and all other groups are as defined in embodiment 35. In another embodiment, R²⁴ and R²⁵ are hydrogen and R²³ is phenyl; and all other groups are as defined in embodiment 35. In another embodiment, R²⁴ and R²⁵ are hydrogen and R²³ and R^{23a} together with the carbon to which they are attached form C(O), C=CH₂ or cycloalkyl; and all other groups are as defined in embodiment 35. In another embodiment, R²⁴ and R²⁵ are hydrogen and R²³ is halo; and all other groups are as defined in embodiment 35. In another embodiment, R²³ and R²⁵ are hydrogen and R²⁴ is alkyl; and all other groups are as defmed in embodiment 35. In another embodiment, R²⁴ is hydrogen and R²⁵ and R²³ together with the carbons to which they are attached form cycloalkyl; and all other groups are as defined in embodiment 35.

Embodiment 35f: In another embodiment of embodiment 35, the compound of Formula XI is according to Formula XIg 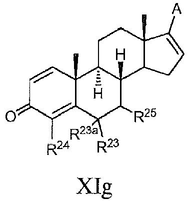 where all groups are as defined in embodiment 35. In another embodiment, the Compound of Formula XIg is that where R²³ is alkyl or cycloalkyl or R²³ and R^{23a} together with the carbon to which they are attached form C=CH₂ or cycloalkyl; and R²⁴ and R²⁵ are hydrogen; and all other groups are as defined in embodiment 35. In another embodiment, R²⁴ and R²⁵ are hydrogen and R²³ is cycloalkyl; and all other groups are as defined in embodiment 35. In another embodiment, R²⁴ and R²⁵ are hydrogen and R²³ is alkyl; and all other groups are as defined in embodiment 35. In another embodiment, R²⁴ and R²⁵ are hydrogen and R²³ and R^{23a} together with the carbon to which they are attached form C=CH₂ or cycloalkyl; and all other groups are as defined in embodiment 35. In another embodiment of embodiment 35f, when A is unsubstituted imidazolyl or unsubstituted pyrazolyl then
a) one of R²³, R²⁴, and R²⁵ is not hydrogen, or
b) R²³ and R²⁵ together with the carbons to which they are attached form cycloalkyl; and all other groups are defined in embodiment 35.

Embodiment 35g: In another embodiment of embodiment 35, the compound of Formula XI is according to Formula XIh 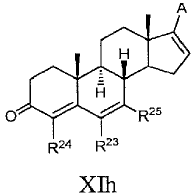 where all groups are as defined in embodiment 35. In another embodiment, the Compound of Formula XIh is that where R²³ is hydrogen; R²⁴ is hydrogen or hydroxy; and R²⁵ is hydrogen; and all other groups are as defined in embodiment 35. In another embodiment, R²³, R²⁴, and R²⁵ are hydrogen; and all other groups are as defined in embodiment 35. In another embodiment, R²⁴ is hydroxy, and R²⁵ and R²³ are hydrogen; and all other groups are as defined in embodiment 35. In another embodiment of embodiment 35g, when A is unsubstituted imidazolyl or unsubstituted pyrazolyl then
a) one of R²³, R²⁴, and R²⁵ is not hydrogen, or
b) R²³ and R²⁵ together with the carbons to which they are attached form cycloalkyl; and all other groups are as defmed in embodiment 35.

Embodiment 35h: In another embodiment of embodiment 35, the compound of Formula XI is according to Formula XI, XIa, XIb, XIc, XId, XIe, XIf, XIg, or XIh and is an inhibitor of CYP17 and has an IC₅₀ of less than or equal to about 5 nM, in another example less than or equal to about 2 nM, in another example less than or equal to about 1 nM. In another embodiment, the compound of Formula XI, XIa, XIb, XIc, XId, XIe, XIf, XIg, or XIh has a T_{1/2} (min) in human liver microsomes of greater than or equal to about 80, in another example greater than or equal to about 90, in another example greater than or equal to about 100, in another example greater than or equal to about 120, in another example greater than or equal to about 140, in another example greater than or equal to about 200.

In another embodiment, the Compond of Formula XI is a CYP21 and/or CYP17 inhibitor.

Embodiment 36: Another aspect of the invention is a compound of Formula XII 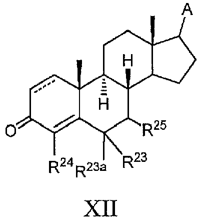 where 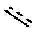 is a single bond or a double bond;
A is a heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl;
R²⁴ is hydrogen, hydroxy, or alkyl; and
R²⁵ is hydrogen.

Embodiment 36a: In another embodiment, the Compound of Formula XII is that where A is not unsubstituted pyrimidinyl or unsubstituted benzimidazolyl; and all other groups are as defined in embodiment 36.

Embodiment 36b: In another embodiment, the Compound of Formula XII is that where A is optionally substituted with one or two R⁴ groups; and R⁴ and all other groups are as defined in any of embodiments 36 and 36a. In another embodiment, the Compound of Formula XII is that where A is imidazolyl, triazolyl, pyridinyl, pyrimidinyl, pyrazinyl, or pyridazinyl, each of which is optionally substituted with one or two R⁴ groups; and R⁴ and all other groups are as defined in any of embodiments 36 and 36a. In another embodiment, the Compound of Formula XII is that where A is imidazolyl, triazolyl, or pyridinyl, each of which is optionally substituted with one or two groups independently selected from alkyl, halo, haloalkyl, and alkoxy; and all other groups are as defined in embodiment 36. In another embodiment, the Compound of Formula XII is that where A is unsubstituted imidazolyl, unsubstituted triazolyl, or pyridinyl optionally substituted with one or two groups independently selected from alkyl, halo, haloalkyl, and alkoxy; and all other groups are as defined in embodiment 36. In another embodiment, the Compound of Formula XII is that where A is pyridinyl optionally substituted with alkyl, halo, haloalkyl, or alkoxy; and all other groups are as defined in embodiment 36. In another embodiment, the Compound of Formula XII is that where A is pyridinyl optionally substituted with methyl, ethyl, chloro, fluoro, difluoromethyl, trifluoromethyl, methoxy, or ethoxy; and all other groups are as defined in embodiment 36.

Embodiment 36c: In another embodiment, the Compound of Formula XII is that where R²⁴ is hydrogen; and all other groups are as defined in any of embodiments 36, 36a, and 36b.

Embodiment 36d: In another embodiment, the Compound of Formula XII is that where R²⁵ is hydrogen; and all other groups are as defined in any of embodiments 36, 36a, 36b, and 36c.

Embodiment 36e: In another embodiment, the Compound of Formula XII is that where 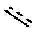 is a single bond; and all other groups are as defined in any of embodiments 36, 36a, 36b, 36c, and 36d. In another embodiment, the Compound of Formula XII is that where 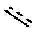 is a double bond; and all other groups are as defined in any of embodiments 36, 36a, 36b, 36c, and 36d.

Embodiment 36f: In another embodiment, the Compound of Formula XII is that where R²³ is alkyl or heteroaryl; and all other groups are as defined in any of embodiments 36, 36a, 36b, 36c, 36d, and 36e. In another embodiment, the Compound of Formula XII is that where R²³ is alkyl or heteroaryl optionally substituted with halo; and all other groups are as defined in any of embodiments 36, 36a, 36b, 36c, 36d, and 36e. In another embodiment, the Compound of Formula XII is that where R²³ is methyl, ethyl, pyridinyl, or imidazolyl where the pyridinyl and imidazolyl are each optionally substitituted with halo; and all other groups are as defined in any of embodiments 36, 36a, 36b, 36c, 36d, and 36e. In another embodiment, the Compound of Formula XII is that where R²³ is methyl, unsubstituted imidazolyl, or pyridinyl optionally substitituted with fluoro; and all other groups are as defined in any of embodiments 36, 36a, 36b, 36c, 36d, and 36e.

In another embodiment, the Compound of Formula XII is a CYP21 inhibitor.

Embodiment 37: Another aspect of the invention is a compound of Formula XIII 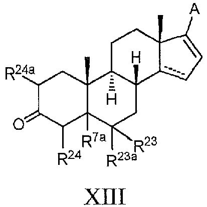 where
A is a heteroaryl optionally substituted with 1, 2, 3, or 4 R⁴;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}*,* or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms; 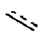 is a single bond and at least one of R²³ and R^{7a} is not hydrogen; or 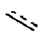 is a double bond; 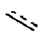 R^{7a} is hydrogen or hydroxy;
R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl; and
R²⁴ and R^{24a} are independently hydrogen, hydroxy, or alkyl.

Embodiment 37a: In another embodiment, the compound of Formula XIII is that where A is heteroaryl optionally substituted with one or two R⁴; and R⁴ and all other groups are as defined in embodiment 37. In another embodiment, the compound of Formula XIII is that where A is pyridinyl optionally substituted with one or two R⁴; and R⁴ and all other groups are as defined in embodiment 37. In another embodiment, the compound of Formula XIII is that where A is pyridinyl optionally substituted with alkyl, haloalkyl, halo, or alkoxy; and all other groups are as defined in embodiment 37. In another embodiment, the compound of Formula XIII is that where A is pyridinyl optionally substituted with halo; and all other groups are as defined in embodiment 37.

Embodiment 37b: In another embodiment, the compound of Formula XIII is that where 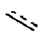 is a single bond and at least one of R²³ and R^{7a} is not hydrogen; and all other groups are as defined in any of embodiments 37 and 37a. In another embodiment, the compound of Formula XIII is that where 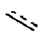 is a double bond; and all other groups are as defined in any of embodiments 37 and 37a.

Embodiment 37c: In another embodiment, the compound of Formula XIII is that where R^{7a} is hydrogen or hydroxy; R^{23a} is hydrogen and R²³ is hydrogen or halo or R²³ and R^{23a} together with the carbon to which they are attached form C(O); and all other groups are as defined in any of embodiments 37, 37a, and 37b.

Embodiment 38: Another aspect of the invention is a compound of Formula XIV 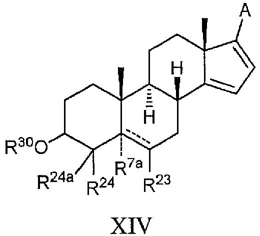 where 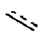 is a single bond or a double bond;
A is a heteroaryl optionally substituted with 1, 2, 3, or 4 R⁴; provided that A is not unsubstituted benzimidazolyl; A is not furyl; and A is not pyridinyl optionally substituted with alkyl when both R²⁴ and R^{24a} are hydrogen;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a} -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen or alkyl and R^{7a} is hydrogen; or R^{7a} and R²³ together with the carbons to which they are attached form oxiranyl;
R²⁴ and R^{24a} are independently hydrogen or alkyl; and
R³⁰ is hydrogen or alkylcarbonyl where the alkyl is optionally substituted with one or two groups independently selected from hydroxy, amino, alkylamino, and dialkylamino; and
provided that when R³⁰ is hydroxy, then A is not unsubstituted imidazolyl;
provided that when R³⁰ is hydroxy or alkylcarbonyloxy, then A is not unsubstituted pyridinyl or pyridinyl substituted with one alkyl.

Embodiment 38a: The compound of Formula XIV is that where A is monocyclic heteroaryl optionally substituted with one or two R⁴; and R⁴ and all other groups are as defined in embodiment 38. In another embodiment, the compound of Formula XIV is that where A is pyridinyl, imidazolyl, or triazolyl, each of which is optionally substituted with one or two R⁴; and R⁴ and all other groups are as defined in embodiment 38. In another embodiment, the compound of Formula XIV is that where A is pyridinyl, imidazolyl, or triazolyl, each of which is optionally substituted with one or two R⁴; and each R⁴ is independently alkyl, haloalkyl, halo, or alkoxy; and all other groups are as defined in embodiment 38. In another embodiment, the compound of Formula XIV is that where A is unsubstituted imidazolyl, unsubstituted triazolyl, or pyridinyl optionally substituted with one alkyl, haloalkyl, halo, or alkoxy; and all other groups are as defined in embodiment 38. In another embodiment, the compound of Formula XIV is that where A is unsubstituted imidazolyl, unsubstituted triazolyl, or pyridinyl optionally substituted with one alkyl or halo; and all other groups are as defined in embodiment 38. In another embodiment, the compound of Formula XIV is that where A is unsubstituted imidazolyl, unsubstituted triazolyl, or pyridinyl optionally substituted with halo; and all other groups are as defined in embodiment 38.

Embodiment 38b: The compound of Formula XIV is that where R²⁴ and R^{24a} are hydrogen; and all other groups are as defined in any of embodiments 38 and 38a. In another embodiment, the compound of Formula XIV is that where R²⁴ and R^{24a} are alkyl; and all other groups are as defined in any of embodiments 38 and 38a. In another embodiment, the compound of Formula XIV is that where R²⁴ is hydrogen and R^{24a} is alkyl; and all other groups are as defined in any of embodiments 38 and 38a.

Embodiment 38c: The compound of Formula XIV is that where R³⁰ is hydrogen; and all other groups are as defined in any of embodiments 38, 38a, and 38b. In another embodiment, the compound of Formula XIV is that where R³⁰ is alkylcarbonyl, in another example acetyl, ethylcarbonyl, *iso*-propylcarbonyl, or tert-butylcarbonyl; and all other groups are as defined in any of embodiments 38, 38a, and 38b. In another embodiment, the compound of Formula XIV is that where R³⁰ is alkylcarbonyl substituted with one hydroxy, amino, alkylamino, or dialkylamino; and all other groups are as defined in any of embodiments 38, 38a, and 38b. In another embodiment, the compound of Formula XIV is that where R³⁰ is dimethylaminomethylcarbonyloxy, ethylaminocarbonyloxy, *tert*-butylaminocarbonyloxy, or 1,1-di-(hydroxymethyl)-ethylcarbonyloxy; and all other groups are as defined in any of embodiments 38, 38a, and 38b.

Embodiment 38d: The compound of Formula XIV is that where R^{7a} and R²³ are hydrogen; and all other groups are as defined in any of embodiments 38, 38a, 38b, and 38c. In another embodiment, the compound of Formula XIV is that where R^{7a} and R²³ are together with the carbons to which they are attached form oxiranyl; and all other groups are as defined in any of embodiments 38, 38a, 38b, and 38c.

In another embodiment, the compound of Formula XIV is a CYP17 inhibitor.

Embodiment 38d-1: The compound of Formula XIV is according to Formula XIV-1 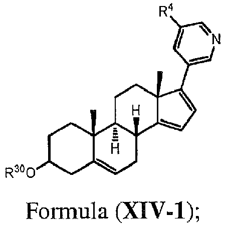 where R⁴ is halo, haloalkyl, or alkoxy; R³⁰ is hydrogen or alkylcarbonyl where the alkyl is optionally substituted with one or two groups independently selected from hydroxy, amino, alkylamino, and dialkylamino. In another embodiment, the Compound of Formula IX1 is that where R⁴ is halo. In another embodiment, the compound of Formula XIV-1 is a CYP17 inhibitor.

Embodiment 38e: The compound of Formula XIV is an inhibitor of CYP17. The compound of Formula XIV is an inhibitor of CYP17 and has an IC₅₀ of less than or equal to about 5 nM, inanother example less than or equal to about 2 nM, in another example less than or equal to about 1 nM. In another embodiment, the compound of Formula XIV has a T_{1/2} (min) in human liver microsomes of greater than or equal to about 80, in another example greater than or equal to about 90, in another example greater than or equal to about 100, in another example greater than or equal to about 120, in another example greater than or equal to about 140, in another example greater than or equal to about 200.

Embodiment 39: Another aspect of the invention is a compound of Formula XV 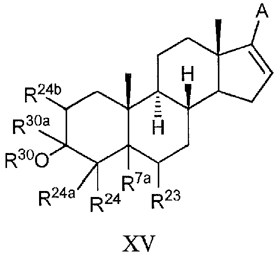 where
A is a heteroaryl optionally substituted with 1, 2, 3, or 4 R⁴; provided that
A is not unsubstituted benzimidazolyl, unsubstituted benzotriazolyl, unsubstituted triazolyl, unsubstituted imidazolyl, unsubstituted pyrimidinyl, or unsubstituted pyrazinyl;
A is not furyl; and
A is not pyridinyl optionally substituted with alkyl when R³⁰ and both R²⁴ and R^{7a} are hydrogen;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R²³ is hydrogen, halo, or alkyl; or R²³ and R^{7a} together with the carbons to which they are attached form cycloalkyl;
R^{7a} is hydrogen, alkyl, or hydroxy; and R²⁴, R^{24a}, and R^{24b} are independently hydrogen or alkyl; or R²⁴ and R^{7a} together with the carbon to which they are both attached form cycloalkyl, R^{24a} is hydrogen, and R^{24b} is hydrogen or alkyl; and
R³⁰ is hydrogen or alkyl and R^{30a} is hydrogen or R³⁰ and R^{30a} together with the carbon to which they are attached form oxiranyl or 2(3*H*)-oxo-dihydrofuranyl.

Embodiment 39a: The compound of Formula XV is that where A is monocyclic heteroaryl optionally substituted with one or two R⁴; and R⁴ and all other groups are as defined in embodiment 39. In another embodiment, the compound of Formula XV is that where A is pyridinyl or triazolyl, each of which is optionally substituted with one or two R⁴; and R⁴ and all other groups are as defined in embodiment 39. In another embodiment, the compound of Formula XV is that where A is pyridinyl or triazolyl, each of which is optionally substituted with one or two R⁴; and each R⁴ is independently alkyl, haloalkyl, halo, or alkoxy; and all other groups are as defmed in embodiment 39. In another embodiment, the compound of Formula XV is that where A is unsubstituted triazolyl or pyridinyl optionally substituted with one alkyl, haloalkyl, halo, or alkoxy; and all other groups are as defined in embodiment 39. In another embodiment, the compound of Formula XV is that where A is unsubstituted triazolyl or pyridinyl optionally substituted with one methyl, ethyl, methoxy, ethoxy, difluoromethyl, trifluoromethyl, or fluoro; and all other groups are as defined in embodiment 39. In another embodiment, the compound of Formula XV is that where A is pyridinyl substituted with one or two R⁴; and each R⁴ is independently haloalkyl, halo, or alkoxy; and all other groups are as defined in embodiment 39. In another embodiment, the compound of Formula XV is that where A is pyridinyl substituted with one haloalkyl, halo, or alkoxy; and all other groups are as defined in embodiment 39. In another embodiment, the compound of Formula XV is that where A is pyridinyl substituted with one methoxy, ethoxy, difluoromethyl, trifluoromethyl, or fluoro; and all other groups are as defined in embodiment 39.

Embodiment 39b: The compound of Formula XV is that where R²⁴ is hydrogen or alkyl and R^{24a} and R^{24b} are hydrogen; and all other groups are as defined in any of embodiments 39 and 39a. In another embodiment, the compound of Formula XV is that where R²⁴ and R^{7a} together with the carbon to which they are both attached form cyclopropyl and R^{24a} and R^{24a} are hydrogen; and all other groups are as defined in any of embodiments 39 and 39a. In another embodiment, the compound of Formula XV is that where R^{7a} is hydroxy and R²⁴, R^{24a}, and R^{24b} are hydrogen; and all other groups are as defined in any of embodiments 39 and 39a.

Embodiment 39c: The compound of Formula XV is that where R²³ is hydrogen; and all other groups are as defined in any of embodiments 39, 39a, and 39b. In another embodiment, the compound of Formula XV is that where R²³ is alkyl; and all other groups are as defined in any of embodiments 39, 39a, and 39b.

Embodiment 39c: The compound of Formula XV is that where R³⁰ is hydrogen or alkyl and R^{30a} is hydrogen; and all other groups are as defined in any of embodiments 39, 39a, 39b, and 39c. In another embodiment, the compound of Formula XV is that where R³⁰ and R^{30a} together with the carbon to which they are attached form oxiranyl or 2(3*H*)-oxo-dihydrofuranyl; and all other groups are as defined in any of embodiments 39, 39a, 39b, and 39c. In another embodiment, the compound of Formula XV is that where R³⁰ is hydrogen or methyl and R^{30a} is hydrogen; and all other groups are as defined in any of embodiments 39, 39a, 39b, and 39c.

Embodiment 40: Another aspect of the invention is a compound of Formula XVI 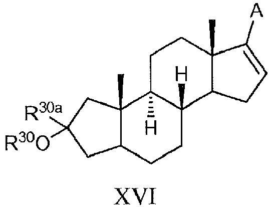 where
A is a heteroaryl optionally substituted with 1, 2, 3, or 4 R⁴; provided that A is not pyridinyl optionally substituted with alkyl;
each R⁴ is independently halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, NR¹³S(O)₂R^{13a}, -NR¹³C(O)R^{13a}, or -NR_{A}R_{B};
each R¹³ is independently hydrogen or alkyl;
each R^{13a} is independently hydrogen, alkyl, haloalkyl, alkoxyalkyl, cycloalkyl, aryl, arylalkyl, heteroaryl, or heteroarylalkyl;
R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
R³⁰ is hydrogen, alkyl, or halo; and
R^{30a} is hydroxy or halo.

Embodiment 40a: The compound of Formula XVI is that where R³⁰ is hydrogen and R^{30a} is hydroxy; and all other groups are as defined in embodiment 40. In another embodiment, the compound of Formula XVI is that where R³⁰ is alkyl and R^{30a} is hydroxy; and all other groups are as defined in embodiment 40. In another embodiment, the compound of Formula XVI is that where R³⁰ is halo and R^{30a} is halo; and all other groups are as defined in embodiment 40.

Embodiment 40b: The compound of Formula XVI is that where A is monocyclic heteroaryl optionally substituted with one or two R⁴; and R⁴ and all other groups are as defined in any of embodiments 40 and 40a. In another embodiment, the compound of Formula XVI is that where A is pyridinyl optionally substituted with one or two R⁴; and R⁴ and all other groups are as defined in any of embodiments 40 and 40a. In another embodiment, the compound of Formula XVI is that where A is pyridinyl substituted with one or two R⁴; and R⁴ and all other groups are as defined in any of embodiments 40 and 40a. In another embodiment, the compound of Formula XVI is that where A is pyridinyl substituted with one halo, haloalkyl, or alkoxy; and R⁴ and all other groups are as defined in any of embodiments 40 and 40a.

Embodiment 41: Another aspect of the invention is a compound of Formula XVII 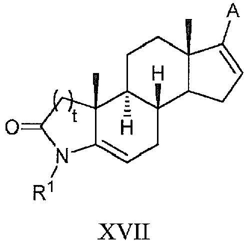 where
t is 1 or 3;
A is a heteroaryl optionally substituted with 1, 2, 3, or 4 R⁴;
each R¹ is independently selected from the group consisting of hydrogen, alkyl, cycloalkyl, alkenyl, alkynyl, alkoxyalkyl, hydroxyl, and haloalkoxyalkyl;
each R⁴, when present, is independently selected from the group consisting of halogen, cyano, hydroxyl, alkoxy, alkyl, alkenyl, haloalkyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR^{13a}C(O)R^{13a}, and -NR_{A}R_{B};
R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
each R¹³ is independently hydrogen or alkyl; and
each R^{13a} is independently hydrogen, alkyl, haloalkyl, alkoxyalkyl, cycloalkyl, aryl, arylalkyl, heteroaryl, or heteroarylalkyl.

Embodiment 41a: In another embodiment, the compound of Formula XVII is that where A is a monocyclic heteroaryl optionally substituted with one or two R⁴; and R⁴ and all other groups are as defined in embodiment 41. In another embodiment, the compound of Formula XVII is that where A is imidazolyl or pyridinyl, each of which is optionally substituted with one or two R⁴; and R⁴ and all other groups are as defined in embodiment 41. In another embodiment, the compound of Formula XVII is that where A is unsubstituted imidazolyl or pyridinyl optionally substituted with one alkyl, alkoxy, haloalkyl, or halo; and all other groups are as defined in embodiment 41. In another embodiment, the compound of Formula XVII is that where A is unsubstituted imidazolyl or pyridinyl optionally substituted with one alkyl or alkoxy; and all other groups are as defined in embodiment 41.

Embodiment 41b: In another embodiment, the compound of Formula XVII is that where R¹ is hydrogen or alkyl; and all other groups are as defined in any of embodiments 41 and 41a. In another embodiment, the compound of Formula XVII is that where R¹ is alkyl, in another example methyl; and all other groups are as defined in any of embodiments 41 and 41a.

Embodiment 41c: In another embodiment, the compound of Formula XVII is that where t is 1; and all other groups are as defined in any of embodiments 41, 41a, and 41b. In another embodiment, the compound of Formula XVII is that where t is 3; and all other groups are as defined in any of embodiments 41, 41a, and 41b.

Embodiment 41d: In another embodiment, the compound of Formula XVII is an inhibitor of CYP17 and/or CYP11B. In another embodiment, the compound of Formula XVII is an inhibitor of CYP17 and/or CYP11B and has a percent inhibition of CYP11B when tested at 100 nM of greater than or equal to about 30, in another example greater than or equal to about 40, in another example greater than or equal to about 50, in another example greater than or equal to about 60, in another example greater than or equal to about 70, in another example greater than or equal to about 80. In another embodiment, the compound of Formula XVII is an inhibitor of CYP17 and/or CYP11B and has a percent inhibition of CYP17 when tested at 1 nM of greater than or equal to about 30, in another example greater than or equal to about 40, in another example greater than or equal to about 50, in another example greater than or equal to about 60. In another embodiment, the compound of Formula XVII is an inhibitor of CYP17 and/or CYP11B and has a percent inhibition of CYP17 when tested at 10 nM of greater than or equal to about 50, in another example greater than or equal to about 60, in another example greater than or equal to about 70, in another example greater than or equal to about 80, in another example greater than or equal to about 90.

Embodiment 43: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, IV-1, XV, XVI, or XVII, where A is a heteroaryl consisting of one, two, three, or four heteroatoms selected from N, S, and O; and A is optionally substituted with 1, 2, 3, or 4 R⁴; and each R⁴, when present, and all other groups are independently as defined for a compound of Formula (Z) or as in any one of Embodiments 1-41. In some or any embodiments, disclosed is a compound of Formula (Z), I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, XI, X2, X3, X4, XI, XIa, XIb, XIc, XId, XIe, XIf, XIg, XIh, XII, XIII, XIV, XIV-1, XV, XVI, or XVII, where A is a heteroaryl and is selected from the group consisting of pyridinyl, pyrimidinyl, pyrazinyl, pyrazolyl, oxazolyl, thiazolyl, isoxazolyl, isothiazolyl, 1,3,4-oxadiazolyl, pyridazinyl, 1,3,5-triazinyl, 1,2,4-tizazinyl, quinoxalinyl, benzimidazolyl, benzotriazolyl, purinyl, 1*H*-[1,2,3]triazolo[4,5-d]pyrimidinyl, triazolyl, imidazolyl, thienyl, furanyl, isobenzofuranyl, pyrrolyl, indolizinyl, isoindolyl, indolyl, indazolyl, isoquinolinyl, quinolinyl, phthalazinyl, naphthyridinyl, quinazolinyl, cinnolinyl, and pteridinyl; and A is optionally substituted with 1, 2, 3, or 4 R⁴; and each R⁴, when present, and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is a heteroaryl and is selected from the group consisting of pyridinyl, pyrimidinyl, pyrazinyl, pyrazolyl, oxazolyl, thiazolyl, isoxazolyl, isothiazolyl, 1,3,4-oxadiazolyl, pyridazinyl, 1,3,5-triazinyl, 1,2,4-triazinyl, quinoxalinyl, benzimidazolyl, benzotriazolyl, purinyl, 1*H*-[1,2,3]triazolo[4,5-d]pyrimidinyl, triazolyl, imidazolyl, thienyl, furanyl, isobenzofuranyl, pyrrolyl, indolizinyl, isoindolyl, indolyl, indazolyl, isoquinolinyl, quinolinyl, phthalazinyl, naphthyridinyl, quinazolinyl, cinnolinyl, and pteridinyl; and A is optionally substituted with 1 or 2 R⁴; and each R⁴, when present, is independently alkoxy, alkyl, alkenyl, haloalkyl, or halogen; and all other groups are independently as defined for a compound of Formula (Z) or as in any one of Embodiments 1-41.

Embodiment 44: In some or any embodiments, disclosed is a compound of Formula (Z) having the structure of Formula (I), (II), (III), (IV), (V), (VI), (VII), (VIII), (IA), (IB), (IC), (ID), (IE), (IF), (IG), (IIA), (IIIB), (IIC), (IID), (IIIA), (IIIB), (IIIC), (IIID), (IVA), (IVB), (IVC), (IVD), (IVE), (VA), (VB), (VC) or (VD), where A is a heteroaryl and is selected from pyridinyl, imidazolyl, triazolyl, benzimidazolyl, pyrrolyl, pyrazolyl, pyrimidinyl, pyrazinyl, and pyridazinyl; and A is optionally substituted with 1, 2, 3, or 4 R⁴; and each R⁴, when present, and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is a heteroaryl and is selected from pyridinyl, imidazolyl, triazolyl, benzimidazolyl, pyrrolyl, pyrazolyl, pyrimidinyl, pyrazinyl, and pyridazinyl; and A is optionally substituted with 1, 2, 3, or 4 R⁴; and each R⁴, when present, is independently alkoxy, alkyl, alkenyl, haloalkyl, or halogen; and all other groups are independently as defined for a compound of Formula (Z) or as in any one of Embodiments 1-41.

Embodiment 45: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is pyridinyl; and A is optionally substituted with 1, 2, 3, or 4 R⁴; and each R⁴, when present, and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is pyridinyl; and A is optionally substituted with 1 or 2 R⁴; each R⁴, when present, is independently alkoxy, alkyl, alkenyl, or halogen; and all other groups are independently as defined for a compound of Formula (Z) or as in any one of Embodiments 1-41. In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is pyridinyl; and A is optionally substituted with 1 or 2 R⁴; each R⁴, when present, is independently methoxy, ethoxy, n-propxy, isopropoxy, methyl, ethyl, propen-3-yl, difluoromethyl, trifluoromethyl, fluoro, or chloro; and all other groups are independently as defined for a compound of Formula (Z) or as in any one of Embodiments 1-41.

Embodiment 46: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is benzimidazolyl; and A is optionally substituted with 1, 2, 3, or 4 R⁴; and each R⁴, when present, and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is unsubstituted benzimidazolyl; and all other groups are independently as defined for a compound of Formula (Z) or as in any one of Embodiments 1-41.

Embodiment 47: In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is imidazolyl; and A is optionally substituted with 1 or 2 R⁴; each R⁴ is alkyl; and all other groups are independently as defined for a compound of Formula (Z) or as in any one of Embodiments 1-41. In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is unsubstituted imidazolyl; and all other groups are independently as defined for a compound of Formula (Z) or as in any one of Embodiments 1-41.

Embodiment 48: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is pyrazinyl or pyrimidinyl; and A is optionally substituted with 1 or 2 R⁴; and each R⁴, when present, and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF,IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is unsubstituted pyrazinyl or unsubstituted pyrimidinyl; and all other groups are independently as defined for a compound of Formula (Z) or as in any one of Embodiments 1-41.

Embodiment 49: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF,IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is triazolyl optionally substituted with 1 or 2 R⁴ groups; and each R⁴, when present, and all other groups are independently as defined for a compound of Formula (Z) or as in any one of Embodiments 1-41. In some specific embodiments, the triazolyl is a 1,2,3-triazolyl optionally substituted with 1 or 2 R⁴ groups; and each R⁴, when present, and all other groups are independently as defined for a compound of Formula (Z) or as in any one of Embodiments 1-41. In a further embodiment, the triazolyl is a 1,2,4-triazolyl optionally substituted with 1 or 2 R⁴ groups; and each R⁴, when present, and all other groups are independently as defined for a compound of Formula (Z) or as in any one of Embodiments 1-41. In yet a further embodiment, the 1,2,3-triazolyl is substituted with one R⁴ where R⁴ is alkyl, cycloalkyl, alkenyl, alkynyl, alkoxyalkyl, hydroxy, or haloalkoxyalkyl; and all other groups are independently as defined for a compound of Formula (Z) or as in any one of Embodiments 1-41. In yet a further embodiment, the 1,2,4-triazolyl is substituted with one R⁴ where R4 is alkyl, cycloalkyl, alkenyl, alkynyl, alkoxyalkyl, hydroxyl, or haloalkoxyalkyl; and all other groups are independently as defined for a compound of Formula (Z) or as in any one of Embodiments 1-41. In some specific embodiments, A is unsubstituted triazolyl and all other groups are independently as defined for a compound of Formula (Z) or as in any one of Embodiments 1-41.

Embodiment 50: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is tetrazolyl; and A is optionally substituted with one R⁴; and R⁴ and all other groups are independently as defined for a compound of Formula (Z) or as in any one of Embodiments 1-41. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is unsubstituted tetrazolyl; and all other groups are independently as defined for a compound of Formula (Z) or as in any one of Embodiments 1-41.

Embodiment 51: In some or any embodiments, disclosed is a compound of Formula Z, I, IE, IF, IG, IH, IJ, IK, IL, II, IIC, IID, IIE, III, IIIA, IIIC, IIID, IIIE, IIIF-1, IIIF-2, IV, IVA, IVB, IVF, IVG, VI, VIa, VIII, or VIIIa, where R¹ is hydrogen, alkyl, or cycloalkyl and where the alkyl and cycloalkyl groups are optionally substituted with 1, 2, or 3 substituents independently selected from the group consisting of halogen, alkyl, alkenyl, alkoxy, alkoxycarbonyl, hydroxyl, hydroxyalkyl, alkynyl, cyano, haloalkoxy, haloalkyl, nitro, -NR_{A}R_{B}, and -C(O)NR_{A}R_{B} and all other groups are independently as defined for a compound of Formula (Z) or as in any one of Embodiments 1-41 and 43-50. In some or any embodiments, disclosed is a compound of Formula Z, I, IE, IF, IG, IH, IJ, IK, IL, II, IIC, IID, IIE, III, IIIA, IIIC, IIID, IIEE, IIIF-1, IIIF-2, IV, IVA, IVB, IVF, IVG, VI, VIa, VIII, or VIIIa, where R¹ is hydrogen, C₁-C₆ alkyl or C₃-C₆ cycloalkyl; and all other groups are independently as defined for a compound of Formula (Z) or as in any one of Embodiments 1-41 and 43-50. In some or any embodiments, disclosed is a compound of Formula Z, I, IE, IF, IG, IH, IJ, IK, IL, II, IIC, IID, IIE, III, IIIA, IIIC, IIID, IIIE, IIIF-1, IIIF-2, IV, IVA, IVB, IVF, IVG, VI, VIa, VIII, or VIIIa, where R¹, when present, is hydrogen or C₁-C₆ alkyl; and all other groups are independently as defined for a compound of Formula (Z) or as in any one of Embodiments 1-41 and 43-50. In some or any embodiments, disclosed is a compound of Formula Z, I, IE, IF, IG, IH, IJ, IK, IL, II, IIC, IID, IIE, III, IIIA, IIIC, IIID, IIIE, IIIF-1, IIIF-2, IV, IVA, IVB, IVF, IVG, VI, VIa, VIII, or VIIIa, where R¹, when present, is hydrogen or methyl; and all other groups are independently as defined for a compound of Formula (Z) or as in any one of Embodiments 1-41 and 43-50.

Embodiment 52: In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R² is selected from the group consisting of hydrogen, halogen, hydroxyl, oxo, alkyl, cycloalkyl, cyano, and nitro; and all other groups are independently as defined for a compound of Formula (Z) or as in any one of Embodiments 1-41. In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R² is hydrogen, oxo, or C₁-C₆ alkyl; and all other groups are independently as defined for a compound of Formula (Z) or as in any one of Embodiments 1-41 and 43-51. In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R² is hydrogen or oxo; and all other groups are independently as defined for a compound of Formula (Z) or as in any one of Embodiments 1-41 and 43-51. In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R² is hydrogen; and all other groups are independently as defined for a compound of Formula (Z) or as in any one of Embodiments 1-41 and 43-51.

Embodiment 53: In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R³ is selected from the group consisting of hydrogen, halogen, alkyl, cycloalkyl, cyano, hydroxyl, and nitro; and all other groups are independently as defined for a compound of Formula (Z) or as in any one of Embodiments 1-41 and 43-52. In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R³ is hydrogen or C₁-C₆ alkyl; and all other groups are independently as defined for a compound of Formula (Z) or as in any one of Embodiments 1-41 and 43-52. In some or any embodiments, disclosed is a compound of Formula Z, II, IIA, IIB, IIC, IID, IIE, III, IIIA, IIIB, IIIC, IIID, IIIE, IV, IVC, IVD, IVE, IVF, V, VA, VB, VC, VD, VE, VI, VIa, VII, or VIII, where R³ is hydrogen; and all other groups are independently as defined for a compound of Formula (Z) or as in any one of Embodiments 1-41 and 43-52.

Embodiment 54: In some or any embodiments, disclosed is a compound of Formula (Z), (II), (III), (IV), (V), (VI), (VII), and (VIII), where Q¹'''''Q²'''''Q³ is Q¹-Q²=Q³ ; where R^{7a} is absent or is hydrogen or hydroxy; and Q² and Q³ are CH; and all other groups are independently as defined for a compound of Formula (Z) or as in any one of Embodiments 9, 9a, 14,14a,14b, 14d, 14e, 14e1, 19,19a, 19b, 19d, 19e, 19f, 19g, 25, 25a, 25b, 30, 30a, 30b, 30c, 31, 31a, 31c, 32, 32a, 32b and 43-53. In some or any embodiments, disclosed is a compound of Formula (Z), (IV), or (VII), where Q¹'''''Q²'''''Q³ is C-Q²=Q³ ; and all other groups are independently as defined for a compound of Formula (Z) or as in any one of Embodiments 19,19a, 19b, 19d, 19e, 19f, 19g, 31, 31a, 31c, and 43-53. In some or any embodiments, disclosed is a compound of Formula (Z) or (V) where Q¹'''''Q²'''''Q³ is N-Q²=Q³; and all other groups are independently as defined for a compound of Formula (Z) or as in any one of Embodiments 25, 25a, and 25b. In some or any embodiment, the compound of Formula Z, I is that where Q¹'''''Q²'''''Q³ is -CH-CH=CH-; and all other groups are independently as defmed for a compound of Formula (Z) or as in any one of Embodiments 1 and 43-53.

Embodiment 55: In some or any embodiments, disclosed is a compound of Formula Z, II, III, VI, or VIII, where Q¹'''''Q²'''''Q³ is Q¹=Q²-Q³ ; where R^{7a} is absent; Q² is CH; and Q³ is CHR⁸ or C(O) where R⁸ is hydrogen; and all other groups are independently as defined for a compound of Formula (Z) or as in any one of Embodiments 1, Ij-lq, 2-8a, 9, 9a, 9c, 10-13, 30-30d, 32-32b, 32d, 33, 33a, 41-41d, and 43-53. In some or any embodiments, disclosed is a compound of Formula Z, I, IA, IB, IC, ID, IE, IF, IG, IK, IL, II, IIA, IIB, IIC, IID, IIE, III, IIIA, IIIB, IIIC, IIID, IIIE, IIIF-1, IIF-2, VI, VIa, VIII, VIIIa, IX, or XVII where Q¹'''''Q²'''''Q³ is C=CH-CH₂; and all other groups are independently as defined for a compound of Formula (Z) or as in any one of Embodiments 1, lj-lq, 2-8a, 9, 9a, 9c, 10-13, 30-30d, 32-32b, 32d, 33, 33a, 41-41d, and 43-53.

Embodiment 56: In some or any embodiments, disclosed is a compound of Formula (Z), (II), (III), (IV), (V), (VI), (VII), or (VIII), where Q¹'''''Q²'''''Q³ is Q¹-Q²-Q³ ; where R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when Q² is C(R^{7b})(R^{7c}), then
a) R^{7a}, when present, and R^{7b} together with the carbons to which they are attached form cycloalkyl or heterocycloalkyl;
b) R^{7b} and R^{7c} together with the carbon to which they are attached form C=CH₂, cycloalkyl, or carbonyl; or
c) R⁸ and R^{7b} together with the carbons to which they are attached form cycloalkyl; and
all other groups are independently as defined for a compound of Formula (Z) or as in any one of Embodiments 1-1h, 2-8a, 9, 9a, 9c, 10-13, 14-14g, 15-19b, 19d-19j, 22-23a, 24, 24a, 25-25c, 26-32d, and 43-53. In another embodiment, the compound of Formula Z, I, IA, IB, IC, ID, IE, IF, IG, IJ, II, IIA, IIB, IIC, IID, IIE, III, IIIA, IIIB, IIIC, IIID, IIIF-1, IIIF-2, IV, IVA, IVB, IVC, IVD, IVE, IVF, IVG, IVH, VI, VIa, VIII, VIIIa, or XVI is that where Q¹'''''Q²'''''Q³ is CHCH₂CH₂; and all other groups are independently as defined for a compound of Formula (Z) or as in any one of Embodiments 1-1h, 2-8a, 9, 9a, 9c, 10-13, 14-14g, 15-19b, 19d-19j, 22-23a, 30-30d, 32-32d, and 43-53.

Embodiment 57: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently alkoxy, alkyl, alkenyl, haloalkyl, or halogen; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴ is independently methoxy, ethoxy, n-propxy, isopropoxy, methyl, ethyl, propen-3-yl, difluoromethyl, trifluoromethyl, fluoro, or chloro; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently hydrogen; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently halogen; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently selected from Cl, Br, or F; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently C₁-C₆ alkoxy; and all other groups are independently as defined for a compound of Formula (Z) or as in any one of Embodiments 1-41 and 43-56. In some or any embodiments, disclosed is a compound of Formula (Z) having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently selected from methoxy, ethoxy, n-propoxy, and iso-propoxy; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently C₁-C₆ alkyl; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently selected from methyl, ethyl, n-propyl, iso-propyl, n-butyl, iso-butyl, and tert-butyl; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, ILA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently alkenyl; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently propen-1-yl, or propen-3-yl; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently haloalkyl; and all other groups are independently as defined for a compound of Formula (Z) or as in any one of Embodiments 1-41 and 43-56. In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where each R⁴, when present, is independently difluoromethyl or trifluoromethyl; and all other groups are independently as defined for a compound of Formula (Z) or as in any one of Embodiments 1-41 and 43-56.

Embodiment 61: In some or any embodiments, disclosed is a compound of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is heteroaryl optionally substituted with 1, 2, 3, or 4 R⁴ groups and is attached to the parent moiety via a heteroatom of the heteroaryl group; and each R⁴, when R⁴ is present, and all other groups are independently as defined for a compound of Formula (Z) or as defined in any one of Embodiments 1-41 and 43-56. By way of example only, A is an imidazolyl attached as depicted 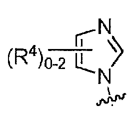 to the parent moiety and each R⁴, when present, and all other groups are independently as defined in for a compound of Formula (Z) or as defined in any one of Embodiments 1-41 and 43-56.

Embodiment 59: In some or any embodiments, disclosed is a compound of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII, where A is a heteroaryl optionally substituted with 1, 2, 3, or 4 R⁴ groups and is attached to the parent moiety via a carbon atom of the heteroaryl group; and each R⁴, when R⁴ is present, and all other groups are independently as defined for a compound of Formula (Z) or as defined in any one of Embodiments 1-41 and 43-56. Also by way of example only, A is pyridine-3-yl optionally substituted with 1, 2, or 3 R⁴ groups and each R⁴, when present, and all other groups are independently as defined in for a compound of Formula (Z) or as defined in any one of Embodiments 1-41 and 43-56.

Embodiment 60: Also described herein is a compound selected from Table 2 and Table 3; or a single stereoisomer or tautomer or mixture thereof, optionally as a pharmaceutically acceptable salt or solvate thereof.

Embodiment 60a: Also described herein is a compound selected from

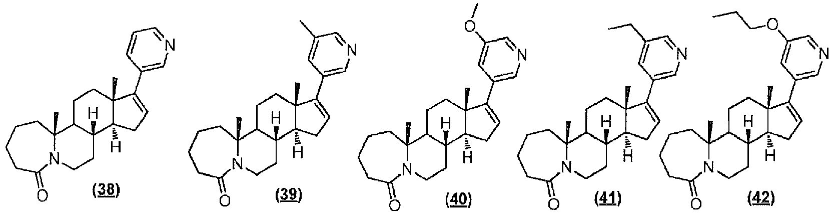

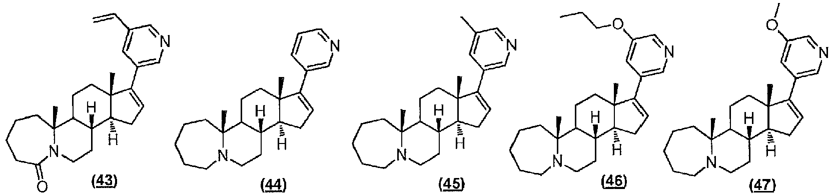

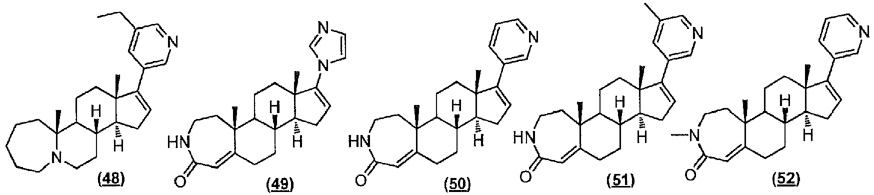

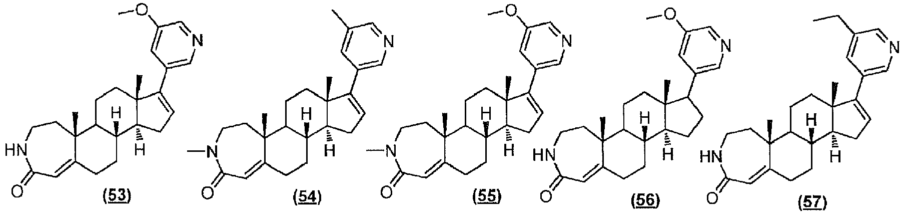

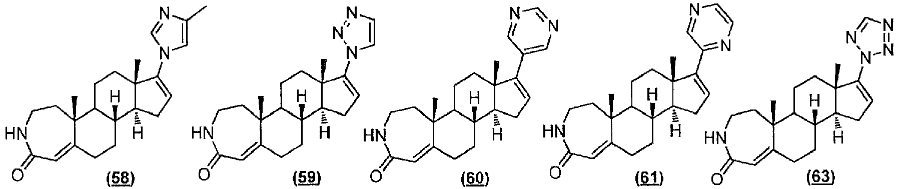

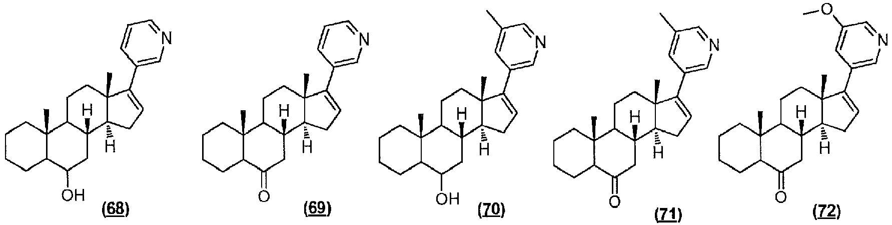

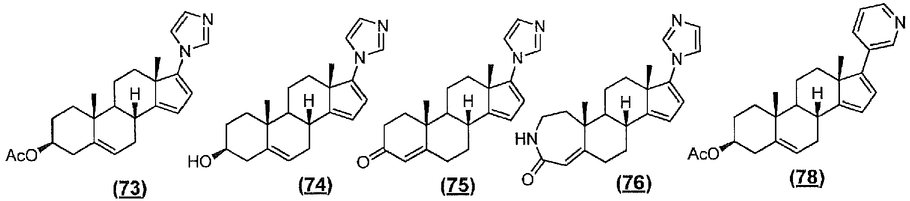

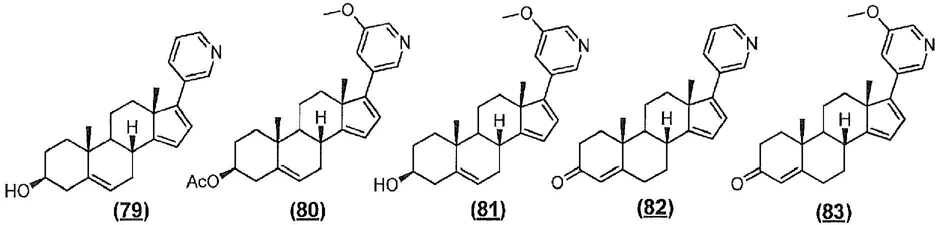

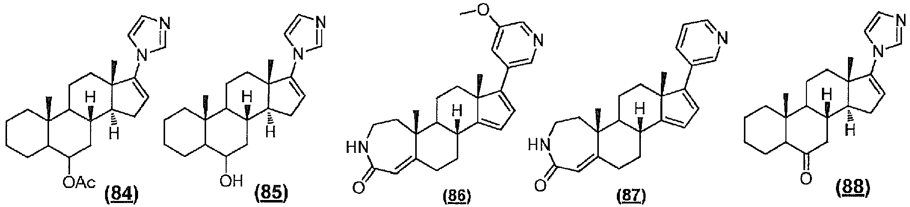

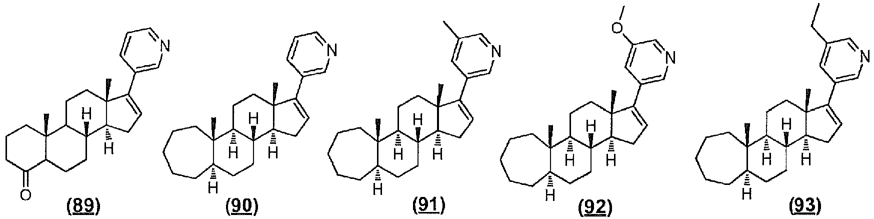

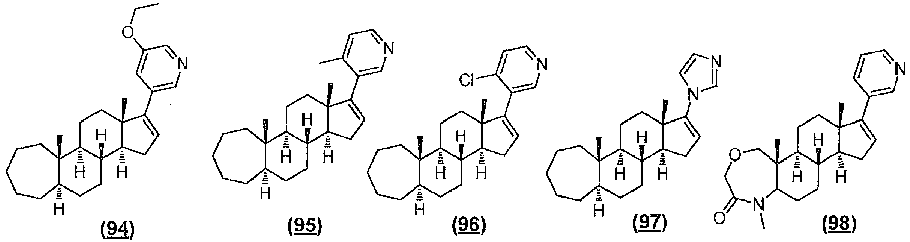

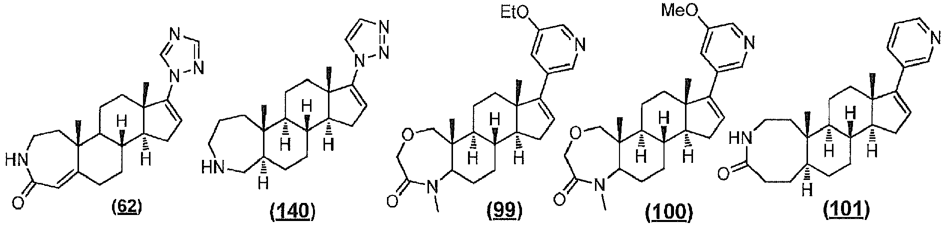

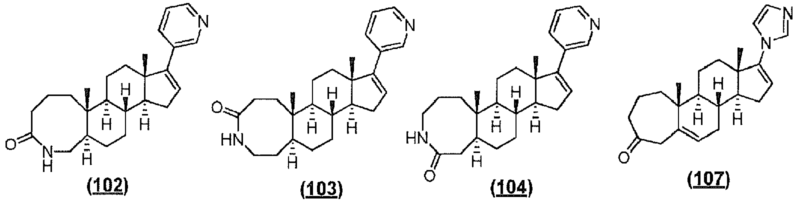

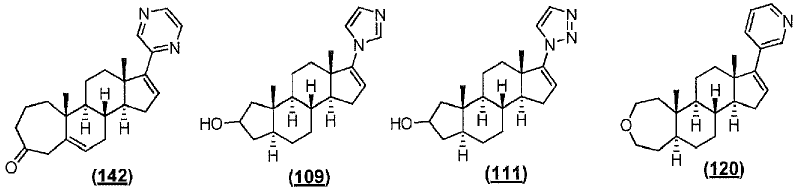

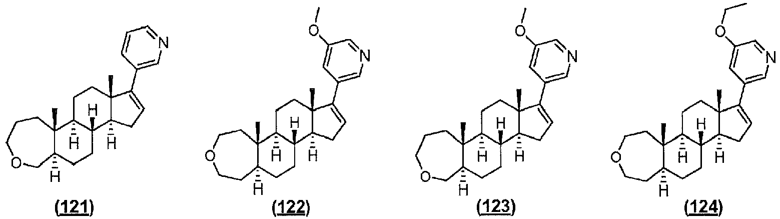

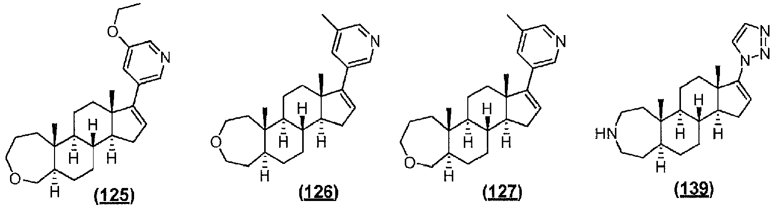

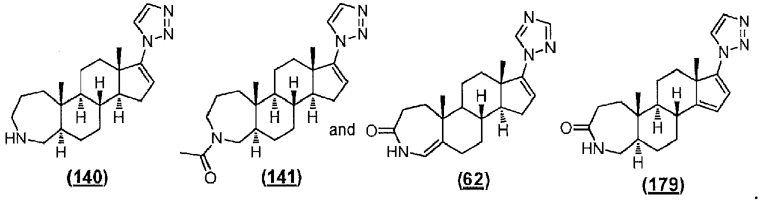

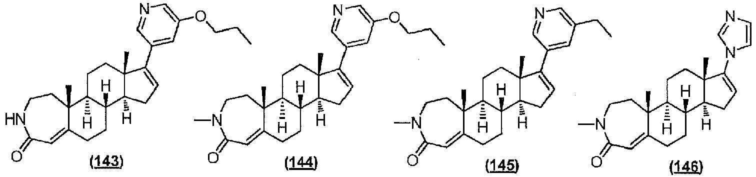

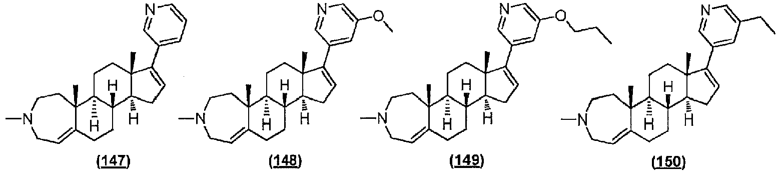

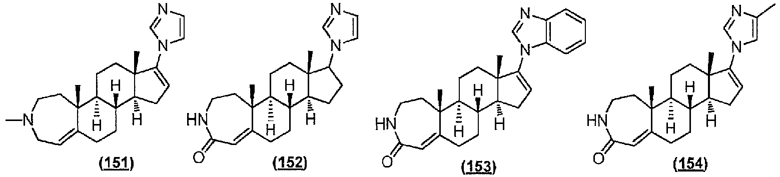

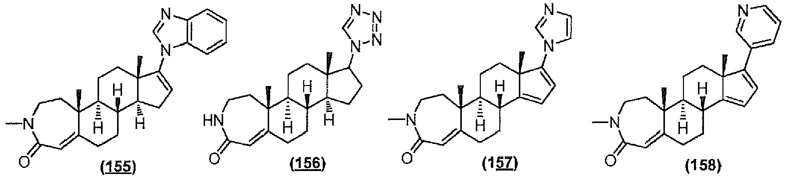

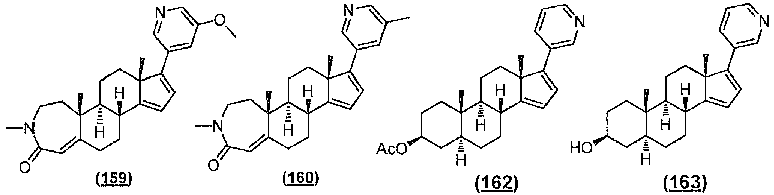

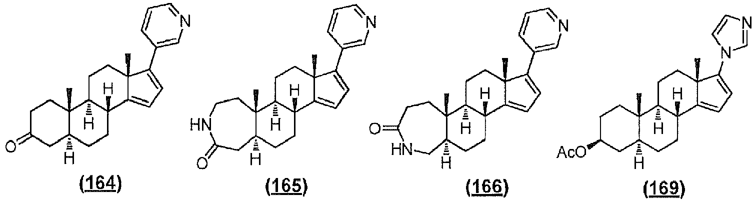

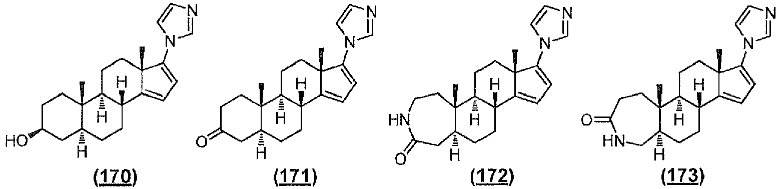

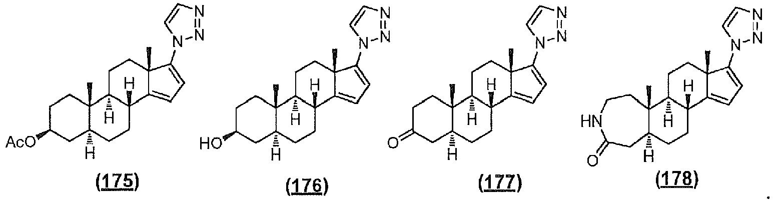

Embodiment 60b: Also described herein is a compound selected from

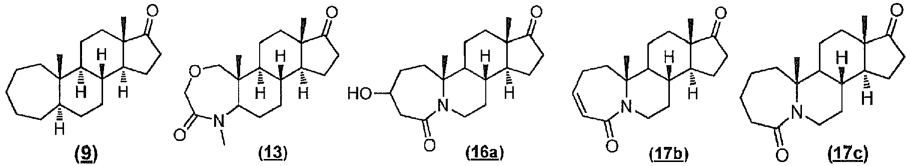

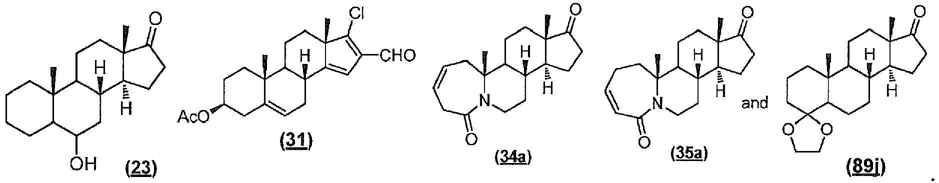

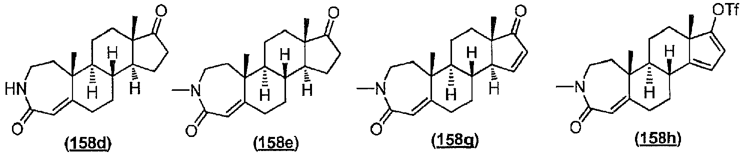

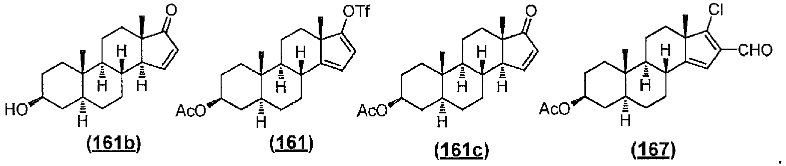

Embodiment 61: Provided herein are pharmaceutical compositions comprising
1) a compound from Table 2 or Table 3 or having the structure of Formula Z, I, IA, IB, IC, ID, IE, IF, IG, IH, U, IK, IL, II, ILA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a pharmaceutically acceptable salt, a pharmaceutically acceptable solvate, pharmaceutically acceptable prodrug thereof; and
2) a pharmaceutically acceptable carrier, excipient, binder or diluent thereof.
In some cases, the pharmaceutical formulation is an oral dosage form.

Also provided herein are methods of treating an androgen-dependent disease in a subject in need of such treatment comprising administering to the subject a therapeutically effective amount of a compound of Table 2, Table 3, or having a structure of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In another embodiment the androgen-dependent disorder is selected from the group consisting of prostate cancer, benign prostatic hyperplasia, prostatic intraepithelial neoplasia, hirsutism, acne, androgenic alopecia, and polycystic ovary syndrome. In some or any embodiments, the androgen-dependent disorder is prostate cancer. In another embodiment, the disease is prostate cancer.

In some embodiments, disclosed is a method for treating cancer in a subject comprising administering to a subject in need a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In another embodiment, the cancer is bladder cancer, brain cancer, breast cancer, cervical cancer, colorectal cancer, endometrial cancer, gastric cancer, glioblastoma, head and neck cancer, Kaposi's sarcoma, kidney cancer, leiomyosarcoma, leukemia, liver cancer, lung cancer, melanoma, multiple myeloma, Non-Hodgkin lymphoma, ovarian cancer, pancreatic cancer, papillary renal cell carcinoma, prostate cancer, renal cancer, squamous cell cancer, and thoracic cancer. In another embodiment, the cancer is prostate cancer, breast cancer, or ovarian cancer.

In some embodiments, disclosed is a method for treating breast cancer in a subject comprising administering to a subject in need a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In another embodiment the interconversion of estrogens and adrenal and ovarian androgens is inhibited.

In some embodiments, disclosed is a method for treating prostate cancer in a subject comprising administering to a subject in need a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61.

In other embodiments, the compositions described herein are suited to treating or preventing hormone-dependent cancers in individuals genetically predisposed to such cancers, particularly those predisposed due to an alteration in the CYP11B, CYP17, and/or CYP21 genes.

In some or any embodiments, the method of treating cancer further comprises providing to the subject in need thereof an additional therapy selected from the group consisting of surgery, radiation therapy, chemotherapy, gene therapy, immunotherapy, and a combination thereof. In some or any embodiments, disclosed is a method for treating cancer in a subject comprising administering to a subject in need thereof a therapeutically acceptable amount of a compound of Table 2, Table 3, or having the structure of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In some or any embodiments, the administration of the at least one additional agent is performed concurrently or sequentially. In some or any embodiments, the method further comprises administering a therapeutically effective amount of at least one additional agent or performing an additional therapy, said agent or therapy selected from the group consisting of a chemotherapeutic agent, a biological agent, surgery, and radiation therapy. In some or any embodiments, the cancer is breast cancer.

In some or any embodiments, the additional pharmaceutically active agent for combination therapy is an anti-cancer agent. In some or any embodiments, the active ingredient(s) and pharmaceutically active agents are administered separately or together. In further embodiments, separate administration occurs simultaneously or separately in any order. The amounts of the active ingredients(s) and pharmaceutically active agent(s) and the relative timings of administration will be selected in order to achieve the desired combined therapeutic effect.

In another embodiment, disclosed is a method for treating a hormone-dependent cancer in a subject comprising administering to a subject 1) an aromatase inhibitor and 2) a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In another embodiment, the cancer is breast cancer. In another embodiment, the cancer is prostate cancer. In some or any embodiments, the administration is performed concurrently or sequentially.

In another embodiment, disclosed is a method for treating cancer in a subject comprising administering to a subject 1) surgery and 2) a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In some or any embodiments, the administration is performed concurrently or sequentially.

In another embodiment, disclosed is a method for treating cancer in a subject comprising administering to a subject 1) radiation and 2) a therapeutically effective amount of a compound of Table 2, Table 3, or having the structure of Formula Z, I, IA, IB, IC, ID, IE, IF, IG, IH, IJ, IK, IL, IJ, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In some or any embodiments, the administration is performed concurrently or sequentially.

In another embodiment, disclosed is a method for treating cancer in a subject comprising administering to a subject 1) chemotherapy and 2) a therapeutically effective amount of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In some or any embodiments, the chemotherapy is a therapeutically effective amount of at least one anti-androgenic agent. In some or any embodiments, the anti-androgenic agent is selected from the group consisting of flutamide, nicalutamide, bicalutamide, inhibitors of 17α-hydroxylase/C17-20 lyase, luteinizing hormone-releasing hormone agonists, luteinizing hormone-releasing hormone antagonists, and 5α-reductase type 1 and/or type 2 and combinations thereof. In some or any embodiments, the administration is performed concurrently or sequentially.

Also disclosed herein is a method of inhibiting CYP11B, CYP17, and/or CYP21 enzyme comprising contacting a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIA, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61 with CYP11B, CYP17, and/or CYP21 enzymes where the contacting step is *in vivo.*

Presented herein is a method of treating a proliferative disease comprising administering to a subject in need thereof a therapeutically effective amount of a compound of Table 2, Table 3, or having the structure of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61.

In some or any embodiments, disclosed is a method of treating a disease associated with cancer ameliorated by the inhibition of CYP11B, CYP17, and/or CYP21 enzymes comprising administering to a subject in need thereof a therapeutically-effective amount of a compound of Table 2, Table 3, or having the structure of Formula Z, I, IA, IB, IC, ID, IE, IF, IG, IH, U, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61.

Also provided herein are methods of treating a CYP11B, CYP17, and/or CYP21-associated disease or disorder in a subject in need of such treatment comprising administering to the subject a therapeutically effective amount of a compound of Table 2, Table 3, or having a structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61. In some or any embodiments, the disease is a sex steroid hormone dependent cancer, such as androgen-dependent prostate cancer, which in some or any embodiments is treated by inhibiting CYP11B, CYP17, and/or CYP21-mediated androgen synthesis, and estrogen-dependent breast cancer or ovarian cancer, which in other embodiments is treated by inhibiting CYP11B, CYP17, and/or CYP21-mediated estrogen synthesis.

Also provided herein are methods of treating a CYP11B, CYP17, and/or CYP21-associated disease or disorder in a subject in need of such treatment comprising administering to the subject a therapeutically effective amount of a compound of Table 2, Table 3, or having a structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61 where the CYP11B, CYP17, and/or CYP21-associated disease or disorder includes those associated with mineralocorticoid excess such as hypertension caused by sodium retention at renal tubules. In some or any embodiments, a decrease in CYP11B, CYP17, and/or CYP21 activity results in an alteration in mineralocorticoid (e.g. aldosterone) biosynthesis. Accordingly, in some or any embodiments, the CYP11B, CYP17, and/or CYP21-associated diseases include those associated with altered levels of aldosterone production (e.g. hypertension, primary adrenal hyperplasia).

Also provided herein are methods of treating a CYP11B, CYP17, and/or CYP21-associated disease or disorder in a subject in need of such treatment comprising administering to the subject a therapeutically effective amount of a compound of Table 2, Table 3, or having a structure of Formula Z, I, IA, IB, IC, ID, IE, IF, IG, IH, IJ, IK, IL, II, IIA, IIB, IIC, IID, IIIE, III, IIIA, IIIB, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61 where the disease or disorder is Cushing's disease, prostatic hyperplasia, glucocorticoid deficiency, or endometrial cancer.

In another aspect, disclosed is a method of treating a disease associated with hypercortisolism comprising administering to a subject in need thereof a therapeutically effective amount of a compound having the structure of Formula (Z), IX, X, XI, XII, XIII, XIV, XIV-1, XV, XVI, or XVII, or a pharmaceutically acceptable salt or solvate thereof. In another embodiment, the disease associated with hypercortisolism is Cushing's Syndrome.

In some aspects, disclosed is an article of manufacture, comprising
1) packaging material;
2) a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, 111B, IIIC, IIID, IIIE, IIIF-1, IIIF-2, IV, IVA, IVB, IVC, IVD, IVE, IVF, IVG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61; and
3) a label;
where the compound is effective for the treatment of an androgen dependent disorder, where the compound is packaged within the packaging material, and where the label indicates that the compound, or pharmaceutically acceptable salt or solvate thereof is used for the treatment of an androgen dependent disorder.

Certain embodiments provide a use of a compound of Table 2, Table 3, or having the structure of Formula Z, I, IA, IB, IC, ID, IE, IF, IG, IH, IJ, IK, IL, II, IIA, IIB, IIC, IID, IIE, III, IIIA, IIIB, IIIC, IIID, IIIE, IIIF-1, IIIF-2, IV, IVA, IVB, IVC, IVD, IVE, IVF, NG, IVH, V, VA, VB, VC, VD, VE, VI, VIa, VII, VIIa, VIIb, VIII, VIIIa, IX, X, X1, X2, X3, X4, XI, XIa, XIb, XIc, XId, XIe, XIf, XIg, XIh, XII, XIII, XIV, XIV-1, XV, XVI, or XVII where all groups are as defined for a compound of Formula (Z) or as defined in any of embodiments 1-59 or a therapeutically acceptable salt or solvate thereof or administering a pharmaceutical composition according to any of the embodiments in Embodiment 61, in preparation of a medicament for treating one or more diseases associated with the CYP11B, CYP17, and/or CYP21 enzymes. In another embodiment, the disease is cancer or an androgen-dependent disorder. In some or any embodiments, the androgen-dependent disorder is prostate cancer.

### Abbreviations

The following abbreviations have these meanings.

| | |
|---|---|
| DCC | *N,N'*-dicyclohexylcarbodiimide |
| DCM | dichloromethane |
| DDQ | 2,3-dichloro-5,6-dicyanobenzoquinone |
| DEAD | diethylazodicarboxylate |
| DHEA | dehydroepiandrosterone |
| DIPEA | *N,N*-diisopropylethylamine |
| DMAP | 4-dimethylaminopyridine |
| DMP | Dess-Martin periodinane |
| DMSO | dimethylsulfoxide |
| DPPA | diphenylphosphorylazide |
| equiv | equivalent |
| LC-MS | liquid chromatography-mass spectrometry |
| mL | milliliter |
| TEA | triethylamine |
| THF | tetrahydrofuran |
| TLC | thin layer chromatography |
| v/v | volume/volume |

### Definitions

Unless defined otherwise, all technical and scientific terms used herein have the standard meaning pertaining to the claimed subject matter belongs. In the event that there are a plurality of definitions for terms herein, those in this section prevail. Where reference is made to a URL or other such identifier or address, it understood that such identifiers can change and particular information on the internet can come and go, but equivalent information can be found by searching the internet. Reference thereto evidences the availability and public dissemination of such information.

It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

"Acyl" means a -C(O)R where R is alkyl, cycloalkyl, aryl, heteroaryl (bonded through a ring carbon) or heteroalicyclic (bonded through a ring carbon).

"Alkenyl" means a linear monovalent hydrocarbon radical of two to ten carbons or a branched monovalent hydrocarbon radical of three to six carbon atoms which hydrocarbon contains at least one double bond, in another example one or two double bonds. Illustrative examples of alkenyl are ethenyl, 2-propenyl, 2-methyl-2-propenyl, 3-butenyl, 4-pentenyl, 5-hexenyl, 2-heptenyl, 2-methyl-1-heptenyl, and 3-cecenyl. Unless stated otherwise, alkenyl can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, halo, haloalkoxy, -NR_{C}R_{D} and protected derivatives thereof, and -C(O)NR_{C}R_{D}.

"Alkoxy" as used herein, means an -OR group where R is an alkyl group, as defined herein. Illustrative examples of alkoxy are methoxy, ethoxy, propoxy, 2-propoxy, butoxy, tert-butoxy, pentyloxy, and hexyloxy. Unless stated otherwise, the alkoxy can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, halo, haloalkoxy, -NR_{C}R_{D} and protected derivatives thereof, and -C(O)NR_{C}R_{D}.

"Alkoxyalkyl," as used herein, means an alkyl group substituted with at least one, for example one or two, alkoxy, as defined herein. Illustrative examples of alkoxyalkyl are 2-methoxyethyl, 2-ethoxyethyl, tert-butoxyethyl and methoxymethyl. Unless stated otherwise, the alkoxyalkyl can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfiinyl, arylsulfinyl, cyano, carboxy, halo, haloalkoxy, -NR_{C}R_{D} and protected derivatives thereof, and -C(O)NR_{C}R_{D}.

"Alkoxycarbonyl" as used herein, means a -C(O)OR group where R is alkyl, as defined herein. Illustrative examples of alkoxycarbonyl are methoxycarbonyl, ethoxycarbonyl, and tert-butoxycarbonyl.

"Alkyl" as used herein means a linear saturated monovalent hydrocarbon radical of one to ten carbons or a branched saturated monovalent hydrocarbon radical of three to six carbon atoms. Illustrative examples of alkyl are methyl, ethyl, n-propyl, iso-propyl, n-butyl, sec-butyl, tert-butyl, n-pentyl, isopentyl, neopentyl, n-hexyl, 3-methylhexyl, 2,2-dimethylpentyl, 2,3-dimethylhexyl, n-heptyl, n-octyl, n-nonyl, and n-decyl. "C₁-C₆ alkyl" as used herein means an hydrocarbon of one to six carbons.

"Alkylamino" means a -NHR group where the R is alkyl, as defined herein.

"Dialkylamino" means an -NRR group where R is alkyl, as defined herein.

"Alkylcarbonyl" as used herein, means a -C(O)R group where R is alkyl, as defined herein. Illustrative examples of alkylcarbonyl are acetyl, 1-oxopropyl, 2,2-dimethyl-1-oxopropyl, 1-oxobutyl, and 1-oxopentyl.

"Alkylcarbonyloxy" as used herein, means an -OC(O)R group where R is alkyl, as defined herein. Illustrative examples of alkylcarbonyloxy are acetyloxy, ethylcarbonyloxy, and *tert*-butylcarbonyloxy. Unless stated otherwise, alkylcarbonyloxy can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, halo, haloalkoxy, -NR_{C}R_{D} and protected derivatives thereof, and -C(O)NR_{C}R_{D}.

"Alkylsulfinyl" means an -S(O)R group where R is alkyl, as defined herein.

"Alkylsulfonyl" means an -S(O)₂R group where R is alkyl, as defined herein.

"Alkylthio" as used herein, means an -SR group where R is alkyl group, as defined herein. Illustrative examples of alkylthio are methylthio, ethylthio, butylthio, *tert*-butylthio, and hexylthio.

"Alkylthioalkyl" as used herein, means an alkyl group substituted with at least one, for example one or two, alkylthio groups, as defined herein. Illustrative examples of alkylthioalkyl are methylthiomethyl, 2-(ethylthio)ethyl, butylthiomethyl, and hexylthioethyl.

"Alkynyl" as used herein, means a linear monovalent hydrocarbon radical of two to ten carbons or a branched monovalent hydrocarbon radical of three to six carbon atoms which hydrocarbon contains at least one triple bond, in another example one or two triple bonds. Illustrative examples of alkynyl are acetylenyl, 1-propynyl, 2-propynyl, 3-butynyl, 2-pentynyl, and 1-butynyl. Unless stated otherwise, the alkynyl can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, halo, haloalkoxy, -NR_{C}R_{D} and protected derivatives thereof, and -C(O)NR_{C}R_{D}.

"Aryl" means a monovalent six- to fourteen-membered, mono- or bi-carbocyclic ring, where the monocyclic ring is aromatic and at least one of the rings in the bicyclic ring is aromatic. Unless stated otherwise, the valency of the group may be located on any atom of any ring within the radical, valency rules permitting. Representative examples include phenyl, naphthyl, and indanyl, and indenyl and the like. Unless stated otherwise, aryl can be optionally substituted with 1, 2, 3, 4, or 5 of the following groups independently selected from alkyl, alkenyl, alkynyl, alkoxyalkyl, alkoxycarbonyl, alkylcarbonyl, alkylcarbonyloxy, cycloalkyl, heteroaryl, heteroalicyclic, hydroxy, alkoxy, aryloxy, mercapto, alkylthio, alkylthioalkyl, arylthio, alkylsulfonyl, arylsulfonyl, alkylsulfinyl, arylsulfinyl, cyano, carboxy, halo, isocyanato, thiocyanato, isothiocyanato, nitro, formyl, haloalkoxy, haloalkyl, hydroxyalkyl, oxo, -NR_{C}R_{D} and the protected derivatives thereof, and -C(O)NR_{C}R_{D}.

"Arylalkyl" as used herein, means an alkyl group substituted with one or two aryl group(s), as defined herein. Illustrative examples of arylalkyl are benzyl, 2-phenylethyl, - phenylpropyl, 1-methyl-3-phenylpropyl, and 2-naphth-2-ylethyl.

"Arylcarbonyloxy" means an -OC(O)R group where R is aryl as defined herein.

"Arylsulfinyl" means an -S(O)R group where R is aryl, as defined herein.

"Arylsulfonyl" means an -S(O)₂R group where R is aryl, as defined herein.

"Bond" or "single bond" as used herein, refers to a chemical bond between two atoms, or two moieties when the atoms joined by the bond are considered to be part of larger substructure.

"Cycloalkyl" means a monocyclic or fused or bridged bicyclic or tricyclic, saturated or partially unsaturated (but not aromatic), monovalent hydrocarbon radical of three to ten carbon ring atoms. Unless stated otherwise, the valency of the group may be located on any atom of any ring within the radical, valency rules permitting. One or two ring carbon atoms may be replaced with a -C(O)-, -C(S)-, or -C(=NH)- group. More specifically, the term cycloalkyl includes, but is not limited to, 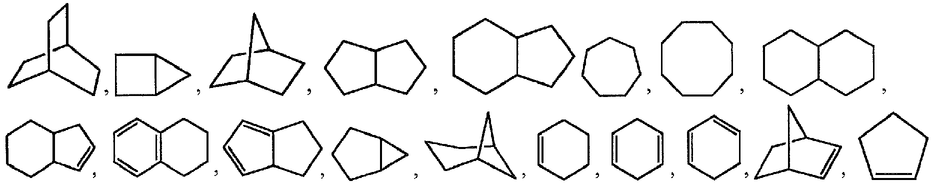 , cyclopropyl, cyclobutyl, cyclopentyl, cyclohexyl, and the like. Unless stated otherwise, cycloalkyl can be optionally substituted with 1, 2, 3, or 4 of the following groups independently selected from alkyl, alkenyl, alkynyl, alkoxyalkyl, alkoxycarbonyl, alkylcarbonyl, alkylcarbonyloxy, aryl, heteroaryl, heteroalicyclic, hydroxy, alkoxy, aryloxy, mercapto, alkylthio, alkylthioalkyl, arylthio, alkylsulfonyl, arylsulfonyl, alkylsulfinyl, arylsulfinyl, cyano, carboxy, halo, isocyanato, thiocyanato, isothiocyanato, nitro, formyl, haloalkoxy, haloalkyl, hydroxyalkyl, oxo, -NR_{C}R_{D} and the protected derivatives thereof, and -C(O)NR_{C}R_{D}.

"Ester" means a -C(O)OR group where R is alkyl, cycloalkyl, aryl, heteroaryl (bonded through a ring carbon) or heteroalicyclic (bonded through a ring carbon). Any hydroxy, or carboxyl side chain on the compounds described herein is optionally esterified.

"Formyl" as used herein, means a -C(O)H group.

"Halo" or "halogen" as used herein, means a -Cl, -Br, -I or -F.

"Haloalkyl" means an alkyl group, as defined herein, substituted with at least one halo, for example 1, 2, 3, 4, or 5 halo. Where the haloalkyl has more than one halo, the halo can be the same or different. Unless stated otherwise, the haloalkyl can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, haloalkoxy, -NR_{C}R_{D} and protected derivatives thereof, and -C(O)NR_{C}R_{D}.

"Haloalkoxy" means an -OR group where R is haloalkyl, as defined herein.

"Haloalkoxyalkyl" means an alkyl group substituted with one or two haloalkoxy as defined herein.

"Hydroxyalkyl" means an alkyl group, as defined herein, substituted with at least one, in another example 1, 2, or 3, hydroxy groups. Unless stated otherwise, the hydroxyalkyl can be optionally substituted with 1, 2, 3, 4, or 5 of the following groups independently selected from alkoxycarbonyl, alkylcarbonyl, alkylcarbonyloxy, hydroxy, alkoxy, aryloxy, mercapto, alkylthio, arylthio, alkylsulfonyl, arylsulfonyl, alkylsulfinyl, arylsulfinyl, cyano, carboxy, haloalkoxy,-NR_{C}R_{D} and protected derivatives thereof, and -C(O)NR_{C}R_{D}.

"Heteroalkyl" means an unsubstituted alkyl group as defined herein where one or more skeletal chain atoms are selected from an atom other than carbon, e.g., oxygen, nitrogen, sulfur, silicon, phosphorus or combinations thereof.

"Heteroatom" as used herein refers to an atom other than carbon or hydrogen. Heteroatoms are typically independently selected from among oxygen, sulfur, nitrogen, silicon and phosphorus, but are not limited to these atoms. In embodiments in which two or more heteroatoms are present, the two or more heteroatoms are the same as one another, or some or all of the two or more heteroatoms are different from the other or others.

As used herein, the term "ring system" refers to two or more rings, where two or more of the rings are fused. The term "fused" refers to structures in which two or more rings share one or more bonds.

"Heteroaryl" means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), -NH-, -N=, or N-oxide, with the remaining ring atoms being carbon, where the ring comprising a monocyclic radical is aromatic and where at least one of the fused and bridged rings comprising a bicyclic radical is aromatic. One or two ring carbon atoms of any nonaromatic rings comprising the bicyclic radical may be replaced by a -C(O)-, -C(S)-, or -C(=NH)- group. Unless stated otherwise, the valency of the group may be located on any atom of any ring within the radical, valency rules permitting. More specifically the term heteroaryl includes, but is not limited to, triazolyl, phthalimidyl, pyridinyl, pyrrolyl, imidazolyl, thienyl, furanyl, indolyl, 2,3-dihydro-1*H*-indolyl (including, for example, 2,3-dihydro-1*H*-indol-2-yl or 2,3-dihydro-1*H*-indol-5-yl, and the like), isoindolyl, indolinyl, isoindolinyl, benzimidazolyl, benzodioxol-4-yl, benzofuranyl, cinnolinyl, indazolyl, indolizinyl, naphthyridin-3-yl, phthalazin-3-yl, phthalazin-4-yl, pteridinyl, purinyl, quinazolinyl, quinoxalinyl, tetrazoyl, pyrazolyl, pyrazinyl, pyrimidinyl, pyridazinyl, oxazolyl, isooxazolyl, oxadiazolyl, benzoxazolyl, quinolinyl, isoquinolinyl, tetrahydroisoquinolinyl (including, for example, tetrahydroisoquinolin-4-yl or tetrahydroisoquinolin-6-yl, and the like), pyrrolo[3,2-c]pyridinyl (including, for example, pyrrolo[3,2-c]pyridin-2-yl or pyrrolo[3,2-c]pyridin-7-yl, and the like), benzopyranyl, thiazolyl, isothiazolyl, thiadiazolyl, benzothiazolyl, benzothienyl, thieno[3,2-b]thienyl, benzotriazolyl, 1H-[1,2,3]triazolo[4,5-d]pyrimidinyl, isobenzofuranyl, and the derivatives thereof, or N-oxide or a protected derivative thereof. Unless stated otherwise, heteroaryl can be optionally substituted with 1, 2, 3, 4, or 5 of the following groups independently selected from alkyl, alkenyl, alkynyl, alkoxyalkyl, alkoxycarbonyl, alkylcarbonyl, alkylcarbonyloxy, cycloalkyl, aryl, heteroaryl, heteroalicyclic, hydroxy, alkoxy, aryloxy, mercapto, alkylthio, alkylthioalkyl, arylthio, alkylsulfonyl, arylsulfonyl, alkylsulfinyl, arylsulfinyl, cyano, carboxy, halo, isocyanato, thiocyanato, isothiocyanato, nitro, formyl, haloalkoxy, haloalkyl, hydroxyalkyl, oxo, -NR_{C}R_{D} and the protected derivatives thereof, and -C(O)NR_{C}R_{D}.

"Heteroarylalkyl" means an alkyl group, as defined herein, substituted with at least one, for example one or two, heteroaryl, as defined herein.

"Heteroarylamino" means an -NRR' group where R is hydrogen or alkyl, as defined herein, and R' is heteroaryl, as defined herein.

"Heteroarylcarbonyloxy" means an -OC(O)R group where R is heteroaryl as defined herein.

"Non-aromatic heterocycle", "non-aromatic heterocyclic", "heterocycloalkyl" or "heteroalicyclic" means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon. One or two ring carbon atoms may be replaced by a -C(O)-, -C(S)-, or -C(=NH)- group. Unless otherwise stated, the valency of the group may be located on any atom of any ring within the radical, valency rules permitting. Illustrative examples include lactams, lactones, cyclic imides, cyclic thioimides, cyclic carbamates, More specifically the term heterocycloalkyl includes, but is not limited to, azetidinyl, pyrrolinyl, pyrrolidinyl, 2-oxopyrrolidinyl, 2,5-dioxo-1*H*-pyrrolyl, 2,5-dioxo-pyrrolidinyl, 2,5-dihydro-1*H*-pyrrolyl, piperidinyl, 4-piperidonyl, morpholinyl, piperazinyl, 2-oxopiperazinyl, dioxopiperazinyl, pyranyl, tetrahydropyranyl, tetrahydrothiopyranyl, 1,3-dioxinyl, 1,3-dioxanyl, 1,4-dioxinyl, 1,4-dioxanyl, 2-oxopiperidinyl, thiomorpholinyl, thiamorpholinyl, perhydroazepinyl, pyrazolidinyl, imidazolinyl, imidazolidinyl, 2,4-dioxo-imidazolidinyl, dihydropyridinyl, tetrahydropyridinyl, oxazolinyl, oxazolidinyl, isoxazolidinyl, thiazolinyl, thiazolidinyl, quinuclidinyl, isothiazolidinyl, octahydroindolyl, octahydroisoindolyl, decahydroisoquinolyl, 1,3-oxathianyl, 1,4-oxathiinyl, 1,4-oxathianyl, tetrahydro-1,4-thiazinyl, 2*H*-1,2-oxazinyl, tetrahydrofuryl, 2,4,6-trioxo-(1*H*,3*H*,5*H*)pyrimidinyl, 4,6-dioxo-2-(1*H*,5*H*)thioxodihydropyrimidinyl, 2,4(1*H*,3*H*)-dioxo-dihydropyrimidinyl, trioxanyl, hexahydro-1,3,5-triazinyl, tetrahydrothienyl, tetrahydrofuranyl, pyrazolinyl, 1,3-dioxolyl, 1,3-dioxolanyl, 1,3-dithiolyl, 1,3-dithiolanyl, isoxazolinyl, isoxazolidinyl, oxazolidinonyl, 1,3-oxathiolanyl, 2(3*H*)-oxo-dihydrothienyl, 2(3H)-oxo-dihydrofuranyl, 1,1-dioxo-tetrahydrothienyl, 2-oxo-1,3-dioxolanyl, 4,5-dihydrooxazolyl, oxiranyl, (1s,4s)-7-oxabicyclo[2.2.1]heptanyl, 2,3-dihydrobenzo[*b*][1,4]dioxinyl, 4*H*-1,4-thiazinyl, octahydro-1*H*-quinolizinyl, and tetrahydropyranyl, and the derivatives thereof and N-oxide or a protected derivative thereof. Additional examples include 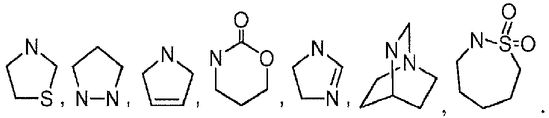 The term heteroalicyclic also includes all ring forms of the carbohydrates, including but not limited to the monosaccharides, the disaccharides and the oligosaccharides. Unless stated otherwise, the heterocycloalkyl can be optionally substituted with 1, 2, 3, 4, or 5 of the following groups independently selected from alkyl, alkenyl, alkynyl, alkoxyalkyl, alkoxycarbonyl, alkylcarbonyl, alkylcarbonyloxy, cycloalkyl, aryl, heteroaryl, heteroalicyclic, hydroxy, alkoxy, aryloxy, mercapto, alkylthio, alkylthioalkyl, arylthio, alkylsulfonyl, arylsulfonyl, alkylsulfinyl, arylsulfinyl, cyano, carboxy, halo, isocyanato, thiocyanato, isothiocyanato, nitro, formyl, haloalkoxy, haloalkyl, hydroxyalkyl, oxo, -NR_{C}R_{D} and the protected derivatives thereof, and - C(O)NR_{C}R_{D}.

"Heterocycloalkylcarbonyloxy" means an -OC(O)R group where R is heterocycloalkyl as defined herein.

"Heterocycle" means a heteroaryl, as defined herein, or heterocycloalkyl, as defined herein. In addition to those listed for "heterocycloalkyl," examples of non-aromatic heterocyclic groups are pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. In addition to those listed for "heteroaryl," examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. Unless stated otherwise, heterocycle can be optionally substituted with 1, 2, 3, 4, or 5 of the following groups independently selected from alkyl, alkenyl, alkynyl, alkoxyalkyl, alkoxycarbonyl, alkylcarbonyl, alkylcarbonyloxy, cycloalkyl, aryl, heteroaryl, heteroalicyclic, hydroxy, alkoxy, aryloxy, mercapto, alkylthio, alkylthioalkyl, arylthio, alkylsulfonyl, arylsulfonyl, alkylsulfinyl, arylsulfinyl, cyano, carboxy, halo, isocyanato, thiocyanato, isothiocyanato, nitro, formyl, haloalkoxy, haloalkyl, hydroxyalkyl, oxo, -NR_{C}R_{D} and the protected derivatives thereof, and -C(O)NR_{C}R_{D}.

As used herein, the term "moiety" refers to a specific segment or functional group of a molecule. Chemical moieties are often recognized chemical entities embedded in or appended to a molecule.

As used herein, the term "sulfonyl" refers to a -S(O)₂-R, where R is alkyl, cycloalkyl, aryl, heteroaryl (bonded through a ring carbon) or heteroalicyclic (bonded through a ring carbon).

"Isocyanato" means an -NCO group.

"Thiocyanato" means a -CNS group.

"Isothiocyanato" means a -NCS group.

"Mercapto" as used herein, means an -SH group.

"Oxo" as used herein, means a =O group.

In the groups "-NR_{C}R_{D}" and "-C(O)NR_{C}R_{D}," R_{C} and R_{D} are independently selected from the group consisting of hydrogen, alkyl, haloalkyl, alkoxyalkyl, cycloalkyl, aryl, arylalkyl, heteroaryl, and heteroarylalkyl; or R_{C} and R_{D} taken together with the nitrogen atom form a 4 to 7 membered heterocyclic ring, as defined herein.

In some or any embodiments, the compounds described herein exist as stereoisomers, where asymmetric or chiral centers are present. Stereoisomers are designated (R) or (S) depending on the configuration of substituents around the chiral carbon atom. The term (R) and (S) used herein are configurations as defined in IUPAC 1974 Recommendations for Section E, Fundamental Stereochemistry, Pure Appl. Chem., (1976), 45:13-30, hereby incorporated by reference for this purpose. The embodiments described herein specifically includes the various stereoisomers and mixtures thereof. Stereoisomers include geometric isomers, regioisomers, enantiomers, diastereomers, and mixtures thereof. In some or any embodiments, individual stereoisomers of compounds are prepared synthetically from commercially available starting materials which contain asymmetric or chiral centers or by preparation of racemic mixtures followed by resolution. These methods of resolution are exemplified by (1) attachment of a mixture of enantiomers to a chiral axillary, separation of the resulting mixture of diastereomers by recrystallization or chromatography and liberation of the optically pure product from the auxiliary or (2) direct separation of the mixture of optical enantiomers on chiral chromatographic column.

The methods and formulations described herein include the use of *N*-oxides, crystalline forms (also known as polymorphs), or pharmaceutically acceptable salts of compounds described herein, as well as active metabolites of these compounds having the same type of activity. In some situations, compounds exist as tautomers. All tautomers are included within the scope of the compounds presented herein. In some or any embodiments, the compounds described herein exist in unsolvated as well as solvated forms with pharmaceutically acceptable solvents such as water, ethanol, and the like. The solvated forms of the compounds presented herein are also considered to be disclosed herem

Throughout the specification, groups and substituents thereof are chosen, in certain embodiments, to provide stable moieties and compounds

### Preparation of Compounds

In certain embodiments, the compounds described herein are synthesized using any synthetic techniques including standard synthetic techniques and the synthetic processes described herein. In specific embodiments, the following synthetic processes are utilized.

### Formation of Covalent Linkages by Reaction of an Electrophile with a Nucleophile

Selected examples of covalent linkages and precursor functional groups which yield them are given in the Table entitled "Examples of Covalent Linkages and Precursors Thereof." Precursor functional groups are shown as electrophilic groups and nucleophilic groups In certain embodiments, a functional group on an organic substance is attached directly, or attached via any useful spacer or linker as defined below

**Table 1: Examples of Covalent Linkages and Precursors Thereof**

| Covalent Linkage Product | Electrophile | Nucleophile |
|---|---|---|
| Carboxamides | Activated esters | amines/anilines |
| Carboxamides | acyl azides | amines/anilines |
| Carboxamides | acyl halides | amines/anilines |
| Esters | acyl halides | alcohols/phenols |
| Esters | acyl nitriles | alcohols/phenols |
| Carboxamides | acyl nitriles | amines/anilines |
| Imines | Aldehydes | amines/anilines |
| Hydrazones | aldehydes or ketones | Hydrazines |
| Oximes | aldehydes or ketones | Hydroxylamines |
| Alkyl ammes | alkyl halides | amines/anilines |
| Esters | alkyl halides | carboxylic acids |
| Thioethers | alkyl halides | Thiols |
| Ethers | alkyl halides | alcohols/phenols |
| Thioethers | alkyl sulfonates | Thiols |
| Esters | alkyl sulfonates | carboxylic acids |
| Ethers | alkyl sulfonates | alcohols/phenols |
| Esters | Anhydrides | alcohols/phenols |
| Carboxamides | Anhydrides | amines/anilines |
| Thiophenols | aryl halides | Thiols |
| Aryl amines | aryl halides | Amines |
| Thioethers | Azmdines | Thiols |
| Boronate esters | Boronates | Glycols |
| Carboxamides | carboxylic acids | amines/anilines |
| Esters | carboxylic acids | Alcohols |
| hydrazines | Hydrazides | carboxylic acids |
| N-acylureas or Anhydrides | carbodiimides | carboxylic acids |
| Esters | diazoalkanes | carboxylic acids |
| Thioethers | Epoxides | Thiols |
| Thioethers | haloacetamides | Thiols |
| Ammotriazmes | halotnazines | amines/anilines |
| Triazinyl ethers | halotnazines | alcohols/phenols |
| Amidines | imido esters | amines/anilines |
| Ureas | Isocyanates | amines/anylines |
| Urethanes | Isocyanates | alcohols/phenols |
| Thioureas | isothiocyanates | amines/anilines |
| Thioethers | Maleimides | Thiols |
| Phosphite esters | phosphoramidites | Alcohols |
| Silyl ethers | silyl halides | Alcohols |
| Alkyl amines | sulfonate esters | amines/anilines |
| Thioethers | sulfonate esters | Thiols |
| Esters | sulfonate esters | carboxylic acids |
| Ethers | sulfonate esters | Alcohols |
| Sulfonamides | sulfonyl halides | amines/anilines |
| Sulfonate esters | sulfonyl halides | phenols/alcohols |

In general, carbon electrophiles are susceptible to attack by complementary nucleophiles, including carbon nucleophiles, where an attacking nucleophile brings an electron pair to the carbon electrophile in order to form a new bond between the nucleophile and the carbon electrophile.

Suitable carbon nucleophiles include, but are not limited to alkyl, alkenyl, aryl and alkynyl Grignard, organohthium, organozmc, alkyl-, alkenyl, aryl- and alkynyl-tin reagents (organostannanes), alkyl-, alkenyl-, aryl- and alkynyl-borane reagents (organoboranes and organoboronates); these carbon nucleophiles have the advantage of being kinetically stable in water or polar organic solvents. Other carbon nucleophiles include phosphorus ylids, enol and enolate reagents; these carbon nucleophiles have the advantage of being relatively easy to generate from precursors Carbon nucleophiles, when used in conjunction with carbon electrophiles, engender new carbon-carbon bonds between the carbon nucleophile and carbon electrophile

Non-carbon nucleophiles suitable for coupling to carbon electrophiles include but are not limited to primary and secondary amines, thiols, thiolates, and thioethers, alcohols, alkoxides, azides, semicarbazides, and the like. These non-carbon nucleophiles, when used in conjunction with carbon electrophiles, typically generate heteroatom (C-X-C), where X is a heteroatom, e. g, oxygen or nitrogen.

### Use of Protecting Groups

The term "protecting group" refers to chemical moieties that block some or all reactive moieties and prevent such groups from participating in chemical reactions until the protective group is removed. In specific embodiments, more than one protecting group is utilized. In more specific embodiments, each protective group is removable by a different process. Protective groups that are cleaved under totally disparate reaction conditions fulfill the requirement of differential removal. In various embodiments, protective groups are removed by acid, base, or hydrogenolysis. Groups such as trityl, dimethoxytrityl, acetal and t-butyldimethylsilyl are acid labile and are, in some or any embodiments, used to protect carboxy and hydroxy reactive moieties in the presence of amino groups protected with Cbz groups, which are removable by hydrogenolysis, and Fmoc groups, which are base labile. In some or any embodiments, carboxylic acid and hydroxy reactive moieties are blocked with base labile groups such as, without limitation, methyl, ethyl, and acetyl in the presence of amines blocked with acid labile groups such as t-butyl carbamate or with carbamates that are both acid and base stable but hydrolytically removable.

In certain embodiments, carboxylic acid and hydroxy reactive moieties are blocked with hydrolytically removable protective groups such as the benzyl group, while, in some or any embodiments, amine groups capable of hydrogen bonding with acids are blocked with base labile groups such as Fmoc. In various embodiments, carboxylic acid reactive moieties are protected by conversion to simple ester derivatives as exemplified herein, or they are blocked with oxidatively-removable protective groups such as 2,4-dimethoxybenzyl, while, in some or any embodiments, co-existing amino groups are blocked with fluoride labile silyl carbamates.

In certain instances, allyl blocking groups are useful in the presence of acid- and base-protecting groups since the former are stable. In some or any embodiments, such groups are subsequently removed by metal or pi-acid catalysts. For example, in some or any embodiments, an allyl-blocked carboxylic acid is deprotected with a Pd⁰-catalyzed reaction in the presence of acid labile t-butyl carbamate or base-labile acetate amine protecting groups. In some or any embodiments, a protecting group is a resin to which a compound or intermediate is attached. As long as the residue is attached to the resin, that functional group is blocked and cannot react. Once released from the resin, the functional group is available to react.

In some or any embodiments, blocking/protecting groups are selected from, by way of non-limiting example:

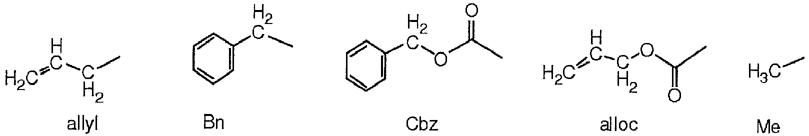

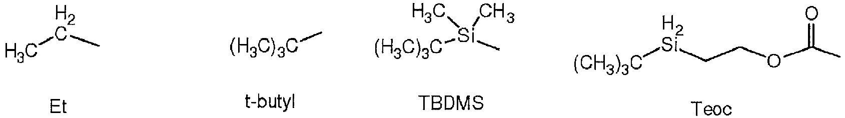

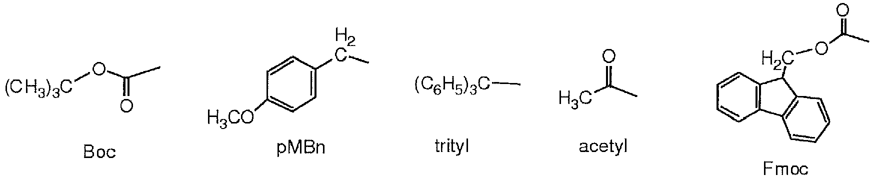

Other protecting groups are described in Greene and Wuts, Protective Groups in Organic Synthesis, 3rd Ed., John Wiley & Sons, New York, NY, 1999.

In certain embodiments, compounds of Formula (I), (II), (III), (IV), (V), (VI), (VII), (VIII), (IA), (IB), (IC), (ID), (IE), (IF), (IG), (IH), (IIA), (IIB), (IIC), (IID), (IIIA), (IIIB), (IIIC), (IIID), (IVA), (IVB), (IVC), (IVD), (IVE), (VA), (VB), (VC) and (VD) are prepared by various methods, as outlined in Synthetic Schemes I-XXXXIV. In each scheme, the variables (e.g., R¹, R², R³, etc) are represented by hydrogen. In some or any embodiments, compounds are synthesized using methodologies analogous to those described below by the use of appropriate alternative starting materials. When R² and R³ are other than hydrogen, the appropriate starting material is obtained before subsequent synthetic steps are performed.

In certain embodiments, compounds of Formula (I) where d is 2, E is O, and e is 2 or where d is 3, E is O, e is 1, and 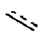 is a single bond are synthesized according to Synthetic

### Scheme I.

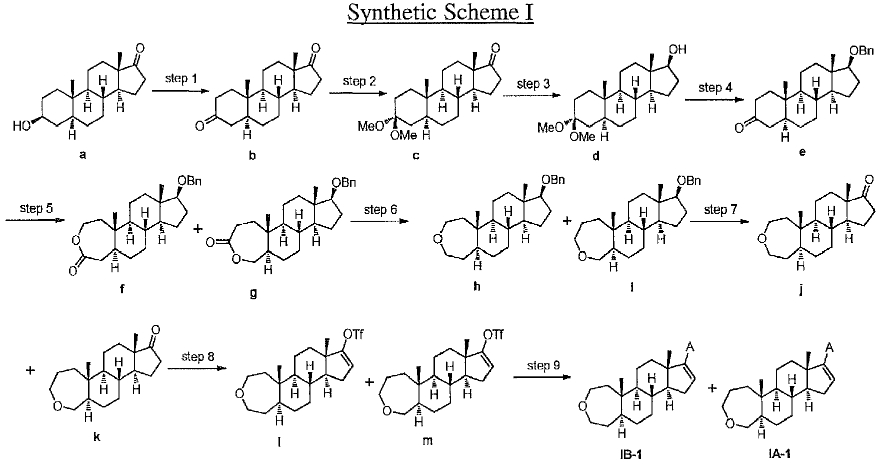

Compounds of Formula **(IA-1)** and **(1B-1)** where 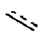 is a single bond are synthesized from commercially available starting material epiandrosterone **(a).** Jones oxidation of the hydroxyl group of compound **(a)** using chromium trioxide in acetic acid or in acetone in the presence of dilute sulphuric gives the diketone compound **(b)** (step 1), Selective.ketalization of the 6-membered ring (ring A) keto group of compound **(b)** with iodine in methanol gives the ketal compound **(c)** (step 2). Sodium borohydride reduction on compound **(c)** in methanol yields the alcohol compound **(d)** (step 3). Treatment of compound **(d)** with benzyl bromide in the presence of sodium hydride in organic solvent such as tetrahydrofuran (THF) followed by treatment with dilute acid gives the alcohol protected keto compound **(e)** (step 4). Oxidation of compound **(e)** with m-chloroperbenzoic acid in methylene dichloride gives a mixture of cyclic lactones (**f**) and (g) (step 5). Reduction of the mixture lactones **(f)** and **(g)** with triethylsilane, trimethylsilyl trifluoromethanesulfonate, trifluoroborane and pyridine in methylene dichloride yields a mixture of the ether compounds **(h)** and **(i)** (step 6). Deprotection of the benzyl group of compounds **(h)** and (i) in hydrogen atmosphere in the presence of catalytic amount of palladium on carbon in ethylacetate gave a mixture of the alcohols which is oxidized in situ in the presence of chromium trioxide in dilutic acid yields a mixture of keto compounds (**j**) and **(k)** which are purified and separated by flash column chromatography (step 7). Conversion of compounds compounds (**j**) and **(k)** to compounds (**l**) and **(m)** is achieved by using enol triflate formation condition by the use of triflic anhydride (trifluoromethanesulfonic anhydride) in the presence of base such as triethylamine and the like (step 8). Suzuki coupling reaction on compounds **(l)** and **(m)** with A-B(ethyl)₂ or A-B(OH)₂, and (Ph₃P)₂PdCl₂ in THF - water or dioxane - water in the presence of a base such as sodium carbonate followed by acidification yields compounds of formula **(IA-1)** and **(1B-1)** where 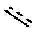 is a single bond.

In certain embodiments, compounds of Formula (I) where the heteroaryl A is attached to the rest of scaffold at a nitrogen of the heteroaryl group, e.g. imidazol-1-yl, d is 2 or 3, E is O, e is 1 or 2, and 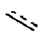 is a single bond are synthesized according to Synthetic Scheme II. 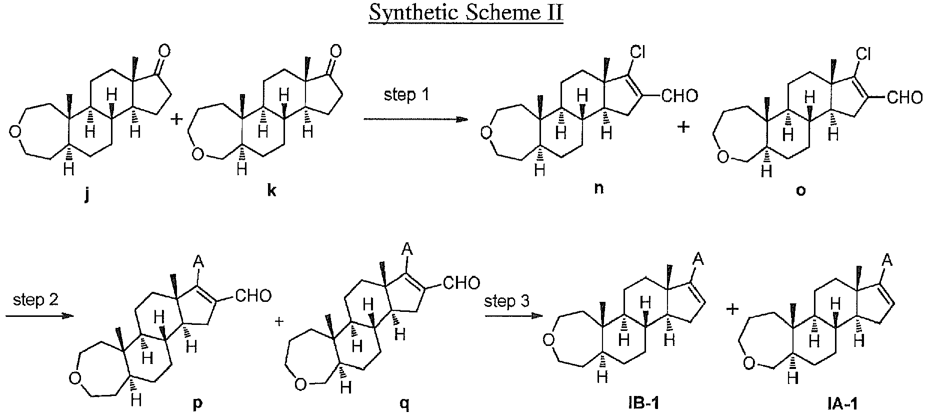 Compounds having the structure of Formula **(IA-1)** and **(1B-1)** where 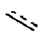 is a single bond are synthesized from compounds (**j**) and (k) which is synthesized by a route mentioned above. Formylation of the keto compounds (**j**) and **(k)** with Vilsmeier-Haack reagents of DMF and phosphoryl chloride in dichloromethane gives the vinyl chloro aldehyde compounds (n) and (**o**) (step 1).Displacement of the chloro group of compounds **(n)** and (**o**) with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the imidazolyl derivatives compound **(p)** and **(q)** (step 2). Treatment of compounds **(p)** and **(q)** with palladium on carbon in benzonitrile at elevated temperature removed the formyl group to give compounds **(IA-1)** and **(1B-1)** where 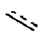 is a single bond (step 3). Compounds with A as other heteroaryl groups are prepared using similar synthetic route with the use of appropriate heteroaryl in synthetic step 2.

In certain embodiments, compounds of Formula **(I)** where d is 1 or 2, E is O, e is 1 or 2, and 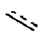 is a single bond are synthesized according to Synthetic Scheme III. 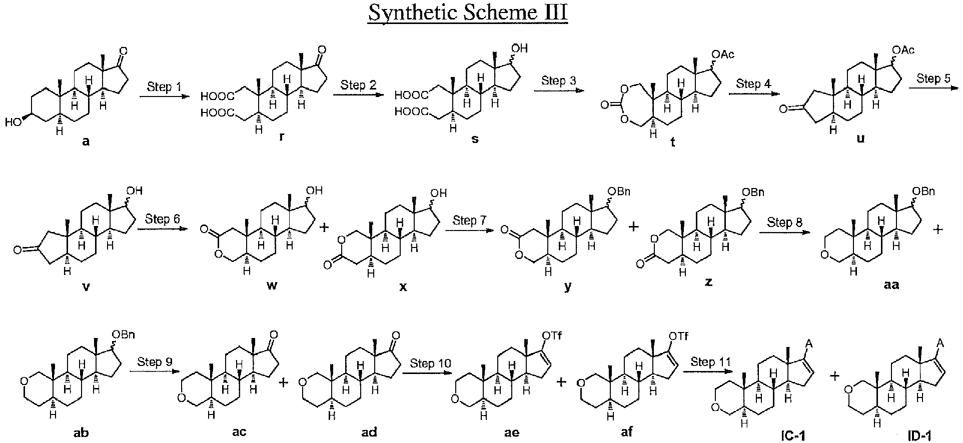 Compounds of structure of Formula **(IC-1)** and **(ID-1**) where 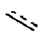 is a single bond are synthesized from epiandrosterone compound **(a)** which is commericially available. Oxidation of compound **(a)** with chromium (VI) oxide in the presence of sulfuric acid and acetic acid -water yields the diacid compound **(r)** (step 1). Reduction the the diacid compound **(r)** with sodium borohydride gives the alcohol compound **(s)** (step 2). Treatment of compound (s) with sodium acetate in acetic anhydride yields the anhydride compound **(t)** (step 3). Compound **(t)** upon heating gives the ketone acetate **(u)** (step 4). Hydrolysis of the O-acetyl group with sodium hydroxide solution yields the ketone alcohol compound **(v)** (step 5). Treatment of compound **(v)** with m-chloroperbenzoic acid in dichloromethane yields a mixture of cyclic lactones **(w)** and **(x)** (step 6). Treatment of the mixture compounds **(w)** and (**x**) with benzyl bromide in the presence of sodium hydride in organic solvent such as tetrahydrofuran (THF) followed by treatment with dilute acid gives the protected alcohol mixture of compounds **(y)** and **(z)** (step 7). Reduction of the mixture lactones **(y)** and (**z**) with triethylsilane, trimethylsilyl trifluoromethanesulfonate, trifluoroborane and pyridine in methylene dichloride yields a mixture of the ether compounds **(aa)** and **(ab)** (step 8). Deprotection of the benzyl group of compounds **(aa)** and **(ab)** in hydrogen atmosphere in the presence of catalytic amount of palladium on carbon in ethylacetate gave a mixture of the alcohols which is oxidized in situ in the presence of chromium trioxide in dilutic acid yields a mixture of keto compounds **(ac)** and **(ad)** which are purified and separated by flash column chromatography (step 9). Conversion of compounds **(ac)** and **(ad)** to vinyl triflate compounds **(ae)** and (**af**) is achieved by using enol triflate formation condition by the use of triflic anhydride (trifluoromethanesulfonic anhydride) in the presence of base such as triethylamine and the like (step 10). Suzuki coupling reaction on compounds **(ae)** and (**af**) with A-B(ethyl)₂ or A-B(OH)₂, and (Ph₃P)₂PdCl₂ in THF - water or dioxane - water in the presence of a base such as sodium carbonate followed by acidification yields compounds of formula **(IC-1)** and **(ID-1)** where 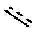 is a single bond (step 11).

In certain embodiments, compounds of Formula (**I**) where the A is attached to the rest of scaffold at a nitrogen of the heteroaryl group, e.g. imidazol-1-yl, d is 1 or 2, E is O, e is 1 or 2, and 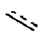 is a single bond are synthesized according to Synthetic Scheme IV.. 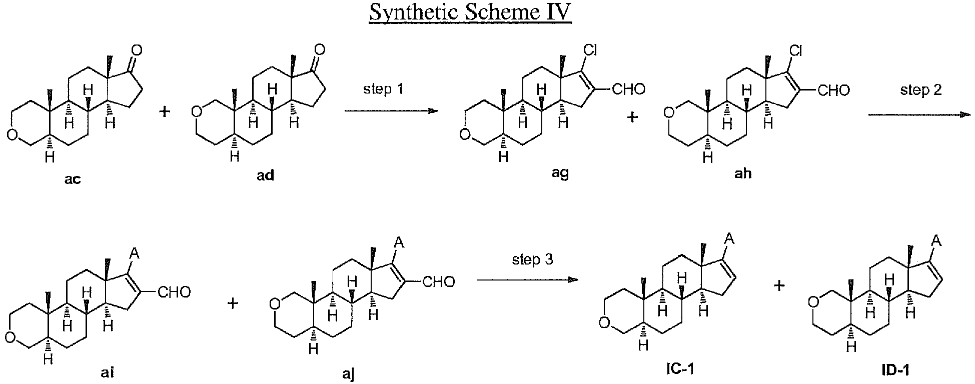 Compounds having the structure of Formula **(IC-1)** and **(ID-1)** where 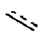 is a single bond are synthesized from compounds **(ac)** and **(ad)** which is synthesized by a route mentioned above. Formylation of the keto compounds **(ac)** and **(ad)** with Vilsmeier-Haack reagents of DMF and phosphoryl chloride in dichloromethane gives the vinyl chloro aldehyde compounds **(ag)** and **(ah)** (step 1).Displacement of the chloro group of compounds **(ag)** and **(ah)** with A-H (where AH is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivatives compound **(ai)** and (**aj**) (step 2). Treatment of compounds **(ai)** and (**aj**) with palladium on carbon in benzonitrile at elevated temperature removes the formyl group to give compounds **(IC-1)** and **(ID-1)** where 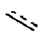 is a single bond (step 3).

In certain embodiments, compounds of Formula (**I**) are synthesized according to Synthetic Scheme V where d is 2 or 3, E is N-C(O)R¹, e is 1 or 2, and 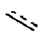 is a single bond. 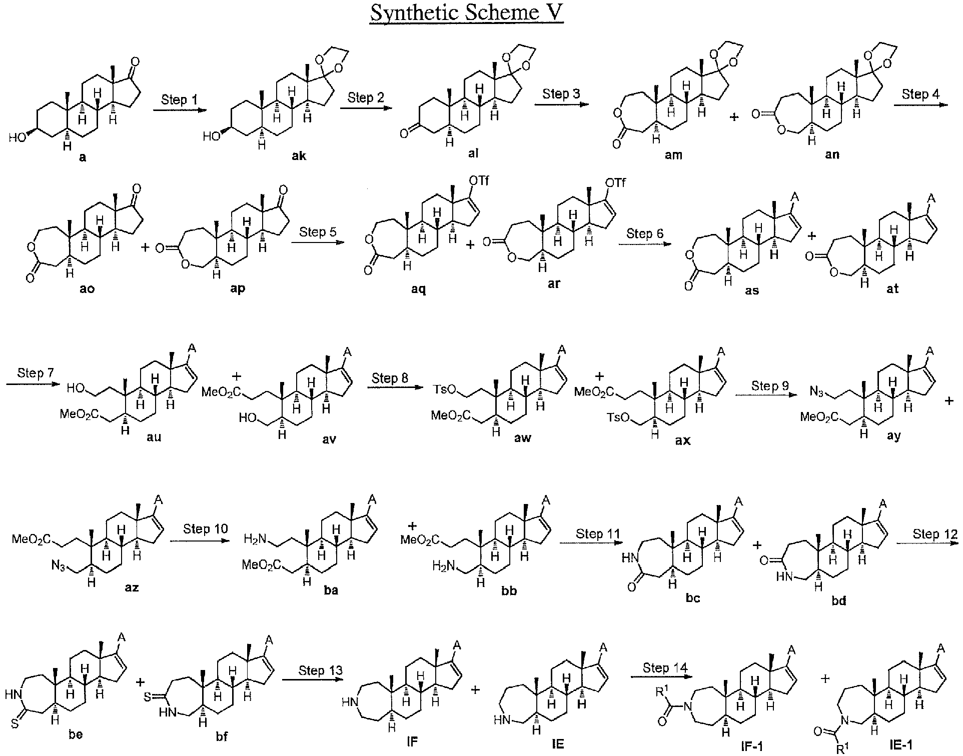 Compounds of structure of Formula **IE-1** and **IF-1** where 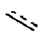 is a single bond are synthesized from commercially available starting material epiandrosterone **(a).** Ketalization of the keto group of compound **(a)** with p-toluenesulfonic acid and ethylene glycol in organic solution such as toluene and the like at reflux with removal of water gives compound (**ak**) (step 1). Oxidation of compound (**ak**) with Dess-Martin Periodinane in dichloromethane gives the keto product compound **(al)** (step 2). Treatment of compound (**al**) with m-chloroperbenzoic acid in dichloromethane yields a mixture of compounds **(am** and **an)** (step 3). These two compounds are separated by flash column chromatography. Treatment of the mixture of compounds **(am)** and **(an)** with p-toluenesulfonic acid in water-acetone solution yields a mixture of the keto compounds **(ao)** and **(ap)** which are purified and separated by flash column chromatography (step 4). Conversion of compounds **(ao)** and **(ap)** to compounds (**aq**) and **(ar)** is achieved by using condition involving the enol triflate formation by the use of triflic anhydride ( trifluoromethanesulfonic anhydride) in the presence of base such as triethylamine and the like (step 5). Suzuki coupling reaction on compounds (**aq**) and **(ar)** with A-B(ethyl)₂ or A-B(OH)₂, and (Ph₃P)₂PdCl₂ in THF - water or dioxane - water in the presence of a base such as sodium carbonate followed by acidification yields compounds of formula **(as)** and **(at)** (step 6). Hydrolysis the lactone ring of mixture of compounds of formula **(as)** and **(at)** with the use of p-toluenesulfonic acid in methanol yields the mixture of ester-alcohol of compounds of formula **(au)** and **(av)** (step 7). Treatment of the mixture of ester-alcohol compounds of formula **(au)** and **(av)** with p-toluenesulfonyl chloride in the presence of pyridine or triethyl amine and the like in dichloromethane or other organic solvent yields the mixture tosyl compound **(aw)** and **(ax)** (step 8). Treatment of the mixture tosyl compound **(aw)** and **(ax)** with sodium azide in DMF or similar organic solvent yields the azide compound **(ay)** and **(az)** (step 9). The azide group of compounds **(ay)** and **(az)** is reduced by the use of tributylphosphine in THF-water to give the mixture of amino compounds **(ba)** and **(bb)** (step 10). (step10). Cyclization of the mixture of amino compounds **(ba)** and **(bb)** with the use of strong base yields mixture of lactam compounds (**bc)** and (**bd**) (step 11). Treatment of mixture of amino compounds **(ba)** and **(bb)** with P₂S₅ in toluene in the presence of pyridine yields a mixture of thiolactam compounds **(be)** and **(bf)** (step 12). Raney Nickel reduction of mixture of thiolactam compounds **(be)** and (**bf**) in methanol yields compounds of Formula **(IF-1)** and **(IE-1)** where 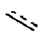 is a single bond (step 13). Reaction of these mixture of compounds with R¹C(O)Cl (or acetic anhydride where R¹ is CH₃) in the presence of base such as pyridine or triethyl amine yields compounds of Formula **(IF-1)** and **(IE-1)** where 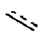 is a single bond (step 14).

In certain embodiments, compounds of Formula (I) where A is attached to the rest of scaffold at a nitrogen of the heteroaryl group, e.g. imidazole-1-yl, d is 2 or 3, E is NH or N-C(O)R¹, e is 1 or 2, and 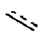 is a single bond, are synthesized according to Synthetic Scheme VI. 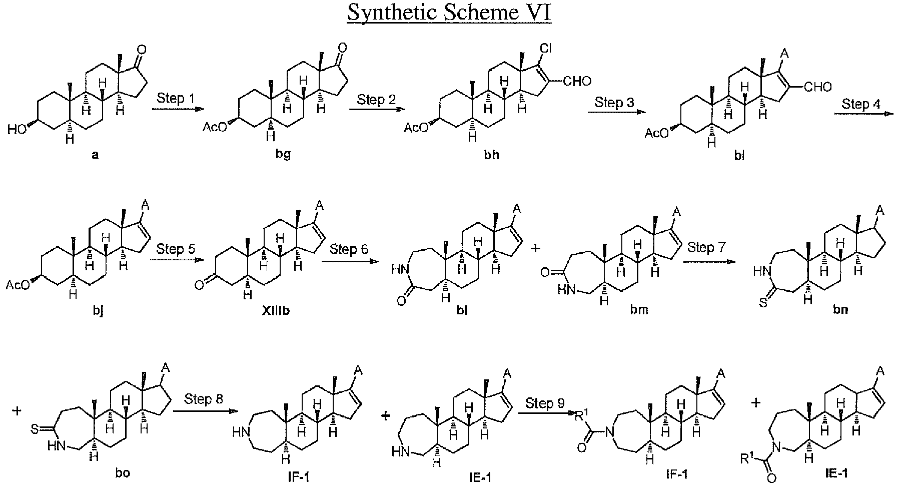 Compounds having the structure of Formula **(IE-1)** and **(IF-1)** where 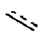 is a single bond are synthesized from epi-androsterone **(a).** Treatment of **(a)** with acetic anhydride in methylene chloride in the presence of a base such as dimethylaminopyridine gives the keto-acetyl compound **(bg)** (step 1). Formylation of the keto-acetyl compound **(bg)** with Vilsmeier-Haack reagents of DMF and phosphoryl chloride in dichloromethane gives the vinyl chloro aldehyde compounds **(bh)** (step 2). Displacement of the chloro group of compound **(bh)** with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(bi)** (step 3). Treatment of compounds **(bi)** with palladium on carbon in benzonitrile at elevated temperature removes the formyl group to give compound **(bj)** (step 4). The compound (**bj**) is subjected to hydrolysis reaction with potassium carbonate in methanol water to yield the corresponding alcohol which is then oxidized of with Dess-Martin Periodinane in dichloromethane gives the keto product compound **(XIIIb)** (step 5). Reaction of compound **(XIIIb)** with hydroxylamine hydrochloride in the presence of triethylamine and organic solvent yields the oxime compound. Beckmann rearrangement reaction on the oxime with thionyl chloride in the presence of pyridine yields a mixture of compounds of formula (**bl**) and **(bm)** (step 6). Treatment of mixture of lactam compounds (**bl**) and **(bm)** with P₂S₅ in toluene in the presence of pyridine yields a mixture of thiolactam compounds **(bn)** and **(bo)** (step 7). Raney Nickel reduction of mixture of thiolactam compounds **(bn)** and **(bo)** in methanol yields compounds of Formula **(IF-1)** and **(IE-1)** where 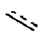 is a single bond (step 8), Reaction of these mixture of compounds with R¹C(O)Cl (or acetic anhydride where R¹ is CH₃) in the presence of base such as pyridine or triethyl amine yields compounds of Formula **(IF-1)** and **(IE-1)** where the A is a heteroaryl attached via a nitrogen and 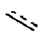 is a single bond (step 9).

In certain embodiments, compounds of Formula **(I)** are synthesized according to Synthetic Scheme VII where d is 2, E is CH₂, e is 2, and 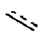 is a single bond. 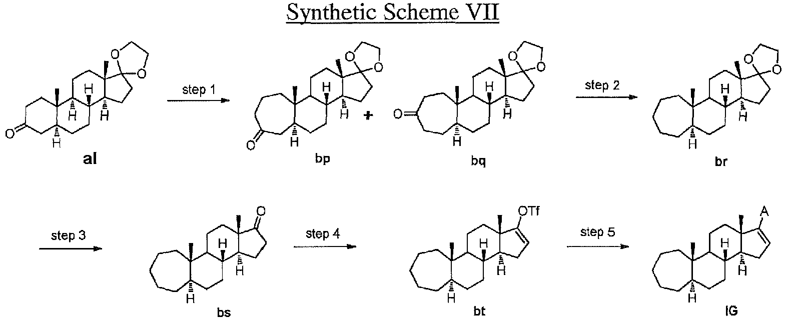 Compounds of structure of Formula **(IG)** where 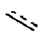 is a single bond are synthesized from compound **(al).** Reaction of compound **(al)** with boron trifluoride etherate and ethyl 2-diazoacetate in organic solvent gives the ring expansion ester. Hydrolysis of this ester derivatives with sodium hydrogen in methanol water and similar solvent system yields the acid with is decarboxylated *in situ* to give a mixture of compounds **(bp)** and **(bq)** (step 1). Treatment of a mixture of compounds **(bp)** and **(bq)** with 4-methylbenzenesulfonohydrazide yields the imine derivatives which are then reduced by the use of sodium borohydride to give compound **(br)**
(step 2). Treatment of the compound **(br)** with p-toluenesulfonic acid in water-acetone solution yields the keto compounds **(bs)** (step 3). Conversion of compound **(bs)** to vinyl triflate compound **(bt)** is achieved by using enol triflate formation condition by the use of triflic anhydride) (trifluoromethanesulfonic anhydride) in the presence of base such as triethylamine and the like (step 4). Suzuki coupling reaction on compound **(bs)** with A-B(ethyl)₂ or A-B(OH)₂ and (Ph₃P)₂PdCl₂ in THF - water or dioxane - water in the presence of a base such as sodium carbonate followed by acidification yields compound of formula **(IG)** where 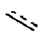 is a single bond
(step 5).

In certain embodiments, compound of Formula **(I)** where A is attached to the rest of scaffold at a nitrogen of the heteroaryl group, e,g, imidazol-1-yl, d is 2, E is CH₂, e is 2, and 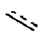 is a single bond, are synthesized according to Synthetic Scheme VIII. 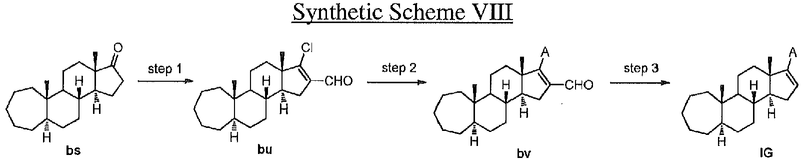 Compound having the structure of Formula **(IG)** where A is an heteroaryl group attached to the rest of scaffold at a nitrogen atom and 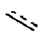 is a single bond is synthesized from compound **(bs)** which is synthesized by a route mentioned above. Formylation of the keto compound **(bs)** with Vilsmeier-Haack reagents of DMF and phosphoryl chloride in dichloromethane gives the vinyl chloro aldehyde compound **(bu)** (step 1). Displacement of the chloro group of compound **(bu)** with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(bv)** (step 2). Treatment of compound **(bv)** with palladium on carbon in benzonitrile at elevated temperature removes the formyl group to give compound **(IG)** where A is an heteroaryl group attached to the rest of scaffold at a nitrogen atom and 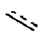 is a single bond (step 3).

In certain embodiments, compounds of Formula **(IH)** where Q² is CH₂, d is 2, E is CH₂, and e is 1 are synthesized according to Synthetic Scheme IX where d is 1, e is 2, E is CH₂ and 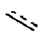 is a single bond. 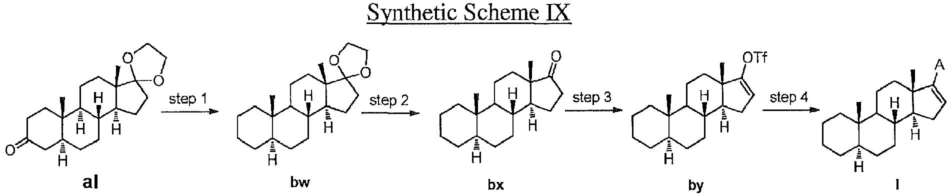 Compounds of structure of Formula **(I)** where Q² is CH₂, d is 2, E is CH₂, and e is 1 are synthesized from compound **(al).** Reaction of compound (**al**) with 4-methylbenzenesulfonohydrazide yields the imine derivative which is then reduced by the use of sodium borohydride to give compound **(bw)** (step1). Treatment of the compound **(bw)** with p-toluenesulfonic acid in water-acetone solution yields the keto compounds **(bx)** (step 2). Conversion of compound **(bx)** to vinyl triflate compound **(by)** is achieved by using enol triflate formation condition by the use of triflic anhydride (trifluoromethanesulfonic anhydride) in the presence of base such as triethylamine and the like (step 3). Suzuki coupling reaction on compound **(by)** with A-B(ethyl)₂ A-B(OH)₂, and (Ph₃P)₂PdCl₂ in THF - water or dioxane - water in the presence of a base such as sodium carbonate followed by acidification yields compound of formula **(I)** where Q² is CH₂, d is 2, E is CH₂, and e is 1.

In certain embodiments, compound of Formula **(I)** where A is attached to the rest of scaffold at a nitrogen of the heteroaryl group, d is 1, e is 2, E is CH₂ and 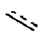 is a single bond represented by an imidazolyl group are synthesized according to Synthetic Scheme X. 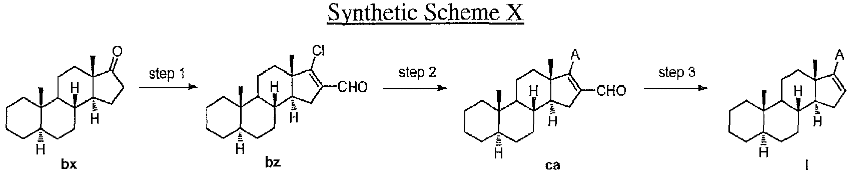 Compounds having the structure of Formula **(I)** where the A is attached to the rest of scaffold at a nitrogen atom, d is 1, e is 2, E is CH₂ and 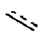 is a single bond, are synthesized from compound **(bx)** which is synthesized by a route mentioned above. Formylation of the keto compound **(bx)** with Vilsmeier-Haack reagents of DMF and phosphoryl chloride in dichloromethane gives the vinyl chloro aldehyde compound **(bz)** (step 1).Displacement of the chloro group of compound **(bz)** with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound (ca) (step 2). Treatment of compound **(ca)** with palladium on carbon in benzonitrile at elevated temperature removes the formyl group to give compound **(I)** (step 3).

In certain embodiments, compounds of Formula **(I)** where d is 1, e is 2, E is CH₂, and 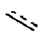 is a double bond are synthesized according to Synthetic Scheme XI. 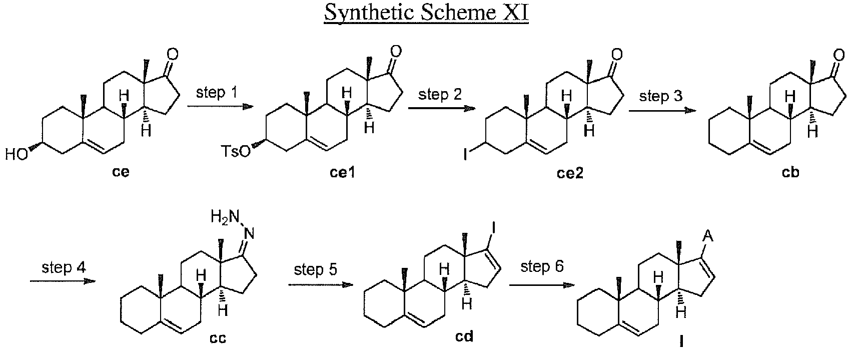 Compounds of structure of Formula **(I)** where d is 1, e is 2, E is CH₂, and 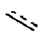 as a double bond, are synthesized from compound (ce). Treatment of compound **(ce)** with tosyl chloride in pyridine gives the tosylate compound (**ce1**) (step 1).Reaction of compound (**ce1**) with sodium iodide in actone at reflux yields the iodo compound **(ce2)** (step 2). Reduction on the iodo compound (ce2) with zinc in acetic acid gives the ring A saturated compound **(cb)** (step 3). Reaction of compound **(cb)** with hydrazine hydrate yields the hydrazone derivative (cc) (step 4). Treatment of the compound **(cc)** with iodine in methylene chloride gives the iodo compounds **(cd)** (step 5). Coupling reaction on compound **(cd)** with A-B(ethyl)₂ or A-B(OH)₂, and Pd(dppf)Cl₂ [(1,1'-Bis(diphenylphosphino)ferrocencepalladium(II) dichloromethane complex]) in 1,4-dioxane-water or in the presence of a base such as sodium carbonate or potassium carbonate followed by acidification yields compound of formula (**I**)) where d is 1, e is 2, E is CH₂, and 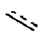 is a double bond (step 6).

In certain embodiments, compound of Formula (**IIIB**) where 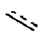 is a double bond and R² and R³ are hydrogen are synthesized according to Synthetic Scheme XII. 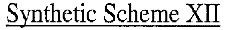 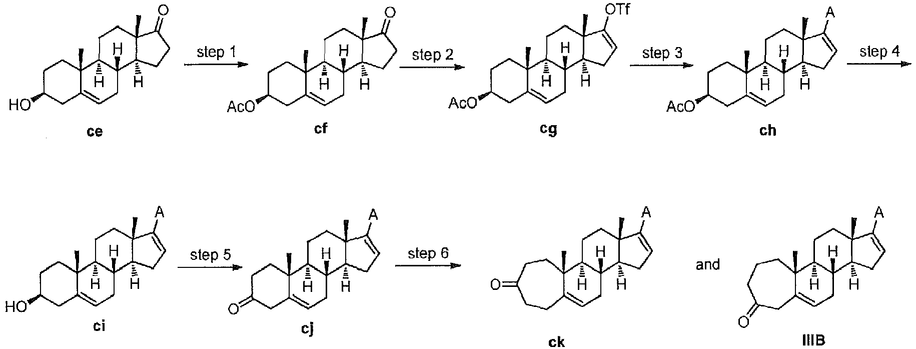 Compounds of structure of Formula **IIIB** where 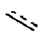 is a double bond and R² and R³ are hydrogen are synthesized from commercially available starting material dehydro epiandrosterone (**ce**). Treatment of **(ce)** with acetic anhydride in methylene chloride in the presence of a base such as sodium acetate or dimethylaminopyridine gives the keto-acetyl compound (**cf**) (step 1). Conversion of compound (**cf**) to triflate compound (**cg**) is achieved by using condition involving the enol triflate formation by the use of triflic anhydride (trifluoromethanesulfonic anhydride) in the presence of base such as triethylamine and the like (step 2). Suzuki coupling reaction on compound (**cg**)) with A-B(ethyl)₂ or A-B(OH)₂, and (Ph₃P)₂PdCl₂ in THF - water or dioxane-water in the presence of a base such as sodium carbonate followed by acidification yields compounds of formula **(ch)** (step 3). Hydrolysis the acetyl group of compound **(ch)** with potassium carbonate in methanol water yields the alcohol compound (**ci**) (step4). Oxidation of compound (**ci**) with oxalyl dichloride and dimethylsulfoxide in methylene chloride gives the keto product compound **(cj)** (step 5). Treatment of compound **(cj)** with greater than 1 equivalent such as 5 equivalent amount of ethyl 2-diazoacetate and borontiifluoride etherate in dichloromethane yields the ring expansion products followed by the addition of dilute sodium hydroxide and then followed by acidification with dilute hydrochloric acid to give decarboxylated mixture of compounds **(ck** and **IIIB**) (step 6). These two compounds are separated by flash column chromatography to give compound of formula **(IIIB)** where 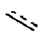 is a double bond and R² and R³ are hydrogen.

In certain embodiments, compounds of Formula (**IIIB**) where A is attached to the rest of scaffold at a nitrogen of the heteroaryl, e.g. imidazolyl, R² and R³ are hydrogen, and 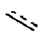 is a double bond are synthesized according to Synthetic Scheme XIII. 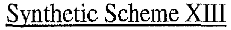 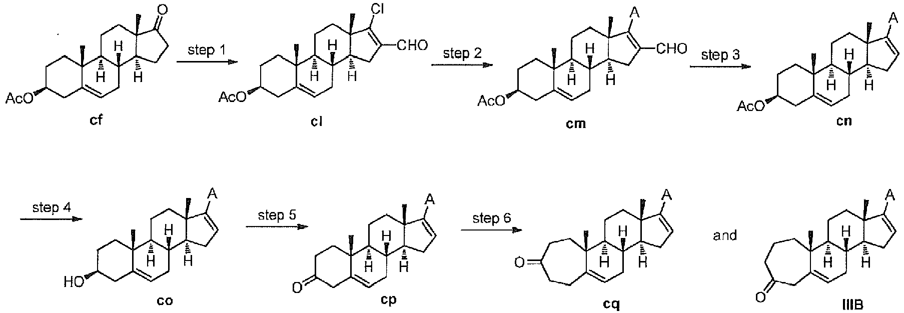 Compounds having the structure of Formula (**IIIB**) where A is attached to the rest of scaffold at a nitrogen of the heteroaryl, e.g. imidazolyl, R² and R³ are hydrogen, and 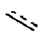 is a double bond are synthesized from keto acetyl compound (**cf**). Formylation of the keto-acetyl compound **(cf)** with Vilsmeier-Haack reagents of DMF and phosphoryl chloride in dichloromethane gives the vinyl chloro aldehyde compounds (**cl**) (step 1). Displacement of the chloro group of compound (**cl**) with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(cm)** (step 2). Treatment of compounds **(cm)** with palladium on carbon in benzonitrile at elevated temperature removes the formyl group to give compound **(cn)** (step 3). Hydrolysis the acetyl group of compound (**cn**) with potassium carbonate in methanol water yields the alcohol compound **(co)** (step4). Oxidation of compound (co) with oxalyl dichloride and dimethylsulfoxide in methylene chloride gives the keto product compound **(cp)** (step 5). Treatment of compound **(cp)** with greater than 1 equivalent such as 5 equivalent amount of ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds **(cq** and **IIIB**) where A is attached to the rest of scaffold at a nitrogen of the heteroaryl, e.g. imidazolyl, R² and R³ are hydrogen, and 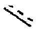 is a double bond (step 6). These two compounds are separated by flash column chromatography to give compound of formula (**IIIB**) where A is attached to the rest of scaffold at a nitrogen of the heteroaryl, e.g. imidazolyl, R² and R³ are hydrogen, and 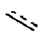 is a double bond.

In certain embodiments, compound of Formula (**IIIB**) where R² and R³ are hydrogen and 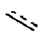 is a single bond are synthesized according to Synthetic Scheme XIV. 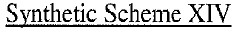 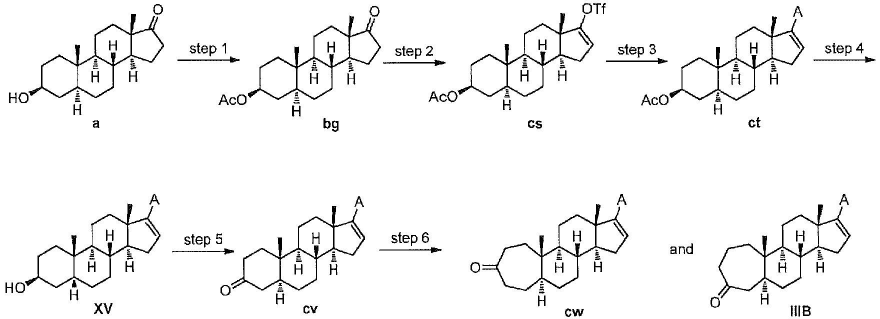 Compounds of structure of Formula **IIIB** where R² and R³ are hydrogen and 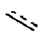 is a single bond are synthesized from commercially available starting material epiandrosterone **(a).** Treatment of **(a)** with acetic anhydride in methylene chloride in the presence of a base such as sodium acetate or dimethylaminopyridine gives the keto-acetyl compound **(bg)** (step 1). Conversion of compound **(bg)** to triflate compound **(cs)** is achieved by using condition involving the enol triflate formation by the use of triflic anhydride (trifluoromethanesulfonic anhydride) in the presence of base such as triethylamine and the like (step 2). Suzuki coupling reaction on compound (**cs**)) with A-B(ethyl)₂ or A-B(OH)₂, and (Ph₃P)₂PdCl₂ in THF - water or dioxane-water in the presence of a base such as sodium carbonate followed by acidification yields compounds of formula **(ct)** (step 3). Hydrolysis the acetyl group of compound **(ct)** with potassium carbonate in methanol water yields the alcohol compound **(XV** where R^{7a}, R²³, R²⁴, R^{24a}, R^{24b}, and R^{30a} are hydrogen) (step 4). Oxidation of compound (**XV** where R^{7a}, R²³, R²⁴, R^{24a}, R^{24b}, and R^{30a} are hydrogen) with oxalyl dichloride and dimethylsulfoxide in methylene chloride gives the keto product compound **(cv)** (step 5). Treatment of compound **(cv)** with greater than 1 equivalent such as 5 equivalent amount of ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds **(cw** and **IIIB**) (step 6). These two compounds are separated by flash column chromatography to give pure compound of formula (**IIIB**) where R² and R³ are hydrogen and 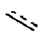 is a single bond.

In certain embodiments, compounds of Formula (**IIIB**) where A is attached to the rest of scaffold at a nitrogen of the heteroaryl, e.g. imidazolyl, R² and R³ are hydrogen, and 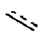 is a single bond, are synthesized according to Synthetic Scheme XV. 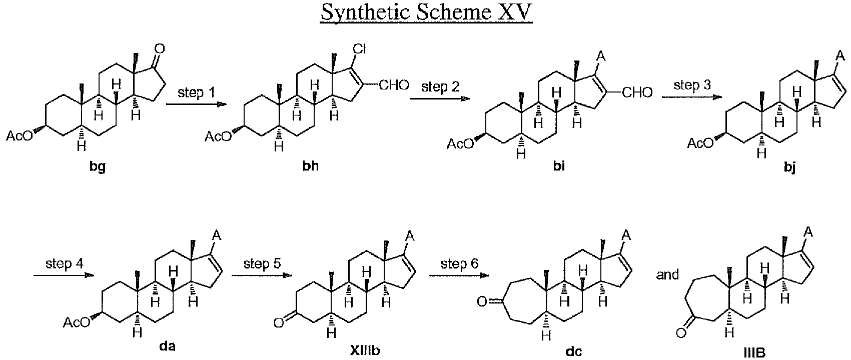 Compounds having the structure of Formula **(IIIB)** where the A is attached to the rest of scaffold at a nitrogen atom, R² and R³ are hydrogen, and 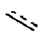 is a single bond are synthesized from keto acetyl compound **(bg).** Formylation of the keto-acetyl compound **(bg)** with Vilsmeier-Haack reagents of DMF and phosphoryl chloride in dichloromethane gives the vinyl chloro aldehyde compounds **(bh)** (step 1). Displacement of the chloro group of compound **(bh)** with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(bi)** (step 2). Treatment of compounds **(bi)** with palladium on carbon in benzonitrile at elevated temperature removes the formyl group to give compound **(bj)** (step 3). Hydrolysis the acetyl group of compound **(bj)** with potassium carbonate in methanol water yields the alcohol compound **(da)** (step4). Oxidation of compound **(da)** with oxalyl dichloride and dimethylsulfoxide in methylene chloride gives the keto product compound **(XIIIb)** (step 5). Treatment of compound **(XIIIb)** with greater than 1 equivalent such as 5 equivalent amount of ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds **(dc** and **IIIB**) (step 6). These two compounds are separated by flash column chromatography to give pure compound of formula (**IIIB**) where the A is attached to the rest of scaffold at a nitrogen atom, R² and R³ are hydrogen, and 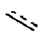 is a single bond.

In certain embodiments, compound of Formula **(IVB)** are synthesized according to Synthetic Scheme XVI, where R¹ is hydrogen or alkyl; R^{7b}, R^{7c}, and R⁸ are hydrogen; the 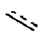 bond between carbons 16 and 17 is a double bond; and the 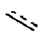 bond between carbons 14 and 15 is a single bond. 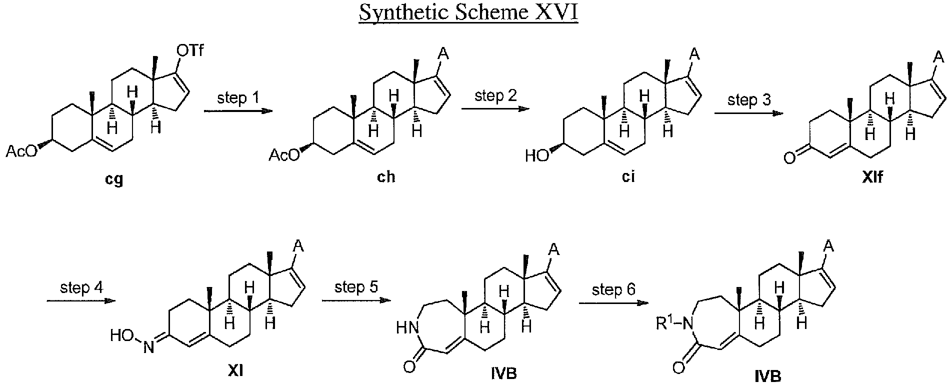 Compounds of structure of Formula **IVB** are synthesized from compound (**cg**) mentioned above. Suzuki coupling reaction on compound (**cg**) with A-B(ethyl)₂ or A-B(OH)₂ and (Ph₃P)₂PdCl₂ in THF - water or dioxane - water in the presence of a base such as sodium carbonate followed by acid treatment yields compounds of formula **(ch)** (step 1). Hydrolysis the acetyl group of compound **(ch)** with potassium carbonate in methanol water yields the alcohol compound (**ci**) (step2). Oxidation of the alcohol group of compound (**ci**) with triisopropoxyaluminum and 2-butanone in toluene gives theα, β- unsaturated keto product compound (**XIf** where R²³, R^{23a}, R²⁴, and R²⁵ are hydrogen) (step 3). Treatment of compound (**XIf**) with hydroxylamine hydrochloride in ethanol in the presence of pyridine or triethylamine yields oxime (**XIf**) (step 4). Beckmann rearrangement reaction on the oxime (**XI** where T is C=N-OH and R²³, R^{23a}, R²⁴, and R²⁵ are hydrogen) with thionyl chloride in the presence of pyridine in tetrahydrofuran yields a ring expansion compound of formula **(IVB)** (step 5). Compounds of structure of Formula **IVB** where R¹ is alkyl are synthesized by alkylation of compound of Formula **(IVB)** where R¹ is hydrogen with R¹-X (where X is halo) in the presence of sodium hydride (step 6).

In certain embodiments, compound of Formula **(IVB)** are synthesized according to Synthetic Scheme XVII, where A is attached to the rest of scaffold at a nitrogen of the heteroaryl group (e.g. imidazolyl); R¹ is hydrogen or alkyl; R^{7b}, R^{7c}, and R⁸ are hydrogen; the 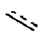 bond between carbons 16 and 17 is a double bond; and the 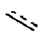 bond between carbons 14 and 15 is a single bond. 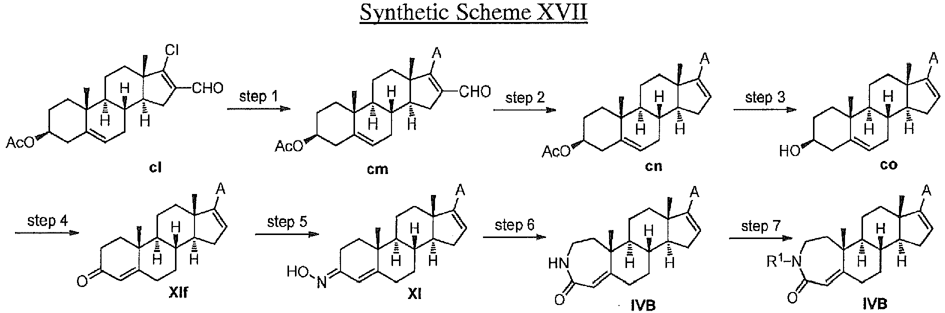 Compounds having the structure of Formula **(IVB)** are synthesized from the vinyl chloro aldehyde compounds (**cl**) mentioned above. Displacement of the chloro group of compound (**cl**) with A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(cm)** (step 1). Treatment of compounds **(cm)** with palladium on carbon in benzonitrile at elevated temperature removes the formyl group to give compound (**cn**) (step 2). Hydrolysis of the acetyl group of compound (**cn**) with potassium carbonate in methanol water yields the alcohol compound **(co)** (step3). Oxidation of the alcohol group of compound **(co)** with triisopropoxyaluminum and 2-butanone in toluene gives theα, β- unsaturated keto product compound (**XIf** where R²³, R^{23a}, R²⁴, and R²⁵ are hydrogen) (step 4). Treatment of compound (**XIf**) with hydroxylamine hydrochloride in ethanol in the presence of pyridine or triethylamine yields oxime (**XI** where T is C=N-OH and R²³, R^{23a}, R²⁴, and R²⁵ are hydrogen) (step 5). Beckmann rearrangement reaction on the oxime (**XI**) with thionyl chloride in the presence of pyridine in tetrahydrofuran yields a ring expansion compound of formula **(IVB)** where the A is an heteroaryl group attached to the rest of scaffold at a nitrogen atom, R¹ is hydrogen (step 6). Compounds of structure of Formula **IVB**, where R¹ is alkyl are synthesized by alkylation of compound of Formula **(IVB)** where R¹ is hydrogen with R¹-X (where X is halo) in the presence of sodium hydride (step 7).

In certain embodiments, compounds of Formula **(IVB)** are synthesized according to Synthetic Scheme XVIII where R¹ is hydrogen or alkyl; R^{7b}, R^{7c}, and R⁸ are hydrogen; and the 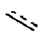 bonds are single bonds. 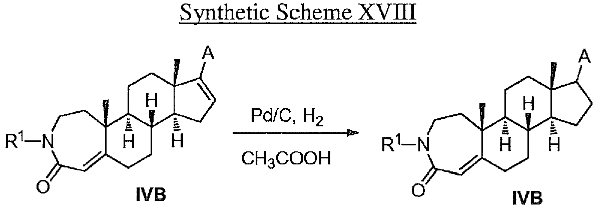 Compounds having the structure of Formula **(IVB)** where the 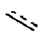 bonds are single bonds can be synthesized from compounds having Formula **(IVB)** where the 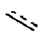 bond between carbonds 16 and 17 is a double bond and the bond between carbons 14 and 15 is a single bond by hydrogenation reaction with palladium on carbon in acetic acid under hydrogen atmosphere at room temperature (synthetic scheme XVIII).

In certain embodiments, compounds of Formula (**IVC**) where R² and R³ are hydrogen are synthesized according to Synthetic Scheme XIX. 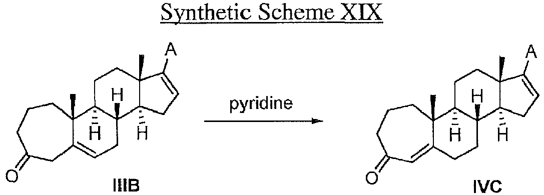 Compounds having the structure of Formula (**IVC**) can be synthesized from compounds having Formula (**IIIB**) (where R² and R³ are hydrogen and the 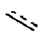 bond is a single bond) by treatment with base such as pyridine of dimethylaminopyridine and the like in an organic solvent at elevated temperature with the migration of the double bond (synthetic scheme XVIII).

In certain embodiments, compounds of Formula (**XIV**) are synthesized according to Synthetic Scheme XX where 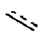 is a double bond and R^{7a}, R²³, R²⁴, and R^{24a} are hydrogen and R³⁰ is hydrogen or acetyl. 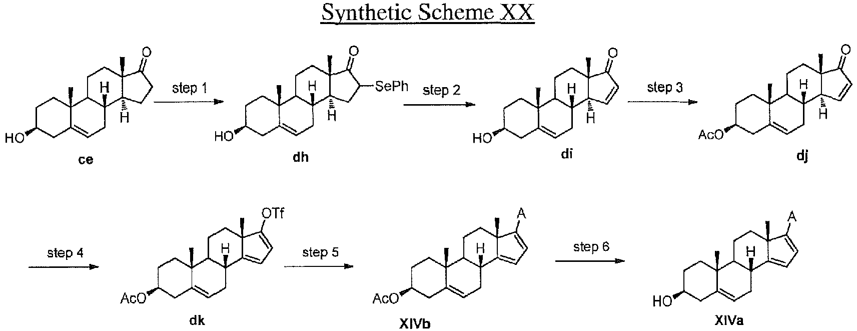 Compounds of structure of Formula **XIV** where 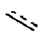 is a double bond and R^{7a}, R²³, R²⁴, and R^{24a} are hydrogen and R³⁰ hydrogen or acetyl are synthesized from commercially available starting material dehydro epiandrosterone **(ce).** Treatment of **(ce)** with lithium diisopropylamide in THF at -78 °C followed by the addition of phenyl selenium bromide yielded compound **(dh)** (step 1) Oxidation of compound **(dh)** with m-chloroperbenzoic acid in carbon tetrachloride at -40 °C followed by the addition of diethylamine at reflux gives the di unstaturated keto compound **(di)** (step 2). Treatment of compound **(di)** with acetic anhydride in pyridine or in methylene chloride in the presence of a base such as sodium acetate or dimethylaminopyridine gives the keto-acetyl compound **(dj)** (step 3). Conversion of compound **(dj)** to triflate compound (**dk**) is achieved by using condition involving the enol triflate formation by the use of triflic anhydride ( trifluoromethanesulfonic anhydride) in the presence of base such as triethylamine and the like (step 4). Suzuki coupling reaction on compound **(dk)** with A-B(ethyl)₂ or A-B(OH)₂, and [1,1'-Bis(diphenylphosphino)ferrocencepalladium(II) dichloromethane complex in 1,4-dioxane-water in the presence of a base such as potassium carbonate followed by acidification yields compounds of formula (**IX**) where 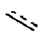 is a double bond and R^{7a}, R²³, R²⁴, and R^{24a} are hydrogen and R³⁰ is acetyl (step 5). Hydrolysis the acetyl group of compound (**XIVb**) with sodium hydroxide in methanol-water yields a compound of Formula **(XIVa)** where 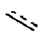 is a double bond and R^{7a}, R²³, R²⁴, R^{24a}, and R³⁰ are hydrogen (step 6).

In certain embodiments, compounds of Formula (**XIV**) where the heteroaryl A is attached to the rest of scaffold at a nitrogen of the heteroaryl group, e.g. imidazolyl, and 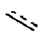 is a double bond and R^{7a}, R²³, R²⁴, and R^{24a} are hydrogen and R³⁰ is hydrogen or acetyl, are synthesized according to Synthetic Scheme XXI. 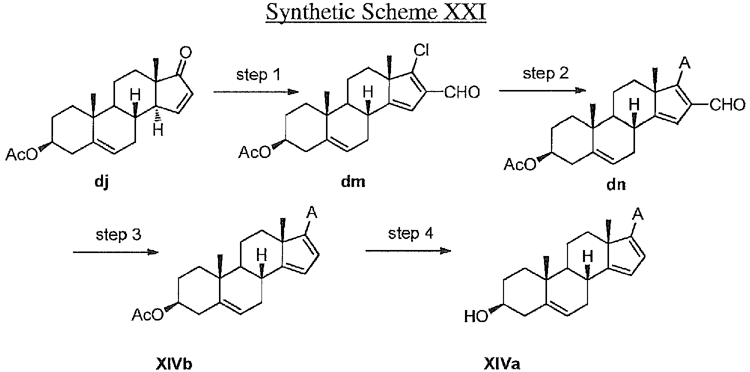 Compounds having the structure of Formula **(XIV)** where the heteroaryl A is attached to the rest of scaffold at a nitrogen of the heteroaryl group, e.g. imidazolyl, and 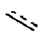 is a double bond and R^{7a}, R²³ , R²⁴, and R^{24a} are hydrogen and R³⁰ is hydrogen or acetyl, are synthesized from keto acetyl di-unsaturated compound **(dj)** mentioned above. Formylation of the keto-acetyl compound (**dj**) with Vilsmeier-Haack reagents of DMF and phosphoryl chloride in dichloromethane gives the unsaturated vinyl chloro aldehyde compounds **(dm)** (step 1). Displacement of the chloro group of compound **(dm)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(dn)** (step 2). Treatment of compounds **(dn)** with 10% palladium on carbon in benzonitrile at elevated temperature removes the formyl group to give compound **(XIYb)** where A is attached to the rest of scaffold at a nitrogen of the heteroaryl group, e.g. imidazolyl, and 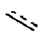 is a double bond and R^{7a}, R²³, R²⁴, and R^{24a} are hydrogen and R³⁰ is acetyl (step 3). Hydrolysis of the acetyl group with potassium carbonate or potassium hydroxide in methanol water yields the alcohol compound of Formula **XIVa** where A is attached to the rest of scaffold at a nitrogen of the heteroaryl group, e.g. imidazolyl, and 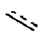 is a double bond and R^{7a}, R²³, R²⁴, R^{24a}, and R³⁰ are hydrogen (step 4).

In certain embodiments, compounds of Formula **(XIb)**, where R²³, R^{23a}, R²⁴, and R²⁵ are hydrogen, are synthesized according to Synthetic Scheme XXII by oxidation of the alcohol group of compound **(XIVa)** with triisopropoxyaluminum and 2-butanone in toluene to give the compound of formula **(XIb)**.

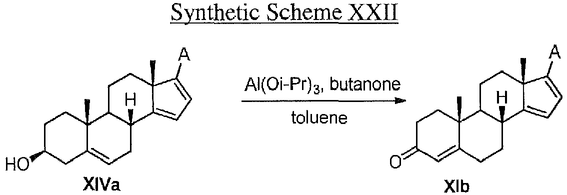

In certain embodiments, compounds of Formula **(XIb)** where A is attached to the rest of scaffold at a nitrogen of the heteroaryl group, e.g. imidazolyl, and R²³, R^{23a}, R²⁴, and R²⁵ are hydrogen, are synthesized according to Synthetic Scheme XXIII, by oxidation of the alcohol group of compound **(XIVa)** where with triisopropoxyaluminum and 2-butanone in toluene to give the compound of formula **(XIb)**.

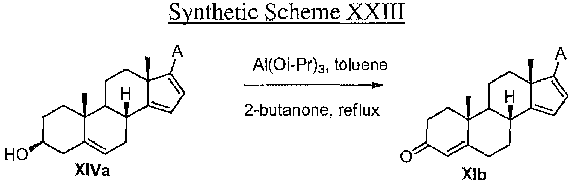

In certain embodiments, compounds of Formula **(IVB)** are synthesized according to Synthetic Scheme XXIV where both 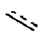 bonds are double bonds and R¹ is hydrogen or alkyl. 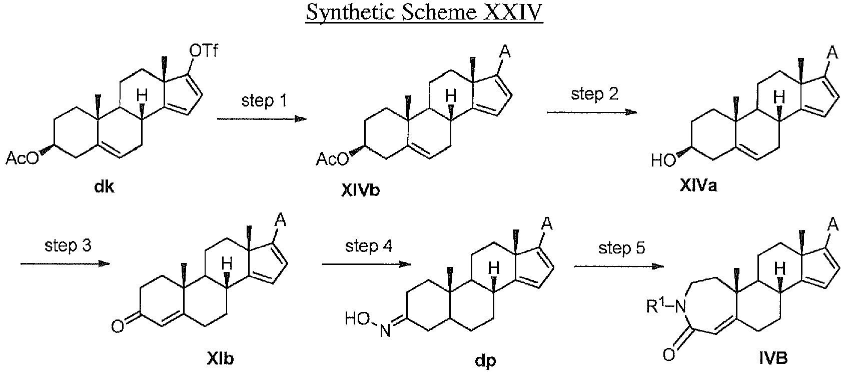 Compounds of structure of Formula **IVB** where both 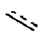 bonds are double bonds and R¹ is hydrogen or alkyl, are synthesized from triflate compound **(dk)** mentioned above. Suzuki coupling reaction on compound **(dk)** with A-B(ethyl)₂ or A-B(OH)₂ and 1,1'-bis(diphenylphosphino)ferrocencepalladium(II) dichloromethane complex in 1,4-dioxane-water in the presence of a base such as potassium carbonate followed by acidification yields compounds of formula **(XIVb)** (step 1). Hydrolysis the acetyl group of compound **(XIVb)** with sodium hydroxide in methanol-water yields the alcohol compound **(XIVa)** (step 2). Oxidation of the alcohol group of compound **(XIVa)** with triisopropoxyaluminum and 2-butanone in toluene to give the compound of formula **(XIb)** where R²³, R^{23a}, R²⁴, and R²⁵ are hydrogen (step 3). Treatment of this compound **(XIb)** with hydroxylamine hydrochloride in ethanol in the presence of pyridine or triethylamine yields oxime **(dp)** (step 4). Beckmann rearrangement reaction on the oxime **(dp)** with thionyl chloride in the presence of pyridine in tetrahydrofuran yields a ring expansion compound of formula (**IVB**) where R¹ is hydrogen (step 5). The compound of Formual **IVB** where R¹ is hydrogen can be treated with R¹-X (where X is halo) to yield another compound of Formula **IVB** where R¹ is alkyl.

In certain embodiments, compounds of Formula **(IVB)** are synthesized according to Synthetic Scheme XXV where A is attached to the rest of scaffold at a nitrogen of the heteroaryl group, e.g. an imidazolyl, both 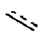 bonds are double bonds, and R¹ is hydrogen or alkyl. 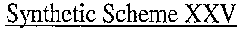 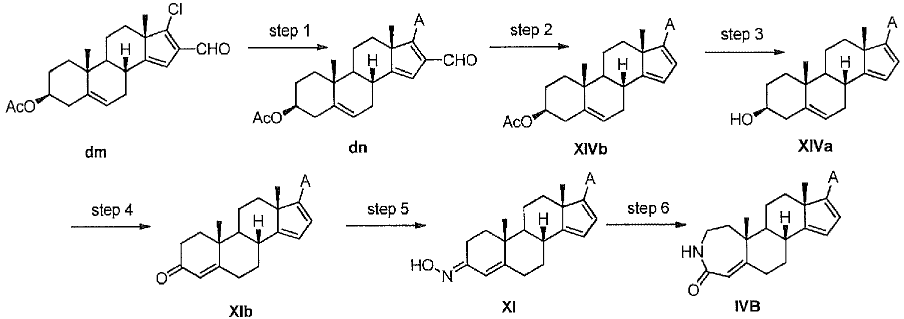 Compounds having the structure of Formula **(IVB)** are synthesized from unsaturated vinyl chloro aldehyde compounds **(dm)** synthesis of which is mentioned above. Displacement of the chloro group of compound **(dm)** with imidazole in organic solvent such as DMF in the presence of a base such as potassium carbonate yields the imidazolyl derivative compound **(dn)** (step 1). Treatment of compounds **(dn)** with 10% palladium on carbon in benzonitrile at elevated temperature removes the formyl group to give compound (**XIVb**) (step 2). Hydrolysis the acetyl group with potassium carbonate or potassium hydroxide in methanol water yields the alcohol compound of Formula **XIVa** (step 3). Oxidation of the alcohol group with triisopropoxyaluminum and 2-butanone in toluene to give the compound of formula (**XIb**) where R²³, R^{23a}, R²⁴, and R²⁵ are hydrogen (step 4). Treatment of this compound (**XIb**) with hydroxylamine hydrochloride in ethanol in the presence of pyridine or triethylamine yields oxime (**XI** where T is C=N-OH and the bonds between carbons 1 and 2 and carbons 6 and 7 are single and the bond between carbons 14 and 15 is double) (step 5). Beckmann rearrangement reaction on the oxime (**XI**) with thionyl chloride in the presence of pyridine in tetrahydrofuran yields a ring expansion compound of formula (**IVB**) where A is attached to the rest of scaffold at a nitrogen of the heteroaryl group, e.g. an imidazolyl, both 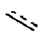 bonds are double bonds, and R¹ is hydrogen (step 6). The compound of Formual **IVB** where R¹ is hydrogen can be treated with R¹-X (where X is halo) to yield another compound of Formula **IVB** where R¹ is alkyl.

In certain embodiments, compounds of Formula (**III**) are synthesized according to Synthetic Scheme XXVI where each 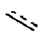 is a double bond; C(R^{7a})'''''Q²'''''Q³ is C=CH-CH₂; R² and R³ are hydrogen; and K is C(O), L is CH₂, and M is CH₂CH₂ or K is CH₂, L is C(O) and M is CH₂CH₂. 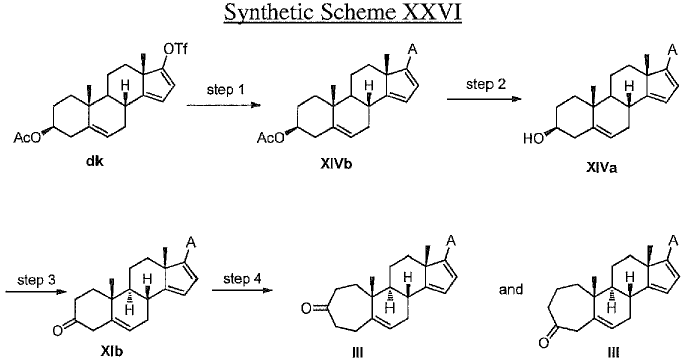 Compounds of structure of Formula **III** are synthesized from triflate compound **(dk)** mentioned above. Suzuki coupling reaction on compound **(dk)** with A-B(ethyl)₂ or A-B(OH)₂, and 1,1'-bis(diphenylphosphino)ferrocencepalladium(II) dichloromethane complex in 1,4-dioxane-water in the presence of a base such as potassium carbonate followed by acidification yields compounds of formula (**XIVb**) (step 1). Hydrolysis the acetyl group of compound (**XIVb**) with sodium hydroxide in methanol-water yields the alcohol compound (**XIVa**) (step 2). Oxidation of this compound with oxalyl dichloride and dimethylsulfoxide in methylene chloride followed by the addition of diisopropylethylamine gives the keto compound of Formula **XIb** (step 3). Treatment of this compound with greater than 1 equivalent such as 5 equivalent amount of ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds (**III**) (step 4).

In certain embodiments, compounds of the following structures where A is attached to the rest of scaffold at a nitrogen of the heteroaryl group, e.g. an imidazolyl are synthesized according to Synthetic Scheme XXVII. 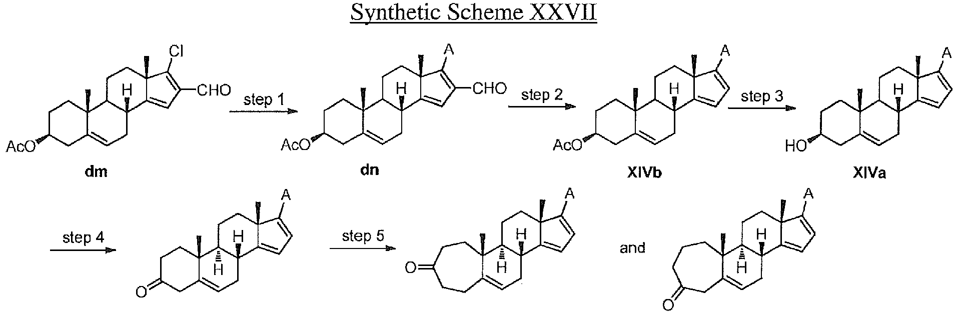 Displacement of the chloro group of compound **(dm)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the imidazolyl derivative compound **(dn)** (step 1). Treatment of compounds **(dn)** with 10% palladium on carbon in benzonitrile at elevated temperature removes the formyl group to give compound ((**XIVb**)) (step 2). Hydrolysis the acetyl group of compound (**XIVb**) with potassium carbonate or potassium hydroxide and the like in methanol water yields the alcohol compound (**XIVa**) (step 3). Oxidation of the alcohol compound with oxalyl dichloride and dimethylsulfoxide in methylene chloride followed by the addition of diisopropylethylamine gives the keto compound (step 4). Treatment of this keto with greater than 1 equivalent such as 5 equivalent amount of ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds (step 5).

In certain embodiments, compounds of the following structures are synthesized according to Synthetic Scheme XXVIII. 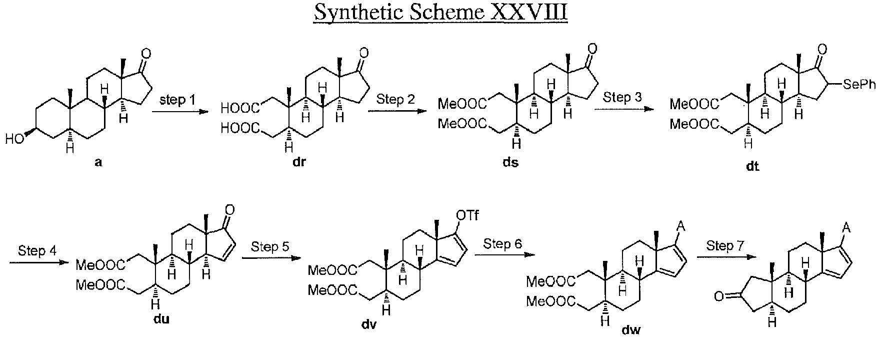 Epiandrosterone **(a)** is commericially available. Oxidation of compound (a) with chromium (VI) oxide in the presence of sulfuric acid and acetic acid -water yields the diacid compound **(dr)** (step 1). The diacid compound **(dr)** prepared as above is esterified by first converting it to acid chloride with the treatment of thionyl chloride and the treated with in methanol to give the methyl ester compound **(ds)** (step 2). Treatment of **(ds)** with 1.1 equivalent of lithium diisopropylamide in THF at -78 °C followed by the addition of phenyl selenium bromide yielded compound **(dt)** (step 3) Oxidation of compound **(dt)** with 1.1 equivalent of m-chloroperbenzoic acid in carbon tetrachloride at -40 °C followed by the addition of diethylamine at reflux gives the di unstaturated keto compound **(du)** (step 4). Conversion of compound **(du)** to triflate compound **(dv)** is achieved by using condition involving the enol triflate formation by the use of triflic anhydride (trifluoromethanesulfonic anhydride) in the presence of base such as triethylamine and the like (step 5). Suzuki coupling reaction on compounds **(dv)** with A-B(ethyl)₂ or A-B(OH)₂, and (Ph₃P)₂PdCl₂ in THF - water or dioxane - water in the presence of a base such as sodium carbonate followed by acidification yields compound **(dw)** (step 6). Preparation of the final compound from compound **(dw)** is achieved by first hydrolysis of the ester to the corresponding diacid followed by treatment of it with sodium acetate in acetic anhydride and subsequence heating (step 7).

In certain embodiments, compounds of the following Formula are synthesized according to Synthetic Scheme XXIX. 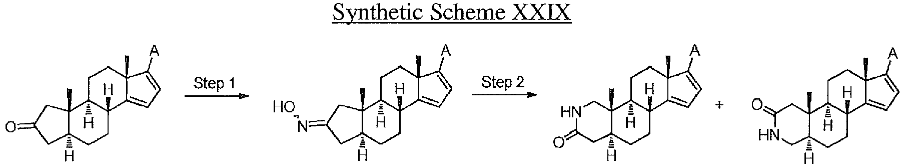 Treatment of the first intermediate with hydroxylamine hydrochloride in ethanol in the presence of pyridine or triethylamine yields an oxime (step 1). Beckmann rearrangement reaction on the oxime with thionyl chloride in the presence of pyridine in tetrahydrofuran yields a mixture of two ring expansion compounds (step 2).

In certain embodiments, compounds of Formula (**IIIF-1**) and (**IIIF-2**) where 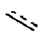 is a single bond and R¹ is hydrogen or alkyl are synthesized according to Synthetic Scheme XXX. 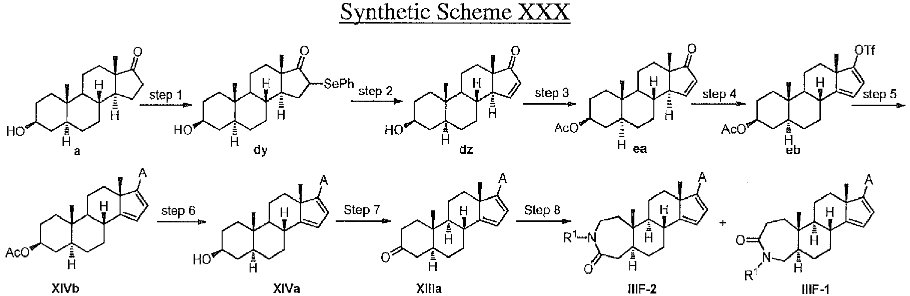 Compounds structure of Formula (**IIIF-1**) and (**IIIF-2**) where 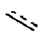 is a single bond and R¹ is hydrogen or alkyl are synthesized from commercially available starting material epiandrosterone **(a).** Treatment of **(a)** with lithium diisopropylamide in THF at -78 °C followed by the addition of phenyl selenium bromide yielded compound **(dy)** (step 1) Oxidation of compound **(dy)** with m-chloroperbenzoic acid in carbon tetrachloride at -40 °C followed by the addition of diethylamine at reflux gives the di unstaturated keto compound **(dz)** (step 2). Treatment of compound **(dz)** with acetic anhydride in pyridine or in methylene chloride in the presence of a base such as sodium acetate or dimethylaminopyridine gives the keto-acetyl compound **(ea)** (step 3). Conversion of compound **(ea)** to triflate compound **(eb)** is achieved by using condition involving the enol triflate formation by the use of triflic anhydride (trifluoromethanesulfonic anhydride) in the presence of base such as triethylamine and the like (step 4). Suzuki coupling reaction on compound **(eb)** with A-B(ethyl)₂ or A-B(OH)₂, and 1,1'-bis(diphenylphosphino)ferrocencepalladium(II) dichloromethane complex in 1,4-dioxane-water in the presence of a base such as potassium carbonate followed by acidification yields compound **(XIVb)** (step 5). Hydrolysis of the acetyl group with potassium carbonate in methanol water yields the alcohol compound **(XIVa)** (step 6). Oxidation of the alcohol group with triisopropoxyaluminum and 2-butanone in toluene gives keto compound of Formula **XIIIA** (step 7). Treatment of compound of Formula **XIIIA** with hydroxylamine hydrochloride in ethanol in the presence of pyridine or triethylamine yields oxime followed by Beckmann rearrangement reaction on the oxime with thionyl chloride in the presence of pyridine in tetrahydrofuran yields a mixture of ring expansion compound **(IIIF-1)** and compound **(IIIF-2)** where R¹ is hydrogen. Compounds of Formula **(IIIF-1)** and **(IIIF-2)** where R¹ is alkyl can be prepared by alkylation where R¹ is hydrogen with R¹-X (where X is halo).

In certain embodiments, compounds of Formula **(IIIF-1)** and **(IIIF-2)** where A is attached to the rest of scaffold at a nitrogen of the heteroaryl, e.g. an imidazolyl, and 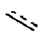 is a single bond and R¹ is hydrogen or alkyl, are synthesized according to Synthetic Scheme XXXI. 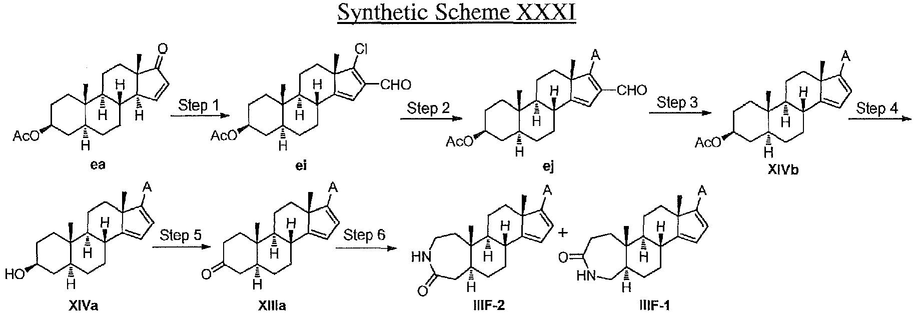 Compounds having the structure of Formula **(IIIF-1)** and **(IIIF-2),** where A is attached to the rest of scaffold at a nitrogen of the heteroaryl, e.g. an imidazolyl, and 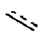 is a single bond and R¹ is hydrogen or alkyl, are synthesized from unsaturated keto compound **(ea)** synthesis of which is mentioned above. Formylation of the keto-acetyl compound **(ea)** with Vilsmeier-Haack reagents of DMF and phosphoryl chloride in dichloromethane gives the unsaturated vinyl chloro aldehyde compounds **(ei)** (step 1). Displacement of the chloro group of compound **(ei)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(ej)** (step 2). Treatment of compounds **(ej)** with 10% palladium on carbon in benzonitrile at elevated temperature removes the formyl group to give compound **(XIVb)** (step 3). Hydrolysis of the acetyl group with potassium carbonate in methanol water yields the alcohol compound **(XIVa)** (step 4). Oxidation of the alcohol group of compound **(XIVa)** with triisopropoxyaluminum and 2-butanone in toluene gives keto product compound **(XIIIa)** (step 5). Treatment of compound **(XIIIa)** with hydroxylamine hydrochloride in ethanol in the presence of pyridine or triethylamine yields oxime followed by Beckmann rearrangement reaction on the oxime with thionyl chloride in the presence of pyridine in tetrahydrofuran yields a mixture of ring expansion compound **(IIIF-1)** and compound **(IIIF-2)** (step 6).

In certain embodiments, compounds of Formula **(IJ)** where d is 2, E is CH₂, and e is 2 are synthesized according to Synthetic Scheme XXXII. 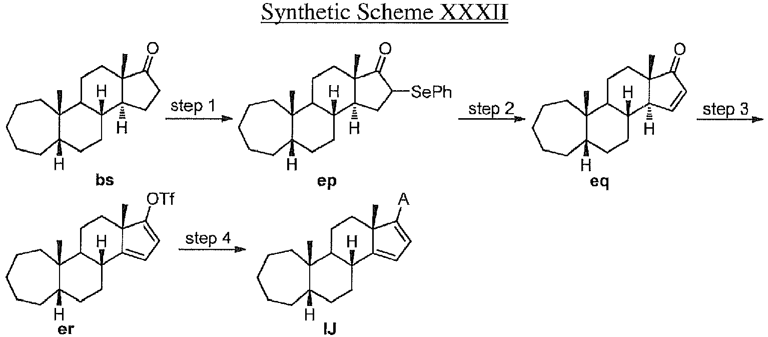 Compounds structure of Formula **IJ** where d is 2, E is CH₂, and e is 2 are synthesized from compound **(bs)** synthesis of which is mentioned above. Treatment of **(bs)** with lithium diisopropylamide in THF at -78 °C followed by the addition of phenyl selenium bromide yielded compound **(ep)** (step 1) Oxidation of compound **(ep)** with m-chloroperbenzoic acid in carbon tetrachloride low temperature such as at -40 °C followed by the addition of diethylamine at reflux gives the di unstaturated keto compound **(eq)** (step 2). Conversion of compound **(eq)** to triflate compound **(er)** is achieved by using condition involving the enol triflate formation by the use of triflic anhydride (trifluoromethanesulfonic anhydride) in the presence of base such as triethylamine and the like (step 3). Suzuki coupling reaction on compound **(er)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields a compound of formula **(IJ)** where where d is 2, E is CH₂, and e is 2 (step 4).

In certain embodiments, compounds of Formula **(IJ)** where A is attached to the rest of scaffold at a nitrogen of the heteroaryl, e.g. an imidazolyl, d is 2, E is CH₂, and e is 2 are synthesized according to Synthetic Scheme XXXIII. 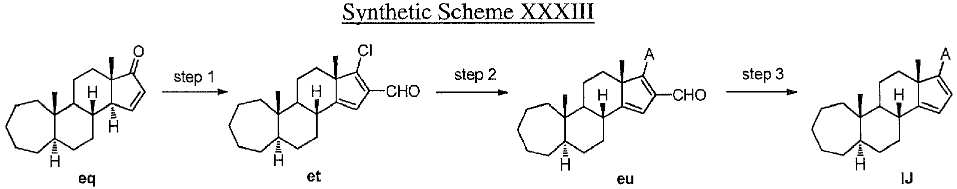 Compounds having the structure of Formula **(IJ)** where A is attached to the rest of scaffold at a nitrogen of the heteroaryl, e.g. an imidazolyl, d is 2, E is CH₂, and e is 2 are synthesized from unsaturated keto compound **(eq)** synthesis of which is mentioned above. Formylation of the unstaturated keto compound **(eq)** with Vilsmeier-Haack reagents of DMF and phosphoryl chloride in dichloromethane gives the unsaturated vinyl chloro aldehyde compounds (et) (step 1). Displacement of the chloro group of compound **(et)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(eu)** (step 2). Treatment of compounds **(eu)** with 10% palladium on carbon in benzonitrile at elevated temperature removes the formyl group to give compound **(ev)** of formula **(IJ)** where A is attached to the rest of scaffold at a nitrogen of the heteroaryl, e.g, an imidazolyl, d is 2, E is CH₂, and e is 2 (step 3).

In certain embodiments, compounds of Formula **(IJ)** where d is 2, E is CH₂, and e is 1 are synthesized according to Synthetic Scheme XXXIV. 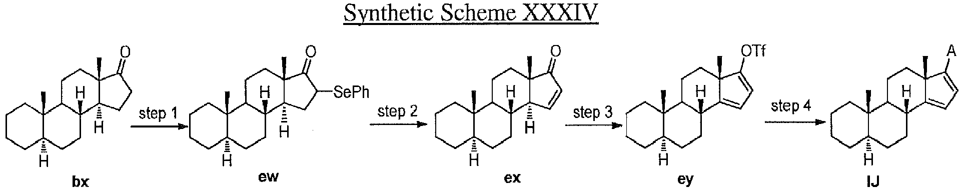 Compounds structure of Formula **IJ** where d is 2, E is CH₂, and e is 1 are synthesized from compound **(bx)** synthesis of which is mentioned above. Treatment of **(bx)** with lithium diisopropylamide in THF at -78 °C followed by the addition of phenyl selenium bromide yielded compound **(ew)** (step 1) Oxidation of compound **(ew)** with m-chloroperbenzoic acid in carbon tetrachloride low temperature such as at -40 °C followed by the addition of diethylamine at reflux gives the di unstaturated keto compound **(ex)** (step 2). Conversion of compound **(ex)** to triflate compound **(ey)** is achieved by using condition involving the enol triflate formation by the use of triflic anhydride (trifluoromethanesulfonic anhydride) in the presence of base such as triethylamine and the like (step 3). Suzuki coupling reaction on compound **(ey)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields compound **(ez)** of formula **(IJ)** where d is 2, E is CH₂, and e is 1 (step 4).

In certain embodiments, compounds of Formula **(IJ)** where A is attached to the rest of scaffold at a nitrogen of the heteroaryl, e.g. an imidazolyl, a d is 2, E is CH₂, and e is 1 are synthesized according to Synthetic Scheme XXXV. 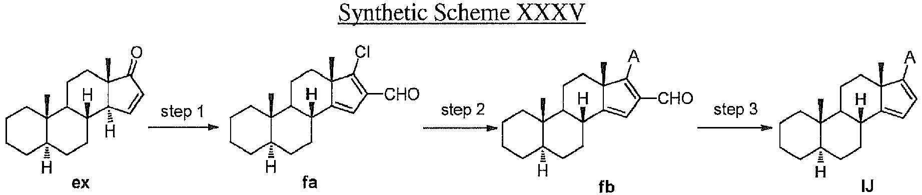 Compounds having the structure of Formula **(IJ)** where A is attached to the rest of scaffold at a nitrogen of the heteroaryl, e.g. an imidazolyl, a d is 2, E is CH₂, and e is 1 are synthesized from unsaturated keto compound **(ex)** synthesis of which is mentioned above. Formylation of the unstaturated keto compound **(ex)** with Vilsmeier-Haack reagents of DMF and phosphoryl chloride in dichloromethane gives the unsaturated vinyl chloro aldehyde compounds **(fa)** (step 1). Displacement of the chloro group of compound **(fa)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(fb)** (step 2). Treatment of compounds **(fb)** with 10% palladium on carbon in benzonitrile at elevated temperature removes the formyl group to give compound **(fc)** of formula **(IJ)** where A is attached to the rest of scaffold at a nitrogen of the heteroaryl, e.g. an imidazolyl, a d is 2, E is CH₂, and e is 1 (step 3).

In certain embodiments, compounds of Formula **(IH)** where d is 2, E is CH₂, e is 1, and Q² is CH(OH) are synthesized according to Synthetic Scheme XXXVI. 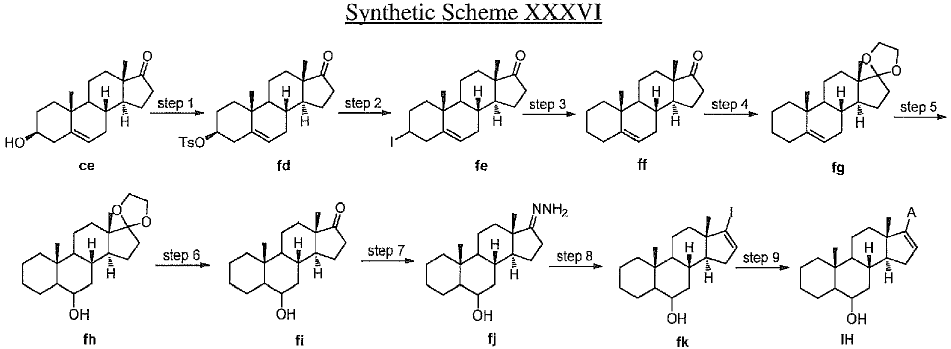 Compounds of Formula **(IH)** where d is 2, E is CH₂, e is 1, and Q² is CH(OH) are synthesized from commercially available starting material dehydro epiandrosterone (ce). Treatment of **(ce)** with p-toluenesulfonyl in pyridine gives tosylate compound **(fd)** (step 1). Displacement of the tosyl group of **(fd)** with sodium iodide in acetone yields the iodo compound **(fe)** (step 2). Treatment of compound **(fe)** with zinc in acetic acid removes the iodine to provide compound **(ff)** (step 3). Ketalization of the keto group of compound **(ff)** with 1,2 ethane-1,2-diol in triethoxymethane in the presence of p-tolenesulfonic acid gives the cyclic ketal compound **(fg)** (step 4). Treatment of compound (fg) with borane in THF followed by reaction with sodium hydroxide and subsequent oxidation with hydrogen peroxide yields the hydroxyl compound **(fh)** (step 5). De-ketalization of compound **(fh)** is achieved by treatment of it in methanol in the presence of hydrochloric acid to give compound **(fi)** (step 6). Reacting compound **(fi)** with hydrazine in ethanol gives the hydrazone compound **(fj)** (step 7). Treatment of **(fj)** with iodine solution in THF yields the iodo compound **(fk)** (step 8) Suzuki coupling reaction on compound **(fk)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields compound **(IH)** where d is 2, E is CH₂, e is 1, and Q² is CH(OH) (step 9).

In certain embodiments, compounds of Formula **(IH)** where d is 2, E is CH₂, e is 1, anfd Q² is C(O) are synthesized according to Synthetic Scheme XXXVII. 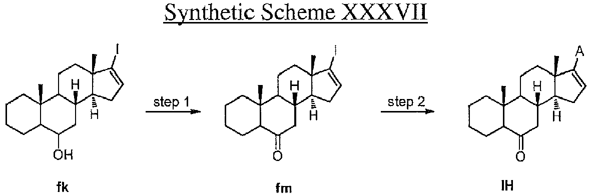 Compounds having the structure Formula **(IH)** where d is 2, E is CH₂, e is 1, anfd Q² is C(O) are synthesized from unsaturated iodo compound **(fk)** synthesis of which is mentioned above. Oxidation of the alcohol group of compound **(fk)** with pyridinium chlorochromate in dichloromethane in the presence of barium carbonate, give the keto compound **(fm)** (step 1). Coupling reaction on compound **(fk)** with A-B(ethyl)₂ or A-B(OH)₂, and 1,1'-bis(diphenylphosphino)ferrocencepalladium(II) dichloromethane complex in 1,4-dioxane-water in the presence of a base such as potassium carbonate followed by acidification yields compound **(IH)** where d is 2, E is CH₂, e is 1, anfd Q² is C(O) (step 2).

In certain embodiments, compounds of Formula **(VA)** where R² and R³ are hydrogen are synthesized according to Synthetic Scheme XXXVIII. 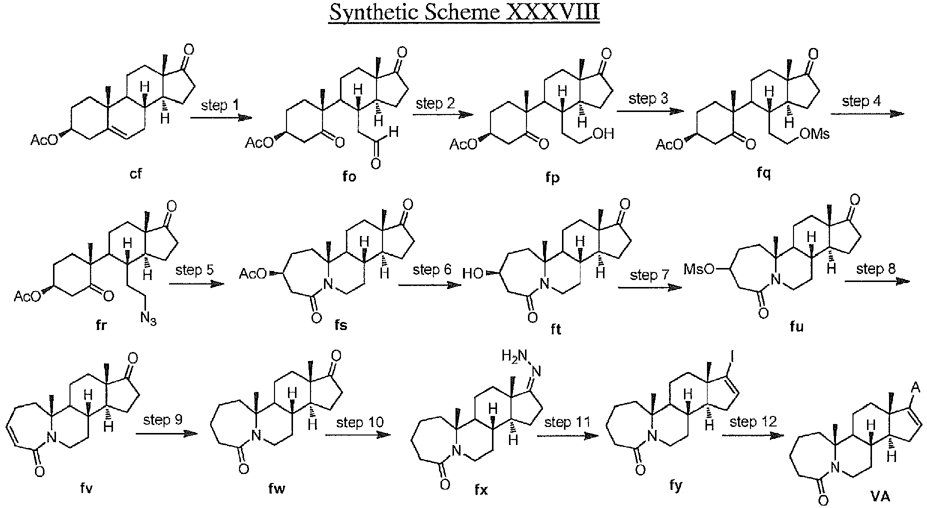 Compounds of Formula (VA) where R² and R³ are hydrogen are synthesized from dehydro epiandrosterone acetate **(cf),** synthesis of which has been described above. Ozonolysis of **(cf)** by passing ozone to a solution of compound **(cf)** in dichloromethane-methanol at -78 °C followed by reaction with zinc in acetic acid provides the ring opened compound **(fo)** (step 1).The aldehyde group of compound **(fo)** in THF is reduced to the alcohol with NaBH(OAc)₃ at -60 °C and then followed by the addition of methanol to give compound **(fp)** (step 2). Compound **(fp)** is then converted to the mesylate by reacting with methanesulfonyl chloride in THF in the presence of a base such as triethylamine to give compound **(fq)** (step 3). Displacement of the mesylate of compound **(fq)** with sodium azide in DMF provides the compound **(fr)** (step 4). Treatment of compound **(fr)** with titanium (VI) chloride at low temperature gives the ring cyclized product compound **(fs)** (step 5). Treatment of a solution of **(fs)** in methanol-tetrahydrofuran with potassium hydroxide yields the alcohol compound **(ft)** (step 6). Reaction of compound **(ft)** in dichloromethane with methanesulfonyl chloride in the presence of pyridine at low temperature gives the mesylate compound **(fu)** (step 7). Reaction of compound **(fu)** in toluene with a base such DBU at elevated temperature gives unsaturated keto compound **(fv)** (step 8). Reduction of the double bond of compound **(fv)** is achieved by hydrogenation under hydrogen atmosphere in the presence of 10% palladium on carbon catalyst to yield compound **(fw)** (step 9). Reacting compound **(fw)** with hydrazine in ethanol gives the hydrazone compound **(fx)** (step 10). Treatment of **(fx)** with iodine solution in THF yields the iodo compound **(fy)** (step 11). Coupling reaction on compound **(fy)** with A-B(ethyl)₂ or A-B(OH)₂, and 1,1'-Bis(diphenylphosphino)ferrocencepalladium(II) dichloromethane complex (or the like) in 1,4-dioxane-water in the presence of a base such as potassium carbonate followed by acidification yields compound of formula **(VA)** where R² and R³ are hydrogen (step 9).

In certain embodiments, compounds of Formula **(VC)** and Formula **(VD)** where R² and R³ are hydrogen are synthesized according to Synthetic Scheme XXXIX 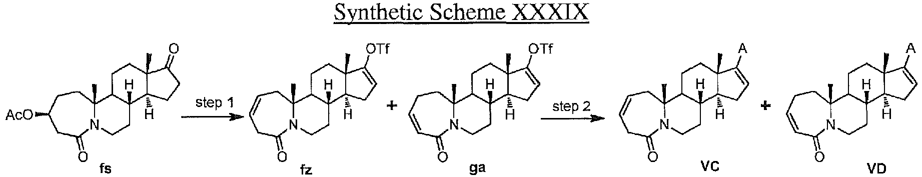 Compounds of Formula **(VC)** and Formula **(VD)** where R² and R³ are hydrogen are synthesized from compound **(fs),** synthesis of which has been described above. Treatment of compound **(fs)** with trifluoromethane sulfonic anhydride in dichloromethane at room temperature for a short period of time such as 10 minutes followed by the addition dropwise of one equivalent of triethylamine yields a mixture of compounds **(fz)** and **(ga)** of formula **(VC)** and of formula **(VD)** where R² and R³ are hydrogen. Coupling reaction on compounds **(fz)** and (ga) with A-B(ethyl)₂ or A-B(OH)₂, and 1,1'-Bis(diphenylphosphino)ferrocencepalladium(II) dichloromethane complex (or the like) in 1,4-dioxane-water in the presence of a base such as potassium carbonate followed by acidification yield compounds of formula **(VC)** and **(VD)** where R² and R³ are hydrogen (step 2).

In certain embodiments, compounds of Formula **(VIa)** where 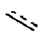 is a single bond, R¹ is alkyl, and R² and R³ are hydrogen are synthesized according to Synthetic Scheme XXXX. 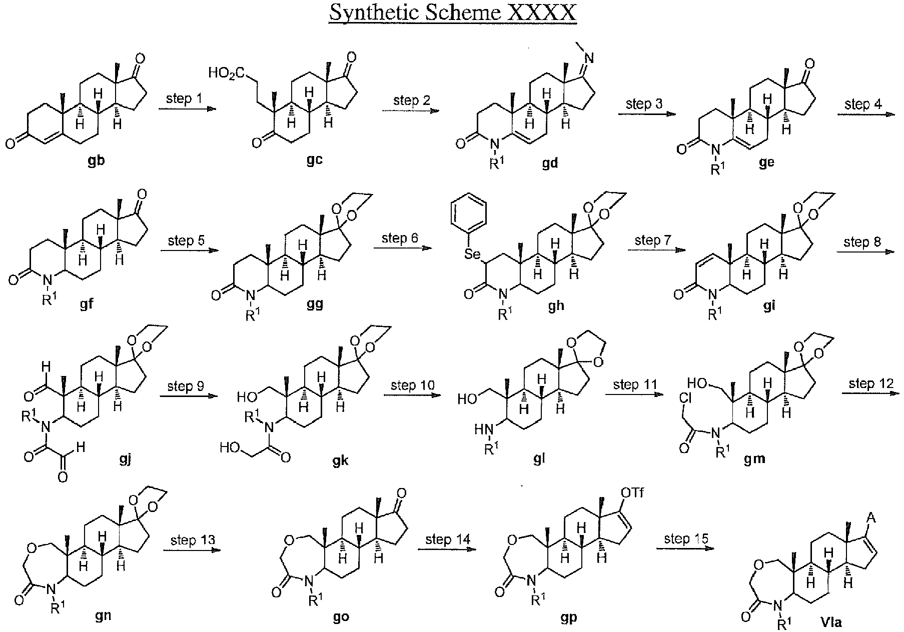 Compounds of structure of Formula **VIa** where 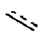 is a single bond, R¹ is alkyl, and R² and R³ are hydrogen are synthesized from commercially available starting material androsteronedione **(gb).** A suspension of **(gb)** in t-butanol and potassium carbonate in water at 80 °C was added with dropwise of potassium permanganate and sodium periodate. Reaction for a few hours gives the compound **(gc)** (step 1). Reaction of compound **(gc)** with R¹amine in ethanol in seasl tube at evaluated temperature such as 140 °C yields the imine ring cyclized compound **(gd)** (step 2) which is then subjected to hydrolysis with hydrochloric acid to give comound **(ge)** (step 3). Reduction of compound (ge) in hydrogen atmosphere with 10 % palladium on carbon catalyst gives compound **(gf)** (step 4). Ketalization of the keto group of compound **(gf)** with 1,2 ethane-1,2-diol in triethoxymethane in the presence of p-tolenesulfonic acid gives the cyclic ketal compound (gg) (step 5). Reacion of compound (gg) with lithium diisopropylamide in THF at -78 °C followed by the addition of phenyl selenium bromide yielded compound **(gh)** (step 6) Oxidation of compound **(gh)** with hydrogen peroxide yielded unstaturated amide compound **(gi)** (step 7). Ozonolysis of compound **(gi)** by passing ozone to a solution of compound **(gi)** in dichloromethane-methanol at -78 °C yields the aldehyde compound **(gj)** (step 8). Sodium borohydride reduction of **(gj)** in ethanol gives the diol compound **(gk)** (step 9). Hydrolysis of the amide of compound **(gk)** with sodium hydroxide in methanol provides compound **(gl)** (step 10).Acetylation of compound **(gl)** with 3-chloropropanoyl chloride in THF in the present of potassium carbonate gives compound **(gm)** (step 11). Treatment of compound **(gm)** with sodium hydride in THF at acetic anhydride at low temperature such as 0°C gives the ring cyclized compound **(gn)** (step 12). Deketalization of compound **(gn)** with dilute hydrochloric acid in THF-water at room temperature yields the keo compound **(go)** (step 13). Conversion of compound **(go)** to triflate compound **(gp)** is achieved by using condition involving the enol triflate formation by the use of triflic anhydride ( trifluoromethanesulfonic anhydride) in the presence of base such as triethylamine and the like (step 14). Suzuki coupling reaction on compound **(gp)** with A-B(ethyl)₂ or A-B(OH)₂, and (Ph₃P)₂PdCl₂ in 1,4-dioxane-water in the presence of a base such as potassium carbonate followed by acidification yields compounds of formula **(VIa)** where 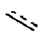 is a single bond, R¹ is alkyl, and R² and R³ are hydrogen (step 15).

In certain embodiments, compounds of Formula (VIIIa) where 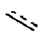 is a single bond and R¹ is hydrogen or alkyl, are synthesized according to Synthetic Scheme XXXXI. 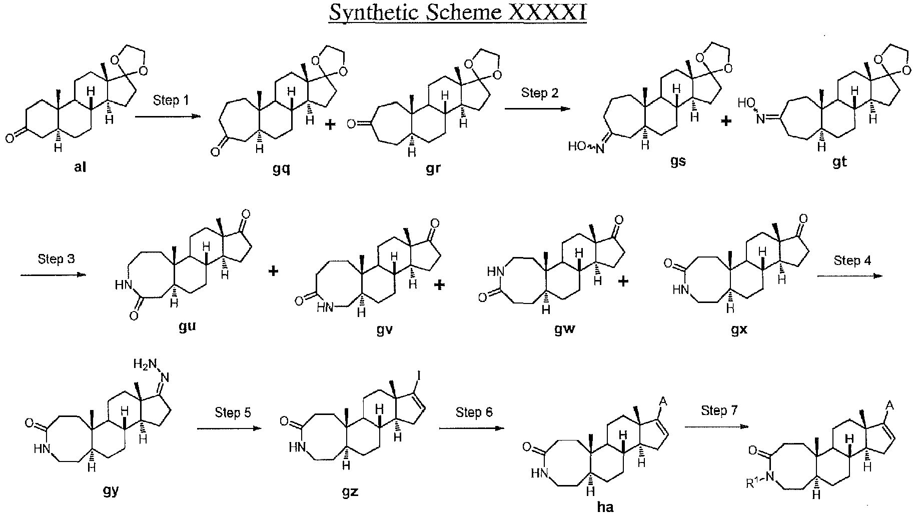 Compounds of structure of Formula (VIII) where 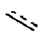 is a single bond and R¹ is hydrogen or alkyl, are synthesized from compound **(al)** the synthsis of which is described above. Treatment of this compound **(al)** with ethyl 2-diazoacetate and borontrifluoride etherate in dichloromethane yields the ring expansion products followed by the addition of dilute sodium hydroxide and then followed by acidification with dilute hydrochloric acid to give decarboxylated mixture of compounds **(gq)** and **(gr)** (step 1).Treatment of this mixture of compounds **(gq)** and **(gr)** with hydroxylamine hydrochloride in ethanol in the presence of pyridine or triethylamine yields a mixture of oximes **(gs)** and **(gt)** (step 2). Beckmann rearrangement reaction on these mixture of oximse **(gs)** and **(gt)** with thionyl chloride in the presence of pyridine in tetrahydrofuran yields a mixture of four ring expansion compounds **(gu), (gv), (gw)** and **(gx)** which are separated by chromatography.(step 3). Reacting compound **(gx)** with hydrazine in ethanol gives the hydrazone compound **(gy)** (step 4). Treatment of compound **(gy)** with iodine solution in THF yields the iodo compound **(gz)** (step 5) Suzuki coupling reaction on compound **(gz)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields compound **(ha)** of formula **(VIIIa)** where 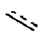 is a single bond and R¹ is hydrogen (step 6). Alkylation of compound (ha) with R¹-X (where X is halo) in the presence of sodium hydride yields compound of Formula **(VIII)** where 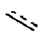 is a single bond and R¹ is alkyl (step 7).

In certain embodiments, compounds of Formula **(IIA)** where R² and R³ are hydrogen and 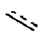 is a single bond are synthesized according to Synthetic Scheme XXXXII. 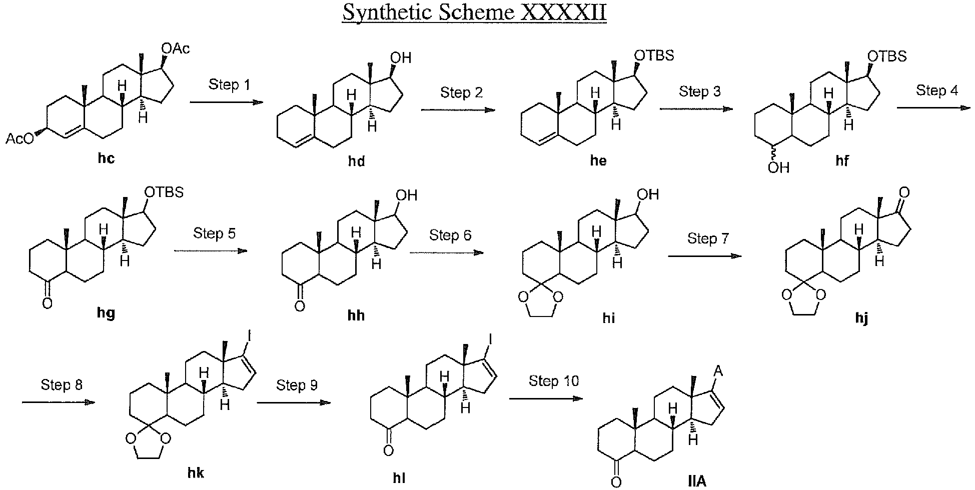 Compounds of Formula **(IIA)** where R² and R³ are hydrogen and 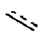 as a single bond are synthesized from compound **(he).** Treatment of **(he)** with sodium in liquid ammonia gives the compound **(hd)** (step 1). Reaction of compound **(hd)** with tri-tert-butylchlorosilane in the presence of imidazole in methylene yields the hydroxyl protected compound **(he)** (step 2). Hydroxylation of compound **(he)** with borane in THF followed with the addition of hydrogen peroxide and then sodium hydroxide yields the hydroxyl compound **(hf)** (step 3). Oxidation of the alcohol group of compound **(hf)** with pyridinium chlorochromate in dichloromethane gives the keto compound **(hg)** (step 4). Treatment of compound **(hg)** with dilute hydrochloric acid yields the hydroxyl compound **(hh)** (step 5). Ketalization of the keto group of compound **(hh)** with 1,2 ethane-1,2-diol in triethoxymethane in the presence of p-tolenesulfonic acid gives the cyclic ketal compound **(hi)** (step 6). Oxidation of the alcohol group of compound **(hi)** with pyridinium chlorochromate in dichloromethane in the presence of barium carbonate, give the keto compound **(hj)** (step 7). Reacting compound **(4j)** with hydrazine in ethanol gives the hydrazone compound which is treated with iodine solution in THF yields the iodo compound **(hk)** (step 8). Treatment of compound **(hk)** with dilute hydrochloric acid yields the keto compound **(hl)** (step 9). Suzuki coupling reaction on compound **(hl)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields compound of formula **(IIA)** where R² and R³ are hydrogen and 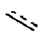 as a single bond (step 10).

In certain embodiments, compounds of Formula **(IIB)** where R² and R³ are hydrogen and 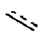 is a single bond are synthesized according to Synthetic Scheme XXXXIII. 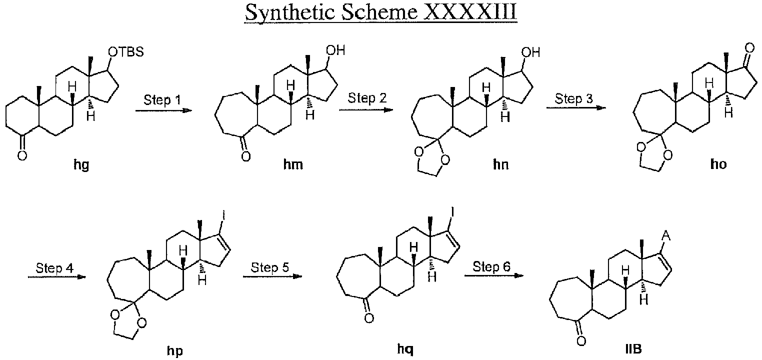 Compounds of Formula **(IIB)** where R² and R³ are hydrogen and 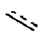 a single bond are synthesized from the keto compound **(hg),** the synthesis of which is described above. Treatment of this compound **(hg)** with diazomethane in dichloromethane followed by the addition of aluminium trichloride yields the ring expansion product compounds **(hm)** (step 1). Ketalization of the keto group of compound **(hm)** with 1,2 ethane-1,2-diol in triethoxymethane in the presence of p-tolenesulfonic acid gives the cyclic ketal compound **(hn)** (step 2). Oxidation of the alcohol group of compound **(hn)** with pyridinium chlorochromate in dichloromethane in the presence of barium carbonate, give the keto compound **(ho)** (step 3). Reacting compound **(ho)** with hydrazine in ethanol gives the hydrazone compound which is treated with iodine solution in THF yields the iodo compound **(hp)** (step 4). Treatment of compound **(hp)** with dilute hydrochloric acid yields the keto compound **(hq)** (step 5). Suzuki coupling reaction on compound **(hq)** with A-B(ethyl)₂ or A-B(OH)₂, and 1,1'-Bis(diphenylphosphino)ferrocencepalladium(II) dichloromethane complex in 1,4-dioxane-water in the presence of a base such as potassium carbonate followed by acidification yields compound of formula **(IIB)** where R² and R³ are hydrogen and 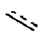 as a single bond (step 16).

In certain embodiments, compound of Formula **(IH)** where A is attached to the rest of scaffold at a nitrogen of the heteroaryl, e.g. an imidazolyl, d is 2, E is CH₂, e is 1, and Q² is CH(OH) or C(O) are synthesized according to Synthetic Scheme XXXXIV. 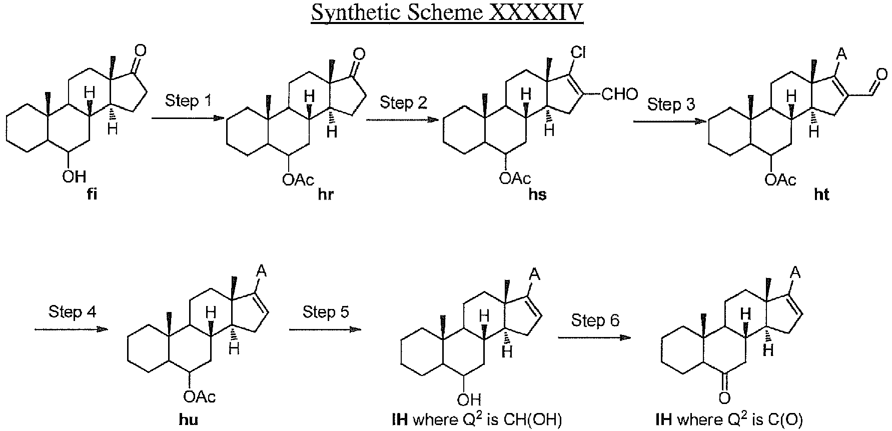 Compound having the structure of Formula **(IH)** where A is attached to the rest of scaffold at a nitrogen of the heteroaryl, e.g. an imidazolyl, d is 2, E is CH₂, e is 1, and Q² is CH(OH) or C(O) is synthesized from compound (fi) which is synthesized by a route described above. Treatment of **(fi)** with acetic anhydride in methylene chloride in the presence of a base such as dimethylaminopyridine gives the keto-acetyl compound (hr) (step 1). Formylation of the keto compound **(hr)** with Vilsmeier-Haack reagents of DMF and phosphoryl chloride in dichloromethane gives the vinyl chloro aldehyde compound **(hs)** (step 2).Displacement of the chloro group of compound **(hs)** with the heteroaryl A-H (where A-H is a heteroaryl group optionally substituted with 1, 2, 3, or 4 R⁴ groups and which heteroaryl comprises an NH atom; e.g. imidazole) in organic solvent such as DMF in the presence of a base such as potassium carbonate yields the derivative compound **(ht)** (step 3). Treatment of compound **(ht)** with palladium on carbon in benzonitrile at elevated temperature removes the formyl group to give compound **(hu)** (step 4). Hydrolysis of the acetyl group of (ht) with sodium hydroxide in methanol water yields the compound of formula **(IH)** where A is attached to the rest of scaffold at a nitrogen of the heteroaryl, e.g. an imidazolyl, d is 2, E is CH₂, e is 1, and Q² is CH(OH) (step 5). Oxidation of the alcohol group of this compound with triisopropoxyaluminum and 2-butanone in toluene gives keto product compound of formula **(IH)** where A is attached to the rest of scaffold at a nitrogen of the heteroaryl, e.g. an imidazolyl, d is 2, E is CH₂, e is 1, and Q² is C(O) (step 6).

In certain embodiments, compound of Formula **(IX)** where R²⁴ and R^{24a} are hydrogen and R²⁰ is arylcarbonyloxy, heterocycloalkylcarbonyloxy, heteroarylcarbonyloxy, -OC(O)NR²¹R^{21a}, or -OC(O)-alkylene-NHR^{21a} are synthesized according to Synthetic Scheme XXXXV. In addition, a Compound of Formula XIV where 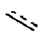 is a double bond, R³⁰ is alkylcarbonyl, R^{7a} , R²³, R²⁴, and R^{24a} are hydrogen can be prepared ccording to this scheme. 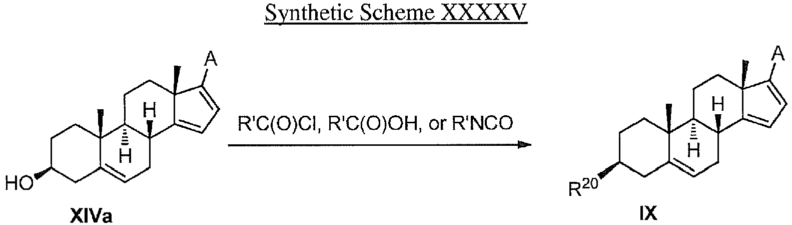 The compound of Formula XIVa, prepared using procedures described above is treated with an intermediate of formula R'C(O)Cl in the presence of a base such as TEA or DIPEA, with an intermediate of formula R'C(O)OH in the presence of amide coupling agents such as DCC and/or DMAP, or with an intermediate of formula R^{21a}N=C=O in the presence of a base such as DIPEA or TEA, in the presence of a solvent such as DMF and/or THF to yield a compound of Formula IX. R' is alkyl, aryl, heterocycloalkyl, heteroaryl, or alkylene-NR²¹R^{21a}.

In certain embodiments, compound of Formula **(X)** are synthesized according to Synthetic Scheme XXXXVI where R²² is hydroxy, R^{22a} is alkyl, R^{22b} is hydrogen, the bond between carbon 14 and 15 is a single bond, and the bond between carbons 5 and 6 is a double bond. 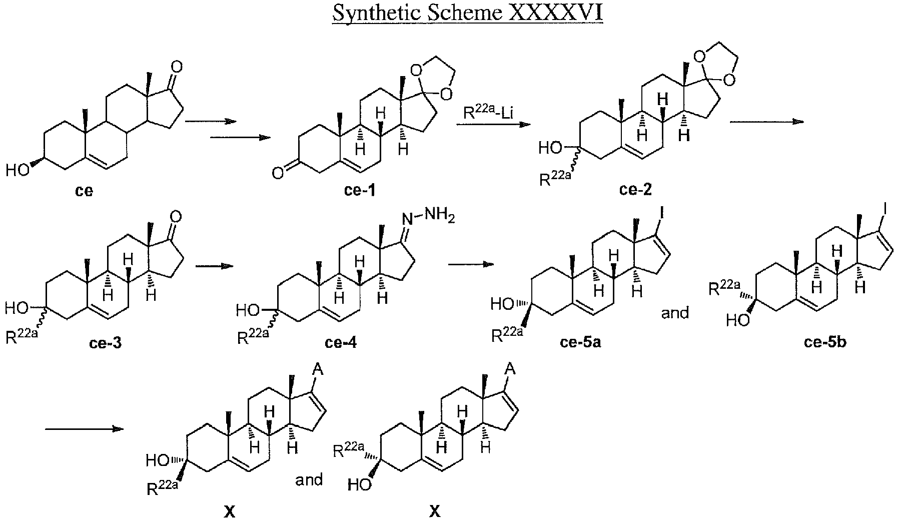 Starting with ce and using conditions similar to those in Scheme V, Steps 1 and 2, ce is converted into **ce-1.** The intermediate **ce-1** is then treated with R^{22a}-Li where R^{22a} is alkyl in a solvent such as THF and/or ether at a temperature of about 0 °C to yield the intermediate of formula **ce-2** and the mixture is quenched and worked up using standard extraction conditions and chromatography. The intermediate **ce-2** is then treated with aqueous HCl and acetone in a solvent such as THF at room temperature. The mixture is then neutralized and worked up using standard conditions, and the product **ce-3** is purified using chromatography. The intermediate **ce-3** is then treated with hydrazine in a solvent such as ethanol and stirred at a temperature of about 80 °C to yield **ce-4.** The intermediate **ce-4** is then treated with I₂ in ther presence of a base such as triethylamine in a solvent such as THF. The reaction was quenched and worked up using standard conditions and the products **ce-5a** and **ce-5b** were obtained after purification using chromatography. Compounds of Formula X (where R²² is hydroxy, R^{22a} is alkyl, R^{22b} is hydrogen, the bond between carbon 14 and 15 is a single bond, and the bond between carbons 5 and 6 is a double bond) were then prepared using Suzuki coupling as described in some of the Schemes above.

In certain embodiments, compound of Formula **(X)** are synthesized according to Synthetic Scheme XXXXVI where R²² is hydroxy, R^{22a} is alkyl, R^{22b} is hydrogen, the bond between carbon 14 and 15 is a single bond, and the bond between carbons 5 and 6 is a double bond. 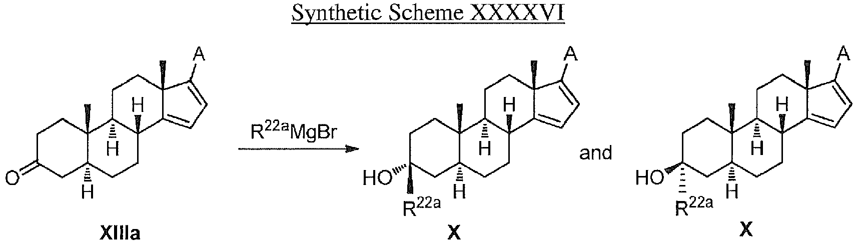 A compound of Formula XIIIA, prepared using procedures described in Scheme XXX, is treated with R²²MgBr in a solvent such as THF at room temperature. The reaction was quenched and worked up using standard procedures. The solution was purified using chromatography to yield compounds of Formula X.

In certain embodiments, additional compounds of Formula **(X)** (where R²² is hydroxy and R^{22a} is alkyl, R^{22b} is hydrogen, the bonds between carbon 14 and 15 and between carbons 5 and 6 are single bonds) are synthesized using procedures similar to those in Synthetic Scheme XXXXVI by replacing the Compound of Formula XIIIa in Scheme XXXXVI with a Compound of Formula XIIIb 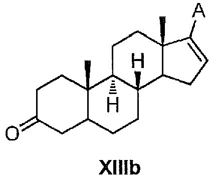 which can be prepared according to Scheme VI or XV.

In certain embodiments, compound of Formula **(X)** are synthesized according to Synthetic Scheme XXXXVII where R²² and R^{22a} are fluoro, R^{22b} is hydrogen, the bond between carbon 14 and 15 is a double bond, and the bond between carbons 5 and 6 is a single bond. 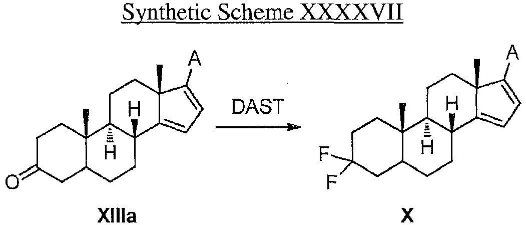 The Compound of Formula XIII where the bond between carbons 14 and 15 is a double bond and R^{7a} , R²³, R^{23a}, R²⁴, and R^{24a} are hydrogen is preapared using conditions described in Scheme XXX. The Compound of Formula XIIIa can be treated with DAST in a solvent such as DCM at room temperature. The reaction mixture is quenched and worked up and the Compound of Formula **X** (where R²² and R^{22a} are fluoro, R^{22b} is hydrogen, the bond between carbon 14 and 15 is a double bond, and the bond between carbons 5 and 6 is a single bond) is purified by chromatography.

In certain embodiments, additional compounds of Formula **(X)** (where R²² and R^{22a} are fluoro, R^{22b} is hydrogen, the bonds between carbon 14 and 15 and between carbons 5 and 6 are single bonds) are synthesized using procedures similar to those in Synthetic Scheme XXXXVII by replacing the Compound of Formula XIIIa in Scheme XXXXVII with a Compound of Formula XIIIb 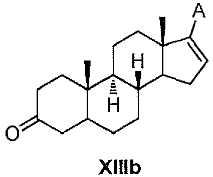 which can be prepared according to Scheme VI or XV.

In certain embodiments, compound of Formula **(XIg)** is synthesized according to Synthetic Scheme XXXXVIII where R²³ and R^{23a} form C=CH₂, and R²⁴ and R²⁵ are hydrogen. 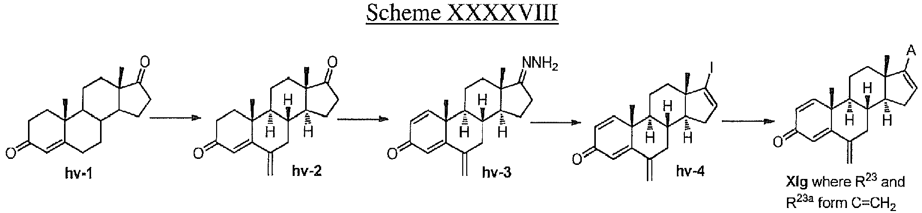 The intermediate of formula hv-1 is treated with triethylorthoformate (30 mL) and p-toluenesulphonic acid and heated at about 45 °C for about one hour. *N*-Methylaniline and aqueous formaldehyde were added to yield the intermediate of formula **hv-2**. After the completion of the reaction, the mixture was cooled to about 10 °C and acidified with conc. HCl. The mixture was then worked up using standard conditions and the product was purified using chromatography. The intermediate of formula **hv-2** is then treated with hydrazine hydrate in a solvent such as ehtanol at about 0 °C. The mixture was then worked up using standard conditions and the product was purified using chromatography to yield the intermediate of formula **hv-3**. Hv-3 is then treated with I₂ in the presence of a base such as TEA in a solvent such as DCM. The mixture was then worked up using standard conditions and the product was purified using chromatography to yield the intermediate of formula hv-4. **Hv-4** is then treated with a boronic acid using procedures described above to yield the compound of formula XIg where R²³ and R^{23a} form C=CH₂, and R²⁴ and R²⁵ are hydrogen.

In certain embodiments, compound of Formula **(XIg)** is synthesized according to Synthetic Scheme XXXXIX where R²³ and R^{23a} form cyclopropyl, and R²⁴ and R²⁵ are hydrogen. 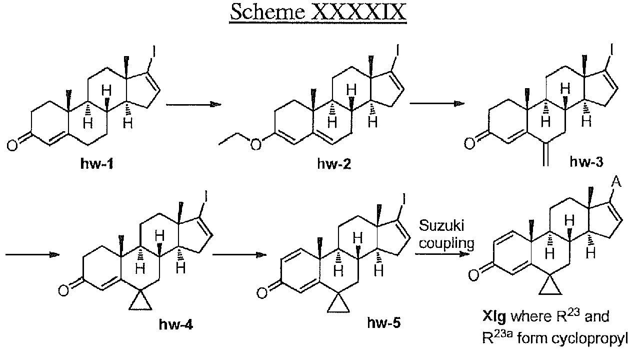 The intermediate of formula **hw-1** is treated with triethoxymethane and p-TsOH.H2O in a solvent such as ethanol and stirred at about 40 °C. The reaction was neutralized with a base such as TEA and the mixture was worked up using standard conditions to yield the intermediate of formula **hw-2. Hw-2** is then treated with *p*-TsOH.H₂O, N-methylbenzenamine, and 37% aq. HCHO solution and stirred at about 40 °C. The mixture was acidified and worked up using standard conditions. The product **hw-3** was then purified by chromatography. The intermediate of formula **hw-3** is then used to prepare the intermediate of formula **hw-4** using conditions known to one of ordinary skill in the art, including for example treating with CH₂I₂ in the presence of a methal catalyst. The intermediate of formula **hw-4** is then treated with MnO2 and DDQ in a solvent such as toluene at about reflux. The product **hw-5** was obtained after using standard workup conditions and purifying by chromatography. The intermediate of formula **hw-4** is then used in a Suzuki coupling to obtain the compound of Formula XIg where R²³ and R^{23a} form cyclopropyl, and R²⁴ and R²⁵ are hydrogen

In certain embodiments, compound of Formula **(XId)** where R²³ is methyl and R^{23a}, R²⁴, and R²⁵ are hydrogen is synthesized from a compound of Formula **XIb** where R²³ is methyl and R^{23a}, R²⁴, and R²⁵ are hydrogen by treating with an oxidizing agent such as MnO₂ in the presence of DDQ in a solvent such as 1,4-dioxane at a temperature of about 100 °C. The mixture is worked up using standard conditions and the product isolated using chromatography. Alternatively, an intermediate of formula 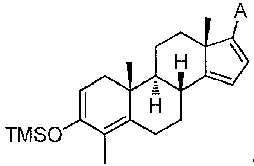 can be treated with a catalyst such as Pd(OAc)₂ in a solvent such as DCM and/or acetonitrile.

In certain embodiments, compound of Formula **(XIh)** where R²³, R^{23a}, R²⁴, and R²⁵ are hydrogen is synthesized by treating an intermediate of formula 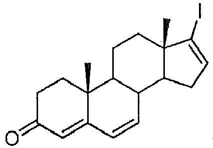 with a boronic acid using Suzuki coupling conditions as described above.

In certain embodiments, a Compound of Formula XIc where R^{23a}, R²³, R²⁴, and R²⁵ are hydrogen is prepared by treating a compound of Formula XIV where R^{7a}, R²³, R²⁴, R^{24a}, and R³⁰ are hydrogen with DMP in a solvent such as DCM.

In certain embodiments, compound of Formula **(XIg)** is synthesized according to Synthetic Scheme XXXXX where R²³ is methyl and R^{23a}, R²⁴, and R²⁵ are hydrogen. 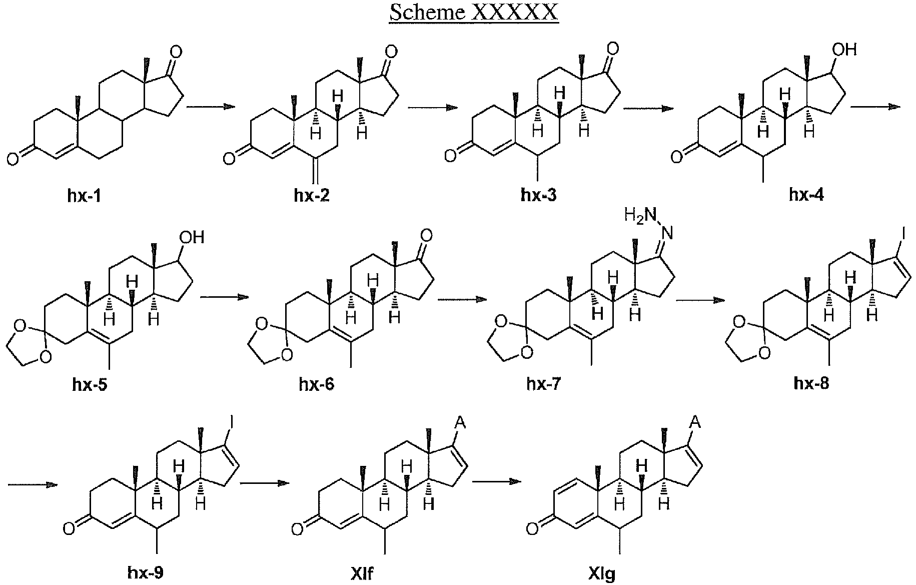 The intermediate of formula **hx-1** is treated with triethylorthoformate, *p*-toluenesulphonic acid at about 45 °C. After about 1 hour, *N*-methylaniline and aqueous formaldehyde (37%, 11.25 mL) are added. The mixture is acidified and worked up using standard conditions and the product **hx-**2 is isolated by chromatography. The intermediate of formula **hx-2** is then treated with a catalyst such as palladium on carbon in the presence of H₂ and cyclohexene in a solvent such as ethanol at about 80 °C. The mixture is worked up using standard conditions to yield the product **hx-3.** The intermediate of formula **hx-3** is then treated with a reducing agent such as NaBH4 in a solvent such as DCM and/or ethanol at a temperature of about 0 °C. The mixture is quenched with acetone and worked up using standard conditions to yield **hx-4.** The intermediate of formula **hx-4** is treated with triethoxymethane, ethane-1,2-diol, and *p*-toluenesulfonic acid in a solvent such as THF at room temperature. The pH is hten adjusted to about 8with a base such as TEA and worked up using standard conditions to yield **hx-5.** The intermediate of formula **hx-5** is treated with DMP at about 0 °C in a solvent such as DCM. The reaction is quenched with ethanol and worked up using standard conditions. The product **hx-6** is isolated by chromatography. The intermediate of formula **hx-6** is then treated with hydrazine in a solvent such as ethanol and the mixture is worked up to yield an intermediate of formula **hx-7.** The intermediate of formula **hx-7** is then treated with a base such as TEA in a solvent such as DCM and I₂ and stirred at 0 °C. The reaction is quenched and worked up using standard conditions. The product is isolated by chromatography to yield **hx-8**. The intermediate of formula **hx-8** is treated with Concentrated HCl in a solvent such as THF. The reaction is worked up using standard conditions. The product is isolated by chromatography to yield **hx-9.** The compound of Formula **XIf** is prepared from the intermediate of formula hx-9 using Suzuki coupling conditions such as those described above. The compound of formula **XIf** is treated with a base such as TEA in a solvent such as DCM followed by TMSOTf and stirred for a few hours. A catalyst such as Pd(OAc)2 and a solvent such as acetonitrile are added and the mixture is heated to about 40 °C. The mixture is then worked up using standard conditions and the product **XIg** is purified by chromatography.

In certain embodiments, compound of Formula **(XII)** is synthesized according to Synthetic Scheme XXXXXI where R²³ is methyl and R^{23a}, R²⁴, and R²⁵ are hydrogen. 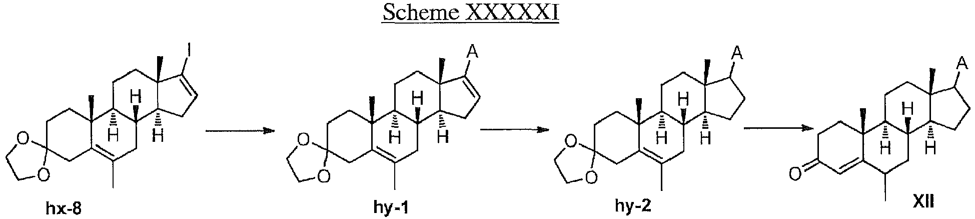 The intermediate of formula **hx-8** is treated with a catalyst such as Pd(PPh₃)₂Cl₂ in the presence of a base such as K₂CO₃ in a solvent to yield the intermediate of formula **hy-1.** The intermediate of formula **hy-1** is hydrogenated with a catalyst such as Pd/C to yield the intermediate of formul **hy-2. Hy-2** is then treated with concentrated HCl to yield **XII.**

### Certain Pharmaceutical Terminology

"Acceptable" with respect to a formulation, composition or ingredient, as used herein, means having no persistent detrimental effect on the general health of the subject being treated.

As used herein, the term "selective binding compound" refers to a compound that selectively binds to any portion of one or more target proteins.

As used herein, the term "selectively binds" refers to the ability of a selective binding compound to bind to a target protein, such as, for example, CYP11B, CYP17, and/or CYP21 enzymes, with greater affinity than it binds to a non-target protein. In certain embodiments, specific binding refers to binding to a target with an affinity that is at least about 10, about 50, about 100, about 250, about 500, about 1000 or more times greater than the affinity for a non-target.

As used herein, the term "target protein" refers to a molecule or a portion of a protein capable of being bound by a selective binding compound. In certain embodiments, a target protein is the CYP11B, CYP17, and/or CYP21 enzymes.

As used herein, the terms "treating" or "treatment" encompass either or both responsive and prophylaxis measures, e.g., designed to inhibit, slow or delay the onset of a symptom of a disease or disorder, achieve a full or partial reduction of a symptom or disease state, and/or to alleviate, ameliorate, lessen, or cure a disease or disorder and/or its symptoms.

As used herein, amelioration of the symptoms of a particular disorder by administration of a particular compound or pharmaceutical composition refers to any lessening of severity, delay in onset, slowing of progression, or shortening of duration, whether permanent or temporary, lasting or transient that can be attributed to or associated with administration of the compound or composition.

As used herein, the term inhibitor refers to a compound that decreases in the magnitude of a certain activity of a target protein or molecule compared to the magnitude of the activity in the absence of the inhibitor.

As used herein, the term "selective inhibitor" refers to a compound that selectively inhibits a target activity.

As used herein, the IC₅₀ refers to an amount, concentration or dosage of a particular test compound that achieves a 50% inhibition of a maximal response, such as modulation of CYP11B, CYP17, and/or CYP21, in an assay that measures such response.

As used herein, EC₅₀ refers to a dosage, concentration or amount of a particular test compound that elicits a dose-dependent response at 50% of maximal expression of a particular response that is induced, provoked or potentiated by the particular test compound.

In one embodiment, toxicity and therapeutic efficacy of the compounds is determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and is expressed as the ratio LD₅₀/ED₅₀. Compounds which exhibit large therapeutic indices are contemplated herein. While in some or any embodiments, compounds that exhibit toxic side effects are used, care should be taken to design a delivery system that targets such reagents to the site of affected tissue in order to minimize potential damage to normal cells and, thereby, reduce side effects.

"Carrier," as used herein, refers to relatively nontoxic chemical compounds or agents that facilitate the incorporation of a compound into cells or tissues.

The terms "co-administration" or the like, as used herein, are meant to encompass administration of the selected therapeutic agents to a single patient, and are intended to include treatment regimens in which the agents are administered by the same or different route of administration or at the same or different time.

"CYP17 substrate" includes any of the various steroid hormones acted upon by a CYP17 or a CYP17-like P₄₅₀ enzyme. Examples include pregnenolone, progesterone and their 17α-hydroxylated forms. Pregnenolone is converted to DHEA via a CYP17 C_{17,20}-lyase reaction, but is also subject to C17α-hydroxylation via the C_{17,20-}lyase activity. Progesterone is converted to δ 4-androstenedione via a CYP17 C_{17,20-}lyase reaction, but is also subject to C17α-hydroxylation via the C 17-hydroxylase activity to form 17-hydroxy-progesterone, a precursor to hydrocortisone (i.e. cortisol).

"CYP11B, CYP17, and/or CYP21 metabolite-associated disease or disorder" refers to a disease or disorder which in some or any embodiments is treated by alteration of the level of one or more CYP11B, CYP17, and/or CYP21 metabolites. Examples include a hormone dependent cancer, such as an androgen-dependent prostate cancer, which in other embodiments is treated by inhibiting CYP11B, CYP17, and/or CYP21-mediated androgen synthesis, and an estrogen-dependent breast cancer or ovarian cancer, which in further embodiments is treated by inhibiting CYP11B, CYP17, and/or CYP21-mediated estrogen synthesis.

The term "diluent" refers to chemical compounds that are used to dilute the compound of interest prior to delivery. Diluents include chemicals used to stabilize compounds because they provide a more stable environment. Salts dissolved in buffered solutions (which also can provide pH control or maintenance) are utilized as diluents in certain embodiments, including, but not limited to a phosphate buffered saline solution.

The terms "effective amount" or "therapeutically effective amount," as used herein, refer to a sufficient amount of an agent or a compound being administered which will relieve to some extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The terms "enhance" or "enhancing," as used herein, means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system.

The term "enzymatically cleavable linker," as used herein refers to unstable or degradable linkages which are degraded by one or more enzymes.

The terms "kit" and "article of manufacture" are used as synonyms.

A "metabolite" of a compound disclosed herein is a derivative of that compound that is formed when the compound is metabolized. "Active metabolite" refers to a biologically active derivative of a compound that is formed when the compound is metabolized. The term "metabolized," as used herein, refers to the sum of the processes (including, but not limited to, hydrolysis reactions and reactions catalyzed by enzymes) by which a particular substance is changed by an organism. Thus, in certain instances, enzymes produce specific structural alterations to a compound. In some or any embodiments, metabolites of the compounds disclosed herein are identified either by administration of compounds to a host and analysis of tissue samples from the host, or by incubation of compounds with hepatic cells *in vitro* and analysis of the resulting compounds.

The term "modulate," as used herein, means to interact with a target either directly or indirectly so as to alter the activity of the target, including, by way of example only, to enhance the activity of the target, to inhibit the activity of the target, to limit the activity of the target, or to extend the activity of the target.

By "pharmaceutically acceptable" or "therapeutically acceptable", as used herein, refers a material, such as a carrier or diluent, which does not abrogate the biological activity or properties of the compound, and is relatively nontoxic. In certain instances, nontoxic and non-abrogative materials includes materials that when administered to an individual do not cause substantial, undesirable biological effects and/or do not interact in a deleterious manner with any of the components of the composition in which it is contained.

The term "pharmaceutically acceptable salt" or "therapeutically acceptable salt", refers to a formulation of a compound that does not cause significant irritation to an organism to which it is administered and does not abrogate the biological activity and properties of the compound. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. In some instances, pharmaceutically acceptable salts are obtained by reacting a compound having acidic group described herein with a base to form a salt such as an ammonium salt, an alkali metal salt, such as a sodium or a potassium salt, an alkaline earth metal salt, such as a calcium or a magnesium salt, a salt of organic bases such as dicyclohexylamine, *N*-methyl-D-glucamine, tris(hydroxymethyl)methylamine, and salts with amino acids such as arginine, lysine, and the like, or by other methods known in the art.

The term "pharmaceutical combination" as used herein, means a product that results from the mixing or combining of more than one active ingredient and includes both fixed and non-fixed combinations of the active ingredients. The term "fixed combination" means that the active ingredients, e.g. a compound described herein and a co-agent, are both administered to a patient simultaneously in the form of a single entity or dosage. The term "non-flxed combination" means that the active ingredients, e.g. a compound described herein and a co-agent, are administered to a patient as separate entities either simultaneously, concurrently or sequentially with no specific intervening time limits, where such administration provides effective levels of the two compounds in the body of the patient. The latter also applies to cocktail therapy, e.g. the administration of three or more active ingredients.

The term "pharmaceutical composition" refers to a mixture of a compound described herein with other chemical components, such as carriers, stabilizers, diluents, dispersing agents, suspending agents, thickening agents, and/or excipients. The pharmaceutical composition facilitates administration of the compound to an organism. Multiple techniques of administering a compound exist in the art including, but not limited to: intravenous, oral, aerosol, parenteral, ophthalmic, pulmomary and topical administration.

A "prodrug" refers to an agent that is converted into the parent drug *in vivo.* Prodrugs are often useful because, in some situations, they are easier to administer than the parent drug. In certain instances, a prodrug is bioavailable by oral administration whereas the parent is not. In some instances, a prodrug has improved solubility in pharmaceutical compositions over the parent drug. An example, without limitation, of a prodrug is a compound described herein, which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid or amino group where the peptide is metabolized to reveal the active moiety. In certain embodiments, upon *in vivo* administration, a prodrug is chemically converted to the biologically, pharmaceutically or therapeutically more active form of the compound. In certain embodiments, a prodrug is enzymatically metabolized by one or more steps or processes to the biologically, pharmaceutically or therapeutically active form of the compound. To produce a prodrug, a pharmaceutically active compound is modified such that the active compound will be regenerated upon *in vivo* administration. In some or any embodiments, the prodrug is designed to alter the metabolic stability or the transport characteristics of a drug, to mask side effects or toxicity, to improve the flavor of a drug or to alter other characteristics or properties of a drug.

The term "subject" or "patient" encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one embodiment of the methods and compositions provided herein, the mammal is a human.

The terms "treat," "treating" or "treatment," as used herein, include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

### Pharmaceutical Composition/Formulation

In certain embodiments, pharmaceutical compositions are formulated in any manner, including using one or more physiologically acceptable carriers comprising excipients and/or auxiliaries which facilitate processing of the active compounds into pharmaceutical preparations. In some or any embodiments, proper formulation is dependent upon the route of administration chosen. In various embodiments, any techniques, carriers, and excipients are used as suitable.

Provided herein are pharmaceutical compositions that include a compound described herein and a pharmaceutically acceptable diluent(s), excipient(s), and/or carrier(s). In addition, in some or any embodiments, the compounds described herein are administered as pharmaceutical compositions in which compounds described herein are mixed with other active ingredients, as in combination therapy.

A pharmaceutical composition, as used herein, refers to a mixture of a compound described herein with other chemical components, such as carriers, stabilizers, diluents, dispersing agents, suspending agents, thickening agents, and/or excipients. In certain embodiments, a pharmaceutical composition facilitates administration of the compound to an organism. In some or any embodiments, practicing the methods of treatment or use provided herein, includes administering or using a pharmaceutical composition comprising a therapeutically effective amount of a compound provided herein. In specific embodiments, the methods of treatment provided for herein include administering such a pharmaceutical composition to a mammal having a disease or condition to be treated. In one embodiment, the mammal is a human. In some or any embodiments, the therapeutically effective amount varies widely depending on the severity of the disease, the age and relative health of the subject, the potency of the compound used and other factors. In various embodiments, the compounds described herein are used singly or in combination with one or more therapeutic agents as components of mixtures.

In certain embodiments, the pharmaceutical compositions provided herein are formulated for intravenous injections. In certain aspects, the intravenous injection formulations provided herein are formulated as aqueous solutions, and, in some or any embodiments, in physiologically compatible buffers such as Hank's solution, Ringer's solution, or physiological saline buffer. In certain embodiments, the pharmaceutical compositions provided herein are formulated for transmucosal administration. In some aspects, transmucosal formulations include penetrants appropriate to the barrier to be permeated. In certain embodiments, the pharmaceutical compositions provided herein are formulated for other parenteral injections, appropriate formulations include aqueous or nonaqueous solutions, and in one embodiment, with physiologically compatible buffers or excipients.

In certain embodiments, the pharmaceutical compositions provided herein are formulated for oral administration. In certain aspects, the oral formulations provided herein comprise compounds described herein that are formulated with pharmaceutically acceptable carriers or excipients. Such carriers enable the compounds described herein to be formulated as tablets, powders, pills, dragees, capsules, liquids, gels, syrups, elixirs, slurries, suspensions and the like, for oral ingestion by a patient to be treated.

In some or any embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients include, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. If desired, disintegrating agents are optionally added, such as the cross-linked croscarmellose sodium, polyvinylpyrrolidone, agar, or alginic acid or a salt thereof such as sodium alginate.

In certain embodiments, provided herein is a pharmaceutical composition formulated as dragee cores with suitable coatings. In certain embodiments, concentrated sugar solutions are used in forming the suitable coating, and optionally contain gum arabic, talc, polyvinylpyrrolidone, carbopol gel, polyethylene glycol, and/or titanium dioxide, lacquer solutions, and suitable organic solvents or solvent mixtures. In some or any embodiments, dyestuffs and/or pigments are added to tablets, dragees and/or the coatings thereof for, e.g., identification or to characterize different combinations of active compound doses.

In certain embodiments, pharmaceutical preparations which are used include orally include push-fit capsules made of gelatin, as well as soft, sealed capsules made of gelatin and a plasticizer, such as glycerol or sorbitol. In some or any embodiments, the push-fit capsules contain the active ingredients in admixture with filler such as lactose, binders such as starches, and/or lubricants such as talc or magnesium stearate and, optionally, stabilizers. In certain embodiments, in soft capsules, the active compounds are dissolved or suspended in suitable liquids, such as fatty oils, liquid paraffin, or liquid polyethylene glycols. In addition, stabilizers are optionally added. In certain embodiments, the formulations for oral administration are in dosages suitable for such administration.

In certain embodiments, the pharmaceutical compositions provided herein are formulated for buccal or sublingual administration. In certain embodiments, buccal or sublingual compositions take the form of tablets, lozenges, or gels formulated in a conventional manner. In certain embodiments, parenteral injections involve bolus injection or continuous infusion. In some or any embodiments, formulations for injection are presented in unit dosage form, e.g., in ampoules or in multi-dose containers, with an added preservative. In some or any embodiments, the pharmaceutical composition described herein is in a form suitable for parenteral injection as a sterile suspensions, solutions or emulsions in oily or aqueous vehicles, and optionally contains formulatory agents such as suspending, stabilizing and/or dispersing agents. Pharmaceutical formulations for parenteral administration include aqueous solutions of the active compounds in water-soluble form. In some or any embodiments, suspensions of the active compounds are prepared as appropriate oily injection suspensions. Suitable lipophilic solvents or vehicles include fatty oils such as sesame oil, or synthetic fatty acid esters, such as ethyl oleate or triglycerides, or liposomes. In certain embodiments, aqueous injection suspensions contain substances which increase the viscosity of the suspension, such as sodium carboxymethyl cellulose, sorbitol, or dextran. Optionally, the suspensions also contain suitable stabilizers or agents which increase the solubility of the compounds to allow for the preparation of highly concentrated solutions. In alternative embodiments, the active ingredient is in powder form for constitution with a suitable vehicle, *e.g*., sterile pyrogen-free water, before use.

In some or any embodiments, the compounds described herein are administered topically. In specific embodiments, the compounds described herein are formulated into a variety of topically administrable compositions, such as solutions, suspensions, lotions, gels, pastes, medicated sticks, balms, creams or ointments. Such pharmaceutical compounds optionally contain solubilizers, stabilizers, tonicity enhancing agents, buffers and/or preservatives.

In certain embodiments, the pharmaceutical compositions provided herein are formulated for transdermal administration of compounds described herein. In some or any embodiments, administration of such compositions employs transdermal delivery devices and transdermal delivery patches. In certain embodiments, the compositions are lipophilic emulsions or buffered, aqueous solutions, dissolved and/or dispersed in a polymer or an adhesive. Such patches include those constructed for continuous, pulsatile, or on demand delivery of pharmaceutical agents. In some or any embodiments, transdermal delivery of the compounds described herein is accomplished by use of iontophoretic patches and the like. In certain embodiments, transdermal patches provide controlled delivery of the compounds provided herein, such as, for example, compounds of Formula (I), (II), or (III). In certain embodiments, the rate of absorption is slowed by using rate-controlling membranes or by trapping the compound within a polymer matrix or gel. Conversely, absorption enhancers are optionally used to increase absorption. Absorption enhancer and carrier include absorbable pharmaceutically acceptable solvents that assist in passage of the compound through the skin. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin.

In certain embodiments, the pharmaceutical compositions provided herein are formulated for administration by inhalation. In certain embodiments, in such pharmaceutical compositions formulated for inhalation, the compounds described herein are in a form as an aerosol, a mist or a powder. In some or any embodiments, pharmaceutical compositions described herein are conveniently delivered in the form of an aerosol spray presentation from pressurized packs or a nebuliser, with the use of a suitable propellant, *e.g.,* dichlorodifluoromethane, trichlorofluoromethane, dichlorotetrafluoroethane, carbon dioxide or other suitable gas. In certain aspects of a pressurized aerosol, the dosage unit is determined by providing a valve to deliver a metered amount. In certain embodiments, capsules and cartridges of, such as, by way of example only, gelatin for use in an inhaler or insufflator is formulated containing a powder mix of the compound described herein and a suitable powder base such as lactose or starch.

In some or any embodiments, the compounds described herein are formulated in rectal compositions such as enemas, rectal gels, rectal foams, rectal aerosols, suppositories, jelly suppositories, or retention enemas. In certain embodiments, rectal compositions optionally contain conventional suppository bases such as cocoa butter or other glycerides, as well as synthetic polymers such as polyvinylpyrrolidone, PEG, and the like. In certain suppository forms of the compositions, a low-melting wax such as, but not limited to, a mixture of fatty acid glycerides, optionally in combination with cocoa butter is first melted.

In various embodiments provided herein, the pharmaceutical compositions are formulated in a conventional manner using one or more physiologically acceptable carriers comprising excipients and auxiliaries which facilitate processing of the active compounds into pharmaceutically acceptable preparations. In certain embodiments, proper formulation is dependent upon the route of administration chosen. In various embodiments, any of the techniques, carriers, and excipients is used as suitable. In some or any embodiments, pharmaceutical compositions comprising a compound described herein are manufactured in a conventional manner, such as, by way of example only, by means of conventional mixing, dissolving, granulating, dragee-making, levigating, emulsifying, encapsulating, entrapping or compression processes.

In certain embodiments, the pharmaceutical compositions include at least one pharmaceutically acceptable carrier, diluent or excipient and a compound described herein described herein as an active ingredient in free-acid or free-base form, or in a pharmaceutically acceptable salt form. In addition, the methods and pharmaceutical compositions described herein include the use of *N*-oxides, crystalline forms (also known as polymorphs), as well as active metabolites of these compounds having the same type of activity. In some situations, compounds described herein exist as tautomers. All tautomers are included within the scope of the compounds presented herein. Additionally, included herein are the solvated and unsolvated forms of the compounds described herein. Solvated compounds include those that are solvated with pharmaceutically acceptable solvents such as water, ethanol, and the like. The solvated forms of the compounds presented herein are also considered to be disclosed herein. In some or any embodiments, the pharmaceutical compositions described herein include other medicinal or pharmaceutical agents, carriers, adjuvants, such as preserving, stabilizing, wetting or emulsifying agents, solution promoters, salts for regulating the osmotic pressure, and/or buffers. In additional embodiments, the pharmaceutical compositions described herein also contain other therapeutically valuable substances.

Methods for the preparation of compositions containing the compounds described herein include formulating the compounds with one or more inert, pharmaceutically acceptable excipients or carriers to form a solid, semi-solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein. Semi-solid compositions include, but are not limited to, gels, suspensions and creams. In various embodiments, the compositions are in liquid solutions or suspensions, solid forms suitable for solution or suspension in a liquid prior to use, or as emulsions. These compositions optionally contain minor amounts of nontoxic, auxiliary substances, such as wetting or emulsifying agents, pH buffering agents, and so forth.

In some or any embodiments, a composition comprising a compound described herein takes the form of a liquid where the agents are present in solution, in suspension or both. In some or any embodiments, when the composition is administered as a solution or suspension a first portion of the agent is present in solution and a second portion of the agent is present in particulate form, in suspension in a liquid matrix. In some or any embodiments, a liquid composition includes a gel formulation. In other embodiments, the liquid composition is aqueous.

Useful aqueous suspension optionally contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Useful compositions optionally comprise an mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Useful compositions optionally include solubilizing agents to aid in the solubility of a compound described herein. The term "solubilizing agent" generally includes agents that result in formation of a micellar solution or a true solution of the agent. Solubilizing agents include certain acceptable nonionic surfactants, for example polysorbate 80, and ophthalmically acceptable glycols, polyglycols, *e.g.,* polyethylene glycol 400, and glycol ethers.

Useful compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Useful compositions optionally include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Certain useful compositions optionally include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Some useful compositions optionally include one or more surfactants to enhance physical stability or for other purposes. Suitable nonionic surfactants include polyoxyethylene fatty acid glycerides and vegetable oils, *e.g.,* polyoxyethylene (60) hydrogenated castor oil; and polyoxyethylene alkylethers and alkylphenyl ethers, *e.g*., octoxynol 10, octoxynol 40.

Certain useful compositions optionally one or more antioxidants to enhance chemical stability where required. Suitable antioxidants include, by way of example only, ascorbic acid and sodium metabisulfite.

In some or any embodiments, aqueous suspension compositions are packaged in single-dose non-reclosable containers. In alternative embodiments, multiple-dose reclosable containers are used, in which case it is typical to include a preservative in the composition.

In various embodiments, any delivery system for hydrophobic pharmaceutical compounds is employed. Liposomes and emulsions are examples of delivery vehicles or carriers for hydrophobic drugs. In certain embodiments, certain organic solvents such as *N-*methylpyrrolidone are employed. In some or any embodiments, the compounds are delivered using a sustained-release system, such as semipermeable matrices of solid hydrophobic polymers containing the therapeutic agent. Various sustained-release materials are utilized in the embodiments herein. In certain embodiments, sustained-release capsules release the compounds for a few weeks up to over 100 days. In some or any embodiments, depending on the chemical nature and the biological stability of the therapeutic reagent, additional strategies for protein stabilization are employed.

In certain embodiments, the formulations or compositions described herein benefit from and/or optionally comprise antioxidants, metal chelating agents, thiol containing compounds and other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

### Methods of Dosing and Treatment Regimens

In certain embodiments, the compounds described herein are used in the preparation or manufacture of medicaments for the treatment of diseases or conditions that are mediated by the CYP11B, CYP17, and/or CYP21 enzymes. Inhibition of the enzymes ameliorates the disease or condition associated with CYP11B, GYP17, and/or CYP21. In some or any embodiments, a method for treating any of the diseases or conditions described herein in a subject in need of such treatment, involves administration of pharmaceutical compositions containing at least one compound described herein, or a pharmaceutically acceptable salt, pharmaceutically acceptable N-oxide, pharmaceutically active metabolite, pharmaceutically acceptable prodrug, or pharmaceutically acceptable solvate thereof, in therapeutically effective amounts to said subject.

In certain embodiments, the compositions containing the compound(s) described herein are administered for prophylactic and/or therapeutic treatments. In certain therapeutic applications, the compositions are administered to a patient already suffering from a disease or condition, in an amount sufficient to cure or at least partially arrest the symptoms of the disease or condition. In some or any embodiments, amounts effective for this use will depend on the severity and course of the disease or condition, previous therapy, the patient's health status, weight, and response to the drugs, and the judgment of the treating physician.

In certain prophylactic applications, compositions containing the compounds described herein are administered to a patient susceptible to or otherwise at risk of a particular disease, disorder or condition. In some or any embodiments, the amount administered is defined to be a "prophylactically effective amount or dose." In certain embodiments of this use, the precise amounts of compound administered depend on the patient's state of health, weight, and the like. In certain embodiments, when used in a patient, effective amounts for this use will depend on the severity and course of the disease, disorder or condition, previous therapy, the patient's health status and response to the drugs, and the judgment of the treating physician.

In some or any embodiments, a patient's condition does not improve or does not significantly improve following administration of a compound or composition described herein and, upon the doctor's discretion the administration of the compounds is optionally administered chronically, that is, for an extended period of time, including throughout the duration of the patient's life in order to ameliorate or otherwise control or limit the symptoms of the patient's disease or condition.

In certain embodiments, once improvement of the patient's conditions has occurred, a maintenance dose is administered if necessary. In some or any embodiments, the dosage, e.g., of the maintenance dose, or the frequency of administration, or both, are reduced, as a function of the symptoms, to a level at which the improved disease, disorder or condition is retained. In certain embodiments, however, patients are optionally given intermittent treatment on a long-term basis upon any recurrence of symptoms.

In certain embodiments, the amount of a given agent that corresponds to an effective amount varies depending upon factors such as the particular compound, disease or condition and its severity, the identity (*e.g.,* weight) of the subject or host in need of treatment. In some or any embodiments, the effective amount is, nevertheless, determined according to the particular circumstances surrounding the case, including, *e.g.,* the specific agent that is administered, the route of administration, the condition being treated, and the subject or host being treated. In certain embodiments, however, doses employed for adult human treatment is in the range of about 0.02 to about 5000 mg per day. In one embodiment, dose employment for adult human treatment is about 1 to about 1500 mg per day. In various embodiments, the desired dose is conveniently presented in a single dose or as divided doses administered simultaneously (or over a short period of time) or at appropriate intervals, for example as two, three, four or more sub-doses per day.

In some or any embodiments, while the dose varies depending on age, body weight, symptom, treatment effect, administration method and the like, the pharmaceutical compositions described herein are given at a dose from about 0.01 mg to about 1 g per administration for an adult given once or several times a day orally or in a dosage form of an injection such as intravenous injection and the like. An anti-cancer agent is generally required to sustain its effect for a long time, so that can be effective not only for temporary suppression but also for prohibition on a long term basis. In one embodiment, the compounds described herein are administered on a long term basis.

In some or any embodiments, the pharmaceutical compositions described herein are in a unit dosage form suitable for single administration of precise dosages. In some instances, in unit dosage form, the formulation is divided into unit doses containing appropriate quantities of one or more compound. In certain embodiments, the unit dosage is in the form of a package containing discrete quantities of the formulation. Non-limiting examples are packaged tablets or capsules, and powders in vials or ampoules. In some or any embodiments, aqueous suspension compositions are packaged in single-dose non-reclosable containers. In alternative embodiments, multiple-dose reclosable containers are used, in which case it is typical to include a preservative in the composition. By way of example only, formulations for parenteral injection are, in some or any embodiments, presented in unit dosage form, which include, but are not limited to ampoules, or in multi-dose containers, with an added preservative.

In certain embodiments, the daily dosages appropriate for the compounds described herein are from about 0.01 to about 5 mg/kg per body weight. In some or any embodiments, an indicated daily dosage in the larger subject, including, but not limited to, humans, is in the range from about 0.5 mg to about 1000 mg, conveniently administered in divided doses, including, but not limited to, up to four times a day or in extended release form. In certain embodiments, suitable unit dosage forms for oral administration comprise from about 1 to about 500 mg active ingredient. The foregoing ranges are merely suggestive, as the number of variables in regard to an individual treatment regime is large, and considerable excursions from these recommended values are not uncommon. In certain embodiments, the dosages are altered depending on a number of variables, not limited to the activity of the compound used, the disease or condition to be treated, the mode of administration, the requirements of the individual subject, the severity of the disease or condition being treated, and the judgment of the practitioner.

In certain embodiments, toxicity and therapeutic efficacy of such therapeutic regimens are determined by standard pharmaceutical procedures in cell cultures or experimental animals, including, but not limited to, the determination of the LD₅₀ (the dose lethal to 50% of the population) and the ED₅₀ (the dose therapeutically effective in 50% of the population). The dose ratio between the toxic and therapeutic effects is the therapeutic index and it can be expressed as the ratio between LD₅₀ and ED₅₀. In certain embodiments, compounds exhibiting high therapeutic indices are preferred. In some or any embodiments, the data obtained from cell culture assays and animal studies is used in formulating a range of dosage for use in human. In specific embodiments, the dosage of such compounds lies within a range of circulating concentrations that include the ED₅₀ with minimal toxicity. In certain embodiments, the dosage varies within this range depending upon the dosage form employed and the route of administration utilized.

### Combination Treatments

Presented herein are compounds having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) in combination with a second therapeutic agent for the treatment of an androgen dependent disease, disorder or condition. In one embodiment, the compounds described herein are administered in combination with a second active agent which is effective against cancer.

Suitable compounds used in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IITA), (IIIB), (IIIC), (IIID), (IVA), (IVB), (IVC), (IVD), (IVE), (VA), (VB), (VC) or (VD) include anti-cancer agents, such as for example, hormone ablation agents, anti-androgen agents, differentiating agents, anti-neoplastic agents, kinase inhibitors, anti-metabolite agents, alkylating agents, antibiotic agents, immunological agents, interferon-type agents, intercalating agents, growth factor inhibitors, cell cycle inhibitors, enzymes, topoisomerase inhibitors, biological response modifiers, mitotic inhibitors, matrix metalloprotease inhibitors, genetic therapeutics, and anti-androgens. The amount of the additional anti-cancer agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD). Below are lists of examples of some of the classes of anti-cancer agents. The examples are not all inclusive and are for purposes of illustration and not for purposes of limitation. Many of the examples below are not restricted in any way to the class in which they are listed in and in some or any embodiments are listed in multiple classes of anti-cancer agents.

Suitable hormonal ablation agents include, but are not limited to, androgen ablation agents and estrogen ablation agents. In some or any embodiments, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIA), (IIIB), (IIIC), (IIID), (IVA), (IVB), (IVC), (IVD), (IVE), (VA), (VB), (VC) or (VD) is administered with a hormonal ablation agent, such as deslorelin, leuprolide, goserelin or triptorelin. The amount of the hormonal ablation agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD). (IVE), (VA), (VB), (VC) or (VD).

Suitable anti-androgen agents include but are not limited to bicalutamide, flutamide and nilutamide. The amount of the anti-androgen agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In another embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered with a differentiating agent. Suitable differentiating agents include, but are not limited to, polyamine inhibitors; vitamin D and its analogs, such as, calcitriol, doxercalciferol and seocalcitol; metabolites of vitamin A, such as, ATRA, retinoic acid, retinoids; short-chain fatty acids; phenylbutyrate; and nonsteroidal anti-inflammatory agents. The amount of the differentiating agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (TD), (IE), (IF), (IG), (IIA), (IIIB), (IIC), (IID), (IIIA), (IIIB), (IIIC), (IIID), (IVA), (IVB), (IVC), (IVD), (IVE), (VA), (VB), (VC) or (VD).

In a further embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered with an anti-neoplastic agent, including, but not limited to, tubulin interacting agents, topoisomerase inhibitors and agents, acitretin, alstonine, amonafide, amphethinile, amsacrine, ankinomycin, anti-neoplaston, aphidicolin glycinate, asparaginase, baccharin, batracylin, benfluron, benzotript, bromofosfamide, caracemide, carmethizole hydrochloride, chlorsulfaquinoxalone, clanfenur, claviridenone, crisnatol, curaderm, cytarabine, cytocytin, dacarbazine, datelliptinium, dihaematoporphyrin ether, dihydrolenperone, dinaline, distamycin, docetaxel, elliprabin, elliptinium acetate, epothilones, ergotamine, etoposide, etretinate, fenretinide, gallium nitrate, genkwadaphnin, hexadecylphosphocholine, homoharringtonine, hydroxyurea, ilmofosine, isoglutamine, isotretinoin, leukoregulin, lonidamine, merbarone, merocyanlne derivatives, methylanilinoacridine, minactivin, mitonafide, mitoquidone, mitoxantrone, mopidamol, motretinide, N-(retinoyl)amino acids, N-acylated-dehydroalanines, nafazatrom, nocodazole derivative, ocreotide, oquizanocine, paclitaxel, pancratistatin, pazelliptine, piroxantrone, polyhaematoporphyrin, polypreic acid, probimane, procarbazine, proglumide, razoxane, retelliptine, spatol, spirocyclopropane derivatives, spirogermanium, strypoldinone, superoxide dismutase, teniposide, thaliblastine, tocotrienol, topotecan, ukrain, vinblastine sulfate, vincristine, vindesine, vinestramide, vinorelbine, vintriptol, vinzolidine, and withanolides. The amount of the anti-neoplastic agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In some or any embodiments, the compounds described herein, such as for example, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is used with a kinase inhibitor including p38 inhibitors and CDK inhibitors, TNF inhibitors, metallomatrix proteases inhibitors (MMP), COX-2 inhibitors including celecoxib, rofecoxib, parecoxib, valdecoxib, and etoricoxib, SOD mimics or αᵥβ₃ inhibitors. The amount of the kinase inhibitor administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IC), (IIA), (IIIB), (IIC), (IID), (IIIA), (IIIB), (IIIC), (IIID), (IVA), (IVB), (IVC), (IVD), (IVE), (VA), (VB), (VC) or (VD).

In another embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered with an anti-metabolite agent. In one embodiment, suitable anti-metabolite agents are selected from, but not limited to, 5-FU-fibrinogen, acanthifolic acid, aminothiadiazole, brequinar sodium, carmofur, cyclopentyl cytosine, cytarabine phosphate stearate, cytarabine conjugates, dezaguanine, dideoxycytidine, dideoxyguanosine, didox, doxifluridine, fazarabine, floxuridine, fludarabine phosphate, 5-fluorouracil, N-(2'-furanidyl)-5-fluorouracil, isopropyl pyrrolizine, methobenzaprim, methotrexate, norspermidine, pentostatin, piritrexim, plicamycin, thioguanine, tiazofurin, trimetrexate, tyrosine kinase inhibitors, and uricytin. The amount of the anti-metabolite agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In another embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered with an alkylating agent. In another embodiment, suitable alkylating agents are selected from, but not limited to, aldo-phosphamide analogues, altretamine, anaxirone, bestrabucil, budotitane, carboplatin, carmustine, chlorambucil, cisplatin, cyclophosphamide, cyplatate, diphenylspiromustine, diplatinum cytostatic, elmustine, estramustine phosphate sodium, fotemustine, hepsul-fam, ifosfamide, iproplatin, lomustine, mafosfamide, mitolactol, oxaliplatin, prednimustine, ranimustine, semustine, spiromustine, tauromustine, temozolomide, teroxirone, tetraplatin and trimelamol. The amount of the alkylating agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIA), (IIIB), (IIIC), (IIID), (IVA), (IVB), (IVC), (IVD), (IVE), (VA), (VB), (VC) or (VD).

In yet another embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered with an antibiotic agent. In another embodiment, suitable antibiotic agents are selected from, but not limited to, aclarubicin, actinomycin D, actinoplanone, adriamycin, aeroplysinin derivative, amrubicin, anthracycline, azino-mycin-A, bisucaberin, bleomycin sulfate, bryostatin-1, calichemycin, chromoximycin, dactinomycin, daunorubicin, ditrisarubicin B, dexamethasone, doxorubicin, doxorubicin-fibrinogen, elsamicin-A, epirubicin, erbstatin, esorubicin, esperamicin-Al, esperamicin-Alb, fostriecin, glidobactin, gregatin-A, grincamycin, herbimycin, corticosteroids such as hydrocortisone, idarubicin, illudins, kazusamycin, kesarirhodins, menogaril, mitomycin, neoenactin, oxalysine, oxaunomycin, peplomycin, pilatin, pirarubicin, porothramycin, prednisone, prednisolone, pyrindanycin A, rapamycin, rhizoxin, rodorubicin, sibanomicin, siwenimycin, sorangicin-A, sparsomycin, talisomycin, terpentecin, thrazine, tricrozarin A, and zorubicin. The amount of the antibiotic agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In a further embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is used with other anti-cancer agents, including but not limited to, acemannan, aclarubicin, aldesleukin, alemtuzumab, alitretinoin, altretamine, amifostine, amsacrine, anagrelide, anastrozole, ancestim, bexarotene, broxuridine, capecitabine, celmoleukin, cetrorelix, cladribine, clotrimazole, daclizumab, dexrazoxane, dilazep, docosanol, doxifluridine, bromocriptine, carmustine, cytarabine, diclofenac, edelfosine, edrecolomab, eflornithine, emitefur, exemestane, exisulind, fadrozole, filgrastim, finasteride, fludarabine phosphate, formestane, fotemustine, gallium nitrate, gemcitabine, glycopine, heptaplatin, ibandronic acid, imiquimod, iobenguane, irinotecan, irsogladine, lanreotide, leflunomide, lenograstim, lentinan sulfate, letrozole, liarozole, lobaplatin, lonidamine, masoprocol, melarsoprol, metoclopramide, mifepristone, miltefosine, mirimostim, mitoguazone, mitolactol, molgramostim, nafarelin, nartograstim, nedaplatin, nilutamide, noscapine, oprelvekin, osaterone, oxaliplatin, pamidronic acid, pegaspargase, pentosan polysulfate sodium, pentostatin, picibanil, pirarubicin, porfimer sodium, raloxifene, raltitrexed, rasburicase, rituximab, romurtide, sargramostim, sizofiran, sobuzoxane, sonermin, suramin, tasonermin, tazarotene, tegafur, temoporfin, temozolomide, teniposide, tetrachlorodecaoxide, thalidomide, thymalfasin, thyrotropin alfa, topotecan, toremifene, trastuzumab, treosulfan, tretinoin, trilostane, trimetrexate, ubenimex, valrubicin, verteporfin, vinorelbine. The amount of the anti-cancer agent administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In yet another embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is administered or combined with steroids, such as corticosteroids or glucocorticoids. In a further embodiment, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) and the steroid are administered in the same or in different compositions. Non-limiting examples of suitable steroids include hydrocortisone, prednisone, or dexamethasone. The amount of the steroid administered to a mammal having cancer is an amount that is sufficient to treat the cancer whether administered alone or in combination with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD).

In some or any embodiments, if one of the side effects experienced by a patient upon receiving one of the compounds herein is inflammation, then, in some or any embodiments, it is appropriate to administer an anti-inflammatory agent in combination with the initial therapeutic agent. In some or any embodiments, the therapeutic effectiveness of one of the compounds described herein is enhanced by administration of an adjuvant (*i.e.,* by itself the adjuvant may have minimal therapeutic benefit, but in combination with another therapeutic agent, the overall therapeutic benefit to the patient is enhanced). In certain embodiments, the benefit experienced by a patient is increased by administering one of the compounds described herein with another therapeutic agent (which also includes a therapeutic regimen that also has same therapeutic benefit (e.g. anti-cancer agent against the same enzyme as the compound described herein but of different mode of action) so as to reduce the chance of enzyme resistant development. In some or any embodiments, regardless of the disease, disorder or condition being treated, the overall benefit experienced by the patient as a result of a combination treatment is additive or synergistic.

In certain embodiments, therapeutically-effective dosages vary when the drugs are used in treatment combinations. In some or any embodiments, therapeutically-effective dosages of drugs and other agents for use in combination treatment regimens is determined in any suitable manner, e.g., through the use of metronomic dosing, i.e., providing more frequent, lower doses in order to minimize toxic side effects. In some or any embodiments, combination treatment regimen described herein encompass treatment regimens in which administration of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIA), (IIIB), (IIIC), (IID), (IVA), (IVB), (IVC), (IVD), (IVE), (VA), (VB), (VC) or (VD) described herein is initiated prior to, during, or after treatment with a second agent described above, and continues until any time during treatment with the second agent or after termination of treatment with the second agent. It also includes treatments in which a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) described herein and the second agent being used in combination are administered simultaneously or at different times and/or at decreasing or increasing intervals during the treatment period.

In certain embodiments, compositions and methods for combination therapy are provided herein. In accordance with one aspect, the pharmaceutical compositions disclosed herein are used to in a method of treating a CYP11B, CYP17, and/or CYP2 mediated condition or a disease or condition that is ameliorated by inhibition of these enzymes.

In certain embodiments, combination therapies described herein are used as part of a specific treatment regimen intended to provide a beneficial effect from the co-action of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) described herein and a concurrent treatment. It is understood that the dosage regimen to treat, prevent, or ameliorate the condition(s) for which relief is sought, is optionally modified in accordance with a variety of factors.

In certain combination therapies described herein, dosages of the co-administered compounds vary depending on the type of co-drug employed, on the specific drug employed, on the disease or condition being treated and so forth. In some or any embodiments, when co-administered with one or more biologically active agents, the compound provided herein is administered either simultaneously with the biologically active agent(s), or sequentially. In certain aspects where the agents are administered sequentially, the attending physician will decide on the appropriate sequence of administering protein in combination with the biologically active agent(s).

In various embodiments, the multiple therapeutic agents (one of which is one of the compounds described herein) are administered in any order or even simultaneously. In certain instances, administration is simultaneous and the multiple therapeutic agents are, optionally, provided in a single, unified form, or in multiple forms (by way of example only, either as a single pill or as two separate pills). In some or any embodiments, one of the therapeutic agents is given in multiple doses, or both are given as multiple doses. In some instances, administration is not simultaneous and the timing between the multiple doses varies, by way of non-limiting example, from more than zero weeks to less than four weeks. In addition, the combination methods, compositions and formulations are not to be limited to the use of only two agents; the use of multiple therapeutic combinations is also contemplated herein.

In certain embodiments, the compounds described herein and combination therapies are administered before, during or after the occurrence of a disease or condition. In certain embodiments, the timing of administering the composition containing a compound varies. Thus, for example, in some or any embodiments, the compounds are used as a prophylactic and are administered continuously to subjects with a propensity to develop conditions or diseases in order to prevent the occurrence of the disease or condition. In some or any embodiments, the compounds and compositions are administered to a subject during or as soon as possible after the onset of the symptoms. The initial administration is achieved via any route practical, such as, for example, an intravenous injection, a bolus injection, infusion over 5 minutes to about 5 hours, a pill, a capsule, transdermal patch, buccal delivery, and the like, or combination thereof.

Compounds disclosed herein are inhibitors of CYP17 and some are additionally inhibitors of CYP21 and/or CYP11B. A person of ordinary skill in the art would know how to make and test the compounds using the disclosures herein to determine which compounds have CYP21 and CYP11B activity.

### Kits/Articles of Manufacture

For use in the therapeutic applications described herein, kits and articles of manufacture are also described herein. In various embodiments, such kits comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. In some or any embodiments, the containers are formed from a variety of materials such as glass or plastic.

In some or any embodiments, the articles of manufacture provided herein contain packaging materials. Packaging materials for use in packaging pharmaceutical products include, but are not limited to, blister packs, bottles, tubes, inhalers, pumps, bags, vials, containers, syringes, bottles, and any packaging material suitable for a selected formulation and intended mode of administration and treatment.

In some or any embodiments, the container(s) described herein comprise one or more compounds described herein, optionally in a composition or in combination with another agent as disclosed herein. The container(s) optionally have a sterile access port (for example the container can be an intravenous solution bag or a vial having a stopper pierceable by a hypodermic injection needle). Such kits optionally comprise a compound with an identifying description or label or instructions relating to its use in the methods described herein.

In some or any embodiments, a kit will comprises one or more additional containers, each with one or more of various materials (such as reagents, optionally in concentrated form, and/or devices) desirable from a commercial and user standpoint for use of a compound described herein. Non-limiting examples of such materials include, but are not limited to, buffers, diluents, filters, needles, syringes; carrier, package, container, vial and/or tube labels listing contents and/or instructions for use, and package inserts with instructions for use. A set of instructions is optionally included.

In certain embodiments, a label is on or associated with the container. In some or any embodiments, a label is on a container when letters, numbers or other characters forming the label are attached, molded or etched into the container itself; a label can be associated with a container when it is present within a receptacle or carrier that also holds the container, e.g., as a package insert. In certain embodiments, a label indicates that the contents are to be used for a specific therapeutic application. In some or any embodiments, the label indicates directions for use of the contents, such as in the methods described herein.

In certain embodiments, the pharmaceutical compositions are presented in a pack or dispenser device which contains one or more unit dosage forms containing a compound provided herein. In some or any embodiments, the pack contains a metal or plastic foil, such as a blister pack. The pack or dispenser device is optionally accompanied by instructions for administration. In some or any embodiments, the pack or dispenser is accompanied with a notice associated with the container in form prescribed by a governmental agency regulating the manufacture, use, or sale of pharmaceuticals, which notice is reflective of approval by the agency of the form of the drug for human or veterinary administration. In certain embodiments, such notice is, for example, the labeling approved by the U.S. Food and Drug Administration for prescription drugs, or the approved product insert. In some or any embodiments, compositions containing a compound provided herein are formulated in a compatible pharmaceutical carrier and are placed in an appropriate container labeled for treatment of an indicated condition.

### EXAMPLES

### Synthetic Examples

The following Examples are intended as an illustration of the various embodiments as defined in appended claims. In some or any embodiments, the compounds are prepared by a variety of synthetic routes.

Unless otherwise indicated, conventional methods of mass spectroscopy, NMR, HPLC, protein chemistry, biochemistry, recombinant DNA techniques and pharmacology are employed. Unless specific definitions are provided, the standard nomenclature employed in connection with, and the standard laboratory procedures and techniques of, analytical chemistry, synthetic organic chemistry, and medicinal and pharmaceutical chemistry are employed. In certain instances, standard techniques are used for chemical syntheses, chemical analyses, pharmaceutical preparation, formulation, and delivery, and treatment of patients. In certain embodiments, standard techniques are used for recombinant DNA, oligonucleotide synthesis, and tissue culture and transformation (e.g., electroporation, lipofection). In some or any embodiments, reactions and purification techniques are performed e.g., using kits of manufacturer's specifications or as commonly accomplished or as described herein.

### Example 1

### Example 1a: Parenteral Composition

To prepare a parenteral pharmaceutical composition suitable for administration by injection, 100 mg of a water-soluble salt of a compound having the structure of Formula (I), (II), (III), (IV), (V), (VI), (VII), (VIII), (IA), (IB), (IC), (ID), (IE), (IF), (IG), (IIA), (IIIB), (IIC), (IID), (IIIA), (IIIB), (IIIC), (IID), (IVA), (IVB), (IVC), (IVD), (IVE), (VA), (VB), (VC) or (VD) is mixed with 2-hydroxypropyl-ß-cyclodextrin and then dissolved in 10 mL of 0.9% sterile saline. The mixture is incorporated into a dosage unit form suitable for administration by injection.

### Example 1b: Oral Composition

To prepare a capsule suitable for oral administration, a water-soluble salt of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) (20 mg) is mixed with lactose (180 mg), microcrystalline cellulose (140 mg) and magnesium stearate (20 mg). The mixture is granulated and the remaining 10 mg of magnesium stearate is added. The content is then sealed in a gelation capsule.

To prepare a tablet suitable for oral administration, a water-soluble salt of a compound having the structure of Formula (I), (II), (IIIA), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) (20 mg) is mixed with lactose (70 mg), corn starch (300 mg), microcrystalline cellulose (60 mg) and magnesium stearate (10 mg). The mixture is granulated and the remaining 10 mg of microcrystalline cellulose and 2.5 mg of magnesium stearate is added. The mixture is compression formed to give a suitable tablet.

To prepare a syrup suitable for oral administration, a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) (15 mg per 5 ml of syrup) is added to a solution of 0.1% benzoic acid, 5% alcohol, citric acid, edetate disodium, ethyl maltol, flavors, glycerin, ammoniated glycyrrhizin, propylene glycol, purified water, sodium saccharin, sucrose, FD&C blue #1 and FD&C red #40.

### Example 1c: Sublingual (Hard Lozenge) Composition

To prepare a pharmaceutical composition for buccal delivery, such as a hard lozenge, mix 100 mg of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) with 420 mg of powdered sugar mixed, with 1.6 mL of light corn syrup, 2.4 mL distilled water, and 0.42 mL mint extract. The mixture is gently blended and poured into a mold to form a lozenge suitable for buccal administration.

### Example 1d: Inhalation Composition

To prepare a pharmaceutical composition for inhalation delivery, 20 mg of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is mixed with 50 mg of anhydrous citric acid and 100 mL of 0.9% sodium chloride solution. The mixture is incorporated into an inhalation delivery unit, such as a nebulizer, which is suitable for inhalation administration.

### Example 1e: Rectal Gel Composition

To prepare a pharmaceutical composition for rectal delivery, 100 mg of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is mixed with 2.5 g of methylcelluose (1500 mPa), 100 mg of methylparapen, 5 g of glycerin and 100 mL of purified water. The resulting gel mixture is then incorporated into rectal delivery units, such as syringes, which are suitable for rectal administration.

### Example If: Topical Gel Composition

To prepare a pharmaceutical topical gel composition, 100 mg of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is mixed with 1.75 g of hydroxypropyl cellulose, 10 mL of propylene glycol, 10 mL of isopropyl myristate and 100 mL of purified alcohol USP. The resulting gel mixture is then incorporated into containers, such as tubes, which are suitable for topical administration.

### Example 2

### Preparation of synthetic intermediate Compound (1)

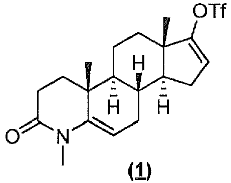

### Example 2A

### Preparation of Compound (1a)

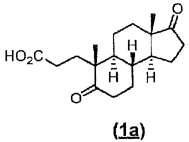

To a mixture of (8*R*,9*S*,10*R*,13*S*,14*S*)-10,13-dimethyl-7,8,9,10,11,12,13,14,15,16-decahydro-1*H*-cyclopenta[a]phenanthrene-3,17(2*H*,6*H*)-dione (androstenedione, 5 g, 17.5 mmol) suspended in t-BuOH (200 mL) was added K₂CO₃ (2.9 g, 20.9 mmol, 1.2 equiv) in water (15 mL). After the mixture was heated to 80 °C, a solution of KMnO₄ (166 mg, 1.05 mmol, 0.06 equiv) and NaIO₄ (21 g, 99.8 mmol, 5.7 equiv) in water (150 mL) was added dropwise over 1.5 hours. The mixture was heated to 80-90 °C for 5 hours, cooled to room temperature, and filtered. The solid was washed with water (3x). The filtrate was concentrated to remove most of t-BuOH, adjusted pH to 1.5 with 1N HCl, extracted with dichloromethane (DCM) (3x), dried (Na₂SO₄), concentrated to dryness to give compound **(1a)** as a colorless gum. MS calcd for (C₁₈H₂₆O₄)⁺: 306.2; MS found (electrospray): (M-H)⁻= 305.0; ¹H NMR (CDCl₃, 300 MHz) major characteristic peaks: δ 1.15 (s, 3H), 0.90 (s, 3H).

### Example 2B

### Preparation of Compound (1b)

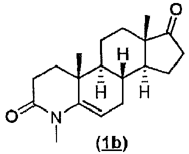

To the compound **(1a)** (7.0 g, 22.8 mmol) in sealed bottle was added methylamine (33% w/w in ethanol, 28 mL, 228 mmol, 10 equiv). The mixture was heated at 140 °C overnight. After being cooled to room.temperature, the residue was washed out with water, acidified to pH 1.5 with 1N HCl, extracted with ethyl acetate (3x), dried (Na₂SO₄), and concentrated to give compound **(1b)** (5 g, 73 %). MS calcd for (C₁₉H₂₇NO₂+H)⁺: 302.2; MS found (electrospray): (M+H)⁺= 302.2; ¹H NMR (CDCl₃, 300 MHz) major characteristic peaks: δ 4.80 (brs, 1H), 2.85 (s, 3H), 0.80 (s, 3H), 0.60 (s, 3H). 0.88 (3H).

### Example 2C

### Preparation of Compound (1)

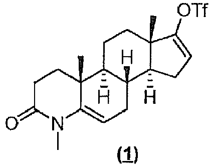

To a solution of compound **(1b)** (1.0 g, 3.3 mmol) in dichloromethane (DCM) (5 mL) was added trifluoromethane sulfonic anhydride (0.61 mL, 3.63 mmol, 1.1 equiv) at room temperature and stirred for 10 min. To the solution was added triethylamine (TEA) (0.46 mL, mmol) in dichloromethane (2 mL) dropwise within 20 min. The mixture was stirred 4h. TLC indicated starting material remained. Additional 0.5 equiv of reagents was added. The mixture was stirred overnight and water (5 mL) was added. The mixture was extracted with DCM (3x). The organic layers were combined, washed with 1N HCl, brine, dried with Na₂SO₄, concentrated, and purified by chromatography on silica gel (hexanes/ethyl acetate,1:1) to give compound **(1)** (460 mg, 32%). MS calcd for (C₂₀H₂₆F₃NO₄S +H)⁺: 434.1; MS found (electrospray): (M+H)⁺= 434.2; ¹H NMR (CDCl₃, 300 MHz) major characteristic peaks: δ 5.60 (s, 1H), 5.05 (brs, 1 H), 3.15 (s, 3H), 1.10 (s, 3H), 1.05 (s, 3H).

### Example 3

### Preparation of synthetic intermediate Compound (2)

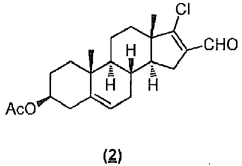

### Example 3A

### Preparation of Compound (2a)

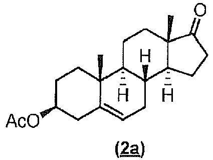

To a suspension of (3*S*,8*R*,9*S*,10*R*,13*S*,14*S*)-3-hydroxy-10,13-dimethyl-3,4,7,8,9,10,11,12,13,14,15,16-dodecahydro-1*H*-cyclopenta[*a*]phenanthren-17(2*H*)-one (dehydroisoandrosterone, 20.0 g, 69.4 mmol) in DCM (50 mL) was added BF₃-Et₂O (436 µL, 3.47 mmol, 0.05 equiv) and followed by acetic anhydride (8.52 mL, 90.2 mmol, 1.3 equiv) within 15 min. The mixture was stirred at 25°C for 4h until no starting material left. To the mixture was added water, extracted with DCM (3x). The organic layers were combined, washed with NaHCO₃ (saturated), dried with Na₂SO₄, and concentrated to give compound (**2a**) as a white powder (22 g, 96%). MS calcd for (C₂₁H₃₀O₃)⁺: 330.2. ¹H NMR (CDCl₃, 300 MHz) major characteristic peaks: δ 5.40 (br s, 1H), 4.65-4.50 (m, 1H), 2.05 (s, 3H), 1.05 (s, 3H), 0.85 (s, 3H).

### Example 3B

### Preparation of Compound (2)

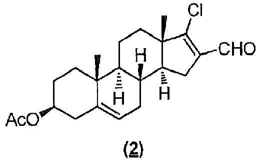

Dimethylformamide (DMF) (5 mL, 64 mmol) was added into a cold solution of phosphorus oxychloride (5 mL, 55 mmol) in chloroform (5 mL) in ice/water bath, followed by addition of a solution of 3β-acetoxyandrost-5-en-17-one (**2a**)(1 g, 3.0 mmol) in chloroform (15 mL) dropwise into the reaction flask. After the addition, the mixture was allowed to attain room temperature and then refluxed under N₂ for 5 hours. It was then concentrated under reduced pressure and poured onto ice followed by extraction with a mixture of ether and ethyl acetate (8:2, v/v). The combined extracts were washed with brine and dried over Na₂SO₄. Purification with flash column chromatography (hexane/ EtOAc, 15:1) gave the synthetic intermediate compound **(2)** (0.83 g, yield 78.7%). MS calculated for (C₂₂H₂₉O₃Cl)⁺ 376.2. ¹HNMR (CDCl₃, 300 MHz) major characteristic peaks: δ 9.9 (1H, s), 5.4 (1H, d), 4.6 (1H, m), 2.0 (3H, s), 1.1 (3H, s), 0.99 (3H, s).

### Example 4

### Preparation of synthetic intermediate Compound (3)

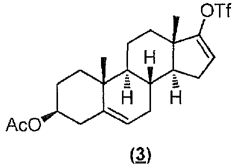

To a suspension of (3*S*,8*R*,9*S*,10*R*,13*S*,14*S*)-10,13-dimethyl-17-oxo-2,3,4,7,8,9,10,11,12,13,14,15,16,17-tetradecahydro-1*H*-cyclopenta[*α*]phenanthren-3-yl acetate (**2a**) (10 g, 30.3 mmol) in DCM (120 mL) was added triethylamine (4.2 mL, 30.3 mmol,1.0 equiv) at 0 °C. trifluoromethane sulfonic anhydride (Tf₂O)(5.6 mL, 33.4 mmol, 1.1 equiv) was diluted with DCM (50 mL) and added dropwise into the above solution over 30 min. The mixture was stirred at room temperature overnight. Water (100 mL) was added, and the mixture was stirred for 40 minutes to quench the reaction, followed by extraction with DCM (3x). The organic layers were combined, washed with 2N HCl (100 mL), brine (100 mL), dried over Na₂SO₄, and concentrated to afford crude compound (3). After purified with flash column chromatography (Hexane/EtOAc, 30:1 to 10:1), compound (**3**) (5.0 g, yield 35.7%) and un-reacted starting material (4.3 g, 43%) were obtained.

### Example 5

### Preparation of synthetic intermediate Compound (4)

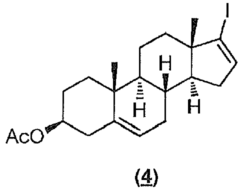

### Example 5A

### Preparation of Compound (4a)

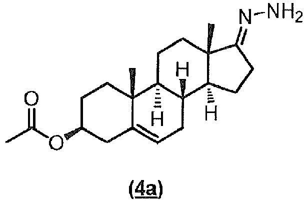

3β-Acetoxyandrost-5-en-17-one **(2a)**((1 g, 3.3 mmol) was dissolved in ethanol (15 mL), and the resulting solution was treated with hydrazine hydrate (∼ 80%, 750 µL, 12 mmol) followed by a solution of hydrazine sulfate (2 mg, 15 µmol) in 70 µL of water. The mixture was stirred under N₂ at room temperature for 12 hours and then poured into ice water. The resulting precipitate was filtered, washed with water and dried to give compound **(4a)** (0.87 g, yield 83%). MS calculated for (C₂₁H₃₂N₂O₂)⁺ 345.5. ¹HNMR (CDCl₃, 300 MHz) major characteristic peaks: δ 5.4 (1H, d), 4.6 (1H, m), 2.0 (3H, s), 1.0 (3H, s), 0.92 (3H, s).

### Example 5B

### Preparation of Compound (4)

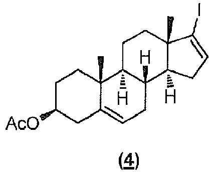

Iodine (3.5 g, 13.8 mmol) was dissolved in dry THF (40 mL) and dry ether (20 mL). The solution was cooled in an ice bath and then treated with 1,1,3,3-tetramethylguanidine (2 mL, 15 mmol). A solution of 3β-Acetoxyandrost-5-en-17-hydrozone (860 mg, 2.5 mmol) in THF (22 mL) was added dropwise into the iodine solution over 2 hours maintaining the reaction temperature at 0 °C. Then solvents were removed in vacuum, and the residue was re-dissolved with methylene chloride and washed with Na₂SO₃ and brine. The solution was dried over Na₂SO₄ and then concentrated to give a residue, which was purified with flash column chromatography (Hexane/EtOAc, 20:1) to give compound (**4**) (630 mg, yield 57%). MS calculated for (C₂₁H₂₉IO₂)⁺ 440.4; ¹HNMR (CDCl₃, 300 MHz) major characteristic peaks: δ 6.15 (1H, m), 4.6 (1H, m), 2.0 (3H, s), 1.0 (3H, s), 0.76 (3H, s).

### Example 6

### Preparation of synthetic intermediate Compounds (5) and (6)

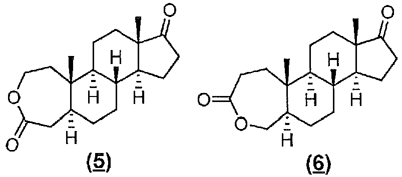

### Example 6A

### Preparation of Compound (5a)

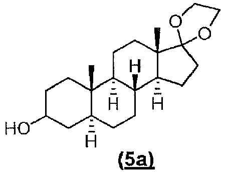

A solution of epiandrosterone (29 g), ethylene alcohol (16.8 mL) and p-toluenesulfonic acid (0.517 g) in toluene (200 ml) were refluxed under a Dean-Stark trap for 2 h. The mixture was cooled to room temperature, diluted with dichloromethane (100 mL), and washed with saturated NaHCO₃ (100 mL) and brine (100 mL). The organic phase was dried over anhydrous sodium sulfate, filtered, and evaporated to provide 35 g of compound (**5a**). ¹H NMR (CDCl₃, 300 MHz): 3.85-4.0(4H), 3.75(1H), 3.56(1H), 0.9-0.95(6H).

### Example 6B

### Preparation of Compound (5b)

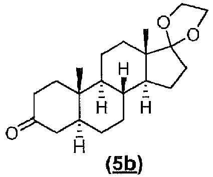

A solution of compound (**5a**) (5g) in dichloromethane (300 mL) was treated with Dess-Martin Periodinane (9.5 g) at room temperature. The reaction was stirred until all the start material was consumed. The mixture was washed with sat.Na₂SO₃ (150 mL), saturated NaHCO₃ (2X 150 mL) and brine (150 mL). The solvent was dried over anhydrous sodium sulfate, filtered, and evaporated to dryness. The residue was purified by flash column chromatograph (silica gel, hexane: EtOAc=95:5) to afford 3.5 g (69.9%) of compound (**5b**). ¹H NMR (CDCl₃, 300 MHz): 3.7-4.1(4H), 2.2-2.5(3H), 1.9-2.1(3H), 1.05(3H), 0.91(3H). ¹³C NMR (CDCl₃, 300 MHz): 211.84, 119.27, 65.16, 64.49, 53.58, 50.10, 14.37, 11.43.

### Example C

### Preparation of Compounds (5c) and (6a)

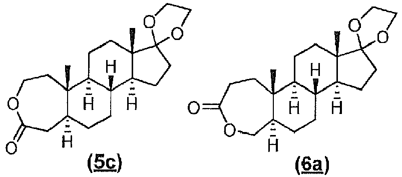

A solution of compound (**5b**) (3.2 g) in dichloromethane (250 mL) was treated with mCPBA (4.9 g). The mixture was stirred overnight at reflux. The reaction was monitored by TLC and the starting material was completely consumed. The mixture was washed with 10% aqueous Na₂CO₃ (150 mL), 10% aqueous NaHCO₃ (150 mL) and brine (150 mL). The organic phase was dried over anhydrous sodium sulfate, filtered, and evaporated to dryness. The residue was purified by flash column chromatograph to afford 3.1 g (92.4%) of the regioisomeric mixture of compound **(5c)** and compound **(6a).** ¹H NMR (CDCl₃, 300 MHz): 4.25-4.35(1H), 4.1-4.2(0.47H), 3.8-4.0(4H), 3.65-3.7(0.0.53H), 2.45-2.90(2H), 0.95(3H), 0.85(3H). ¹³C NMR (CDCl₃, 300 MHz): 176.17, 119.19, 70.01, 65.19, 64.53.53.59, 53.37, 14.33, 12.3.

### Example 6D

### Preparation of Compounds (5) and (6)

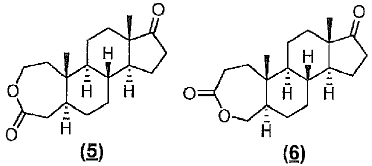

The regioisomeric mixture of compound **(5c)** and compound **(6a)** (2 g), p-TsOH (0.2 g) and water (150 mg) in acetone (60 mL) was stirred at room temperature for 4 h. The solvent was removed under reduce pressure, 20 mL of EtOAc was added, the organic phase was separated, washed with saturated NaHCO₃ (15 mL) and brine (20 mL). The solvent was dried over anhydrous sodium sulfate, filtered, and evaporated to give 1.5 g (85.8%) of the regioisomeric mixture of compound **(5)** and compound **(6).** ¹H NMR (CDCl₃, 300 MHz): 4.35-4.45(1H), 4.15-4.2(0.47H), 3.68-3.71(0.52H), 2.6-2.9(1H), 2.4-2.5(1H), 2.0-2.1(2H), 0.96(3H), 0.85(3H). ¹³C NMR (CDCl₃, 300 MHz): 175.94, 175.70, 69.83, 64.52, 53.83, 53.62, 51.14, 48.54, 47.46, 43.31, 41.63, 13.74, 12.31, 12.10.

### Example 7

### Preparation of intermediate Compounds (7) and (8)

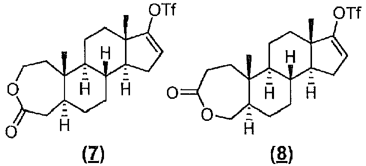

To a solution of compound **(5)** and compound **(6)** (1.45 g) in DCM (60 mL) was added trifluoromethane sulfonic anhydride (1.55 g, 1.5 equiv) at room temperature. The solution was stirred over 10 min and TEA (0.5 g, 1 eq.) in DCM (10 mL) was added dropwise within 30 min. The mixture was stirred overnight. The reaction mixture was poured into saturated NaHCO₃ (40 mL), and the layers were separated. The aqueous layer was extracted with DCM (3 X 50 mL). The organic layers were combined, washed with water (40 ml) and brine (40 ml), dried over sodium sulfate. The solvent was concentrated and purified by column chromatography on silica gel (hexanes/ethyl acetate=95:5) to give 0.8 g (36.7%) of a mixture of compound **(7)** and compound **(8).** ¹H NMR (CDCl₃, 300 MHz): 5.6(1H), 4.25-4.35(1H), 4.1-4.15(0.449H), 3.65-3.75(0.646H), 2.5-2.9(2H), 0.98-1.05(6H). ¹³C NMR (CDCl₃, 300 MHz): 174.89, 174.66, 157.98, 119.14, 115.96, 113.55, 113.45, 68.82, 63.48, 53.06, 52.87, 47.70, 14.22, 11.24, 11.03.

### Example 8

### Preparation of synthetic intermediate Compound (9)

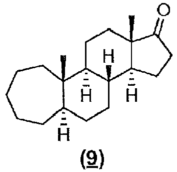

### Example 8A

### Preparation of Compounds (9a) and (9b)

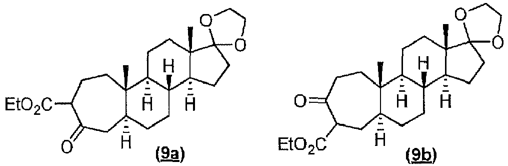

To a solution of compound **(5b)** (10.0 g, 30.0 mmol, 1.00 equiv.) and ethyl diazoacetate (3.60 g, 31.6 mmol, 1.05 equiv.) in CH₂Cl₂ (150 mL) was added dropwise BF₃·Et₂O (8.51 g, 60.0 mmol, 2.0 equiv.) at -78 °C with stirring. The reaction was stirred for an additional 3 h until the starting material was consumed completely (TLC: Hexanes/EtOAc=5/1). The reaction solution was admixed with 10% aqueous NaHCO₃ (80 mL). The organic layer was partitioned. The water phase was extracted with 60 mL of CH₂Cl₂. The combined organic phase was washed with brine, dried on MgSO₄ and condensed by rotary evaporator to give 12.6 g of the crude intermediates compounds **(9a)** and **(9b).**

### Example 8B

### Preparation of Compounds (9c) and (9d)

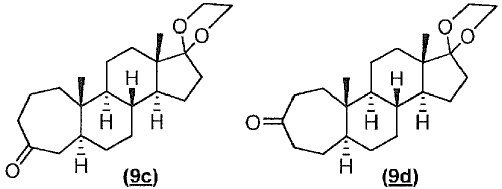

A mixture of compounds **(9a)** and **(9b)** was dissolved into 150 mL of methanol. 100 mL of aqueous NaOH (1 mol/L) was added and the mixture was stirred overnight at room temperature. TLC (Hexanes/EtOAc=5/1) indicated the reaction came to completion. MeOH was removed by rotary evaporator. The remaining aqueous solution was adjusted to pH→5 by adding 10% citric acid. Extraction with EtOAc (3x50 mL), washing with brine, drying on MgSO₄ and purification with FCC (Hexanes/EtOAc=10/1→8/1) provided 7.2 g of the keto compounds **(9c)** and **(9d)** in 68.3% yield.

### Example 8 C

### Preparation of Compound (9e)

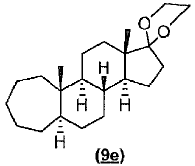

A mixture of the keto compounds **(9c)** and **(9d)** (2.5 g, 7.215 mmol) in methanol (160 ml) was added *p*-TosNHNH₂(1.88 g 10.1 mmol) at room temperature with stirring for 8 h. TLC (Hexanes/ethyl acetate=4/1) showed the reaction completed. NaBH₄ (0.6 g, 15.87 mmol) was added in one portion. The reaction mixture was refluxed overnight. TLC (Hexanes/EtOAc=4/1) showed the reaction completed. Water (60 mL) was added Methanol was removed under rotary evaporator. The residue solvent was was extracted with EtOAc. The organic phase was washed with brine, dried on Na₂SO₄, condensed under vacumn and purified via FCC (Hexanes/EtOAc=15/1) to give 1.2 g of compound **(9e)** as a solid.

### Example 8D

### Preparation of Compound (9)

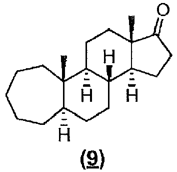

Compound **(9e)** (1.2 g) was treated with 5*N* HCl (10 ml) in 30 ml of THF at room temperaturet for 1 hr. TLC (Hexanes/EtOAc=9/1) showed completed. THF was removed by rotary evaporator. Extraction with EtOAc, washed with 10% NaHCO₃, drying on MgSO₄ and condensation gave 800 mg of the ketone compound **(9)** as an oil.

### Example 9

### Preparation of synthetic intermediate Compound (10)

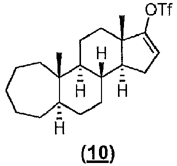

Compound **(9e)** (1.2 g) was treated with 5*N* HCl (10 ml) in 30 ml of THF at room temperature for 1 hr. TLC (Hexanes/EtOAc=9/1) showed completed. THF was removed by rotary evaporator. Extraction with EtOAc, washed with 10% NaHCO₃, drying on MgSO₄ and condensation gave 800 mg of the ketone as an oil. The ketone (800 mg, 2.78 mmol) was treated with TEA (560 mg, 2 eq.) and trifluoromethane sulfonic anhydride (780 mg, 1 eq.) in 10 mL of dichloromethane with an ice-water bath with stirring. The reaction was allowed to warm to room temperature and stirred for an additional 4 hr. 10 mL of icy water was added to quench the reaction. The organic phase was partitioned by a separating funnel, washed with brine, dried over sodium sulfate and condensed under reduced pressure to give 1.2 g of compound (10) in 65% yield as a yellow solid.

### Example 10

### Preparation of synthetic intermediate Compound (11)

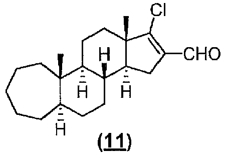

A solution of compound **(9)** (350 mg, 1.21 mmol) in dry chloroform (15 ml) was added to a cold solution of phosphorus oxychloride (1.5 mL) in DMF (1.5 mL) at 0 °C. The mixture was allowed to warm to room temperature and then refluxed under nitrogen over night. The reaction mixture was poured into ice-water, followed by extraction with EtOAc (3x15 ml). The organic layers was washed with brine, dried over Na₂SO₄, and condensed to give an oil. Purification by FCC (Hexanes/EtOAc=25/1) gave compound **(11)** (286 mg, 70.7%). ¹H NMR 9.9(1H), 2.49-2.51(1 H), 1.93-2.11(1H), 1.13-1.92(18H), 0.85-1.12(10H).

### Example 11

### Preparation of synthetic intermediate Compound (12)

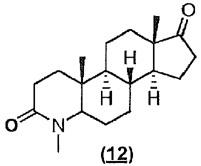

A solution of compound **(1b)** (27.7 g) in 450 mL of methanol was hydrogenated in the presence of 5% Pd/C (13.8 g) at 40 °C for 28 hr. The catalyst was removed by filtration. The filtrate was condensed to give 26.7 g of compound **(12)** in 92.5% yield.

### Example 12

### Preparation of synthetic intermediate Compound (13)

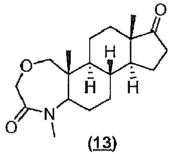

### Example 12A

### Preparation of Compound (13a)

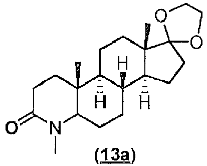

A mixture of compound **(12)** (20.5 g, 67.6 mmol), TsOH (233 mg) and ethylene glycol (14.0 g) in 300 mL of toluene was azeotropic distilled through a Dean-Stark receiver at reflux for 8 hr. The mixture was cooled to room temperature and treated with 200 mL of icy saturated NaHCO₃. The layers were partitioned. The water layer was extracted with EtOAc (2x200 mL). The combined organic layers were washed with brine, dried over MgSO₄, and condensed under reduced pressure to give 20.7 of compound (13a) in 88.1 % yield.

### Example 12B

### Preparation of Compound (13b)

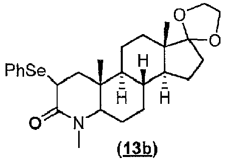

To a solution of 42.0 g of diisopropylamine in 250 mL of THF was cooled to -70 °C. 165 mL of n-butyl lithium (1.6 M in hexanes) was added with stirred at that temperature for 30 min. The mixture was allowed to warm to room temperature and stirred for an additional 30 min. This solution was added dropwise to a mixture of compound **(13a)** (24.0 g, 69.2 mmol) and PhSeSePh (23.0 g, 72.7 mmol) in THF (250 mL) at -78 °C. The formed mixture was allowed to warm to room temperature and stirred overnight. The reaction mixture was quenched with ice-water. THF was removed under reduces pressure. The mixture was extracted with EtOAc (3x500 mL). The combined organic phases was washed with 400 mL of brine, dried over MgSO₄, and condensed under reduced pressure to give 22.0 g of compound **(13b)** in 63.4 % yield.

### Example 12C

### Preparation of Compound (13c)

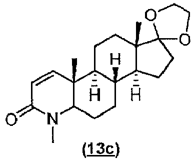

A mixture ofcompound **(13b)** (2.04 g, 4.06 mmol) and pyridine (99.6 mg) in 20 mL of CH₂Cl₂ was treated with H₂O₂-H₂O (4.5 mL/2.1 mL) at room temperature for 15 hr. The mixture was quenched with 10% Na₂SO₃, and extracted with CH₂Cl₂ (2x20 mL). The combined organic layers were washed with 25 mL of brine, dried on Na₂SO₄ and condensed under reduced pressure to give 1.3 g of compound (**13c**) in 93% yield.

### Example 12D

### Preparation of Compound (13d)

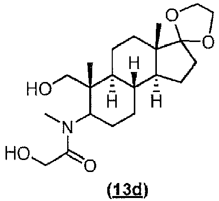

A solution of compound (**13c**) (3.60 g, 10.4 mmol) in 56 mL of CH₂Cl₂ and 44 mL of MeOH was treated with O₃ for 1 hr at -65 °C. The solvents were removed under reduced pressure and re-dissolved into 56 mL of EtOH and 6.5 mL of CH₂Cl₂. 2.64 g of NaBH₄ was added and stirred for 1 hr. The solvents were removed under reduced pressure. 50 mL of water and 50 mL of EtOAc was added, stirred and partitioned. The water phase was extracted with EtOAc (2x50 mL). The combined organic phase was washed with 100 mL of brine, dried over Na2S04 and condensed under reduced pressure to give 3.1 g of compound **(13d)** in 88% yield. ¹H NMR (CDCl₃) 3.65 (d, 1H), 3.40 (d, 1H), 2.31 (s, 3H), 2.20 (1H), 0.85 (s, 3H), 0.71 (s, 3H); ESI MS: 281.5 (M+Na⁺).

### Example 12E

### Preparation of Compound (13e)

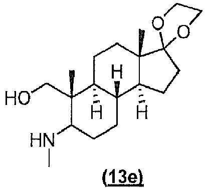

To a solution of compound **(13d)** (2.60 g) in MeOH (200 mL) was added dropwise aqueous KOH (3.34 g of KOH in 25.0 mL of H₂O). The resulting solution was refluxed overnight. MeOH was removed under reduced pressure. The aqueous phase was extracted with CH₂Cl₂ (2x50 mL). The combined CH₂Cl₂ layers were washed with brine (2x30 mL), dried on Na₂SO₄ and condensed under reduced pressure to get 2.4 g of compound (**13e**) as an off-white solid.

### Example 12F

### Preparation of Compound (13f)

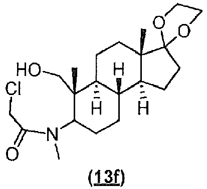

To a solution of compound **(13e)** (5.00 g, 15.46 mmol) in 200 ml of THF, was added K₂CO₃ (6.4 g, 46.38 mmol) in one portion. The mixture was cooled to 0 °C under argon. ClCH₂COCl (2.10 g, 1.4 mL, 18.55 mmol) was added dropwise for 1 hr. The reaction was allowed to warm to room temperature and was stirred overnight. 50 mL of icy water was added. The organic phase was partitioned. The water phase was extracted with CH₂Cl₂ (2x50 ml). The combined organic phase was dried over Na₂SO₄. FCC on silica gel (Hexanes/ethyl acetate=4/1) gave compound **(13f)** (2.72 g, in 44.0% yield).

### Example 12G

### Preparation of Compound (13g)

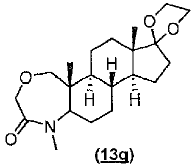

A solution of compound **(13f)** (2.72 g, 6.80 mmol) in 120 ml of THF was cooled to 0 °C under nitrogen protection. Sodium hydride (0.54 g, 13.60 mmol) was added at 0 °C. The mixture was allowed to warm to room temperature overnight. The volatile was evaporated. The residue was treated with 100 mL of ethyl acetate and 50 mL of water. The organic layer was partitioned. The water layer was treated with ethyl acetate (2x60 mL). The combined organic layers was dried over anhydrous sodium sulphate, and condensed to provide compound **(13g)** (2.0 g, in 83.3% yield).

### Example 12H

### Preparation of Compound (13)

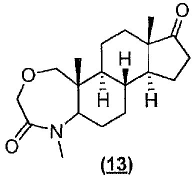

The compound **(13g)** (2.00 g, 5.50 mmol) was treated with 20 mL of 5 *N* HCl in 20 mL of THF at room temperature for 2 hr. THF was removed by rotary evaporator. Extraction (ethyl acetate, 3x20 mL), drying on anhydrous sodium sulphate and condensation gave compound **(13)** (1.50 g, in 88.0% yield).

### Example 13

### Preparation of synthetic intermediate Compound (14)

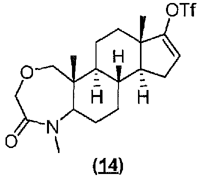

A solution of compound **(13)** (1.20 g, 3.75 mmol) in 50 mL of CH₂Cl₂ was treated with trifluoromethane sulfonic anhydride (1.50 g, 5.63 mmol) at 0 °C, followed with triethylamine (0.38 g, 3.75 mmol). The reaction was allowed to warm to room temperature with stirring overnight. 20 mL of water was added. Extraction (ethyl acetate, 2x50 mL), drying (over Na₂SO₄) and separation by FCC (Hexanes/ethyl acetate=3/1) provided compound **(14)** (0.5 g) with 0.4 g of compound **(13)** recovered.

### Example 14

### Preparation of synthetic intermediate Compound (15)

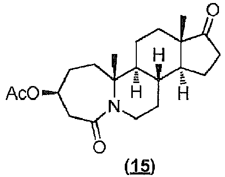

### Example 14A

### Preparation of Compound (15a)

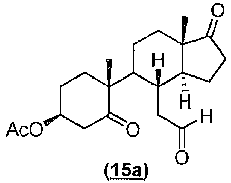

A solution of compound (**2a**) (20 g, 67 mmol) in dichloromethane-methanol (133 mL, 3:1 v/v) was oxidation by passing Ozone through the solution at -78 °C for 2 hour (checked by TLC). As soon as the material was disappeared, the excess ozone was purged with nitrogen. The mixture was evaporated and stirred with Zinc (16 g, 240 mmol) in water-acetic acid (1:9 v/v, 1 L) for 2 h at room temperature, checked by TLC. After complication, the solvent was removed and the mixture was extracted with dichloromethane (3 × 60 mL). The combined organic layers were washed with water, brine, dried over Na₂SO₄ and evaporated to obtain compound (**15a**).MS calcd for (C₂₈R₄₉O₅Si)⁺: 362.2;. ¹H NMR (CDCl₃, 400 MHz) major characteristic peaks: δ 9.66 (s, 1 H), 5.37 (m, 1 H), 2.10 (s, 3 H), 1.05 (s, 3 H), 0.89 (s, 3 H).

### Example 14B

### Preparation of Compound (15b)

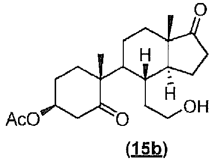

A solution of compound **(15a)** (1.6 g, 4.4 mmol) dissolved in tetrahydrofuran (8 mL) cooled to -60 °C. NaBH(OAc)₃ (933 mg, 4.4 mmol) was added. Then ethanol (3 mL) was added to the mixture. The mixture was stirred at room temperature for 3 hours. Water (60 mL) was added, the solution was extracted with dichloromethane (3 × 20 mL), washed with water, brine, dried over Na₂SO₄ and evaporated to obtain crude product and purified by column chromatography (dichloromethane: methanol = 30:1, v/v) to obtain compound **(15b).** (810 mg, yield:50%) as a white solid. MS calcd for (C₂₈H₄₉O₅Si)⁺: 364.2; MS found (electrospray):[M+23]=387.2. ¹H NMR (CDCl₃, 400 MHz) major characteristic peaks: δ 6.80 (m, 1 H), 5.98 (m, 1 H), 5.40 (m, 1 H), 3.49 (m, 2 H), 2.09 (s, 3 H), 1.12 (s, 3 H), 0.87 (s, 3 H).

### Example 14C

### Preparation of Compound (15c)

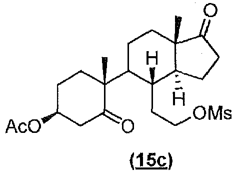

A solution of compound **(15b)** (560 mg, 1.54 mmol) and triethylamine (249 mg, 2.46 mmol) dissolved in tetrahydrofuran (8 mL) cooled by ice-bath. Then, methanesulfonyl chloride (210 mg, 1.85 mmol) was added. The mixture was stirred at 0 °C for 3 hours. Water (30 mL) was added, and extracted with dichloromethane (3 × 30 mL) and washed with brine, dried over Na₂SO₄ and evaporated to obtain the crude product and purified by column chromatography (dichloromethane: methanol=30:1, v/v) to obtain compound **(15c).** (440 mg, yield: 65%) as a white solid. MS calcd for (C₂₈H₄₉O₅Si)⁺: 442.2; MS found (electrospray):[M+23]=465.2. ¹H NMR (CDCl₃, 400 MHz) major characteristic peaks: δ 5.39 (m, 1 H), 4.11 (m, 2 H), 3.04 (s, 3 H), 2.03 (s, 3 H), 1.09 (s, 3 H), 0.87 (s, 3 H).

### Example 14D

### Preparation of Compound (15d)

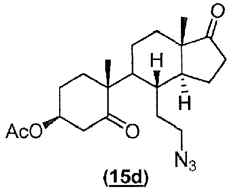

A solution of compound **(15c)** (670 mg, 1.52 mmol) dissolved in N,N-DMF (5 mL) was treated with NaN₃ (108 mg, 1.67 mmol). Then, the mixture was stirred at 50 °C for 5 hours, and monitored by TLC. Water (50 mL) was added, and extracted with ethyl acetate (3 × 20 mL) and washed with brine, dried over Na₂SO₄ and evaporated to obtain the crude product and purified with silica gel chromatography to obtain compound **(15d)** (240 mg, yield: 40%) as a white solid.. MS calcdfor (C₂₈H₄₉O₅Si)⁺: 389.2; MS found (electrospray):[M+23]=412.2. ¹H NMR (CDCl₃, 400 MHz) major characteristic peaks: δ 5.39 (m, 1 H), 2.52 (m, 2 H), 2.03 (s, 3 H), 1.09 (s, 3 H), 0.87 (s, 3 H).

### Example 14E

### Preparation of Compound (15)

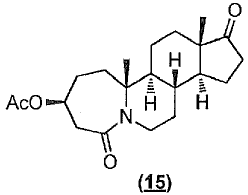

A solution of compound **(15d)** (1.8 g, 4.6 mmol) dissolved in dichloromethane (10 mL) was treated with TiCl₄ (1.29 g, 6.9 mmol) at 0 °C under N₂. A yellow precipitate was obtained. Then, the mixture was stirred at room temperature for 4 hours. Water (50 mL) was added, and extracted with dichloromethane (3 × 20 mL) and washed with brine, dried over Na₂SO₄ and evaporated to obtain the crude product and purified by column chromatography (petroleum -ether/ethyl acetate=1:1, v/v) to obtain compound (**15**) (760 mg, yield: 46%) as a white solid. MS calcd for (C₂₈H₄₉O₅Si)⁺: 361.2; MS found (electrospray):[M+23]=394.2. ¹H NMR (CDCl₃, 400 MHz) major characteristic peaks: δ 5.13 (m, 1 H), 4.68 (d, J = 8.0 Hz, 2 H), 2.03 (s, 3 H), 1.36 (s, 3 H), 0.91 (s, 3 H).

### Example 15

### Preparation of synthetic intermediate Compound (16)

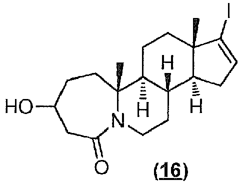

### Example 15A

### Preparation of Compound (16a)

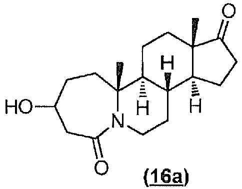

A mixture of compound **(15)** (666 mg, 1.84 mmol), K₂CO₃ (254 mg, 1.84 mmol) in MeOH (10 mL) was stirred at 10 °C for 2 hours. Methanol was removed and the residue was diluted with water (30 mL), extracted with dichloromethane (DCM) (3 x 15 mL), the organic layer was washed with brine (1 x 20 mL), dried and concentrated to get the final compound **(16a)** (587 mg, 100%) as a white solid. MS calcd for (C₂₄H₃₀N₂O₂)⁺:319.21; MS found (electrospray): 320.21; ¹H NMR (CDCl₃, 400 MHz) major characteristic peaks: δ 0.91 (s, 3H), 1.36 (s, 3H), 2.46 (m, 1H), 2.86 (m, 3H), 4.21 (m, 1H), 4.70 (m, 1H). (s, 3 H).

### Example 15B

### Preparation of Compound (16b)

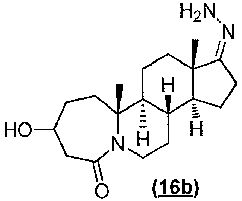

The mixture of compound **(16a)** (587 mg, 1.84 mmol) was dissolved in ethanol (10 mL), Et₃N (0.766 mL) and N₂H₄. H₂O (85%) (920 mg, 18.4 mmol) were added. After that, the mixture was reflux for 2 hours, and the solvent was removed and the residue was dissolved in DCM (60 mL), washed with water (2 x 20 mL), brine (1 x 20 mL), dried and concentrated to give the final compound **(16b)** (613 mg, 100%) as a white solid. MS calcd for (C₂₄H₃₀N₂O₂)⁺:333.24; MS found (electrospray): 334.24; ¹H NMR (CDCl₃, 400 MHz) major characteristic peaks: δ 0.89 (s, 3H), 1.35 (s, 3H), 2.85 (m, 3H), 4.21 (m, 1H), 4.67 (m, 1H), 4.80 (s, 2H). (m, 2 H), 2.09 (s, 3 H), 1.12 (s, 3 H), 0.87 (s, 3 H).

### Example 15C

### Preparation of Compound (16)

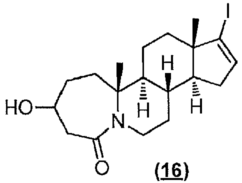

Compound **(16b)** (613 mg, 1.84 mmol) was dissolved in CHCl₃ (10 mL) and benzene (10 mL), then Et₃N (5.2 mL) was added. After that, the solution of iodine (1.19 g, 4.69 mmol) in benzene (10 mL) was added dropwise. The mixture was tirred at 10 °C for 2 hours. Washed with sat.Na₂S₂O₃, diluted with ethyl acetate (60 mL), the organic layer was separated and washed with brine (1 x 20 mL), dried and concentrated to give the compound **(16)** (745 mg, crude) as a light yellow solid. MS calcd for (C₂₄H₃₀N₂O₂)⁺:429.12; MS found (electrospray): 430.12; ¹H NMR (CDCl₃, 400 MHz) major characteristic peaks: δ 0.70 (s, 3H), 1.28 (s, 3H), 2.79 (m, 4H), 4.13 (m, 1H), 4.60 (m, 1H), 6.07 (m, 1H).

### Example 16

### Preparation of synthetic intermediate Compound (17)

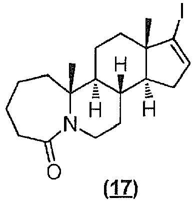

### Example 16A

### Preparation of Compound (17a)

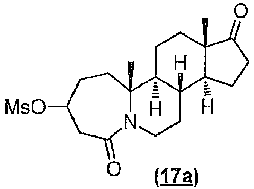

To a solution of compound **(16a)** (3 g, 9.4 mmol) in DCM (100 mL) was added methanesulfonyl chloride (MsCl) (1.5 mL) and pyridine (2.3 mL) at 0 °C. The mixture was stirred at reflux overnight. Water was added to the mixture, extracted with DCM (2 x 200 mL), washed with brine (1 x 100 mL), dried over anhydrous sodium sulphate. The crude product was purified by column chromatography (methanol in dichloromethane, 1% v/v) to give compound **(17a)** (2.90 g, 78%) as a white solid. MS calculate for (C20H31NO5S)⁺: 397; MS found (electrospray): 398. ¹H NMR (CDCl₃, 400 MHz) major characteristic peaks: δ 0.91 (s, 3H), 1.37 (s, 3H), 3.03 (s, 3H), 4.65 (m, 1H), 5.07 (m, 1H).

### Example 16B

### Preparation of Compound (17b)

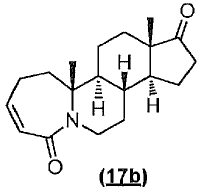

To a solution of compound **(17a)** (2.9 g, 7.3 mmol) in toluene (20 mL) was added 1,8-diazabicyclo[5.4.0]undec-7-ene (DBU) (2.1 mL). The mixture was stirred at reflux for 2 h, then cooled to room temperature, added water, extracted with DCM (2 x 200 mL), washed with water (3 x 100 mL) and brine (1 x 100 mL), dried over Na₂SO₄ giving compound **(17b)** (2.1 g, 95%) as a white solid.. MS calculate for (C19H27NO2)⁺: 301; MS found (electrospray): 302. ¹H NMR (CDCl₃, 400 MHz) major characteristic peaks: δ 0.93 (s, 3H), 1.28 (s, 3H), 4.68 (m, 1H), 6.05 (m, 1H), 6.29 (m, 1H).

### Example 16C

### Preparation of Compound (17c)

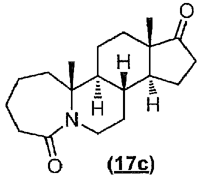

A mixture of compound **(17b)** (2.12 g, 7 mmol) and Pd/C (10%, 200 mg) in methanol (30 mL) was hydrogenated overnight. The reaction mixture was filtered and the excess of methanol was evaporated under reduced pressure to give compound **(17c)** (2 g, 94%) as a white solid. MS calculate for (C19H29NO2)⁺: 303. MS found (electrospray): 304. ¹H NMR (CDCl₃, 400 MHz) major characteristic peaks: δ 0.91 (s, 3H), 1.33 (s, 3H), 2.46 (m, 1H), 2.64 (m, 2H), 2.83 (m, 1H), 4.64 (m, 1H).

### Example 16D

### Preparation of Compound (17d)

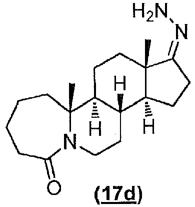

A mixture of compound (**17c**) (2 g, 6.6 mmol) was dissolved in ethanol (20 mL), Et₃N (665 mg, 6.6 mmol) and N₂H₄. H₂O (85%) (2.1 g, 66 mmol) were added. After the addition, the mixture was reflux for 2 hours, and the solvent was removed and the residue was dissolved in DCM (150 mL), washed with water (2 x 100 mL), brine (1 x 100 mL), dried and concentrated to give compound **(17d)** 2 g, 96%) as a white solid. MS calcd for (C₁₉H₃₁N₃O)⁺:317. MS found (electrospray): 318.

### Example 16E

### Preparation of Compound (17)

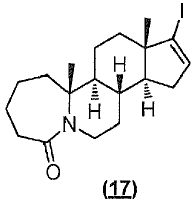

Compound **(17d)** (2 g, 6.3 mmol) was dissolved in CHCl₃ (50 mL) and benzene (50 mL), then Et₃N (1.9 g, 18.9 mmol) was added. After that, the solution of iodine (4 g, 15.7 mmol) in benzene (30 mL) was added dropwise at 0 °C. The mixture was stirred at room temperature for 2 hours. It was washed with sat.Na₂S₂O₃, diluted with ethyl acetate (200 mL). The organic layer was separated and washed with brine (1 x 100 mL), dried and the crude product was purified by column chromatography (methanol in dichloromethane, 1% v/v) to give compound **(17)** (1.6 g, 62%) as a light yellow solid. MS calcd for (C19H28INO)⁺:413. MS found (electrospray): 414. ¹H NMR (CDCl₃, 400 MHz) major characteristic peaks: δ 0.77 (s, 3H), 1.33 (s, 3H), 4.59 (m, 1H), 6.13 (s, 1H).

### Example 17

### Preparation of synthetic intermediate Compound (18)

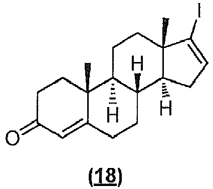

### Example 17A

### Preparation of Compound (18a)

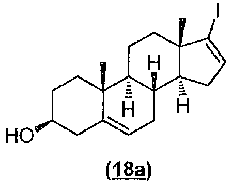

To a solution of compound **(4)** (5.0 g, 11.36 mmol) in methanol (30 mL) and THF (10 mL) was added potassium hydroxide (636 mg, 11.36 mmol) under N₂. The mixture was stirred at 30 °C for 1 hr. The solvent was evaporated, and the residue was extracted with ethyl acetate (ethyl acetate) (100 mL*2), and washed with water, brine and dried over Na₂SO₄. The crude product was recrystallized in propyl ethyl to give compound **(18a)** (4.5 g, 99%) as a white solid. LC-MS (m/z) 381[M-OH]⁺. ¹H-NMR δ (400 MHz, CHCl₃-*d*) major characteristic peaks: 0.76 (s, 3H), 1.04 (s, 3H), 3.53 (m, 1H), 5.36 (m, 1H), 6.14 (s, 1H).

### Example 17B

### Preparation of Compound (18)

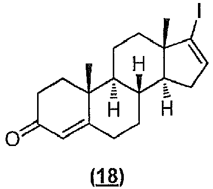

To a solution of compound **(18a)** (4.5 g, 11.3 mmol) in 2-butanone (40 mL) and toluene (10 mL) was added 25 % Al(Oi-Pr)₃ (14.76 g, 18.1 mmol) under N₂. The mixture was stirred and heated to reflux overnight. The mixture was diluted with ethyl acetate (200 mL) and filtrated. The filtrate was evaporated, and the residue was recrystallized by propyl ether to give compound **(18)** (3.1 g, 70%) as a white solid. LC-MS (m/z) 397[M+H]⁺. ¹H-NMR δ (400 MHz, CHCl₃-*d*) major characteristic peaks: 0.78 (s, 3H), 1.21 (s, 3H), 5.75 (s, 1H), 6.14 (m, 1H).

### Example 18

### Preparation of synthetic intermediate Compound (19)

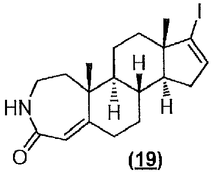

### Example 18A

### Preparation of Compound (19a)

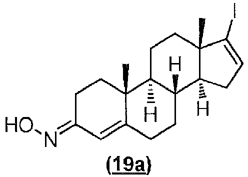

To a solution of compound **(18)** (1.62 g, 4.1 mmol) in ethanol (20 mL) was added pyridine (5 mL) and hydroxylamine hydrochlororide (427 mg, 6.15 mmol) under N₂. The mixture was stirred at room temperature overnight. The mixture was poured into ice-water (100 mL) and stirred 30 min. to give a white precipitate. Filtrated and dried to get the compound **(19a)** (1.68 g, 98%) as a white solid. LC-MS (m/z) 412 [M+H]⁺. ¹H-NMR δ (400 MHz, CHCl₃-*d*) major characteristic peaks: δ 0.77 (s, 3H), 1.09 (s, 3H), 3.05 (m, 1H), 5.78 (s, 1H), 6.13 (m, 1H).

### Example 18B

### Preparation of Compound (19)

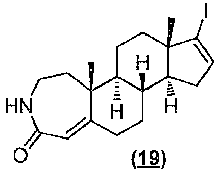

To a solution of compound **(19a)** (1.68 g, 4.08 mmol) in dry THF (10 mL) was added dropwise a solution of SOCl₂ (2 mL) in THF (2 mL) at 0 °C. The mixture was stirred at room temperature overnight. The mixture was poured into ice-water (100 mL) and extracted with DCM (100 mL * 2). It was then washed with water, brine, and dried over Na₂SO₄. Filtered, concentrated to give compound **(19)** (1.68 g, 99%) as a brown solid. LC-MS (m/z) 412 [M+H]^{+ 1}H-NMR δ (400 MHz, CHCl₃-*d*) major characteristic peaks: δ 0.76 (s, 3H), 1.17 (s, 3H), 2.53 (m, 1H), 3.18 (m, 1H), 5.75 (s, 1H), 6.13 (m, 1H), 6.56 (brs, 1H).

### Example 19

### Preparation of synthetic intermediate Compound (20)

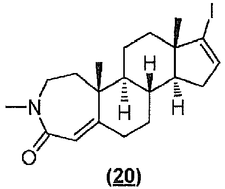

To a solution of compound **(19)** (1.68 g, 4.08 mmol) in dry DMF (20 mL) was added 60 % NaH (245 mg, 6.13 mmol) and iodomethane (697 mg, 4.91 mmol) under N₂ at -30 °C. Then the mixture was warmed to room temperature and stirred overnight. The mixture was quenched with water (20 mL) and extracted with ethyl acetate (100 mL*3), washed with water, brine and dried over Na₂SO₄.After removal of solvent, the residue was purified with silica gel chromatography (ethyl acetate in petroleum, 33% v/v) to give compound (**20**) (875 mg, 50%, three steps) as a yellow solid. LC-MS (m/z) 426 [M+H]⁺.¹H-NMR δ (400 MHz, CHCl₃-*d*) major characteristic peaks: δ 0.76 (s, 3H), 1.20 (s, 3H), 2.49 (m, 1H), 3.04 (s, 3H), 3.21 (m, 1H), 3.41 (m, 1H), 5.83 (s, 1H), 6.12 (m, 1H). 1H).

### Example 20

### Preparation of synthetic intermediate Compound (21)

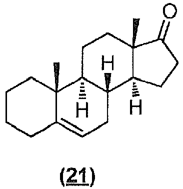

### Example 20A

### Preparation of Compound (20a)

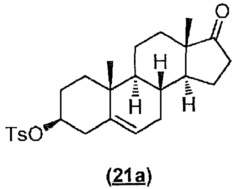

A mixture of (3*S*,8*R*,9*S*,10*R*,13*S*,14*S*)-3-hydroxy-10,13-dimethyl-3,4,7,8,9,10,11,12,13,14,15,16-dodecahydro-1*H*-cyclopenta[*a*]phenanthren-17(2*H*)-one (dehydroisoandrosterone) (10.00 g, 34.7 mmol), TsCl (9.92 g, 52.0 mmol) in pyridine (60 mL) was stirred for overnight at room temperature. Then the mixture was poured into ice-water (500 mL), filtered. The solid was washed with water, dried to get compound (**21a**) (14.6 g, 95%) as a white solid. ¹H NMR (DMSO-*d*₆, 400 MHz) major characteristic peaks: δ 0.78 (s, 3H), 0.95 (s, 3H), 2.42 (s, 3H), 4.21 (m, 1H), 5.30 (m, 1H), 7.47 (d, *J =* 8.0 Hz, 2H), 7.79 (d, *J* = 8.0 Hz, 2H).

### Example 20B

### Preparation of Compound (21b)

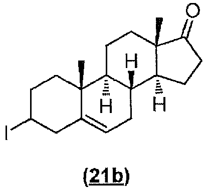

A mixture of compound (**21a**) (14.6 g, 33 mmol), NaI (14.8 g, 99 mmol) in acetone (300 mL) was stirred at 50 °C overnight. The solvent was removed and the residue was dissolved in water (300 mL). It was extracted with dichloromethane (200 mL * 3). The organic layer was washed with water, brine, dried, concentrated to get a crude product. The crude product was purified by column chromatography (ethyl acetate/petroleum ether = 1/15. v/v) to give compound (**21b**) (10.4 g, 80%) as a white solid. ¹H NMR (CDCl₃, 400 MHz) major characteristic peaks: δ 0.88 (s, 3H), 1.07 (s, 3H), 4.04 (m, 1H), 5.36 (m, 1H).

### Example 20C

### Preparation of Compound (21)

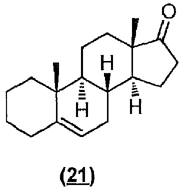

To a solution of compound (**21b**) (398 mg, 1 mmol) in acetic acid (5 mL) was added Zn powder (260 mg, 4 mmol). The mixture was stirred at 80 °C overnight. After being filtered through a pad of Celite, the solvent was evaporated to afford a crude product which was purified by column chromatography (ethyl acetate in petroleum, 10% v/v) to give compound (**21**) (200 mg, 73%) as a yellow solid. ¹H NMR (CDCl₃, 400 MHz) major characteristic peaks: δ 0.81 (s, 3H), 0.95 (s, 3H), 5.23 (m, 1H).

### Example 21

### Preparation of synthetic intermediate Compound (22)

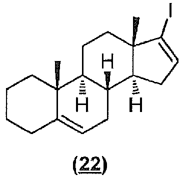

### Example 21A

### Preparation of Compound (22a)

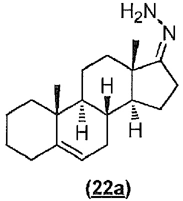

To a solution of compound (**21**) (272 mg, 1 mmol) in EtOH (5 mL) was added NH₂NH₂ (3 mL) and triethylamine (0.2 mL). The mixture was stirred at 80 °C overnight. Evaporated solvent to afford the crude product which was washed with H₂O to give compound (22a) (300 mg, 100%) as a white solid. ¹H NMR (CDCl₃, 400 MHz) major characteristic peaks: δ 0.81 (s, 3H), 0.95 (s, 3H), 4.73 (s, 2H), 5.23 (m, 1H).

### Example 21B

### Preparation of Compound (22)

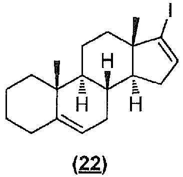

To a solution of compound **(22a)** (283 mg, 1 mmol) in DCM (5 mL) was added Et₃N (1 mL). Then I₂ (510 mg, 2 mmol) in THF (2 mL) was added dropwise. The mixture was stirred at room temperature overnight. The mixture quenched with Na₂SO₃ solution. Evaporated the organic layers to afford the crude product which was purified with column chromatography (petroleum ether) to give compound **(22)** (200 mg, 52%) as a white solid. ¹H NMR (CDCl₃, 400 MHz) major characteristic peaks: δ 0.68 (s, 3H), 0.96 (s, 3H), 5.21 (m, 2H), 6.07 (m, 1H).

### Example 22

### Preparation of synthetic intermediate Compound (23)

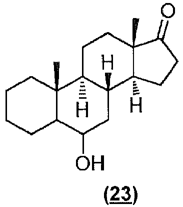

### Example 22A

### Preparation of Compound (23a)

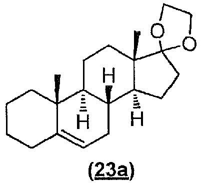

A solution of compound (21) (7.6 g,26.8 mmol), ethane-1,2-diol (3.3 g,53.6 mmol) and TsOH (2.3 g,13.4 mmol) in CH(OEt)₃ (100 ml) was stirred at 80 °C for 2 h. It was then evaporated under reduce pressure to remove the excess solvent. The crude product was purified by column chromatography (ethyl acetate in petroleum ether, 5% v/v) to afford the compound (**23a**) (7.6 g, 86%). ¹H NMR (CDCl₃, 400 MHz) major characteristic peaks: δ 0.79 (s, 3 H), 0.93 (s, 3 H), 3.77-3.85 (m, 4 H), 5.20 (s, 1 H).

### Example 22B

### Preparation of Compound (23b)

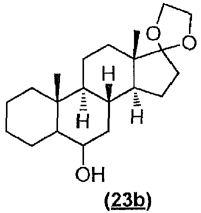

To a solution of compound (**23a**) (7.6 g, 24 mmol) in dry THF (130 mL) under an argon atmosphere at 0 °C wasadded BH₃ (72 mL, 72 mmol,1M in THF) slowly. The mixture was allowed to stir for overnight at room temperature. Aqueous NaOH solution (3 N, 40 mL,120 mmol) was added over 30 min at 0 °C. Subsequently.30% hydrogen peroxide (13.6 mL, 120 mmol) was added and vigorous stirring continued at room temperature for 2 hr. The reaction mixture was poured into ice-water and filtered to give the crude product. Purified by column chromatography (ethyl acetate in petroleum ether, 10% v/v) to afford the compound (**23b**) (3.2 g, 40% yield) as a colorless oil. ¹H NMR (CDCl₃, 400 MHz) major characteristic peaks: δ 0.72 (s, 3 H), 0.77 (s, 3 H), 3.28-3.34 (m, 1 H), 3.77-3.86 (m, 4 H).

### Example 22C

### Preparation of Compound (23)

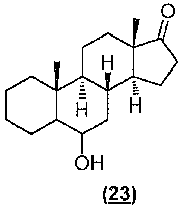

To a solution of compound **(23b)** (2.6 g, 7.8 mmol) in MeOH (30ml) was added concentrated hydrochloric acid (36%, 2 mL). The mixture at 0 °C was stirred for 10 min. It was concentrated in vacuum and the residue was diluted with water (20 mL) and extracted with dichloromethane (30 mL * 3). The organic layer was washed with saturated NaHCO₃ solution (20 mL * 2), brine (30 mL * 1), dried over Na₂SO₄, concentrated to afford the crude product compound (**23**) (2.3 g, 100%) which was used in next step without further purification. ¹H NMR (CDCl₃, 400 MHz) major characteristic peaks: δ 0.82 (s, 3 H), 0.86 (s, 3 H), 2.42-2.48 (m, 1 H), 3.40-3.46 (m, 1 H).

### Example 23

### Preparation of synthetic intermediate Compound (24)

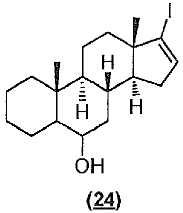

### Example 23A

### Preparation of Compound (24a)

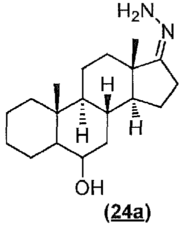

A solution of compound (**23**) (2.3 g, 8 mmol), N2H4.H2O (4 g, 80 mmol) and Et3N (0.81 g, 8 mmol) in EtOH (50 mL) was heated to reflux for 2 hr. The excess solvent was removed under reduce pressure. Then the residue was diluted with water (30 mL) and extracted with dichloromethane (30 mL * 3). The organic layer was washed with water (20 mL * 2), brine and dried over Na₂SO₄, and concentrated to afford a product compound (**24a**) (2.2 g, 91.6%). MS calcd for (C19H32N2O)⁺: 304.25; MS found (electrospray):

### Example 23B

### Preparation of Compound (24)

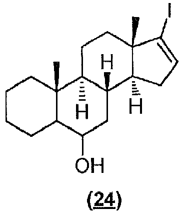

To a solution of compound (24a) (0.83 g, 2.7 mmol), Et₃N (1.5 g, 15.3 mmol) in CHCl₃ (20 ml) was added dropwise a solution of I₂ (2.9 g, 11.5 mmol) in THF (20 mL) 0 °C. The mixture was stirred for 1 hr at this temperature. Saturated aqueous Na₂S₂O₃ solution (20 mL) was added to the mixture and extracted with dichloromethane (30 mL * 3). The organic layer was washed with water (20 mL * 2), brine and dried over Na₂SO₄, concentrated to afford the crude product. The crude product was purified by column chromatography (petroleum ether) to afford the compound **(24)** (1.5 g, 66.6%). ¹H NMR (CDCl₃, 400 MHz) major characteristic peaks: δ 0.72 (s, 3H), 0.82 (s, 3H), 3.40-3.45 (m, 1H), 6.12-6.13 (m, 1H).

### Example 24

### Preparation of synthetic intermediate Compound (25)

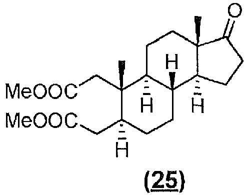

### Example 24A

### Preparation of Compound (25a)

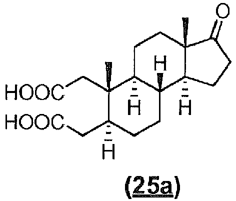

Epiandrosterone (50 g, 0.1722 mol) was dissolved in glacial acetic acid (250 mL) and heated to 75°C. To the resulting solution was added dropwise with a solution of CrO₃ (23 g) in H₂O (160 mL) and sulfuric acid (34 mL) at this temperature. After addition, the mixture was heated to 90-100 °C and stirred for additional 2 h. The reaction was monitored by TLC analysis (Hexane/EtOAc=3/1, 5% HOAc). The mixture was cooled to room temperature with ice water bath and extracted with EtOAc (2X 500 mL). The combined organic solution was concentrated to dryness in vacuo. The residue was then dissolved in 5N aqueous NaOH (1.5 L) and washed with t-butyl methyl ether (2X 500 mL). The aqueous solution was acidified to PH 2 with 10N aqueous HCl. The product was extracted with t-butyl methyl ether (2X 500 mL). The combined organic layers were washed with water and brine, dried over anhydrous Na₂SO₄, filtered, and evaporated in vacuo. The residue was purified by column chromatograph (silica gel) to afford 33.6 g (58%) of compound (**25a**) as a white powder. ¹H NMR (DMSO 300MHz): 11.6-11.9(2H), 2.2-2.3(2H), 1.9-2.2(3H), 1.75-1.85(2H), 0.85-1.0(6H). ¹³C NMR (DMSO, 300MHz): 174.31, 172.35, 50.70, 47.93, 46.75, 35.36, 34.32, 15.35, 13.30

### Example 24B

### Preparation of Compound (25)

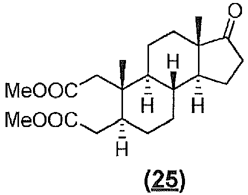

Compound (**25a**) (5 g) was dissolved in methanol (75 mL) and treated with SOCl₂ (8.8 g) at 5°C. After addition, the mixture was heated at reflux for 2 h and all the starting material was consumed by TLC analysis (Hexane/EtOAc=3/1, 5% HOAc). The volatile solvent was removed under reduce pressure. The residue was then dissolved in t-butyl methyl ether (50 mL), washed with diluted NaHCO₃ (2X 25 mL) and brine (25 mL), dried over anhydrous Na₂SO₄, and evaporated to afforded 4.8 g (88.6%) of compound (**25**) as a white powder. ¹H NMR (CDCl₃, 300MHz): 3.3.6-3.7(6H), 3.6-3.65(1H), 2.3-2.45(2H), 2.2-2.25(1H), 0.75-0.85(6H).

### Example 25

### Preparation of synthetic intermediate Compound (26)

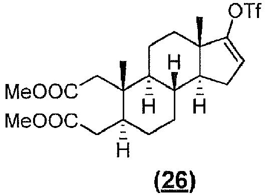

To a solution of compound (**25**) (3.2 g) in dichloromethane (30 mL) was added dropwise with Tf₂O (3.7 g) at 5°C, followed by addition of Et₃N (0.8 g). The mixture was warmed to room temperature and stirred overnight. The reaction was monitored by TLC analysis (hexane: EtOAc=3:1) and all the starting material was consumed. The reaction was quenched with water (20 mL). The mixture was extracted with dichloromethane (2X 20 mL). The combined organic layers were washed brine (20 mL), dried over anhydrous Na₂SO₄, and evaporated to dyness. The residue was purified by column chromatograph (silica gel) to give 4.1 g (94%) of compound (**26**). ¹H NMR (CDCl₃, 300MHz): 5.55(1H), 3.3.6-3.7(6H), 3.6-3.65(1H), 3.3-3.4(1H), 2.1-2.3(2H), 0.95(3H), 0.85(3H). ¹³C NMR (CDCl3, 300MHz): 173.74, 171.36, 159.10, 120.13, 116.94, 114.38, 53.93, 14.03, 13.90.

### Example 26

### Preparation of synthetic intermediate Compound (27)

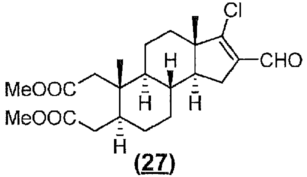

A solution of compound (**25**) (785 mg, 2.0 mmol) in 10 mL of chloroform was treated with a mixture of DMF-POCl₃-CHCl₃ (volume: 3.35 mL-3.35 mL-3.35 mL) within an ice-bath. The reaction was heated at reflux overnight. After being cooled to rt, the mixture was poured into 15 mL of ice-water. Extracted with chloroform, washed with 10% citric acid, 10% sodium biscarbonte and saturated sodium chloride, dried on magnesium sulfate, and condensed by rotary evaporator to give the crude product. Purification by FCC on silica gel provided 560 mg of compound (**27**) in 64% yield.

### Example 27

### Prepared of synthetic intermediate Compound (28)

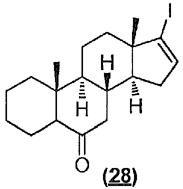

A mixture of compound **(24)** (550mg, 1.8mmol), pyridium chlorochromate (781mg, 3.6mmol) and BaCO₃ (1.07g, 5.4mmol) was stirred in DCM (50 mL) at 40°C for 2 h. It was then cooled to room temperature, diluted with DCM, and filtered. The organic phase was washed by water, brine, and dried over Na₂SO₄. The concentrated crude product was purified by flash column chromatography (petroleum ether:ethyl acetate =50:1, v/v) to give compound (**28**) (460 mg, 70%) as a white solid. ¹H NMR (DMSO, 400 MHz) major characteristic peaks: δ 0.66 (s, 3H), 0.67 (s, 3H), 6.15 (m, 1H).

### Example 28

### Prepared of synthetic intermediate Compound (29)

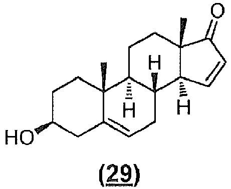

### Example 28A

### Preparation of Compound (29a)

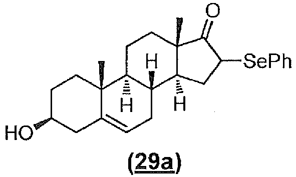

n-Butyl lithium in hexane (8.6 mL, 13.7 mmol) was added, under a nitrogen atomosphere to a cold (-78 °C) solution of dry THF (250 mL) and diisopropylamine (2 mL, 13.6 mmol). A solution of of (3*S*,8*R*,9*S*,10*R*,13*S*,14*S*)-3-hydroxy-10,13-dimethyl-3,4,7,8,9,10,11,12,13,14,15,16-dodecahydro-1*H*-cyclopenta[*a*]phenanthren-17(2*H*)-one (dehydroisoandrosterone) (1.8 g, 6.25 mmol) in dry THF (30 mL) was added and the reaction mixture was left 45 min at -78 °C, before the dropwise addition of a THF solution (20 mL) of benzeneselenenyl bromide (1.6 g, 6.8 mmol). After a further 10 min the mixture was poured into hydrochloric acid (40 mL, 1M) and the mixture was extracted with ethyl acetate (100 mL*2). The organic phase washed with sodium bicarbonate and water then dried over Na₂SO₄. The crude was purified by column chromatography (33%, ethyl acetate in petroleum, v/v) to give compound (**29a**) (2.3 g, 82%) as a white foam. LC-MS (m/z) 445 [M+H]⁺. ¹H-NMR δ (400 MHz, CHCl₃-*d*) major characteristic peaks: δ 0.93 (s, 3H), 1.04 (s, 3H), 3.52 (m, 1H), 5.38 (s, 1H), 7.30 (m, 3H), 7.65 (m, 2H).

### Example 28B

### Preparation of Compound (29)

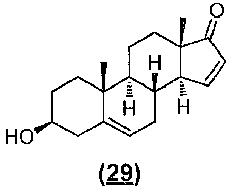

m- Chloroperbenzoic acid (962 mg, 5.6 mmol) was added to a cold (-40 °C) solution of the compound (**29a**) (2.07 g, 4.66 mmol) in dry DCM (30 mL) and the mixture was stirred at -40 °C for 1 h. Diethylamine (2 mL) was added and the reaction mixture was transferred to a refluxing solution of CCl₄(100 mL) and diethylamine(10 mL). After 15 min. the solution was cooled to room temperature, washed with hydrochloric acid (60 mL, 1M), aq.NaHCO₃, water, brine, and dried over Na₂SO₄. The crude was purified by column chromatography (ethyl acetate in petroleum, 33% v/v) to give compound (**29**) (665 mg, 50%) as a yellow solid. LC-MS (m/z) 287[M+H]⁺. ¹H-NMR δ (400 MHz, CHCl₃-*d*) major characteristic peaks: δ 1.10 (s, 6H), 3.56 (m, 1H), 5.43 (s, 1H), 6.06 (m, 1H), 7.52 (m, 1H).

### Example 29

### Preparation of synthetic intermediate Compound (30)

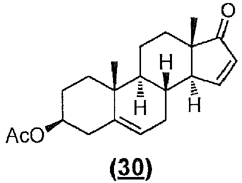

To a solution of compound (**29**) (665 mg, 2.33 mmol) in DCM (10 mL) was added 2 drops of BF₃-Et₂O and followed by the addition of acetic anhydride (308 mg, 3.02 mmol). The mixture was stirred at room temperature for 2h until no starting material left. To the mixture was added water, extracted with DCM (3 x 50 mL). The organic layers were combined, washed with aq.NaHCO₃, brine, and dried over Na₂SO₄. The crude was purified by column chromatography (ethyl acetate in petroleum, 10% v/v) to give compound (**30**) (370 mg, 49%) as a yellow solid. LC-MS (m/z) 269 [M-OAc]⁺. ¹H-NMR δ (400 MHz, CHCl₃-*d*) major characteristic peaks: δ 1.08 (s, 3H), 1.10 (s, 3H), 2.04 (s, 3H), 4.61 (m, 1H), 5.43 (s, 1H), 6.06 (m, 1H), 7.50 (m, 1H).

### Example 30

### Preparation of synthetic intermediate Compound (31)

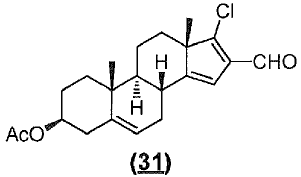

DMF (1.35 mL, 17.5 mmol) was added into a cold solution of phosphorus oxychloride (1.35 mL, 15 mmol) in chloroform (5 mL) in ice/water bath and then followed by the addition dropwise of a solution of compound (**30**) (270 mg, 0.82 mmol) in chloroform (5 mL). After the addition, the mixture was allowed to warm up to room temperature and then heated to 40 °C under N₂ for 2 hours. It was then concentrated under reduced pressure and poured onto ice-water (100 mL). It was then extracted with ethyl acetate (50 mL * 2), washed with brine and dried over Na₂SO₄. The crude was purified by column chromatography (ethyl acetate in petroleum, 5% v/v) to give compound (**31**) (211 mg, 69%) as a yellow solid. LC-MS (m/z) 375 [M+H]⁺. ¹H-NMR δ (400 MHz, CHCl₃-*d*) major characteristic peaks: δ 1.13 (s, 3H), 1.19 (s, 3H), 2.04 (s, 3H), 4.61(m, 1H), 5.48 (s, 1H), 6.23 (s, 1H), 10.01 (s, 1H).

### Example 31

### Preparation of synthetic intermediate Compound (32)

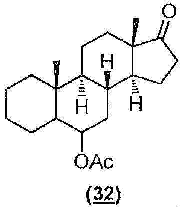

To a solution of compound (**23**) (6.7 g, 23.1 mmol) in THF (100 mL) was added acetic anhydride (8 mL), triethylamine (13 mL) and dimethylaminopyridine (280 mg, 2.3 mmol), stirred at 50 °C for 2 h.The solution was cooled and concentrated. Water was added, and then extracted with ethyl acetate (50 mL * 3). The combined organic phase was washed by water, brine and dried over Na₂SO₄, filtered, and concentrated to give the compound (**32**) (7.7 g, 100%) as a yellow solid. The product was used without further purification. ¹H NMR (CDCl₃, 400 MHz) major characteristic peaks: δ 0.78 (s, 3H), 0.80 (s, 3H), 1.97 (s, 3H), 4.64 (m, 1H).

### Example 32

### Preparation of synthetic intermediate Compound (33)

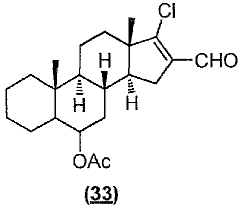

Phosphorus oxychloride (13.8 g, 90.2 mmol) and DMF (1.3 g, 18.0 mmol) was stirred together for 15 minutes at room temperature. Compound (**32**) (3.0 g, 9.0 mmol)was added at one portion, and then it was heated to 90 °C for 1 h.. The reaction was cooled down, and concentrated and dissolved with ethyl acetate (200 mL). The organic mixture was poured into ice-water and the organic phase was separated, and washed with water (200 mL * 2), brine and dried over Na₂SO₄, and concentrated to afford the crude compound (**33**) (7.8 g, 82 %) as a yellow solid. The product was used in next step without further purification. ¹H NMR (CDCl₃, 400 MHz) major characteristic peaks: δ 0.88 (s, 3H), 0.95 (s, 3H), 2.03 (s, 3H), 4.69 (m, 1H), 9.97 (s, 1H).

### Example 33

### Preparation of Compound (34) and Compound (35)

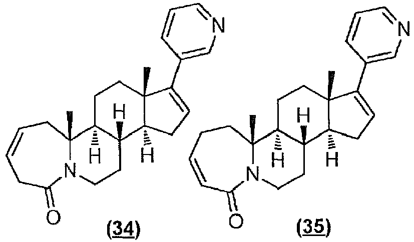

### Example 33A

### Preparation of Compound (34a) and Compounds (35)

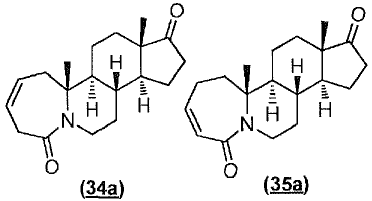

To a solution of compound (**15**) (760 mg, 2.1 mmol) in dichloromethane (10 mL) was added trifluoromethane sulfonic anhydride (830 mg, 2.9 mmol) at room temperature. The solution was stirred over 10 min and triethylamine (275 mg, 2.7 mmol) in dichloromethane (2 mL) was added dropwise within 10 min. The mixture was stirred for 18 h and all the starting material was consumed by TLC analysis. Water (20 mL) was added and the layers were separated. The aqueous layer was extracted with dichloromethane (3x20 mL). The combined organic layers were washed with water (15 mL) and brine (15 mL), dried over Na₂SO₄ and evaporated to dryness. The residue was purified by column chromatography on silica gel (hexanes/ ethyl acetate =2/1) to give a mixture of compound (**34a**) and compound (**35a**) (200 mg, yield: 22%). MS calcd for (C₂₈H₄₉O₅Si)⁺: 433.2;MS found (electrospray):434.2 .

### Example 33B

### Preparation of Compound (34) and Compound (35)

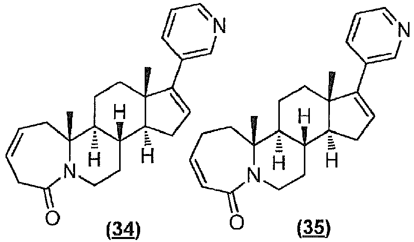

To a solution of the mixture of compound **(34a)** and compound (**35a** (200 mg, 0.46 mmol) in tetrahydrofuran (8 mL) was added pyridin-3-ylboronic acid (96 mg, 0.78 mmol), potassium carbonate (285 mg, 2.07 mmol) in water (1 mL), and Pd(dppf)Cl₂ (20 mg). The mixture was thoroughly degassed, and heated under nitrogen at 80 °C for 2 hours. After being filtered through a pad of Celite, the crude product was purified by pre-TLC (hexanes/ethyl acetate =1/1, v/v) to give compound (**34**) (35 mg, yield: 21%) and compound (**35**) (30 mg, yield: 18%). Compound (**34**): MS calcd for (C₂₈H₄₉O₅Si)⁺: 362.2; MS found (electrospray):363.2. ¹H NMR (CDCl₃, 400 MHz) major characteristic peaks: δ 8.61 (m, 1 H), 8.47 (m, 1 H), 7.64 (m, 1 H), 7.22 (m, 1 H), 6.01 (m, 2 H), 5.92 (m, 1 H), 4.76 (m, 1 H), 3.41 (m, 1 H), 3.24 (m, 1 H), 1.39 (s, 3 H), 1.03 (s, 3 H). Compound (**35**): MS calcd for (C₂₈H₄₉O₅Si)⁺: 362.2; MS found (electrospray):363.2. ¹H NMR (CDCl₃, 400 MHz) major characteristic peaks: δ 8.61 (m, 1 H), 8.47 (m, 1 H), 7.64 (m, 1 H), 7.22 (m, 1 H), 6.30 (m, 1 H), 6.05 (m, 1 H), 6.00 (m, 1 H), 4.69 (m, 1 H), 2.94 (m, 1 H), 1.30 (s, 3 H), 1.07 (s, 3 H).

### Example 34

### Preparation of Compound (36)

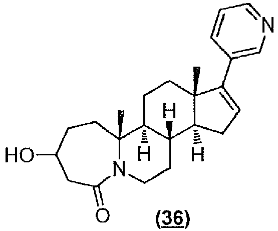

To a solution of compound (**16**) (150 mg, 0.35 mmol) in THF (20 mL) was added pyridin-3-ylboronic acid (86 mg, 0.70 mmol), potassium carbonate (241 mg, 1.75mmol) in water (2 mL), and bis(triphenylphosphine) palladium chloride (20 mg). The mixture was thoroughly degassed, and heated under nitrogen at 80 °C for 2 hour. After being filtered through a pad of Celite, the crude product was purified with pre-TLC (methanol in dichloromethane, 5% v/v) to give compound (**36**) (4 mg, 3% yield). MS calculate for (C₂₄H₃₂N₂O₂)⁺: 380.52; MS found (electrospray): 381.3; ¹H NMR (CDCl₃, 400 MHz) major characteristic peaks: δ 1.05 (s, 3H), 1.38 (s, 3H), 2.89 (m, 3H), 4.22 (m, 1H), 4.69 (d, J=13.6 Hz, 1H), 6.01 (s, 1H), 7.25(s, 1H), 7.67(d, J = 7.6 Hz, 1 H), 8.48 (s, 1 H), 8.61 (s, 1H).

### Example 35

### Prepared of Compound (37)

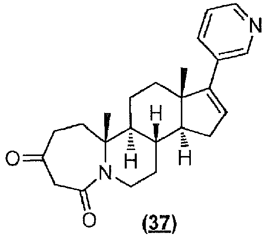

### Example 35A

### Preparation of Compound (37a)

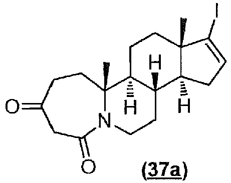

The mixture of compound (**16**) (549 mg, 1.29 mmol), NMO (225 mg, 1.92 mmol), (n-Pr)₄NRuO₄ (22 mg, 0.06 mmol) in DCM (20 mL) was stirred at 25 °C for 16 hours. Diluted with DCM (60 mL), washed with water (1 x 20 mL) and brine (1 x 20 mL), dried and concentrated to give the crude product which purified by column chromatography (DCM/MeOH = 50/1, v/v) to get compound (**37a**) (138 mg, 25%) as a light yellow solid. MS calcd for (C₂₄H₃₀N₂O₂)⁺:427.10; MS found (electrospray): 428.10; ¹H NMR (CDCl₃, 400 MHz) major characteristic peaks: δ 0.70 (s, 3H), 1.35 (s, 3H), 2.79 (m, 1H), 3.77 (m, 2H), 4.53 (s, 1H), 6.07 (m, 1H).

### Example 35B

### Preparation of Compound (37)

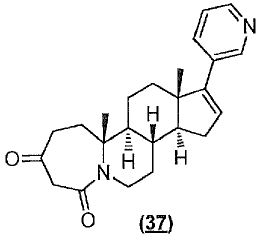

The mixture of compound (**37a**) (130 mg, 0.30 mmol), pyridin-3-ylboronic acid (64 mg, 0.52 mmol), Pd(dppf)Cl₂ (25 mg, 0.03 mmol), K₂CO₃ (186 mg, 1.35 mmol) in THF (6 mL), water (1 mL) was stirred at 80 °C for 4 hours. Then THF was removed and the residue was dissolved in DCM (60 mL) washed with water (1 x 20 mL) and brine (1 x 20 mL), dried and concentrated to give the crude product which purified by Prep-TLC (ethyl acetate as eluent) to get compound (**37**) (7 mg, 6%) as a white solid. MS calcd for (C₂₄H₃₀N₂O₂)⁺:378.23; MS found (electrospray): 379.23; ¹H NMR (CDCl₃, 400 MHz) major characteristic peaks: δ 0.99 (s, 3H), 1.38 (s, 3H), 2.83 (m, 1H), 3.77 (m, 2H), 4.56 (m, 1H), 5.94 (m, 1H), 7.19 (m, 1H), 7.58 (m, 1H), 8.41 (m, 1H), 8.54 (m, 1H).

### Example 36

### Preparation of Compound (38)

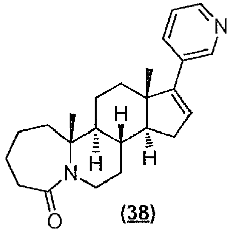

To a solution of compound **(17)** (400 mg, 0.97 mmol) in THF (20 mL) was added pyridin-3-ylboronic acid (238 mg, 1.94 mmol), potassium carbonate (668 mg, 4.8 mmol) in water (2 mL), and bis(triphenylphosphine) palladium chloride (30 mg). The mixture was thoroughly degassed, and heated under nitrogen at 80 °C for 2 hour. After being filtered through a pad of Celite, the crude product was purified by pre-TLC (methanol in dichloromethane, 5% v/v) to give compound (**38**) (69 mg, 19%). MS calculate for (C₂₄H₃₂N₂O₂)⁺: 364; MS found (electrospray): 365;¹H NMR (CDCl₃, 400 MHz) major characteristic peaks: δ 1.05 (s, 3H), 1.35 (s, 3H), 2.63 (m, 2H), 2.87 (m, 1H), 4.63 (m, 1H), 5.99 (s, 1H), 7.22 (m, 1H), 7.63 (d, J= 8.0 Hz, 1 H), 8.45 (d, J = 4.4 Hz, 1 H), 8.61 (s, 1H).

### Example 37

### Preparation of Compound (39)

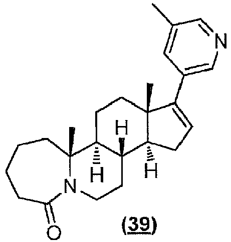

To a solution of compound **(17)** (400 mg, 0.97 mmol) in THF (20 mL) was added 5-methylpyridin-3-ylboronic acid (266 mg, 1.94 mmol), potassium carbonate (668 mg, 4.8 mmol) in water (2 mL), and bis(triphenylphosphine) palladium chloride (30 mg). The mixture was thoroughly degassed, and heated under nitrogen at 80 °C for overnight. After being filtered through a pad of Celite, the crude product was purified by pre-TLC (methanol in dichloromethane, 5% v/v) to give compound (**39**) (69 mg, 18%). MS calculate for (C25H34N2O)⁺: 378; MS found (electrospray): 379; ¹H NMR (CDCl₃, 400 MHz) major characteristic peaks: δ 1.04 (s, 3H), 1.35 (s, 3H), 2.33 (s, 3H), 2.87 (t, J = 14 Hz, 1H), 4.62 (d, J = 14 Hz, 1H), 5.97 (s, 1H), 7.45 (s, 1H), 8.30 (s, 1 H), 8.41 (s, 1H).

### Example 38

### Preparation of Compound (40)

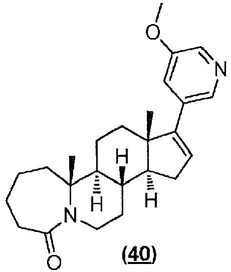

To a solution of compound **(17)** (400 mg, 0.97 mmol) in THF (20 mL) was added 5-methoxypyridin-3-ylboronic acid (296 mg, 1.94 mmol), potassium carbonate (668 mg, 4.8 mmol) in water (2 mL), and bis(triphenylphosphine) palladium chloride (30 mg). The mixture was thoroughly degassed, and heated under nitrogen at 80 °C for 2 hour. After being filtered through a pad of Celite, the crude product was purified with pre-TLC (methanol in dichloromethane, 5% v/v) to give compound (**40**) (68 mg, 18%). MS calculate for (C₂₄H₃₂N₂O₂)⁺: 394; MS found (electrospray): 395;¹H NMR (CDCl₃, 400 MHz) major characteristic peaks: δ 1.05 (s, 3H), 1.35 (s, 3H), 2.64 (m, 2H), 2.86 (m, 1H), 3.86 (s, 3H), 4.63 (m, 1H), 6.00 (s, 1H), 7.14 (s, 1H), 8.18 (s, 1 H), 8.23 (s, 1H).

### Example 39

### Preparation of Compound (41)

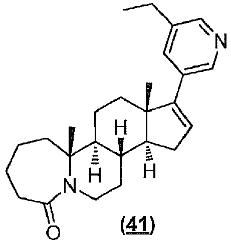

To a solution of compound **(17)** (400 mg, 0.97 mmol) in THF (20 mL) was added 5-ethylpyridin-3-ylboronic acid (293 mg, 1.94 mmol), potassium carbonate (668 mg, 4.8 mmol) in water (2 mL), and bis(triphenylphosphine) palladium chloride (30 mg). The mixture was thoroughly degassed, and heated under nitrogen at 80 °C for overnight. After being filtered through a pad of Celite, the crude product was purified with pre-TLC (methanol in dichloromethane, 5% v/v), then further purified by HPLC to give compound (**41**) (28 mg, 7%). MS calculate for (C26H36N2O)⁺: 392; MS found (electrospray): 393; ¹H NMR (CDCl₃, 400 MHz) major characteristic peaks: δ 1.04 (s, 3H), 1.26 (t, J= 8.0 Hz, 3H), 1.35 (s, 3H), 2.63 (m, 4H), 2.87 (t, *J* = 14 Hz, 1H), 4.62 (d, J = 14 Hz, 1H), 5.98 (s, 1H), 7.46 (s, 1H), 8.32 (s, 1 H), 8.42 (s, 1H).

### Example 40

### Preparation of Compound (42)

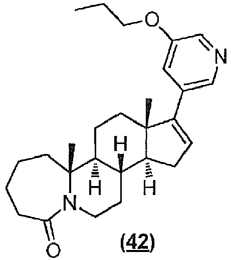

To the solution of compound **(17)** (400 mg, 0.97 mmol) in 1, 4-dioxane (20 mL) was added 5-propoxypyridin-3-ylboronic acid (350 mg, 1.94 mmol), PdCl₂(dppf) (80 mg, 0.097 mmol), potassium carbonate (535 mg, 3.88 mmol) and water (4 mL). The reaction was stirred and heated to 80 °C for 3 h. The reaction was cooled to room temperature and partitioned with ethyl acetate (50 mL) and water (50 mL). The layers were separated and the aqueous layer was extracted with ethyl acetate (50 mL). The combined organic layers was washed with brine (100 mL) and dried over Na₂SO₄ and concentrated. The crude product was purified by column chromatogram (ethyl acetate / petroleum ether = 1:10, then ethyl acetate) to get compound compound (**42**) (185 mg, 44%) as a yellow solid. LCMS: (M+H)⁺= 423; ¹HNMR (CDCl₃, 400 MHz) major characteristic peaks: δ 1.03-1.07 (m, 6H), 1.35 (s, 3H), 2.62-2.66 (m, 2H), 2.87 (t, J = 13.6 Hz, 1H), 3.97 (t, *J* = 6.4 Hz, 2H), 4.61-4.65 (m, 1H), 5.99 (s, 1H), 7.14 (s, 1H), 8.17 (s, 1H), 8.21 (s, 1H).

### Example 41

### Preparation of Compound (43)

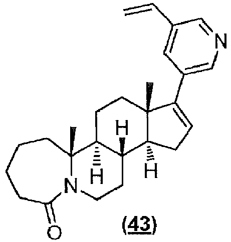

To a solution of compound (**17**) (400 mg, 0.97 mmol) in THF (20 mL) was added 5-vinylpyridin-3-ylboronic acid (289 mg, 1.94 mmol), potassium carbonate (668 mg, 4.8 mmol) in water (2 mL), and bis(triphenylphosphine) palladium chloride (30 mg). The mixture was thoroughly degassed, and heated under nitrogen at 80 °C for 2 hour. After being filtered through a pad of Celite, the crude product was purified by pre-TLC (methanol in dichloromethane, 5% v/v) to give compound (**43**) (54 mg, 14%). MS calculate for (C₂₆H₃₄N₂O)⁺: 390; MS found (electrospray): 391;¹H NMR (CDCl₃, 400 MHz) major characteristic peaks: δ 1.05 (s, 3H), 1.35 (s, 3H), 2.64 (m, 2H), 2.87 (m, 1H), 4.63 (m, 1H), 5.38 (d, J = 11.2 Hz, 1H), 5.82 (d, *J* = 17.6 Hz, 1H), 6.00 (s, 1H), 6.69 (dd, *J* ₁,₂= 11.2, 17.6 Hz, 1H),7.65 (s, 1H), 8.48 (s, 1 H).

### Example 42

### Preparation of Compound (44)

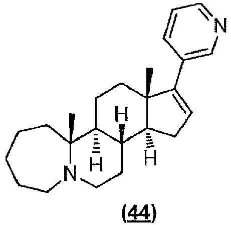

To a solution of compound (**38**) (100 mg, 0.275 mmol) in THF (3 mL) was added LiAlH₄ (21 mg, 0.549 mmol) at 25 °C, then the mixture was heated to 45 °C and stirred for 1 h. After the reaction completed, the mixture was quenched with water (0.02 mL), aqueous NaOH solution (15%, 0.02 mL) and water (0.06 mL) at ice bath, filtered and the solution was concentrated, purified by Pre-HPLC to obtain compound (**44**) (60 mg, 62%). MS calculate for (C₂₄H₃₂N₂O₂)⁺: 350; MS found (electrospray): 351; ¹H NMR (CDCl₃, 400 MHz) major characteristic peaks: δ 1.0 (s, 3H), 1.03 (s, 3H), 2.03 (m, 2H), 2.24 (m, 1H), 2.62 (m, 2H), 3.00 (t, J = 13.2 Hz, 1H), 6.00 (s, 1H), 7.20 (m, 1H), 7.63 (d, J = 7.6 Hz, 1H), 8.45 (s, 1H), 8.61 (s, 1H).

### Example 43

### Preparation of Compound (45)

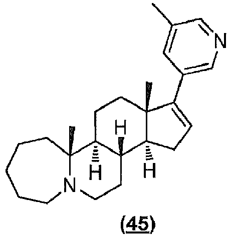

To a solution of compound (**39**) (160 mg, 0.423 mmol) in THF (5 mL) was added AlLiH₄ (32 mg, 0.846 mmol) at 25 °C, then the mixture was heated to 45 °C and stirred for 1 h. After the reaction completed, the mixture was quenched with water (0.03 mL), solution of NaOH (0.03 mL, 1.5%) and water (0.09 mL) at ice bath, filtered and the solution was purified by Pre-HPLC to obtain compound (**45**) (100 mg, 66%). MS calculate for (C₂₄H₃₂N₂O₂)⁺: 364; MS found (electrospray): 365; ¹H NMR (CDCl₃, 400 MHz) major characteristic peaks: δ 1.01 (s, 3H), 1.02 (s, 3H), 2.01 (m, 2H), 2.23 (m, 1H), 2.32 (s, 3H), 2.62 (m, 2H), 3.03 (t, J= 13.2 Hz, 1H), 5.95 (s, 1H), 7.44 (s, 1H), 8.29 (s, 1H), 8.41 (s, 1H).

### Example 44

### Preparation of Compound (46)

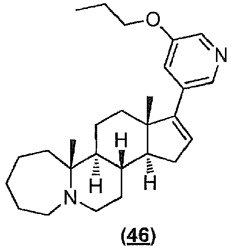

To a solution of compound (**42**) (150 mg, 0.355 mmol) in dry THF (10 mL) was added LiAlH₄ (30 mg, 0.711 mmol) at 25 °C, then the mixture was heated to 45 °C and stirred for 1 h. After the reaction completed, the mixture was quenched with water (0.03 mL), solution of NaOH (0.03 mL, 15%) and water (0.06 mL) at ice bath, filtered and the solution was purified by Pre-TLC (DCM/Methanol = 20:1, include 1% NH₃.H₂O) to obtain compound (**46**) (60 mg, 42%). LCMS: (M+H)⁺= 409; ¹H NMR (CDCl₃, 400 MHz) major characteristic peaks: δ 1.01-1.07 (m, 9H), 1.80-1.85 (m, 2H), 2.20-2.26 (m, 1H), 3.04 (t, J = 12.4 Hz, 1H), 3.96 (t, *J* = 6.8 Hz, 2H), 5.98 (s, 1H), 7.14 (s, 1H), 8.16 (d, J = 2.4 Hz, 1H), 8.22 (s, 1H).

### Example 45

### Preparation of Compound (47)

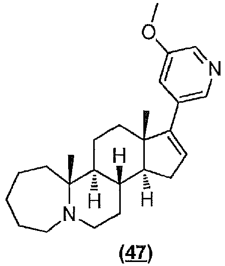

To a solution of compound (**40**) (400 mg, 1.01 mmol) in THF (8 mL) was added AlLiH₄ (77 mg, 2.03 mmol) at room temperature, then the mixture was stirred at 45°C for 1h. The mixture was cooled to room temperature and to the mixture was added water (0.08 mL), 15% NaOH (0.08 mL), water (0.24 mL) .After being filtered through a pad of Celite, the crude product was purified by prep-HPLC to give compound (**47**) (40 mg, 10%). MS calculate for (C₂₅H₃₆N₂O)⁺: 380.57; MS found (electrospray): 381; ¹H NMR (CDCl₃, 400 MHz) major characteristic peaks: δ 1.01-1.03 (m, 6H), 2.01 (m, 2H), 2.24 (m, 1H), 2.61 (m, 3H), 3.04 (m, 1H), 3.86 (s, 3H), 5.99 (s, 1H), 7.15 (s, 1H), 8.17(s, 1H), 8.24 (s, 1H).

### Example 46

### Preparation of Compound (48)

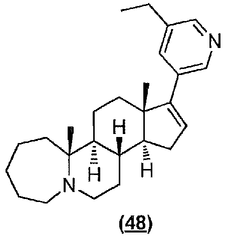

To a solution of compound (**41**) (300 mg, 0.76 mmol) in THF (8 mL) was added AlLiH₄ (58 mg, 1.5 mmol) at room temperature. The mixture was then stirred at 45°C for 1h, and then the mixture was cooled to room temperature. To the mixture was added water (0.06 mL), 15% NaOH (0.06 mL), water (0.18 mL) .After being filtered through a pad of Celite, the crude product was purified by prep-HPLC to give compound (**48**) (22 mg, 7.6%). MS calculate for (C₂₆H₃₈N₂)⁺: 378.59; MS found (electrospray): 379; ¹H NMR (CDCl₃, 400 MHz) major characteristic peaks: δ 0.94-0.98 (m, 6H), 1.18 (m, 5H), 1.95 (m, 2H), 2.16 (m, 1H), 2.57 (m, 5H), 3.98 (m, 1H), 5.89 (s, 1H), 7.39 (s, 1H), 8.24(s, 1H), 8.36 (s, 1H).

### Example 47

### Prepared of Compound (49)

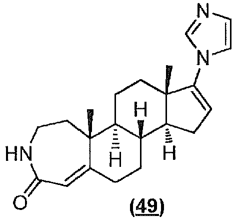

### Example 47A

### Preparation of Compound (49a)

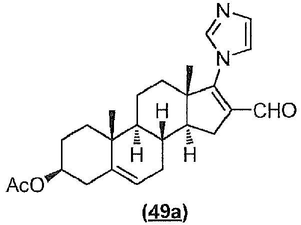

To a solution of compound (**2**) (5.0 g, 13.3 mmol) in DMF (30 mL) were added K₂CO₃ (5.5 g, 40 mmol) and imidazole (1.09 g, 16 mmol). The mixture was stirred and heated to 80 °C for 3 hours. The mixture was cooled to room temperature, and evaporated in vacuum .The residue was extracted with ethyl acetate (100 mL*3), washed with water, brine and dried over Na₂SO₄. After removal of solvent, the residue was purified by silica gel chromatography (33 %, ethyl acetate in petroleum, v/v) to give compound **(49a)** (2.87 g, 53% yield) as a white solid. LC-MS (m/z) 409 [M+H]⁺. ¹H-NMR (400 MHz, CHCl₃-*d* major characteristic peaks: δ 1.08 (m, 6H), 2.04 (s, 3H), 2.70 (dd, J = 14.4 Hz, J = 5.6 Hz, 1H), 4.61 (m, 1H), 5.42 (m, 1H), 7.12 (s, 1H), 7.24 (s, 1H), 7.66 (s, 1H), 9.74 (s, 1H).

### Example 47B

### Preparation of Compound (49b)

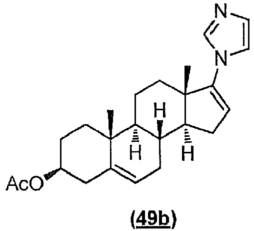

To a solution of compound (**49a**) (100 mg, 0.25 mmol) in dry PhCN (4 mL) was added 10 % Pd/C (50 mg) under N₂. The mixture was stirred and heated to reflux for 3.5 hrs. After cooling to room temperature, the catalyst was removed by filtration through a Celite pad. The filtrate was evaporated, and the residue was purified by pre-TLC (methanol in dichloromethane, 1% v/v) to give compound (**49b**) (10 mg, 11% yield) as a yellow solid. LC-MS (m/z) 381 [M+H]⁺. ¹H-NMR δ (400 MHz, CHCl₃-*d*) major characteristic peaks: δ 1.00 (s, 3H), 1.07 (s, 3H), 2.04 (s, 3H), 4.62 (m, 1H), 5.42 (m, 1H), 5.69 (s, 1H), 7.05 (s, 1H), 7.09 (s, 1H), 7.64 (s, 1H).

### Example 47C

### Preparation of Compound (49c)

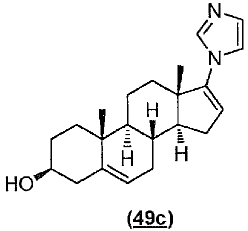

To a solution of compound (49b) (50 mg, 0.13 mmol) in methanol (5 mL) was added KOH (9 mg, 0.16 mmol) under N₂. The mixture was stirred at room temperature for 1 hr. The solvent was evaporated, and the residue was extracted with ethyl acetate (50 mL*2), washed with water, brine and dried over Na₂SO₄. The crude product was purified by pre-TLC (methanol in dichloromethane, 4% v/v) to give compound (**49c**) (6 mg, 14% yield) as a white solid. LC-MS (m/z) 339 [M+H]⁺. ¹H-NMR δ (400 MHz, CHCl₃-*d* major characteristic peaks: δ 1.01 (s, 3H), 1.06 (s, 3H), 3.55 (m, 1H), 5.39 (m, 1H), 5.69 (s, 1H), 7.05 (s, 1H), 7.09 (s, 1H), 7.64 (s, 1H).

### Example 47D

### Preparation of Compound (49d)

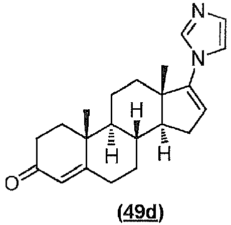

To a solution of compound (**49c**) (100 mg, 0.30 mmol) in 2-butanone (10 mL) was added Al(O*i*-Pr)₃ (122 mg, 0.60 mmol) under N₂. The mixture was stirred and heated to reflux overnight. The mixture was diluted with ethyl acetate (100 mL) and filtrated. The filtrate was evaporated and the residue was purified by pre-TLC (methanol in dichloromethane, 4% v/v) to give compound (**49d**) (21 mg, yield 21 %) as a white solid. LC-MS (m/z) 337 [M+H]⁺. ¹H-NMR δ (400 MHz, CDCl₃) major characteristic peaks: δ 1.01 (s, 3H), 1.22 (s, 3H), 5.68 (m, 1H), 5.74 (s, 1H), 7.03 (s, 1H), 7.08 (s, 1H), 7.61 (s, 1H).

### Example 47E

### Preparation of Compound (49e)

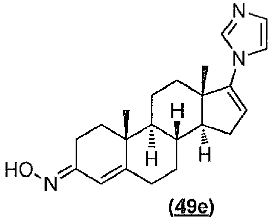

To a solution of compound (**49d**) (200 mg, 0.60 mmol) in ethanol (10 mL) was added pyridine (1 mL) and hydroxylamine hydrochlororide (64 mg, 0.90 mmol) under N₂. The mixture was stirred at room temperature overnight. The mixture was poured into ice-water (50 mL) and stirred 30 min. to give a white precipitate. Filtrated and dried to get the compound (**49e**) (210 mg, yield 99%) as a white solid. LC-MS (m/z) 352 [M+H]⁺. ¹H-NMR (400 MHz, CHCl₃-*d*) major characteristic peaks: δ 1.01 (s, 3H), 1.11 (s, 3H), 3.09 (m, 1H), 5.68 (m, 1H), 5.81 (s, 1H), 7.05 (s, 1H), 7.10 (s, 1H), 7.63 (s, 1H), 7.84 (brs, 1H).

### Example 47F

### Preparation of Compound (49)

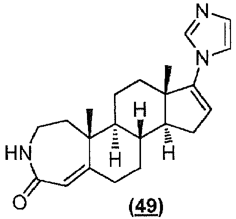

To a solution of compound (**49e**) (210 mg, 0.60 mmol) in dry THF (10 mL) was added dropwise a solution of SOCl₂ in THF (5 mL) at 0 °C. The mixture was stirred at room temperature overnight. The mixture was poured into ice-water (50 mL) and extracted with DCM (100 mL*2), washed with water, brine, and dried over Na₂SO₄. The residue was purified by pre-TLC (methanol in dichloromethane, 4% v/v) to give compound (**49**) (40 mg, yield 18%) as a brown solid.LC-MS (m/z) 352 [M+H]⁺. ¹H-NMR (400 MHz, CHCl₃-*d*) major characteristic peaks: δ 1.00 (s, 3H), 1.19 (s, 3H), 5.68 (m, 1H), 5.76 (s, 1H), 7.02 (brs, 1H), 7.04 (s, 1H), 7.09 (s, 1H), 7.62 (s, 1H).

### Example 48

### Preparation of Compound (50)

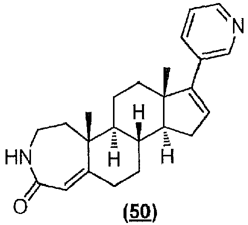

### Example 48A

### Preparation of Compound (50a)

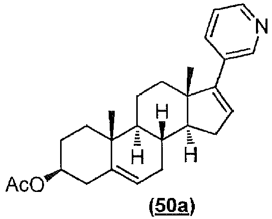

To a solution of compound (**3**) (2.0 g, 4.33 mmol) in 1,4-dioxane (30 mL) was added pyridin-3-ylboronic acid (905 mg, 7.36 mmol), potassium carbonate (2.7 g, 19.5 mmol) in water (5 mL), and Pd(dppf)Cl₂ (317 mg, 0.43 mmol). The mixture was thoroughly degassed, and heated under nitrogen at 80 °C for 1 hour. After being filtered through a pad of Celite, the crude product was purified silica gel chromatography (ethyl acetate in petroleum, 10 % v/v) to give compound (**50a**) (1.24 g, 73%) as a yellow solid. LC-MS (m/z) 392[M+H]⁺. ¹H-NMR δ (400 MHz, CHCl₃-*d* major characteristic peaks: 1.05 (s, 3H), 1.08 (s, 3H), 2.04 (s, 3H), 4.62 (m, 1H), 5.42 (m, 1H), 6.01 (s, 1H), 7.24 (m, 1H), 7.67 (d, *J* = 8.4 Hz, 1H), 8.47 (d, *J* = 4.8 Hz, 1H), 8.63 (s, 1H).

### Example 48B

### Preparation of Compound (50b)

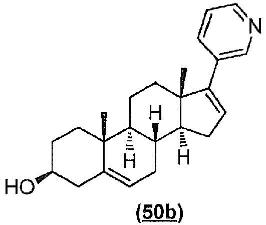

To a solution of compound (**50a**) (1.24 g, 3.17 mmol) in methanol (15 mL) and THF (4 mL) was added KOH (177 mg, 3.17 mmol) under N₂. The mixture was stirred at 30 °C for 1 hr. The solvent was evaporated, and the residue was extracted with ethyl acetate (100 mL*2), washed with water, brine and dried over Na₂SO₄. The crude product was recrystallized from petroleum ether to give compound **(50b)** (1.03 g, 94%) as a white solid. LC-MS (m/z) 350 [M+H]⁺. ¹H-NMR δ (400 MHz, CHCl₃-*d* major characteristic peaks: 1.05 (s, 3H), 1.07 (s, 3H), 3.55 (m, 1H), 5.40 (m, 1H), 6.01 (s, 1H), 7.24 (m, 1H), 7.66 (d, *J* = 8.0 Hz, 1H), 8.45 (d, *J* = 4.0 Hz, 1H), 8.62 (s, 1H).

### Example 48C

### Preparation of Compound (50c)

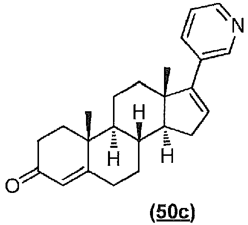

To a solution of compound (**50b**) (1.03 g, 2.96 mmol) in 2-butanone (20 mL) and toluene (10 mL) was added 25 % Al(O*i*-Pr)₃ (4.1 g, 4.97 mmol) under N₂. The mixture was stirred and heated to reflux overnight. The mixture was diluted with ethyl acetate (100 mL) and filtrated. The filtrate was evaporated, and the residue was recrystallized by petroleum ether to give compound (**50c**) (1.0 g, 97%) as a white solid. LC-MS (m/z) 348 [M+H]⁺. ¹H-NMR δ (400 MHz, CHCl₃-*d*) major characteristic peaks: 1.07 (s, 3H), 1.24 (s, 3H), 5.76 (s, 1H), 5.99 (m, 1H), 7.23 (m, 1H), 7.64 (d, *J* = 8.4 Hz, 1H), 8.46 (d, *J* = 4.4 Hz, 1H), 8.61 (s, 1H).

### Example 48D

### Preparation of Compound (50d)

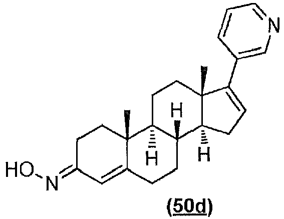

To a solution of compound (**50c**) (1.0 g, 2.88 mmol) in ethanol (15 mL) was added pyridine (4 mL) and hydroxylamine hydrochlororide (300 mg, 4.32 mmol) under N₂. The mixture was stirred at room temperature overnight. The mixture was poured into ice-water (100 mL) and stirred 30 min. to give a white precipitate. Filtrated and dried to get the compound (**50d**) (875 mg, 84%) as a white solid. LC-MS (m/z) 363 [M+H]⁺. ¹H-NMR δ (400 MHz, CHCl₃-*d*) major characteristic peaks: δ 1.06 (s, 3H), 1.12 (s, 3H), 3.07 (m, 1H), 5.80 (s, 1H), 6.00 (m, 1H), 7.25 (m, 1H), 7.67 (d, *J* = 8.0 Hz, 1H), 8.47 (d, *J* = 4.0 Hz, 1H), 8.62 (s, 1H).

### Example 48E

### Preparation of Compound (50)

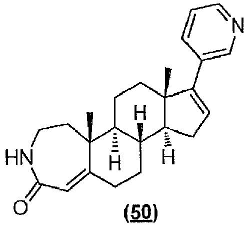

To a solution of compound (**50d**) (300 mg, 0.83 mmol) in dry THF (10 mL) was added dropwise a solution of SOCl₂ (1 mL) in THF (5 mL) at 0 °C. The mixture was stirred at room temperature overnight. The mixture was poured into ice-water (100 mL) and extracted with DCM (100 mL* 2), washed with water, brine, and dried over Na₂SO₄. The residue was purified by pre-TLC (methanol in dichloromethane, 4% v/v) to give compound (**50**) (38 mg, 13%) as a brown solid. LC-MS (m/z) 363 [M+H]⁺. ¹H-NMR δ (400 MHz, CHCl₃-*d*) major characteristic peaks: δ 1.04 (s, 3H), 1.20 (s, 3H), 2.56 (m, 1H), 3.16 (m, 1H), 3.24 (m, 1H), 5.77 (s, 1H), 6.00 (m, 1H), 6.14 (brs, 1H), 7.24 (m, 1H), 7.65 (d, *J* = 8.0 Hz, 1H), 8.47 (s, 1H), 8.62 (s, 1H).

### Example 49

### Prepared of Compound (51)

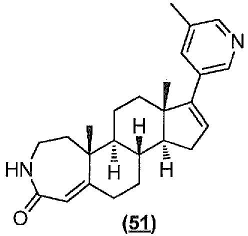

### Example 49A

### Preparation of Compound (51a)

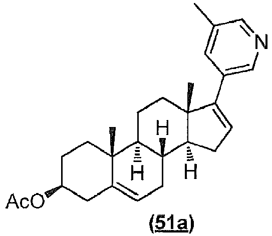

To a solution of compound (**3**) (2.0 g, 4.33 mmol) in 1, 4-dioxane (30 mL) was added 5-methylpyridin-3-ylboronic acid (830 mg, 6.06 mmol), potassium carbonate (2.688 g, 19.5 mmol) in water (5 mL), and Pd(dppf)Cl₂ (212 mg, 0.26 mmol). The mixture was thoroughly degassed, and heated under nitrogen at 80 °C for 1 hour. After being filtered through a pad of Celite, the crude product was purified by silica gel chromatography (ethyl acetate in petroleum, 10 %v/v) to give compound (**51a**) (1.6 g, 82%) as a yellow solid. LC-MS (m/z): 406 [M+H]⁺. ¹H-NMR δ (400 MHz, CHCl₃-*d* major characteristic peaks: 1.04 (s, 3H), 1.08 (s, 3H), 2.04 (s, 3H), 2.33 (s, 3H), 4.60-4.65 (m, 1H), 5.42 (s, 1H), 5.98 (s, 1H), 7.46 (s, 1H), 8.30 (s, 1H), 8.43 (s, 1H).

### Example 49B

### Preparation of Compound (51b)

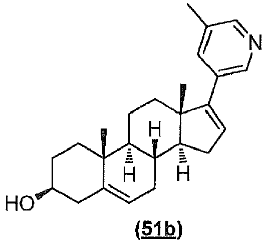

To a solution of compound (**51a**) (490 mg, 1.21 mmol) in methanol (15 mL) and THF (4 mL) was added NaOH (96 mg, 2.42 mmol) under N₂. The mixture was stirred at 30 °C for 1 hr. The solvent was evaporated, and the residue was extracted with ethyl acetate (100 mL x 2), washed with water, brine and dried over Na₂SO₄ and concentrated to give compound (**51b**) (439 mg, ∼100%) as a white solid. LC-MS (m/z): 364 [M+H]⁺. ¹H-NMR δ (400 MHz, CHCl₃-*d* major characteristic peaks: 0.97 (s, 3H), 1.00 (s, 3H), 2.27 (s, 3H), 3.45-3.50 (m, 1H), 5.33 (m, 1H), 5.92 (s, 1H), 7.42 (s, 1H), 8.23 (s, 1H), 8.36 (s, 1H).

### Example 49C

### Preparation of Compound (51c)

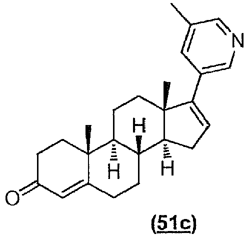

To a solution of compound (**51b**) (439 mg, 1.21 mmol) in 2-butanone (15 mL) and toluene (5 mL) was added 25 % Al(Oi-Pr)₃ (1.645 g, 1.936 mmol) under N₂. The mixture was stirred and heated to reflux overnight. The mixture was diluted with ethyl acetate (100 mL) and washed with NaHCO₃ (50 mL), H₂O (50 mL), brine (50 mL), dried over anhydrous Na₂SO₄ and filtrated. The filtrate was evaporated, and the residue was recrystallized by petroleum ether to give compound (**51c**) (320 mg, 72%) as a white solid. LC-MS (m/z): 362 [M+H]⁺.¹H-NMR δ (400 MHz, CHCl₃-*d* major characteristic peaks: 1.06 (s, 3H), 1.24 (s, 3H), 2.33 (s, 3H), 5.76 (s, 1H), 5.97 (m, 1H), 7.46 (s, 1H), 8.31 (s, 1H), 8.42 (s, 1H).

### Example 49D

### Preparation of Compound (51d)

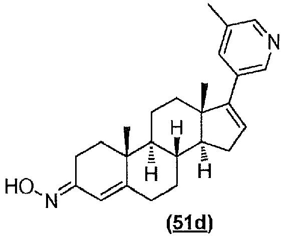

To a solution of compound (**51c**) (300 mg, 0.83 mmol) in ethanol (10 mL) was added pyridine (1 mL) and hydroxylamine hydrochlororide (86 mg, 1.245 mmol) under N₂. The mixture was stirred at room temperature overnight. The mixture was poured into ice-water (100 mL) and stirred 30 min. to give a white precipitate. Filtrated and dried to give the compound (**51d**) (310 mg, 99%) as a white solid.LC-MS (m/z): 377 [M+H]⁺. ¹H-NMR δ (400 MHz, CHCl₃-*d*) major characteristic peaks: δ 0.98 (s, 3H), 1.05 (s, 3H), 2.27 (s, 3H), 2.97-3.03 (m, 1H), 5.73 (s, 1H), 5.90 (m, 1H), 7.40 (s, 1H), 8.24 (s, 1H), 8.36 (s, 1H).

### Example 49E

### Preparation of Compound (51)

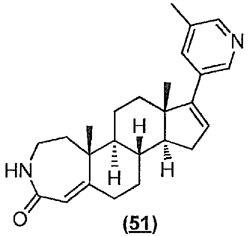

To a solution of compound (**51d**) (80 mg, 0.213 mmol) in dry THF (10 mL) was added dropwise SOCl₂ (0.3 mL) at 0 °C. The mixture was stirred at room temperature overnight. The mixture was poured into ice-water (100 mL) and extracted with ethyl acetate (100 mL*2), washed with water, brine, and dried over Na₂SO₄. The residue was purified by pre-TLC (methanol in dichloromethane, 4% v/v) to give compound (**51**) (34 mg, 43%) as a brown solid. LC-MS (m/z): 377 [M+H]⁺. ¹H-NMR δ (400 MHz, CHCl₃-*d*) major characteristic peaks: δ 1.04 (s, 3H), 1.20 (s, 3H), 2.35 (s, 3H), 3.16-3.24 (m, 2H), 5.77 (s, 1H), 5.99 (m, 1H), 6.13 (brs, 1H), 7.50 (s, 1H), 8.31 (s, 1H), 8.42 (s, 1H).

### Example 50

### Preparation of Compound (52)

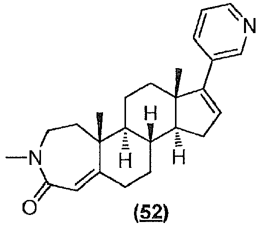

To a solution of compound (**50**) (174 mg, 0.48 mmol) in dry DMF (10 mL) was added NaH (60%, 38 mg, 0.96 mmol) and iodomethane (82 mg, 0.58 mmol) under N₂ at -30 °C. Then the mixture was warmed to room temperature and stirred overnight. The mixture was quenched with water (5 mL) and extracted with ethyl acetate (50 mL*3), washed with water, brine and dried over Na₂SO₄. After removal of solvent, the residue was purified with pre-TLC (methanol in dichloromethane, 4 % v/v) to give compound (**52**) (27 mg, 15%) as a brown solid. LC-MS (m/z) 377 [M+H]⁺. ¹H-NMR δ (400 MHz, CHCl₃-*d*) major characteristic peaks: δ 1.04 (s, 3H), 1.16 (s, 3H), 2.52 (m, 1H), 3.04 (s, 3H), 3.21 (m, 1H), 3.41 (m, 1H), 5.84 (s, 1H), 5.99 (m, 1H), 7.24 (m, 1H), 7.64 (d, *J* = 7.6 Hz, 1H), 8.47 (s, 1H), 8.62 (s, 1H).

### Example 51

### Preparation of Compound (53)

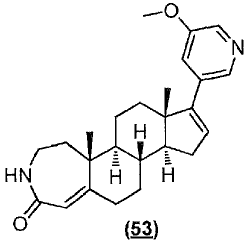

To a solution of compound (**19**) (500 mg, 1.18 mmol) in 1,4-dioxane (20 mL) was added 5-methoxypyridin-3-ylboronic acid (274 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 1 hour. After being filtered through a pad of Celite, the crude product was purified by silica gel chromatography (ethyl acetate in petroleum, 50% v/v) to give compound (**53**) (200 mg, 43%) as a yellow solid. LC-MS (m/z) 393 [M+H]⁺. ¹H-NMR δ (400 MHz, CHCl₃-*d*) major characteristic peaks: 1.03 (s, 3H), 1.20 (s, 3H), 3.86 (s, 3H), 5.76 (s, 1H), 5.99 (m, 1H), 6.39 (s, 1H), 7.14 (s, 1H), 8.18 (s, 1H), 8.23 (s, 1H).

### Example 52

### Preparation of Compound (54)

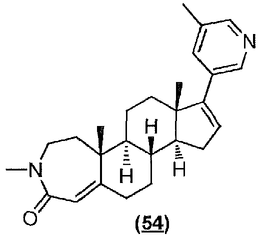

To a solution of compound (**20**) (500 mg, 1.18 mmol) in 1,4-dioxane (20 mL) was added 5-methylpyridin-3-ylboronic acid (274 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 1 hour. After being filtered through a pad of Celite, the crude product was purified by silica gel chromatography (ethyl acetate in petroleum, 50% v/v) to give compound (**54**) 152 mg, 33%) as a yellow solid. LC-MS (m/z) 391 [M+H]⁺. ¹H-NMR δ (400 MHz, CHCl₃-*d*) major characteristic peaks: 1.03 (s, 3H), 1.16 (s, 3H), 2.34 (s, 3H), 2.53 (m, 1H), 3.04 (s, 3H), 3.21 (m, 1H), 3.41 (m, 1H), 5.84 (s, 1H), 5.99 (m, 1H), 7.50 (s, 1H), 8.31 (s, 1H), 8.42 (s, 1H).

### Example 53

### Preparation of Compound (55)

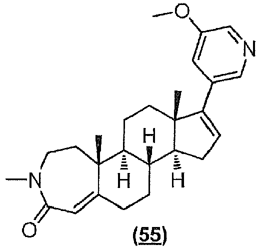

To a solution of compound (**20**) (176 mg, 0.41 mmol) in 1,4-dioxane (10 mL) was added 5-methoxypyridin-3-ylboronic acid (108 mg, 0.70 mmol), potassium carbonate (257 mg, 1.86 mmol) in water (1 mL), and Pd(dppf)Cl₂ (830 mg). The mixture was thoroughly degassed, and heated under nitrogen at 80 °C for 1 hour. After being filtered through a pad of Celite, the crude product was purified by silica gel chromatography (ethyl acetate in petroleum, 50% v/v) to give compound (**55**) 45 mg, 27%) as a white solid. LC-MS (m/z) 407 [M+H]⁺. ¹H-NMR δ (400 MHz, CHCl₃-*d* major characteristic peaks: 1.04 (s, 3H), 1.16 (s, 3H), 2.53 (m, 1H), 3.04 (s, 3H), 3.21 (m, 1H), 3.41 (m, 1H), 3.86 (s, 3H),5.84 (s, 1H), 6.00 (m, 1H), 7.15 (s, 1H), 8.17 (s, 1H), 8.23 (s, 1H).

### Example 54

### Preparation of Compound (56)

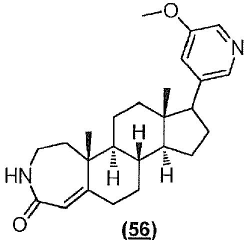

To a solution of compound (**53**) (200 mg, 0.51 mmol) in acetic acid (5 mL) was added Pd/C (80 mg) was stirred at room temperature under H₂ atmosphere. After being filtered through a pad of Celite, the crude product was purified by silica gel chromatography (ethyl acetate in petroleum ether, 50% v/v) to give compound (**56**) (32 mg, 16%) as a yellow solid. LC-MS (m/z) 395 [M+H]⁺. ¹H-NMR δ (400 MHz, CHCl₃-*d*) major characteristic peaks: δ 0.51 (s, 3H), 1.14 (s, 3H), 3.86 (s, 3H), 5.76 (s, 1H), 6.21 (s, 1H), 7.06 (s, 1H), 8.08 (s, 1H), 8.16 (s, 1H).

### Example 55

### Preparation of Compound (57)

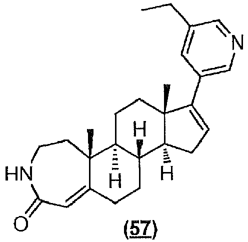

To a solution of compound (**19**) (500 mg, 1.18 mmol) in 1,4-dioxane (20 mL) was added 5-methoxypyridin-3-ylboronic acid (274 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 1 hour. After being filtered through a pad of Celite, the crude product was purified by silica gel chromatography (ethyl acetate in petroleum, 50% v/v) to give compound (**57**) (150 mg, 32%) as a yellow solid. LC-MS (m/z) 391 [M+H]⁺. ¹H-NMR δ (400 MHz, CHCl₃-*d*) major characteristic peaks: 1.04 (s, 3H), 1.20 (s, 3H), 1.26 (m, 3H), 2.63 (m, 2H), 5.76 (s, 1H), 5.97 (s, 1H), 6.27 (s, 1H), 7.46 (s, 1H), 8.34 (s, 1H), 8.43 (s, 1H).

### Example 56

### Preparation of Compound (58)

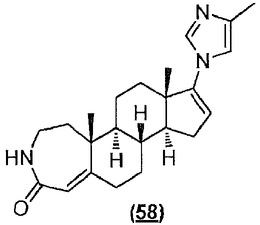

Using a synthetic procedure and conditions similar to Examples 47A - 47F in the preparation of compound (**49**), replacing imidazole in Example 47A, with 4-methyl-1*H-*imidazole, compound (**58**) was prepared. MS calcd for (C23H32N2O)⁺: 365.25; MS found (electrospray): 366.3; ¹H NMR (CDCl₃, 400 MHz) major characteristic peaks: δ 0.99 (s, 3H), 1.19 (s, 3H), 2.23 (s, 3H), 2.54 (m, 1H), 3.16 (m, 1H), 3.25(m, 1H), 5.61 (s, 1H), 5.77 (s, 1H), 6.20(s, 1H), 6.78 (s, 1H),7.52(s,1H).

### Example 57

### Preparation of Compound (59)

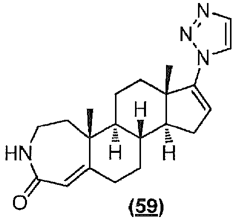

Using a synthetic procedure and conditions similar to Examples 47A - 47F in the preparation of compound (**49**), replacing imidazole in Example 47A, with 1*H*-1,2,3-triazole, compound (**59**) was prepared. LC-MS (m/z) 353 [M+H]⁺. ¹H-NMR δ (400 MHz, CHCl₃-*d*) major characteristic peaks: δ 1.08 (s, 3H), 1.14 (s, 3H), 2.50 (m, 1H), 3.16 (m, 1H), 5.71 (s, 1H), 5.86 (m, 1H), 6.31 (brs, 1H), 7.65 (m, 2H).

### Example 58

### Preparation of Compound (60)

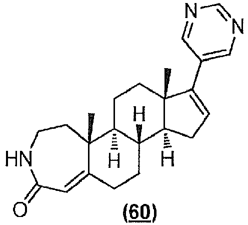

### Example 58A

### Preparation of Compound (60a)

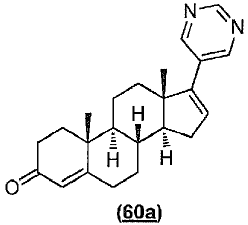

A mixture of compound (**18**) (1.0 g, 2.53 mmol), pyrimidin-5-ylboronic acid (626 mg, 5.05 mmol), Pd(dppf)₂Cl₂ (204 mg, 0.25 mmol), K₂CO₃ (1.75 g, 12.65 mmol) in 1,4-dioxane (15 mL) and water (3 mL) was stirred at 115 °C for 2 hours. Then filtered and the solvent was removed and the residue was dissolved in DCM (100 mL) washed with water (1 x 20 mL) and brine (1 x 20 mL), dried and concentrated to give the crude product which purified by column chromatography (ethyl acetate in petroleum, 20% v/v) to give compound (**60a**) (287 mg, 33%) as a light yellow solid. LC-MS (m/z) 349 [M+H]⁺.

### Example 58B

### Preparation of Compound (60b)

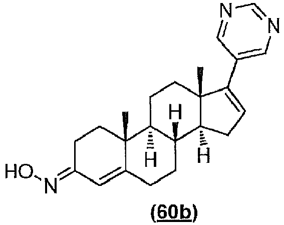

A solution of compound (**60a**) (287 mg, 0.82 mmol) in EtOH (6 mL) was added hydroxylamine hydrochloride (131 mg, 1.65 mmol) and pyridine (1 mL), The mixture was stirred at room temperature for 2 hours. Then it was poured into ice-water, filtered and the solid was dried to give compound (**60b**) (330 mg). LC-MS (m/z) 364 [M+H]⁺.

### Example 58C

### Preparation of Compound (60)

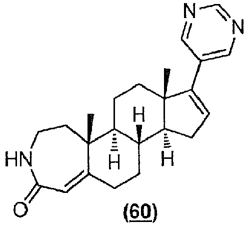

A solution of compound (**60b**) (330 mg, Crude) in dry THF (6 mL) was added SOCl₂ (2 mL) and the mixture was stirred at room temperature overnight. Then it was poured into ice-water, adjusted to pH7 with NH₄OH, extracted with DCM (3 x 30 mL). The organic layer was washed with brine (1 x 30 mL), dried and concentrated to give the crude product which purified by column chromatography (ethyl acetate) to give compound (**60**) (75 mg, 25%, two steps) as a brown solid. LC-MS (m/z) 364 [M+H]⁺. ¹R NMR (CDCl₃, 400 MHz) major characteristic peaks: δ 0.98 (s, 3H), 1.14 (s, 3H), 3.13 (m, 2H), 5.70 (m, 1H), 6.03 (m, 1H), 6.25 (s, 1H), 8.65 9m, 2H), 9.00 (m, 1H).

### Example 59

### Preparation of Compound (61)

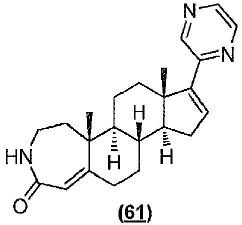

### Example 59A

### Preparation of Compound (61a)

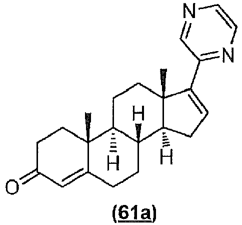

A The mixture of compound (**18**) (443 mg, 1.12 mmol), 2-(tributylstannyl)pyrazine (414 mg, 1.12 mmol), Pd(PPh₃)₄ (150 mg) in DMF (10 mL) was stirred at 90 °C for 16 hours. Then filtered and the solvent was dissolved in DCM (150 mL) and washed with water (4 x 20 mL) and brine (1 x 20 mL), dried and concentrated to give the crude product which purified by column chromatography (ethyl acetate in petroleum, 10% v/v) to give compound (61a) (128 mg, 14%) as a light yellow solid. LC-MS (m/z) 349 [M+H]⁺.

### Example 59B

### Preparation of Compound (61b)

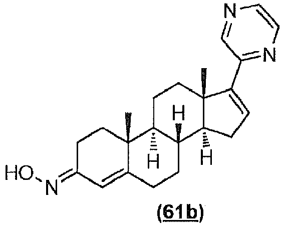

A solution of compound (**61a**) (128 mg, 0.37 mmol) in ethanol (3 mL) was added NH₂OH.HCl (58 mg, 0.74 mmol) and pyridine (0.5 mL), The mixture was stirred at room temperature for 2 hours. Then it was poured into ice-water, filtered and the solid was dried to give compound (**61b**) (100 mg, Crude).LC-MS (m/z) 364 [M+H]⁺.

### Example 59C

### Preparation of Compound (61)

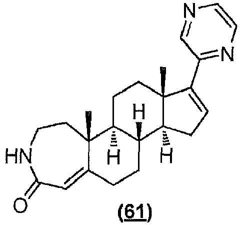

To a solution of compound (**61b**) (100 mg) in anhydrous THF (2 mL) was added SOCl₂ (0.2 mL), the mixture was stirred at room temperature for 1 hour. Then it was poured into ice-water, adjusted to pH7 with NH₄OH and extracted with DCM (3 x 30 mL). The organic layer was washed with brine (1 x 30 mL), dried and concentrated to give the crude product which purified by Prep-TLC (ethyl acetate) to give compound (**61**) (10 mg, 10%, two steps) as a light yellow solid. LC-MS (m/z) 364 [M+H]⁺. ¹H NMR (CDCl₃, 400 MHz) major characteristic peaks: δ 1.07 (s, 3H), 1.14 (s, 3H), 3.13 (m, 2H), 5.70 (m, 1H), 6.31 (s, 1H), 6.39 (m, 1H), 8.27 (m, 1H), 8.39 (m, 1H), 8.62 (m, 1H).

### Example 60

### Preparation of Compound (62)

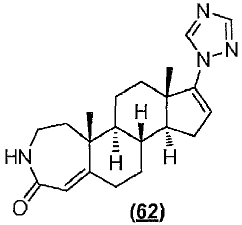

Using a synthetic procedure and conditions similar to Examples 47A - 47F in the preparation of compound (**49**), replacing imidazole in Example 47A, with 1*H-*1,2,4-triazole, compound (**62**) was prepared. LCMS: (M+H)⁺= 353; ¹H-NMR δ (400 MHz, CDCl₃) major characteristic peaks: δ 1.03 (s, 3H), 1.14 (s, 3H), 3.46-2.54 (m, 1H), 3.12-3.19 (m, 1H), 5.71 (s, 1H), 5.87 (s, 1H), 6.42 (s, 1H), 7.92 (s, 1H), 8.19 (s, 1H).

### Example 61

### Preparation of Compound (63)

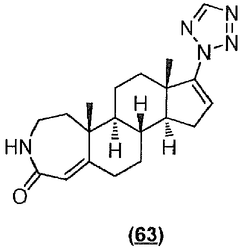

Using a synthetic procedure and conditions similar to Examples 47A - 47F in the preparation of compound (**49**), replacing imidazole in Example 47A, with 2H-tetrazole, compound (**63**) was prepared. LCMS: (M+H)⁺= 354; ¹H-NMR δ (400 MHz, CDCl₃) major characteristic peaks: δ 1.16 (s, 3H), 1.22 (s, 3H), 5.78 (s, 1H), 6.38 (s, 1H), 6.45 (s, 1H), 8.52 (s, 1H).

### Example 62

### Preparation of Compound (64)

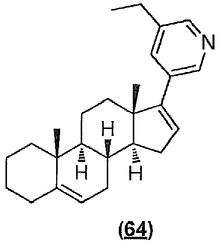

To a solution of compound **(22**) (420 mg, 1.1 mmol) in 1,4-dioxane (20 mL) was added 5-ethylpyridin-3-ylboronic acid (302 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 4 hour. After being filtered through a pad of Celite, the crude product was purified by silica gel chromatography (ethyl acetate in petroleum, 50% v/v) to give compound (**64**) (35 mg, 9.3%) as a white solid. LC-MS (m/z): 362 [M+H]⁺. ¹H NMR (CDCl₃, 400 MHz) major characteristic peaks: δ 0.98 (m, 6H), 1.19 (t, J= 7.6, 3H), 2.57 (m, 2H), 5.24 (m, 1H), 5.93 (m, 1H), 7.38 (s, 1H), 8.24 (s, 1H), 8.37 (s, 1H).

### Example 63

### Preparation of Compound (65)

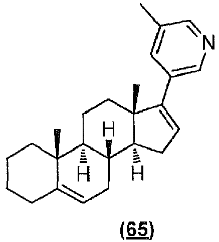

A solution of compound **(22**) (384 mg, 1.000 mmol), 5-methylpyridin-3-ylboronic acid (274 mg, 2.000 mmol), K₂CO₃ (552 mg, 4.000 mmol) and PdCl₂(dppf)₂ (78 mg, 0.100 mmol) in dioxane/H₂O (20/5 mL) was heated to 85 °C, and stirred overnight under N₂. Then it was purified by Pre-HPLC to obtain compound (**65**) (100 mg, 29%) as a white solid. MS calculate for (C25H33N)⁺: 347; MS found (electrospray): 348; ¹H NMR (CDCl₃, 400 MHz) major characteristic peaks: δ 1.04 (s, 3H), 1.06 (s, 3H), 2.33 (s, 3H), 5.30 (s, 1H), 5.98 (s, 1H), 7.46 (s, 1H), 8.30 (s, 1H), 8.43 (s, 1H).

### Example 64

### Preparation of Compound (66)

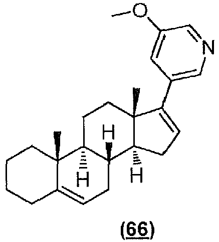

A solution of compound **(22**) (384 mg, 1.000 mmol), 5-methoxypyridin-3-ylboronic acid (309 mg, 2.000 mmol), K₂CO₃ (552 mg, 4.000 mmol) and PdCl₂(dppf)₂ (78 mg, 0.100 mmol) in dioxane/H₂O (20/5 mL) was heated to 85 °C, and stirred for overnight under N₂. Then it was purified by Pre-HPLC to obtain compound (**66**) (110 mg, 30%) as a white solid. MS calculate for (C25H33NO)⁺: 363; MS found (electrospray): 364; ¹H NMR (CDCl₃, 400 MHz) major characteristic peaks: δ 1.04 (s, 3H), 1.06 (s, 3H), 3.87 (s, 3H), 5.30 (s, 1H), 6.02 (s, 1H), 7.20 (s, 1H), 8.17 (s, 1H), 8.26 (s, 1H).

### Example 65

### Preparation of Compound (67)

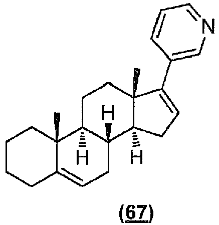

To a solution of compound **(22**) (420 mg, 1.1 mmol) in 1,4-dioxane (20 mL) was added pyridine boronic acid (246 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 4 hour. After being filtered through a pad of Celite, the crude product was purified by silica gel chromatography (ethyl acetate in petroleum, 50% v/v) to give compound (**67**) (40 mg, 11%) as a white solid. LC-MS (m/z): 334 [M+H]⁺. ¹H NMR (CDCl₃, 400 MHz) major characteristic peaks: δ 0.98 (m, 6H), 5.24 (m, 1H), 5.93 (m, 1H), 7.16 (m, 1H), 7.61 (m, 1H), 8.39 (m, 1H), 8.56 (m, 1H).

### Example 66

### Preparation of Compound (68)

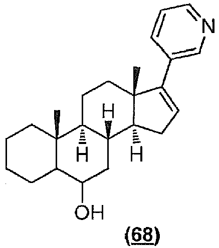

To a solution of compound (**24**) (0.54 g, 1.35 mmol), K₂CO₃ (0.932 g, 6.75 mmol) in H₂O (3 mL) and pyridin-3-ylboronic acid (0.332 g, 2.7 mmol) in 1,4-dioxane(50 mL) was stirred at room temperature for 15 min.Then Pd(PPh₃)₄ (59 mg, 0.135 mmol) was added to the reaction mixture. Then it was heated to 70 °C for 30 min. The solvent was evaporated and diluted with water (20 mL). It was then extracted with dichloromethane (30 mL * 3) and the organic layer was washed with water (20 mL * 2), brine and dried over Na₂SO₄, concentrated to afford the crude product. The crude product was purified by column chromatography (ethyl acetate in petroleum ether, 15% v/v) to afford the compound (**68**) (0.3 g, 63.4%) as a brown solid. LCMS: 352 [M+1] ⁺; ¹H NMR (CDCl₃, 400 MHz) major characteristic peaks: δ 87 (s, 3H), 1.03 (s, 3H), 3.45-3.52 (m, 1H), 6.00-6.02 (m, 1H),7.23-7.27(m,1H),7.67(d,J=8Hz,1H),8.47-8.49 (m,1H),8.63(s,1H).

### Example 67

### Preparation of Compound (69)

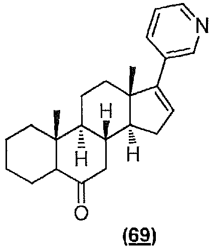

To a solution of compound (**68**) (0.24 g, 0.68 mmol), pyridinium chlorochromate (0.3 g, 1.37 mmol) and BaCO₃ (0.4 g, 2.05 mmol) in DCM (50 mL) was stirred at 40 °C for 0.5 h. The reaction mixture was filtered. The filtrate was washed with water (20 mL *2), brine. Then the organic layer was dried over Na₂SO₄, concentrated to afford the crude product .The crude product was purified by column chromatography (ethyl acetate in petroleum ether, 15% v/v) to afforded the compound (**69**) (0.03 g, 12.6%). LCMS: 350 [M+1]⁺; ¹H NMR (CDCl₃, 400 MHz) major characteristic peaks: δ 0.79 (s, 3 H), 1.01 (s, 3 H), 5.99-6.00 (m, 1 H), 7.23-7.26 (m,1H),7.67 (d, J = 7.6 Hz,1H), 8.48 (d, *J* = 4 Hz, 1H),8.62(s, 1H).

### Example 68

### Preparation of Compound (70)

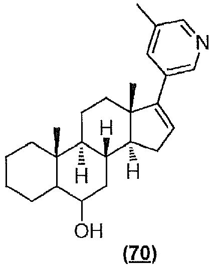

To a solution of compound (**24**) (0.5 g, 1.6 mmol), K₂CO₃ (0.45 g, 3.2 mmol) in H₂O (5 ml) and 5-methylpyridin-3-ylboronic acid (0.45 g, 3.3 mmol) in 1,4-dioxane (50 mL) was stirred at room temperature for 15min.Then Pd(PPh₃)₄ (0.19 mg, 0.16 mmol) was added to the reaction mixture. It was then heated to 70°C for 30min.The solvent was evaporated under reduce pressure and diluted with water (20 mL). Tt was then extracted with dichloromethane (30 mL * 3), washed with water (20 mL * 2), brine and dried over Na₂SO₄, concentrated to afford the crude product. The crude product was purified by column chromatography (ethyl acetate in petroleum ether, 15% v/v) to afford the compound (**70**) (0.2 g, 33.4%) as a white solid. LCMS: 366 [M+1] ⁺; ¹H NMR (CDCl₃, 400 MHz) major characteristic peaks: δ 0.85 (s, 3 H), 1.00 (s, 3 H), 2.33 (s, 3 H), 3.46 (m, 1 H), 5.96-5.97 (m,1 H),7.47 (s,1 H), 8.29 (s,1H), 8.42 (s,1 H).

### Example 69

### Preparation of Compound (71)

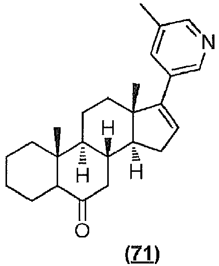

To a solution of compound (**70**) (0.12 g, 0.33 mmol), pyridinium chlorochromate (0.14 g, 0.66 mmol) and BaCO₃ (0.19 g, 0.99 mmol) in DCM (20 mL) was stirred at 40 °C for 0.5 h. The reaction mixture was filtered. The filtrate was washed with water (20 mL *2), brine. Then the organic layer was dried over Na₂SO₄, concentrated to afford the crude product .The crude product was purified by column chromatography (ethyl acetate in petroleum ether, 15% v/v) to afforded the compound (**71**) (32 mg, 27%) as a white solid. LCMS: 364 [M+1⁺; ¹H NMR (CDCl₃, 400 MHz) major characteristic peaks: δ 0.79 (s, 3 H), 1.00 (s, 3 H), 2.34 (s, 3 H), 5.96-5.97 (m, 1 H), 7.46 (s, 1 H), 8.31 (s,1 H), 8.42 (s,1 H).

### Example 70

### Preparation of Compound (72)

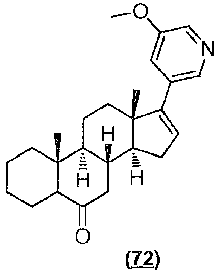

To a solution of compound (**28**) (0.20 g, 0.5 mmol), K₂CO₃ (0.35 g, 2.5 mmol) in H₂O (3 mL) and 5-methoxypyridin-3-ylboronic acid (0.30g, 2.0 mmol) in 1,4-dioxane(50 mL) was stirred at room temperature for 15 mins. Then Pd(PPh₃)₄ (59 mg, 0.135 mmol) was added to the reaction mixture. Tt was then was heated to 80 °C for 3hs. The solvent was evaporated and diluted with water (20 mL). Then it was extracted with dichloromethane (30 mL * 3) and the organic layer was washed with water (20 mL * 2), brine and dried over Na₂SO₄, concentrated to afford the crude product. The crude product was purified by pre-HPLC to afford the compound (**72**) (26 mg, 18%) as a white solid. LCMS: 380 [M+1⁺; ¹H NMR (CDCl₃, 400 MHz) major characteristic peaks: δ 0.80 (s, 3H), 1.02 (s, 3H), 3.91 (s, 3H), 6.08 (m, 1H), 7.34(s, 1H), 8.37(s, 2H).

### Example 71

### Preparation of Compound (73)

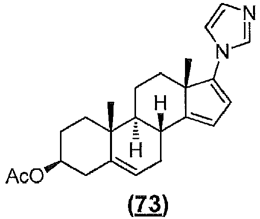

### Example 71A

### Preparation of Compound (73a)

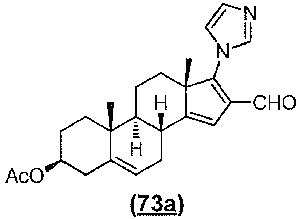

To a solution of compound (**31**) (273 mg, 0.73 mmol) in DMF (5 mL) were added K₂CO₃ (302 mg, 2.2 mmol) and imidazole (75 mg, 1.1 mmol). The mixture was stirred and heated to 80 °C overnight. The mixture was cooled to room temperature, and poured onto ice-water (100 mL) followed by extracted with ethyl acetate (50 mL*3), washed with water, brine and dried over Na₂SO₄. After removal of solvent, the residue was purified with silica gel chromatography (ethyl acetate in petroleum, 33% v/v) to give compound (**73a**) (60 mg, 20%) as a yellow solid. LC-MS (m/z) 407 [M+H]⁺. ¹H-NMR δ (400 MHz, CHCl₃-*d*) major characteristic peaks: δ 1.13 (m, 6H), 2.04 (s, 3H), 4.62 (m, 1H), 5.51 (s, 1H), 6.32 (s, 1H), 7.14 (s, 1H),7.26 (s, 1H), 7.64 (s, 1H), 9.75 (s, 1H).

### Example 71B

### Preparation of Compound (73)

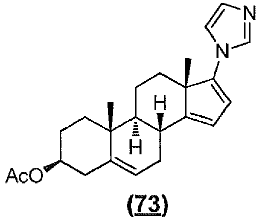

To a solution of compound (**73a**) (60 mg, 0.15 mmol) in dry PhCN (5 mL) was added 10 % Pd/C (30 mg) under N₂. The mixture was stirred and heated to reflux for 3.5 hrs. After cooling to room temperature, the catalyst was removed by filtration through a Celite pad. The filtrate was evaporated, and the residue was purified by pre-TLC (ethyl acetate in petroleum, 33% v/v) to give compound (**73**) (14 mg, 25%) as a yellow solid. LC-MS (m/z) 379 [M+H]⁺. ¹H-NMR δ (400 MHz, CHCl₃-*d*) major characteristic peaks: δ 1.13 (s, 3H), 1.14 (s, 3H), 2.04 (s, 3H), 4.62 (m, 1H), 5.50 (m, 1H), 5.91 (s, 1H), 6.26 (s, 1H), 7.10 (s, 1H), 7.13 (s, 1H), 7.66 (s, 1H).

### Example 72

### Preparation of Compound (74)

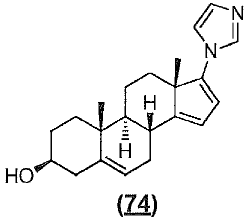

To a solution of compound (**73**) (200 mg, 0.53 mmol) in methanol (5 mL) was added KOH (73 mg, 0.53 mmol) under N₂. The mixture was stirred at room temperature for 1 hr. The solvent was evaporated, and the residue was extracted with ethyl acetate (100 mL * 2), washed with water, brine and dried over Na₂SO₄. The crude product was purified by pre-TLC (ethyl acetate in petroleum, 50% v/v) to give compound (**74**) (106 mg, 60%) as a white solid. LC-MS (m/z) 337 [M+H]⁺ .¹H-NMR δ (400 MHz, CHCl₃-*d*) major characteristic peaks: δ 1.13 (s, 6H), 3.56 (m, 1H), 5.48 (m, 1H), 5.91 (s, 1H), 6.26 (m, 1H), 7.11 (s, 1H), 7.13 (s, 1H), 7.66 (s, 1H).

### Example 73

### Preparation of Compound (75)

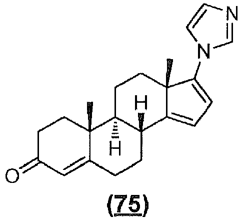

To a solution of compound (**74**) (119 mg, 0.35 mmol) in 2-butanone (5 mL) and toluene (5 mL) was added Al(Oi-Pr)₃ (491 mg, 0.57 mmol) under N₂. The mixture was stirred and heated to reflux overnight. The mixture was diluted with ethyl acetate (100 mL) and filtrated. The filtrate was evaporated, and the residue was purified by pre-TLC (4%, methanol in dichloromethane, v/v) to give compound (**75**) as a white solid (59 mg, 50% yield). LC-MS (m/z) 335 [M+H]⁺. ¹H-NMR δ (400 MHz, CHCl₃-*d*) major characteristic peaks: δ 1.15 (s, 3H), 1.31 (s, 3H), 5.79 (s, 1H), 5.92 (m, 1H), 6.26 (d, *J* = 2.4 Hz, 1H), 7.10 (s, 1H), 7.14 (s, 1H), 7.66 (s, 1H).

### Example 74

### Preparation of Compound (76)

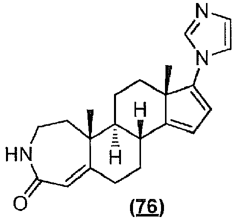

### Example 74A

### Preparation of Compound (76a)

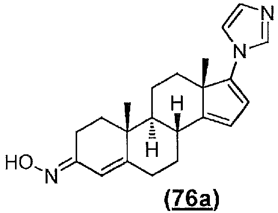

To a solution of compound (**75**) (154 mg, 0.46 mmol) in ethanol (6 mL) was added pyridine (0.5 mL) and hydroxylamine hydrochlororide (64 mg, 0.92 mmol) under N₂. The mixture was stirred at room temperature overnight. The mixture was poured into ice-water (100 mL) and stirred 30 min. to give a white precipitate. Filtrated and dried to get the compound (**76a**) as a white solid (150 mg, 93% yield). LC-MS (m/z) 350 [M+H]⁺. ¹H-NMR δ (400 MHz, CHCl₃-. d) major characteristic peaks: δ 1.13 (s, 3H), 1.18 (s, 3H), 3.07 (m, 1H), 5.85 (s, 1H), 5.89 (m, 1H), 6.25 (s, 1H), 7.10 (s, 1H), 7.14 (s, 1H), 7.67 (s, 1H).

### Example 74B

### Preparation of Compound (76)

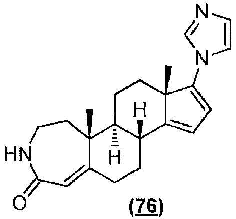

To a solution of compound (**76a**) (150 mg, 0.43 mmol) in dry THF (5 mL) was added dropwise SOCl₂ (0.3 mL) at 0 °C. The mixture was stirred at room temperature overnight. The mixture was poured into ice-water (100 mL), adjusted pH = 7 with NH₃.H₂O, Extracted with DCM (100 mL * 2), washed with water, brine, and dried over Na₂SO₄. The residue was purified by pre-TLC (methanol in dichloromethane, 4% v/v) to give compound (**76**) (35 mg, 23%) as a brown solid. LC-MS (m/z) 350 [M+H]⁺. ¹H-NMR δ (400 MHz, CHCl₃-*d* major characteristic peaks: δ 1.13 (s, 3H), 1.26 (s, 3H), 5.81 (s, 1H), 5.90 (m, 1H), 6.25 (d, J= 2.4 Hz, 1H), 6.29 (brs, 1H), 7.09 (s, 1H), 7.13 (s, 1H), 7.66 (s, 1H).

### Example 75

### Preparation of synthetic intermediate Compound (77)

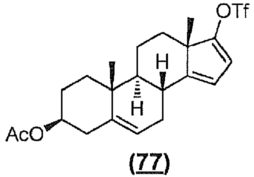

Compound (**30**) (1.73 g, 5.27 mmol) and Et₃N (0.73 mL) was dissolved in dry DCM (20 mL). When it was cooled to -60 °C, the solution of Tf₂O (0.95 mL) in DCM (10 mL) was added dropwise at this temperature. The mixture was warmed to -20 °C for 1 hour, diluted with DCM (10 mL), and water (20 mL) was added. Extracted with DCM (3 x 50 mL), washed with HCl (1M, 1 x 50 mL), NaHCO₃ (1 x 50 mL) and brine (1 x 50 mL), dried and concentrated to give the crude product which purified by column chromatography (ethyl acetate in petroleum ether, 20% v/v) to get compound (**77**) (614 mg, 25%) as a yellow solid. LC-MS (m/z) 329 [M+H]⁺. ¹H-NMR δ (400 MHz, CDCl₃) : 1.12 (s, 3H), 1.16 (s, 3H), 2.04 (s, 3H), 4.61 (m, 1H), 5.47 (m, 1H), 5.79 (m, 1H), 6.09 (m, 1H).

### Example 76

### Preparation of Compound (78)

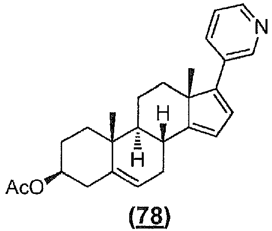

The mixture of compound (**77**) (60 mg, 0.13 mmol), pyridin-3-ylboronic acid (24 mg, 0.20 mmol), Pd(dppf)Cl₂ (11 mg, 0.01 mmol) and K₂CO₃ (90 mg, 0.65 mmol) in 1,4-dioxane (2 mL) and water (0.5 mL) was stirred at 80 °C under N₂ atmosphere for 2 hours. Filtered and the solvent was removed and the residue was dissolved in CH₂Cl₂ (60 mL), washed with water (3 x 20 mL) and brine (1 x 20 mL), dried and concentrated to give the crude product which purified by Prep-TLC (ethyl acetate in petroleum, 30% v/v)) to give compound (**78**) (33 mg, 33%) as a brown solid. LC-MS (m/z) 390 [M+H]⁺. ¹H-NMR δ (400 MHz, CDCl₃) : 1.17 (s, 3H), 1.21 (s, 3H), 2.05 (s, 3H), 4.63 (m, 1H), 5.51 (m, 1H), 6.03 (m, 1H), 6.81 (m, 1H), 7.24 (m, 1H), 7.78 (m, 1H), 8.42 (m, 1H), 8.77 (m, 1H).

### Example 77

### Preparation of Compound (79)

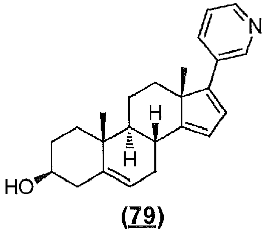

A solution of compound **(78)** (326 mg, 0.84 mmol) and NaOH (67 mg, 1.68 mmol) in MeOH (10 mL) and water (2 mL) was stirred at room temperature for 2 hours. Then most of MeOH was removed, and extracted with DCM (3 x 30 mL). The organic layer was washed with water (2 x 30 mL) and brine (1 x 30 mL), dried and concentrated, dried to give compound (**79**) (285 mg, 98%) as a white solid. LC-MS (m/z) 348 [M+H]⁺. ¹H-NMR δ (400 MHz, CDCl₃) : 1.16 (s, 3H), 1.21 (s, 3H), 3.56 (m, 1H), 5.49 (m, 1H), 6.02 (m, 1H), 6.81 (m, 1H), 7.25 (m, 1H), 7.78 (m, 1H), 8.41 (m, 1H), 8.77 (m, 1H).

### Example 78

### Preparation of Compound (80)

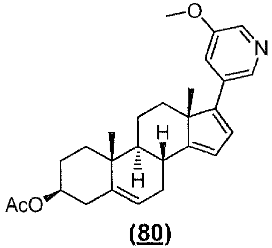

To a solution of compound (**77**) (460 mg, 1 mmol) in 1,4-dioxane (20 mL) was added 5-methoxypyridin-3-ylboronic acid (306 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 4 hour. After being filtered through a pad of Celite, the crude product was purified by silica gel chromatography (ethyl acetate in petroleum ether, 20% v/v) to give compound (**80**) (372 mg, 83%) as a white solid. LC-MS (m/z): 420 [M+H]⁺. ¹H NMR (CDCl₃, 400 MHz) major characteristic peaks: δ 1.16 (s, 3H), 1.21 (s, 3H), 2.05 (s,3H), 3.89 (s, 3H), 4.62 (m, 1H), 5.52 (m, 1H), 6.02 (m, 1H), 6.82 (s, 1H), 7.27 (s, 1H), 8.14 (s, 1H), 8.40 (s, 1H).

### Example 79

### Preparation of Compound (81)

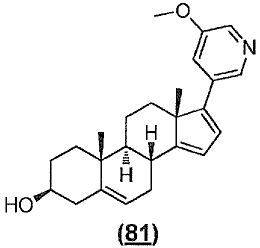

To a solution of compound (**80**) ( (375 mg, 1 mmol) in MeOH/H2O (20/4 mL) was added NaOH (60 mg, 1.5 mmol). The mixture was stirred at rt about 2 hours. Evaporated the solvent and the residue was poured into ice. It was extracted with DCM (10 mL * 2) and the combined the organic layers was dried and evaporated to give compound (**81**) ( (312 mg, 83%) as a white solid. LC-MS (m/z): 378 [M+H]⁺. ¹H NMR (CDCl₃, 400 MHz) major characteristic peaks: δ 1.15 (s, 3H), 1.21 (s, 3H), 3.56 (m, 1H), 3.89 (s, 3H), 5.50 (m, 1H), 6.02 (m, 1H), 6.83 (s, 1H), 7.30 (s, 1H), 8.13 (s, 1H), 8.40 (s, 1H).

### Example 80

### Preparation of Compound (82)

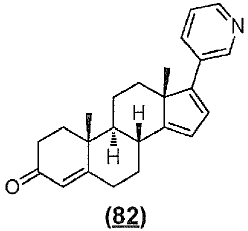

A mixture of compound (**79**) (270 mg, 0.78 mmol) and Al(Oi-Pr)₃ (24%, 1.06 g, 1.24 mmol) in 2-butanone (10 mL) and toluene (2 mL) was stirred at 80 °C overnight. Water (40 mL) was added and filtered through a celite pad. The filtrate was extracted with DCM (3 x 50 mL). The organic layer was washed with brine (1 x 50 mL), dried and concentrated to give the crude product which purified by column chromatography (ethyl acetate in petroleum, 30%v/v) to give compound (**82**) (135 mg, 50%) as a light yellow solid. LC-MS (m/z) 346 [M+H]⁺. ¹H-NMR δ (400 MHz, CDCl₃) : 1.23 (s, 3H), 1.33 (s, 3H), 5.80 (m, 1H), 6.02 (m, 1H), 6.80 (m, 1H), 7.25 (m, 1H), 7.77 (m, 1H), 8.43 (m, 1H), 8.76 (m, 1H).

### Example 81

### Preparation of Compound (83)

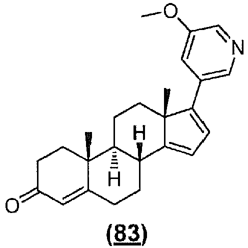

To a solution of compound (**81**) (377 mg, 1 mmol) in butaneone (5 mL) / toluene (20 mL) was added Al(Oi-Pr)₃ (1.6 g, 2.01 mmol). The mixture was heated under nitrogen at 80 °C for 4 hour. After being filtered through a pad of Celite, the crude product was purified by column chromatography (ethyl acetate in petroleum, 30% v/v) to give compound (**83**) (178 mg, 47%) as a white solid. LC-MS (m/z): 376 [M+H]⁺. ¹H NMR (CDCl₃, 400 MHz) major characteristic peaks: δ 1.23 (s, 3H), 1.33 (s, 3H), 3.89 (s, 3H), 5.79 (m, 1H), 6.01 (m, 1H), 6.80 (s, 1H), 7.29 (s, 1H), 8.15 (s, 1H), 8.39 (s, 1H).

### Example 82

### Preparation of Compound (84)

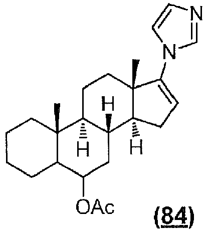

### Example 82A

### Preparation of Compound (84a)

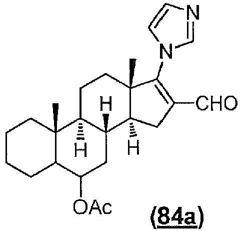

A mixture of compound (**33**) (7.8 g, 20.6 mmol), K₂CO₃ (5.7 g, 41.2 mmol) and imidazole (2.1 g, 30.9 mmol) in DMF (50 mL) was stirred at 80 °C for 6 h. It was then cooled to room temperature, and the DMF was removed and the residue was diluted with ethyl acettate (200 mL) and water (200 mL). The organic phase was washed with water, brine, and dried over Na₂SO₄. Concentrated and purified by column chromatography (petroleum ether:ethyl acetate =5:1-1:1, v/v) to give compound (**84a**) (7.0 g, 83 %) as a yellow solid. LC-MS: 411 [M+1]⁺. ¹H NMR (CDCl₃, 400 MHz) major characteristic peaks: δ 0.89 (s, 3H), 1.03 (s, 3H), 2.04 (s, 3H), 4.71 (m, 1H), 7.09 (s, 1H), 7.21 (s, 1H), 7.62 (s, 1H), 9.73 (s, 1H).

### Example 82B

### Preparation of Compound (84)

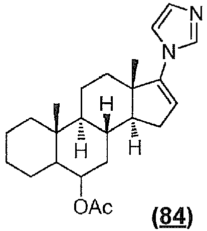

A solution of compound (**84a**) (1.0 g, 2.4 mmol) and Pd/C (10%, 500 mg) in PhCN (10 mL) was stirred at 250 °C by microwave for 8 h. The solvent was evaporated and purified by column chromatography (petroleum ether:ethyl acetate 5:1-1:1, v/v) to afford the compound (**84**) (670 mg). LCMS: 383 [M+1]⁺; ¹H NMR (DMSO, 400 MHz) major characteristic peaks: δ 0.85 (s, 3H), 0.94 (s, 3H), 1.99 (s, 3H), 4.59 (m, 1H), 5.79 (m, 1H), 6.98 (s, 1H), 7.33 (s, 1H), 7.80 (s, 1H).

### Example 83

### Preparation of Compound (85)

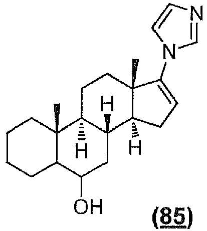

To a solution of compound (**84**) (400 mg, 1.0 mmol) in MeOH (15 mL) and THF (5 mL) was added a solution of NaOH (84 mg, 2.1 mmol) in H₂O (3 mL). The mixture was stirred at 50 °C for 2 h. The solution was concentrated and the residue was diluted with ethyl acetate (40 mL) and water (40 mL). The organic phase was separated, washed with water, brine and dried over Na₂SO₄. The crude product was purified by pre-HPLC to afford the title compound (**85**) (65 mg, 18 %) as a white solid. LCMS: 341 [M+1]⁺; ¹H NMR (CDCl₃, 400 MHz) major characteristic peaks: δ 0.84 (s, 3H), 1.04 (s, 3H), 3.47 (m, 1H), 6.04 (m, 1H), 7.21 (s, 1H), 7.44 (s, 1H), 8.64 (s, 1H).

### Example 84

### Preparation of Compound (86)

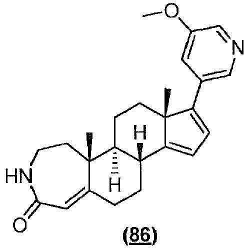

### Example 84A

### Preparation of Compound (86a)

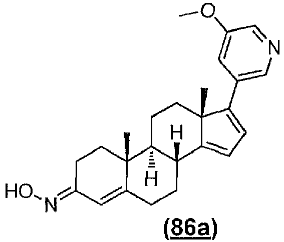

To a solution of compound (**83**) (375 mg, 1 mmol) in EtOH (20 mL) was added NH₂OHHCl (105 mg, 1.5 mmol) and pyridine (0.2 ml). The mixture was stirred at room temperature about 4 hour. The reaction mixture was poured into ice and filtered the solid, dried to to give compound (**86a**) (350 mg, 92%) as a white solid. LC-MS (m/z): 406 [M+H]⁺.

### Example 84B

### Preparation of Compound (86)

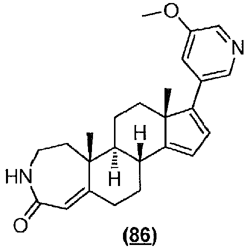

To a solution of compound (**86a**) (405 mg, 1 mmol) in THF (20ml) was added SOCl2 (0.5 ml).The mixture was stirred at room temperature about 4 hour. The reaction mixture was poured into ice and filtered the solid. After being filtered through a pad of Celite, the crude product was purified by column chromatography (ethyl acetate in petroleum, 80% v/v) to give compound (**86**) (40 mg, 10%) as a white solid. LC-MS (m/z): 391 [M+H]⁺. ¹H NMR (CDCl₃, 400 MHz) major characteristic peaks: δ 1.13 (s, 3H), 1.21 (s,3H), 3.81 (s, 3H), 5.73 (m, 1H), 5.93 (m, 1H), 6.41 (s, 1H), 6.73 (s, 1H), 7.19 (s, 1H), 8.07 (s, 1H), 8.30 (s, 1H).

### Example 85

### Preparation of Compound (87)

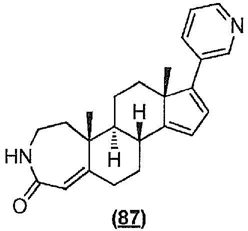

### Example 85A

### Preparation of Compound (87a)

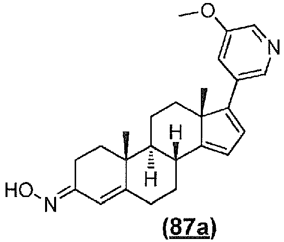

A solution of compound (**82**) (125 mg, 0.36 mmol) in EtOH (2 mL) was added NH₂OH.HCl (58 mg, 0.72 mmol) and pyridine (0.5 mL). The mixture was stirred at room temperature for 2 hours. Then it was poured into ice-water, filtered and the solid was dried to give the compound (**87a**) (157 mg) LC-MS (m/z) 361 [M+H]⁺.

### Example 85B

### Preparation of Compound (87)

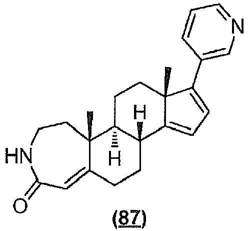

A solution of compound (**87a**) (157 mg) in dried THF (6 mL) was added SOCl₂ (0.2 mL) in dry THF (2 mL). The mixture was stirred at room temperature overnight. Then it was poured into ice-water, adjusted to pH7 with NH₄OH, extracted with DCM (3 x 30 mL). The organic layer was washed with brine (1 x 30 mL), dried and concentrated to give the crude product which purified by Prep-TLC (ethyl acetate) to get final compound (**87**) (21 mg, 16% two steps) as a yellow solid. LC-MS (m/z) 361 [M+H]⁺. ¹H NMR (CDCl₃, 400 MHz) major characteristic peaks: δ 1.21 (s, 3H), 1.29 (s, 3H), 5.81 (m, 1H), 6.01 (m, 1H), 6.16 (s, 1H), 6.80 (m, 1H), 7.27 (m, 1H), 7.78 (m, 1H), 8.43 (m, 1H), 8.75 (m, 1H).

### Example 86

### Preparation of Compound (88)

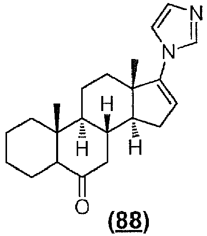

To a solution of compound (**85**) (120 mg, 0.35 mmol) in THF (10 mL) was added tetrapropylammonium perruthenate (TPAP, 62 mg, 0.18mmol), 4-methylmorpholine- N-oxide monohydrate (NMO, 165 mg, 1.41 mmol). The mixture was stirred at 50 °C overnight. The solution was cooled and concentrated and the crude product was purified by pre-TLC (petroleum ether:ethyl acetate = 2:1, v/v) to afford compound **(88)** (30 mg, 25 %) as a white solid. LCMS(m/z): 339 [M+1]⁺; ¹H NMR (CDCl₃, 400 MHz) major characteristic peaks: δ 0.78 (s, 3H), 0.97 (s, 3H), 5.69 (m, 1H), 7.05 (s, 1H), 7.10 (s, 1H), 7.64 (s, 1H).

### Example 87

### Preparation of Compound (89)

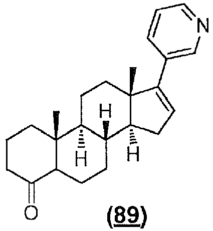

### Example 87A

### Preparation of Compound (89a)

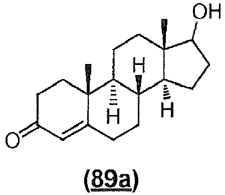

To a stirred solution of androstenedione (20 g, 70 mmol) in DCM (100mL) and MeOH (10 mL) at 0 °C was added NaBH₄ (8 g, 210 mmol). The mixture was stirred at room temperature for 10 h and then quenched with acetic acid (100 mL). The mixture was diluted with water (200 mL) and extracted with ethyl acetate (3 x 100mL). The organic layers were combined and dried over Na₂SO₄, concentrated to give compound (**89a**) (20.0 g) as a white solid. ¹HNMR: (400 MHz, CDCl₃) major characteristic peaks: δ 0.795 (s, 3H), 1.197 (s, 3H), 3.654 (m, 1H), 5.732 (s, 1H).

### Example 87B

### Preparation of Compound (89b)

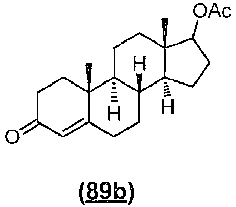

To a solution of compound (**89a**) (20.0 g) in acetic anhydride (100 mL) was added DMAP (160 mg, 1.4 mmol) and pyridine (10 mL). The reaction mixture was stirred for 6 h at room temperature. Then the mixture was diluted with water (100 mL) and extracted with ethyl acetate(3 x 100 mL). The organic layers were combined and dried over Na₂SO₄. The solvent was evaporated and the residue was purified by column chromatography (petroleum ether: ethyl acetate = 5:1, v/v) to give compound **(89b)** (11.6 g, 50%) as a white solid. ¹HNMR: (400 MHz, CDCl₃) major characteristic peaks: δ 0.769 (s, 3H), 1.123 (s, 3H), 1.978 (s, 3H), 4.528 (t, *J* = 8.4 Hz, 1H), 5.661 (s, 1H).

### Example 87C

### Preparation of Compound (89c)

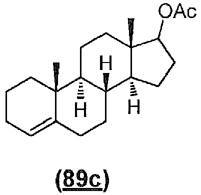

NaBH₄ (1.3 g, 34.4 mmol) was gradually added to a stirred mixture of TFA (8 mL, 107.4 mmol), acetic acid (8 mL, 133.3 mmol) and CH₃CN (8 mL), which was cooled in an ice-bath. compound **(89b)** (2.3 g, 7.0 mmol) in DCM (40 mL) was then added to the mixture. The reaction mixture was stirred overnight at room temperature. The sat.NaHCO₃ solution was added to the mixture. The mixture was extracted with ethyl acetate (3 x 50 mL). The organic layers were combined and dried over Na₂SO₄, concentrated to give compound **(89c)** (2.3 g) as a white solid. ¹HNMR: (400 MHz, CDCl₃) major characteristic peaks: δ 0.74 (s, 3H), 0.94 (s, 3H), 2.03 (s, 3H), 4.51 (m, 1H), 5.22 (m, 1H).

### Example 87D

### Preparation of Compound (89d)

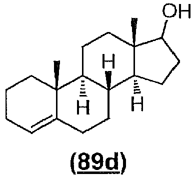

To a solution of compound **(89c)** (2.3 g) in MeOH (300 mL) was added KOH (814 mg, 14.5 mmol). The reaction mixture was stirred overnight at room temperature. Then H₂O (100 mL) was slowly added to the mixture. The mixture was extracted with ethyl acetate (5 x 100 mL). The organic layers were combined and dried over Na₂SO₄, concentrated to give compound **(89d)** (1.6 g, 80%) as a white solid. ¹HNMR: (400 MHz, CDCl₃) major characteristic peaks: δ 0.76 (s, 3H), 1.02 (s, 3H), 3.62 (t, *J* = 8.4 Hz, 1H), 5.29 (m, 1H).

### Example 87E

### Preparation of Compound (89e)

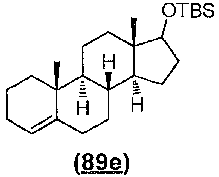

To a solution of compound **(89d)** (13 g, 47.4 mmol) in DCM (200 mL) were added TBSCl (8.8 g, 58.4 mmol) and imidazole (6.6 g, 97.4 mmol) at 0 °C. The reaction mixture was stirred overnight at room temperature. Then the reaction mixture was diluted with H₂O (50 mL) and extracted with DCM (3 x 200 mL). The organic layers were combined, dried over Na₂SO₄ and evaporated to give residue. The residue was purified by column chromatography (petroleum ether:ethyl acetate = 50:1, v/v) to give compound (**89e**) (18 g, 92%) as a white solid. ¹HNMR: (400 MHz, CDCl₃) major characteristic peaks: δ 0.001 (s, 3H), 0.007 (s, 3H), 0.71 (s, 3H), 0.87 (s, 9H), 1.01 (s, 3H), 3.53 (t, *J*= 8.4 Hz, 1H), 5.29 (s, 1H).

### Example 87F

### Preparation of Compound (89f)

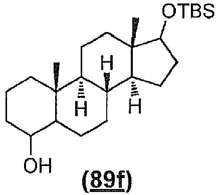

Compound (**89e**) (5.1 g, 13.1 mmol) in THF (150 mL) was treated with borane in THF (1.0 M, 82.6 mL) at 0 °C under N₂. The reaction was followed by TLC until the formation of borane was complete. The mixture was carefully treated with 10% NaOH solution (100 mL), followed by 30% H₂O₂ (150 mL). The mixture was stirred overnight. Na₂SO₃ (10 g) was added to it followed by acetic acid (50 mL), water (250mL). The mixture was stirred for a further 15min and extracted with ethyl acetate (5 x 100 mL). The organic layers were combined and dried over Na₂SO₄, concentrated to give compound (**89f**) (4.0 g, 75%) as a yellow solid.

### Example 87G

### Preparation of Compound (89g)

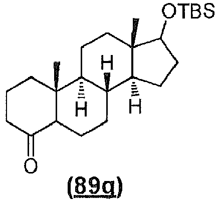

To a solution of compound (**82f**) (12.2 g, 30 mmol) in DCM (100 mL) was added pyridinium chlorochromate (12.93 g, 60 mmol) and celite (5.0 g). The mixture was stirred at room temperature for 3 h. Filtrated through celite and washed with DCM (100 mL x 2). The filtrate was washed with water (100 mL x 2), brine (100 mL x 2), dried over anhydrous Na₂SO₄ and concentrated to give a mixture compound (**89g**) (**α/β** =1/1,12.1 g, 99%) as yellow solid. ¹HNMR: (400 MHz, CDCl₃) major characteristic peaks: δ 0.001 (s, 12H), 0.689 (s, 3H, 18-CH₃ of α-isomer), 0.743 (s, 3H, 19-CH₃ of α-isomer), 0.685 (s, 3H, 18-CH₃ of β-isomer), 0.874 (s, 18H), 1.121 (s, 3H, 19-CH₃ of β-isomer), 3.516 (m, 2H).

### Example 87H

### Preparation of Compound (89h)

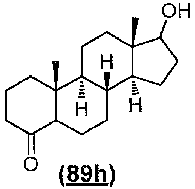

To a solution of compound (**89g**) (5.0 g, 12.35 mmol) in DCM (100 mL) was added BF₃.Et₂O (1.7 mL, 13.6 mmol) and the reaction mixture was stirred at room temperature overnight. The mixture was washed with saturated aqueous NaHCO₃ solution (100 mL x 1), brine (100 mL x 1), dried over anhydrous Na₂SO₄ and concentrated to give the crude product. The crude was purified by column chromatogram (ethyl acetate/ petroleum ether: = 1:15 to 1:10, v/v) to obtain the product compound (**89h**) (α/β = 1/1, 2.6 g, 68%) as white solid. ¹HNMR: (400 MHz, CDCl₃) major characteristic peaks: δ 0.72 (s, 3H, 18-CH₃ of α-isomer), 0.74 (s, 3H, 19-CH₃ of α-isomer), 0.75 (s, 3H, 18-CH₃ of β-isomer), 0.874 (s, 18H), 1.12 (s, 3H, 19-CH₃ of β-isomer), 3.62 (m, 2H).

### Example 87I

### Preparation of Compound (89i)

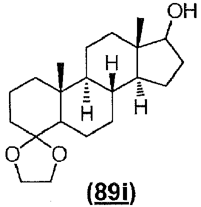

To a solution of compound (**89h**) (2.45 g, 8.45 mmol) in ethyl orthoformate (50 mL) was added ethylene glycol (1.05 g, 16.9 mmol), toluenesulfonic acid (145 mg, 0.845 mmol) and stirred at 90 °C overnight. After reaction the mixture was washed with saturated aqueous NaHCO₃ solution (100 mL x 1), brine (100 mL x 1), dried over anhydrous Na₂SO₄ and concentrated to give the crude product. The crude was purified by column chromatogram (ethyl acetate/ petroleum ether: = 1:15 to 1:8, v/v) to obtain the product compound **(89i)** (440 mg, 16%) as a white solid. ¹HNMR: (400 MHz, CDCl₃) major characteristic peaks: δ 0.73 (s, 3H), 0.96 (s, 3H), 3.62 (t, *J*= 8.4 Hz, 1H), 3.72-3.75 (m, 1H), 3.90-3.97 (m, 3H).

### Example 87J

### Preparation of Compound (89j)

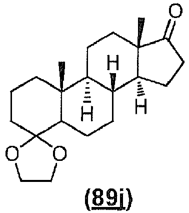

To a solution of compound **(89i)** (440 mg, 1.32 mmol) in DCM (20 mL) was added pyridinium chlorochromate (560 mg, 2.6 mmol), celite (600 mg) and stirred at room temperature for 3h. After reaction the mixture was filtrated through celite and washed with DCM (50 mL x 1). The filtrate was washed with water (50 mL x 2), brine (50 mL x 2), dried over anhydrous Na₂SO₄ and concentrated to give the crude product. The crude product was purified by column chromatogram (ethyl acetate/ petroleum ether: = 1:20 to 1:10, v/v) to obtain the compound (**89j**) (200 mg, 46%) as a white solid. ¹HNMR: (400 MHz, CDCl₃) major characteristic peaks: δ 0.86 (s, 3H), 0.97 (s, 3H), 2.04-2.11 (m, 1H), 2.40-2.47 (m, 1H), 3.74-3.77 (m, 1H), 3.91-3.98 (m, 3H).

### Example 87K

### Preparation of Compound (89k)

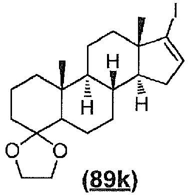

To a solution of compound (**89j**) (200 mg, 0.6 mmol) in ethanol (15 mL) was added hydrazine hydrate (300 mg, 6.0 mmol), triethylamine (1 drop) and stirred at 90 °C for 3h. After reaction the mixture was concentrated to dryness it was dissolved in DCM (15 mL) and triethylamine (1.3 mL). A solution of I₂ (381 mg, 1.5 mmol) in THF (8 mL) was added dropwise to the above solution at 0 °C. After addition the mixture was stirred at room temperature for 2h. The mixture was quenched with aqueous Na₂SO₃ (30 mL) and extracted with DCM (50 mL x 2). The organic phase was washed with water (50 mL x 1), brine (50 mL x 1), dried over anhydrous Na₂SO₄ and concentrated to give the compound **(89k)** (190 mg, 72%) as a yellow solid. ¹HNMR: (400 MHz, CDCl₃) major characteristic peaks: δ 0.72 (s, 3H), 0.97 (s, 3H), 3.73-3.76 (m, 1H), 3.90-3.98 (m, 3H).

### Example 87L

### Preparation of Compound (89)

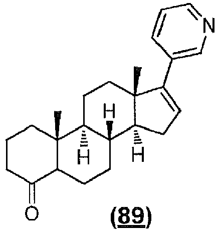

To a solution of compound **(89k)** (190 mg, 0.43 mmol), pyridin-3-ylboronic acid (158 mg, 1.28 mmol) in dioxane (10 mL) and water (2 mL) was added potassium carbonate (267 mg, 4.5 mmol), PdCl₂(dppf) (20 mg, 0.0258 mmol) and stirred at 90 °C for 3h. After reaction the mixture was quenched with water (10 mL) and extracted with DCM (50 mL x 2), washed with brine (50 mL x 2), dried over anhydrous Na₂SO₄ and concentrated to give the crude product. The crude product was dissolved in methanol (20 mL) and added aqueous hydrochloric acid solution (1N, 5 mL) and stirred at room temperature for 30 min. After reaction the mixture was adjusted pH to 7-8 with aqueous NaHCO₃ (10 mL) and extracted with DCM (50 mL x 2), washed with brine (50 mL x 2), dried over anhydrous Na₂SO₄ and concentrated to give the crude product. The crude product was purified by pre-TLC (ethyl acetate/ petroleum ether: = 1:5, v/v) to obtain the product compound (**89**) (80 mg, 60%) as a white solid. LCMS: (M +H)⁺ = 350, ¹HNMR: (400 MHz, CDCl₃) major characteristic peaks: δ 0.80 (s, 3H), 1.01 (s, 3H), 2.04-2.11 (m, 1H), 5.99 (s, 1H), 7.21-7.24 (m, 1H), 7.64-7.67 (m, 1H), 8.45-8.47 (m, 1H), 8.62 (s, 1H).

### Example 88

### Preparation of Compound (90)

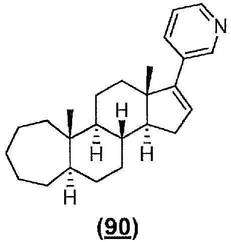

A mixture of compound (**10**) (220 mg), 3-pyridyl boronic acid (2 equiv.), Na₂CO₃ (272 mg, 3 equiv.) and Pd(Ph₃P)₂Cl₂ (18.4 mg, 5% mol) in THF-H₂O (8 mL/8 mL) was degassed within an ultrasonic cleaner for 1 hr. Under argon protection, the reaction mixture was stirred at 45 °C for 6 hr. THF was removed under reduced pressure. The water phase was extracted with EtOAc (3×15 mL). The combined organic layers were washed with water, dried over MgSO₄, and condensed under reduced pressure. The residue was purified through FCC (eluent: Hexanes/EtOAc=15/1→10/1) on silica gel to give compound **(90)** (110 mg) as a white solid. ¹H NMR 8.60 (doublet, 1H, Ar), 8.43(quartet, 1H, Ar), 7.64(quintet, 1H, Ar), 7.20(quartet, 1H, Ar), 5.97(quartet, 1H, vinyl), 2.24(multiplet, 1H), 2.00(multiplet, 2H), 0.99, (singlet, 3H, Me), 0.798 (singlet, 3H, Me) ¹³C NMR 151.81, 147.90, 147.74, 133.59, 133.06, 129.26, 122.98, 57.79, 53.63, 48.31, 47.38, 40.80, 38.92, 35.74, 33.81, 32.32, 31.78, 31.29, 27.84, 26.96, 21.81, 16.71, 13.94 ESI-MS 350.1 HPLC 93.5% CH₃CN(60→100%)+H₂O(0.1% TFA), 0.8 mL/min, 242nm, zorbax Eclipse XDB-C8, 5 u (150x4.6 mm)

### Example 89

### Preparation of Compounds (91), (92), (93), (94), (95) and (96)

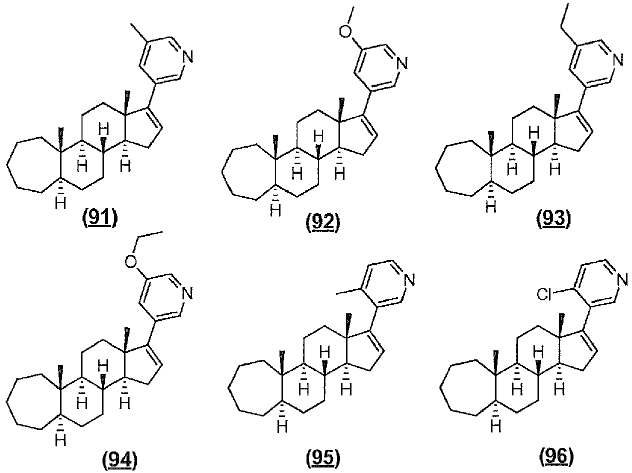

Using a synthetic procedure and conditions similar to Example 88 in the preparation of compound (**90**), replacing 3-pyridyl boronic acid with various substituted 3-pyridyl boronic acid compounds (**91**), (**92**), (**93**), (**94**), (**95**) **and** (**96**) were prepared.

**Compound (91):** ¹H NMR 8.48(singlet, 1H, Ar), 8.27(singlet, 1H, Ar), 7.44(singlet, 1H, Ar), 5.94 (triplet, 1H, vinyl), 2.30 (singlet, 3H, Me), 2.21 (mutiplet, 1H), 2.00 (mutiplet, 2H), 0.95 (singlet, 3H, Me), 0.80 (singlet, 3H, Me) ¹³C NMR 151.91, 148.22, 145.04, 134.33, 132.59, 132.31, 129.03, 57.82, 53.66, 48.32, 47.39, 40.81, 38.93, 35.82, 33.84, 32.33, 31.74, 31.30, 27.84, 26.95, 21.82, 18.43, 16.72, 13.94 ESI MS 364.1 HPLC 93.8%

**Compound (92):** ¹H NMR 8.25(singlet, 1H, Ar), 8.17(singlet, 1H, Ar), 7.16(singlet, 1H, Ar), 6.00 (singlet, 1H, vinyl), 3.86 (singlet, 3H, MeO), 2.24 (mutiplet, 1H), 2.05 (mutiplet, 2H), 1.00 (singlet, 3H, Me), 0.80 (singlet, 3H, Me) ¹³C NMR 155.25, 151.66, 140.53, 135.12, 133.66, 129.56, 118.62, 57.82, 55.51, 53.66, 48.33, 47.45, 40.80, 38.94, 35.81, 33.84, 32.33, 31.78, 31.31, 27.85, 26.96, 21.83, 16.76, 13.95 ESI MS 380.1 .

**Compound (93):** ¹H NMR 8.42(singlet, 1H, Ar), 8.29(singlet, 1H, Ar), 7.44(singlet, 1H, Ar), 5.95(triplet, 1H, vinyl),3.62 (quartet, 2H, CH₂), 2.22 (mutiplet, 1H),2.00 (mutiplet, 3H), 1.00 (singlet, 3H, Me), 0.80 (singlet, 3H, Me) ¹³C NMR 152.01, 147.57, 145.32, 138.43, 133.08, 132.66, 128.95, 57.82, 53.66, 48.32, 47.40, 40.80, 38.93, 35.81, 33.84, 32.33, 31.75, 31.30, 27.84, 26.95, 26.05, 21.82, 16.73, 15.31, 13.94 ESI MS 378.1.

**Compound (94) two atropisomers:** ¹H NMR 8.22(singlet, 1H, Ar), 8.13(singlet, 1H, Ar), 7.15(singlet, 1H, Ar), 5.97(triplet, 1H, vinyl),4.07 (quartet, 2H, OCH₂), 2.22 (mutiplet, 1H),2.00 (mutiplet, 2H), 1.00 (singlet, 3H, Me), 0.80 (singlet, 3H, Me) ¹³C NMR 154.63, 151.64, 140.36, 135.40, 133.66, 129.45, 119.33, 119.28, 63.83, 57.79, 57.62, 55.03, 53.64, 48.30, 47.59, 47.41, 47.26, 40.80, 38.91, 38.49, 36.45, 35.79, 35.49, 34.04, 33.81, 32.31,31.97, 31.75, 31.69, 31.28, 28.97, 27.83, 26.94, 26.80, 22.15, 21.81, 20.77, 16.74, 14.77, 13.93, 12.21 ESI MS 380.1.

**Compound (95) two atropisomers:** ¹H NMR 8.31(doublet, 1H, Ar), 8.27(singlet, 1H, Ar), 7.10(doublet, 1H, Ar), 5.64(singlet, 1H, vinyl),2.40 (singlet, 3H, Me), 2.22 (mutiplet, 1H),2.00 (mutiplet, 2H), 0.95 (singlet, 3H, Me), 0.81 (singlet, 3H, Me) ¹³C NMR 150.79, 149.27, 147.48, 145.59, 133.74, 130.51, 130.42, 125.02, 57.44, 57.28, 55.24, 53.84, 49.74, 49.59, 48.36, 47.34, 40.83, 38.97, 38.53, 36.51, 35.39, 35.10, 34.42, 34.17, 32.46, 32.20, 32.13, 31.33, 31.28, 29.06, 29.01, 27.86, 26.97, 26.81, 22.15, 21.80, 21.71, 20.66, 20.23, 16.44, 13.98, 12.22 ESI MS 364.1.

**Compound (96) two atropisomers:** ¹H NMR 8.35(singlet, 1H, Ar), 8.33(singlet, 1H, Ar), 7.32(doublet, 1H, Ar), 5.82(singlet, 1H, vinyl),2.32 (singlet, 3H, Me), 2.05 (mutiplet, 1H),2.00 (mutiplet, 2H), 0.92 (singlet, 3H, Me), 0.81 (singlet, 3H, Me) ¹³C NMR 150.61, 148.52, 148.47, 143.41, 133.18, 132.52, 124.64, 57.17, 57.01, 55.14, 53.73, 49.98, 49.82, 48.31, 47.31, 40.81, 38.96, 38.51, 36.50, 35.17, 34.88, 34.35, 34.12, 32.40, 32.33, 32.26, 32.07, 31.30, 31.26, 29.05, 29.00, 27.87, 26.97, 26.80, 22.15, 21.79, 21.70, 20.65, 16.45, 13.99, 12.22 ESI MS 384.1.

### Example 90

### Preparation of Compound (97)

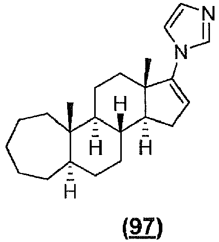

### Example 90A

### Preparation of Compound (97a)

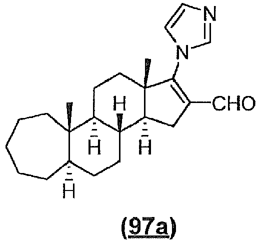

A mixture of compound (**11**) (270 mg, 0.806 mmol), K₂CO₃ (336 mg, 2.41 mmol) and imidazole (82.3 mg, 1.21mmol) in dry DMF (15 mL) was heated to 80 °C nuder N₂ over night. The reaction mixture was cooled to room temperature and concentrated under reduce pressure. The residue was treated with water (15 mL), followed with extraction with EtOAc (3x15 mL). The combined extracts was washed with brine, dried over Na₂SO₄, and concentrated to give compound (**97a**) (243 mg, 80.3% yield). ¹H NMR: 10 (1H), 7.60 (1H), 7.03-7.06(2H), 2.49-2.51(1H), 1.93-2.08(2H), 1.13-1.92(18H), 0.85-1.12(10H).

### Example 90B

### Preparation of Compound (97)

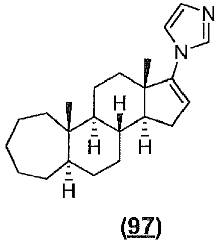

A solution of compound (**97a**) (240 mg, 0.655 mmol) in dry benzonitrile (15 mL) was refluxed in the presence of 10% palladium on activated charcoal (260 mg) overnight. The catalyst was pre-washed with methanol and benzonitrile in turn. After being cooled to room temperature, the catalyst was removed by filtration. The filtrate was condensed. The residue was purified by FCC (Hexanes/EtOAc=5/1) to give compound (**97**). ¹H NMR: 7.61(1H, singlet, imidazolyl), 7.05(1H, imidazolyl), 7.01(1H, imidazolyl), 5.65 (1H, -CHO), 2.25 (1H, multiplet), 2.20 (2H, multiplet), 1.88 (1H, multiplet), 0.95(3H), 0.80(3H). ESI-MS: 339.

### Example 91

### Preparation of Compound (98)

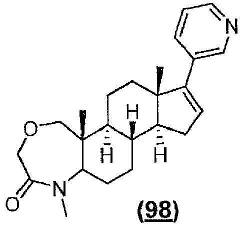

Under argon protection, a mixture of compound (**14**) (250 mg, 0.550 mmol), 3-bromopyridyl boronic acids (820 mmol, 1.5 equiv.), NaHCO₃ (660 mmol, 1.2 equiv.) and Pd(Ph₃P)₂Cl₂ (3% equiv.) in THF-H₂O (10 mL/10 mL) was heated at reflux overnight. THF was removed under reduced pressure. 30 mL of EtOAc was added and stirred. The organic phase was partitioned and washed with water (3x20 mL). The solvents were evaporated. The residue was subjected to prep HPLC to give compound (**98**). ¹H NMR 8.60 (doublet, 1H, Ar), 8.46 (quartet, 1H, Ar), 7.64 (quartet, 1H, Ar), 7.20(quartet, 1H, Ar), 5.98 (triplet, 1H, vinyl), 4.55 (doublet, 1H), 4.40 (doublet, 1H), 4.05 (triplet, 1H), 3.93 (triplet, 1H), 3.06 (doublet, 1H), 2.95(singlet, 1H), 2.25(multiplet, 1H), 2.05(multiplet, 2H), 0.99, (singlet, 3H, Me), 0.79 (singlet, 3H, Me) ¹³C NMR 170.08, 150.57, 146.94, 146.82, 132.65, 131.71, 131.13, 127.99, 122.05, 72.78, 58.06, 55.92, 46.50, 45.19, 43.07, 34.07, 32.96, 30.57, 29.51, 28.93, 23.65, 20.69, 15.67, 10.99 ESI-MS 381.0 HPLC 96.7% CH₃CN(10→100%)+H₂O(0.1% THA), 0.8 mL/min, 254 nm, zorbax Eclipse XDB-C8, 5 u (150×4.6 mm).

### Example 92

### Preparation of Compounds (99) and (100)

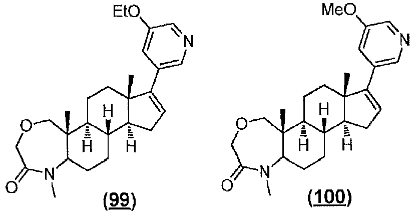

Using a synthetic procedure and conditions similar to Example 91 in the preparation of compound (**98**), replacing 3-pyridyl boronic acid with various substituted 3-pyridyl boronic acid compounds (**99**) and **(100)** were prepared.

**Compound (99):** ¹H NMR 8.19 (singlet, 1H, Ar), 8.13 (doublet, 1H, Ar), 7.11 (singlet, 1H, Ar), 5.96 (triplet, 1H, vinyl), 4.52 (doublet, 1H), 4.36 (doublet, 1H), 4.07 (mutiplet, 3H, OCH₂+H), 3.89 (doublet, 1H), 3.01 (triplet, 1H), 2.95 (singlet, 3H, -NMe), 2.22 (mutiplet, 1H),2.00 (mutiplet, 2H), 1.00 (singlet, 3H, Me), 0.80 (singlet, 3H, Me) ¹³C NMR 171.08, 154.67, 151.40, 140.20, 135.51, 133.32, 129.21, 73.79, 63.88, 59.07, 56.94, 47.52, 46.22, 44.07, 35.11, 33.96, 31.57, 30.51, 29.93, 24.65, 21.70, 16.72, 14.77, 12.00 ESI MS 425.1.

**Compound (100):** ¹H NMR 8.39 (singlet, 1H, Ar), 8.28 (singlet, 1H, Ar), 7.42 (singlet, 1H, Ar), 5.96 (triplet, 1H, vinyl), 4.52 (doublet, 1H), 4.36 (doublet, 1H), 3.89 (doublet, 1H), 3.00 (singlet, 3H), 3.01 (triplet, 1H), 2.95 (singlet, 3H, -NMe), 2.22 (mutiplet, 1H),2.00 (mutiplet, 2H), 1.00 (singlet, 3H, Me), 0.80 (singlet, 3H, Me) ¹³C NMR 171.11, 151.67, 148.43, 144.94, 134.41, 132.49, 132.27, 128.80, 73.80, 59.09, 56.96, 47.51, 46.23, 44.09, 35.13, 33.99, 31.57, 30.53, 29.95, 29.70, 24.67, 21.71, 18.46, 16.71, 12.02 ESI MS 395.0.

### Example 93

### Preparation of Compounds (101), (102), (103) and (104)

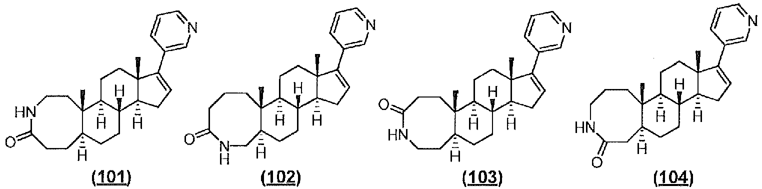

To a solution of a mixture of compound (**9c**) and compound (**9d**) (4.00 g, 11.5 mmol, 1 equiv.) in 150 mL of THF was added pyridine (1.86 mL, 23.1 mmol, 2 equiv.) and NH₂OH·HCl (1.60 g, 23.08 mmol, 2 equiv.) at room tempersature in turn. The reaction mixture was stirred at room temperature for 1 h. TLC (Hexanes/EtOAc=4/1, phosphomolybdic acid) showed the reaction completed and the two products with close polarity formed. The volatile solvents were removed under rotary evaporator. The residue was treated with 150 mL of CH₂Cl₂. The organic phase was washed with water (2x30 mL), dried on Na₂SO₄, and condensed under reduced pressure to provide 4.3 g of oximes. To a solution of these oximes (4.30 g, 11.5 mmol) and pyridine (4.70 g, 4.80 mL, 57.5 mmol) in 30 mL of CH₂Cl₂ was added dropwise thionyl chloride (0.850 mL, 11.5 mmol) at 0 °C within 5 min. The reaction mixture was stirred at low temperature for 10 min. TLC (Hexanes/EtOAc=4/1, phosphomolybdic acid) indicated the reaction came to the end. 60 mL of water was added. The mixture was extracted with EtOAc (3×30 mL). The combined organic phase was washed with water, dried on MgSO₄, and condensed under reduced pressure to give 4.2 g of a mixtures of lactams as solid. ESI-MS: 362. 4.2 g of this mixture lactams was treated with 60 mL of 5*N* HCl in 30 mL of THF at rt for 2 hr. THF was removed under reduced pressure. The residue was treated with EtOAc (3x30 mL). The combined organic layers was washed with water, dried on MgSO₄ and crystallized in EtOAc to give 2.7 of a mixture of ketones. ESI-MS: 318. To a solution of these ketones (1.00 g, 3.15 mmol) in 25 mL of THF was added 80% hydrazine hydrate (2 mL, 31.5 mmol). The reaction mixture was stirred at room temperature overnight. THF was removed by rotary evaporator. The rest aqueous phase was extracted with CH₂Cl₂ (280 mL). The organic phase was washed with water, dried and condensed to give 1.03 g of crude hydrazone. The further purification *via* FCC provided 680 mg of a mixture of hydrazones. ESI-MS: 332. To a mixture of molecular iodine (0.870 g, 3.45 mmol, 2 equiv.) and TMG (tetramethyl guanidine, 1.0 g, 8.60 mmol, 5 equiv.) in 45 mL of THF-Et₂O (2/1), was added a solution of the mixture of hydrazones (0.570 g, 1.72 mmol) in THF dropwise. The reaction mixture was stirred overnight at rt. TLC showed the reaction came to the end. 30 mL of water was added. THF was removed. The residue was extracted with EtOAc (3x30 mL). The combined EtOAc phase was washed with 5% Na₂SO3 (60 mL), dried on MgSO₄, condensed to give 680 mg of the vinyl iodides. The solution of the vinyl iodide (200 mg, 0.460 mmol) was treated with 3-pyridyl boronic acids (0.938 mmol, 2 equiv.) in the presence of Pd(Ph₃P)₂Cl₂ (0.05 equiv.) and potassium carbonate (5 equiv.) in THF-H₂O (8 mL-8mL). The mixture was degassed under argon in ultrasonic cleaner for 1.5 hr, and then was refluxed within an oil till the iodide was completely consumed. The reaction mixture was condensed and extracted with CH₂Cl₂. The CH₂Cl₂ layers was condensed and purified via prep.HPLC to give a mixture of compounds (**101**). (**102**), (**103**) and (**104**). ¹H NMR 8.60 (doublet, 1H, Ar), 8.43 (quartet, 1H, Ar), 7.64 (quintet, 1H, Ar), 7.20 (quartet, 1H, Ar), 5.97(quartet, 1H, vinyl), 2.70 (multiplet, 1H), 2.50 (multiplet, 1H), 2.55 (multiplet, 1H), 2.47 (multiplet, 1H), 2.22 (multiplet, 1H), 1.95 (multiplet, 3H), 1.90 (multiplet, 2H), 0.99, (singlet, 3H, Me), 0.798 (singlet, 3H, Me) ¹³C NMR 163.56, 162.43, 150. 63, 146.78, 132.72, 132.00, 128.26, 122.04, 56.42, 53.50, 52.79, 46.41, 46.22, 46.14, 41.45, 37.75, 35.69, 34.54, 32.72, 32.34, 31.03, 30.73, 30.63, 30.18, 28.68, 27.43, 20.64, 20.52, 18.23, 15.63, 11.24 ESI-MS 379.1 HPLC 95.9% Rt=10.9 CH₃CN(60→100%)+H₂O(0.1% THA), 0.8 mL/min, 242nm, zorbax Eclipse XDB-C8, 5 u (150×4.6 mm).

### Example 94

### Preparation of Compound (105)

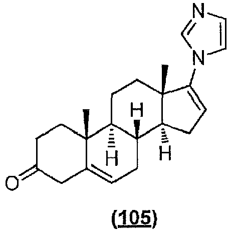

A solution of (COCl)₂ (0.950 ml, 11.2 mmol) in dry DCM (35 ml) was cooled to -75°C under Ar atmosphere. DMSO (2.40 ml, 33.6 mmol) was added and stirred for 1 h at this temperature. Compound (**49c**) (1.90 g, 5.61 mmol) was added and stirred for 2hrs, followed by adding DIPEA (5.00 ml) and stirred overnight. To the resulting mixture was added 10% NaHCO₃ (40 ml). The mixture was partitioned. The aqueous layer was extracted with dichloromethane (2x25 ml). The combined organic layers were dried on Na₂SO₄ and concentrated to provide compound (**105**) (1.83 g). ¹H NMR (CDCl₃, 400 MHz): 7.61 (1H), 7.10 (1H), 7.09 (1H), 5.73(1H), 5.40 (1H),1.5-2.38 (15H), 1.10-1.18 (5H), 1.03-1.1 (3H). ¹³C NMR (CDCl₃, 400 MHz): 209.82, 148.41, 138.95, 136.53, 129.61, 122.23, 129.01, 118.30, 56.03, 49.48, 48.32, 46.20, 37.53, 37.08, 36.80, 34.66, 30.91, 30.22, 29.72, 20.86, 19.28, 15.91.

### Example 95

### Preparation of Compound (106) and Compound (107)

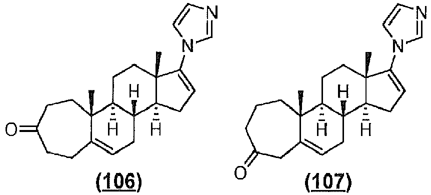

To a solution of compound (**105**) (200 mg, 0.600 mmol) in dry dichloromethane (30 ml), was added ethyl diazoacetate (342 mg, 3.00 mmol) under Ar atmosphere and cooled to -75 °C. BF₃.Et₂O (425 mg, 3.0 mmol) added and stirred for 2 hrs at -75 °C. The resulting was allowed to warm to room temperature and stirred overnight. The mixture was treated with 10% NaHCO₃ (30 ml) for 2 hr. The mixture was partitioned. The aqueous layers were extracted with CH₂Cl₂ (2×25ml). The combined organic layers were dried on Na₂SO₄ and condensed under reduced pressure. Purification by pre HPLC provided compound (**106**) (10 mg) and compound (**107**) (24 mg). Compound (**106**): ¹H NMR 7.58 (singlet, 1H, imidazolyl), 7.04 (1H, imidazolyl), 6.99 (1H, imidazolyl), 5.62 (1H, vinyl), 5.53 (1H, vinyl), 2.54-2.62 (multiplet, 1H). ¹³C NMR 212.9, 148.5, 143.1, 135.9, 129.4, 124.9, 118.9, 118.3, 56.4, 46.1, 45.0, 44.0, 38.8, 34.8, 31.9, 31.0, 30.6, 30.1, 29.7, 27.9, 22.7, 21.9, 21.1, 15.9. ESI MS: 351.0 Compound (**107**): ¹H NMR 7.62 (singlet, 1H, imidazolyl), 7.03 (1H, imidazolyl), 6.99 (1H, imidazolyl), 5.68 (1H, vinyl), 5.60 (1H, vinyl), 3.25-3.30 (doublet, 1H), 2.82-2.87 (doublet, 1H), 2.57-2.64 (mutiplet, 1 H), 1.0 (3H),1.02 (3H). ¹³C NMR 210.5, 148.4, 137.6, 135.9, 129.4, 126.7, 118.9, 118.2, 56.4, 48.6, 46.2, 44.4, 42.5, 39.9, 34.8, 34.7, 30.8, 30.1, 29.7, 22.7, 20.9, 22.7, 20.9 18.5, 15.9. ESI MS: 351.2.

### Example 96

### Preparation of Compound (108)

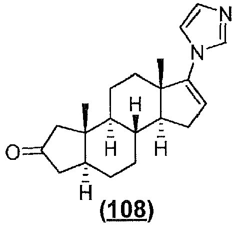

### Example 96A

### Preparation of Compound (108a)

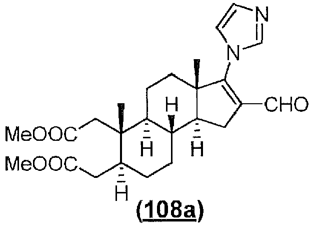

Compound (**27**) (560 mg, 1.28 mmol) was treated with imidazole (275 mg, 4.0 mmol) and potassium carbonate (663 mg, 4.8 mmol) in 10 mL of DMF at 80 °C for 1 hr. The reaction was poured into icy water, and was extracted with dichloromethane (2x50 mL). The combined organic phases were washed with 10% citric acid and saturated brine, dried on magnesium sulfate and condensed by rotary evaporator to give 420 mg of compound **(108a)** in 70% yield..

### Example 96B

### Preparation of Compound (108b)

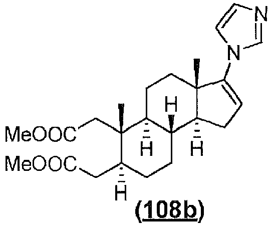

A mixture of compound **(108a)** (420 mg) and Pd/C (850 mg, the catalyst was pretreated with absolute methanol) in benzonitrile (10 mL) was heated at 200 °C for 4 hr. The solvent was removed under reduced pressure. The residue was treated with a mixture of dichloromethane (20 mL) and water (20 mL). The organic phase was partitioned, washed with brine and purified through FCC on silica gel to give 230 mg of compound (**108b**) in 57% yield. ¹H NMR: 7.56 (singlet, 1H, imidazole), 6.97-7.05 (dd, 2H, imidazole), 5.61 (1H, vinyl), 4.00-4.10 (4H, 2CH₃CH₂O-), 2.65 (d, 1H), 2.48 (d, 1H), 2.20 (2H), 1.80-2.20 (5H), 0.90 (3H), 0.80 (3H). ¹³C NMR 173.6, 171.3, 148.8, 136.2, 129.6, 119.3, 118.6, 60.5, 60.4, 56.3, 48.9, 46.4, 41.3, 40.8, 40.1, 36.3, 35.1, 33.9, 30.9, 29.9, 27.7, 21.8, 16.3, 15.9, 14.6, 14.5.

### Example 96C

### Preparation of Compound (108c)

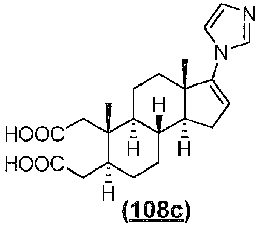

A solution of compound (**108b**) (13.0 g, 29.3 mmol) in 100 mL of methanol was treated with LiOH.H₂O (15.0 g, 176 mmol, 6 equiv.) in 30 mL of water at reflux overnight. Methanol was removed under reduced pressure. The aqueous phase was washed with cool EtOAc (3x40mL), and then adjusted with 5 M HCl to pH→2. A mass of white solid precipitated and was collected by filtration. After dried under reduced pressure, 7.4 g of compound (**108c**) was obtained.

### Example 96D

### Preparation of Compound (108)

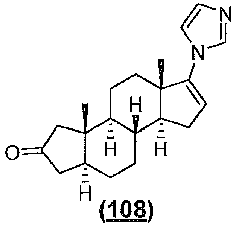

A mixture of compound (**108c**) (1.00 g), acetic anhydride (80 mL) and sodium acetate (210 mg) was heated at reflux overnight. The volatile solvents were removed under reduced pressure. The residue was added into 100 mL of water, and was extracted with dichloromethane (3x150 mL). The combined organic layers were washed with 10% sodium biscarbonate and brine, dried on magnesium sulfate. Purification through prep HPLC provided 80 mg of compound (**108**).

### Example 97

### Preparation of Compound (109)

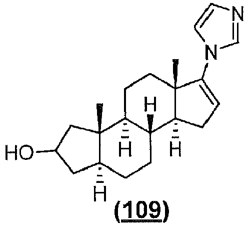

To a solution of compound (**108**) (125 mg, 0.385 mmol) in 5 mL of THF in ice-bath was added NaBH₄ (30.0 mg, 0.770 mmol, 2 equiv.), followed by adding 10 mL of MeOH. The mixture was stirred at -5 °C for 10 min. TLC showed minor compound **(108)** haven't been consumed. An additional 20.0 mg of NaBH₄ was added. After being stirred for 10 min, TLC indicated the starting material has been completely consumed. The solvents were removed under reduced pressure. The residue was partitioned between 20 mL of CH₂Cl₂ and 10 mL of water. The water phase was re-extracted with CH₂Cl₂ twice (each at 15 mL). The combined organic payers were washed with brine, dried on Na₂SO₄ and condensed under rotary evaporator to give the crude. Purification through preparative HPLC provided 40 mg of compound (**109**). ¹H NMR: (CDCl₃, 400 M Hz) 7.60 (1H, imidazolyl), 6.98-7.15 (2H, imidazolyl), 5.68 (1H, vinyl), 4.43 (1H, -CHOH), 0.99 (3H), 0.96 (3H); ESI MS 327.0 (M+H⁺).

### Example 98

### Preparation of Compound (110)

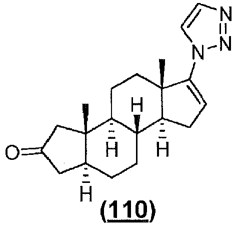

Using a synthetic procedure and conditions similar to Examples 96A to 96D in the preparation of compound (**108**), replacing imidazole with triazole, compound **(110)** was prepared.

### Example 99

### Preparation of Compound (111)

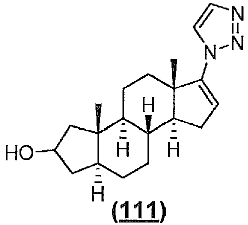

To a solution of compound **(110)** (50 mg) in 3 mL of THF and 10 mL of MeOH was added NaBH₄ (10 mg) within an ice water. The reaction was allowed to warm to rt. The solvents were removed under reduced pressure. The residue was partitioned with 20 mL of CH₂Cl₂ and 20 mL of water. The organic layer was washed with brine, dried on Na₂SO₄ and condensed under reduced pressure to get the crude. Further purification through prep HPLC provided 15.6 mg of compound (**110**). ESI MS: 350.2 (M+Na⁺).

### Example 100

### Preparation of Compounds (112) and (113)

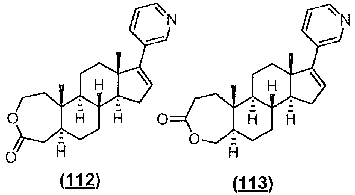

To a solution of compound (**7**) and compound (**8**) (0.6 g) in THF (30 mL) was added diethyl (3-pyridyl)borane (0.255 g), bis(triphenylphosphine)palladium(II) chloride (51 mg) and 2 N aqueous Na₂CO₃ (0.45 g). The mixture was degassed and refilled with Argon gas three times. The mixture was heated at 80 °C overnight. The reaction was monitored by TLC. The mixture was cool to room temperature and extracted with EtOAc (2 X 20 mL). The organic layers were combined, washed with brine (2 X 20 mL) and dried over Na₂SO₄. The solution was concentrated and purified by column chromatography on silica gel (EtOAc/Hexanes=1:5) to give the mixture of compound (**112**) and compound (**113**) (0.4 g, 79.6%). The regioisomeric mixture was purified by preparative HPLC to isolate compound (**112**) and compound (**113**). Compound (**112**): Retention time at HPLC: 10.426 min [Mobile phase: B%=10-100 (gradient 20 min); B=MeCN, A=H₂O (0.1%TFA); Flow rate: 0.8 mL/min; UV=266 nm column: zorbax Eclipse XDB-C8 5u, (150X4.6 mmID)]. MS calculated for (C₂₄H₃₁NO₂) [M+H]+ 366.51 Found: 366.5 ; [2M+Na]+ 754.02 Found: 753.6 ¹H NMR (CDCl₃, 300 MHz): 8.65(1H), 8.45(1H), 7.6-7.7(1H), 7.19(1H), 6.0(1H), 4.26-4.31(1H), 4.12(1H), 2.8-2.9(1H), 2.2-2.3(1H), 2.1-2.13(1H), 1.9-2.05(2H). ¹³C NMR (CDCl₃, 300 MHz): 175.84, 151.51, 147.91, 147.82, 133.69, 132.82, 129.18, 123.05, 64.60, 57.11, 53.98. Compound (**113**): Retention time at HPLC: 10.56 min [Mobile phase: B%=10-100 (gradient 20 min); B=MeCN, A=H₂O 0.1%TFA); Flow rate: 0.8 mL/min; UV=266 nm column : zorbax Eclipse XDB-C8 5u, (150*4.6mmID)]. MS calculated for (C₂₄H₃₁NO₂) [M+H]+ 366.51 Found: 366.4; [2M+Na]+ 754.02 Found: 753.5. ¹H NMR (CDCl₃, 300 MHz): 8.65(1H), 8.45(1H), 7.65(1H), 7.18(1H), 6.0(1H), 4.25(1H), 3.68(1H), 2.7(1H), 2.5(1H). ¹³C NMR (CDCl₃, 300 MHz): 176.08, 151.55, 147.92, 147.84, 133.66, 132.80, 129.09, 123.05, 69.98, 57.12, 53.78, 48.74, 47.25.

### Example 101

### Preparation of Compounds (114) and (115)

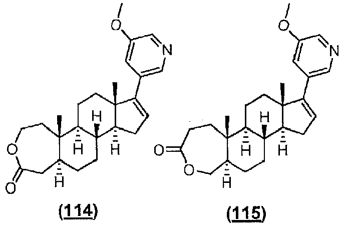

Using a synthetic procedure and condition similar to Examples 100 in the preparation of compound (**112**) and compound (**113**) replacing diethyl (3-pyridyl)borane with 5-methoxypyridin-3-ylboronic acid, compound (**114**) and compound (**115**) were made.

### Example 102

### Preparation of Compounds (116) and (117)

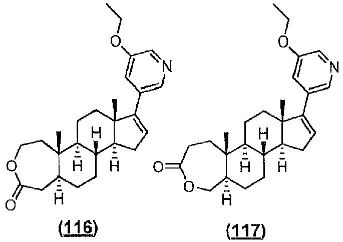

Using a synthetic procedure and condition similar to Examples 100 in the preparation of compound (**112**) and compound (**113**) replacing diethyl (3-pyridyl)borane with 5-ethoxypyridin-3-ylboronic acid, compound **(116)** and compound **(117)** were made.

### Example 103

### Preparation of Compounds (118) and (119)

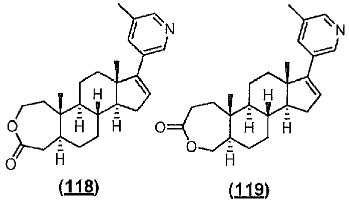

Using a synthetic procedure and condition similar to Examples 100 in the preparation of compound (**112**) and compound (**113**) replacing diethyl (3-pyridyl)borane with 5-methylypyridin-3-ylboronic acid, compound (**118**) and compound (**119**) are made.

### Example 104

### Preparation of Compound (120)

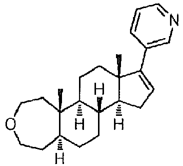

Within an ice-water bath, BF₃·Et₂O (0.03 mL) was dissolved in 1 mL of CH₂Cl₂. The solution was added dropwise to a solution of TMSOTf (0.1 mL) at rt. After being stirred at rt for 2 hr, the resulting mixture was re-cooled within an ice-water bath. A solution of compound (**112**) (120 mg) and Et₃SiH (0.1 mL) was added dropwise to the above-mentioned mixture. The solvents were removed under reduced pressure. The residue was partitioned between 15 mL of EtOAc and 10 mL of saturated aqueous NaHCO₃. The water phase was re-extracted with EtOAc (15 mL). The combined organic phase was washed with brine, dried on MgSO₄ and condensed under reduced pressure to give the crude. Purification through FCC on silica gel eluenting with hexanes/CH₂Cl₂ (1/1)→pure CH₂Cl₂→EtOAc/Hexanes (1/4) provided the cyclic ether compound (**120**). ¹H NMR 8.63 (doublet, 1H, Ar), 8.50 (quartet, 1H, Ar), 7.68(quintet, 1H, Ar), 7.25(quartet, 1H, Ar), 6.00 (quartet, 1H, vinyl), 3.78(multiplet, 2H), 3.65(multiplet, 2H), 2.25(mutiplet, 1H), 2.00 (mutiplet, 2H), 1.85(mutiplet, 1H), 0.99, (singlet, 3H, Me), 0.798 (singlet, 3H, Me) ¹³C NMR 150.15, 145.84, 133.46, 132.42, 128.92, 122.35, 63.42, 56.57, 52.91, 46.31, 45.84, 41.42, 37.64, 34.57, 32.97, 32.42, 30.96, ESI-MS 352.0.

### Example 105

### Preparation of Compounds (121), (122), (123), (124), (125), (126) and (127)

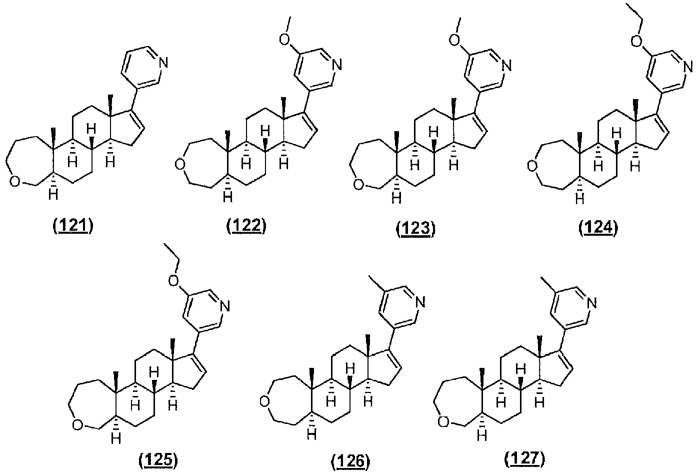

Using a synthetic procedure and conditions similar to Example 104 in the preparation of compound (**120**), replacing compound (**112**) with various compounds (**113 - 119**)), compounds (**121 - 127**) were prepared.

**Compound (121):** ¹H NMR 8.63 (doublet, 1H, Ar), 8.50 (quartet, 1H, Ar), 7.68 (quintet, 1H, Ar), 7.25 (quartet, 1H, Ar), 6.00 (quartet, 1H, vinyl), 3.60-3.78 (multiplet, 2H), 3.55 (multiplet, 2H), 2.25 (mutiplet, 1H), 2.00 (mutiplet, 2H), 1.85 (mutiplet, 1H), 0.99, (singlet, 3H, Me), 0.798 (singlet, 3H, Me) ¹³C NMR 150.54, 146.17, 133.18, 132.13, 128.62, 122.14, 56.51, 52.85, 48.55, 46.34, 37.88, 37.41, 34.62, 33.03, 30.76 ESI-MS 352.0.

**Compound (123):** ¹H NMR 8.25 (singlet, 1H, Ar), 8.17 (singlet, 1H, Ar), 7.16 (singlet, 1H, Ar), 6.00 (singlet, 1H, vinyl), 3.86 (singlet, 3H, OMe), 3.60-3.78 (multiplet, 2H), 3.55 (multiplet, 2H), 2.24 (mutiplet, 1H), 2.05 (mutiplet, 2H), 1.00 (singlet, 3H, Me), 0.80 (singlet, 3H, Me) ¹³C NMR 154.46, 150.26, 138.65, 133.16, 132.92, 129.01, 118.41, 67.61, 56.51, 54.62, 52.85, 48.54, 46.38, 37.87, 34.65, 33.02, ESI MS 382.1.

**Compound (124):** ¹H NMR 8.22 (singlet, 1H, Ar), 8.13 (singlet, 1H, Ar), 7.15 (singlet, 1H, Ar), 5.97 (triplet, 1H, vinyl),4.07 (quartet, 2H, OCH₂), 3.78 (multiplet, 2H), 3.65 (multiplet, 2H), 2.30 (singlet, 3H,-Me), 2.25(mutiplet, 1H), 2.00 (mutiplet, 2H), 1.85 (mutiplet, 1H), 0.99, (singlet, 3H, Me), 0.798 (singlet, 3H, Me) ¹³C NMR 154.71, 151.46, 139.97, 135.08, 135.70, 133.70, 129.60, 119.58, 67.09, 64.43, 63.90, 57.58, 53.96, 47.34, 46.86, 42.12, 38.64, 35.65, 33.99, 33.44, 31.96, 31.73, 30.46, 21.76, 16.70, 14.74, 11.88 ESI-MS 396.1.

**Compound (125):** ¹H NMR 8.22 (singlet, 1H, Ar), 8.13 (singlet, 1H, Ar), 7.15 (singlet, 1H, Ar), 5.97 (triplet, 1H, vinyl),4.07 (quartet, 2H, OCH₂), 3.60-3.78 (multiplet, 2H), 3.55 (multiplet, 2H), 2.30 (singlet, 3H,-Me), 2.25 (mutiplet, 1H), 2.00 (mutiplet, 2H), 1.85 (mutiplet, 1H), 0.99, (singlet, 3H, Me), 0.798 (singlet, 3H, Me) ¹³C NMR 154.73, 151.45, 139.95, 135.04, 133.71, 129.61, 119.60, 68.62, 63.91, 57.52, 53.88, 49.55, 47.37, 38.89, 38.41, 35.67, 34.03, 31.92, 31.74, 31.47, 29.69, 29.36, 25.65, 25.41, 22.69, 21.57, 16.72, 14.76, 14.12, 12.49, ESI MS 396.1.

**Compound (126):** ¹H NMR 8.41(singlet, 1H, Ar), 8.31(singlet, 1H, Ar), 7.50(singlet, 1H, Ar), 6.00 (triplet, 1H, vinyl), 3.78(multiplet, 2H), 3.65(multiplet, 2H), 2.30 (singlet, 3H,-Me), 2.25(mutiplet, 1H), 2.00 (mutiplet, 2H), 1.85(mutiplet, 1H), 0.99, (singlet, 3H, Me), 0.798 (singlet, 3H, Me) ¹³C NMR 151.65, 147.84, 144.59, 134.65, 132.65, 129.25, 64.63, 57.59, 53.95, 47.31, 46.86, 42.12, 38.65, 35.64, 33.98, 33.45, 31.99, 31.73, 30.48, 21.75, 18.46, 16.69, 11.89 ESI-MS 366.1.

**Compound (127):** ¹H NMR 8.41(singlet, 1H, Ar), 8.31(singlet, 1H, Ar), 7.50(singlet, 1H, Ar), 6.00 (triplet, 1H, vinyl), 3.60-3.78(multiplet, 2H), 3.55(multiplet, 2H), 2.30 (singlet, 3H,-Me), 2.25(mutiplet, 1H), 2.00 (mutiplet, 2H), 1.85(mutiplet, 1H), 0.99, (singlet, 3H, Me), 0.798 (singlet, 3H, Me) ¹³C NMR 150.59, 146.54, 143.31, 133.92, 131.79, 131.72, 128.42, 67.61, 56.55, 52.89, 48.55, 46.35, 37.91, 37.42, 34.68, 33.06, 30.74, ESI-MS 366.1, HPLC 96.5.

### Example 106

### Preparation of Compound (128)

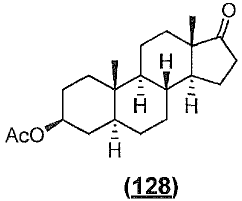

Epiandrosterone (50.0 g, 172 mmol) was dissolved to 300 mL of pyridine, followed by the addition of 25.5 g of Ac₂O and 430 mg of DMAP with stirring. The resulting mixture was heated at 85 °C overnight. After being cooled to room temperature the mixture was admixed with 700 mL of ice-water. 500 mL of EtOAc was added. The layers were partitioned. The water phase was extracted with 500 mL of EtOAc once again. The combined organic layers were washed with 5% citric acid, 5% NaHCO₃ and brine in turn. After being dried over MgSO₄, EtOAc was evaporated under reduced pressure until 80% of EtOAc was removed. The left organic solution was washed with 1 M HCl to get rid of pyridine, followed by washing 10% NaHCO₃ and brine. The organic phase was dried on MgSO₄ and condensed under reduced pressure to give 48.3 g of compound (**128**).

### Example 107

### Preparation of Compound (129)

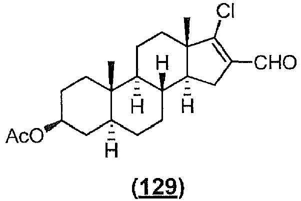

Within an ice-water bath, 5.00 mL of DMF was pre-mixed with 5.00 mL of POCl₃ in 5 mL of CHCl₃ under argon protection. The resulting reagent was added dropwise to a solution of 1.00 g of compound (**128**) in 15.0 mL of CHCl₃ within an ice bath. The reaction mixture was allowed to warm to room temperature and heated at reflux for 5 hr until the starting material was completely consumed. After being cooled to room temperature, the mixture was poured slowly into 20 g of ice-water mixture. Extracted with ether/EtOAc (1/4), dried on MgSO₄, and purified through FCC to give 450 mg of compound **(129)** as yellowish solid.

### Example 108

### Preparation of Compound (130a) and Compound (130)

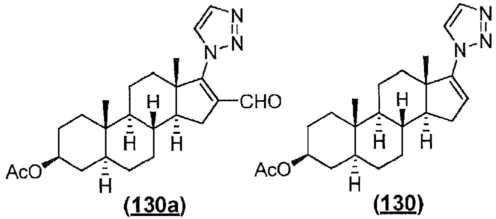

The mixture of compound **(129)** (18.0 g, 47.5 mmol), 1*H*-1,2,3-triazole (10.3 g, 148 mmol), and K₂CO₃ (24.6 g, 178 mmol) in 300 mL of DMF was heated at 85 °C with stirring for 1 hr. After being cooled to room temperature, the mixture was treated with 600 mL of ice-water mixture. The resulting mixture was extracted with EtOAc three times (each 400 mL). The combined organic layers were washed with 5% citric acid three times (each 200 mL) and brine once (200 mL). The solution was dried on MgSO₄ and condensed under reduced pressure to get the crude. Purification through FCC on silica gel (eluent: EtOAc/Hexanes=1/8→1/3) gave 13.0 g of compound (**130a**). A wet Pd/C was pre-dehydrated through washing with MeOH and PhCN. Then, 10 g of dry Pd/C (10%) was added to a solution of compound (**130a**) (5.00 g). After being degassed within an ultrasonic cleaner for 30 min, the mixture was heated at reflux overnight. The palladium on charcoal was removed by filtration. The filtrate was purified through FCC on silica gel (eluent: EtOAc/Hexanes=1/1) to give 3.70 g of compound **(130).** as a white solid.....

### Example 109

### Preparation of Compound (131)

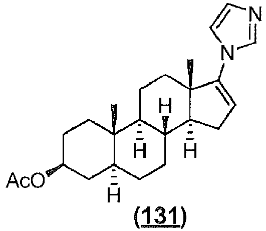

Usingr a synthetic procedure and conditions similar to Example 108 in the preparation of compound (**130**), replacing 1*H*-1,2,3-triazole with imidazole, compound (**131**) was prepared.

### Example 110

### Preparation of Compound (132)

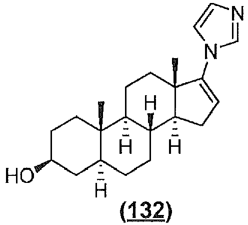

A solution of compound (**131**) (1.50 g, 3.92 mmol) in 40 mL of methanol was treated with potassium hydroxide (0.660 g, 11.8 mmol) at room temperature for 3 hr. TLC indicated that the reaction came to the end. The solvent was removed under reduced pressure. The residue was treated with 30 mL of water and extracted with EtOAc (2x30 mL). The combined organic layers were washed with brine, dried on magnesium sulfate, and condensed by rotary evaporator to give compound (**132**) (1.30 g, 97.4%).

### Example 111

### Preparation of Compound (133)

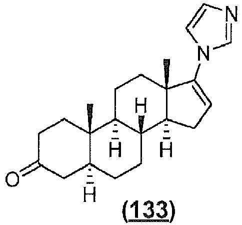

To a solution of compound (**132**) (1.30 g, 3.82 mmol) in 50 mL of dichloromethane was added Des Martin Periodinane (DMP) (3.30 g, 7.64 mmol) at room temperature. The mixture was heated to reflux for 4 hr. After being cooled to room temperature, the undissolved solid was removed by filtration. The filtrate was condensed under reduced pressure and purified through FCC on silica gel (eluent: dichloromethane/ methanol=50/1) to give compound (**133**) (1.23 g, 95.3%).

### Example 112

### Preparation of Compound (134)

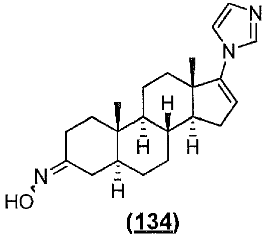

To a solution of compound (**133**) (1.23 g, 3.63 mmol) in 50 mL of ethanol was added hydroxylamine hydrochloride ( 0.510 g, 7.26 mmol) and pyridine (0.580 g, 7.26 mmol) at room temperature. The reaction mixture was stirred at room temperature for 5 hr. The solvents were removed under reduced pressure. The residue was dissolved into 50 mL of EtOAc, washed with brine (2x20 mL), dried on sodium sulfate and condensed by rotary evaporator to give 1.10 g of compound (**134**) in 85.9% yield.

### Example 113

### Preparation of Compound (135) and Compound (136)

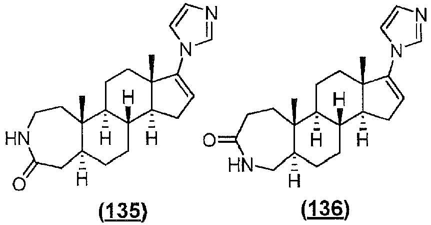

A catalytic To a solution of compound **(134)** (1.00 g, 2.83 mmol) in 100 mL of dichloromethane at 0 °C, was added dropwise 3 mL of thionyl chloride keeping the temperature below 0 °C. At that temperature, the reaction mixture was stirred for 20 min. The reaction was slowly poured into 100 mL of icy saturated sodium biscarbonate. The layers were portioned. The organic phase was washed with water (2x20 mL), dried on magnesium, and condensed under reduced pressure to give 0.840 g of the mixture of compound (**135**) and compound (**136**), which was subjected to the isolation by preparative HPLC to give 21 mg of compound (**135**) and 23 mg of compound (**164**).

**Compound** (**135**): ¹H NMR 7.61 (s, 1H, imidazole), 7.02-7.09 (dd, 2H, imidazole), 5.68 (1H, vinyl), 3.35 (m, 1H), 3.00 (m, 1H), 2.77 (m, 1H), 2.25 (1H); ¹³C NMR 177.5, 147.3, 134.8, 128.0, 118.1, 117.3, 54.9, 53.4, 45.1, 42.4, 40.8, 38.5, 38.0, 36.8, 33.9, 31.9, 30.3, 29.7, 28.6, 20.0, 14.9, 10.9.

**Compound** (**136**): ¹H NMR 7.61 (s, 1H, imidazole), 7.02-7.09 (dd, 2H, imidazole), 5.68 (1H, vinyl), 3.35 (m, 1H), 2.70 (m, 2H), 2.22 (m, 2H); ¹³C NMR 177.6, 147.4, 134.9, 128.2, 117.9, 117.2, 54.9, 53.1, 48.8, 45.2, 43.4, 37.9, 34.1, 33.9, 32.3, 30.3, 29.9, 28.6, 26.5, 19.8, 14.9, 11.0.

### Example 114

### Preparation of Compound (137) and Compound (138)

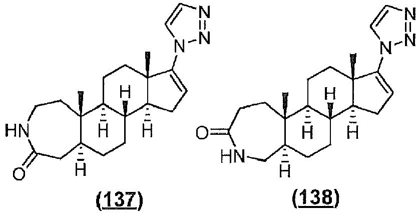

Usingr a synthetic procedure and conditions similar to Examples 111-113 in the preparation of compound (**135**) and compound (**136**), replacing compound (**132**), with compound (**130**), compound (**137**) and compound (**138**), wer prepared.

### Example 115

### Preparation of Compound (139) and Compound (140)

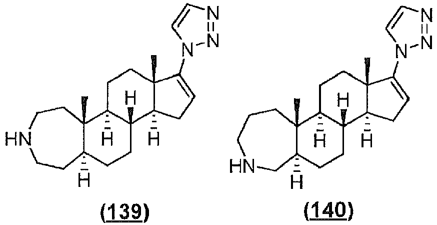

630 mg of LiAlH₄ was suspended in 60.0 mL of THF within an ice-water bath. 500 mg of Compound **(137)** and compound (**138**) was added to the suspension. The formed mixture was stirred at reflux overnight. After being cooled to room temperature, 10 L of methanol was carefully added to quench the reaction until no gas was released. The formed mixture was directly loaded on silica gel and eluented with MeOH/TEA (10/1). The collected eluents were condensed to get 425 mg of the crude mixture, which was then subjected to preparative HPLC isolation to give compound (**139**) and compound (**140**).

**Compound** (**139**): ¹H NMR (400M, CDCl₃) 7.69 (2H), 5.96 (1H), 3.00 (2H), 2.6-2.8 (2H), 1.11 (3H), 0.89 (3H); ESI MS: 341.3 (M+H⁺).

**Compound (140):** ¹H NMR (400M, CDCl₃) 7.69 (2H), 5.96 (1H), 2.80-3.15 (4H), 2.16-2.34 (2H), 2.00-2.10 (1H), 1.11 (3H), 0.89 (3H); ESI MS: 341.3 (M+H⁺).

### Example 116

### Preparation of Compound (141)

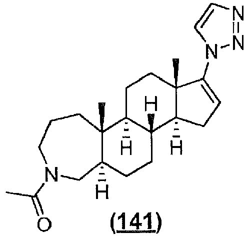

110 mg of compound **(140)** was dissolved in 10.0 mL of pyridine and 110 mg of acetic anhydride and 10.0 mg of DMAP were added in turn. The formed solution was stirred at room temperature overnight. The reaction mixture was partitioned with 25 mL of water and 50 mL of CH₂Cl₂. The water layer was re-extracted with CH₂Cl₂ (50 mL). The combined organic layers was washed with 5% citric acid, brine, dried on MgSO₄, and condensed under reduced pressure to get a crude product as a yellowish solid. Purification through prep HPLC provided 12.8 mg of compound (**141**). ESI MS: 383.3 (M+H⁺)

### Example 117

### Preparation of Compound (142)

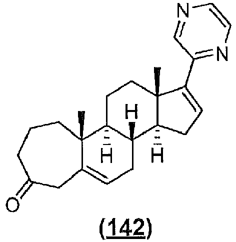

### Example 117A

### Preparation of Compound (142a)

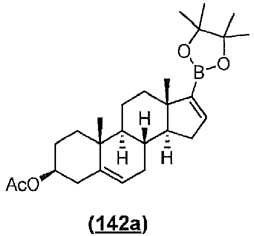

To a solution of compound (**3**) (3.70 g, 8 mmol) in dioxane (60 mL) was added Pin₂B₂ (2.23 g, 8.8 mmol, 1.1 equiv.), KOAc (1.18 g, 12 mmol, 1.5 equiv.), PPh₃ (444 mg, 12%), and Pd(PPh₃)₂Cl₂ (111 mg, 3%). The reaction mixture was degassed within an ultrasonic cleaner for 1 hr. Under argon protection, the reaction was stirred at 90 °C within an oil bath for 3 hr. The mixture was cooled to room temperature. The solvents were removed. The residue was partitioned between EtOAc and water. The water layer was extracted with EtOAc twice. The combined organic layers were washed with brine, dried over MgSO₄, condensed with rotovapor and purified through FCC to give 2.50 g of compound (**142a**). Characteristic analytical data: ¹H NMR 6.46 (1H, vinyl), 5.38 (1 H, vinyl), 4.54-4.62 (m, 1H, AcOCH), 1.25 (12H).

### Example 117B

### Preparation of Compound (142b)

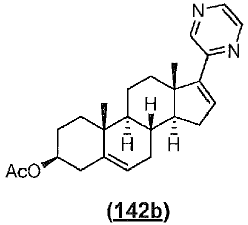

To a solution of compound **(142a)** (1.00 g, 2.27 mmol) in dioxane (30 mL) was added K₂CO₃ (1.57 g, 11.4 mmol, 5 equiv.), Pd(PPh₃)₂Cl₂ (30 mg, 3%) and 2-bromopyrazine (720 mg, 4.45 mmol, 2 equiv.). The mixture was degassed within an ultrasonic cleaner for 1 hr. Under argon protection, the reaction mixture was stirred at 90 °C for 4 hr. The mixture was cooled to room temperature. The solvents were evaporated under reduced pressure. The residue was purified through FCC to give 460 mg of compound **(142b).** Characteristic analytical data: ¹H NMR 8.67 (1H), 8.46 (1H), 8.32 (1H), 6.47 (1H, vinyl), 5.39 (1H), 4.54-4.62 (m, 1H, AcOCH),

### Example 117C

### Preparation of Compound (142c)

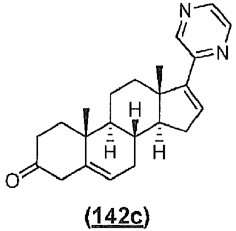

K₂CO₃ (460 mg, 3.36 mmol, 3 equiv.) was added to a solution of compound (**142b**) (440 mg, 1.12 mmol) and stirred at room temperature overnight. TLC (Hexanes/EtOAc=6/1) showed the reaction came to the end. The solid was filtrated. The filtrate was condensed by rotovapor. The residue was purified through FCC to give 360 mg of the hydrolysed product. Characteristic analytical data: ¹H NMR 8.67 (1H), 8.46 (1H), 8.32 (1H), 6.47 (1H, vinyl), 5.39 (1H), 3.48-3.60 (m, 1H). DMSO (0.53 mL, 6 mmol, 6 equiv.) was added to a solution of ClCOCOCl (0.173 mL, 2.05 mmol, 2 equiv.) at -70 °C and stirred at low temperature for 30 min. A solution of the above product (369 mg, 1.03 mmol) in 45 mL of CH₂Cl₂ was added at -70 °C and stirred for hr. 1 mL of DIPEA was added and the mixture was allowed to warm to room temperature overnight. 20 mL of 10% NaHCO₃ was added. The layers were partitioned. The water layer was extracted with CH₂Cl₂ twice. The combined organic phases were washed with brine, dried on Na2S04 and condensed by rotovapor to give 340 mg of compound (**142c**) Characteristic analytical data: ¹H NMR 8.67 (1H), 8.46 (1H), 8.32 (1H), 6.47 (1H, vinyl), 5.39 (1H), 3.27-3.32 (d, 1H), 2.82-2.87 (d, 1H).

### Example 117D

### Preparation of Compound (142)

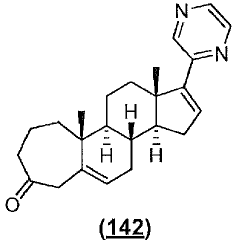

To a solution of compound (**142c**) (340 mg, 1 mmol) in CH₂Cl₂ (60 mL) was added ethyl diazoacetate (572 mg, 5 mmol, 5 equiv.) with stirring at -78 °C, followed by adding BF₃·Et₂O (709 mh, 5 mmol, 5 equiv.) at -78 °C. The mixture was stirred for 2 hr at low temperature. 10% NaHCO₃ was added. Partitioned, extracted, dried and condensed to give the crude. The crude was dissolved in 15 mL of methanol, followed by adding 10 mL of 1 mol/L NaOH and stirred overnight. The volatiles were evaporated. The residue was extracted, washed, and dried to provide the crude product. Purification through prep HPLC provided 16 mg compound (**142**). Ret. time at HPLC: 10.774 min [Mobile phase: B%=10-100(gradient 20 min); B=MeCN, A=H2O (0.1%TFA); Flow rate: 0.8mL/mm; UV=266 nm column: zorbax Eclipse XDB-C8 5u, (150*4.6 mmID)]. ¹H NMR 8.68 (1H), 8.46 (1H), 8.32 (1H), 6.48 (1H, vinyl), 5.60(1H), 3.25-3.29 (d, 1H, *J*=14.4), 2.83-2.86 (d, 1H, *J*=14.4); 2.57-2.68 (m, 1H); ESI MS 362.7 [M+H]⁺.

### Example 118

### Preparation of Compound (143)

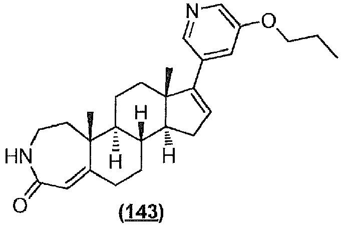

To a solution of compound (**19**) (300 mg, 0.73 mmol) in 1,4-dioxane (10 mL) was added 5-propoxypyridin-3-ylboronic acid (265 mg, 1.46 mmol), potassium carbonate (604 mg, 4.38 mmol) in water (1 mL), and Pd(dppf)Cl₂ (107 mg). The mixture was thoroughly degassed and heated under nitrogen at 80 °C overnight. After being filtered through a pad of Celite, the crude product was purified by silica gel chromatography (ethyl acetate) to give compound (**143**) (76 mg, 25%) as a white solid. LC-MS (m/z) 421 [M+H]⁺. ¹H-NMR δ (400 MHz, CHCl₃-*d*) major characteristic peaks: 1.05 (m, 6H), 1.20 (s, 3H), 1.82 (m, 4H), 2.56 (m, 1H), 3.17 (m, 1H), 3.25 (m, 1H), 3.97 (t, *J*= 6.4 Hz, 2H), 5.77 (s, 1H), 5.99 (m, 1H), 6.28 (s, 1H), 7.15 (s, 1H), 8.16 (d, *J* = 2.4 Hz, 1H), 8.21 (s, 1H).

### Example 119

### Preparation of Compound (144)

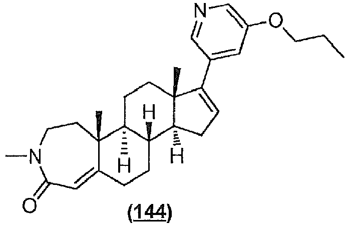

To a solution of compound (**20**) (500 mg, 1.18 mmol) in 1,4-dioxane (20 mL) was added 5-propoxypyridin-3-ylboronic acid (365 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 1 hour. After being filtered through a pad of Celite, the crude product was purified by silica gel chromatography (ethyl acetate in petroleum ether, 50% v/v) to give compound (**144**) (180 mg, 39%) as a yellow solid. LC-MS (m/z) 435 [M+H]⁺ . ¹H-NMR δ (400 MHz, CHCl₃-*d*) major characteristic peaks: 1.05 (m, 6H), 1.16 (s,3H), 3.04 (s, 3H), 3.97 (t, *J* = 5.2 Hz, 2H), 5.83 (s, 1H), 5.98 (s, 1H), 7.14 (s, 1H), 8.16 (s, 1H), 8.21 (s, 1H).

### Example 120

### Preparation of Compound (145)

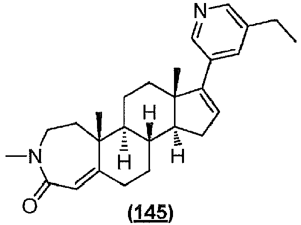

To a solution of compound (**20**) (500 mg, 1.18 mmol) in 1,4-dioxane (20 mL) was added 5-ethylpyridin-3-ylboronic acid (274 mg, 2.01 mmol), potassium carbonate (732 mg, 5.31 mmol) in water (2 mL), and Pd(dppf)Cl₂ (86 mg). The mixture was thoroughly degassed and heated under nitrogen at 80 °C for 1 hour. After being filtered through a pad of Celite, the crude product was purified by silica gel chromatography (ethyl acetate in petroleum ether, 50% v/v) to give compound (**145**) (200 mg, 43%) as a yellow solid. LC-MS (m/z) 405 [M+H]⁺. ¹H-NMR δ (400 MHz, CHCl₃-*d*) major characteristic peaks: 1.03 (s, 3H), 1.16 (s,1H), 1.26 (t, *J*= 7.6 Hz, 3H), 2.64 (m, 2H), 3.04 (s, 3H), 5.84 (s, 1H), 5.98 (s, 1H), 7.47 (s, 1H), 8.32 (s, 1H), 8.42 (s, 1H).

### Example 121

### Preparation of Compound (146)

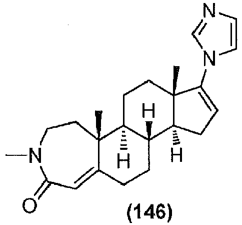

To a solution of compound (**49**) (160 mg, 0.456 mmol) in dry DMF (10 mL) was added 60 % NaH (36 mg, 0.912 mmol) and iodomethane (78 mg, 0.547 mmol) under N₂ at 0°C. Then the mixture was warmed to room temperature and stirred overnight. The mixture was quenched with water (5 mL) and extracted with ethyl acetate (50 mL * 3), washed with water, brine and dried over Na₂SO₄. After removal of solvent, the residue was purified with pre-TLC (methanol in dichloromethane, 4% v/v) to give compound (**146**) (30 mg, 20%) as a yellow solid.LCMS: 366 (M +H)⁺. ¹H NMR (CDCl₃, 400 MHz) major characteristic peaks: δ 1.00 (s, 3H), 1.15 (s, 3H), 3.05 (s, 3H), 3.18-3.24 (m, 1H), 3.40-3.45 (m, 1H), 5.69 (s, 1H), 5.85 (s, 1H), 7.04 (s, 1H), 7.09 (s, 1H), 7.63 (s, 1H).

### Example 122

### Preparation of Compound (147)

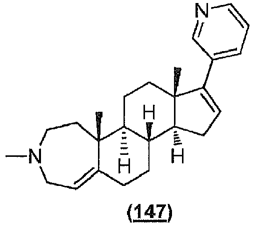

To a mixture of compound (**52**) (280 mg, 0.74 mmol) in THF (6 mL) was added LiAlH₄ (73 mg, 1.92 mmol) at room temperature. The mixture was stirred at room temperature for 2 hours. The mixture was worked-up by addition of water (0.1 mL), 15% aquesous NaOH solution (0.1 mL) and water (0.3 mL) sequently. Filtered and the filtrate was diluted with DCM (30 mL), washed with brine (1 x 10 mL), dried and concentrated to give the crude product which purified by Prep-HPLC to to get target compound (**147**) (31 mg, 12%) as a white solid. LC-MS (m/z) 363 [M+H]⁺. ¹H-NMR δ (400 MHz, CDCl₃) : 1.04 (s, 3H), 1.17 (s, 3H), 2.32 (s, 3H), 2.95 (m, 2H), 3.15 (m, 1H), 5.38 (m, 1H), 5.98 (s, 1H), 7.21 (m, 1H), 7.64 (m, 1H), 8.45 (m, 1H), 8.62 (s, 1H).

### Example 123

### Preparation of Compound (148)

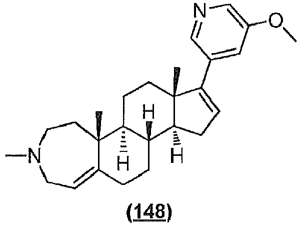

To a solution of compound (**55**) (190 mg, 0.468 mmol) in THF (5 mL) was added AlLiH₄ (36 mg, 0.936 mmol) at 28 °C, then the mixture was stirred for 1 h. After the reaction completely, the mixture was quenched with water (0.04 mL), solution of NaOH (0.04 mL, 15%) and water (0.12 mL) at ice bath, filtered and the solution was purified by Pre-HPLC to obtain compound (**148**) (100 mg, 54%). MS calculate for (C26H36N2O)⁺: 392; MS found (electrospray): 393. ¹H NMR (CDCl₃, 400 MHz) major characteristic peaks: δ 1.03 (s, 3H), 1.16 (s, 3H), 2.36 (s, 3H), 3.86 (s, 3H), 2.32 (s, 3H), 5.38 (t, *J* = 5.2 Hz, 1H), 6.00 (s, 1H), 7.15 (s, 1H), 8.17 (s, 1H), 8.24 (s, 1H).

### Example 124

### Preparation of Compound (149)

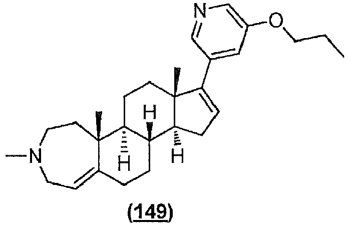

To a solution of compound (**144**) (500 mg, 1.15 mmol) in THF (10 mL) was added LiAlH₄ (87 mg, 2.3 mmol) at 28 °C, then the mixture was stirred for 1 h. After the reaction completely, the mixture was quenched with water, filtered and the solution was purified by Pre-HPLC to obtain compound (**149**) (50 mg, 10%). LC-MS (m/z) 421 [M+H]⁺. ¹H NMR (CDCl₃, 400 MHz) major characteristic peaks: δ 0.97 (m, 6H), 1.09 (s, 3H), 2.41 (s, 3H), 3.89 (m, 3H), 5.27 (t, *J* = 5.2 Hz, 1H), 5.91 (s, 1H), 7.07 (s, 1H), 8.08 (s, 1H), 8.14 (s, 1H).

### Example 125

### Preparation of Compound (150)

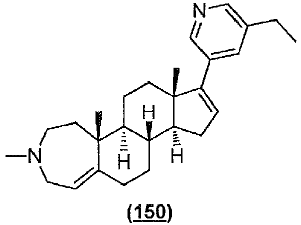

To a solution of compound (**145**) (500 mg, 1.15 mmol) in THF (10 mL) was added LiAlH₄ (87 mg, 2.3 mmol) at 28 °C, then the mixture was stirred for 1 h. After the reaction completely, the mixture was quenched with water, filtered and the solution was purified by Pre-HPLC to obtain compound (**150**) (30 mg, 10%). LC-MS (m/z) 391 [M+H]⁺. ¹H NMR (CDCl₃, 400 MHz) major characteristic peaks: δ 1.03 (s, 3H), 1.16 (s, 3H), 1.25 (m, 3H), 2.31 (s, 3H), 2.64 (m, 2H), 5.28 (m, 1H), 5.97 (s, 1H), 7.46 (s, 1H), 8.31 (s, 1H), 8.44 (s, 1H).

### Example 126

### Preparation of Compound (151)

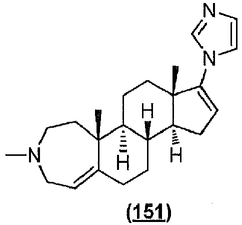

To a solution of compound (**146**) (150 mg, 0.41 mmol) in dry THF (10 mL) was added LiAlH₄ (33 mg, 0.82 mmol) under N₂ at 0°C. Then the mixture was heated to 45 °C and stirred for 2h. The mixture was quenched with water (5 mL) and extracted with DCM (100 mL x 2). The organic phase was washed with brine (50 mL x 2) and dried over Na₂SO₄. After removal of solvent, the residue was purified with pre-TLC (DCM/MeOH = 25:1, include 1% NH₃.H₂O, v/v) to give compound (**151**) (26 mg, 18%) as yellow solid. LCMS: (M+H)⁺= 352. ¹H NMR (CDCl₃, 400 MHz) major characteristic peaks: δ 0.92 (s, 3H), 1.09 (s, 3H), 2.28 (s, 3H), 5.32 (s, 1H), 5.59 (s, 1H), 6.97 (s, 1H), 7.01 (s, 1H), 7.55 (s, 1H).

### Example 127

### Preparation of Compound (152)

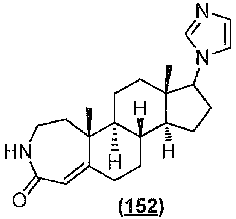

### Example 127A

### Preparation of Compound (152a)

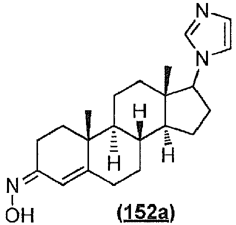

To a solution of (10*R*, 13*S*)-17-(1*H*-imidazol-1-yl)-10,13-dimethyl-1,7,8,10,11,12, 13,15,16,17-decahydro-2*H*-cyclopenta[a]phenanthren-3(6*H*,9*H*,14*H*-one (1.2 g, 3.55 mmol) in ethanol (15 mL) was added pyridine (2 mL) and hydroxylamine hydrochlororide (296 mg, 4.26 mmol). The mixture was stirred at room temperature overnight. The mixture was poured into ice-water (100 mL) and stirred 30 min. to give a white precipitate. Filtrated and dried to give the compound (**152a**) (1.0 g, 80%) as a white solid. LCMS: (M+H)⁺= 354. ¹H-NMR δ (400 MHz, CDCl₃) major characteristic peaks: δ 0.60 (s, 3H), 1.06 (s, 3H), 3.04-3.09 (m, 1H), 3.97 (t, *J* = 9.6 Hz, 1H), 5.79 (s, 1H), 6.97 (s, 1H), 7.09 (s, 1H), 7.64 (s, 1H).

### Example 127B

### Preparation of Compound (152)

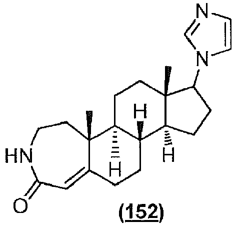

To a solution of (9a*R*, 11a*S*, *E*)-17-(1*H*-imidazol-1-yl)-10,13-dimethyl-1,7,8,10,11,12, 13,15,16,17-decahydro-2*H*-cyclopenta[a]phenanthren-3(6*H*,9*H*,14*H*)-one oxime, compound (**152a**), (300 mg, 0.85 mmol) in dry THF (20 mL) was added dropwise SOCl₂ (4 mL) at 0 °C. The mixture was stirred at room temperature overnight. The mixture was poured into ice-water (100 mL), adjusted pH to 7 with saturate NaHCO₃ solution and extracted with ethyl acetate (100 mL x 2), washed with brine (50 mL x2), and dried over Na₂SO₄ and concentrated. The residue was purified by pre-TLC (DCM/Methanol = 20:1) to give the product compound (**152**), (80 mg, 26%) as a brown solid. LCMS: (M+H)⁺ = 354. ¹H-NMR δ (400 MHz, CDCl₃) major characteristic peaks: δ 0.59 (s, 3H), 1.15 (s, 3H), 3.11-3.25 (m, 2H), 3.96 (t, *J* = 9.6 Hz, 1H), 5.76 (s, 1H), 6.32 (s, 1H), 6.95 (s, 1H), 7.06 (s, 1H), 7.57 (s, 1H).

### Example 128

### Preparation of Compound (153)

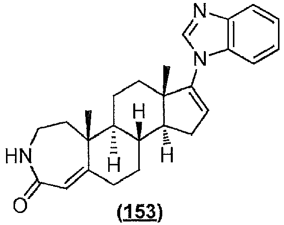

### Example 128A

### Preparation of Compound (153a)

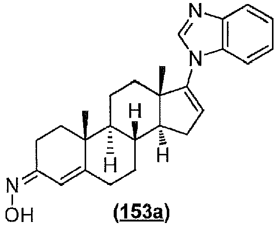

To a solution of (10R,13S)-17-(1H-benzo[d]imidazol-1-yl)-10,13-dimethyl-6,7,8,9,10,11,12,13,14,15-decahydro-1H-cyclopenta[a]phenanthren-3(2H)-one (770 mg, 1.99 mmol) in EtOH (10 mL) was added NH₂OH.HCl (244 mg, 3.07 mmol) and pyridine (2 mL), The mixture was stirred at room temperature for 2 hours. Then it was poured into ice-water, filtered and the solid was dried to give the crude product (9aR,11aS,E)-17-(1H-benzo[d]imidazol-1-yl)-10,13-dimethyl-1,7,8,9,10,11,12,13,14,15-decahydro-2H-cyclopenta[a]phenanthren-3(6H)-one oxime, compound (**153a**) (802 mg). LC-MS (m/z) 459 [M+H]⁺.

### Example 128B

### Preparation of Compound (153)

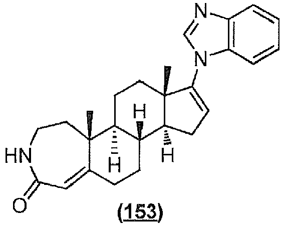

The solution of compound (**153a**) (802 mg, Crude) in dried THF (10 mL) was added SOCl₂ (4 mL), the mixture was stirred at room temperature overnight. Then it was poured into ice-water, adjusted to pH7 with NH₄OH, extracted with DCM (3 x 30 mL). The organic layer was washed with brine (1 x 30 mL), dried and concentrated to give the crude product which purified by column chromatography (ethyl acetate) to get compound (**153**) (349 mg, 49%, two steps) as a brown solid. LC-MS (m/z) 402 [M+H]⁺. ¹H NMR (CDCl₃, 400 MHz) major characteristic peaks: δ 0.94 (s, 3H), 1.12 (s, 3H), 5.71 (m, 1H), 6.10 (m, 1H), 6.61 (s, 1H), 7.23 (m, 2H), 7.41 (m, 1H), 7.74 (m, 1H), 7.87 (s, 1H).

### Example 129

### Preparation of Compound (154)

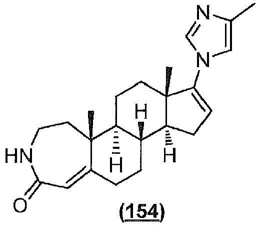

### Example 129A

### Preparation of Compound (154a)

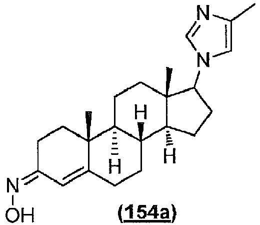

A solution of (10R, 13S)-10,13-dimethyl-17-(4-methyl-1H-imidazol-1-yl)-6,7,8,9,10, 11,12,13,14,15-decahydro-1H-cyclopenta[a}phenanthren-3(2H)-one (0.27 g, 0.77 mmol), hydroxylamine hydrochloride (0.064 g, 0.92 mmol) and pyridine (0.091 g, 1.15 mmol) in EtOH (20 mL) was stirred at room temperature for overnight. After the reaction, the mixture was poured into ice-water and stirred for 0.5 h. Then filtered and filter cake was washed with H2O. Dried under reduce pressure to obtain the compound (**154a**) (170 mg, 60.7%) as a white solid. MS calcd for (C23H32N20)⁺: 365.25; MS found (electrospray): 366.1.

### Example 129B

### Preparation of Compound (154)

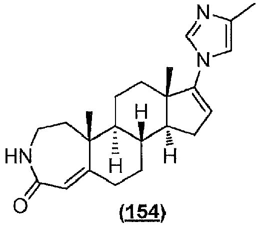

A solution of compound (**154a**) (0.17 g, 0.465 mmol), SOC12 (1 mL) and THF (20 mL) was stirred at room temperature for overnight. After the reaction, the mixture was poured into ice-water and extracted with ethyl acetate (20mL * 3), washed with brine, dried over Na₂SO₄. Purification by pre-TLC (dichloromethane/MeOH = 20/1, v/v) gave the product compound (**154**) (90 mg, 52.9%). MS calcd for (C23H32N2O)⁺: 365.25; MS found (electrospray): 366.3. ¹H NMR (CDCl₃, 400 MHz) major characteristic peaks: δ 0.99 (s, 3H), 1.19 (s, 3H), 2.23 (s, 3H), 2.54 (m, 1H), 3.16 (m, 1H), 3.25(m, 1H), 5.61 (s, 1H), 5.77 (s, 1H), 6.20(s, 1H), 6.78 (s, 1H),7.52(s,1H).

### Example 130

### Preparation of Compound (155)

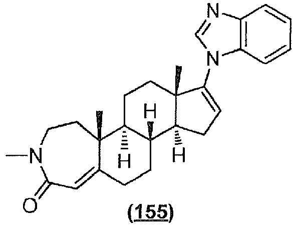

To a solution of compound (**153**) (401 mg, 1 mmol) in DMF (6 mL) was added NaH (80 mg, 2 mmol).When it was stirred and added CH₃I (215 mg, 1.5 mmol). After that, the mixture was stirred at room temperature for 18 hours. 10 mL of water was added and extracted with CH₂Cl₂ (3 x 30 mL), the organic layer was combined and washed with water (4 x 30 mL) and brine (1 x 30 mL), dried and concentrated to give the crude product which purified by Prep-TLC (ethyl acetate) to get final compound compound (**155**) (94 mg, 23%) as a light yellow solid. LC-MS (m/z) 416 [M+H]⁺. ¹H NMR (CDCl₃, 400 MHz) major characteristic peaks: δ 0.94 (s, 3H), 1.08 (s, 3H), 2.98 (s, 3H), 5.79 (m, 1H), 5.91 (m, 1H), 7.24 (m, 2H), 7.41 (m, 1H), 7.75 (m, 1H), 7.88 (s, 1H).

### Example 131

### Preparation of Compound (156)

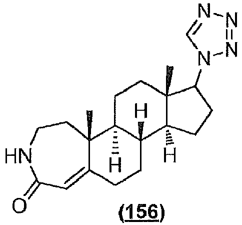

### Example 131A

### Preparation of Compound (156a)

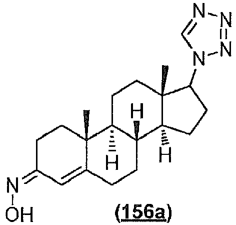

To a solution of (10*R*, 13*S*)-10,13-dimethyl-17-(1*H*-tetrazol-1-yl)-1,7,8,9,10,11,12,13, 14,15-decahydro-2*H*-cyclopenta[a]phenanthren-3(6*H*)-one (100 mg, 0.29 mmol) in ethanol (5 mL) was added pyridine (0.5 mL) and hydroxylamine hydrochlororide (25 mg, 0.348 mmol). The mixture was stirred at room temperature overnight. The mixture was poured into ice-water (100 mL) and stirred 30 min. to give a white precipitate. Filtrated and dried to give compound (156a) (100 mg, ∼ 100%) as a white solid. LCMS: (M+H)⁺ = 354.

### Example 131B

### Preparation of Compound (156)

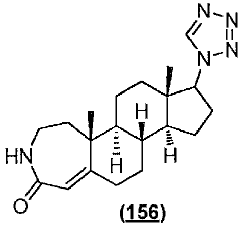

To a solution of (9a*R*,11a*S,E*)-10,13-dimethyl-17-(1*H*-tetrazol-1-yl)-1,7,8,9,10,11,12, 13,14,15-decahydro-2*H*-cyclopenta[a]phenanthren-3(6*H*)-one oxime, compound (**156a**), (100 mg, 0.283 mmol) in dry THF (8 mL) was added dropwise SOCl₂ (0.15 mL) at 0°C. The mixture was stirred at room temperature overnight. The mixture was poured into ice-water (100 mL), adjusted pH to 7 with saturate NaHCO₃ solution and extracted with ethyl acetate (100 mL x 2), washed with brine (50 mL x2), and dried over Na₂SO₄ and concentrated. The residue was purified by pre-TLC (ethyl acetate/ petroleum ether = 2:1) to give the product compound (**156**) (10 mg, 10%) as a brown solid. LCMS: (M +H)⁺= 354. ¹H-NMR δ (400 MHz, CDCl₃) major characteristic peaks: δ 1.13 (s, 3H), 1.21 (s, 3H), 5.79 (s, 1H), 6.10 (s, 1H), 6.24 (s, 1H), 8.72 (s, 1H).

### Example 132

### Preparation of Compound (157)

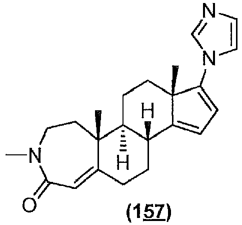

To a solution of compound (**76**) (349 mg, 1 mmol) in DMF (10 mL) was added NaH (60 mg, 1.5 mmol) at 28 °C, then the mixture was stirred overnight. After the reaction completely, the mixture was quenched with water, evaporated the solution and the residue was purified by Pre-HPLC to obtain compound (**157**) (7 mg, 2%) as a yellow solid. LC-MS (m/z) 364 [M+H]⁺. ¹H NMR (CDCl₃, 400 MHz) major characteristic peaks: δ 1.12 (s, 3H), 1.22 (s, 3H), 3.04 (s, 3H), 5.88 (m, 2H), 6.25 (s, 1H), 7.08 (s, 1H), 7.13 (s, 1H), 7.64 (s, 1H).

### Example 133

### Prepared of Compound (158)

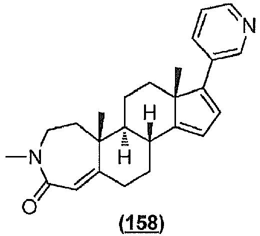

### Example 133A

### Preparation of Compound (158a)

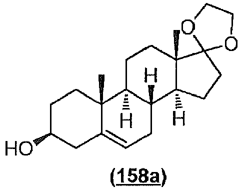

A mixture of dehydro epiandrosterone (7.0 g, 24.3 mmol), TsOH (462 mg, 2.43 mmol), glycol (4.1 mL, 72.9 mmol) in triethyl orthoformate (30 mL) was stirred for 3 h at 80-90 °C. Then most of solvents was removed. It was diluted with water (50 mL), extracted with dichloromethane (50 mL * 3). The combined organic layers were washed with saturated aqueous NaHCO₃ solution (50 mL), brine (50 mL), dried, concentrated to get compound (**158a**) (7.27 g, 90%) as a yellow solid. ¹H-NMR δ (400 MHz, CHCl₃-*d*) major characteristic peaks: δ 0.86 (s, 3H), 1.02 (s, 3H), 3.87 (m, 1H), 3.92 (m, 4H), 5.34 (m, 1H).

### Example 133B

### Preparation of Compound (158b)

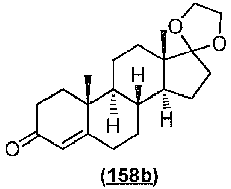

A mixture of compound (**158a**) (7.27 g, 21.8 mmol), Al(Oi-Pr)₃ (24.7%, 28.9 g, 34.98 mmol) in butanone (60 mL) and toluene (30 mL) was refluxed overnight. Cooled to room temperature, diluted with water (100 mL), filtered. The cake was washed with ethyl acetate (200 mL * 3) repeatedly. The combined filtrate was washed with brine (200 mL), dried, concentrated, purified by column chromatography (petroleum ether/ethyl acetate = 30/1-20/1, v/v) to get compound (**158b**) (3.6 g, 50%) as a white solid. ¹H-NMR δ (400 MHz, CHCl₃-*d*) major characteristic peaks: δ 0.89 (s, 3H), 1.19 (s, 3H), 3.87 (m, 4H), 5.73 (s, 1H).

### Example 133C

### Preparation of Compound (158c)

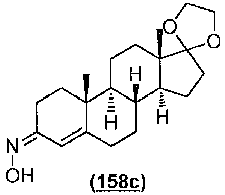

To a solution of compound (**158b**) (7.0 g, 21.2 mmol) in ethanol (150 mL) was added pyridine (25 mL) and hydroxylamine hydrochlororide (2.2 g, 31.8 mmol) under N₂. The mixture was stirred at room temperature overnight. The mixture was poured into ice-water (300 mL) and stirred 30 min. to give a white precipitate. Filtrated and dried to get the compound **(158c)**(6.0 g, 85%) as a white solid. LC-MS (m/z) 346 [M+H]⁺.

### Example 133D

### Preparation of Compound (158d)

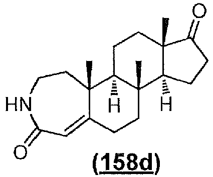

To a solution of compound **(158c)** (6.0 g, 17.4 mmol) in dry THF (50 mL) was added dropwise a solution of SOCl₂ (5 mL) in THF (10 mL) at 0 °C. The mixture was stirred at room temperature overnight. The mixture was poured into ice-water (300 mL) and extracted with DCM (100 mL* 3), washed with water, brine, and dried over Na₂SO₄. Filtered, concentrated to give a brown solid. The solid was dissolved in 300 mL of THF, and aq. HCl (20 mL, 1.0M) was added .The mixture was stirred for 1 h at 25 °C. After removal of solvent, the residue was purified with silica gel chromatography (methanol in dichloromethane, 2% v/v) to give compound (**158d**) (2.72 g, 52%) as a brown solid. LC-MS (m/z) 302 [M+H]⁺. ¹H-NMR δ (400 MHz, CHCl₃-*d*) major characteristic peaks: δ 0.89 (s, 3H), 1.17 (s, 3H), 2.50 (m, 2H), 3.15 (m, 2H), 5.75 (s, 1H), 6.64 (brs, 1H).

### Example 133E

### Preparation of Compound (158e)

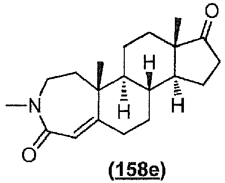

To a solution of compound **(158d)** (2.2 g, 7.31 mmol) in dry DMF (20 mL) was added 60 % NaH (439 mg, 11 mmol) and iodomethane (1.25 g, 8.8 mmol) under N₂ at -30 °C. Then the mixture was warmed to room temperature and stirred overnight. The mixture was quenched with water (50 mL) and extracted with ethyl acetate (100 mL * 3), washed with water, brine and dried over Na₂SO₄. After removal of solvent, the residue was purified with silica gel chromatography (ethyl acetate in petroleum, 50% v/v) to give compound **(158e)** (1.487 g, 52%) as a yellow solid. LC-MS (m/z) 316 [M+H]⁺. ¹H-NMR δ (400 MHz, CHCl₃-*d*) major characteristic peaks: δ 1.06 (s, 3H), 1.36 (s, 3H), 2.97 (s, 3H), 3.14 (m, 1H), 3.35 (m, 1H), 5.77 (s, 1H).

### Example 133F

### Preparation of Compound (158f)

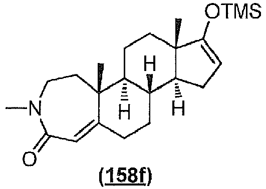

n-BuLi (2.5 M, 3.4 mL, 8.5 mmol) was added dropwise to a solution of diisopropylamine (1.27 mL, 9.44 mmol) in dried THF (10 mL) at -78 °C, and stirred for 15 min. The solution of compound **(158e)** (1.49 g, 4.72 mmol) in THF (20 mL) was added slowly. Stirred for another 15 min, triethylamine (1.4 mL) and TMSCl (0.97 mL, 7.08 mmol) was added. The mixture was warmed to room temperature and stirred for 1 hour. The mixture was quenched by sat.NaHCO₃, extracted by ethyl acetate (100 mL*2). The organic layer was washed with water, brine, dried and concentrated to give the crde product compound **(158f)** (1.82 g,). LC-MS (m/z) 388 [M+H]⁺.

### Example 133G

### Preparation of Compound (158g)

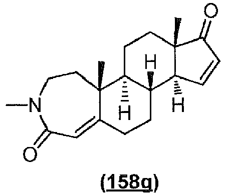

A mixture of compound **(158f)** (1.82 g) and Pd(OAc)₂ (1.17 g, 5.0 mmol) in DCM (30 mL) and MeCN (10 mL) was stirred at 35 °C for 2 hours. Filtered and the filtrate was concentrated, purified by column chromatography (methanol in dichloromethane, 2% v/v) to get compound **(158g)** (617 mg, 42%, two steps) as a brown solid. LC-MS (m/z) 314 [M+H]⁺. ¹H NMR (CDCl₃, 400 MHz) major characteristic peaks: δ 0.90 (s, 3H), 0.98 (s, 3H), 2.85 (s, 3H), 3.02 (m, 1H), 3.23 (m, 1H), 5.67 (s, 1H), 6.05 (m, 1H), 7.51 (m, 1H).

### Example 133H

### Preparation of Compound (158h)

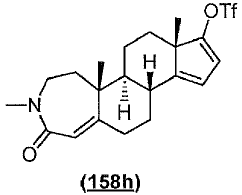

A mixture of compound **(158g)** (617 mg, 1.97 mmol) and triethylamine (0.35 mL, 2.52 mmol) in DCM (20 mL) was cooled to -60 °C. Tf₂O (0.49.mL, 2.96 mmol) was added into the mixture slowly. Stirred at the temperature for 30 min, diluted by DCM (dichloromethane) (20 mL) and quenched by water (10 mL), extracted by DCM (50 mL * 3). The organic layer was washed with saturated NaHCO₃,water and brine, dried and concentrated, purified by column chromatography (methanol in dichloromethane, 1% v/v) to get compound (**158h**) (436 mg, 51%) as a yellow solid. LC-MS (m/z) 446 [M+H]⁺. ¹H NMR (CDCl₃, 400 MHz) major characteristic peaks: δ 0.97 (s, 3H), 1.02 (s, 3H), 2.85 (s, 3H), 3.04 (m, 1H), 3.21 (m, 1H), 5.58 (m, 1H), 5.68 (m, 1H), 5.90 (m, 1H).

### Example 133I

### Preparation of Compound (158)

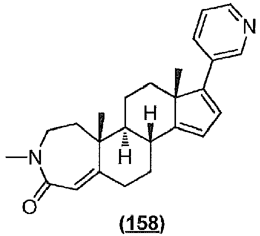

A mixture of compound **(158h)** (200 mg, 0.45 mmol), pyridin-3-ylboronic acid (83 mg, 0.68 mmol), Pd(dppf)₂Cl₂ (37 mg, 0.05 mmol) and K₂CO₃ (279 mg, 2.03 mmol) in 1,4-dioxane (10 mL) and water (1 mL) was stirred at 80 °C for 2 hours. Filtered and the filtrate was diluted with ethyl acetate (60 mL), washed with water, brine, dried and concentrated to give the crude product. The crude product was purified by prepatory TLC (methanol in dichloromethane, 4% v/v) to get compound **(158)** (36 mg, 21%) as a white solid. LC-MS (m/z): 375 [M+H]⁺. ¹H NMR (CDCl₃, 400 MHz) major characteristic peaks: δ 1.13 (s, 3H), 1.17 (s, 3H), 2.97 (s, 3H), 3.16 (m, 1H), 3.33 (m, 1H), 5.81 (s, 1H), 5.92 (m, 1H), 6.72 (m, 1H), 7.18 (m, 1H), 7.69 (d, *J* = 8.0 Hz, 1H), 8.36 (s, 1H), 8.67 (s, 1H).

### Example 134

### Preparation of Compound (159)

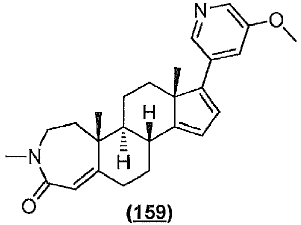

A mixture of compound (**158h**) (200 mg, 0.45 mmol), 5-methoxypyridin-3-ylboronic acid (83 mg, 0.68 mmol), Pd(dppf)₂Cl₂ (37 mg, 0.05 mmol) and K₂CO₃ (279 mg, 2.03 mmol) in 1,4-dioxane (10 mL) and water (1 mL) was stirred at 80 °C for 2 hours. Filtered and the filtrate was diluted with ethyl acetate (60 mL), washed with water, brine, dried and concentrated the solvent and the crude product was purified by column chormatography (4:1 petroleum ether/ethyl acetate, v/v) to afford compound **(159)** (30 mg, 18%) as a yellow solid. LC-MS (m/z) 405 [M+H]⁺. ¹H NMR (CDCl₃, 400 MHz) major characteristic peaks: δ 1.19 (s, 3H), 1.23 (s, 3H), 3.04 (s, 3H), 3.22 (m, 1H), 3.43 (m, 1H), 3.88 (s, 3H), 5.81 (s, 1H), 5.92 (m, 1H), 6.72 (m, 1H), 7.26 (m, 1H), 8.13 (s, 1H), 8.37 (s, 1H).

### Example 135

### Preparation of Compound (160)

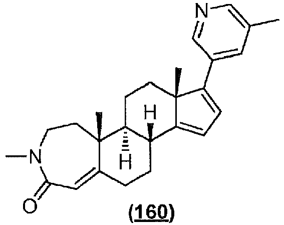

A mixture of compound (**158h**) (190 mg, 0.43 mmol), 5-methylpyridin-3-ylboronic acid (89 mg, 0.64 mmol), Pd(dppf)₂Cl₂ (31 mg, 0.04 mmol) and K₂CO₃ (267 mg, 1.94 mmol) in 1,4-dioxane (10 mL) and water (1 mL) was stirred at 80 °C for 2 hours. Filtered and the filtrate was diluted with ethyl acetate (60 mL), washed with water, brine, dried and concentrated, purified by Prep-TLC (methanol in dichloromethane, 4% v/v) to get final Compound compound **(160)** (43 mg, 26%) as a yellow solid. LC-MS (m/z): 389 [M+H]⁺. ¹H NMR (CDCl₃, 400 MHz) major characteristic peaks: δ 1.19 (s, 3H), 1.24 (s, 3H), 2.34 (s, 3H), 3.04 (s, 3H), 3.23 (m, 1H), 3.40 (m, 1H), 5.88 (s, 1H), 5.98 (m, 1H), 6.76 (m, 1H), 7.56 (s, 1H), 8.26 (s, 1H), 8.54 (s, 1H).

### Example 136

### Prepared of Synthetic Intermediate Compound (161)

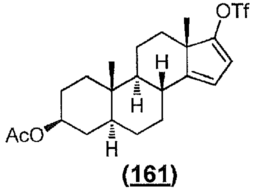

### Example 136A

### Preparation of Compound (161a)

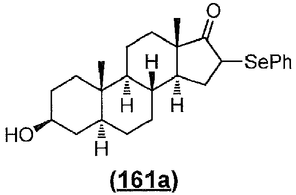

To a solution of epiandrosterone (2.60 g, 8.95 mmol) in DMF (50 mL) was added 60% of sodium hydride (2.00 g, 50 mmol) in five portions at room temperature. The mixture was stirred at 60 °C for 1 hr. After being cooled to 0 °C, PhSeBr (4.65 g, 19.7 mmol) was added with stirring. The mixture was warmed to room temperature and stirred for 30 min. 30 mL of ice-water was added. The mixture was extracted with EtOAc (2x30 mL). The combined organic layers was washed with 5% citric acid (2x30 mL) and brine (50 mL), dried over NaSO₄ and purified by FCC on silica get to give 2.60 g of compound **(161a).** ESI-MS (m/z): 445.5[M+H]⁺; ¹H NMR δ (400 MHz, CHCl₃-*d*) major characteristic peaks: 0.83 (s, 3H), 0.98 (s, 3H), 2.01 (m, 1H), 3.58 (m, 3H), 7.35 (m, 3H), 7.67 (m, 2H).

### Example 136B

### Preparation of Compound (161b)

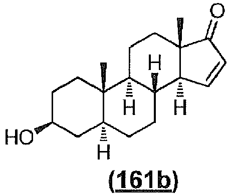

6.06 g of *m*CPBA (25.3 mmol) was added in three portions to a solution of the above compound **(161a)** (10.1 g, 22.6 mmol) in 200 mL of CH₂Cl₂ at -40 °C with stirring. The mixture was stirred at low temperature for 1.5 hr. 12 mL of Et₂NH was added. The formed mixture was added to a refluxed solution of 55 mL of Et₂NH in 315 mL of CCl₄, and kept at reflux for 30 min. After being cooled to room temperature the solution was washed with 5% Na₂SO₃ (3x100 mL) and brine (100 mL), dried over MgSO₄, and condensed under vacuum to get the crude compound (**161b**). ¹H NMR δ (400 MHz, CHCl₃-*d*) major characteristic peaks: 0.97 (s, 3H), 1.12 (s, 3H), 3.61 (m, 3H), 6.02 (m, 1H), 7.53 (m, 1H).

### Example 136C

### Preparation of Compound (161c)

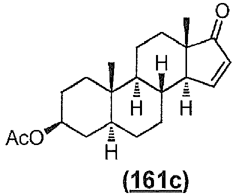

To a solution of above compound **(161b)** in 50 mL of CH₂Cl₂ was added 10 mL of pyridine (135 mmol), 6.00 g of acetic anhydride (45.2 mmol) and 75 mg of DMAP (0.450 mmol) in turn. The mixture was refluxed for 45 min. The heating was stopped. The mixture was washed with 10% citric acid (4x40 mL) and brine (40 mL), dried over MgSO₄,and purified through FCC on silica gel (eluting with EtOAc/Hexanes=1/6) to get 4.20 g of compound **(161c).** ¹H NMR δ (400 MHz, CHCl₃₋*d*): major characteristic peaks: δ 0.97 (s, 3H), 1.12 (s, 3H), 2.06 (s,3H),4.71 (m, 1H), 6.02 (m, 1H), 7.51 (m, 1H).

### Example 136D

### Preparation of Compound (161)

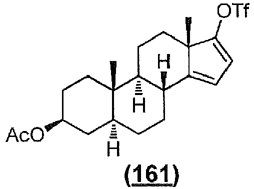

To a mixture of compound **(161c)** (1.50 g, 4.50 mmol) and triethylamine (460 mg, 4.50 mmol) in 100 mL of DCM was added a solution of Tf₂O (3.00 g, 9.90 mmol) in 10 mL of DCM at -60 °C. The mixture was stirred at room temperature for 2 hr. 30 mL of ice-water was added. The organic phase was washed with brine (80 mL), dried over Na₂SO₄ and purified by FCC to give 1.47 g of compound **(161)** in 70% yield. ¹H NMR δ (400 MHz, CHCl₃-*d*): major characteristic peaks: δ 0.97 (s, 3H), 1.12 (s, 3H), 2.06 (s,3H), 4.68 (m, 1H), 5.67 (m, 1H), 6.02 (m, 1H).

### Example 137

### Preparation of Compound (162)

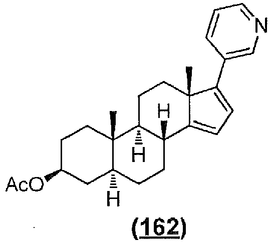

Under argon protection, a mixture of compound **(161)** (1.47 g, 3.10 mmol, K₂CO₃ (2.2 g, 15.5 mmol) , 3-(diethyboryl)pyridine (700.0 mg, 4.65 mmol) , Pd(PPh₃)₂Cl₂(220 mg, 0.310 mmol) in 30 mL of dioxane and 10 mL of water was degassed within an ultrasonic cleaner for 30 min. The mixture was stirred at 80 °C overnight. 50 mL of water and 50 mL of DCM was added. The layers were partitioned. The aqueous phase was extracted with DCM (50 mL). The combined DCM layers were washed with brine (100 mL), dried over Na₂SO₄ and purified through FCC on silica gel ro get 1.00 g of compound **(162)** in 98.5 % yield. LC-MS (m/z) 391.5 [M+H]⁺; ¹H NMR δ (400 MHz, CHCl₃-*d*): 0.97 (s, 3H), 1.12 (s, 3H), 2.05(s, 3H), 4.70 (s, 1H), 5.98 (m, 1H),6.78 (m,1H),7.20 (m,1H),7.74(m,1H),8.40 (m,1H), 8.75 (s,1H). ¹³C NMR δ (400 MHz, CHCl₃-*d*):170.65, 164.52, 151.36, 147.20, 146.99, 132..55, 131.95, 127.71, 123.24, 118.46, 73.47, 53.69, 44.39, 37.07, 36.90, 36.04, 35.78, 33.87, 29.53, 28.19, 27.38, 21.44, 21.26, 19.58, 12.29.

### Example 138

### Preparation of Compound (163)

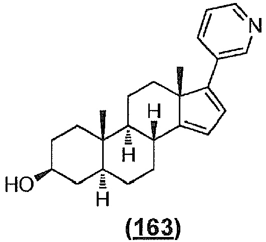

A mixture of 1.00 g of compound **(162)** and NaOH (275 mg, 5.00 mmol) in 30 mL of MeOH and 30 mL of THF and 10 mL of water was heated at 50 °C for 1 hr. The solvents were evaporated. 30 mL of water and 50 mL of DCM were added. The organic phase was partitioned, washed with brine, and condensed under vacuum to get 750 mg of compound **(163).** LC-MS (m/z) 349.9[M+H]⁺. ¹H NMR δ (400 MHz, CHCl₃-*d*) major characteristic peaks: δ 0.97 (s, 3H), 1.18 (s, 3H), 3.61 (s, 1H), 5.98 (m, 1H), 6.78 (m,1H), 7.20(m,1H), 7.74(m,1H), 8.40(m,1H), 8.75(s,1H). ¹³C NMR δ (400 MHz, CHCl₃-*d*) : 164.83, 151.34, 147.18, 146.94, 132.58, 131.99, 127.72, 123.27, 118.35, 71.12, 57.46, 53.74, 44.61, 38.04, 37.36, 37.00, 36.08, 35.85, 31.42, 29.64, 28.33, 21.32, 19.60, 12.41.

### Example 139

### Preparation of Compound (164)

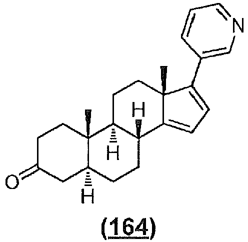

A mixture of compound **(163)** (509 mg, 1.46 mmol) and Dess-Martin periodinane (4.00 g, 8.74 mmol) in 80 mL of DCM was heated at reflux for 2 hr. 30 mL of water was added, followed by 2.00 g of Na₂SO₃. The solid was removed by filtration. The filtrate was partitioned by separatory funnel. The water layer was extracted with DCM (50 mL). The combined DCM layers were washed with brine, dried over Na₂SO₄, and condensed under vacuum to give 300 mg of compound **(164)** in 58.9% yield. ¹H-NMR δ (400 MHz, CHCl₃-*d*) major characteristic peaks: δ 1.12 (s, 3H), 1.23 (s, 3H), 5.98 (m, 1H), 6.78(m,1H), 7.20(m, 1H), 7.74(m, 1H), 8.40(m, 1H), 8.75(s, 1H).

### Example 140

### Preparation of Compound (165) and Compound (166)

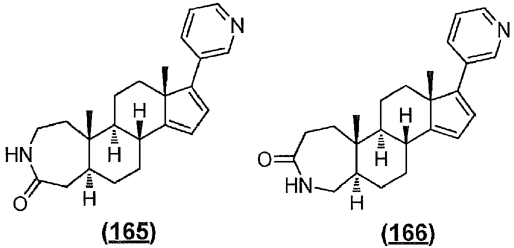

A mixture of compound **(164)** (300 mg, 0.863 mmol), NH₂OH·HCl (180 mg) and pyridine (220 mg) in 20 mL of EtOH was stirred at 40 °C for 1.5 hr. EtOH was evaporated under reduced pressure. 30 mL of DCM and 15 mL of water was added. The layers were partitioned with separatory funnel. The water phase was extracted with 30 mL of DCM. The combined DCM layers were washed with brine, dried over MgSO₄ and condensed under vacuum to give 300 mg of the oxime. The oxime was dissolved in 20 mL of DCM and cooled to 0 °C. 2.30 g of SOCl₂ was added dropwise within 30 min. The mixture was stirred at room temperature for 5 hr. The mixture was poured to 30 mL of ice-water. The layers were partitioned. The organic phase was washed with brine, dried on MgSO₄ and purified by prep HPLC to give compound **(165)** and compound **(166). Compound (165):** LC-MS (m/z) 363.3 [M+H]⁺. ¹H-NMR δ (400 MHz, CHCl₃-*d*) major characteristic peaks: δ 1.13(s, 3H), 1.25(s, 3H), 2.80(m, 1H), 3.08(m, 1H), 3.49(m, 1H), 6.02 (m, 1H), 6.11(s, 1H), 6.78(m, 1H), 7.20(m, 1H), 7.74(m, 1H), 8.40(m, 1H), 8.75(s, 1H). ¹³C NMR δ (400 MHz, CHCl₃-*d*) : 178.22, 163.96, 151.40, 147.12, 147.07, 132.61, 131.86, 127.77, 123.29, 118.93, 57.10, 53.41, 43.04, 39.34, 37.79, 36.76, 34.97, 31.92, 30.65, 29.69, 29.36, 21.47, 19.43, 12.10. **Compound (166):** LC-MS (m/z) 363.1[M+H]⁺. ¹H-NMR δ (400 MHz, CHCl₃-*d*) major characteristic peaks: δ 1.13 (s, 3H), 1.25 (s, 3H), 2.68(m, 2H), 3.42(m, 1H), 5.98 (m, 1H), 6.37 (s, 1H), 6.78 (m, 1H), 7.20 (m, 1H), 7.74 (m, 1H), 8.40 (m, 1H), 8.75 (s, 1H). ¹³C-NMR δ (400 MHz, CHCl₃-*d*) : 178.47, 163.96, 151.51, 147.21, 147.11, 132.59, 131.85, 127.67, 123.28, 118.77, 56.38, 53.48, 49.41, 39.18, 35.60, 35.35, 31.29, 29.70, 29.28, 27.43, 21.13, 19.49, 12.17.

### Example 141

### Preparation of Synthetic Intermediate Compound (167)

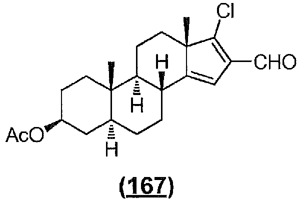

35 mL of POCl₃ was pre-mixed with 35 mL of DMF and 35 mL of CHCl₃ at 0 °C. The resulting mixture was added dropwise to a solution of 5.20 g of compound **(161c)** (15.8 mmol) in 50 mL of CHCl₃ at 0 °C with stirring. The mixture was stirred at reflux for 1 hr. The mixture was cooled to room temperature and poured into 200 mL of ice-water. 100 mL of DCM was added. The layers were partitioned. The water phase was extracted with DCM (2x80 mL). The combined organic layers were washed with brine, dried over MgSO₄ and purified by FCC to give 4.1 g of compound **(167)** in 70% yield. ¹H-NMR δ (400 MHz, CHCl₃-*d*) : major characteristic peaks: 1.02 (s, 3H), 1.23 (s, 3H), 2.09(s, 3H), 4.68 (m, 1H), 6.18(s, 1H), 10.02(s, 1H).

### Example 142

### Preparation of Compound (168)

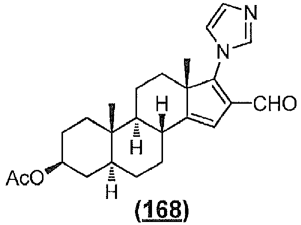

A mixture of 1.40 g of compound **(167)** (3.71 mmol), 1.03 g of K₂CO₃ (7.42 mmol) and 266 mg of imidazole (3.90 mmol) in 50 of DMF was stirred at 80 °C for 3 hr. After being cooled to room temperature the mixture was poured to ice-water, and extracted with DCM. The combined DCM phase was washed with brine, dried on MgSO₄ and purified by FCC to give 820 mg of compound **(168)** in 55% yield. ¹H-NMR δ (400 MHz, CHCl₃-*d*) : major characteristic peaks: δ 1.02(s, 3H), 1.23(s, 3H), 2.05(s, 3H), 4.68(m, 1H), 6.28(s, 1H), 7.15(m, 1H), 7.23(m, 1H), 9.75(s, 1H). LC-MS (m/z) 380.6[M+H]⁺. ¹H-NMR δ (400 MHz, CHCl₃-*d*) major characteristic peaks: 0.95 (s, 3H), 1.07 (s, 3H), 2.05(s, 3H), 4.68 (m, 1H), 5.83(m, 1H), 6.28(m, 1H), 7.08(m, 1H), 7.13(m, 1H), 7.65(s, 1H). ¹³C-NMR δ (400 MHz, CHCl₃-*d*) : 170.63, 158.55, 149.46, 136.01, 129.38, 118.83, 118.36, 116.61, 73.36, 56.76, 52.10, 44.36, 37.02., 35.95, 35.60, 33.82, 31.91, 29.69, 29.35, 28.04, 27.33, 21.41, 18.81, 12.24

### Example 143

### Preparation of Compound (169)

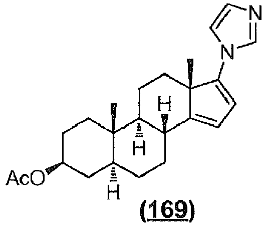

To a solution of compound **(168)** (820 mg, 2.01 mmol) in PhCN (20 ml) was added 1.60 g of dry Pd/C (10%). The mixture was refluxed for 3 hr under N₂ protection. PhCN was evaporated. The residue was purified through FCC eluenting with EtOAc/Hexanes/Et3N=1/3/0.05 to get 360 mg of compound **(169)** in 48% yield. LC-MS (m/z) 380.6[M+H]⁺. ¹H-NMR δ (400 MHz, CHCl₃-*d*) major characteristic peaks: 0.95 (s, 3H), 1.07 (s, 3H), 2.05(s, 3H), 4.68 (m, 1H), 5.83(m, 1H), 6.28(m, 1H), 7.08(m, 1H), 7.13(m, 1H), 7.65(s, 1H). ¹³C-NMR δ (400 MHz, CHCl₃-*d*) : 170.63, 158.55, 149.46, 136.01, 129.38, 118.83, 118.36, 116.61, 73.36, 56.76, 52.10, 44.36, 37.02., 35.95, 35.60, 33.82, 31.91, 29.69, 29.35, 28.04, 27.33, 21.41, 18.81, 12.24.

### Example 144

### Preparation of Compound (170)

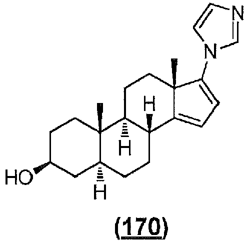

A mixture of compound **(169)** (360 mg, 0.95 mmol) and NaOH (80.0 mg) in MeOH (40 ml) and water (50 mL) was refluxed for 2 hr. After being cooled to room temperature the mixture was extracted with DCM (2x40 mL). The combined DCM phase was washed with brine (80 mL), dried over MgSO₄ and condensed under vacuum to get 330 mg of compound **(170).** LC-MS (m/z) 339.3[M+H]⁺. ¹H-NMR δ (400 MHz, CHCl₃-*d*) major characteristic peaks: δ 0.95 (s, 3H), 1.07 (s, 3H), 3.58 (m, 1H), 5.83(m, 1H), 6.18(m, 1H), 7.05(m, 1H), 7.08(m, 1H), 7.61(s, 1H). ¹³C-NMR δ (400 MHz, CHCl₃-*d*) : 158.85, 149.40, 135.98, 129.26, 118.88, 118.40, 116.47, 70.98, 56.93, 52.13, 44.57, 37.96, 37.30, 35.98, 35.70, 35.66, 31.35, 29.28, 28.18, 21.05, 18.82, 12.36.

### Example 145

### Preparation of Compound (171)

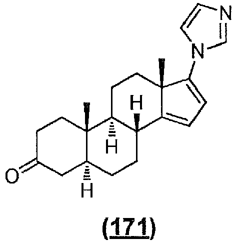

Dess-Martin periodinane (2.48 g, 5.60 mmol) was added to a solution of compound **(170)** (330 mg, 0.980 mmol) in CH₂Cl₂ (25 ml). The mixture was refluxed for 12 hr. The solid was removed by filtration. The filtrate was washed with 5% Na₂SO₃ (2x30 mL), 10% NaHCO₃ (30 mL), and brine (60 mL). The solution was dried on Na₂SO₄ and condensed under reduced pressure to give 250 mg of compound **(171)** in 76% yield. LC-MS (m/z) 337.1 [M+H]⁺. ¹H-NMR δ (400 MHz, CHCl₃-*d*) major characteristic peaks: δ 1.20(s, 3H), 1.23 (s, 3H), 5.83(m, 1H), 6.25(m,1H), 7.05(m, 1H), 7.13(m, 1H), 7.61(s, 1H). ¹³C-NMR δ (400 MHz, CHCl₃-*d*) : 211.35, 157.92, 149.55, 136.02, 129.44, 118.82, 118.36, 116.99, 56.72, 52.10, 46.27, 44.48, 38.63, 38.01, 36.08, 35.48, 28.95, 28.38, 21.29, 18.80, 11.54.

### Example 146

### Preparation of Compound (172) and Compound (173)

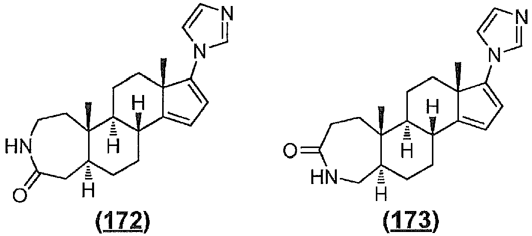

A mixture of compound **(171)** (135 mg, 0.402 mmol), NH₂OH·HCl (56 mg, 0.804 mmol) and pyridine (63.6 mg) in 30 mL of EtOH was stirred at rt for 2 hr. EtOH was evaporated under reduced pressure. 25 mL of DCM and 10 mL of water was added. The layers were partitioned with separatory funnel. The water phase was extracted with 30 mL of DCM. The combined DCM layers were washed with brine, dried over MgSO₄ and condensed under vacuum to give 150 mg of the oxime. The oxime was dissolved in 30 mL of DCM and cooled to 0 °C. 0.86 g of SOCl₂ was added dropwise within 30 min. The mixture was stirred at room temperature for 5 hr. The mixture was poured to 30 mL of icy saturated NaHCO₃. The layers were partitioned. The organic phase was washed with brine, dried on MgSO₄ and purified by prep HPLC to give compound **(172)** and compound **(173). Compound (172):** LC-MS (m/z) 351.9[M+H]⁺. ¹H-NMR δ (400 MHz, CHCl₃-*d*) major characteristic peaks: δ 1.20(s, 3H), 1.23 (s, 3H), 2.80(m, 1H), 3.05(m, 1H), 3.42(m,1H), 5.83(m, 1H), 6.29(s, 2H), 7.11(m, 1H), 7.13(m, 1H), 7.81(s, 1H). ¹³C-NMR δ (400 MHz, CHCl₃-*d*) : 177.21, 157.55, 147.91, 118.66, 166.07, 75.96, 55.60, 50.90, 41.97, 41.28, 38.49, 38.25, 36.69, 34.44, 33.81, 29.45, 28.67, 28.18, 20.09, 17.61, 11.06. **Compound (173):** LC-MS (m/z) 351.9[M+H]⁺. ¹H-NMR δ (400 MHz, CHCl₃-*d*) major characteristic peaks: δ 1.20(s, 3H), 1.23 (s, 3H), 2.65(m, 2H), 3.45(m, 1H), 5.83(m, 1H), 6.29(s, 1H), 6.61(s, 1H), 7.11(m, 1H), 7.13(m, 1H), 7.81(s, 1H). ¹³C-NMR δ (400 MHz, CHCl₃-*d*) : 177.45, 157.48, 1488.07, 118.46, 115.92, 75.72, 55.31, 50.92, 48.30, 43.82, 38.08, 34.53, 34.45, 34.19, 30.27, 27.91, 26.22, 19.83, 17.68, 11.13.

### Example 147

### Preparation of Compound (174)

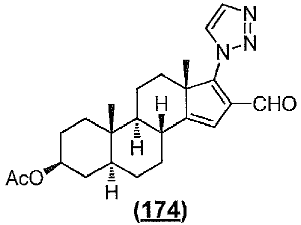

A mixture of 2.41 g of compound **(167)** (6.30 mmol), 3.30 g of K₂CO₃ (23.6 mmol) and 1.40 mg of 1*H*-1,2,3-triazole (20.2 mmol) in 45 mL of DMF was stirred at 80 °C for 2 hr. After being cooled to room temperature, the mixture was poured to ice-water (150 mL), and extracted with EtOAc (3x60). The combined EtOAc phase was washed with brine, dried on MgSO₄ and purified by FCC to give 2.20 g of compound **(174)** in 84% yield. ¹H-NMR δ (400 MHz, CHCl₃-*d*) major characteristic peaks: δ1.01(s, 3H), 1.33(s, 3H), 2.02(S, 3H), 4.72(m, 1H), 6.30(s, 1H), 7.86(m, 2H), 10.68(s, 1H).

### Example 148

### Preparation of Compound (175)

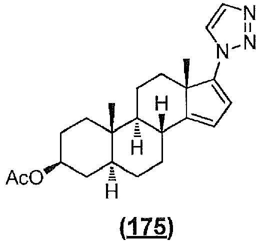

To a solution of compound **(174)** (2.20 g) in PhCN (40 ml) was added 5.00 g of dry Pd/C (5%). The mixture was refluxed for 3 hr under N₂ protection. PhCN was evaporated. The residue was purified through FCC eluenting with EtOAc/Hexanes (1/9→1/8) to get 1.4 g of compound **(175)** in 68.3% yield. LC-MS (m/z) 381.9[M+H]⁺. ¹H-NMR δ (400 MHz, CHCl₃-*d*) major characteristic peaks: δ1.03(s, 3H), 1.33(s, 3H), 2.01(s, 3H), 4.71(m, 1H), 5.83(m, 1H), 6.71(m, 1H), 7.70(m, 2H). ¹³C-NMR δ (400 MHz, CHCl₃-*d*) : 170.63, 158.96, 151.94, 134.18, 116.39, 116.15, 73.46, 57.59, 52.04, 44.37, 37.03, 36.04, 35.88, 35.42, 33.87, 29.24, 27.37, 21.46, 20.85, 18.69, 12.28.

### Example 149

### Preparation of Compound (176)

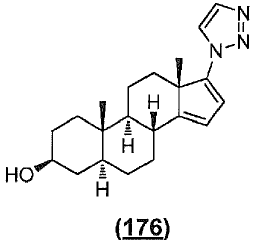

A mixture of compound **(175)** (500 mg, 1.31 mmol) and NaOH (130 mg) in MeOH (20 ml), THF (20 mL) and water (2 mL) was refluxed for 1 hr. After being cooled to room temperature the mixture was extracted with EtOAc (2x50 mL). The combined organic phase was washed with brine (80 mL), dried over MgSO₄ and condensed under vacuum to get 480 mg of compound **(176).** LC-MS (m/z) 339.9[M+H]⁺. ¹H-NMR δ (400 MHz, CHCl₃-*d*) major characteristic peaks: δ*0.93*(s, 3H), 1.33(s, 3H), 3.53(m, 1H), 5.83(m, 1H), 6.71(m,1H), 7.70(m, 2H). ¹³C-NMR δ (400 MHz, CHCl₃-*d*) : 159.42, 151.90, 134.18, 116.26, 116.23, 71.03, 57.57, 52.08, 44.58, 37.98, 36.07, 35.96, 35.48, 31.34, 29.33, 28.28, 20.91,18.70, 12.40.

### Example 150

### Preparation of Compound (177)

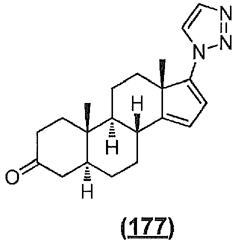

Dess-Martin periodinane (8.40 g, 19.8 mmol) was added to a solution of compound **(176)** (1.15 g, 3.30 mmol) in CH₂Cl₂ (80 ml). The mixture was refluxed overnight. The solid was removed by filtration. The filtrate was washed with 5% Na₂SO₃ (2x30 mL), 10% NaHCO₃ (30 mL), and brine (60 mL). The solution was dried on Na₂SO₄, condensed under reduced pressure and purified by FCC to give 900 mg of compound **(177)** in 79% yield. LC-MS (m/z) 350.9[M+Na]⁺. ¹H-NMR δ (400 MHz, CHCl₃-*d*) major characteristic peaks: δ*1.13*(s, 3H), 1.33(s, 3H) 5.83(m, 1H), 6.71(m,1H), 7.70(m, 2H). ¹³C-NMR δ (400 MHz, CHCl₃-*d*) : 211.45, 158.29, 152.00, 134.26, 116.74, 116.09, 57.03, 52.03, 46.27, 44.53, 38.64, 38.04, 36.16, 35.76, 35.30, 29.00, 28.48, 21.14, 18.67, 11.56.

### Example 151

### Preparation of Compounds (178) and (179)

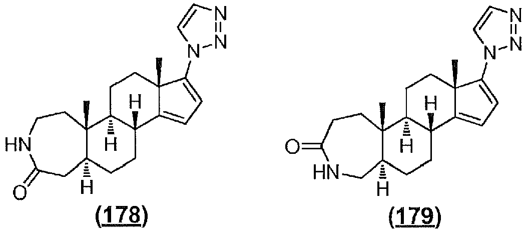

A mixture of compound **(177)** (300 mg, 0.889 mmol), NH₂OH·HCl (130 mg, 1.77 mmol) and pyridine (150 mg) in 30 mL of EtOH was stirred at rt for 2.5 hr. EtOH was evaporated under reduced pressure. 50 mL of DCM and 20 mL of water was added. The layers were partitioned with separatory funnel. The water phase was extracted with 50 mL of DCM. The combined DCM layers were washed with brine, dried over MgSO₄ and condensed under vacuum to give 350 mg of the oxime. The oxime was dissolved in 30 mL of DCM and cooled to 0 °C. 1.76 g of SOCl₂ was added dropwise within 30 min. The mixture was stirred at room tenmeprature for 5 hr. The mixture was poured to 30 mL of ice-water. The layers were partitioned. The organic phase was washed with brine, dried on MgSO₄ and purified by prep HPLC to give compound **(178)** and compound **(179). Compound (178):** LC-MS (m/z) 352.9[M+H]⁺. ¹H-NMR δ (400 MHz, CHCl₃-*d*) major characteristic peaks: δ1.03(s, 3H), 1.33(s, 3H), 2.65(m, 1H), 2.73(m, 1H), 3.02(m, 1H), 3.47(m, 1H), 5.83(m, 1H), 5.98(s, 1H), 6.71(m,1H), 7.70(m, 2H). ¹³C-NMR δ (400 MHz, CHCl₃-*d*) : 178.50, 157.39, 151.03, 133.24, 115.89, 115.19, 76.22, 56.40, 50.78, 42.00, 38.51, 38.34, 36.79, 34.72, 33.62, 30.92, 29.59, 28.36, 21.69, 17.54, 11.07. **Compound (179)** LC-MS (m/z) 352.9[M+H]⁺. ¹H-NMR δ (400 MHz, CHCl₃-*d*) major characteristic peaks: δ 1.03(s, 3H), 1.33(s, 3H), 2.67(m, 2H), 3.47(m, 1h), 5.83(m, 1H), 6.28(s, 1H),6.71(m, 1H), 7.70(m, 2H). ¹³C-NMR δ (400 MHz, CHCl₃-*d*) : 178.51, 158.42, 152.03, 134.27, 116.72, 116.08, 77.24, 57.16, 51.48, 49.38, 44.42, 39.19, 35.75, 35.59, 35.00, 31.29, 29.70, 29.00, 27.38, 20.72, 18.61, 12.15.

### Example 152

### Preparation of Compounds (180), (181), and (182)

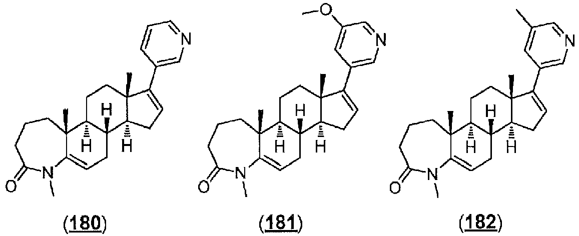

### Example 152A

### Preparation of Compound (180a)

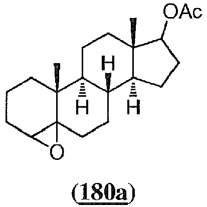

To a solution of the compound (**89c**) (1.0 g, 3.16 mmol) in DCM (20 mL) was added a solution of *m*-CPBA (1.5 g, 8.85 mmol) in DCM (20 mL) at 0 °C. The mixture was stirred at room temperature for 2 h. and sat. aq. NaHSO₃ solution (100 mL) was added. The organic phase was separated, washed by water and brine, dried over Na₂SO₄, and purified by column chromatography (petroleum ether/ethyl acetate = 50/1-25/1, v/v) to afford the title compound **(180a)** (1.0 g, 95 %) as a white solid. ¹H NMR (CDCl₃, 400 MHz) major characteristic peaks: *δ* (ppm) 0.74 (s, 3 H), 0.94 (s, 1.5 H), 0.99 (s, 1.5 H), 1.97 (s, 3 H), 2.86 (m, 1 H), 4.53 (t, *J=* 8.4, 1 H).

### Example 152B

### Preparation of Compound (180b)

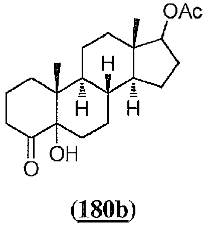

To a solution of compound **(180a)** (1.0 g, 3.0 mmol) in MEK (40 mL) was added a solution of CrO₃ (1.1 g, 10.5 mmol) in water (3.3 mL) at 0 °C and the reaction was stirred at 0 °C for 20 mins. Water (100 mL) was added and the product was extracted by ethyl acetate (100 mL x 3). The organic phase was washed with water and brine, dried over Na₂SO₄, and purified by column chromatography (petroleum ether/ethyl acetate = 20/1-10/1, v/v) to afford the title compound (**180b**) (0.5 g, 49 %) as a white solid.¹H NMR (CDCl₃, 400 MHz) major characteristic peaks: *δ* 0.54 (s, 3 H), 0.58 (s, 3 H), 1.80 (s, 3 H), 2.86 (m, 1 H), 4.37 (t, *J=* 8.4 Hz, 1 H).

### Example 152C

### Preparation of Compound (180c)

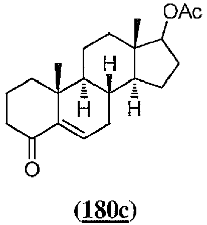

To a solution of compound (**180b**) (10.0 g, 30.0 mmol) in pyridine (100 mL) was added SOCl₂ (14.0 g, 120.0 mmol) at 0 °C and stirred for 45 mins. Ethyl acetate (300 mL) was added and the organic phase was washed by aq. HCl solution (1 M), water, and brine, was dried over Na₂SO₄, and was purified by column chromatography (petroleum ether/ethyl acetate = 20/1-10/1, v/v) to afford the title compound (**180c**) (8.5 g, 87 %) as a white solid. ¹H NMR (CDCl₃, 400 MHz) major characteristic peaks: *δ* 0.82 (s, 3 H), 0.97 (s, 3 H), 2.05 (s, 3 H), 4.62 (t, *J =* 8.0 Hz, 1 H), 6.41 (m, 1 H).

### Example 152D

### Preparation of Compound (180d)

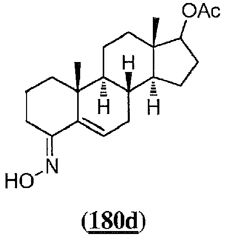

A mixture of compound (**180c**) (1.0 g, 3.0 mmol), NH₂OH HCl (0.42 g, 6.1 mmol) and Pyridine (0.72 g, 9.1 mmol) in EtOH (50 mL) was stirred at 25 °C for 1 h. The mixture was then poured into water (100 mL). The solid was filtered and dried under reduced pressure to give the title compound (**180d**) (0.9 g, 86 %) as a white solid. ¹H NMR (CDCl₃, 400 MHz) major characteristic peaks: *δ* (ppm) 0.81 (s, 3 H), 0.95 (s, 3 H), 2.04 (s, 3 H), 3.30 (m, 1 H), 4.62 (t, *J =* 8.0 Hz, 1 H), 5.88 (m, 1 H).

### Example 152E

### Preparation of Compound (180e)

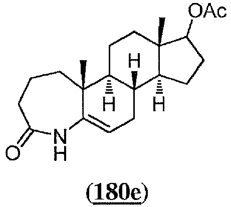

To a solution of compound (**180d**) (0.9 g, 2.6 mmol) in THF (20 mL) was added dropwise SOCl₂ (1.1 mL) at 0 °C under N₂ protection and the mixture was stirred at 0 °C for 1 h. Ethyl acetate (100 mL) was added, the organic phase was washed with sat. aq. NaHCO₃ solution, water, and brine, was dried over Na₂SO₄, and was purified by column chromatography (petroleum ether/ethyl acetate = 5/1-1/1, v/v) to afford the title compound (**180e**) (210 mg, 23 %) as a yellow solid.LC-MS (m/z): 346 (M+1) ¹H NMR (CDCl₃, 400 MHz) major characteristic peaks: *δ* (ppm) 0.81 (s, 3 H), 1.02 (s, 3 H), 2.04 (s, 3 H), 4.61 (t, *J* = 8.8 Hz, 1 H), 5.55 (m, 1 H), 6.96 (s, 1 H).

### Example 152F

### Preparation of Compound (180f)

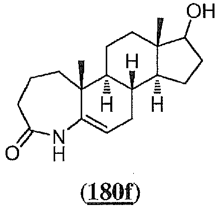

To a solution of compound **(180e)** (200 mg, 0.58 mmol) in MeOH (20 mL) was added LiOH H₂O (84 mg, 2.32 mmol) and the mixture was stirred at 30 °C for 2 h. It was diluted with water (50 mL) and treated with ethyl acetate (100 mL) three times. The organic phases were collected and washed with water and brine, and were dried over Na₂SO₄. The filtrate was concentrated to give the title compound (**180f**) (190 mg, >100 %) as a yellow solid. LC-MS (m/z): 304 (M+1) ¹H NMR (CDCl₃, 400 MHz) major characteristic peaks: *δ* (ppm) 0.76 (s, 3 H), 1.03 (s, 3 H), 3.67 (t, *J=* 8.4 Hz, 1 H), 5.55 (m, 1 H), 6.82 (s, 1 H).

### Example 152G

### Preparation of Compound (180g)

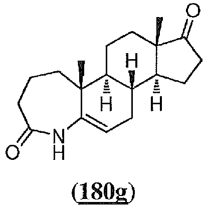

To a solution of compound (**180f**) (1.0 g, 3.3 mmol) in DCM (50 mL) was added pyridinium chlorochromate (1.4 g, 6.6 mmol) and the mixture then was stirred at 25 °C for 1 h and then filtered. The filtrate was diluted with DCM (100 mL) and washed with water, brine, dried over Na₂SO₄, concentrated, purified by column chromatography (petroleum ether/ethyl acetate = 1/1-1/2, v/v) to afford the title compound (**180g**) (250 mg, 25%) as a yellow-brown oil. LC-MS (m/z): 302 (M+1) ¹H NMR (CDCl₃, 400 MHz) major characteristic peaks: *δ* (ppm) 0.86 (s, 3 H), 1.05 (s, 3 H), 5.59 (m, 1 H), 6.90 (s, 1 H).

### Example 152H

### Preparation of Compound (180h)

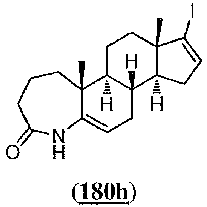

A mixture of compound (**180g**) (230 mg, 0.76 mmol), N₂H₄.H₂O (732mg, 22.89 mmol) and Et₃N (230mg, 2.29 mmol) in EtOH (10 mL) was stirred at reflux for 2 hrs. The reaction solution was concentrated to give the yellow solid. To a mixture of aboved solid in CHCl₃ (5 mL) and Et₃N (307 mg, 3.04 mmol) was added dropwise a solution of I₂ (580 mg, 2.28 mmol) in THF (5 mL) at 0 °C, then the mixture was stirred for 2 h at this temperature. After the concentration, it was dissolved in ethyl acetate (100 mL), washed with sat. aq. NaHSO₃ solution, water, brine, and dried over Na₂SO₄. Then was purified by pre-TLC (petroleum ether/ethyl acetate =2:3, v/v) to afford the title compound **(180h)** (150 mg, 65%) as a light yellow solid. LC-MS (m/z): 412 (M+1) ¹H NMR (CDCl₃, 400 MHz) major characteristic peaks: *δ* (ppm) 0.76 (s, 3 H), 1.05 (s, 3 H), 5.57 (m, 1 H), 6.15 (m, 1 H), 7.09 (s, 1 H).

### Example 152I

### Preparation of Compound (180i)

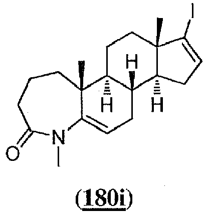

To a solution of compound **(180h)** (150 mg, 0.36 mmol) in DMF (5 mL) was added NaH (18 mg, 0.72 mmol) in portions, then the mixture was stirred at 25 °C for 0.5 h. Then MeI (258 mg, 1.82 mmol) was added and was stirred for another 2 h. It was diluted with ethyl acetate (100 mL), washed with water, brine, dried over Na₂SO₄, the filtrate was concentrated to give the crude compound (**180i**) (100 mg, 65%) as a yellow solid. LC-MS (m/z): 426 (M+1) ¹H NMR (CDCl₃, 400 MHz) major characteristic peaks: *δ* (ppm) 0.77 (s, 3 H), 1.04 (s, 3 H), 3.14 (s, 3H), 5.63 (m, 1 H), 6.16 (m, 1 H).

### Example 152J

### Preparation of Compounds (180), (181), and (182)

A mixture of compound (**180i**) (100 mg, 0.24 mmol), pyridin-3-ylboronic acid (58 mg, 0.47 mmol), K₂CO₃ (97 mg, 0.71 mmol) and Pd(dppf)Cl₂ (18 mg, 0.02 mmol) in dioxane (10 mL) and H₂O (2 mL) was stirred at 80 °C for 2 h under N₂ protection. Then was cooled down, diluted by water, and extracted with ethyl acetate (100 mL x 3), the combined organic phase was washed with water, brine and dried over Na₂SO₄. The crude product was purified by prep-TLC (petroleum ether/ethyl acetate = 1:3, v/v) to afford the title compound (**180**) (15 mg, 18 %) as a white solid. LC-MS (m/z): 377 [M+1]⁺;
¹H NMR (CDCl₃, 400 MHz) major characteristic peaks: *δ* (ppm) 1.06 (s, 3 H), 1.07 (s, 3 H), 3.15 (s, 3 H), 5.66 (m, 1 H), 6.01 (m, 1 H), 7.24 (dd, *J*₁ = 4.8, 7.6 Hz, 1 H), 7.64 (d, *J =* 8.0 Hz, 1 H), 8.47 (d, *J=* 4.0 Hz, 1 H), 8.63 (s, 1 H).

Compound (**181**) was prepared in a similar manner as a white solid (yield 35%). LC-MS: (m/z) 407 [M+1]⁺; ¹H NMR (CDCl₃, 400 MHz) major characteristic peaks: *δ* (ppm) 1.06 (s, 3H), 1.07 (s, 3H), 3.15 (s, 3H), 3.87 (s, 3H), 5.65 (s, 1H), 6.02 (s, 1H), 7.16 (s, 1H), 8.19 (s, 1H), 8.25 (s, 1H).

Compound **(182)** was prepared in a similar manner as a white solid (yield 29%) as a white solid. LC-MS: 391.2 [M+1]⁺; ¹H NMR (CDCl₃, 400 MHz) major characteristic peaks: *δ* (ppm) 1.05 (s, 3 H), 1.07 (s, 3 H), 2.33 (s, 3 H), 3.15 (s, 3H), 5.65-5.67 (m, 1H), 5.99 (s,1 H), 7.46 (s,1H), 8.31 (s,1H), 8.43 (s, 1H).

### Example 153

### Preparation of Compound (183)

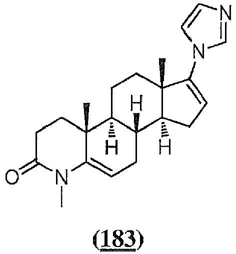

(4aR,6aS)-1,4a,6a-Trimethyl-7-iodo-4,4a,4b,5,6,6a,9,9a,9b,10-decahydro-1H-indeno[5,4-f]quinolin-2(3H)-one (200 mg, 0.49 mmol), imidazole (296 mg, 4.41 mmol), *L-*proline (33.8 mg, 0.294 mmol), K₂CO₃ (135 mg, 0.98 mmol) were charged with a 50 mL of dried three-necked round bottom flask equipped with magnetic stirrer, thermometer. The mixture was degassed with N₂ for 3 times and CuI (39 mg, 0.196 mmol) was added under N₂, DMSO (9 mL) was added subsequently by syringe. After this, the reaction mixture was rapidly placed into a preheated oil bath (110 °C) and stirred at 110 °C for 35 min until the color of the solution became green from blue, then heated to 130 °C and stirred for 17 hours. Then the reaction mixture was cooled to room temperature and diluted with water (40 mL) and extracted with dichloromethane (25 mL x 3). The combined organic layers were washed with brine (25 mL) and dried over Na₂SO₄, evaporated to obtain the crude product. The crude product was purified by prep-HPLC to afford pure product (**183**) (41.5 mg, 24%) as a yellow solid. LC-MS (ESI) m/z: 352 (M+H)⁺; ¹H-NMR (.400 MHz, CDCl₃) major characteristic peaks: *δ* (ppm) 1.031 (s, 3H), 1.106 (s, 3H), 3.144 (s, 3H), 5.069 (m, 1H), 5.717 (m, 1H), 7.058 (s, 1H), 7.100 (s, 1H), 7.645 (s, 1H).

### Example 154

### Preparation of Compound (184)

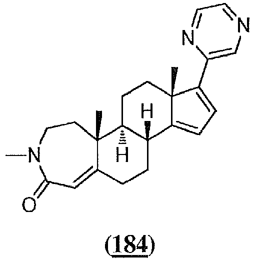

A mixture of **(158h)** (380 mg, 0.85 mmol), 2-(tributylstannyl)pyrazine (632 mg, 1.70 mmol), LiCl (216 mg, 5.1 mmol) and CuI (846 mg, 4.25 mmol) in THF (15 mL) was stirred at 80 °C under N₂ atmospher overnight. Filtered and the cake was washed with ethyl acetate (10 mL). The filtrate was concentrated to give the crude product which purified by Prep-HPLC. The final compound (**184**) (38 mg, 12%) was obtained as a white solid. LC-MS (m/z): 376 [M+H]^{+ 1}H NMR (CDCl₃, 400 MHz) major characteristic peaks: *δ* 1.25 (s, 3H), 1.32 (s, 3H), 3.03 (s, 3H), 5.88 (m, 1H), 6.04 (m, 1H), 7.18 (m, 1H), 8.24 (m, 1H), 8.44 (m, 1H), 8.82 (m, 1H).

### Example 155

### Preparation of Compounds (185), (186), (187), (188), and (189)

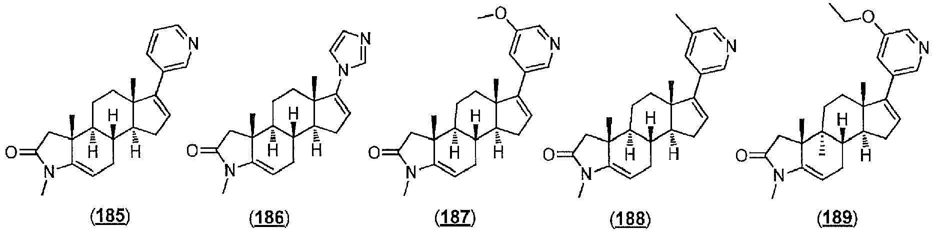

### Example 155A

### Preparation of Compound (185a)

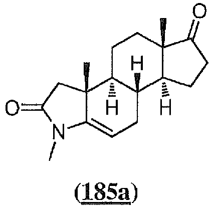

To a solution of 2-((3aS,6R)-3a,6-dimethyl-3,7-dioxododecahydro-1H-cyclopenta[a]naphthalen-6-yl)acetic acid (2.3 g, 7.9 mmol) in MeOH (50 mL), MeNH₂ (11.5 mL) was added to the mixture, heated to reflux for 2 h, then evaporated to remove MeOH and MeNH₂. The residue (2.4 g, 7.9 mmol) was dissolved in xylene (50 mL) and the mixture was stirred at 140 °C for 2 h. Then cooled to room temperature, the mixture was evaporated to remove xylene under the reduce pressure. The crude product was purified by column chromatography (petroleum ether/ethyl acetate/DCM =3/1/1, v/v/v) to give **(185a)** (2.3 g, 92%) as a white solid. LC-MS (m/z): 288 [M+H]^{+ 1}H NMR (CDCl3, 400 MHz) peaks: *δ* (ppm) 0.92 (s, 3H), 1.14 (s, 3H), 2.92 (s, 3H), 4.74 (s, 1H).

### Example 155B

### Preparation of Compound (185b)

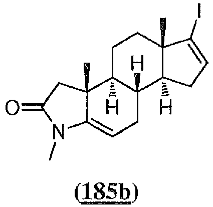

To a mixture of **(185a)** (1.8 g, 6.3 mmol) in ethanol (50 mL) was added Et₃N (633 mg, 6.3 mmol) and N₂H₄. H₂O (85%) (2.5 g, 63 mmol) and the mixture was reflux for 2 hours, and the solvent was removed and the residue was dissolved in DCM (60 mL), washed with water (2 x 50 mL), brine (1 x 50 mL), dried and concentrated to give the crude product as a white solid. The crude product (1.9 g, 6.3 mmol) was dissolved in DCM (50 mL) and Et₃N (1.27 g, 12.6 mmol) was added. A solution of iodine (2.4 g, 9.45 mmol) in THF (10 mL) was added dropwise at 0 °C. The mixture was stirred at room temperature for 2 hours. Washed with sat.Na₂S₂O₃, diluted with ethyl acetate (100 mL), the organic layer was separated and washed with brine (1 x 50 mL), dried and concentrated. The crude product was purified by column chromatography (ethyl acetate in petroleum, 33% v/v) to give (**185b**) (1.5 g, 60%) as a yellow solid. LC-MS (m/z): 398 [M+H]^{+ 1}H NMR (CDCl₃, 400 MHz) peaks: *δ* (ppm) 0.72 (s, 3H), 1.08 (s, 3H), 2.84 (s, 3H), 4.65 (s, 1H), 6.08 (m, 1H).

### Example 155C

### Preparation of Compounds (185), (186), (187), (188), and (189)

To a solution of (**185b**) (300 mg, 0.76 mmol) in dioxane (30 mL) was added pyridin-3-ylboronic acid (186 mg, 1.5 mmol), potassium carbonate (521 mg, 3.76 mmol) in water (5 mL), and bis(triphenylphosphine) palladium chloride (50 mg). The mixture was thoroughly degassed, and heated under nitrogen at 80 °C for 1 hour. After being filtered through a pad of Celite, the crude product was purified by pre-HPLC to give (**185**) (42 mg, 16%) as a white solid. LC-MS (m/z): 349 [M+H]^{+ 1}H NMR (CDCl3, 400 MHz) peaks: *δ* (ppm) 1.07 (m, 3H), 1.17 (m, 3H), 2.23 (s, 2H), 2.92 (s, 3H), 4.76 (t, *J =* 3.6 Hz, 1H), 6.00 (s, 1H), 7.23 (m, 1H), 7.64 (d, *J* = 7.6 Hz, 1H), 8.47 (d, *J =* 4.4 Hz, 1H), 8.62 (s, 1H).

Compound (**186**) (yield 16%) was obtained as a white solid. LC-MS (m/z): 338 [M+H]^{+ 1}H NMR (CDCl₃, 400 MHz) major characteristic peaks: *δ* (ppm) 1.04 (s, 3H), 1.17 (s, 3H), 2.23 (s, 2H), 2.92 (s, 3H), 4.75 (m, 1H), 5.71 (m, 1H), 7.05 (m, 1H), 7.10 (m, 1H), 7.63 (m, 1H).

Compound (**187**) (yield 12%) was obtained as a white solid. LC-MS (m/z): 379 [M+H]^{+ 1}H NMR (CDC13, 400 MHz) peaks: *δ* (ppm) 1.07 (s, 3H), 1.17 (s, 3H), 2.22 (s, 2H), 2.92 (s, 3H), 3.87 (s, 3H), 4.76 (t, *J =* 3.6 Hz, 1H), 6.01 (t, *J =* 1.2 Hz, 1H), 7.17 (t, *J =* 2.4 Hz, 1H), 8.18 (d, *J* = 2.8 Hz, 1H), 8.24 (d, *J =* 1.6 Hz, 1H).

Compound (**188**) (yield 16%) was obtained as a white solid. LC-MS (m/z): 363 [M+H]^{+ 1}H NMR (CDC13, 400 MHz) peaks: *δ* (ppm) 1.07 (s, 3H), 1.17 (s, 3H), 2.22 (s,2H), 2.33 (s, 3H), 2.92 (s, 3H), 4.76 (t, *J =* 3.6 Hz, 1H), 5.97 (m, 1H), 7.45 (s, 1H), 8.31 (s, 1H), 8.42 (d, *J* = 1.6 Hz, 1H).

Compound (**189**) (yield 39%) was obtained as a white solid. LC-MS (m/z): 393 [M+H]^{+ 1}H NMR (CDCl₃, 400 MHz) peaks: *δ* (ppm) 1.07 (s, 3H), 1.17 (s, 3H), 1.45 (t, *J* = 6.8 Hz, 3H), 2.22 (s, 2H), 2.92 (s, 3H), 4.09 (q, *J* = 6.8 Hz, 2H), 4.75 (m,1H), 5.99 (m, 1H), 7.15 (t, *J =* 2 Hz, 1H), 8.17 (d, *J* = 2.8 Hz, 1H), 8.22 (d, *J* = 1.6 Hz, 1H).

### Example 156

### Preparation of Compound (190)

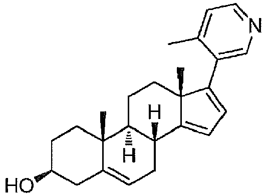

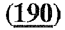

### Example 156A

### Preparation of Compound (190a)

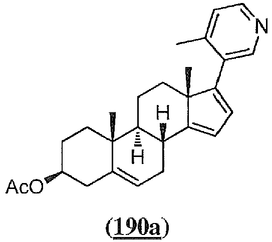

To a solution of (**189**) (460 mg, 1 mmol) in 1,4-dioxane (20 mL) was added 4-methylpyridin-3-ylboronic acid (306 mg, 2.01 mmol), potassium carbonate (732 mg, 5.31 mmol) in water (2 mL), and Pd(dppf)Cl₂ (86 mg). The mixture was thoroughly degassed, and heated under nitrogen at 80 °C for 4 hour. After being filtered through a pad of Celite, the crude product was purified by silica gel chromatography (ethyl acetate in petroleum, 20% v/v) to give (**190a**) (120 mg, 34%) as a white solid. LC-MS (m/z) 404[M+H]⁺.

### Example 156B

### Preparation of Compound (190)

To a solution of (**190a**) (120 mg, 0.21 mmol) in MeOH/H₂O (10/2 mL) was added NaOH (60 mg, 1.5 mmol). The mixture was stirred at room temperature for 2 hours. Evaporated the solvent and the residue was poured into ice. Extracted with DCM (10 mL * 2) and combined the organic layers, dried and evaporated to give (**190**) (50 mg, 45%) as a grey solid. LC-MS (m/z): 362[M+H]⁺. ¹H NMR (CDCl₃, 400 MHz) major characteristic peaks: *δ* (ppm) 1.03 (s, 3H), 1.13 (s,3H), 2.26 (s,3H), 3.56 (m, 1H), 5.49 (m, 1H), 6.00 (m, 1H), 6.26 (s, 1H), 7.16 (m, 1H), 8.35 (s, 1H).

### Example 157

### Preparation of Compounds (191), (192), (193), (194), and (195)

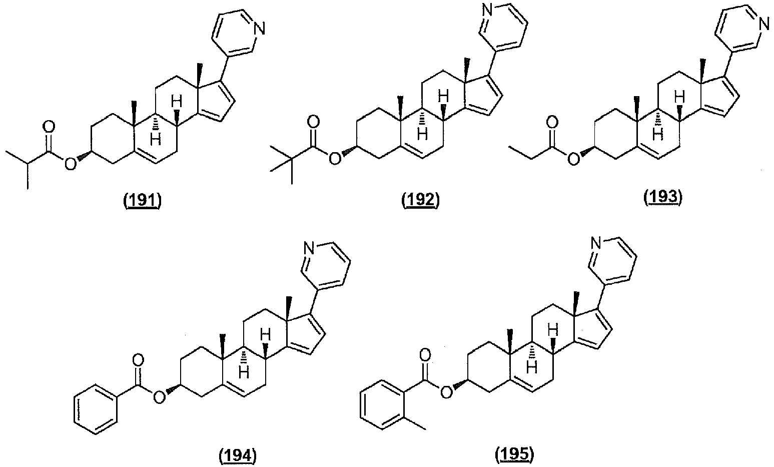

To a solution of (**79**) (347 mg, 1 mmol) in DCM (10 mL) was added Et₃N (2 mL). When the reaction was stirred at room temperature, isobutyryl chloride (0.5 mL) was added dropwise. The reaction mixture was stirred at room temperature for 2 hours. Poured the mixture into ice, sepearted and the solvent was evaporated under the vacumm. The residue was purified with silicagel chromatography (petroleum ether/ethyl acetate = 5/1, v/v) to afford (**191**) (50 mg, 14%) as a yellow solid.
LC-MS (m/z): 418 [M+H]⁺. ¹H NMR (CDCl₃, 400 MHz) major characteristic peaks: *δ* (ppm) 1.15 (s, 3H), 1.17 (s, 6H), 1.21 (s, 3H), 4.61 (m, 1H), 5.50 (m, 1H), 6.02 (m, 1H), 6.81 (s, 1H), 7.24 (m, 1H), 7.80 (m, 1H), 8.42 (m, 1H), 8.77 (s, 1H).

Compound (**192**) was prepared in a similar manner as a white solid (yield 80%). LC-MS: (m/z) (M+H)⁺ = 432. ¹H-NMR (400 MHz, CDCl₃) major characteristic peaks: *δ* (ppm) 1.17 (s, 3H), 1.19 (s, 9H), 1.21 (s, 3H), 4.58-4.62 (m, 1H), 5.50 (s, 1H), 6.03 (s, 1H), 6.82 (d, *J=* 2.0 Hz, 1H), 7.26 (s, 1H), 7.79 (d, *J=* 8.0 Hz, 1H), 8.44 (s, 1H), 8.79 (s, 1H).

Compound (**193**) was prepared in a similar manner as a white viscous solid (yield 16%). LC-MS (m/z): 404 [M+H]⁺. ¹H NMR (CDCl₃, 400 MHz) major characteristic peaks: *δ* (ppm) 1.14 (s, 3H), 1.15 (m, 3H), 1.19 (s, 3H), 2.31 (m, 2H), 4.63 (m, 1H), 5.50 (s, 1H), 6.01 (s, 1H), 6.80 (s, 1H), 7.24 (m, 1H), 7.77 (m, 1H), 8.41 (m, 1H), 8.76 (s, 1H).

Compound (**194**) was prepared in a similar manner as a white solid (yield 8%). LC-MS: (M+H)⁺ = 452. ¹H-NMR (400 MHz, CDCl₃) major characteristic peaks: *δ* (ppm) 1.22 (s, 6H), 4.87-4.90 (m, 1H), 5.56 (s, 1H), 6.04 (s, 1H), 6.82 (s, 1H), 7.23-7.25 (m, 1H), 7.43-7.46 (m, 2H), 7.54-7.58 (m, 1H), 7.77-7.80 (m, 1H), 8.04-8.07 (m, 2H), 8.42 (s, 1H), 8.78 (s, 1H).

Compound (**195**) was prepared in a similar manner as a white solid (yield 40%). LCMS: (M+H)⁺ = 466. ¹H-NMR *δ* (ppm) (400 MHz, CDCl₃) major characteristic peaks: *δ* (ppm) 1.21 (s, 3H), 1.22 (s, 3H), 2.60 (s, 3H), 4.83-4.91 (m, 1H), 5.56 (s, 1H), 6.04 (s, 1H), 6.82 (s, 1H), 7.23-7.27 (m, 3H), 7.37-7.41 (m, 1H), 7.78-7.81 (m, 1H), 7.89-7.91 (m, 1H), 8.43 (m, 1H), 8.78 (m, 1H).

### Example 158

### Preparation of Compound (195')

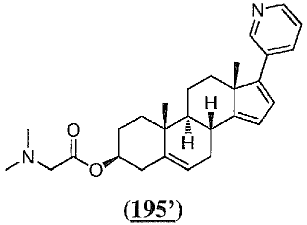

### Example 158A

### Preparation of Compound (195a)

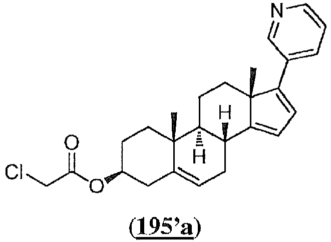

To a solution of (**79**) (200 mg, 0.58 mmol) in dichloromethane (10 mL) was triethylamine (0.5 mL), 2-chloroacetyl chloride (0.5 mL) and stirred at 20 °C for 0.5h. After reaction the mixture was quenched with water (10 mL) and extracted with DCM (50 mL x 2), washed with brine (50 mL x 2), dried over anhydrous Na₂SO₄ and concentrated to give the crude product (**195'a**) (240 mg), which was used in the next step without further purification. LC-MS: (m/z) (M+H)⁺ = 424. ¹H-NMR (400 MHz, CDCl₃) major characteristic peaks: *δ* (ppm) 1.14 (s, 3H), 1.18 (s, 3H), 3.21 (s, 2H), 4.71-4.73 (m, 1H), 5.51 (s, 1H), 6.02 (s, 1H), 6.80 (s, 1H), 7.21-7.25 (m, 1H), 7.76 (d, *J =* 7.6 Hz, 1H), 8.41 (d, *J =* 4.4 Hz, 1H), 8.76 (s, 1H).

### Example 158B

### Preparation of Compound (195')

To a solution of (**195'a**) (240 mg, 0.58 mmol) in dichloromethane (10 mL) was triethylamine (0.8 mL), dimethylamine hydrochloride (475 mg, 5.8 mmol) and stirred at 20 °C for 12 h. After reaction the mixture was quenched with water (10 mL) and extracted with DCM (50 mL x 2), washed with brine (50 mL x 2), dried over anhydrous Na₂SO₄ and concentrated to give the crude product and purified by pre-TLC (ethyl acetate/ petroleum ether = 1/1, v/v) to give the product (**195'**) (60 mg, 24%) as a yellow solid. LCMS: (M+H)⁺ = 433. ¹H-NMR (400 MHz, CDCl₃) major characteristic peaks: *δ* (ppm) 1.16 (s, 3H), 1.20 (s, 3H), 2.36 (s, 6H), 3.16 (s, 2H), 4.70-4.73 (m, 1H), 5.51 (s, 1H), 6.02 (s, 1H), 6.80 (s, 1H), 7.21-7.25 (m, 1H), 7.76 (d, *J=* 7.6 Hz, 1H), 8.41 (d, *J* = 4.4 Hz, 1H), 8.76 (s, 1H).

### Example 159

### Preparation of Compounds (196), (197), and (198)

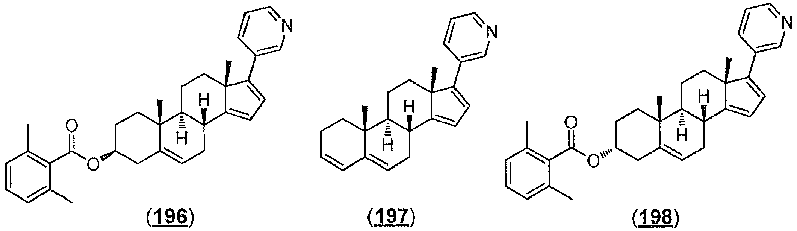

To a solution of compound (**79**) (280 mg, 0.807 mmol), 2,6-dimethylbenzoic acid (182 mg, 1.210 mmol) and PPh₃ (423 mg, 1.614 mmol) in dry THF (2 mL) added dropwise DEAD (182 mg, 1.210 mmol) at 25 °C. After addition completely, the mixture was stirred at this temperature for 2 h. And it was quenched with MeOH and purified by pre-HPLC to obtain (**196**) (40 mg) as a white solid, (**197**) (80 mg) as a white solid, and (**198**) (12 mg) as a white solid.

For Compound (**196**): LC-MS: (m/z) 480 [M+1]⁺; ¹H NMR (CDCl₃, 400 MHz) major characteristic peaks: *δ* (ppm) 1.18 (s, 3H), 1.22 (s, 3H), 2.33 (s, 6H), 4.91-4.99 (m, 1H), 5.58 (s, 1H), 6.04 (s, 1H), 6.81 (s, 1H), 7.03 (d, *J =* 7.6 Hz, 2H), 7.18 (t, *J* = 7.6 Hz, 1H), 7.24 (m, 1H), 7.80 (d, *J=* 8.0 Hz, 1H), 8.42 (s, 1H), 8.78 (s, 1H).

For Compound (**197**): LC-MS: (m/z) 330 [M+1]⁺; ¹H NMR (CDCl₃, 400 MHz) major characteristic peaks: *δ* (ppm) 1.10 (s, 3H), 1.23 (s, 3H), 5.51 (s, 1H), 5.63 (s, 1H), 5.98 (d, *J =* 10.4 Hz, 1H), 6.04 (s, 1H), 6.82 (s, 1H), 7.24 (t, *J=* 4.4 Hz, 1H), 7.77-7.80 (m, 1H), 8.41-8.43 (m, 1H), 8.77 (s, 1H).

For Compound **(198):** LC-MS: (m/z) 480 [M+1]⁺; ¹H NMR (CDCl₃, 400 MHz) major characteristic peaks: *δ* (ppm) 1.19 (s, 3H), 1.21 (s, 3H), 2.32 (s, 6H), 5.32 (s, 1H), 5.44 (s, 1H), 6.82 (s, 1H), 7.00 (d, *J=* 7.6 Hz, 2H), 7.16 (t, *J=* 7.6 Hz, 1H), 7.24 (m, 1H), 7.80 (d, *J=* 8.8 Hz, 1H), 8.41 (s, 1H), 8.76 (s, 1H).

### Example 160

### Preparation of Compounds (199) and (200)

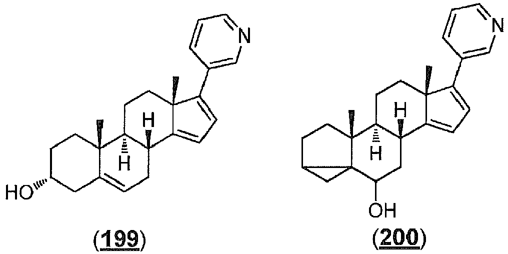

### Example 160A

### Preparation of Compounds (199a) and (200a)

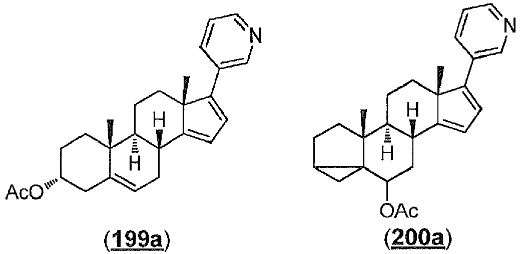

To a solution of Compound (**79**) (1.5 g, 4.3 mmol), AcOH (391 mg, 6.53 mmol) and PPh₃ (1.71 g, 6.53 mmol) in THF (50 mL) was added DEAD (1.14 g, 6.53 mmol) at 0 °C under N₂ protection. Then the mixture was stirred at room temperature overnight. The solution was diluted with ethyl acetate (100 mL), washed with water, brine, dried over Na₂SO₄, concentrated. The crude product was purified by column chromatography (petroleum ether/ethyl acetate =10/1-5/1, v/v) to afford a mixture of (**199a**) and (**200a**) (0.8 g, 47 %) as a colorless oil. LC-MS (m/z): 390 [M+1]⁺.

### Example 160B

### Preparation of Compounds (199) and (200)

To a solution of mixture of (**199a**) and (**200a**) (0.8 g, 2.1 mmol) in MeOH (10 mL) was added a solution of NaOH (0.8 g, 20.5 mmol) in H₂O (2 mL) at room temperature. The mixture was then stirred at 50 °C for 3 h. The solution was diluted with ethyl acetate (100 mL), washed with water, brine, dried over Na₂SO₄, concentrated. The crude product was purified by column chromatography (petroleum ether/ethyl acetate = 5/1-3/1, v/v) to afford the title compound (**199**) (400 mg) and (**200**) (210 mg).

For Compound **(199)** LC-MS (m/z): 348 [M+1]⁺; ¹H NMR (CDCl₃, 400 MHz) major characteristic peaks: *δ* (ppm) 1.15 (s, 3 H), 1.21 (s, 3 H), 4.05 (m, 1H), 5.54 (m, 1 H), 6.02 (m, 1H), 6.81 (m, 1H), 7.23 (m, 1 H), 7.78 (m, 1H), 8.40 (m, 1 H), 8.77 (m, 1H).

For Compound (**200**): LC-MS(m/z): 348 [M+1]⁺; ¹H NMR (CDCl₃, 400 MHz) major characteristic peaks: *δ* 0.37 (m, 1H), 0.58 (t, *J =* 4.4 Hz, 1H), 1.20 (s, 3 H), 1.23 (s, 3 H), 3.44 (m, 1H), 6.00 (m, 1 H), 6.79 (m, 1H), 7.25 (m, 1H), 7.77 (m, 1 H), 8.41 (m, 1H), 8.76 (d, *J* = 2.0 Hz, 1H).

### Example 161

### Preparation of Compound (201)

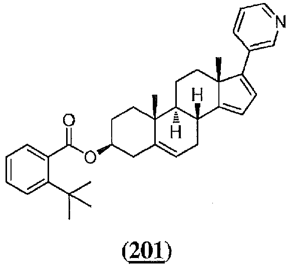

To a solution of compound (**199**) (130 mg, 0.37 mmol) in dry THF (10 mL) 2-(tert-butyl)benzoic acid (80 mg, 0.44 mmol) was added DEAD (0.1 mL, 0.56 mmol), PPh₃ (147 mg, 0.56 mmol) and stirred at 30 °C overnight. After reaction the mixture was diluted with methylene chloride (20 mL), washed with water (20 mL x 2), brine (20 mL x 2), dried over anhydrous Na₂SO₄ and concentrated to give the crude product and purified by pre-HPLC to give the product (**201**) (15 mg, 8%) as a white solid. LC-MS: (m/z) (M+H)⁺ = 508, ¹H-NMR *δ* (400 MHz, CDCl₃) major characteristic peaks: δ (ppm) 1.18 (s, 3H), 1.22 (s, 3H), 1.43 (s, 9H), 4.88-4.92 (m, 1H), 5.58 (s, 1H), 6.05 (s, 1H), 6.82 (s, 1H), 7.21 (t, *J =* 6.8 Hz, 1H), 7.28-7.38 (m, 3H), 7.48 (d, *J =* 8.0 Hz, 1H), 7.80 (d, *J =* 8.0 Hz, 1H).

### Example 162

### Preparation of Compound (202)

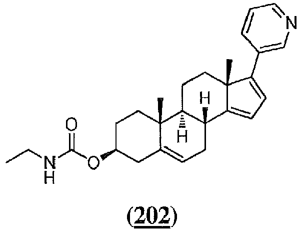

To a solution of Compound (**79**) (200 mg, 0.58 mmol) in THF (20 mL) was added isocyanatoethane (409 mg, 5.76 mmol) and Et₃N (291 mg, 2.88 mmol) at room temperature, then the mixture was stirred at reflux for 48 h. Then the mixture was cooled to room temperature and evaporated to remove solvent and purified by prep-HPLC to give the product (**202**) (40 mg, 17%) as a white solid. LCMS: m/z: 419 (M+1). ¹H NMR (CDCl3, 400 MHz) peaks: *δ* (ppm) 1.14 (m, 6H), 1.21 (s, 3H), 3.22 (q, *J =* 6.4 Hz, 2H), 4.56 (m, 2H), 5.51 (s, 1H), 6.02 (t, *J =* 2.0 Hz, 1H), 6.81 (d, *J* = 2.4 Hz, 1H), 7.23 (m, 1H), 7.77(m.1H), 8.41(d, *J* = 4.0 Hz, 1H), 8.76(d, *J =* 1.6 Hz, 1H).

### Example 163

### Preparation of Compound (203)

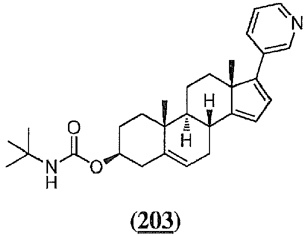

To a mixture of (**79**) (400 mg, 1.15 mmol), 2-methylpropan-2-amine (101 mg, 1.38 mmol) and Et₃N (290 mg, 2.88 mmol) in DCM (20 mL) was added dropwise bis(trichloromethyl) carbonate (676 mg, 2.3 mmol) in DCM (5 mL) at 0 °C under N₂. Then the mixture was stirred at room temperature for 2h, then to the mixture was added ice water, adjusted pH 7 with sat. NaHCO₃. Then the mixture was extracted with DCM (2 x 20 mL), washed with brine, dried over Na₂SO₄. The crude product was purified by pre-HPLC to give the title compound (**203**) (40 mg, 8%) as a white solid. LCMS: m/z: 447 (M+1). ¹H NMR (CDCl₃, 400 MHz) peaks: *δ* (ppm) 1.15 (s, 3H), 1.21 (s, 3H), 1.32 (s, 9H), 4.48 (s, 1H), 4.57 (s, 1H), 0.51(s, 1H), 6.02 (t, *J* = 2.4 Hz, 1H), 6.81 (d, *J* = 2.4 Hz, 1H),7.24 (m, 1H), 7.77 (m, 1H), 8.41 (m, 1H), 8.76 (d, *J =* 2.0 Hz, 1H).

### Example 164

### Preparation of Compound (204)

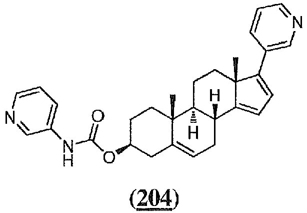

To a suspension of nicotinic acid (36.5 mg, 0.297 mmol) in dry toluene (1.5 mL) was added DIPEA (38.4 mg, 0.297 mmol), followed by adding dropwise DPPA (86 mg, 0.312 mmol). The resulting mixture was stirred at room temperature for 30 mins. Then to the mixture was added Compound (**79**) (50 mg, 0.146 mmol) and the reaction mixture was heated to 80 °C and stirred for 3 hrs. Cooled to room temperature and concentrated. The residue was purified by pre-TLC (DCM/MeOH = 30/1, v/v) to give Compound (**204**) (40 mg, 72%) as a yellow solid. LC-MS (ESI) m/z: 468 (M+1)⁺; ¹H-NMR (400 MHz, CDCl₃) *δ* (ppm) 1.18 (s, 3H), 1.22 (s, 3H), 2.44 (s, 3H), 4.66 (m, 1H), 5.54 (s, 1H), 6.03 (s, 1H), 6.81 (s, 1H), 6.84 (s, 1H), 7.26 (m, 2H), 7.79 (m, 1H), 7.99 (s, 1H), 8.32 (s, 1H), 8.43 (s, 1H), 8.50 (s, 1H), 8.78 (s, 1H).

### Example 165

### Preparation of Compound (205)

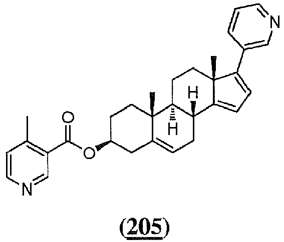

### Example 165A

### Preparation of Compounds (205a), (205a)

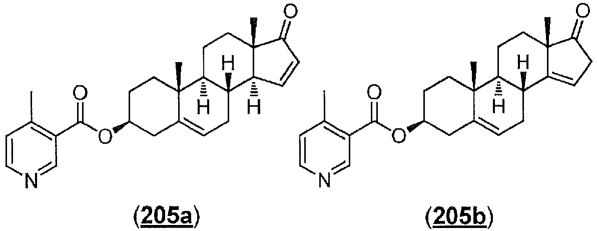

A mixture of compound (**161b**) (700 mg, 2.44 mmol), 4-methylnicotinic acid (1.173 g, 8.55 mmol), DMAP (299 mg, 2.44 mmol) and DCC (1.513 g, 7.332 mmol) in dry toluene (40 mL) was heated to 80 °C and stirred overnight. The mixture was diluted with DCM (30 mL) and filtered. The cake was washed with DCM (5 mL) and the filtrate was concentrated to give crude product. The crude product was purified by column chromatography (ethyl acetate/ petroleum ether = 1/20, v/v) to give Compound (**205a**) (170 mg) and compound (**205b**) (400 mg). Total yield: 57%.

For (**205a**), LC-MS (ESI) m/z: 468 (M+1)⁺; ¹H-NMR (400 MHz, CDCl₃) *δ* (ppm) 1.07 (s, 3H), 1.11 (s, 3H), 2.62 (s, 3H), 4.85 (m, 1H), 5.51 (m, 1H), 6.30 (m, 1H), 7.17 (m, 1H), 7.83 (m, 1H), 8.54 (m, 1H), 9.05 (m, 1H).

For (**205b**), LC-MS (ESI) m/z: 468 (M+1)⁺; ¹H-NMR (400 MHz, CDCl₃) *δ* (ppm) 1.10 (s, 3H), 1.15 (s, 3H), 2.62 (s, 3H), 4.91 (m, 1H), 5.55 (m, 2H), 7.17 (m, 1H), 8.55 (m, 1H), 9.06 (m, 1H).

### Example 165B

### Preparation of Compound (205c)

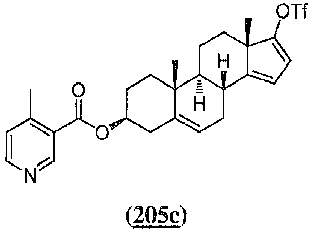

To a stirred solution of compound (**205a**) (140 mg, 0.345 mmol) and imidazole (70.4 mg, 1.035 mmol) in dry DCM (6 mL) was added dropwise Tf₂O (243 mg, 0.862 mmol) at 20 °C. The resulting mixture was stirred at 30 °C for 1.5 hrs. The mixture was poured into ice water, and then extracted with DCM (3 x 40 mL). The combined organic layers were washed with brine (20 mL), dried over Na₂SO₄, and concentrated to give crude product. The crude product was purified by column chromatography (Petroleum ether/EtOAc=20:1 to 5:1, v/v) to give compound (**205c**), (110 mg, 46%) as a brown oil. LC-MS (ESI) m/z: 538 (M+1)⁺.

### Example 165C

### Preparation of Compound (205)

A mixture of compound (**205c**) (110 mg, 0.205 mmol), pyridin-3-ylboronic acid (50.4 mg, 0.41 mmol), K₂CO₃ (84.9 mg, 0.615 mmol) and Pd(PPh₃)₂Cl₂ (14.4 mg, 0.0205 mmol) in dioxane (10 mL) and water (1 mL) was heated to 80 °C and stirred for 1 hr. The mixture was cooled to room temperature and most of solvent was removed under reduce pressure. The residue was diluted with water (10 mL), extracted with DCM (3*20 mL). The combined organic layers were washed with brine (10 mL), dried over Na₂SO₄, and concentrated to give crude product. The crude product was purified by Pre-TLC (DCM/MeOH = 40/1, v/v) to give compound **(205)** (27 mg, 28%) as a yellow oil. LC-MS (ESI) m/z: 467 (M+1)⁺; ¹H-NMR (400 MHz, CDCl₃) *δ* 1.22 (s, 6H), 2.63 (s, 3H), 4.90 (m, 1H), 5.57 (s, 1H), 6.05 (s, 1H), 6.82 (s, 1H), 7.17 (m, 1H), 7.29 (m, 1H), 7.80 (m, 1H), 8.43 (m, 1H), 8.55 (m, 1H), 8.78 (s, 1H), 9.07 (s, 1H).

### Example 166

### Preparation of Compound (206)

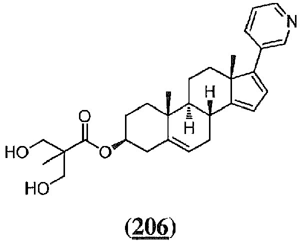

### Example 166A

### Preparation of Compound (206a)

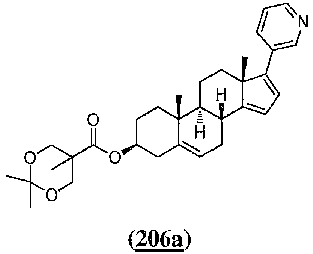

To a solution of Compound (**79**) (90 mg, 0.259 mmol), DMAP (93 mg, 0.778 mmol) of DCM (20 mL) was added 2,2,5-trimethyl-1,3-dioxane-5-carboxylic anhydride (280 mg , 0.778 mmol) in 0 °C. The mixture was stirred at room temperature overnight and then filtered. The filtrate was evaporated in vacuum to obtain a crude product. The crude product was purified by chromatography (petroleum ether/ethyl acetate = 3/1, v/v) to get product (**206a**) (96 mg, 75%). LC-MS (ESI) m/z: 504 (M+H)⁺.

### Example 166B

### Preparation of Compound (206)

To a solution of Compound (**206a**) (96 mg, 0.191 mmol) of THF (30 mL) was added slowly aqueous solution of sulfuric acid (2 N, 3 mL) at 0 °C. And the mixture was stirred at room temperature for 2 h. The mixture was neutralizing with NaHCO₃, and extracted with ethyl acetate (100 mL x 3). The combined extraction was evaporated in vacuum to get crude product. The crude product was purified by prep-HPLC to afford (**206**) (22 mg, 25%) as a white solid. LC-MS (ESI) m/z: 464 (M+H)⁺; ¹H-NMR (400 MHz, CDCL3): *δ* (ppm) 1.03 (s, 3H), 1.13 (s, 3H), 1.16 (s, 3H), 2.53 (s, 3H), 3.35-3.51 (m, 4H), 4.46 (m, 1H), 4.63 (m, 1H), 5.48 (s, 1H), 6.06 (s, 1H), 7.02 (s, 1H), 7.35 (m, 1H), 7.91 (m, 1H), 8.37 (m, 1H), 8.78 (s, 1H).

### Example 167

### Preparation of Compound (207)

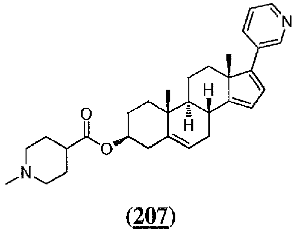

A mixture of 1-methylpiperidine-4-carboxylic acid (358 mg, 2.5 mmol) and DCC (310 mg, 1.5 mmol) in dry DMF (7 mL) was stirred at room temperature for 4 hrs. Then Compound (**79**) (273 mg, 0.625 mmol) was added and the resulting mixture was stirred at room temperature for 72 hrs. The mixture was concentrated to give the residue. The residue was diluted with DCM (7 mL) and filtered. The cake was washed with a little DCM. The filtrate was concentrated to give crude product. The crude product was purified twice by pre-TLC (DCM/MeOH =30/1, v/v) to give compound (**207**) (63 mg, 26%). LC-MS (ESI) m/z: 473 (M+1)⁺; ¹H-NMR (400 MHz, CDCl₃-*d*1) *δ* (ppm) 1.16 (s, 3H),1.21 (s, 3H), 2.27 (s, 3H), 2.80-2.83 (m, 2H), 4.63 (m, 1H), 5.51 (s, 1H), 6.01 (s, 1H), 6.81 (s, 1H), 7.22-7.25 (m, 1H), 7.29 (m, 1H), 7.75-7.78 (m, 1H), 8.40-8.42 (m, 1H), 8.76 (s, 1H).

### Example 168

### Preparation of Compound (208)

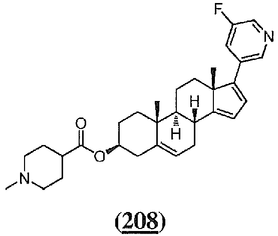

A mixture of 1-methylpiperidine-4-carboxylic acid (358 mg, 2.5 mmol) and DCC (310 mg, 1.5 mmol) in dry DMF (7 mL) was stirred at room temperature for 4 hrs. Then Compound (**79**) (273 mg, 0.625 mmol) was added. The resulting mixture was stirred at room temperature for 72 hrs. The mixture was concentrated to give the residue. The residue was diluted with DCM (7 mL) and filtered. The cake was washed with a little DCM. The filtrate was concentrated to give crude product. The crude product was purified by prep-TLC (DCM/MeOH = 30/1, v/v), then further purified by prep-HPLC to give compound (**208**) (63 mg, 26%) as a white solid. LC-MS (ESI) m/z: 473 (M+1)⁺; ¹H-NMR (400 MHz, CDCl₃-*d*1) *δ* (ppm) 1.16 (s, 3H),1.21 (s, 3H), 2.27 (s, 3H), 2.80-2.83 (m, 2H), 4.63 (m, 1H), 5.51 (s, 1H), 6.01 (s, 1H), 6.81 (s, 1H), 7.22-7.25 (m, 1H), 7.29 (m, 1H), 7.75-7.78 (m, 1H), 8.40-8.42 (m, 1H), 8.76 (s, 1H).

### Example 169

### Preparation of Compound (209)

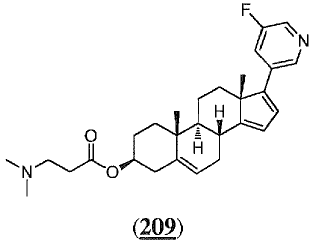

To a solution of Compound (**79**) (384 mg, 1 mmol) in DCM (20 mL) was added DIPEA (4 mL), 3-chloropropanoyl chloride (0.4 mL) added dropwise at 0 °C. The mixture was stirred at room temperature about 2 hrs. Dimethylamine hydrochloride (120 mg, 1.5 mmol) was added and the reaction was stirred at room temperature overnight. The mixture was quenched with water (100 mL) and extracted with ethyl acetate (50 mL x 3). Combined the organic layers, dried and evaporated the solvent. The residue was purified by column chromatography (petroleum ether/ethyl acetate = 1/3, v/v) to afford (**209**) (60 mg) as a white solid. LC-MS (m/z): 465 [M+H]^{+ 1}H NMR (CDCl₃, 400 MHz) major characteristic peaks: *δ* 1.12 (s, 3H), 1.14 (s, 3H), 2.28 (s, 6H), 2.49 (t, *J =* 7.2 Hz, 2H), 2.68 (t, *J=* 7.2 Hz, 2H), 4.58 (m, 1H), 5.42 (s, 1H), 5.96 (s, 1H), 6.79 (s, 1H), 7.40 (m, 1H), 8.20 (s, 1H), 8.52 (s, 1H).

### Example 170

### Preparation of Compounds (210) and (211)

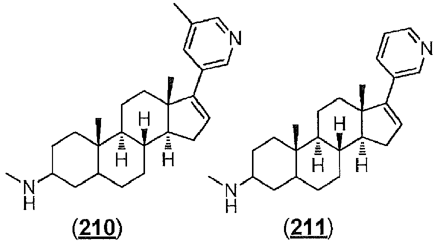

### Example 170A

### Preparation of Compound (210a)

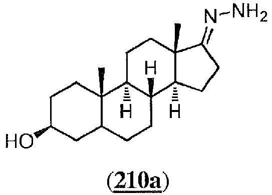

To a solution of (3S,10S)-3-hydroxy-10-methyltetradecahydro-1H-cyclopenta[a]phenanthren-17(2H)-one (10.0 g, 34.5 mmol) in ethanol (100 mL) were added hydrazine hydrate (85%, 10 mL) and TEA (0.2 mL). The mixture was stirred and heated to reflux for 2 hours. The mixture was cooled to room temperature, and evaporated in vacuum to give the Compound (**210a**) (10.0 g, 95%) as a white solid, which was used in next step without purification. LC-MS (m/z) 304 [M+H]⁺.

### Example 170B

### Preparation of Compound (210b)

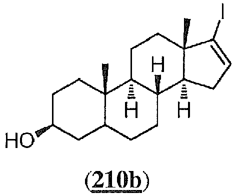

To a solution of compound (**210a**) (10.0 g, 32.9 mmol) in dichloromethane (200 mL) was added TEA (100 mL, 0.66 mol). A solution of iodine (18.4 g, 72.4 mmol) in THF (60 mL) was added dropwise into the above solution at 0 °C. Then the mixture was stirred at room temperature for 2 hours. After complete occurrence of the reaction the obtained solution was diluted with chloroform (50 mL) and successively washed with 10% aqueous hydrochloric acid solution, water, 5% aqueous sodium thiosulfate solution, water, 5 percent aqueous sodium hydrogen carbonate solution, finally with water and dried over anhydrous sodium sulfate, and then concentrated to give product compound. The crude product was purified by column chromatography (ethyl acetate in petroleum, 10% v/v) to get title compound (**210b**) (10.0 g, 76%) as a white solid. LC-MS (m/z) 383 [M-OH]⁺; ¹H-NMR (400 MHz, CHCl₃-*d*) major characteristic peaks: *δ* (ppm) 0.72 (s, 3H), 0.83 (s, 3H), 3.60 (m, 1H), 6.11 (m, 1H).

### Example 170C

### Preparation of Compound (210c)

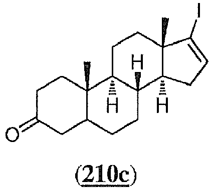

To a solution of compound (**210b**) (5.0 g, 12.5 mmol) in dichloromethane (100 mL) was added diatomite (5.0 g) and pyridinium chlorochromate (5.4 g, 25.0 mmol, 2.0 equiv). The mixture was stirred at room temperature for 2 hours. After reaction,the reaction mixture was diluted with ether and filtered. After filtration, the organic phase was concentrated under vacuum to afford the crude product. The crude product was purified by column chromatography (ethyl acetate in petroleum, 10% v/v) to get title compound (**210c**) (4.1 g, 82%) as a yellow solid. LC-MS (m/z) 399 [M+H]⁺. ¹H-NMR (400 MHz, CHCl₃-*d*) major characteristic peaks: *δ* (ppm) 0.75 (s, 3H), 1.04 (s, 3H), 6.13 (m, 1H).

### Example 170D

### Preparation of Compound (210d)

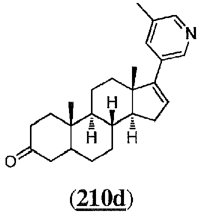

To a mixture of compound (**210c**) (1.0 g, 2.51 mmol), 5-methylpyridin-3-ylboronic acid (693 mg, 5.02 mmol), Pd(dppf)Cl₂ (184 mg, 0.25 mmol) and K₂CO₃ (1.56 g, 11.3 mmol) in 1,4-dixane (20 mL) and water (2 mL) was stirred at 80 °C for 2 hours. Filtered and the solvent was removed and the residue was dissolved in ethyl acetate (100 mL x 2), washed with water and brine, dried and concentrated to give the crude product which purified by column chromatography (ethyl acetate in petroleum, 30% v/v) to get final compound (**210d**) (128 mg, 28%) and un-reacted starting material compound (**210c**) (485 mg, 49%) were recovered. LC-MS (m/z) 364 [M+H]⁺
¹H NMR (CDCl₃, 400 MHz) major characteristic peaks: *δ* (ppm) 1.03 (s, 3H), 1.07 (s, 3H), 2.33 (s, 3H), 5.96 (m, 1H), 7.45 (s, 1H), 8.30 (s, 1H), 8.32 (s, 1H).

### Example 170E

### Preparation of Compound (210)

Compound (**210d**) (128 mg, 0.35 mmol) was dissolved in CH₃NH₂/MeOH (30%, 10 mL) and stirred at room temperature for 2 hours. Then NaBH₄ (53 mg, 1.40 mmol) was added and stirred for another 0.5 hour. After that, 20 mL of water was added and dissolved in ethyl acetate (100 mL x 2), washed with water and brine, dried and concentrated to give the crude filtered to give the crude product which purified by Prep-TLC. The final compound (**210**) (10 mg, 14%) was obtained as a yellow solid. LC-MS (m/z) 379 [M+H]⁺. ¹H NMR (CDCl₃, 400 MHz) major characteristic peaks: *δ* (ppm) 0.84 (s, 3H), 0.99 (s, 3H), 2.22 (m, 1H), 2.32 (s, 3H), 2.43 (s, 3H), 5.94 (m, 1H), 7.44 (s, 1H), 8.29 (s, 1H), 8.42 (s, 1H).

### Example 171

### Preparation of Compound (211)

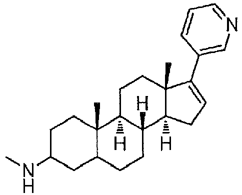

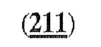

### Example 171A

### Preparation of Compound (211a)

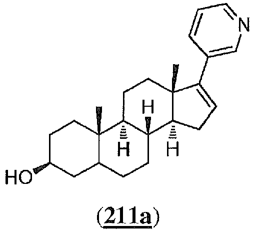

To a solution of Compound **(210c)** (2 g, 5 mmol), K₂CO₃ (3.4 g, 25 mmol) in H₂O (10 mL) and pyridin-3-ylboronic acid (922.5 mg, 7.5 mmol) in 1,4-dioxane (50 mL) was stirred at room temperature for 15 min. Then Pd(PPh₃)₄ (577 mg, 0.5 mmol) was added to the reaction mixture. Then heated to 80 °C for 30 min. The solvent was evaporated, The crude product was diluted with water (10 mL). Then extracted with DCM (50 mL x 3) and the organic layer was washed with water (20 mL x 2), brine and dried over Na₂SO₄, concentrated to afford the crude product. The crude product was purified by column chromatography (ethyl acetate in petroleum ether, 50% v/v) to afford Compound **(211a)** (600 mg, 35%) as a white solid. LC-MS: 352 [M+1] ⁺;
¹H NMR (CDCl₃, 400 MHz) major characteristic peaks: *δ* (ppm) 0.86 (s, 3 H), 1.00 (s, 3 H), 3.61 (m, 1 H), 5.98 (m, 1 H), 7.20-7.23 (m, 1 H), 7.65 (m,1 H), 8.45 (m,1H), 8.61 (m,1H).

### Example 171B

### Preparation of Compound (211b)

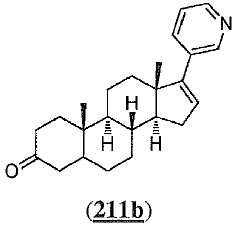

To a solution of Compound **(211a)** (0.5 g, 1.42 mmol), pyridinium chlorochromate (615 mg, 2.8 mmol) in DCM (50 mL) was stirred at 40 °C for 2 h. The reaction mixture was filtered. The filtrate was washed with water (20 mL x 2), brine. Then the organic layer was dried over Na₂SO₄, concentrated to afford the crude product. The crude product was purified by column chromatography (ethyl acetate in petroleum ether, 25% v/v) to afforded Compound **(211b)** (0. 2 g, 40%) as a yellow solid. LC-MS: 350 [M+1] ⁺.

### Example 171C

### Preparation of Compound (211)

To a solution of Compound **(211b)** (0.1 g, 0.28 mmol), NH₂CH₃ (in MeOH, 5 mL) in DCM (20 mL) was stirred at 40 °C for 0.5 h. Then NaBH₄ (120 mg, 0.56 mmol) was added to the reaction mixture. Stirred at room temperature for 0.3 h. 10 mL of acetone was added to the reaction mixture, then diluted by DCM (50 mL), washed with water (20 mL x 2), brine. Then the organic layer was dried over Na₂SO₄, concentrated to afford the crude product .The crude product was purified by column chromatography (methanol in dichloromethane, 5% v/v) to afforded the **(211)** (65 mg, 62.5%) as a white solid. LC-MS: 365 [M+1] ⁺; ¹H NMR (CDCl₃, 400 MHz) major characteristic peaks: *δ* 0.85 (s, 3 H), 1.00 (s, 3 H), 2.53 (s, 3 H), 2.63 (m, 1 H), 5.97 (m, 1 H), 7.19-7.22 (m,1 H), 7.62-7.64 (m, 1H), 8.44-8.46 (m, 1H), 8.60 (m,1H).

### Example 172

### Preparation of Compounds (212) and (213)

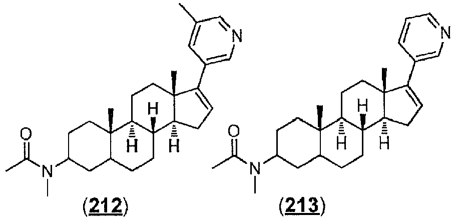

### Example 172A

### Preparation of Compound (212a)

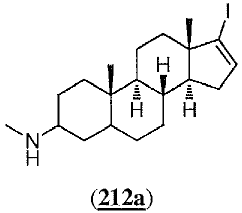

To a solution of compound **(210c)** (2.0 g, 5.0 mmol) in MeOH (30 mL) and THF (10 mL) was add a solution of CH₃NH₂/MeOH (30%, 10 mL). The mixture was stirred at room temperature for 2 hours. Then NaBH₄ (760 mg, 20 mmol) was added and stirred for another 0.5 hour. After that, water (20 mL) was added and extracted with ethyl acetate (100 mL x 3). The organic layer was washed with water and brine, dried and concentrated to give the crude product **(212a)** (1.92 g, 93%) as a white solid ,which was used in next step without purification. LC-MS (m/z): 414 [M+H]⁺. ¹H NMR (CDCl₃, 400 MHz) major characteristic peaks: *δ* (ppm) 0.72 (s, 3H), 0.81 (s, 3H), 2.42 (s, 3H), 6.12 (m, 1H).

### Example 172B

### Preparation of Compound (212b)

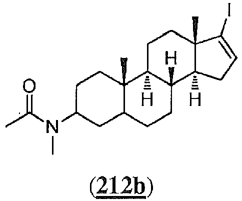

To a solution of Compound **(212a)** (2.0 g, 4.84 mmol) in DCM (60 mL) was added acetic anhydride (2 mL), Pyridine (3 mL) and DMAP (50 mg). The mixture was stirred at room temperature for 2 hrs until no starting material left. To the mixture was added water, extracted with DCM (100 mL x 3). The organic layers were combined, washed with aq.NaHCO₃, brine, and dried over Na₂SO₄. The crude was purified by column chromatography (ethyl acetate in petroleum, 30% v/v) to give Compound **(212b)** (1.7 g, 77%) as a white solid. LC-MS (m/z): 456 [M+H]⁺. ¹H-NMR (400 MHz, CHCl₃-*d*) major characteristic peaks: *δ* (ppm) 0.72 (s, 1.5H), 0.73 (s, 1.5H), 0.83 (s, 1.5H), 0.85 (s, 1.5H), 2.07 (s, 1.5H), 2.11 (s, 1.5H), 3.56 (m, 0.5H), 4.48 (m, 0.5H), 6.12 (m, 1H).

### Example 172C

### Preparation of Compounds (212) and (213)

To a mixture of compound **(212b)** (300 mg, 0.66 mmol), 5-methylpyridin-3-ylboronic acid (364 mg, 2.64 mmol), Pd(dppf)Cl₂ (52 mg, 0.07 mmol) and K₂CO₃ (819 mg, 5.93 mmol) in 1,4-dixoane (20 mL) and water (2 mL) was stirred at 80 °C for 2 hours. Filtered and the solvent was removed and the residue was dissolved in ethyl acetate (50 mL x 3), washed with water and brine, dried and concentrated to give the crude product, which was purified by column chromatography (dichloromethane in methanol, 4% v/v) to get final Compound **(212)** (66 mg, 24%) as a white solid. LC-MS (m/z): 421 [M+H]⁺. ¹H NMR (CDCl₃, 400 MHz) major characteristic peaks: *δ* (ppm) 0.86 (s, 1.5H), 0.88 (s, 1.5H), 0.99 (s, 1.5H), 1.00 (s, 1.5H), 2.08 (s, 1.5H), 2.11 (s, 1.5H), 2.32 (s, 3H), 2.83 (s, 1.5H), 2.86 (s, 1.5H), 3.57 (m, 0.5H), 4.49 (m, 0.5H), 5.95 (m, 1H), 7.45 (s, 1H), 8.29 (s, 1H), 8.42 (s, 1H).

Compound **(213)** was prepared in a similar manner to afford as a white solid (yield: 44%). LC-MS: 407.3 [M+1] ⁺; ¹H NMR (CDCl₃, 400 MHz) major characteristic peaks: *δ* (ppm) 0.86 (s, 1.5 H), 0.88 (s, 1.5 H), 1.00 (s, 1.5 H), 1.01 (s, 1.5H), 2.08 (m, 1.5 H), 2.11 (s,1.5 H), 2.83 (s,1.5H), 2.86 (s,1.5 H), 3.55 (m, 0.5H), 4.48 (m, 0.5H), 5.98 (s, 1 H), 7.23 (m, 1H), 7.66 (m, 1 H), 8.46 (m, 1 H), 8.62 (m, 1H).

### Example 173

### Preparation of Compound (214)

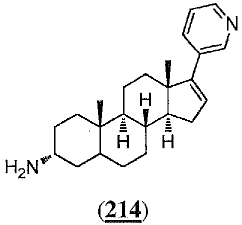

### Example 173A

### Preparation of Compound (214a)

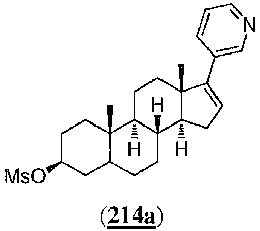

To a solution of Compound **(211a)** (1.1 g, 3.2 mmol) dissolved in Pyridine (20 mL). Then MsCl (535 mg, 4.7 mmol) was dropped to the mixture at 0 °C and stirred for 30min.Diluted with DCM (50 mL), washed by water (30 mL x 3), brine (30 mL x 3). The organic layer was dried over Na₂SO₄, concentrated to afford the crude product. The crude product was purified by column chromatography (ethyl acetate in petroleum ether, 25% v/v) to afford the Compound **(214a)** (1.2 g, 89%) as a white solid. LC-MS: 430 [M+1] ⁺; ¹H NMR (CDCl₃, 400 MHz) major characteristic peaks: *δ* (ppm) 0.89 (s, 3 H), 1.00 (s, 3 H), 3.01 (s, 3 H), 4.63 (m, 1H), 5.97 (m, 1 H), 7.22 (m,1 H), 7.64 (m,1H), 8.46 (m,1H) ,8.60 (m, 1H).

### Example 173B

### Preparation of Compound (214b)

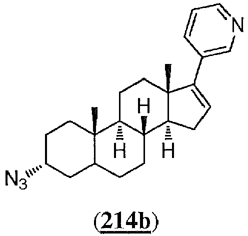

To a solution of Compound **(214a)** (1.2 g, 2.8 mmol) dissolved in DMF (20 mL). Then NaN₃ (363 mg, 5.6 mmol) was added to the mixture and stirred at 80 °C for 1 h. Then the reaction mixture was diluted with DCM (50 mL), washed by water (30 mL x 3), brine (30 mL x 3). The organic layer was dried over Na₂SO₄, concentrated to afford the desired product Compound **(214b)** (1.0 g, 99%) as a white solid. LC-MS: 377 [M+1] ⁺; ¹H NMR (CDCl₃, 400 MHz) major characteristic peaks: *δ* (ppm) 0.85 (s, 3 H), 1.00 (s, 3 H), 3.89 (m, 1 H), 6.00 (m, 1H), 7.24 (m, 1 H), 7.70 (m,1 H), 8.47 (m,1 H), 8.62 (m,1 H).

### Example 173C

### Preparation of Compound (214)

To a solution of Compound **(214b)** (1 g, 2.7 mmol) dissolved in methanol (20 mL). Then PPh₃ (3.4 g, 13.2 mmol) was added to the mixture and stirred at 50 °C overnight. Then the solvent was removed. The mixture was diluted with water (50 mL), adjusted pH2 with aq. HCL solution, extracted by DCM (30 mL x 3). The water layer was adjusted pH = 12 with NaOH solution, then extracted with DCM (30 mL x 3). The organic layer was washed wtih water (30 mL x 3), brine (30 mL x 3), dried over Na₂SO₄, and concentrated to afford a crude product. The crude product was purified by column chromatography (methanol in dichloromethane, 10% v/v) to afford compound **(214)** (0.75 g, 81%) as a white solid. LC-MS: 351 [M+1] ⁺; ¹H NMR (CDCl₃, 400 MHz) major characteristic peaks: *δ* (ppm) 0.84 (s, 3 H), 1.00 (s, 3 H), 3.20 (m, 1 H), 5.97 (m, 1 H), 7.19-7.26 (m, 1 H), 7.63-7.65 (m, 1H), 8.45 (m,1H), 8.61 (m, 1H).

### Example 174

### Preparation of Compounds (215), (216), (217), (218), (219), and (220)

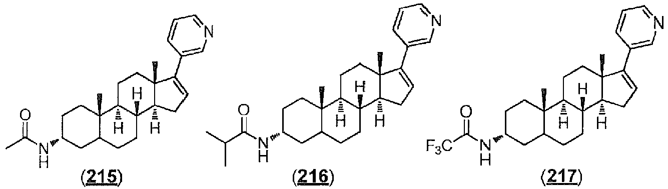

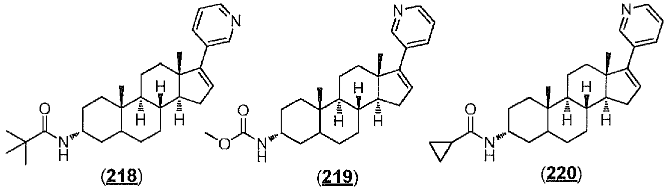

To a solution of Compound (214) (400 mg, 1.1 mmol) dissolved in Ac₂O (20 mL) was stirred at 40 °C for 1 h. Then the reaction mixture was poured into ice-water and stirred for 0.5 hr. Then it was filtered and the solid was washed with H₂O, dried to afford the desired product Compound **(215)** (150 mg, 33%) as a white solid. LC-MS: 393 [M+1] ⁺; ¹H NMR (CDCl₃, 400 MHz) major characteristic peaks: *δ* (ppm) 0.86 (s, 3 H), 1.01 (s, 3 H), 2.00 (s, 3 H), 4.14 (m, 3H), 4.76 (m, 1 H), 5.99 (m,1 H), 7.21-7.65 (m,1 H), 7.66 (m,1 H) ,8.47 (m, 1H), 8.62 (m, 1 H).

Compound **(216)** was prepared in a similar manner to afford a white solid as a white solid (yield 14%). LC-MS: 421 [M+1] ⁺; ¹H NMR (CDCl₃, 400 MHz) major characteristic peaks: *δ* (ppm) 0.87 (s, 3 H), 1.01 (s, 3 H), 1.15 (s, 3 H), 1.17 (s, 3 H), 4.13 (m, 1 H), 5.70 (s, 1 H), 5.99 (s, 1 H), 7.24 (m, 1 H), 7.64 (m,1 H), 8.45 (m,1 H), 8.62 (m,1 H).

Compound **(217)** was prepared in a similar manner to afford a white solid as a white solid (yield 13%). LC-MS: 447 [M+1] ⁺; ¹H NMR (CDCl₃, 400 MHz) major characteristic peaks: *δ* (ppm) 0.89 (s, 3 H), 1.01 (s, 3 H), 4.20 (m, 1 H), 5.99 (s, 1 H), 6.49 (s, 1 H), 7.22 (m, 1 H), 7.64 (m,1 H), 8.45 (m,1 H), 8.62 (m,1 H).

Compound **(218)** was prepared in a similar manner to afford a white solid as a white solid (yield 27%). LC-MS: 434 [M+1] ⁺; ¹H NMR (CDCl₃, 400 MHz) major characteristic peaks: *δ* (ppm) 0.87 (s, 3 H), 1.01 (s, 3 H), 1.21 (s, 9 H), 4.11 (m, 1 H), 5.91 (m, 1 H), 6.01 (s, 1 H), 7.24 (m, 1 H), 7.68 (m,1 H), 8.47 (m,1 H), 8.62 (s,1 H).

Compound **(219)** was prepared in a similar manner to afford a white solid as a white solid (yield 13%). LC-MS: (M+H)⁺ = 409. ¹H-NMR *δ* (400 MHz, CDCl₃) major characteristic peaks: *δ* (ppm) 0.86 (s, 3H), 1.01 (s, 3H), 3.66 (s, 3H), 3.90 (m, 1H), 4.96 (s, 1H), 5.99 (s, 1H), 7.22-7.24 (m, 1H), 7.67 (d, *J* = 8.0 Hz, 1H), 8.45-8.47 (m, 1H), 8.62 (d, *J* = 1.6 Hz, 1H).

Compound **(220)** was prepared in a similar manner to afford a white solid as a white solid (yield 8%). LC-MS: (M+H)⁺ = 419. ¹H-NMR *δ* (400 MHz, CDCl₃) major characteristic peaks: *δ* (ppm) 0.70-0.73 (m, 2H), 0.87 (s, 3H), 0.95-0.96 (m, 2H), 1.01 (s, 3H), 2.22-2.28 (m, 1H), 4.17 (s, 1H), 5.88 (s, 1H), 5.99 (s, 1H), 7.20-7.23 (m, 1H), 7.64 (d, *J*= 8.4 Hz, 1H), 8.46 (d, *J* = 4.8 Hz, 1H), 8.62 (s, 1H).

### Example 175

### Preparation of Compounds (221) and (222)

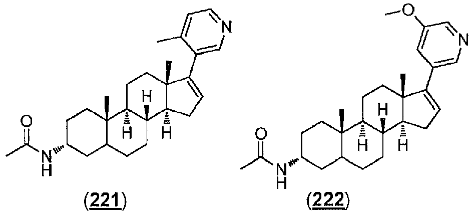

Following the general procedures described in the Example 173 and Example 174, Compounds **(221)** and **(222)** were prepared in a similar manner.

Compound **(221)** a white solid: LC-MS: m/z 407 (M+1). ¹H NMR (CDC13, 400 MHz) peaks: *δ* (ppm) 0.85 (s, 3H), 0.90 (s, 3H), 1.99 (s, 3 H), 2.28 (s, 3 H), 4.13 (m, 1H), 5.66 (m, 1H), 5.80 (d, J = 6.8 Hz, 1 H), 7.11(d, J = 5.2 Hz, 1H), 8.28 (s, 1H), 8.32 (d, J = 5.2 Hz, 1H).

Compound **(222)** a white solid: LC-MS: m/e 423 [M+1] ⁺; ¹H NMR (CDCl₃, 400 MHz) major characteristic peaks: *δ* (ppm) 0.86 (s, 3 H), 1.00 (s, 3 H), 2.00 (s, 3 H), 3.87 (s, 3H), 4.14 (m, 1 H), 5.72 (m,1 H), 6.00 (s,1 H), 7.17 (m, 1H), 8.17 (m, 1H), 8.25 (s, 1 H).

### Example 176

### Preparation of Compounds (223), (224), (225), and (226)

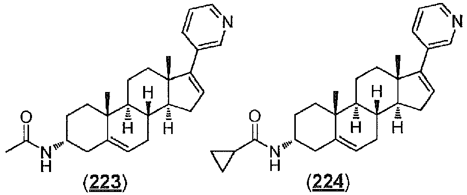

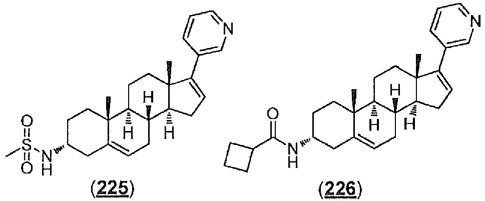

### Example 176A

### Preparation of Compound (223a)

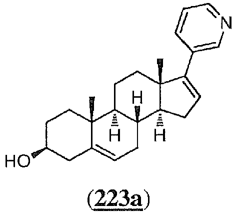

To a solution of (3S,10R,13S)-17-iodo-10,13-dimethyl-2,3,4,7,8,9,10,11,12,13,14,15-dodecahydro-1H-cyclopenta[a]phenanthren-3-ol (3.0 g, 7.54 mmol) in 1,4-dioxane (830 mL) was added pyridin-3-ylboronic acid (1.39 g, 11.3 mmol), potassium carbonate (4.68 g, 34 mmol) in water (5 mL), and bis(triphenylphosphine) palladium chloride (552 mg). The mixture was thoroughly degassed, and heated under nitrogen at 80 °C for 2 hour. After being filtered through a pad of Celite, the crude product was purified with silica gel column (ethyl acetate in petroleum, 50% v/v) to afford Compound **(223a)** (1.6 g, 61%) as a yellow solid. LC-MS (m/z): 350 [M+H]^{+ 1}H NMR (CDCl₃, 400 MHz) major characteristic peaks: *δ* (ppm) 1.05 (s, 3H), 1.07 (s, 3H), 3.55 (m, 1H), 5.40 (m, 1H), 6.01 (s, 1H), 7.24 (m, 1H), 7.66 (d, J = 8.0 Hz, 1H), 8.45 (d, J = 4.0 Hz, 1H), 8.62 (s, 1H).

### Example 176B

### Preparation of Compound (223b)

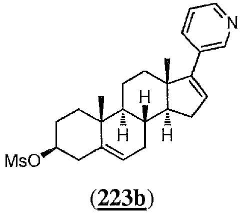

To a solution of Compound **(223a)** (1.6 g, 4.58 mmol) in pyridine (15 mL) was added methanesulfonyl chloride (1 mL, excess). The mixture was stirred at room temperature for 2 hour. After cooling to room temperature, the reaction mixture was poured into ice-water (200 mL) and extracted with ethyl acetate (100 mL x 3). The organic layers were combined, washed with aq.NaHCO₃, brine, and dried over Na₂SO₄. The crude was purified by column chromatography (ethyl acetate in petroleum, 50% v/v) to give Compound **(223b)** (470 mg, 24%) as a yellow solid
LC-MS (m/z): 428 [M+H]⁺. ¹H NMR (CDCl₃, 400 MHz) major characteristic peaks: *δ* (ppm) 1.05 (s, 3 H), 1.09 (s, 3 H), 3.02 (s, 3 H), 4.54 (m, 1 H), 5.46 (m, 1 H), 6.00 (s, 1 H), 7.23 (m, 1 H), 7.65 (d, J = 7.6 Hz, 1 H), 8.46 (d, *J* = 4.4 Hz, 1 H), 8.63 (s, 1 H).

### Example 176C

### Preparation of Compound (223c)

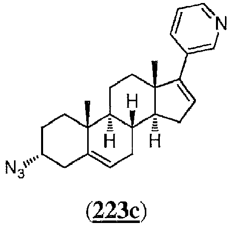

To a solution of Compound **(223b)** (420 mg, 1.0 mmol) in DMF (10 mL) was added sodium azide (195 mg, 3.0 mmol) carefully. The mixture was stirred and heated to 80 °C for 3 hour. After cooling to r.t., the reaction mixture was poured into ice-water (200 mL) and extracted with DCM (100 mL x 3). The organic layers were combined, washed with aq.NaHCO₃, brine, and dried over Na₂SO₄. The crude was purified by column chromatography (ethyl acetate in petroleum, 20% v/v) to give Compound **(223c)** (291 mg, 79%) as a yellow solid. LC-MS (m/z): 375 [M+H]⁺. ¹H NMR (CDCl₃, 400 MHz) major characteristic peaks: *δ* (ppm) 1.05 (s, 3 H), 1.07 (s, 3 H), 3.90 (m, 1 H), 5.44 (m, 1 H), 6.00 (s, 1 H), 7.22 (m, 1 H), 7.65 (d, J = 7.6 Hz, 1 H), 8.45 (d, J = 4.4 Hz, 1 H), 8.62 (s, 1 H).

### Example 176D

### Preparation of Compound (223d)

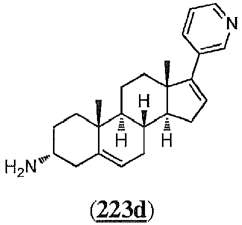

To a solution of Compound **(213c)** (282 mg, 0.754 mmol) in methanol (5 mL) and THF (3 mL) was added PPh₃ (296 mg, 1.13 mmol). The mixture was stirred and heated to 50 °C overnight. The reaction mixture was cooled to r.t. and the pH was adjusted to 1 with 2 N aqueous HCl solution, extracted with DCM (50 mL). The PH of the aqueous solution was adjusted to 9 with sat. NaHCO₃ and extracted with DCM (50 mL x 2). The organic layers were combined, washed with water, brine, and dried over Na₂SO₄. The crude product **(223d)** (210 mg, 80%) was used for next step without further purification. LC-MS (m/z): 349 [M+H]⁺.

### Example 176E

### Preparation of Compounds (223), (224), (225), and (226)

To a solution of Compound **(223d)** (150 mg, 0.43 mmol) in DCM (8 mL) was added acetyl chloride (0.5 mL, excess). The mixture was stirred at r.t. for 1 hour. After reaction, the reaction mixture was quenched with water and extracted with DCM (50 mL x 3). The organic layers were combined, washed with water, brine, and dried over Na₂SO₄. The crude was purified by pre-TLC (methanol in dichloromethane, 20% v/v) to give Compound **(223)** (72 mg, 43%) as a white solid. LC-MS (m/z): 391 [M+H]⁺. ¹H NMR (CDCl₃, 400 MHz) major characteristic peaks: *δ* 1.05 (s, 3 H), 1.09 (s, 3 H), 1.97 (s, 3 H), 2.28 (m, 1 H), 2.62 (m, 1 H), 4.16 (m, 1 H), 5.43 (d, *J* = 4.8 Hz, 1 H), 5.52 (d, *J* = 5.6 Hz, 1 H), 6.01 (m, 1 H), 7.23 (m, 1 H), 7.66 (d, *J* = 7.6 Hz, 1 H), 8.46 (d, J = 4.8 Hz, 1 H), 8.63 (s, 1 H).

Using Compound **(223d)** and corresponding acid chlorides, Compounds **(224), (225),** and **(226)** were prepared in a similar manner.

Compound **(224)** was obtained as a white solid (yield 17%). LC-MS (m/z): 417 [M+H]⁺. ¹H NMR (CDCl₃, 400 MHz) major characteristic peaks: *δ* (ppm) 0.70 (m, 2H), 0.94 (m, 2H), 1.06 (s, 3 H), 1.09 (s, 3 H), 2.28 (m, 1 H), 2.62 (m, 1 H), 4.19 (m, 1 H), 5.46 (d, *J&* = 5.6 Hz, 1 H), 5.71 (d, *J* = 6.8 Hz, 1 H), 6.01 (m, 1 H), 7.23 (m, 1 H), 7.65 (d, *J* = 8.0 Hz, 1 H), 8.47 (d, *J* = 4.8 Hz, 1 H), 8.63 (s, 1 H).

Compound (225) was obtained as a white solid (yield 6%). LC-MS (m/z): 427 [M+H]⁺; ¹H NMR (CDCl₃, 400 MHz) major characteristic peaks: *δ* (ppm) 1.05 (s, 3 H), 1.08 (s, 3 H), 2.66 (m, 1 H), 2.97 (s, 3 H), 3.79 (m, 1 H), 4.49 (d, *J* = 8.0 Hz, 1 H), 5.45 (m, 1 H), 6.00 (m, 1 H), 7.23 (m, 1 H), 7.66 (d, *J* = 8.0 Hz, 1 H), 8.47 (d, *J* = 4.0 Hz, 1 H), 8.63 (s, 1 H).

Compound **(226)** was obtained as a white solid (yield 15%). LC-MS (m/z): 431 [M+H]⁺; ¹H NMR (CDCl₃, 400 MHz) major characteristic peaks: *δ* (ppm) 1.05 (s, 3 H), 1.09 (s, 3 H), 2.60 (m, 1 H), 2.97 (m, 1 H), 4.15 (m, 1 H), 5.42 (m, 2 H), 6.01 (m, 1 H), 7.23 (m, 1 H), 7.66 (d, *J* = 8.0 Hz, 1 H), 8.47 (d, *J* = 4.8 Hz, 1 H), 8.63 (s, 1 H).

### Example 177

### Preparation of Compound (227)

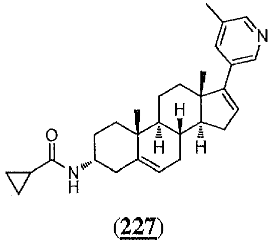

### Example 177A

### Preparation of Compound (227a)

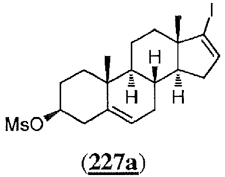

A solution of (3S,10R,13S)-17-iodo-10,13-dimethyl-2,3,4,7,8,9,10,11,12,13,14,15-dodecahydro-1H-cyclopenta[a]phenanthren-3-ol (1.00 g, 2.512 mmol) and MsCl (578 mg, 5.025 mmol) in pyridine (5 mL) was stirred at 25 °C for 2 h. Then it was diluted with ethyl acetate (200 mL) and washed with water and brine, dried with anhydrous Na₂SO₄ and evaporated and purified by silica chromatography (ethyl acetate/ petroleum ether = 1/8, v/v) to obtain compound **(227a)** (720 mg, 60%) as a white solid. LC-MS: (m/z) 477 [M+1] ⁺; ¹H NMR (CDCl₃, 400 MHz) major characteristic peaks: *δ* 0.75 (s, 3H), 1.06 (s, 3H), 3.01 (s, 3H), 4.17-4.57 (m, 1H), 5.43 (m, 1H), 6.14(m, 1H).

### Example 177B

### Preparation of Compound (227b)

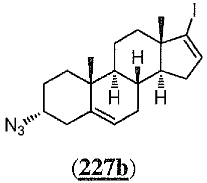

A suspension of Compound **(227a)** (700 mg, 1.470 mmol) and NaN₃ (287 mg, 4.412 mmol) in DMF (5 mL) was stirred at 85 °C for overnight. Then it was diluted with ethyl acetate (200 mL) and washed with water and brine, dried with anhydrous Na₂SO₄ and evaporated, purified by silica chromatography (ethyl acetate/ petroleum ether = 1/50, v/v) to obtain compound **(227b)** (410 mg, 66%) as a white solid. LC-MS: (m/z) 424 [M+1] ⁺; ¹H NMR (CDCl₃, 400 MHz) major characteristic peaks: *δ* (ppm) 0.75 (s, 3H), 1.04 (s, 3H), 3.87-3.90 (m, 1H), 5.40 (m, 1H), 6.14 (m, 1H).

### Example 177C

### Preparation of Compound (227c)

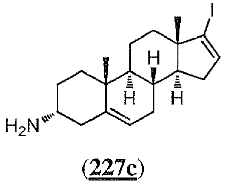

A solution of Compound **(227b)** (400 mg, 0.946 mmol) and PPh₃ (991 mg, 3.782 mmol) in THF/MeOH/H₂O (8/8/2 mL) was stirred at 50 °C for overnight. Then it was diluted with ethyl acetate (150 mL) and adjusted to pH 2 with diluted hydrochloric acid and extracted with pH2 diluted hydrochloric acid for three times. The combined aqueous was adjusted to pH 9 with 2M NaOH solution and extracted with DCM (200 mL). The DCM phase was dried with anhydrous Na₂SO₄ and evaporated to obtain compound **(227c)** (340 mg, 90%) as a white solid. LCMS: 398 [M+1] ⁺; ¹H NMR (CDCl₃, 400 MHz) major characteristic peaks: *δ* (ppm) 0.75 (s, 3H), 1.04 (s, 3H), 3.24 (s, 1H), 5.38 (s, 1H), 6.14 (s, 1H).

### Example 177D

### Preparation of Compound (227d)

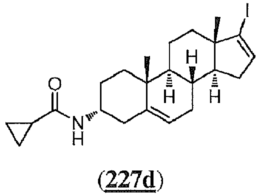

A solution of compound **(227c)** (340 mg, 0.856 mmol), cyclopropanecarboxylic acid (110 mg, 1.284 mmol), EDCI (329 mg, 1.712 mmol), HoBt (231 mg, 1.712 mmol) and Et3N (316 mg, 3.424 mmol) in DCM (10 mL) was stirred at 25 °C for overnight. Then it was diluted with ethyl acetate (150 mL), washed with water and brine, dried with anhydrous Na₂SO₄ and purified by silica chromatography (ethyl acetate/ petroleum ether = 1/4, v/v) to obtain compound **(227d)** (220 mg, 55%) as a white solid. LCMS: 466 [M+1] ⁺; ¹H NMR (CDCl₃, 400 MHz) major characteristic peaks: *δ* (ppm) 0.71 (m, 2H), 0.76 (s, 3H), 0.93 (m, 2H), 1.06 (s, 3H), 2.60 (m, 1H), 4.17 (m, 1H), 5.41 (m, 1H), 5.66 (m, 1H), 6.15 (m, 1H).

### Example 177E

### Preparation of Compound (227)

The suspension of compound **(227d)** (220 mg, 0.473 mmol), 5-methylpyridin-3-ylboronic acid (130 mg, 0.946 mmol), PdCl2(PPh3)2 (33 mg, 0.047 mmol) and K2CO3 (261 mg, 1.892 mmol) in dioxane/H2O (10/1 mL) was stirred at 80 °C for overnight. Then it was diluted with ethyl acetate (150 mL), washed with water and brine, dried with anhydrous Na₂SO₄ and purified by Pre-HPLC to obtain compound **(227)** (130 mg, 64%) as a white solid. LC-MS: 431 [M+1] ⁺; ¹H NMR (CDCl₃, 400 MHz) major characteristic peaks: *δ* (ppm) 0.69-0.71 (m, 2H), 0.93-.095 (m, 2H), 1.05 (s, 3H), 1.09 (s, 3H), 2.33 (s, 3H), 4.18 (m, 1H), 5.46 (m, 1H), 5.70 (m, 1H), 5.99 (m, 1H), 7.47 (s, 1H), 8.30 (s, 1H), 8.43 (s, 1H).

### Example 178

### Preparation of Compound (228)

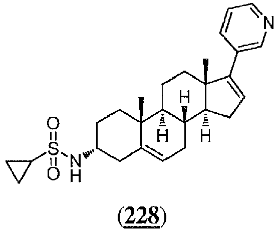

### Example 178A

### Preparation of Compound (228a)

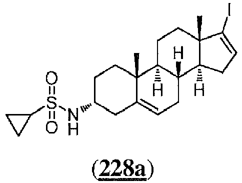

To a solution of Compound **(227b)** (300 mg, 0.76 mmol) in THF (10 mL) was added Et₃N (115 mg, 1.13 mmol) and cyclopropanesulfonyl chloride (106.2 mg, 0.76 mmol). The mixture was stirred at 50 °C for 1h. Then the mixture was cooled to room temperature, added water, extracted with ethyl acetate (2 x 50 mL), washed with brine(1 x 50 mL), dried over Na₂SO₄, concentrated, purified by gel chromatography (petroleum ether/ethyl acetate/DCM = 5/1/1, v/v/v) to give the product Compound **(228a)** (250 mg , 66%) as a white solid. LC-MS: (m/z) 502 (M+1); ¹H NMR (CDCl₃, 400 MHz) peaks: *δ* (ppm) 0.74 (s, 3H), 1.04 (s, 3H), 2.18 (m, 1H), 2.39 (m, 1H), 2.64 (m, 1H), 3.27 (m, 2H), 3.78 (m, 1H), 4.39 (d, *J* = 8.8 Hz, 1H), 5.42 (m, 1H), 6.14 (m, 1H).

### Example 178B

### Preparation of Compound (228)

To a solution of Compound **(228a)** (250 mg, 0.5 mmol) in dioxane (30 mL) was added pyridin-3-yl-boronic acid (122 mg, 0.99 mmol), potassium carbonate (344 mg, 2.5 mmol) in water (3 mL), and bis(triphenylphosphine) palladium chloride (35 mg). The mixture was thoroughly degassed, and heated under nitrogen at 80 °C for 2 hours. After being filtered through a pad of Celite, the crude product was purified by pre-HPLC to give the title Compound **(228)** (45 mg, 20 %) as a white solid. LC-MS: (m/z) 453 (M+1); ¹H NMR (CDCl₃, 400 MHz) peaks: *δ* (ppm) 1.01 (m, 2H), 1.01 (s, 3H), 1.05 (s, 3 H), 1.34 (m, 2H), 2.28 (m, 1H), 2.41 (m,1H), 2.66 (m, 1H), 3.79 (m, 1H), 4.44 (d, *J* = 8.4 Hz, 1H), 5.45 (d, *J* = 5.2 Hz, 1H), 6.01 (m, 1H), 7.23 (m, 1H), 7.66 (m, 1H), 8.47 (d, *J* = 3.6 Hz, 1H), 8.63 (s, 1H).

### Example 179

### Preparation of Compound (229)

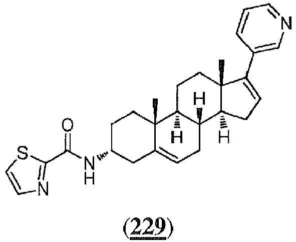

### Example 179A

### Preparation of Compound (229a)

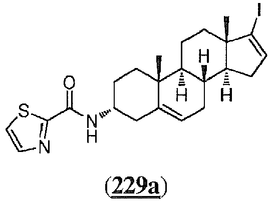

To a solution of Compound **(227b)** (0.3 g, 0.75 mmol), HOBt (204 mg, 1.5 mmol), EDCI.HCl (290 mg, 1.5 mmol), Et₃N (229 mg,2.3 mmol) in DCM (50 mL) was stirred at room temperature for overnight. Then the reaction mixture was diluted with water (20 mL). Then extracted with DCM (50 mL x 3) and the organic layer was washed with water (20 mL x 2), brine and dried over Na₂SO₄, concentrated to afford the crude product. The crude product was purified by column chromatography (ethyl acetate in petroleum ether, 5% v/v) to afford the Compound (**229a**) (240 mg, 63%) as a white solid. LC-MS: (m/z) 509 [M+1] ⁺; ¹H NMR (CDCl₃, 400 MHz) major characteristic peaks: *δ* (ppm) 0.70 (s, 3 H), 1.03 (s, 3 H), 2.64 (m,1 H), 4.26 (m, 1 H), 5.42 (m, 1 H), 6.08 (m, 1 H), 7.47 (d, *J* = 2.8 Hz, 1 H), 7.77 (d, *J* = 2.8 Hz, 1 H).

### Example 179B

### Preparation of Compound (229)

A mixture of Compound (**229a**) (240 mg, 0.47 mmol), pyridin-3-ylboronic acid (87 mg, 0.71 mmol), K₂CO₃ (197 mg, 1.4 mmol) and Pd(dppf)Cl₂ (34 mg, 0.047 mmol) in 10 mL dioxane and 2 mL H₂O was stirred at 80 °C for 2 h under N₂ protection. Then was cooled down, diluted by water, and extracted with ethyl acetate (100 mL) 3 times, the combined organic phase was washed by water, brine and dried over Na₂SO₄. The crude product was purified by pre-TLC (petroleum ether/ethyl acetate = 1/3, v/v) to afford the title compound **(229)** (48 mg, 22 %) as a white solid. LC-MS: (m/z) 460 [M+1] ⁺; ¹H NMR (CDCl₃, 400 MHz) major characteristic peaks: *δ* (ppm) 1.06 (s, 3 H), 1.13 (s, 3 H), 2.73 (m, 1 H), 4.33 (m, 1 H), 5.54 (m, 1 H), 6.02 (s, 1 H), 7.26 (m, 1 H), 7.39 (d, *J*= 8.0 Hz, 1 H), 7.53 (d, *J* = 3.2 Hz, 1 H), 7.70 (d, *J*= 8.0 Hz, 1 H), 7.84 (d, *J* = 3.2 Hz, 1H), 8.47 (d, *J* = 4.0 Hz, 1 H), 8.63 (s, 1 H).

### Example 180

### Preparation of Compound (230)

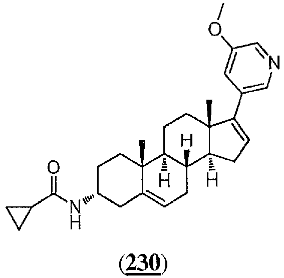

A mixture of Compound (**227d**) (260 mg, 0.56 mmol), 5-methoxypyridin-3-ylboronic acid (171 mg, 1.2 mmol), K₂CO₃ (386 mg, 2.8 mmol) and Pd(dppf)Cl₂ (41 mg, 0.056 mmol) in dioxane (50 mL) and H₂O (2 mL) was stirred at 100 °C for 2 hrs under N₂ protection. The mixture was cooled down, diluted with water, and extracted with ethyl acetate (100 mL) 3 times, the combined organic phase was washed with water, brine and dried over Na₂SO₄. The crude product was purified by silica column chromatography (petroleum ether/ethyl acetate= 1/3, v/v) and further purified by prep-HPLC to afford the title Compound (**230**) (52 mg, 21%) as a white solid. LC-MS: (m/z) 447 [M+1] ⁺; ¹H NMR (CDCl₃, 400 MHz) major characteristic peaks: *δ* (ppm) 0.69-0.70 (m, 2H), 0.71-0.72 (m, 2H), 0.94 (s, 3H), 1.05 (s, 3 H), 1.09 (s, 1 H), 2.26-2.30 (m, 1H), 2.60-2.64 (d, *J* = 16 Hz, 1H), 3.87 (s, 3H), 4.18 (m, 1H), 5.46 (m, 1H), 5.69-5.71 (m, 1H), 6.02 (s, 1H), 7.17 (s, 1H), 8.24 (brs, 2H).

### Example 181

### Preparation of Compound (231)

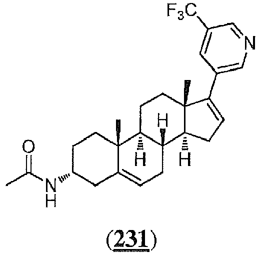

### Example 181A

### Preparation of Compound (231a)

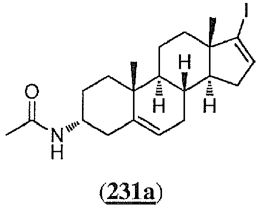

A mixture of Compound **(227b)** (300 mg) and Ac₂O (6 mL) in THF (10 mL) was stirred at 50 °C overnight. Then the mixture was cooled to room temperature and diluted with ethyl acetate (200 mL). The organic layer was washed with water, brine, dried over Na₂SO₄, concentrated and purified by flash column chromatography (petroleum ether/ethyl acetate = 5/1, v/v) to give the title Compound **(231a)** (260 mg, 78 %) as a white solid. ¹H NMR (CDCl₃, 400 MHz) major characteristic peaks: *δ* (ppm) 0.76 (s, 3H), 1.06 (s, 3H), 1.96 (s, 3H), 4.15 (m, 1 H), 5.40 (m, 1H), 5.52 (m, 1H), 6.15 (m, 1H).

### Example 181B

### Preparation of Compound (231)

To a solution of Compound **(231a)** (260 mg, 0.6 mmol) in dioxane (10 mL) was added (5-(trifluoromethyl)pyridin-3-yl)boronic acid (400 mg, 2.3 mmol), potassium carbonate (407 mg, 2.9 mmol) in water (5 mL), and bis(triphenylphosphine) palladium chloride (43 mg, 0.1 mmol). The mixture was thoroughly degassed, and heated under nitrogen at 80 °C for 1 hour. After being filtered through a pad of Celite, concentrated and purified by pre-HPLC, it gave Compound **(231)** (33 mg, 12%) as a white solid. LC-MS: (m/z) 458.6[M+1]; ¹H NMR (CDCl₃, 400 MHz) peaks: *δ* (ppm) 1.07 (s, 3H), 1.09 (s, 3H), 1.98 (s, 3H), 4.16 (m, 1H), 5.43 (m, 1H), 5.53 (m, 1H), 6.13 (m, 1H), 7.26(s, 1H), 7.87 (s, 2H).

### Example 182

### Preparation of Compound (232)

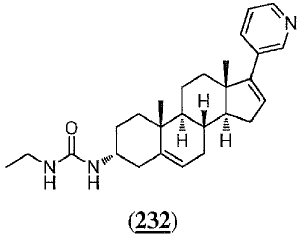

### Example 182A

### Preparation of Compound (232a)

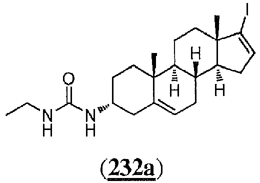

A mixture of Compound **(227b)** (300 mg, 0.76 mmol) and isocyanatoethane (59 mg, 0.83 mmol) in THF (20 mL) was stirred at room temperature for 2 hrs. Then the mixture was evaporated to remove the solvent, purified by column chromatography (petroleum ether/ethyl acetate /DCM = 5/1/1, v/v/v) to give the product **(232a)** (300 mg, 85%) as a yellow solid. LC-MS: (m/z) 469 (M+1); ¹H NMR (CDC13, 400 MHz) peaks: *δ* (ppm) 0.76 (s, 3H), 1.05 (s, 3H), 1.13 (t, *J* = 7.6 Hz, 3H), 3.19 (q, *J* = 7.6 Hz, 2H), 3.94 (s, 1H), 4.32 (s, 2H), 5.37 (d, *J* = 5.2 Hz, 1H), 6.14 (s, 1H).

### Example 182B

### Preparation of Compound (232)

To a solution of Compound **(232a)** (300 mg, 0.64 mmol) in dioxane (50 mL) was added pyridin-4-ylboronic acid (157 mg, 1.28 mmol), potassium carbonate (442 mg, 3.2 mmol) in water (5 mL), and bis(triphenylphosphine) palladium chloride (45 mg). The mixture was thoroughly degassed, and heated under nitrogen at 80 °C for 2 hrs. After being filtered through a pad of Celite, the crude product was purified by prep-HPLC to give the title Compound (**232**) (96 mg, 36%) as a white solid. LC-MS: (m/z) 420 (M+1); ¹H NMR (CDC13, 400 MHz) peaks: *δ* (ppm) 1.04 (s, 3H), 1.07 (s, 3H), 1.13 (t, *J* = 7.2 Hz, 3H), 3.2 (m, 2H), 3.96 (s, 1H), 4.44 (t, *J*= 5.2 Hz, 1H), 4.54 (d, *J* = 8.0 Hz, 1H), 5.36 (d, *J* = 5.2 Hz, 1H), 6.01 (d, *J* = 1.2 Hz, 1H), 7.24 (m, 1H), 7.66 (d, *J*= 8.0 Hz, 1H), 8.47 (s, 1H), 8.63 (s, 1H).

### Example 183

### Preparation of Compound (233)

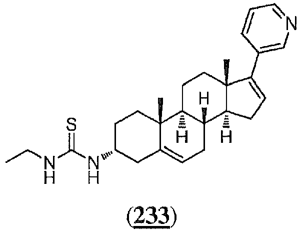

### Example 183A

### Preparation of Compound (233a)

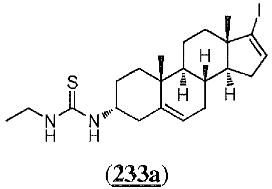

A mixture of Compound **(227b)** (300 mg, 0.76 mmol) and isothiocyanatoethane (72.4 mg, 0.83 mmol) in THF (20 mL) was stirred at room temperature for 2 hrs. Then the mixture was evaporated to remove the solvent and purified by column chromatography (petroleum ether/ethyl acetate/DCM = 5/1/1, v/v) to give the product Compound **(233a)** (174 mg, 48%) as a yellow solid. LC-MS: (m/z) 485 (M+1); ¹H NMR (CDCl₃, 400 MHz) peaks: *δ* (ppm) 0.76 (s, 3H), 1.08 (s, 3H), 1.24 (t, *J* = 7.6 Hz, 3H), 2.65 (m, 1H), 3.35 (s, 2H), 4.33 (s, 2H), 5.45 (d, *J* = 5.2 Hz, 1H), 5.72 (m, 1H), 6.14 (m, 1H).

### Example 183B

### Preparation of Compound (233)

To a solution of Compound **(233a)** (174 mg, 0.36 mmol) in dioxane (20 mL) was added pyridin-4-ylboronic acid (88.4 mg, 0.72 mmol), potassium carbonate (248 mg, 1.79 mmol) in water (2 mL), and bis(triphenylphosphine) palladium chloride (25 mg). The mixture was thoroughly degassed, and heated under nitrogen at 80 °C for 12 hrs. After being filtered through a pad of Celite, the crude product was purified by prep-HPLC to give the title Compound **(233)** (19 mg, 12%) as a white solid. LC-MS: (m/z) 436 (M+1); ¹H NMR (CDCl₃, 400 MHz) peaks: *δ* (ppm) 1.03 (s, 3H), 1.09 (s, 3H), 1.22 (t, *J* = 7.2 Hz, 3H), 3.4 (s, 2H), 4.39 (s, 1H), 5.41 (d, *J* = 4.4 Hz, 1H), 5.93 (s, 1H), 6.01 (m,1H), 6.10 (s, 1H), 7.24 (m, 1H), 7.66 (m, 1H), 8.45 (m, 1H), 8.62 (d, *J* = 1.6 Hz, 1H).

### Example 184

### Preparation of Compound (234)

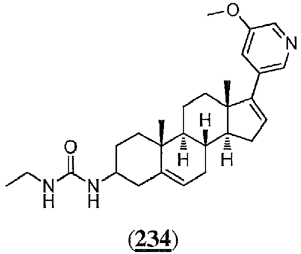

To a solution of Compound **(232a)** (300 mg, 0.64 mmol) in dioxane (50 mL) was added 5-methoxypyridin-3-ylboronic acid (196 mg, 1.28 mmol), potassium carbonate (442 mg, 3.2 mmol) in water (5 mL), and bis(triphenylphosphine) palladium chloride (45 mg). The mixture was thoroughly degassed, and heated under nitrogen at 80 °C for 2 hours. After being filtered through a pad of Celite, the crude product was purified by prep-HPLC to give the title compound (**234**) (45 mg, 16%) as a white solid. LC-MS: (m/z) 450 (M+1); ¹H NMR (CDC13, 400 MHz) peaks: *δ* (ppm) 0.95 (s, 3H), 0.99 (s, 3H), 1.05 (t, *J*= 7.2 Hz, 3H), 3.78 (m, 3H), 3.90 (s, 1H), 4.56 (m, 2H), 5.28 (s, 1H), 5.95 (s, 1H), 7.10 (m, 1H), 8.10 (d, *J* = 2.8 Hz, 1H), 8.17 (d, *J* = 4.8 Hz, 1H).

### Example 185

### Preparation of Compound (235)

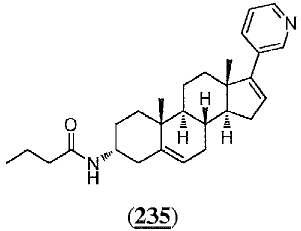

### Example 185A

### Preparation of Compound (235a)

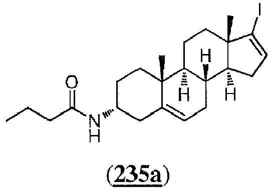

To a mixture of Compound **(227b)** (300 mg, 0.76 mmol) in THF (20 mL) and Et₃N (115 mg, 1.13 mmol) was added butyryl chloride (97 mg, 0.91 mmol) dropwised at 0°C, then the mixture was stirred at room temperature for 30 min, then added water, extracted with ethyl acetate (2 x 20 mL), washed with brine (50 mL), dried over Na₂SO₄. The crude product was purified by prep-TLC (petroleum ether/ethyl acetate=1/1, v/v) to give the Compound **(235a)** (300 mg, 85%) as a colorless liquid. LC-MS: (m/z) 468 (M+1); ¹H NMR (CDCl₃, 400 MHz) characteristic peaks: *δ* (ppm) 0.76 (s, 3H), 0.94 (t, *J* = 7.2 Hz, 3H), 1.06 (s, 3H), 2.13 (t, *J* = 8.0 Hz, 2H), 2.58 (m, 1H), 4.16 (m, 1H), 5.39 (d, *J* = 5.2 Hz, 1H), 5.49 (d, *J* = 5.2 Hz, 1H), 6.14 (m, 1H).

### Example 185B

### Preparation of Compound (235)

To a solution of Compound **(235a)** (300 mg, 0.64 mmol) in dioxane (20 mL) was added pyridin-3-ylboronic acid (158 mg, 1.3 mmol), potassium carbonate (443 mg, 3.2 mmol) in water (5 mL), and bis(triphenylphosphine) palladium chloride (45 mg). The mixture was thoroughly degassed, and heated under nitrogen at 80 °C for 2 hrs. After being filtered through a pad of Celite, the crude product was purified by prep-HPLC to give the title compound **(235)** (100 mg, 37%) as a white solid. LC-MS: (m/z) 419 (M+1); ¹H NMR (CDC13, 400 MHz) characteristic peaks: *δ* (ppm) 0.87 (t, *J* = 7.2 Hz, 3H), 0.98 (s, 3H), 1.01 (s, 3H), 2.07 (t, *J* = 7.8 Hz, 2H), 2.21 (m, 1H), 2.54 (m, 1H), 4.10 (m, 1H), 5.36 (d, *J*= 5.2 Hz, 1H), 5.46 (d, *J*= 7.6 Hz, 1H), 5.94 (s, 1H), 7.15 (m, 1H), 7.69 (m, 1H), 8.40 (d, *J* = 4.4 Hz, 1H), 8.56 (s, 1H).

### Example 186

### Preparation of Compound (236)

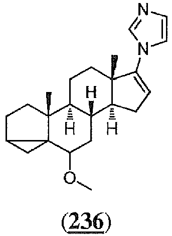

### Example 186A

### Preparation of Compound (236a)

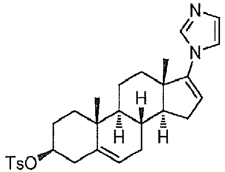

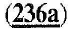

To a solution of Compound (**49c**) (300 mg, 0.89 mmol) in pyridine (10 mL), toluene sulfonyl chloride (510 mg, 2.67 mmol) was added with cat. DMAP under N₂. After stirring at 60 °C for 2 h, the reaction mixture was poured into ice water. The mixture was extracted with DCM (50 mL x 3), washed with water, brine and dried over Na₂SO₄. Filtrate and concentrated to give the crude product Compound **(236a)** (300 mg, 69%), which was used in next step without further purification. LC-MS (m/z): 493 [M+H]⁺; ¹H NMR (CDCl₃, 400 MHz) major characteristic peaks: *δ* (ppm) 0.98 (s, 3H), 1.02 (s, 3H), 2.45 (s, 3H), 4.34 (m, 1H), 5.34 (m, 1H), 5.68 (s, 1H), 7.04 (s, 1H), 7.09 (s, 1H), 7.34 (d, *J =* 8.0 Hz, 2H), 7.63(s, 1H), 7.80 (d, *J*= 8.0 Hz, 2H).

### Example 186B

### Preparation of Compound (236)

Compound **(236a)** (300 mg, 0.61 mmol) was dissolved in methanol (10 mL) and stirred at reflux for 2 hrs. After evaporated of the solvent, the resulted residue was dissolved in ethyl acetate (100 mL) and washed with saturated NaHCO₃ (100 mL), brine (100 mL), dried over anhydrous Na₂SO₄. After the removal of the solvent in vacuum, the residues were purified by prep-HPLC to give title Compound (**236**) (10 mg, 5%) as a white solid. LC-MS (m/z): 353 [M+H]⁺. ¹H NMR (CDCl₃, 400 MHz) major characteristic peaks: *δ* (ppm) 0.41 (m, 1H), 0.62 (m, 1H), 0.96 (s, 3H), 1.00 (s, 3H), 2.77 (m, 1H), 3.30 (s, 3H), 5.60 (m, 1H), 7.00 (s, 1H), 7.02 (s, 1H), 7.57 (s, 1H).

### Example 187

### Preparation of Compounds (237), (238), (239), (240), (241), and (242)

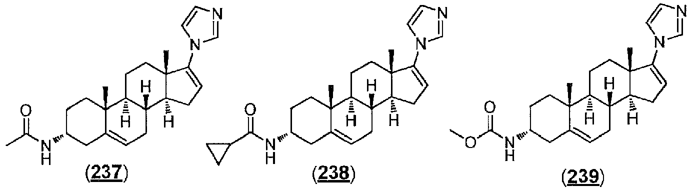

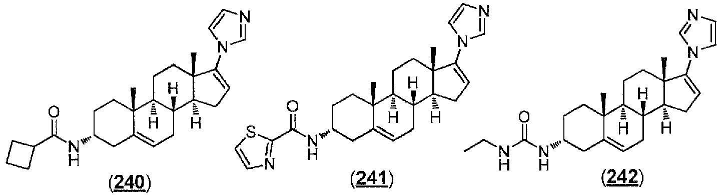

### Example 187A

### Preparation of Compound (237a)

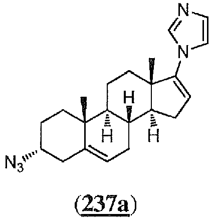

A mixture of Compound **(236a)** (800 mg, 1.63 mmol), NaN₃ (317 mg, 4.88 mmol) in DMF (10 mL) was stirred at 80 °C for 2 hours. Cooled to room temperature and water (100 mL) was added, and extracted with ethyl acetate (3 x 50 mL), washed with water (3 x 20 mL), brine (1 x 20 ml), dried and concentrated to give the crude product which purified by column chromatography (ethyl acetate in petroleum ether, 30% v/v) to give a crude Compound (**237a**) (468 mg) as a light yellow solid. LC-MS (m/z): 364 [M+H]⁺.

### Example 187B

### Preparation of Compound (237b)

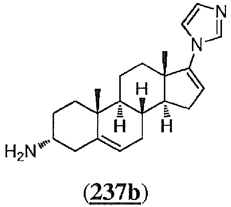

A mixture of the crude Compound **(237a)** (468 mg) and PPh₃ (1.69 g, 6.45 mmol) in THF (5 mL) and MeOH (3 mL) was stirred at 50 °C for overnight. Cooled and the solvent was removed. The residue was diluted with water (20 mL), adjusted to pH = 2 with 2 M HCl. Extracted with ethyl acetate (3 x 30 mL) and discarded off the organic layer. The aqueous layer was adjusted to pH7-8 with Na₂CO₃ aq. Solution and extracted with ethyl acetate (3 x 50 mL), the combined organic layer was dried and concentrated to give a crude Compound **(237b)** (444 mg). LC-MS (m/z): 338 [M+H]⁺.

### Example 187C

### Preparation of Compounds (237), (238), (239), (240), (241), and (242)

A mixture of Compound **(237b)** (444 mg) in Ac₂O (7 mL) was stirred at 36 °C for 1 hour. Then it was poured to ice-water and extracted with ethyl acetate (3 x 60 mL). The combined organic layers were washed with Na₂CO₃ until the pH of the aqueous layer reaching to 7. Then it was dried and concentrated to give the crude product which was purified by Prep-TLC (ethyl acetate) to get the final Compound **(237)** (250 mg, 51%) as a white solid. LC-MS (m/z): 380 [M+H]⁺. ¹H NMR (CDCl₃, 400 MHz) major characteristic peaks: *δ* (ppm) 1.01 (s, 3H), 1.08 (s, 3H), 2.60 (m, 1H), 4.16 (m, 1H), 5.43 (m, 1H), 5.52 (m, 1H), 5.70 (m, 1H), 7.07 (s, 1H), 7.10 (s, 1H), 7.66 (s, 1H).

Similarly, Compounds **(238), (239), (240), (241), (242)** were prepared using **(237b)** and corresponding reactants.

Compound **(238)** as a white solid, yield 20%: LC-MS (m/z): 406 [M+H]⁺; ¹H NMR (CDCl₃, 400 MHz) major characteristic peaks: *δ* (ppm) 0.70 (m, 2H), 0.95 (m, 2H), 1.02 (s, 3H), 1.08 (s, 3H), 4.19 (m, 1H), 5.45 (m, 1H), 5.70 (m, 2H), 7.06 (s, 1H), 7.10 (s, 1H), 7.64 (s, 1H).

Compound **(239)** as a white solid, yield 11%: LC-MS (m/z): 396 [M+H]⁺; ¹H NMR (CDCl₃, 400 MHz) major characteristic peaks: *δ* (ppm) 1.00 (s, 3H), 1.07 (s, 3H), 3.66 (s, 3H), 3.93 (m, 1H), 4.83 (m, 1H), 5.40 (m, 1H), 5.70 (m, 1H), 7.06 (s, 1H), 7.10 (s, 1H), 7.64 (s, 1H).

Compound **(240)** as a white solid, yield 14%: LC-MS (m/z): 420 [M+H]⁺; ¹H NMR (CDCl₃, 400 MHz) major characteristic peaks: *δ* (ppm) 1.01 (s, 3H), 1.08 (s, 3H), 2.60 (m, 1H), 2.97 (m, 1H), 4.13 (m, 1H), 5.43 (m, 2H), 5.70 (m, 1H), 7.06 (m, 1H), 7.09 (m, 1H), 7.64 (m, 1H).

Compound (**241**) as a white solid, yield 46%: LC-MS (m/z): 449.23 [M+H]⁺; ¹H NMR (CDCl₃, 500 MHz) major characteristic peaks: *δ* 1.01 (s, 3H), 1.11 (s, 3H), 2.30 (m, 1H), 2.70 (m, 1H), 4.34 (m, 1H), 5.52 (m, 1H), 5.69 (m, 1H), 7.06 (s, 1H), 7.10 (s, 1H), 7.37 (m, 1H), 7.54 (d, *J* = 3.5 Hz, 1H), 7.64 (s, 1H), 7.84 (d, *J*= 3.5 Hz, 1H).

Compound **(242)** as a white solid, yield 36%: LC-MS: 409 [M+1]⁺; ¹H NMR (CDCl₃, 400 MHz) major characteristic peaks: *δ* (ppm) 1.00 (s, 3H), 1.05 (s, 3H), 1.11-1.15 (t, *J*= 7.2 Hz, 3H), 2.22-2.26 (m, 1H), 2.56-2.60 (m, 1H), 3.20 (m, 3H), 3.99 (m, 1H), 4.71-4.73 (d, *J* = 8.0 Hz, 1H), 4.89 (s, 1H), 5.31 (m, 1H), 5.69 (m, 1H), 7.07 (s, 1H), 7.09 (s, 1H), 7.67 (s, 1H).

### Example 188

### Preparation of Compound (243)

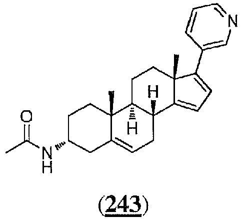

### Example 188A

### Preparation of Compound (243a)

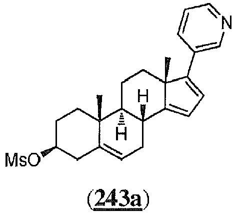

To a solution of Compound **(79)** (700 mg, 2.017 mmol) in pyridine (20 mL) was added MsCl (345 mg, 3.026 mmol) at room temperature and stirred at room temperature for 1 h. Then it was diluted with ethyl acetate (200 mL), washed with water and brine, dried with anhydrous Na₂SO₄ and evaporated to obtain **(243a)** (800 mg, 100%) as a white solid. LCMS: 426 [M+1] ⁺; ¹H NMR (CDC13, 400 MHz) peaks: *δ* (ppm) 1.17 (s, 3H), 1.23 (s, 3H), 3.03 (s,2H), 4.56 (s, 1H), 5.55 (s, 1H), 6.09 (s, 1H), 7.01 (s, 1H), 7.56-7.60 (m, 1H), 8.15 (d, J = 8.4 Hz, 1H), 8.46 (s, 1H), 8.82 (s, 1H).

### Example 188B

### Preparation of Compound (243b)

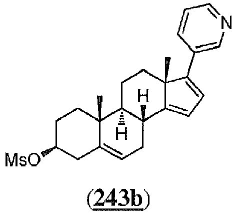

A suspension of Compound **(243a)** (420 mg, 0.988 mmol) and NaN₃ (193 mg, 2.965 mmol) in DMF (10 mL) was stirred at 100 °C for overnight. It was diluted with ethyl acetate (200 mL) was washed with water and brine, dried with anhydrous Na₂SO₄ and evaporated to obtain Compound **(243b)** (300 mg, 82%) as white solid.LC-MS: 373 [M+1]⁺.

### Example 188C

### Preparation of Compound (243c)

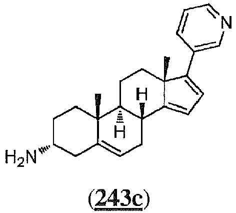

A solution of Compound **(243b)** (300 mg, 0.806 mmol) and PPh₃ (1.056 g, 4.032 mmol) in THF/MeOH (20/5 mL) was stirred at 50 °C for 2 h. The mixture was adjusted to pH 2 with hydrochloric acid and extracted with ethyl acetate (200 mL) to remove by-product. The aqueous phase was adjusted to pH9 with NaOH and extracted with DCM (100 mL x 3). The combined organic phase was dried with anhydrous Na₂SO₄ and evaporated to obtain Compound (**243c**) (200 mg, 72%) as a pale yellow oil. LC-MS: 347 [M+1]⁺.

### Example 188D

### Preparation of Compound (243)

A solution of Compound **(243c)** (200 mg, 0.578 mmol) in Ac₂O (10 mL) was stirred at 50 °C for 3 hrs. The mixture was cooled to room temperature and filtered, and dried to afford Compound (**243**) (100 mg, 45%) as a white solid. LC-MS: 389 [M+1] ⁺; ¹H NMR (CDC13, 400 MHz) peaks: *δ* (ppm) 1.17 (s, 3H), 1.21 (s, 3H), 1.96 (s,2H), 4.18 (s, 1H), 5.49-5.53 (m, 2H), 6.05 (s, 1H), 6.85 (s, 1H), 7.30-7.33 (m, 1H), 7.85 (d, *J* = 8.0 Hz, 1H), 8.42 (d, *J* = 3.6 Hz, 1H), 8.77 (s, 1H).

### Example 189

### Preparation of Compound (244)

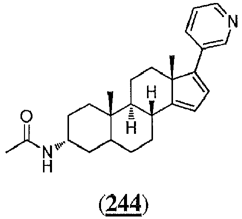

### Example 189A

### Preparation of Compound (244a)

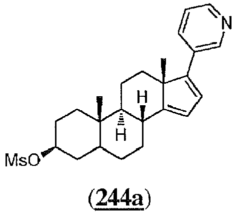

To a solution of Compound **(163)** (1.3 g, 3.7 mmol) in pyridine (20 mL) was added MsCl (852 mg, 7.4 mmol) at room temperature, then was stirred at this temperature for 2 h. The mixture was concentrated to give the crude product, and dissolved in ethyl acetate (200 mL), washed by water, brine, dried over Na₂SO₄. The crude product was washed with ether, filtered to obtain the Compound **(244a)** (1.6 g, crude) as a yellow solid. LC-MS: (m/z) 428(M+1). ¹H NMR (DMSO-*d*₆, 400 MHz) peaks: *δ* (ppm) 0.94 (s, 3H), 1.14 (s, 3H), 3.17 (s, 3 H), 4.55 (m, 1H), 6.05 (s, 1H), 7.18 (d, *J* = 2.0 Hz, 1H), 7.63 (m, 1H), 8.49 (d, *J* = 5.2 Hz, 1H), 8.74 (d, *J* = 4.8 Hz, 1H), 8.89 (s, 1H).

### Example 189B

### Preparation of Compound (244b)

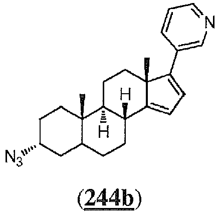

To a solution of Compound **(244a)** (1.6 g, 3.7 mmol) in DMF (20 mL) was added NaN₃ (483 mg, 7.4 mmol) at room temperature, then was stirred at 100 °C for 3 hrs. The mixture was diluted with water (150 mL), extracted with ethyl acetate (100 mL x 3). The combined organic phase was washed with water, brine, and dried over Na₂SO₄. The filtrate was concentrated to give the title compound **(244b)** (1.3 g, 93 %) as a yellow solid. LC-MS: (m/z) 375(M+1). ¹H NMR (DMSO-*d*₆, 400 MHz) peaks: *δ* (ppm) 0.91 (s, 3H), 1.12 (s, 3H), 4.03 (m, 1H), 5.99 (s, 1H), 6.87 (s, 1H), 7.33 (m, 1H), 7.87 (d, *J*= 8.0 Hz, 1H), 8.36 (d, *J*= 3.6 Hz, 1H), 8.76 (s, 1H).

### Example 189C

### Preparation of Compound (244c)

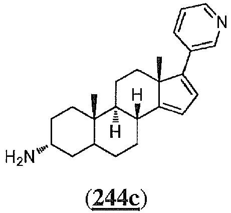

To a solution of Compound **(244b)** (1.3 g, 3.5 mmol) in MeOH and THF (40 mL, 1/ 1 V/V) was added PPh₃ (4.54 g, 17.4 mmol) at room temperature, then was stirred at 50 °C overnight. The solution was diluted with water (100 mL) and adjusted to pH 2-3 with 2N HCl. Extracted with ethyl acetate (100 mL x 3) and the water phase was adjusted to pH 12-13 with 2N NaOH, extracted with ethyl acetate (100 mL x 2). The combined organic phase was washed with water, brine, dried over Na₂SO₄ and evaporated to obtained Compound **(244c)** (1 g, 83 %) as a yellow oil.LC-MS: (m/z) 349 (M+1). ¹H NMR (CDCl₃, 400 MHz) peaks: *δ* (ppm) 0.92 (s, 3H), 1.17 (s, 3H), 5.98 (s, 1 H), 6.79 (d, *J* = 2.0 Hz, 1H), 7.21 (m, 1H), 7.75(d, J= 8.4 Hz, 1H), 8.39 (m, 1H), 8.75 (d, *J* = 1.6 Hz, 1H).

### Example 189D

### Preparation of Compound (244)

The solution of Compound **(244c)** (100 mg, 0.29 mmol) in **Ac₂O** (5 mL)) was stirred at 50 °C for 3 hrs. The mixture was diluted with water (150 mL), extracted with ethyl acetate (100 mL x 3). The combined organic phase was washed with water, brine, and dried over Na₂SO₄. The crude product was purified by prep-TLC (DCM/MeOH= 30/1, v/v) to give the title compound (**244**) (66 mg, 59 %) as a white solid. LC-MS: (m/z) 391 (M+1). ¹H NMR (CDC13, 400 MHz) peaks: *δ* (ppm) 0.95 (s, 3H), 1.17 (s, 3H), 1.98 (s, 3 H), 4.14 (m, 1H), 5.81 (d, *J=* 6.8 Hz, 1 H), 5.98 (m, 1H), 6.80 (d, *J* = 2.0 Hz, 1H), 7.24 (m, 1H), 7.76 (m, 1H), 8.40 (d, *J =* 3.6 Hz, 1H), 8.76 (d, *J =* 1.6 Hz, 1H).

### Example 190

### Preparation of Compound (245)

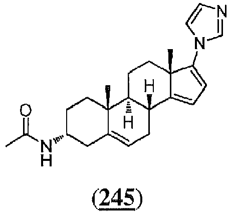

### Example 190A

### Preparation of Compound (245a)

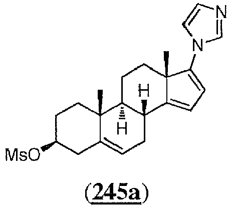

To a solution of Compound **(74)** (672 mg, 2 mmol) in pyridine (10 mL) was added MsCl (0.5 mL). The reaction mixture was stirred at 80 °C for 4 hrs, poured into ice water and extracted with DCM (30 mL x 3). The organic layer was washed with water, brine, dried, concentrated, purified by silica gel column chromatography (petroleum ether/ethyl acetate=1/3, v/v) to afford Compound **(245a)** (420 mg, 50%) as a white solid. LC-MS (m/z) 415[M+H]⁺; ¹H NMR (CDCl₃, 400 MHz) major characteristic peaks: *δ* (ppm) 1.13 (s, 3H), 1.14 (s, 3H), 3.02 (s, 3H), 4.54 (m, 1H), 5.57 (s, 1H), 5.92 (m, 1H), 6.27 (m, 1H), 7.10 (s, 1H), 7.12 (s, 1H), 7.65 (s, 1H).

### Example 190B

### Preparation of Compound (245b)

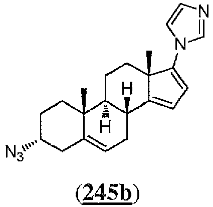

To a solution of Compound **(245a)** (420 mg, 1 mmol) in DMF (10 mL) was added NaN₃ (130 mg, 2 mmol). The reaction mixture was stirred at 100 °C for 4 hrs, poured the mixture into ice and extracted with DCM (30 mL x 3). The organic layer was washed with water, brine, dried, concentrated, and purified by silica gel column chromatography (petroleum ether/ethyl acetate=1/1, v/v) to afford Compound **(245b)** (300 mg, 83%) as a yellow solid. LC-MS (m/z) 362[M+H]⁺; ¹H NMR (CDCl₃, 400 MHz) major characteristic peaks: *δ* (ppm) 1.12 (s, 6H), 3.91 (m, 1H), 5.53 (m, 1H), 5.92 (m, 1H), 6.26 (m, 1H), 7.11 (s, 1H), 7.13 (s, 1H), 7.65 (s, 1H).

### Example 190C

### Preparation of Compound (245c)

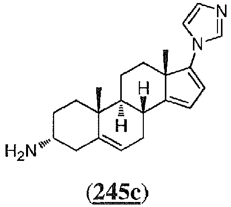

To a solution of Compound **(245b)** (300 mg, 0.8 mmol) in MeOH (10 mL) was added Ph₃P (420 mg, 1.6 mmol). The reaction mixture was stirred at 50 °C overnight. Cooled to room temperature and diluted with water (50 mL). The mixture was adjusted to pH 2 with diluted aqueous HCl and extracted with DCM (30 mL x 3), the organic layer was discarded off. The water phase was adjusted to pH 10 with aqueous NaOH and extracted with DCM (30 mL x 3). The organic layer was washed with water, brine, dried, concentrated and purified by silica gel column chromatography (petroleum ether/ethyl acetate=1/3, v/v) to afford Compound **(245c)** (230 mg, 76%) as a yellow solid. LC-MS (m/z) 336[M+H]⁺.

### Example 190D

### Preparation of Compound (245)

A solution of Compound **(245c)** (230 mg, 0.68 mmol) in Ac₂O (2 mL) was stirred at room temperature for 4 h. The mixture was diluted with water (20 mL) and extracted with DCM (20 mL x 3). The organic layer was washed with water, brine, dried, concentrated and purified by silica gel column chromatography (MeOH/DCM=1/20, v/v) to afford **(245)** (40 mg, 18%) as a yellow solid. LC-MS (m/z) 378[M+H]⁺; ¹H NMR (CDCl₃, 400 MHz) major characteristic peaks: *δ* (ppm) 1.14 (s, 6H), 1.95 (s, 3H), 4.17 (m, 1H), 5.51 (m, 2H), 5.92 (s, 1H), 6.26 (s, 1H), 7.11 (s, 1H), 7.13 (s, 1H), 7.65 (s, 1H).

### Example 191

### Preparation of Compound (246)

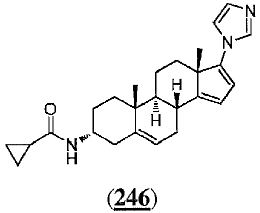

To a solution of Compound **(245c)** (335 mg, 1 mmol) in DCM (10 mL) was added EDCI (286 mg, 1.5 mmol), HOBt (202 mg, 1.5 mmol), Et₃N (2 mL). The mixture was stirred at room temperature about 30 mins. Cyclopropanecarboxylic acid (0.5 mL) was added and the resulted mixture was stirred at room temperature for 4 hrs. The mixture was quenched with water (30 mL), extracted with DCM (30 mL x 3) and the combined organic layers were washed with water, brine, dried, purified with silica gel column chromatography (petroleum ether/ethyl acetate=1/4, v/v), then further purified by prep-HPLC to afford Compound **(246)** (76 mg, 20%) as a white solid. LC-MS (m/z) 404[M+H]⁺; ¹H NMR (CDCl₃, 400 MHz) major characteristic peaks: *δ* (ppm) 0.69 (m, 2H) 0.94 (m, 2H), 1.14 (s, 3H), 1.15 (s, 3H), 2.45 (m, 1H), 2.64 (m, 1H), 4.20 (m, 1H), 5.55 (m, 1H), 5.68 (s, 1H), 5.93 (s, 1H), 6.27 (s, 1H), 7.14 (m, 2H), 7.68 (s, 1H).

### Example 192

### Preparation of Compound (247)

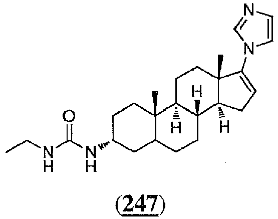

### Example 192A

### Preparation of Compound (247a)

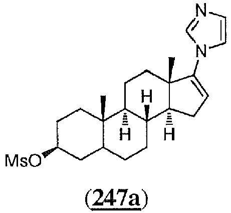

A solution of **(132)** (700 mg, 2.0 mmol) and MsCl (0.8 mL, 5.0 mmol) in Pyridine (10 mL) was stirred at 25 °C for 2 hrs. Then it was diluted with ethyl acetate (200 mL), washed with water and brine, dried with anhydrous Na₂SO₄ and then concentrated to give the product **(247a)** (800 mg, 93%) as a yellow solid. LC-MS: (m/z) 419 [M+1]⁺; ¹H NMR (CDCl₃, 400 MHz) major characteristic peaks: *δ* (ppm) 0.88 (s, 3H), 0.96 (s, 3H), 3.00 (s, 3H), 4.62-4.66 (m, 1H), 5.66 (s, 1H), 7.03 (s, 1H), 7.08 (s, 1H), 7.61 (s, 1H).

### Example 192B

### Preparation of Compound (247b)

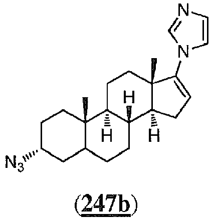

A suspension Compound **(247a)** (800 mg, 1.91 mmol) and NaN₃ (250 mg, 3.82 mmol) in DMF (10 mL) was stirred at 90 °C for 3 hrs. It was then diluted with ethyl acetate (200 mL), washed with water and brine, dried with anhydrous Na₂SO₄. After evaporated to dryness, it gave the product **(247b)** (700 mg, 97%) as a yellow solid. LCMS: 366 [M+1]⁺; ¹H NMR (CDCl₃, 400 MHz) major characteristic peaks: *δ* (ppm) 0.83 (s, 3H), 0.95 (s, 3H), 3.89-3.90 (m, 1H), 5.66 (s, 1H), 7.03 (s, 1H), 7.07 (s, 1H), 7.61 (s, 1H).

### Example 192C

### Preparation of Compound (247c)

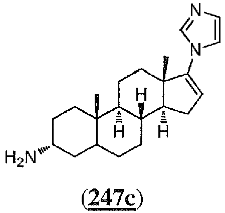

A solution of Compound **(247b)** (700 mg, 1.9 mmol) and PPh₃ (1.49 g, 5.7 mmol) in MeOH (50 mL) was stirred at 50 °C for 5 h. Then it was diluted with ethyl acetate (100 mL) and adjusted PH to 2 with 2N HCl solution and extracted with 2N HCl solution for three times. The combined aqueous was adjusted PH to 9 with 2N NaOH solution and extracted with DCM (50 mL x 3). The organic phase was dried with anhydrous Na₂SO₄ and concentrated to give the product **(247c)** (660 mg, 98%) as a brown yellow solid. LCMS: 340 [M+1]⁺; ¹H NMR (CDCl₃, 400 MHz) major characteristic peaks: *δ* (ppm) 0.82 (s, 3H), 0.95 (s, 3H), 3.21 (m, 1H), 5.66 (s, 1H), 7.03 (s, 1H), 7.07 (s, 1H), 7.61 (s, 1H).

### Example 192D

### Preparation of Compound (247)

To a solution of Compound **(247c)** (100 mg, 0.295 mmol) in dry THF (10 mL) was added isocyanatoethane (42 mg, 0.60 mmol) and stirred at room temperature for 1 h. After reaction the mixture was diluted with DCM (50 mL) and washed with brine (50 mL x 2) and dried over Na₂SO₄, concentrated and purified by column (ethyl acetate/ petroleum ether = 1:5 to ethyl acetate, v/v) to give the product **(247)** (55 mg, 45%) as a white solid. LC-MS: (m/z) 411(M +H)⁺; ¹H NMR (CDCl₃, 400 MHz) major characteristic peaks: *δ* (ppm) 0.80 (s, 3H), 0.95 (s, 3H), 1.13 (t, *J =* 7.2 Hz, 3H), 3.21-3.26 (m, 2H), 3.96 (s, 1H), 5.16 (s, 1H), 5.25 (d, *J =* 7.6 Hz, 1H), 5.69 (s, 1H), 7.09 (s, 2H), 7.72 (s, 1H).

### Example 193

### Preparation of Compound (248)

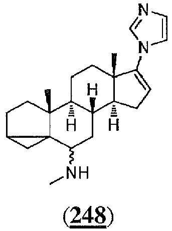

A mixture of Compound **(236a)** (492 mg, 1 mmol) in MeNH₂/EtOH (10 mL) and pyridine (5 mL) in a sealed tube was stirred at 80 °C for 2 hrs. After the removal of solvent by evaporation, the residue was dissolved in ethyl acetate (60 mL), washed with water (3 x 20 mL) and brine (20 mL), dried and concentrated to give the crude product which was purified by prep-HPLC to afford Compound **(248)** (24 mg, 7%) as a light yellow oil. LC-MS (m/z): 352 [M+H]⁺; ¹H NMR (CDCl₃, 400 MHz) major characteristic peaks: *δ* (ppm) 0.24 (m, 1H), 0.52 (m, 1H), 1.01 (s, 1H), 1.03 (s, 2H), 1.05 (s, 1H), 1.06 (s, 2H), 2.39 (m, 2H), 2.46 (m, 1H), 5.68 (m, 1H), 7.05 (s, 1H), 7.08 (s, 1H), 7.63 (s, 1H).

### Example 194

### Alternative Preparation of Compound (236)

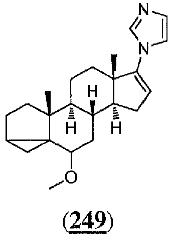

Compound **(236a)** (300 mg, 0.61 mmol) was dissolved in methanol (10 mL) and stirred at reflux for 2 h. After the removal of the solvent, the resulted residue was dissolved in ethyl acetate (100 mL) and washed with saturated NaHCO₃ (100 mL), brine (100 mL), dried over anhydrous Na₂SO₄. The solution was concentrated and purified by prep-HPLC to give Compound **(249)** (10 mg, 5%) as a white solid. LC-MS (m/z): 353 [M+H]⁺; ¹H NMR (CDCl₃, 400 MHz) major characteristic peaks: *δ* (ppm) 0.41 (m, 1H), 0.62 (m, 1H), 0.96 (s, 3H), 1.00 (s, 3H), 2.77 (m, 1H), 3.30 (s, 3H), 5.60 (m, 1H), 7.00 (s, 1H), 7.02 (s, 1H), 7.57 (s, 1H).

### Example 195

### Preparation of Compound (250)

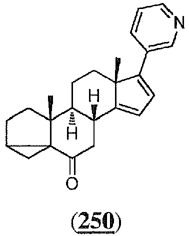

To a solution of Compound **(200)** (190 mg, 0.55 mmol) in THF (10 mL) was added tetrapropylammonium perruthenate (12 mg, 0.03 mmol), 4-methylmorpholine- *N*-oxide monohydrate (109 mg, 0.93 mmol) and the resulted mixture was stirred at 50 °C overnight. The solution was cooled and concentrated and the crude product was purified by pre-HPLC to afford the title compound **(250)** (39 mg, 21 %) as a yellow solid. LC-MS (m/z): 346 [M+1]⁺; ¹H NMR (CDCl₃, 400 MHz) major characteristic peaks: *δ* (ppm) 0.80 (t, *J =* 4.8 Hz, 1 H), 1.16 (s, 3H), 1.28 (s, 3H), 6.12 (m, 1H), 7.13 (s, 1H), 7.80 (m, 1H), 8.39 (d, *J=* 8.0 Hz, 1H), 8.61 (m, 1H), 9.01 (s, 1H).

### Example 196

### Preparation of Compound (251)

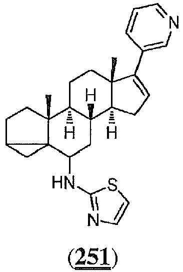

A mixture of Compound **(223c)** (200 mg, 0.4 mmol) and thiazol-2-amine (346 mg, 4.0 mmol) was stirred at 100 °C for 2.0 hrs at this point LC-MS showed that the reaction was complete. The mixture was cooled, dissolved in MeOH (10 mL) and water (50 mL) was added, extracted with DCM (30 mL x 3).The combined organic phases were washed water, brine, dried Na₂SO₄ and concentrated to crude product. The crude product was purified by prep-TLC to afford Compound **(251)** (15 mg, 9%) as a white solid. LCMS (m/z): 432 [M+1]; ¹H-NMR (400 MHz, CDCl₃): *δ* (ppm) 0.39 (m, 1H), 0.58 (m, 1H), 1.06 (s, 3H), 1.15 (s, 3H), 3.07 (m, 1H), 5.62 (s, 1H), 5.96 (s, 1H), 6.48 (m, 1H), 7.12 (m, 1H), 7.22 (m, 1H), 7.64 (m, 1H), 8.46 (m, 1H), 8.61(m, 1H).

### Example 197

### Preparation of Compound (252)

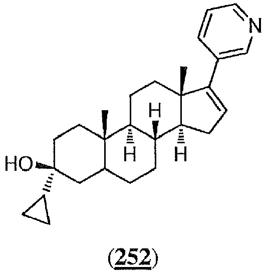

To a solution of Compound **(211b)** (0.2 g, 0.57 mmol) in THF (5 mL) was added cyclopropylmagnesium bromide (0.5 M, 0.57 mL) at room temperature. The mixture was stirred for 30 min before it was diluted by ethyl acetate (20 mL). The mixture was washed with water (30 mL x 3), brine and dried over Na₂SO₄, and then concentrated to give a crude product. The crude product was purified by column chromatography (ethyl acetate in petroleum ether, 30% v/v) to afford compound **(252)** (48 mg, 21.4%) as a white solid. LC-MS (m/z): 392 [M+1]⁺; ¹H NMR (CDCl₃, 400 MHz) major characteristic peaks: *δ* (ppm) 0.33 (s, 2H), 0.35 (s, 2H), 0.81 (s, 3H), 1.00 (s, 1H) ,5.99 (s, 1H), 7.23 (m, 1H), 7.66 (d, *J =* 6.4 Hz, 1H), 8.45 (s, 1H), 8.62 (s,1H).

### Example 198

### Preparation of Compounds (253), (254), (255), (256), (257), and (258)

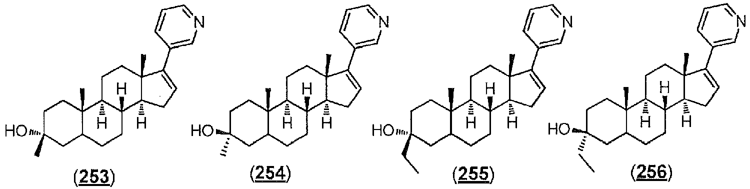

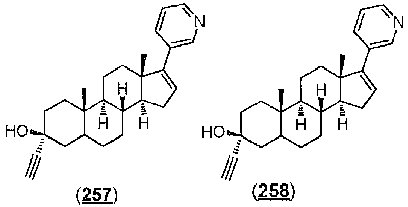

To a solution of Compound **(211b)** (0.2 g, 0.57 mmol) in THF (5 mL) was added methyl magnesium bromide (3 M, 0.57 mL) at room temperature. Stirred for 30 min, the reaction mixture was diluted with ethyl acetate (20 mL) and washed with water (30 mL x 3), brine and dried over Na₂SO₄, and then concentrated to afford a crude product. The crude product was purified by column chromatography (ethyl acetate in petroleum ether, 30% v/v) to obtain the mixture of **(253)** and **(254),** which was separated by prep-HPLC to afford the **(253)** (19 mg, 9%) and **(254)** (62 mg, 30%) as white solids. For **(253),** LC-MS: (m/z) 366 [M+1]⁺; ¹H NMR (CDCl₃, 400 MHz) major characteristic peaks: *δ* (ppm) 0.81 (s, 3 H), 1.00 (s, 3 H), 1.21 (s, 3 H) , 5.97 (s, 1 H), 7.21 (m, 1 H), 7.66 (d, *J* = 8 Hz,1 H), 8.45 (s,1H), 8.62 (s,1H); For **(254)**: LC-MS: (m/z) 366 [M+1]⁺; ¹H NMR (CDCl₃, 400 MHz) major characteristic peaks: *δ* (ppm) 0.87 (s, 3 H), 1.01 (s, 3 H), 1.26 (s, 3 H), 5.99 (s, 1 H), 7.23 (m, 1 H), 7.66 (d, J = 6.4 Hz,1 H), 8.46 (s,1H), 8.62 (s, 1H).

Following the same procedure by employing ethyl magnesium bromide, Compounds **(255)** and **(256)** were prepared.

For Compound **(255),** LC-MS: (m/z) 380 [M+1]⁺;¹H NMR (CDCl₃, 400 MHz) major characteristic peaks: *δ* (ppm) 0.80 (s, 3 H), 0.92 (t, J = 6.0 Hz, 3 H), 1.01 (s, 3 H), 5.98 (s, 1 H), 7.21 (m, 1 H), 7.66 (d, *J* = 9.6 Hz,1 H), 8.45 (s,1H), 8.62 (s,1H).

Compound **(256),** LC-MS: (m/z) 380 [M+1]⁺; ¹HNMR (CDCl₃, 400 MHz) major characteristic peaks: *δ* (ppm) 0.88 (t, J = 6.0 Hz, 6 H), 1.01 (s, 3 H), 1.26 (s, 3 H), 5.99 (s, 1 H), 7.22 (m, 1 H), 7.65 (d, *J =* 8.4 Hz,1 H), 8.46 (s,1H), 8.62 (s,1H).

Following the same procedure by employing ethynyl magnesium bromide, Compounds **(257)** and **(258)** were prepared.

For Compound **(257),** LC-MS: (m/z) 376 [M+1]⁺; ¹H NMR (CDCl₃, 400 MHz) major characteristic peaks: *δ* (ppm) 0.87 (s, 3 H), 1.01 (s, 3 H), 2.48 (s, 1 H), 5.98 (s, 1 H), 7.22 (m, 1 H), 7.64 (d, *J =* 8 Hz,1 H), 8.45 (s,1H), 8.62 (s,1H).

For Compound **(258),** LC-MS: (m/z) 376 [M+1]⁺; ¹H NMR (CDCl₃, 400 MHz) major characteristic peaks: *δ* (ppm) 0.86 (s, 3 H), 1.00 (s, 3 H), 2.44 (s, 1 H), 5.98 (s, 1 H), 7.22 (m, 1 H), 7.65 (d, *J=* 10 Hz,1 H), 8.45 (s,1H), 8.61 (s,1H).

### Example 199

### Preparation of Compounds (259), (260), (261), and (262)

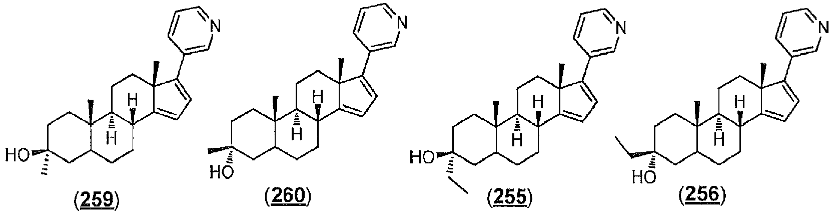

To a solution of Compound **(164)** (85 mg, 0.25 mmol) in dry THF (5 ml) was added dropwise a solution of CH₃MgBr (3.0 M, 0.25 ml, 0.75 mmol) in THF at room temperature under N₂. After addition, the mixture was stirred at the same condition for 1.0 hr. Reaction mixture was quenched with saturated ammonium chloride solution and extracted with DCM (50 mL x 3). The organic layer was washed with brine, dried over Na₂SO₄ and concentrated to give a crude product. The crude product was purified by prep-TLC to afford Compound **(259)** (20 mg, yield 32%) as a white solid and **(260)** (10 mg, yield 16%) as a white solid. For Compound **(259),** LC-MS (ESI) m/z: 364 (M+1)⁺; ¹H-NMR (400 MHz, CDCl₃) δ (ppm) 0.82 (s, 3 H), 1.10 (s, 3 H), 1.15 (s, 3 H), 5.91 (m, 1 H), 6.72 (m, 1 H), 7.16 (m, 1 H), 7.69 (m, 1 H), 8.33 (m, 1 H), 8.68 (s, 1 H); for Compound **(264),** LC-MS (ESI) m/z: 364 (M+1)⁺; ¹H-NMR (400 MHz, CDCl₃) δ (ppm) 0.96 (s, 3 H), 1.17 (s, 3 H), 1.25 (s, 3 H), 5.99 (m, 1 H), 6.79 (m, 1 H), 7.23 (m, 1 H), 7.76 (m, 1 H), 8.41 (m, 1 H), 8.76 (s, 1 H).

Following the same procedure by employing ethyl magnesium bromide, Compounds **(261)** and **(262)** were prepared.

For Compound **(261),** LC-MS (ESI) m/z: 378 (M+1)⁺; ¹H-NMR (400 MHz, CDCl₃) *δ* (ppm) 0.88 (s, 3 H), 0.92 (t, *J =* 7.6 Hz, 3 H), 1.17 (s, 3 H), 5.98 (m, 1 H), 6.80 (m, 1 H), 7.23 (m, 1 H), 7.76 (m, 1 H), 8.40 (m, 1 H), 8.75 (s, 1 H).

For compound **(262),** LC-MS (ESI) m/z: 378(M+1)⁺; ¹H-NMR (400 MHz, CDCl₃) *δ* (ppm) 0.88 (t, *J=* 7.6 Hz, 3 H), 0.97 (s, 3 H), 1.17(s, 3 H), 5.98 (m, 1 H), 6.79 (m, 1 H), 7.23 (m, 1 H), 7.76 (m, 1 H), 8.40 (m, 1 H), 8.76 (s, 1 H).

### Example 200

### Preparation of Compounds (263) and (264)

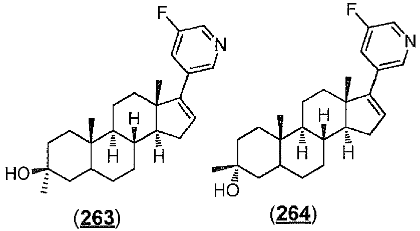

### Example 200A

### Preparation of Compound (263a)

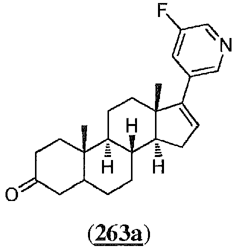

A mixture of Compound **(210c)** (1.12 g, 2.8 mmol), 5-fluoropyridin-3-ylboronic acid (793 mg, 5.6 mmol), Pd(PPh₃)₂Cl₂ (196 mg, 0.28 mmol), K₂CO₃ (1.9 g, 14 mmol) in dioxane (50 mL) and water (10 mL) was stirred at 80°C overnight. After cooled down to room temperature, the mixture was poured into water and extracted with ethyl acetate (100 mL x 3). The organic layer was washed with brine, dried with anhydrous sodium sulfate, and then concentrated to yield a crude product. The crude product was purified by column chromatography (petroleum ether/ethyl acetate = 5/1- 3/1, v/v) to afford Compound **(263a)** (600 mg, 54%) as a white solid. LC-MS (ESI) m/z: 368 (M+1)⁺.

### Example 200B

### Preparation of Compound (263) and (264)

A solution of **(263a)** (600 mg, 1.6 mmol) in THF (15 mL) was added CH₃MgBr solution (3 M,1.6 mL, 4.8 mmol) slowly at 0°C. The mixture was warmed to room temperature and stirred overnight. The mixture was quenched with sat. ammonium chloride solution (10 mL) and extracted with ethyl acetate (50 mL x 2). The extraction was washed with brine, dried, concentrated to yield a crude product. The crude was purified by prep-HPLC to afford **(263)** (68 mg) as a white solid and **(264)** (158 mg) as a white solid. For Compound **(263),** LC-MS (ESI) m/z:384 (M+1)⁺; ¹H-NMR (400 MHz, CDCL3): *δ* (ppm) 0.87 (s, 3H), 1.01 (s, 3H), 1.26 (s, 3H ), 6.04-6.05 (m, 1H), 7.35-7.38 (m, 1H), 8.32 (m, 1H), 8.44 (m, 1H). For Compound **(264),** LC-MS (ESI) m/z:384 (M+1)⁺; ¹H-NMR (400 MHz, CDCL3): *δ* (ppm) 0.81 (s, 3H), 1.01 (s, 3H), 1.21 (s, 3H), 6.04-6.05 (m, 1H), 7.35-7.38 (m, 1H), 8.31 (m, 1H), 8.44 (m, 1H).

### Example 201

### Preparation of Compounds (265) and (266)

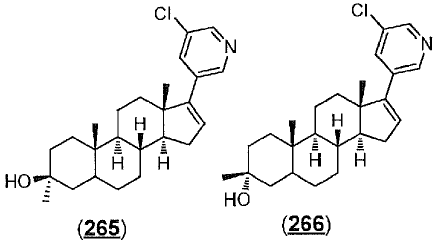

### Example 201A

### Preparation of Compound (265a)

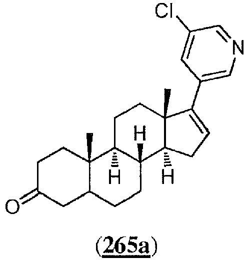

A mixture of compound **(210c)** (398 mg, 1 mmol), boronic acid (157 mg, 1 mmol), K₂CO₃ (600 mg, 4 mmol) and Pd(PPh₃)₂Cl₂ (80 mg, 0.01 mmol) in 1,4-dioxane (20 mL) and water (5 mL) was stirred at 80 °C for 2 hrs. The mixture was cooled down to room temperature, diluted with water (30 mL), and extracted with ethyl acetate (30 mL x 3). The organic layer was washed with water, brine, concentrated to give a crude product. The crude product was purified by column chromatography (petroleum ether, then petroleum ether/EtOAc = 5/1, v/v) to afford Compound **(265a)** (300 mg, 75%) as a yellow solid. LC-MS (ESI) m/z: 384 (M+1)⁺.

### Example 201B

### Preparation of Compounds (265) and (266)

To a solution of Compound **(265a)** (384 mg, 1 mmol) in THF (20 mL) was added CH₃MgBr (3M, 3 mL) at 0 °C. The mixture was stirred at room temperature for about 2 hours and quenched with sat. aq. NH₄Cl solution, extracted with ethyl acetate (30 mL x 3). The combined the organic layers were washed with water, brine, dried and concentrated to yield a crude product. The crude product was purified by column chromatography (petroleum ether/ethyl acetate = 10/1, v/v) to afford Compound **(265)** (60 mg) and **(266)** (60 mg) as white solids.

For Compound **(265),** LC-MS (m/z): 400[M+H]⁺; ¹H NMR (CDCl₃, 400 MHz) major characteristic peaks: δ (ppm) 0.808 (s, 3H), 1.001 (s, 3H), 1.211 (s, 3H), 2.26 (m, 1H), 6.03 (s, 1H), 7.64 (m, 1H), 8.42 (s, 1H), 8.49 (s, 1H).

For Compound **(266),** LC-MS (m/z): 400[M+H]⁺; ¹H NMR (CDCl₃, 400 MHz) major characteristic peaks: δ (ppm) 0.86 (s, 3H), 1.00 (s, 3H), 1.26 (s, 3H), 2.25 (m, 1H), 6.04 (s, 1H), 7.64 (m, 1H), 8.42 (s, 1H), 8.49 (s, 1H).

### Example 202

### Preparation of Compounds (267) and (268)

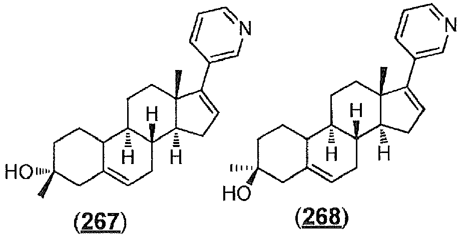

### Example 202A

### Preparation of Compound (267a)

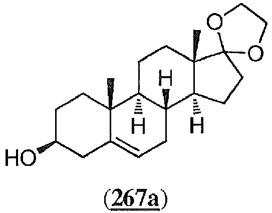

To a solution of (3S,10R,13S)-3-hydroxy-10,13-dimethyl-3,4,7,8,9,10,11,12,13,14,15,16-dodecahydro-1H-cyclopenta[a]phenanthren-17(2H)-one (5 g, 11 mmol) in ethylene glycol/ THF (15 mL/15 mL, v/v) was added CH(OEt)₃ (7.7 mL, 52 mmol) and p-TsOH (165 mg, 0.87 mmol) under N₂. The mixture was stirred at room temperature for 14 hrs. Then it was adjusted pH to 8 with Et₃N and poured into ice-water. The precipitates were filtered and dried to give desired product **(267a)** (5.7 g, 99%) as a white solid, which was directly used for the next step without further purification. LC-MS (ESI) m/z: 315 (M+H-18)⁺.¹H-NMR (400 MHz, CDCl₃-d₁) *δ* (ppm) 0.864 (s, 3H), 1.015 (s, 3H), 3.535 (m, 1H), 3.852-3.950 (m, 4H), 5.346 (m, 1H).

### Example 202B

### Preparation of Compound (267b)

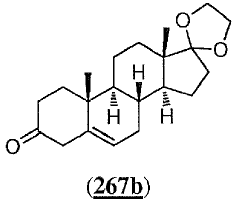

To a solution of **(267a)** (2 g, 6.04 mmol) in dry DCM (350 mL) was added DMP (5.2 g, 12.24 mmol) at 0 °C and the reaction mixture was stirred for 2.5 hrs at 28 °C, then it was diluted with ether (200 mL) and quenched with a mixture of saturated NaHCO₃/ Na₂S₂O₃ (400 mL, 1/3 v/v). The organic layer was washed with brine and dried over anhydrous Na₂SO₄, and then evaporated to dryness to obtain compound **(267b)** (2.2 g, 100%), which was used in next step without further purification. LC-MS (ESI) m/z: 331 (M+H)⁺; ¹H-NMR (400 MHz, CDCl₃) *δ* (ppm) 0.87 (s, 3H), 1.17 (s, 3H), 3.84-3.94 (m, 4H), 5.33-5.35 (t, *J =* 2.6 Hz, 1H).

### Example 202C

### Preparation of Compound (267c)

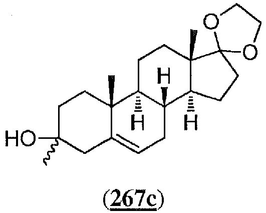

To a solution of **(267b)** (2 g, 6 mmol) in dry THF (100 mL) was added MeLi (1.5 M solution in ether, 16 mL, 24 mmol) at 0 °C, and the resulting mixture was stirred at room temperature for 3 hrs. The reaction was quenched with saturated aqueous NH₄Cl and was extracted with ethyl acetate. The organic layer was washed with brine and dried over Na₂SO₄, and the solvent was removed in vacuum. The residue was purified by flash column chromatography (petroleum ether/ethyl acetate = 85/15, v/v) to afford products **(267c)** (0.7 g, 30%). LC-MS (ESI) m/z: 329 (M+ H-18)⁺, 347 (M+H)⁺; ¹H-NMR (400 MHz, CDCl₃-d₁) *δ* (ppm) 0.86 (s, 3H), 0.98 (s, 3H), 1.21 (s, 3H), 3.84-3.95 (m, 4H), 5.41 (m, 1H).

### Example 202D

### Preparation of Compound (267d)

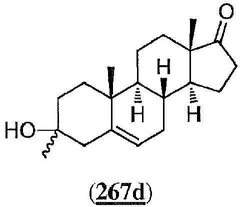

To a solution of **(267c)** (680 mg, 1.97 mmol) in THF (40 mL) was added a mixture of aqueous HCl (2 M, 36 mL) and acetone (36 mL), and the reaction mixture was stirred at room temperature for 12 hrs. Subsequently, the reaction mixture was neutralized by addition of aqueous 1 M NaOH, the organic layer was extracted with ethyl acetate, washed with brine, and dried over anhydrous Na₂SO₄. The solvents were evaporated in vacuo. The crude product was purified by flash column chromatography using petroleum ether, and ethyl acetate (10:1) as elution solvent to yield **(267d)** (570 mg, 96%) as a yellow solid. LC-MS (ESI) m/z: 285 (M+ H-18)⁺, 325 (M+Na)⁺.

### Example 202E

### Preparation of Compound (267e)

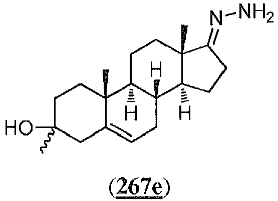

To a suspension of Compound **(267d)** (570 mg, 1.89 mmol) in EtOH (20 mL) was added 80% of NH₂NH₂ (5 mL) and stirred at 80°C for 4 hrs. After the completion of reaction, the mixture was poured into ice water to give a white solid Compound **(267e)** (482 mg, 82%) as white solid.LC-MS (ESI) m/z: 317[M+1]⁺.

### Example 202F

### Preparation of Compounds (267f), (267g)

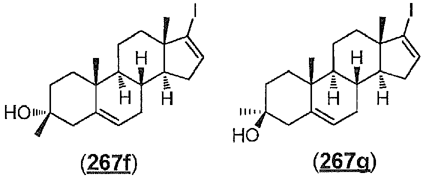

To a solution of Compound **(267e)** (482 mg, 1.53 mmol) and Et₃N (0.3 mL) in dry DCM (10 mL) was added dropwise the solution of I₂ (581 mg, 2.29 mmol) in dry THF (6 mL) at 0 °C. The solution was stirred at 0 °C for 2 hrs. Then it was quenched with Na₂SO₃ solution to destroy additional I₂. The mixture was diluted with DCM (50 mL), washed with water and brine, dried with anhydrous Na₂SO₄, and purified with silica column chromatography (petroleum ether/ethyl acetate = 15/1, v/v) to obtain compound **(267f)** (200 mg) as a yellow solid and compound **(267g)** (150 mg) as a white solid, a total yield: 56%.

For Compound **(267f),** LC-MS (ESI) m/z: 395 (M+ H-18)⁺, 413 (M+H)⁺;¹H-NMR (400 MHz, CDCl₃) *δ* (ppm) 0.79 (s, 3H), 1.01 (s, 3H), 1.24 (s, 3H), 5.41-5.42 (d, *J*=5.2 Hz, 1H), 6.14-6.15 (m, 1H).

For Compound **(267g),** LC-MS (ESI) m/z: 395 (M+ H-18)⁺, 413 (M+H)⁺; ¹H-NMR (400 MHz, CDCl₃) *δ* (ppm) 0.76 (s, 3H), 1.05 (s, 3H), 1.12 (s, 3H), 5.30-5.32 (d, *J*=5.2 Hz, 1H), 6.13-6.15 (m, 1H).

### Example 202H

### Preparation of Compound (267) and (268)

A suspension of Compound **(267f)** (25 mg, 0.06 mmol), pyridin-3-ylboronic acid (25 mg, 0.2 mmol), PdCl₂(PPh₃)₂ (4.5 mg, 0.005 mmol) and K₂CO₃ (33 mg, 0.24 mmol) in dioxane/H₂O (4/2 mL) was stirred at 85 °C for 2 hrs. Then it was diluted with ethyl acetate (15 mL), washed with water and brine, dried with anhydrous Na₂SO₄ and purified by prep-TLC (petroleum ether: ethyl acetate=2:1) to obtain Compound **(267)** (12 mg, 54%) as yellow solid. LC-MS (ESI) m/z: 364 [M+H] ⁺; ¹H-NMR (400 MHz, CDCl₃) *δ* (ppm) 1.05 (s, 6H), 1.24 (s, 3H), 5.45-5.45 (d, *J =* 4.8 Hz, 1H), 6.01 (d, *J =* 1.2 Hz, 1H), 7.23 (s, 1H), 7.67-7.69 (d, *J* = 8 Hz, 1H), 8.47 (s, 1H), 8.64 (s, 1H).

Using the same procedure, Compound **(268)** was prepared with Compound **(267g)** and pyridin-3-ylboronic acid (yield 46%) as a white solid. LC-MS (ESI) m/z: 364 [M+H]⁺; ¹H-NMR (400 MHz, CDCl₃) *δ* (ppm) 1.05 (s, 3H), 1.08 (s, 3H), 1.13 (s, 3H), 5.35-5.36 (d, *J*=4.8 Hz, 1H), 6.01 (s, 1H), 7.24 (s, 1H), 7.67-7.69 (d, *J*= 7.6 Hz, 1H), 8.48 (s, 1H), 8.64 (s, 1H).

### Example 203

### Preparation of Compounds (269) and (270)

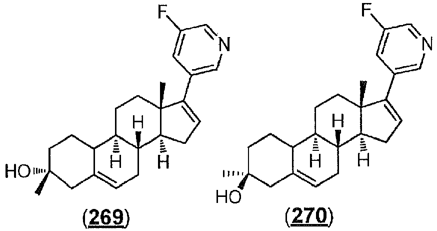

The suspension of Compound **(267f)** (100 mg, 0.243 mmol), (5-fluoropyridin-3-yl)boronic acid (75.3 mg, 0.53 mmol), PdCl₂(PPh₃)₂ (9 mg, 0.012 mmol) and K₂CO₃ (134 mg, 0.97 mmol) in dioxane/H₂O (10/5 mL) was stirred at 92 °C for 2 hrs. The mixture was diluted with ethyl acetate (50 mL), washed by sat NaHCO₃ (15 mL) and brine, dried with anhydrous Na₂SO₄, and purified by prep-HPLC to obtain Compound **(269)** (31 mg, 34%) as a pink solid. LC-MS (ESI) m/z: 382 [M+H]⁺; ¹H-NMR (400 MHz, CDCl₃) *δ* (ppm) 1.05 (s, 6H), 1.24 (s, 3H), 5.45-5.46 (d, *J=* 4.8 Hz, 1H), 6.06-6.08 (m, 1H), 7.36-7.39 (m, 1H), 8.33 (s, 1H), 8.45 (s, 1H).

Similarly compound **(270)** was prepared using Compound **(267g)** and (5-fluoropyridin-3-yl)boronic acid as a white solid (yield 43%). LC-MS (ESI) m/z: 382[M+H]⁺;¹H-NMR (400 MHz, CDCl₃-d₁) *δ* (ppm)1.05 (s, 3H), 1.07 (s, 3H), 1.13 (s, 3H), 5.34-5.36 (t, *J =* 2.4 Hz, 1H), 6.07-6.09 (m, 1H), 7.36-7.41 (m, 1H), 8.33 (s, 1H), 8.45 (s, 1H).

### Example 204

### Preparation of Compound (271)

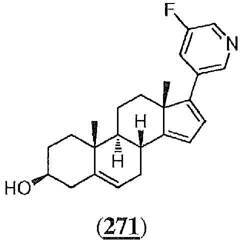

A mixture of Compound **(77)** (701 mg, 1.5 mmol), 5-fluoropyridin-3-ylboronic acid (215 mg, 1.5 mmol), K₂CO₃ (621 mg, 4.5 mmol) and Pd(PPh₃)₂Cl₂ (53 mg, 0.075 mmol) in dioxane (10 mL) and water (5 mL) was stirred at reflux for 4 hrs. The reaction mixture was cooled down to room temperature, quenched with water (10 mL), and then extracted with dichloromethane (50 mL x 3). The organic layers were combined and washed with NaHCO₃ solution (10 mL), brine (10 mL), concentrated and purified by flash column chromatography (ethyl acetate / petroleum ether = 1/20 to 1/8, v/v) to afford Compound **(271)** (140 mg, 25%) as a yellow solid. LC-MS (ESI) m/z: 366 (M+1)⁺; ¹H-NMR (400 MHz, CDCl₃) *δ* (ppm) 1.175 (s, 3H), 1.234 (s, 3H), 3.559-3.570 (m, 1H), 5.502 (s, 1H), 6.051 (s, 1H), 6.886 (s, 1H), 7.479-7.505 (m, 1H), 8.287-8.292 (m, 1H), 8.608 (s, 1H).

### Example 205

### Preparation of Compound (272)

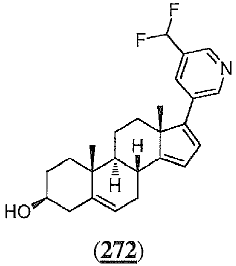

### Example 205A

### Preparation of Compound (272a)

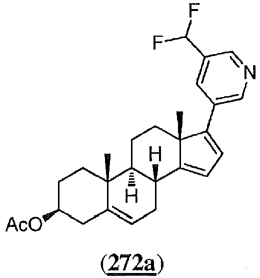

A mixture of Compound **(77)** (460 mg, 1 mmol), 5-(difluoromethyl)pyridin-3-ylboronic acid (173 mg, 1 mmol), K₂CO₃ (600 mg, 1.131 mmol) and Pd(PPh₃)₂Cl₂ (80 mg, 0.0377 mmol) in 1,4-dioxane (6 mL) and water (0.6 mL) was stirred at 80°C for 2 hrs. The mixture was cooled down to room temperature and extracted with ethyl acetate (50 mL x 3). The organic layer was washed with water, dried, and concentrated to yield a crude product. The crude product was purified by silica gel column chromatography (petroleum ether to petroleum ether/ EtOAc = 5/1, v/v) to afford Compound **(272a)** (40 mg, 28%). LC-MS (ESI) m/z: 440 (M+1)⁺.

### Example 205B

### Preparation of Compound (272)

To a solution of Compound **(272a)** (120 mg, 0.21 mmol) in MeOH/H₂O (10/2 mL) was added NaOH (60 mg, 1.5 mmol). The mixture was stirred at room temperature about 2 hours. The mixture was concentrated, poured into ice water, and extracted with DCM (10 mL x 2). The combined the organic layers were dried and evaporated to dryness to afford Compound **(272)** (50 mg, 45%) as a white solid. LC-MS (m/z): 398[M+H]⁺; ¹H NMR (CDCl₃, 400 MHz) major characteristic peaks: *δ* (ppm) 1.16 (s, 3H), 1.22 (s, 3H), 3.56 (m, 1H), 5.49 (m, 1H), 6.00 (s, 1H), 6.72 (t, *J=* 56 Hz, 1H), 6.90 (m, 1H), 7.89 (s, 1H), 8.54 (s, 1H), 8.87 (s, 1H).

### Example 206

### Preparation of Compound (273)

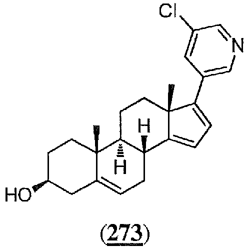

### Example 206A

### Preparation of Compound (273a)

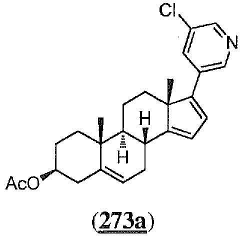

The suspension of compound **(77)** (200 mg, 0.434 mmol), 5-chloropyridin-3-ylboronic acid (124 mg, 0.8 mmol), PdCl₂(PPh₃)₂ (30 mg, 0.042 mmol) and K₂CO₃ (299 mg, 2.17 mmol) in dioxane/H₂O (11/1 mL) was stirred at 80 °C for 3 hours. Then it was diluted with ethyl acetate (150 mL), washed with water and brine, dried with anhydrous Na₂SO₄ and purified by Pre-HPLC to obtain compound **(273a)** (150 mg, 81.7%) as a white solid. LCMS: 424 [M+1]⁺.

### Example 206B

### Preparation of Compound (273)

To a solution of compound **(273a)** (150 mg, 0.35 mmol) in methanol (6 mL) and water (0.5 mL) was added KOH (33 mg, 0.59 mmol) and stirred at 25 °C for 3 h. After reaction the mixture was cooled to room temperature and adjusted pH to 7 with 1N HCl and extracted with DCM (100 mL x 2). The organic phase was washed with brine (50 mL x 2) and dried over Na₂SO₄, concentrated to give the product **(273)** (71 mg, 52%) as a yellow solid. LCMS: (m/z) 382[M+1]⁺; ¹H NMR (CDCl₃, 400 MHz) major characteristic peaks: *δ* (ppm) 1.15 (s, 3H), 1.21 (s, 3H), 3.56 (m, 1H), 5.49 (d, *J =* 2.8 Hz, 1H), 6.02-6.03 (m, 1H), 6.86 (d, *J =* 2.4 Hz, 1H), 7.73-7.75 (m, 1H), 8.36 (d, *J=* 1.6 Hz, 1H), 8.64 (d, *J=* 1.6 Hz, 1H).

### Example 207

### Preparation of Compounds (274), (275), (276), (277), (278), (279), (280), (281), (282), (283), and (284)

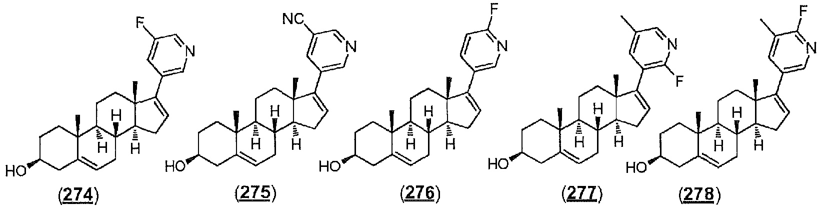

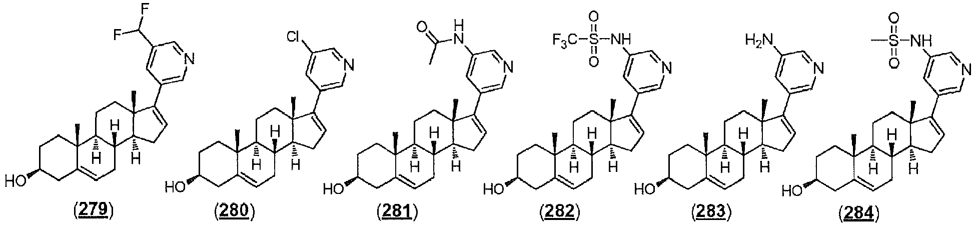

A mixture of Compound **(77)** (398 mg, 1 mmol), 5-fluoropyridin-3-ylboronic acid (143 mg, 1 mmol), Pd(PPh₃)₂Cl₂ (7 mg, 0.1 mmol), 2N Na₂CO₃ solution (2.5 mL) in dioxane (6 mL) was stirred at 80 °C for overnight. Then the mixture was poured into water, and extracted with ethyl acetate (100 mL x 3). The organic layer was washed with brine, dried, concentrated to get crude product. The crude product was purified by prep-TLC (ethyl acetate/ petroleum ether = 1/3, v/v) to afford **(274)** (31 mg, 10%). LC-MS (ESI) m/z: 368 (M+1)⁺; ¹H-NMR (400 MHz, CDCl₃): *δ* (ppm) 1.05 (s, 3H), 1.07 (s, 3H), 3.54 (m, 1H), 5.38 (m, 1H), 6.07 (m, 1H), 7.41 (m, 1H), 8.33 (s, 1H), 8.45 (s, 1H).

Using a synthetic procedure and conditions similar to the preparation of compound **(274),** replacing 5-fluoropyridin-3-ylboronic acid with various pyirdyl boronic acids, Compounds **(275 - 282)** were prepared.

Compound **(275)** (yield 9%): LC-MS (ESI) m/z: 375 (M+1)⁺; ¹H-NMR (400 MHz, CDCL3): *δ* (ppm) 1.04 (s, 3H), 1.07 (s, 3H), 3.54 (m, 1H), 5.39 (m, 1H), 6.12 (m, 1H), 7.89 (s, 1H), 8.72 (s, 1H), 8.80 (s, 1H).

Compound **(276)** (yield 38%): LC-MS (ESI) m/z: 368 (M+1)⁺; ¹H-NMR (400 MHz, CDCL3): *δ* (ppm) 1.05 (s, 3H), 1.07 (s, 3H), 3.54 (m, 1H), 5.38 (m, 1H), 6.07 (m, 1H), 7.41 (m, 1H), 8.33 (m, 1H), 8.45 (s, 1H)

Compound **(277)** (yield 28%): LC-MS (ESI) m/z: 382 (M+1)⁺; ¹H-NMR (400 MHz, CDCl₃-*d*1) δ (ppm) 1.01 (s, 3H), 1.07 (s, 3H), 2.30 (s, 3H), 3.54 (m, 1H), 5.40 (m, 1H), 6.03 (m, 1H), 7.46 (m, 1H), 7.86 (m, 1H).

Compound **(278)** (yield 28%): LC-MS (ESI) m/z: 382 (M+1)⁺; ¹H-NMR (400 MHz, CDCl₃-*d*1) *δ* (ppm) 1.01 (s, 3H), 1.07 (s, 3H), 2.27 (s, 3H), 3.55 (m, 1H), 5.39 (m, 1H), 5.92 (m, 1H), 7.55 (m, 1H), 7.99 (s, 1H).

Compound **(279)** (yield 28%): LC-MS (ESI) m/z: 400 (M+1)⁺; ¹H-NMR (400 MHz, CDCl₃) *δ* (ppm) 1.06 (s, 3H), 1.07 (s, 3H), 3.54 (m, 3H), 5.40 (m, 1H), 6.09 (m, 1H), 6.71 (t, *J=* 56 Hz, 1H), 7.79 (s, 1H), 8.60 (s, 1H), 8.73 (s, 1H).
Compound **(280)** (yield 73%): LCMS: 384 [M+1]⁺; ¹H NMR (CDCl₃, 400 MHz) major characteristic peaks: *δ* (ppm) 1.04 (s, 3H), 1.07 (s, 3H), 3.53 (m, 1H), 5.39 (s, *J* = 5.2 Hz, 1H), 6.05-6.06 (m, 1H), 7.62-7.63 (m, 1H), 8.41 (d, *J*= 2 Hz, 1H), 8.49 (d, *J*= 2 Hz, 1H).

Compound **(281)** (yield 21%): LC-MS (ESI) m/z: 407 (M+1)⁺; ¹H-NMR (400 MHz, CDCl₃): *δ* (ppm) 1.065(s, 3H), 1.075 (s, 3H), 2.238 (s, 3H), 3.491-3.570 (m, 1H), 5.398-5.408 (m, 1H), 6.072-6.075 (m, 1H), 8.308-8.430 (m, 3H).

Compound **(282)** (yield 8%): LC-MS (ESI) m/z: 497 (M+1)⁺. ¹H-NMR (400 MHz, MeOD) *δ* (ppm): 0.974-0.998 (m, 6H), 3.303-3.337 (m, 1H), 4.780-5.262 (m, 1H), 5.291-5.305 (m, 1H), 5.904 (s, 1H). 7.533 (s, 1H), 7.920 (s, 1H), 7.990 (s, 1H).

Compound **(283)** (yield 36%): LC-MS (ESI) m/z: 365 (M+1)⁺.¹H-NMR (400 MHz, MeOD) *δ* (ppm): 1.062-1.087 (m, 6H), 3.303-3.407 (m, 1H), 5.385-5.397 (m, 1H), 5.999-6.002 (m, 1H). 7.148 (s, 1H), 7.801-7.806 (m, 2H).

Compound (284**)** (yield 2%): LC-MS (ESI) m/z: 443 (M+H)⁺; ¹H-NMR (400 MHz, MeOD): *δ* (ppm) 1.09 (s, 3H), 1.09 (s, 3H), 2.95 (s, 3H), 5.39 (s, 1H), 6.08 (s, 1H), 7.69 (s, 1H), 8.14 (s, 1H), 8.19 (s, 1H).

### Example 208

### Preparation of Compounds (285) and (286)

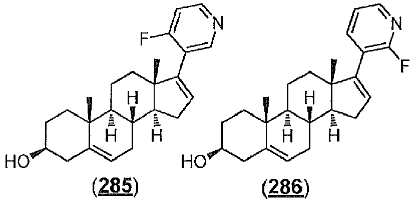

### Example 208A

**Preparation of Compound (285a)**

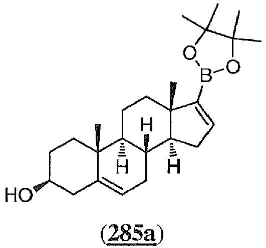

A mixture of Compound (**4**) (398 mg, 1.0 mmol), 4,4,4',4',5,5,5',5'-octamethyl-2,2'-bi(1,3,2-dioxaborolane) (381 mg, 1.5 mmol), anhydrous KOAc (294 mg, 3.0 mmol) and PdCl₂(dppf) (37 mg, 0.05 mmol) in dry DMSO (15 mL) was stirred at 80 °C under N₂ for 2 hrs. The mixture was cooled to room temperature and diluted with EtOAc (30 mL), then washed with H₂O (50 mL). The organic layer was separated. The aqueous layer was extracted with EtOAc (2 x 20 mL). The combined organic layers were washed with brine (20 mL), dried over Na₂SO₄, and concentrated to give a crude product. The crude product was purified by silica gel column chromatography (petroleum ether/EtOAc =10:1 to 5:1, v/v) to afford Compound **(285a)** (61 mg, 15%) as a white solid. LC-MS (ESI) m/z: 421 (M+23)⁺.

### Example 208B

### Preparation of Compounds (285) and (286)

A mixture of Compound **(285a)** (61 mg, 0.153 mmol), 3-bromo-4-fluoropyridine (27 mg, 0.153 mmol), PdCl₂(dppf) (6 mg) and K₂CO₃ (63 mg, 0.46 mmol) in dioxane (6 mL) and water (1.5 mL) was heated to 80 °C for 2 hrs. The mixture was diluted with water (20 mL), extracted with EtOAc (3 x 20 mL). The combined organic layers were washed with brine (30 mL), dried over Na₂SO₄, and concentrated to afford a crude product. The crude product was purified by silica gel column chromatography (petroleum ether/ EtOAc = 3:1, v/v) to afford Compound **(285)** (13 mg, 23%) as a white solid. LC-MS (ESI) m/z: 368 (M+1)⁺; ¹H-NMR (400 MHz, CDCl₃-*d*₁) *δ* (ppm) 1.00 (s, 3H), 1.06 (s, 3H), 3.54 (m, 1H), 5.39-5.40 (m, 1H), 6.02-6.03 (t, *J*=1.6 Hz, 1H), 7.00-7.04 (dd, *J=* 10, 5.6 Hz 1H), 8.42-8.45 (t, *J=* 6.4 Hz, 1H), 8.49-8.51 (d, *J*=10 Hz, 1H).

Using a synthetic procedure and condition similar to the preparation of compound **(285),** replacing 3-bromo-4-fluoropyridine with 3-bromo-2-fluoropyridine, Compound **(286)** was prepared. Compound **(286)** (yield 43%): LC-MS (ESI) m/z: 368 (M+H)⁺; ¹H-NMR (400 MHz, CDCL3): δ (ppm) 1.02 (s, 3H), 1.06 (s, 3H), 3.54 (m, 1H), 5.39 (m, 1H), 6.08 (s, 1H), 7.12 (m, 1H), 7.67 (m, 1H), 8.06 (m, 1H).

### Example 209

### Preparation of Compounds (287) and (288)

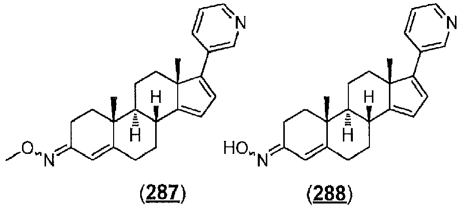

To a solution of Compound **(82)** (347 mg, 1 mmol) in EtOH (5 mL) was added pyridine (0.5 mL), NH₂OMeHCl (124 mg, 1.5 mol) successively. The mixture was stirred at room temperature for about 4 hrs. The mixture was poured into ice water and filtered. The collected solid was washed with MeOH (3 mL x 2) to afford Compound **(287)** (35 mg) as a white solid. LC-MS (m/z) 375 [M+H]⁺; ¹H NMR (CDCl₃, 400 MHz) major characteristic peaks: *δ* (ppm) 1.108 (s, 3H) 1.134 (s, 3H), 2.97 (m, 1H), 3.79 (s, 3H), 5.76 (s, 1H), 5.92 (s, 1H), 6.71 (s, 1H), 7.16 (m, 1H), 7.69 (m, 1H), 8.34 (s, 1H), 8.68 (s, 1H).

Using a synthetic procedure and condition similar to the preparation of compound **(287),** replacing NH₂OMeHCl with H₂OHHCl, Compound **(287)** was prepared. LC-MS (m/z) 361 [M+H]⁺; ¹H NMR (CDCl₃, 400 MHz) major characteristic peaks: δ (ppm) 1.20 (s, 3H) 1.24 (s, 3H), 3.08 (s, 1H), 6.00 (s, 1H), 6.79 (s, 1H), 7.25 (s, 1H), 7.79 (m, 1H), 8.43 (s, 1H), 8.76 (s, 1H).

### Example 210

### Preparation of Compound (289)

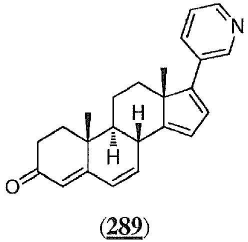

To a solution of Compound (**79**) (347 mg, 1 mmol) in DCM (10 mL) was added DMP (840 mg, 2 mmol). The mixture was stirred at room temperature overnight and diluted with water (20 mL). The organic layer was separated and the water phase was extracted with DCM (20 mL x 2).The combined organic layers were washed with water, dried, and concentrated. The residue was purified by prep-HPLC to afford **(289)** (30 mg) as a gray gum. LC-MS (m/z) 344[M+H]⁺ ; ¹H NMR (CDCl₃, 400 MHz) major characteristic peaks: *δ* (ppm) 1.18 (s, 6H), 3.12 (m, 1H), 5.68 (s, 1H), 6.03 (s, 1H), 6.21 (m, 1H), 6.39 (m, 1H), 6.73 (s, 1H), 7.18 (m, 1H), 7.69 (m, 1H), 8.37 (s, 1H), 8.69 (s, 1H).

### Example 211

### Preparation of Compound (290)

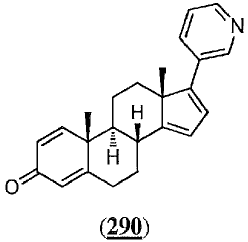

To a solution of Compound **(82)** (345 mg, 1 mmol) in 1,4-dioxane (10 mL) was added DDQ (224 mg, 1 mmol), manganese (IV) oxide (435 mg, 5 mmol) successively. The mixture was stirred at 100 °C for 2 hrs. The mixture was filtered, The solvent of the filtrate was evaporated and the residue was purified by prep-HPLC to afford **(290)** (34 mg) as a gray gum. LC-MS (m/z) 344 [M+H]⁺; ¹H NMR (CDCl₃, 400 MHz) major characteristic peaks: *δ* (ppm) 1.21 (s, 3H), 1.30 (s, 3H), 6.03 (m, 2H), 6.21 (m, 1H), 7.03 (m, 2H), 7.70 (m, 1H), 8.31 (s, 1H), 8.51 (m, 1H), 8.93 (s, 1H).

### Example 21.2

### Preparation of Compound (291) and (292)

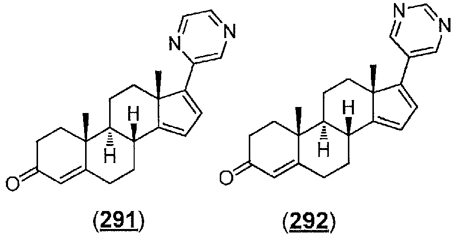

### Example 212A

### Preparation of Compound (291a) and (292a)

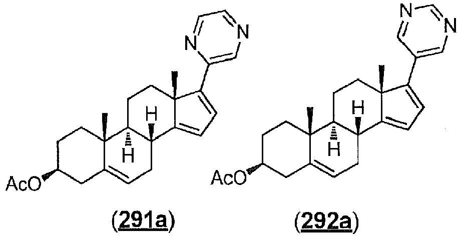

Pd(PPh₃)₄ (100 mg, 0.08 mmol, 0.1 equiv), 2-(tributylstannyl) pyrazine (520 mg, 1.4 mmol, 1.6 equiv), CuI (865 mg, 4.35 mmol, 5.0 equiv) and LiCl (221 mg, 5.22 mmol, 6.0 equiv) were added consecutively to a stirred solution of Compound **(77)** (400 mg, 0.87 mmol) in THF (15 mL) under N₂. The reaction was stirred and heated to 100°C (oil bath) for 2 hrs. After reaction, the reaction was cooled to room temperature and diluted with petroleum (50 mL). After filtration, the organic phase was concentrated under vacuum to yield a crude product. The crude product was purified by silica gel column chromatography (ethyl acetate in petroleum, 10% v/v) to afford Compound **(291a)** (99 mg, 29 %) as a yellow oil. LC-MS (*m*/*z*) 391 [M+H]⁺. Similarly, Compound **(292a)** was prepared under the same reaction conditions.

### Example 212B

### Preparation of Compound (291b) and (292b)

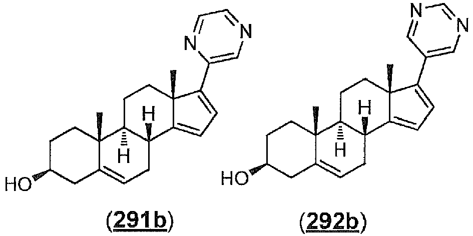

To a solution of Compound **(291a)** (99 mg, 0.25 mmol) in methanol (5 mL) was added KOH (14 mg, 0.25 mmol) under N₂. The mixture was stirred at room temperature for 1 hr. The solvent was evaporated, and the residue was extracted with ethyl acetate (50 mL x 2), washed with water, brine and dried over Na₂SO₄. The crude product was purified by prep-TLC (ethyl acetate in petroleum, 20% v/v) to give Compound **(291b)** (20 mg, 23%) as a yellow solid. LC-MS (m/z) 349 [M+H]⁺. ¹H-NMR (400 MHz, CHCl₃-d) major characteristic peaks: *δ* (ppm)1.09 (s, 3H), 1.25 (s, 3H), 3.48 (m, 1H), 5.41 (m, 1H), 6.00 (s, 1H), 7.14 (d, J = 2.0 Hz, 1H), 8.18 (s, 1H), 8.39 (s, 1H), 8.76 (s, 1H). Under the same condition, Compound **(292a)** was hydrolyzed to form **(292b).**

### Example 212C

### Preparation of Compound (291) and (292)

To a solution of Compound **(291b)** (15 mg, 0.043 mmol) in butanone (5 mL) and toluene (1 mL) was added Al(Oi-Pr)₃ (24%, 73 mg, 0.086 mmol) and stirred at 100 °C for 5 h. After reaction the mixture was cooled to room temperature and quenched with water (10 mL), then filtered through celite. The filtrate was extracted with DCM (30 mL x 2). The organic phase was washed with brine (30 mL x 2) and dried over Na₂SO₄, concentrated and purified by pre-TLC (ethyl acetate/ petroleum ether = 1:3, v/v) to give the product **(291)** (4.3 mg, 29%) as yellow solid. LCMS: 347 (M+H)⁺; ¹H NMR (CDCl₃, 400 MHz) major characteristic peaks: *δ* 1.33 (s, 3H), 1.34 (s, 3H), 5.79 (s, 1H), 6.06 (s, 1H), 7.19 (s, 1H), 8.25 (s, 1H), 8.45 (s, 1H), 8.82 (s, 1H).

Using a synthetic procedure and conditions similar to the preparation of compound **(291),** replacing **(291b)** with **(292b),** Compounds **(292)** was prepared (yield 73%) as a white solid. LC-MS (m/z): 347.2 [M+H]⁺; ¹H NMR (CDCl₃, 400 MHz) major characteristic peaks: *δ* (ppm) 1.24 (s, 3H), 1.33 (s, 3H), 5.80 (s, 1H), 6.05 (m, 1H), 6.90 (m, 1H), 8.85 (s, 2H), 9.03 (s, 1H).

### Example 213

### Preparation of Compound (293)

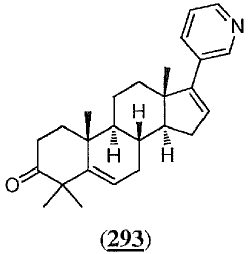

To a solution of (**50c**) (200 mg, 0.58 mmol) in *t*-BuOH (3.5 mL) was added *t*-BuOK (142 mg, 1.27 mmol) at 35 °C under N₂. After 30 min, CH₃I (0.1 mL, 1.62 mmol) was added dropwise. The reaction mixture was stirred for 2.5 hrs, then adjusted to pH = 6 with 1N HCl. Dichloromethane (20 mL) was added, washed with water (5 mL) and brine (5 mL). The organic layer was dried over anhydrous Na₂SO₄, and then evaporated to obtain a crude product, which was purified by column chromatography (petroleum ether/ ethyl acetate = 4/1 to 2 /1, v/v) to afford pure product **(293)** (90 mg, 41 %) as a white solid. LC-MS (ESI) m/z: 376 (M+H)⁺ ; ¹H-NMR (400 MHz, CDCl₃) δ (ppm) 0.92 (s, 3H), 1.05 (s, 3H), 1.25 (s, 3H), 1.26 (s, 3H), 5.60-5.61 (m, 1H), 6.01 (s, 1H); 7.21-7.24 (m, 1H), 7.61-7.67 (m, 1H), 8.45-8.47 (m, 1H), 8.62-8.63 (d, *J* = 2 Hz, 1H).

### Example 214

### Preparation of Compound (294)

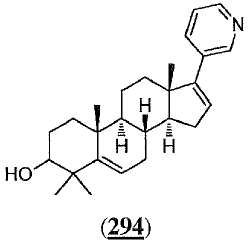

To a solution of **(293)** (70 mg, 0.19 mmol) in MeOH (5 mL) was added NaBH₄ (8 mg, 0.21 mmol) at 20 °C under N₂. The reaction mixture was stirred at 20 °C for 1 hr. Then the reaction was quenched with sat. NaHCO₃ (1.5 mL) solution. The mixture was diluted with 5 mL of water and then extracted with dichloromethane (2 x 15 mL). The combined organic layers were washed with brine (25 mL) and dried over Na₂SO₄, evaporated to obtain a crude product, which was purified by column chromatography (petroleum ether/ ethyl acetate = 3/1 to 1/1, v/v) to afford desired product (**294**) (45 mg, 64%) as a white solid. LC-MS (ESI) m/z: 378 (M+H)⁺ ; ¹H-NMR (400 MHz, CDCl₃) δ: (ppm) 1.03 (s, 3H), 1.09 (s, 3H), 1.14 (s, 3H), 1.17 (s, 3H), 3.23-3.26 (m, 1H), 5.59-5.61 (s, 1H), 5.99-6.00 (s, 1H); 7.19-7.23 (m, 1H), 7.63-7.66 (d, *J* = 8 Hz, 1H), 8.45-8.46 (m, 1H).

### Example 215

### Preparation of Compounds (295), (296) and (297)

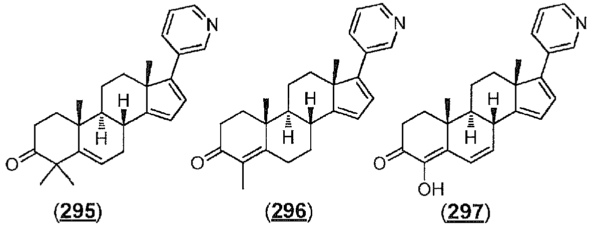

To a solution of (**82**) (200 mg, 0.58 mmol) in *t*-BuOH (3.5 mL) was added *t*-BuOK (142 mg, 1.27 mmol) at 35 °C under N₂. After 30 min, CH₃I (0.1 mL, 1.62 mmol) was added dropwise. The reaction mixture was stirred for 2.5 hours, then adjusted with 1N HCl to pH = 6. Dichloromethane (20 mL) was added, washed with water (5 mL), and followed by brine (5 mL). The organic layer was dried by anhydrous Na₂SO₄ and evaporated to afford a crude product, which was purified by column chromatography (petroleum ether/ethyl acetate = 4 /1 to 2 /1, v/v) to afford Compound (**295**) (80 mg, 36%), Compound (**296**) (80 mg, 36%), and Compound (**297**) (40 mg, 18%). For Compound (**295**): LC-MS (ESI) m/z: 374 (M+H)⁺; Compound (**296**): LC-MS (ESI) m/z: 360 (M+H)⁺; ¹H NMR (CDCl₃, 400 MHz) major characteristic peaks: *δ* (ppm) 1.22 (s, 3H), 1.31 (s, 3H), 2.90 (m, 1H), 6.01 (s, 1H), 6.79 (s, 1H), 7.24 (m, 1H), 7.77 (m, 1H), 8.43 (s, 1H), 8.75 (s, 1H); Compound **(297):** LC-MS (ESI) m/z: 360 (M+H)⁺; ¹H NMR (CDCl₃, 400 MHz) major characteristic peaks: *δ* (ppm) 1.24 (s, 3H), 1.25 (s, 3H), 2.05 (m, 1H), 3.17 (m, 1H), 6.11 (s, 1H), 6.27 (s, 1H), 6.82 (m, 2H), 7.24 (m, 1H), 7.77 (m, 1H), 8.44 (m, 1H), 8.76 (s, 1H).

### Example 216

### Preparation of Compounds (298) and (299)

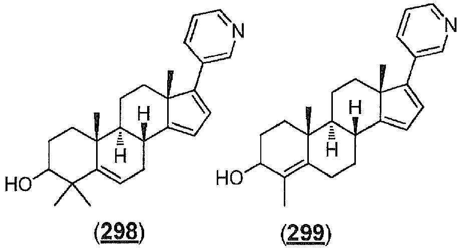

To a solution of Compound (**295**) (70 mg, 0.19 mmol) in MeOH (5 mL) was added NaBH₄ (8 mg, 0.21 mmol) at 20 °C under N₂. The reaction mixture was stirred at 20 °C for 1 hr. The reaction was quenched with sat NaHCO₃ (1.5 mL) solution and diluted with 5 mL of water. it was then extracted with dichloromethane (2 x 15 mL). The combined organic layers were washed with brine (25 mL) and dried over Na₂SO₄, and evaporated to yield a crude product, which was purified by column chromatography (petroleum ether/ ethyl acetate = 3/1 to 1/1, v/v) to afford desired product (**298**) (35 mg, 53%) as a white solid. LC-MS (ESI) m/z: 376 (M+H)⁺ ; ¹H-NMR (400 MHz, CDCl₃) *δ*: (ppm) 1.12 (s, 3H), 1.13 (s, 3H), 1.20 (s, 3H), 1.23 (s, 3H), 2.59 (m, 1H), 3.27 (m, 1H), 5.70 (s, 1H), 6.03 (s, 1H); 6.82 (s, 1H), 7.23 (m, 1H), 7.70 (d, *J* = 8 Hz, 1H), 8.42 (m, 1H), 8.77 (s, 1H).

Using a synthetic procedure and conditions similar to the preparation of compound (**298**), replacing (**295**) with (**296**), Compounds (**299**) was prepared (yield 38%) as a white solid. LC-MS (ESI) m/z: 362 (M+H)⁺; ¹H-NMR (400 MHz, CDCl₃) *δ*: (ppm) 1.11 (s, 3H), 1.13 (s, 3H), 1.12 (s, 3H), 3.93 (m, 1H), 5.90 (s, 1H), 6.72 (s, 1H); 7.17 (m, 1H), 7.68 (m, 1H), 8.34 (m, 1H), 8.68 (s, 1H).

### Example 217

### Preparation of Compound (300)

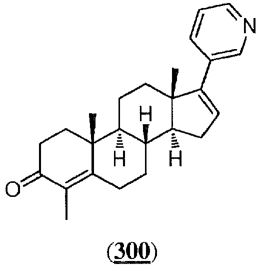

To a solution of (**50c**) (1.165 g, 3.35 mmol) in *t*-BuOH (135 mL) was added *t*-BuOK (3 g, 26.8 mmol) at 35 °C under N₂. After 30 min, CH₃I (0.4 mL, 6.7 mmol) in *t*-BuOH (13.5 mL) was added dropwise. The reaction mixture was stirred for 3 hours, then adjusted with aqueous HCl solution (IN) to pH=7, extracted by Dichloromethane (2 x 150 mL), washed with water (20 mL), followed by brine (25 mL). The organic layer was dried over anhydrous Na₂SO₄, and evaporated to yield a crude product, which was purified by column chromatography (petroleum ether/ethyl acetate = 8/1 to 2/ 1, v/v) to afford pure product (**300**) (260 mg, 21%) as a white solid. LC-MS (ESI) m/z: 362 (M+H)⁺; ¹H-NMR (400 MHz, DMSO-*d*₆) *δ* (ppm) 1.04 (s, 3H), 1.19 (s, 3H), 1.69 (s, 3H), 6.12 (s, 1H), 7.32-7.36 (m, 1H), 7.75-7.77 (d, *J* = 8 Hz, 1H), 8.45 (brs, 1H), 8.60 (brs, 1H).

### Example 218

### Preparation of Compound (301)

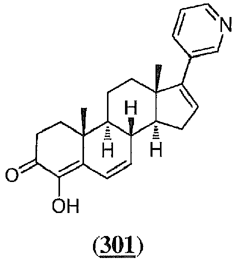

To a solution of (**50c**) (173 mg, 0.5 mmol) in *t*-BuOH (20 mL) was added *t*-BuOK (449 mg, 4 mmol) at 60 °C under O₂. After 2 hrs, a solution of CH₃I (0.06 mL, 1 mmol) in 2 mL of t-BuOH was added dropwise. The reaction mixture was stirred for 2.5 hours at 20 °C, then adjusted with 1N HCl to pH=6. The mixture was diluted with dichloromethane (40 mL) and washed with water (5 mL), followed with brine (5 mL). The organic layer was dried with anhydrous Na₂SO₄, and evaporated to yield a crude product, which was purified by column chromatography eluted with (petroleum ether/ethyl acetate =10/1 ∼ 2 /1, v/v) to afford pure product (**301**) (50 mg, 28%) as a white solid. LC-MS (ESI) m/z: 362 (M+H)⁺; ¹H-NMR (400 MHz, CDCl₃) δ (ppm) 1.10 (s, 3H), 1.15 (s, 3H), 6.02-6.04 (m, 1H), 6.09-6.13 (dd, *J* = 2.0, 9.6 Hz, 1H), 6.27 (brs, 1H), 6.68-6.72 (dd, *J* = 2.8, 9.6 Hz, 1H), 7.23-7.25 (m, 1H), 7.65-7.68 (m, 1H), 8.47-8.49 (dd, *J* = 1.2, 4.8 Hz, 1H), 8.62-8.63 (m, 1H).

### Example 219

### Preparation of Compound (302)

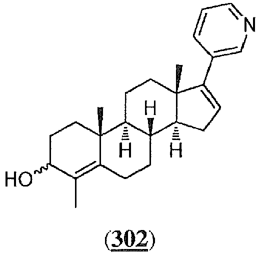

To a solution of (**300**) (50 mg, 0.14 mmol) in MeOH (5 mL) was added NaBH₄ (6 mg, 0.152 mmol) and CeCl₃.7H₂O (3.4 mg, 0.014 mmol) at 25 °C under N₂. The reaction mixture was stirred for 1 hour at 25 °C, then saturated NaHCO₃ (10 mL) was added to quench the reaction, followed by water (5 mL), extracted with dichloromethane (2 x 10 mL). The combined organic layers were dried by anhydrous Na₂SO₄, and then evaporated to yield a crude product, which was purified by column chromatography (petroleum ether/ ethyl acetate = 3/1 to 1/1, v/v) to afford pure product (**302**) (14 mg, 28%) as a white solid. LC-MS (ESI) m/z: 364 (M+H)⁺; ¹H-NMR (400 MHz, DMSO-*d*₆) *δ* (ppm) 1.01 (s, 3H), 1.04 (s, 3H), 1.61 (s, 3H), 3.76 (s, 1H), 4.47-4.49 (d, *J* = 6.4 Hz, 1H), , 6.10 (s, 1H), 7.31-7.34 (m, 1H), 7.74-7.76 (d, *J* = 7.2, 1H), 8.43 (s, 1H), 8.58 (s, 1H).

### Example 220

### Preparation of Compound (303)

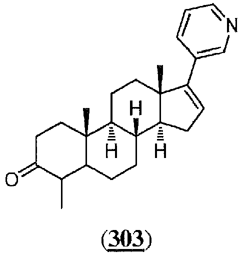

### Example 220A

### Preparation of Compound (303a)

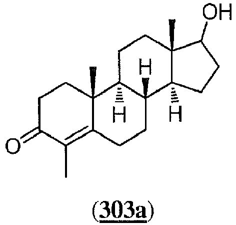

To a solution of (10R,13S)-17-hydroxy-10,13-dimethyl-6,7,8,9,10,11,12,13,14,15,16,17-dodecahydro-1H-cyclopenta[a]phenanthren-3(2H)-one (2.88 g, 0.01 mol) in *t*-BuOH (100 mL) was added *t*-BuOK (2.24 g, 0.02 mmol) under N₂ and the mixture was stirred for 1 hr at 45 °C, then a solution of MeI (1 mL) in *t*-BuOH (5 mL) was added to above mixture at 45 °C. The reaction was stirred at 40 °C for about 4 hrs. The mixture was cooled to room temperature and poured into ice water. It was extracted with ethyl acetate (100 mL x 3). The organic layer was washed with water, brine, dried, concentrated, and purified with column chromatography (petroleum ether/ethyl acetate = 6/1 ∼ 2/1, v/v) to afford compound (**26a**) (1.5 g, 52%) as a white solid. LCMS: 303[M+1] ⁺; ¹H NMR (CDCl₃, 400 MHz) major characteristic peaks: *δ* 0.72(s, 3H) 1.12 (s, 3H), 1.70 (s, 3H), 2.31 (m, 1H), 2.69 (m, 1H).

### Example 220B

### Preparation of Compound (303b)

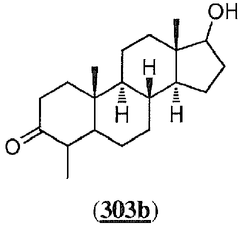

A mixture of Compound (**303a**) (3.0 g, 0.01 mmol), Pd/C (10%, 300 mg) in MeOH (60 mL) was stirred at room temperature overnight. Then it was filtered to remove Pd/C and the solvent was evaporated to obtain Compound (**303b**) (3 g, 100%) as a white solid. LCMS: 305 [M+1]⁺; ¹H NMR (CDCl₃, 400 MHz) major characteristic peaks: *δ* (ppm) 0.751 (s, 1H), 0.763 (s, 2H), 0.972 (d, *J* = 6.4 Hz, 2H), 1.079 (s, 1H), 1.086 (s, 2H), 1.117 (d, *J* = 6.4 Hz, 1H), 3.64 (m, 1H).

### Example 220C

### Preparation of Compound (303c)

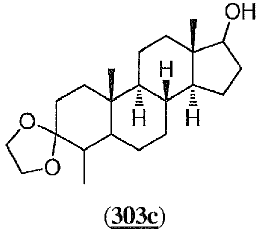

A solution of Compound (**303b**) (3.5 g, 0.01 mol), CH(OEt)₃ (4 mL), ethane-1,2-diol (4 mL) and *p*-TsOH (308 mg, 1.62 mmol) in THF (10 mL) was stirred at room temperature for 2 h. Then it was adjusted to pH = 8 with Et₃N and diluted with ethyl acetate (200 mL). The organic layer was washed with water, brine, dried with anhydrous Na₂SO₄ and evaporated to obtain compound **(303c)** (3.4 g, 83%) as a white solid. LCMS: 349[M+1]⁺.

### Example 220D

### Preparation of Compound (303d)

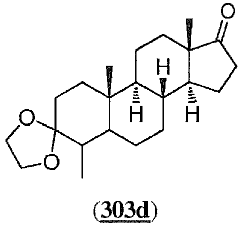

To a solution of Compound **(303c)** (2.10 g, 6.07 mmol) in DCM (20 mL) was added DMP (3.09 g, 7.28 mmol) as 0 °C. Then the mixture was stirred at this temperature for 1 hr. Then it was quenched with ethanol and filtered to remove solid. The filtrate was diluted with ethyl acetate (150 mL), washed with water and brine, dried with anhydrous Na₂SO₄ and purified by column chromatography (petroleum ether/ethyl acetate = 8/1, v/v) to obtain compound (**303d**) (1.87 g, 90%) as a white solid. LCMS: 347[M+1]⁺.

### Example 220E

### Preparation of Compound (303e)

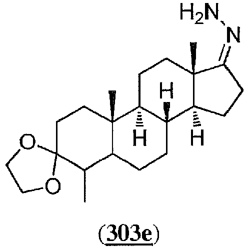

To a suspension of Compound (**303d**) (1.87 g, 5.44 mmol) in EtOH (20 mL) was added NH₂NH₂ aqueous (80%, 8 mL) and stirred at room temperature for 2 hrs. The mixture was poured into ice water. The solid formed was filtered to obtain compound (**303e**) (1.60 g, 82%) as a white solid. LCMS: 361[M+1]⁺.

### Example 220F

### Preparation of Compound (303f)

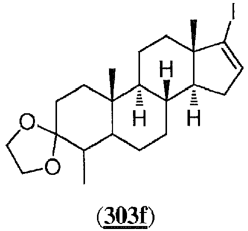

To a solution of Compound (**303e**) (1.60 g, 4.47 mmol) and Et₃N (1 mL) in DCM (10 mL) was added dropwise the solution of I₂ (1.70 g, 6.70 mmol) in THF (2 mL) at 0 °C. The solution was stirred at 0 °C for 2 hrs. Then it was quenched with Na₂SO₃ solution to destroy excess I₂. The mixture was diluted with ethyl acetate (150 mL), washed with water and brine, dried with anhydrous Na₂SO₄, and purified by column chromatography (petroleum ether: ethyl acetate = 15/1, v/v) to obtain compound (**303f**) (1.40 g, 69%) as a white solid. LCMS: 457[M+1] ⁺.

### Example 220G

### Preparation of Compound (303g)

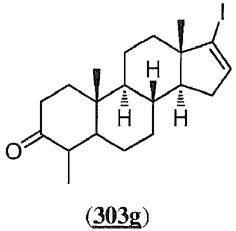

To a solution of Compound (**303f**) (1.20 g, 2.64 mmol) in THF (20 mL) was added con. HCl (1 mL) and stirred at room temperature for 2 hrs. Then it was diluted with ethyl acetate (150 mL), washed with water and brine, dried with anhydrous Na₂SO₄, and purified with silica chromatography (petroleum ether/ ethyl acetate = 8/1, v/v) to afford compound (**303g**) (900 mg, 83%) as a white solid. LCMS: 413[M+1]⁺.

### Example 220H

### Preparation of Compound (303)

The suspension of Compound (**303g**) (520 mg, 1.27 mmol), 5-methylpyridin-3-ylboronic acid (234 mg, 1.90 mmol), PdCl₂(PPh₃)₂ (91 mg, 0.13 mmol) and K₂CO₃ (512 mg, 3.71 mmol) in dioxane/H2O (20/4 mL) was stirred at 80 °C for overnight. Then it was diluted with ethyl acetate (150 mL), washed with water and brine, dried with anhydrous Na₂SO₄ and purified by prep-HPLC to afford Compound (**303**) (420 mg, 92%) as a white solid. LCMS: 364[M+1] ⁺; ¹H NMR (CDCl₃, 400 MHz) major characteristic peaks: *δ* (ppm) 0.92 (s, 3H), 0.95 (s, 3H), 1.05 (s, 3H), 5.90 (s, 1H), 7.14 (m, 1H), 7.55 (m, 1H), 8.38 (m, 1H), 8.54 (s, 1H).

### Example 221

### Preparation of Compound (304)

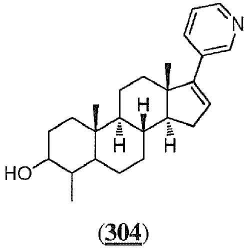

To a solution of Compound (**303**) (70 mg, 0.19 mmol) in MeOH (5 mL) was added NaBH₄ (8 mg, 0.21 mmol) at 20 °C under N₂. The reaction mixture was stirred at 20 °C for 1 hr. Then the reaction was quenched with sat. NaHCO₃ (1.5 mL). the mixture was diluted with 5 mL of water and extracted with dichloromethane (2 x 15 mL). The combined organic layers were washed with brine (25 mL), dried over Na₂SO₄, and evaporated to yield a crude product, which was purified by column chromatography (petroleum ether/ ethyl acetate = 3/1 to 1/1, v/v) to afford desired product (**304**) (35 mg, 53%) as a white solid. LC-MS (ESI) m/z: 366(M+H)⁺; ¹H-NMR (400 MHz, CDCl₃) *δ*: (ppm) 0.81 (s, 3H), 0.91 (s, 3H), 0.93 (s, 3H), 2.18 (m, 1H), 3.02 (m, 1H), 5.91 (s, 1H), 7.14 (m, 1H), 7.57 (d, *J* = 8 Hz, 1H), 8.38 (m, 1H), 8.54 (s, 1H).

### Example 222

### Preparation of Compounds (305), (306), (307), and (308)

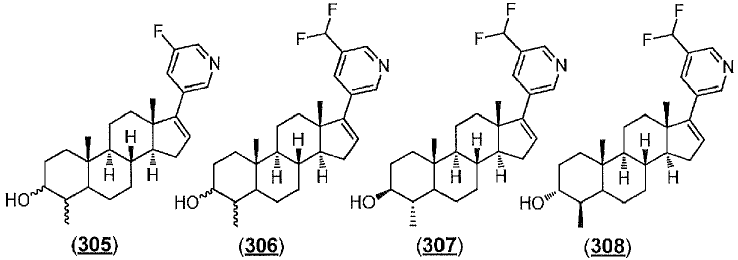

### Example 222A

### Preparation of Compound (305a)

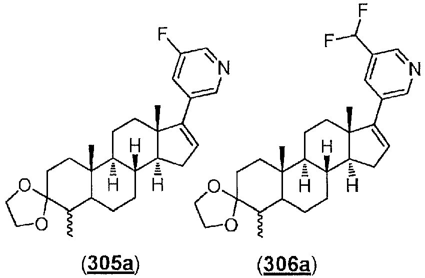

To a solution of Compound (**303f**) (400 mg, 0.88 mmol) in 1,4-dioxane (10 mL) and H₂O (2 mL) was added 5-fluoropyridin-3-ylboronic acid (183 mg, 1.3 mmol), K₂CO₃ (486 mg,3.52 mmol) and Pd(PPh₃)₂Cl₂ (62 mg, 0.88 mmol) at N₂ atmosphere and stirred at 80°C for 2-3 hrs. The mixture was cooled to room temperature, quenched with aq. NH₄Cl (10 mL), and extracted with EtOAc (3 x 15 mL). The organic layer was washed with brine (10 mL), dried over Na₂SO₄ and concentrated to give a crude product. The crude product was purified by column chromatography (petroleum ether/ethyl acetate = 30/1-15/1, v/v) to afford Compound (**305a**) (152 mg, 41%) as a white solid. LCMS: m/z: 426.7(M+1)⁺. ¹H-NMR (400 MHz, CDCl₃) : *δ* 0.857 (s, 3H), 0.917-0.925 (d, *J* = 7.2 Hz, 3H), 0.993 (s, 3H), 2.217-2.281 (m, 1H), 3.916-4.005 (m, 4H), 6.035 (m, 1H), 7.342-7.372 (d, *J* = 12 Hz, 1H), 8.309 (s, 1H), 8.429 (s, 1H).

Using a synthetic procedure and conditions similar to the preparation of compound (**305a**), replacing 5-fluoropyridin-3-ylboronic acid with (5-(difluoromethyl)pyridin-3-yl)boronic acid, Compound (**306a**) was prepared (yield 58%) as a white solid. LCMS: m/z: 426.7(M+1)⁺. ¹H-NMR (400 MHz, CDCl₃) : *δ* (ppm) 0.811 (s, 3H), 0.827-0.9898 (m, 3H), 1.014 (s, 3H), 3.930-3.951 (m, 4H), 6.062 (m, 1H), 6.558-6.837 (t, *J* = 56 Hz, 1H), 7.769 (s, 1H), 8.585 (s, 1H), 8.718 (s, 1H).

### Example 222B

### Preparation of Compounds (305b) and (306b)

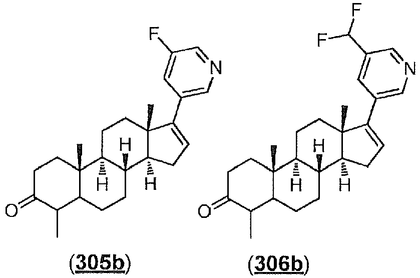

To a solution of Compound (**305a**) (152 mg, 0.36 mmol) in THF (4 mL) was added concentrated HCl (0.5 mL), then stirred at room temperature for 5 hrs and quenched with sat. aq. NaHCO₃ (5 mL) until pH7∼8. The mixture was extracted with EtOAc (3 x 8 mL), dried over Na₂SO₄, concentrated, and purified by column chromatography (petroleum ether/ethyl acetate = 15/1-5/1, v/v) to give Compound (**305b**) (122 mg, 89%) as a white solid. LCMS: m/z: 382.3(M+1)⁺. ¹H-NMR (400 MHz, CDCl₃) : *δ* (ppm) 0.857 (s, 3H), 0.917-0.925 (d, *J* = 7.2 Hz, 3H), 0.993 (s, 3H), 2.217-2.281 (m, 1H), 6.035 (m, 1H), 7.342-7.372 (d, *J* = 12 Hz, 1H), 8.309 (s, 1H), 8.429 (s, 1H).

Using a synthetic procedure and conditions similar to the preparation of compound (**305b**), replacing **(305a)** with (**306a**), Compound (**306b**) was prepared (yield 89%) as a white solid. LCMS: m/z: 414.3(M+1)⁺. ¹H-NMR (400 MHz, CDCl₃) : *δ* (ppm) 0.811 (s, 3H), 0.827-0.9898 (m, 3H), 1.014 (s, 3H), 6.062 (m, 1H), 6.558-6.837 (t, *J* = 56 Hz, 1H), 7.769 (s, 1H), 8.585 (s, 1H), 8.718 (s, 1H).

### Example 222C

### Preparation of Compounds (305), (306), (307), and (308)

To a solution of Compound (**305b**) (122 mg, 0.32 mmol, crude) in MeOH (5 mL) was added NaBH₄ (18 mg, 0.48 mmol) at room temperature and stirred for 1 hr. The mixture was quenched with sat. aq. NH₄Cl (5 mL) and extracted with EtOAc (3 x 8 mL). The organic phase was washed with brine, dried over Na₂SO₄, concentrated, purified by prep-TLC (petroleum ether/ethyl acetate = 2/1, v/v) to afford Compound (**305**) (23 mg) as a white solid. LCMS: m/z: 384.7(M+1)⁺. ¹H-NMR (400 MHz, CDCl₃) : *δ* (ppm) 0.857 (s, 3H), 0.917-0.925 (d, *J* = 7.2 Hz, 3H), 0.993 (s, 3H), 2.217-2.281 (m, 1H), 3.750 (m, 1H), 6.035 (m, 1H), 7.342-7.372 (d, *J* = 12 Hz, 1H), 8.309 (s, 1H), 8.429 (s, 1H).

Using a synthetic procedure and conditions similar to the preparation of compound (**305**), replacing (**305b**) with (**306b**), Compounds (**306 - 308)** were isolated.

Compound (**306**): LCMS: m/z: 416.3(M+1)⁺. ¹H-NMR (400 MHz, CDCl₃) : *δ* (ppm) 0.950-0.967 (d, *J* = 6.8 Hz, 3H), 1.017 (s, 6H), 3.820 (m, 1H), 6.064 (m, 1H), 6.564-6.843 (t, *J* = 56 Hz, 1H), 7.772 (s, 1H), 8.589 (s, 1H), 8.719 (s, 1H).

Compound **(307):** LCMS: m/z: 416.3(M+1)⁺. ¹H-NMR (400 MHz, CDCl₃) : *δ* (ppm) 0.869 (s, 1H), 0.889 (s, 2H), 0.927-0.944 (d, *J* = 6.8 Hz, 1H), 0.963-0.979 (d, *J* = 6.4 Hz, 2H), 1.017 (s, 3H), 3.067-3.132 (m, 0.67H), 3.760 (m, 0.36H), 6.064 (m, 1H), 6.562-6.841 (t, *J* = 56 Hz, 1H), 7.776 (s, 1H), 8.588 (s, 1H), 8.722 (s, 1H).

Compound **(308):** LCMS: m/z: 416.3(M+1)⁺. ¹H-NMR (400 MHz, CDCl₃) : *δ* (ppm) 0.983, 1.007 (s, 9H), 3.174 (m, 1H), 6.059 (m, 1H), 6.564-6.842 (t, *J* = 56 Hz, 1H), 7.769 (s, 1H), 8.588 (s, 1H), 8.717 (s, 1H).

### Example 223

### Preparation of Compound (309)

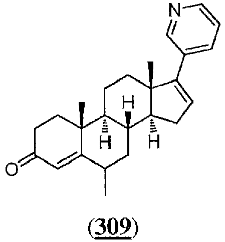

### Example 223A

### Preparation of Compound (309a)

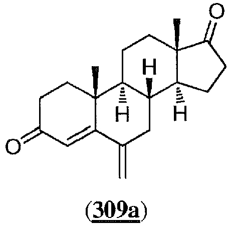

To a solution of (10R,13S)-10,13-dimethyl-1,7,8,9,10,11,12,13,15,16-decahydro-2H-cyclopenta[a]phenanthrene-3,17(6H,14H)-dione (25.0 g, 0.087 mol) in dioxane (125 mL) and triethylorthoformate (30 mL), was added *p*-toluenesulphonic acid (1.25 g, 6.58 mmol) at 45 °C. After 1 hour, N-methylaniline (10 mL) and aqueous formaldehyde (37%, 11.25 mL) were added at 45 °C. The mixture was stirred overnight at the same temperature. After the completion of the reaction, the mixture was cooled to 10 °C and acidified with conc. HCl (100 mL) and stirred for 1 hour. Then it was diluted with ethyl acetate (500 mL), washed with water and brine, and purified by silica chromatography (petroleum ether/ethyl acetate = 8/1, v/v) to give Compound (**309a**) (12.10 g, 47%) as a yellow solid. LCMS: 299[M+1]⁺; ¹H NMR (CDCl₃, 400 MHz) major characteristic peaks: *δ* (ppm) 0.92 (s, 3H), 1.12 (s, 3H), 4.99 (t, *J* = 2.0 Hz, 1H), 5.10 (t, *J* = 2.0 Hz, 1H), 5.93 (s, 1H).

### Example 223B

### Preparation of Compound (309b)

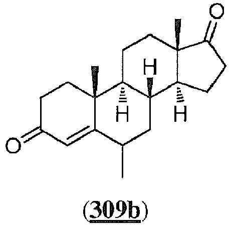

An suspension of Compound (**309a**) (7.00 g, 23.49 mmol), Pd/C (10%, 1.00 g), and cyclohexene (10 mL) in EtOH (30 mL) was stirred at 80 °C for 4 hrs. After cooled down to room temperature, the mixture was filtered to remove Pd/C. The filtrate was evaporated to dryness to yield Compound (**309b**) (5.10 g, 72%) as a white solid. LCMS: 301[M+1]⁺; ¹H NMR (CDCl₃, 400 MHz) major characteristic peaks: *δ* (ppm) 0.92 (s, 3H), 1.10 (d, *J* = 6.4 Hz, 3H), 1.21 (s, 3H), 5.81 (s, 1H).

### Example 223C

### Preparation of Compound (309c)

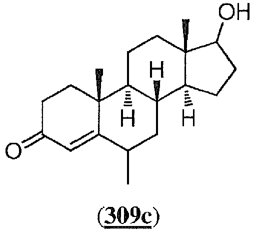

To a solution of Compound (**309b**) (5.10 g, 17.00 mmol) in DCM (10 mL) was added dropwise a solution of NaBH₄ (775 mg, 20.04 mmol) in EtOH (10 mL) at 0 °C. The mixture was stirred at 0 °C for 1 hr. After the completion of the reaction, it was quenched with acetone (2 mL) and diluted with ethyl acetate (200 mL). The organic layer was washed with water and brine, dried with anhydrous Na₂SO₄, and evaporated to dryness to afford Compound (**309c**) (4.90 g, 95%) as a white solid. LCMS: 303[M+1]⁺; ¹H NMR (CDCl₃, 400 MHz) major characteristic peaks: *δ* (ppm) 0.79 (s, 3H), 1.06 (d, *J* = 6.4 Hz, 3H), 1.19 (s, 3H), 3.64 (t, *J* = 8.4 Hz, 1H), 5.78 (s, 1H).

### Example 223D

### Preparation of Compound (309d)

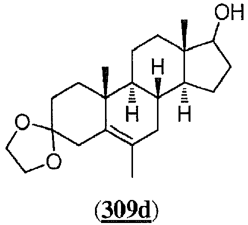

A solution of Compound (**309c**) (4.90 g, 16.22 mmol), CH(OEt)₃ (7.20 g, 48.68 mmol), ethane-1,2-diol (10 mL) andp-TsOH (308 mg, 1.62 mmol) in THF (10 mL) was stirred at room temperature for 2 hrs. Then it was adjusted to pH = 8 with Et₃N and diluted with ethyl acetate (200 mL), washed with water and brine, dried with anhydrous Na₂SO₄, and evaporated to dryness to obtain Compound (**309d**) (3.50 g, 62%) as a white solid. LCMS: 347[M+1]⁺; ¹H NMR (CDCl₃, 400 MHz) major characteristic peaks: *δ* (ppm) 0.76 (s, 3H), 1.02 (s, 3H), 1.16 (s, 3H), 3.92-4.00 (m, 4H).

### Example 223E

### Preparation of Compound (309e)

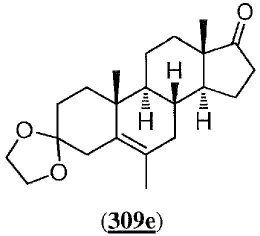

To a solution of Compound (**309d**) (2.10 g, 6.07 mmol) in DCM (20 mL) was added DMP (3.09 g, 7.28 mmol) as 0 °C. Then the mixture was stirred at this temperature for 1 hr. The reaction was quenched with ethanol and filtered to remove solid. The filtrate was diluted with ethyl acetate (150 mL), washed with water and brine, dried with anhydrous Na₂SO₄, and purified by silica chromatography (petroleum ether/ethyl acetate = 8/1, v/v) to afford Compound (**309e**) (1.87 g, 90%) as a white solid. LCMS: 345[M+1]⁺; ¹H NMR (CDCl₃, 400 MHz) major characteristic peaks: *δ* (ppm) 0.88 (s, 3H), 1.03 (s, 3H), 1.65 (s, 3H), 3.92-4.00 (m, 4H).

### Example 223F

### Preparation of Compound (309f)

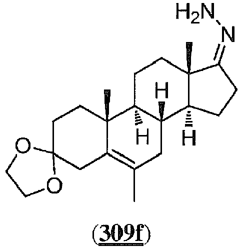

To a suspension of Compound (**309e**) (1.87 g, 5.44 mmol) in EtOH (20 mL) was added NH₂NH₂ aqueous (80%, 8 mL) and stirred at room temperature for 2 hrs. After the completion of the reaction, the mixture was poured into ice water. The precipitate was filtered to give Compound (**309f**) (1.60 g, 82%) as a white solid. LCMS: 359[M+1]⁺; ¹H NMR (CDCl₃, 400 MHz) major characteristic peaks: *δ* (ppm) 0.87 (s, 3H), 1.03 (s, 3H), 1.64 (s, 3H), 3.92-4.00 (m, 4H), 4.77 (s, 2H).

### Example 223G

### Preparation of Compound (309g)

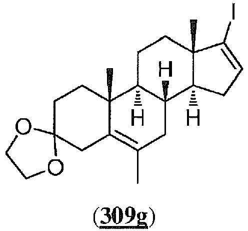

To a solution of Compound (**309f**) (1.60 g, 4.47 mmol) and Et₃N (1 mL) in DCM (10 mL) was added dropwise a solution of I₂ (1.70 g, 6.70 mmol) in THF (2 mL) at 0 °C. The solution was stirred at 0 °C for 2 hrs. The reaction was quenched with Na₂SO₃ solution to destroy the excess I₂. The mixture was diluted with ethyl acetate (150 mL), washed with water and brine, dried with anhydrous Na₂SO₄, and purified with silica chromatography (petroleum ether/ethyl acetate = 15/1, v/v) to obtain Compound (**309g**) (1.40 g, 69%) as a white solid. LCMS: 455[M+1]⁺; ¹H NMR (CDCl₃, 400 MHz) major characteristic peaks: *δ* (ppm) 0.75 (s, 3H), 1.04(s, 3H), 1.63 (s, 3H), 3.92-4.00 (m, 4H), 6.15 (s, 1H).

### Example 223H

### Preparation of Compound (309h)

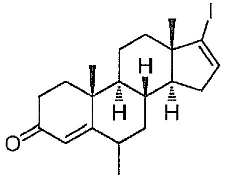

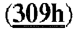

To a solution of Compound (**309g**) (1.20 g, 2.64 mmol) in THF (20 mL) was added con. HCl (1 mL) and stirred at room temperature for 2 hrs. The mixture was diluted with ethyl acetate (150 mL), washed with water and brine, dried with anhydrous Na₂SO₄, and purified with silica chromatography eluted (petroleum ether/ethyl acetate = 8/1, v/v) to give Compound (**309h**) (900 mg, 83%) as a white solid. LCMS: 411[M+1]⁺; ¹H NMR (CDCl₃, 400 MHz) major characteristic peaks: *δ* (ppm) 0.79 (s, 3H), 1.09 (d, *J* = 6.8 Hz, 3H), 1.56 (s, 3H), 5.80 (s, 1H), 6.14(s, 1H).

### Example 223I

### Preparation of Compound (309)

An suspension of Compound (**309h**) (520 mg, 1.27 mmol), 5-methylpyridin-3-ylboronic acid (234 mg, 1.90 mmol), PdCl₂(PPh₃)₂ (91 mg, 0.13 mmol) and K₂CO₃ (512 mg, 3.71 mmol) in dioxane/H2O (20/4 mL) was stirred at 80 °C overnight. Then it was diluted with ethyl acetate (150 mL), washed with water and brine, dried with anhydrous Na₂SO₄, and purified by prep-HPLC to afford Compound (**309**) (420 mg, 92%) as a white solid. LCMS: 362[M+1] ⁺; ¹H NMR (CDCl₃, 400 MHz) major characteristic peaks: *δ* (ppm) 1.07 (s, 3H), 1.11 (d, *J* = 6.8 Hz, 3H), 1.24 (s, 3H), 5.82 (s, 1H), 6.00 (s, 1H), 7.21-7.26 (m, 1H), 7.63-7.66 (m, 1H), 8.46-8.48 (m, 1H), 8.62 (s, 1H).

### Example 224

### Preparation of Compound (310)

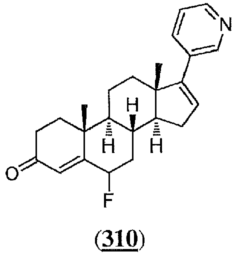

### Example 224A

### Preparation of Compound (310a)

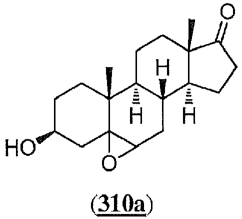

To a solution of (3S,10R,13S)-3-hydroxy-10,13-dimethyl-1,3,4,7,8,9,10,11,12,13,15,16-dodecahydro-2H-cyclopenta[a]phenanthren-17(14H)-one (5.0 g, 17.3 mmol) in DCM (100 mL) was added a solution of *m*-CPBA (4.5 g, 26.0 mmol) in DCM (100 mL) at 0 °C, and the mixture was stirred for 3 hrs at 0 °C. The mixture was filtered and concentrated to give a crude product. The crude product was purified by flash column chromatography (petroleum ether/ethyl acetate = 5/1∼2/1, v/v) to afford the title Compound (**310a**) (5.5 g, 99 %) as a white solid. ¹H NMR (CDCl₃, 400 MHz) major characteristic peaks: *δ* (ppm) 0.82 (s, 3H), 0.85 (s, 0.6H), 1.03 (s, 0.6H), 1.09 (s, 3H), 2.94(m, 1H), 3.13 (m, 0.2H), 3.70 (m, 0.2H), 3.92 (m, 1H).

### Example 224B

### Preparation of Compound (310b)

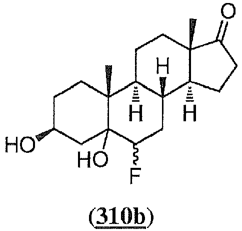

To a pre-cooled solution of Compound (**310a**) (1.0 g, 3.3 mmol) in THF (25 mL) and Et₂O (25 mL) was added BF₃.Et₂O (3.0 g, 47%, 9.9 mmol), anhydrous magnesium sulfate (1.0 g, 8.3 mmol) and a solution of hydrofluoric acid in pyridine (65%, 1.0 g, 6.6 mmol). The mixture was stirred at this temperature for 0.5 hr. The reaction mixture was concentrated and then diluted with water. It was extracted with ethyl acetate (100 mL x 3) and the combined organic phases were washed with water, aq. NaHCO₃, brine, dried over Na₂SO₄, and concentrated, purified by flash column chromatography (petroleum ether/ethyl acetate = 10/1∼2/1, v/v) to give Compound (**310b**) (0.4 g, 35 %) as a white solid. ¹H NMR (CDCl₃, 400 MHz) major characteristic peaks: *δ* (ppm) 0.82-0.83 (s, 3H), 1.04 (s, 1.9H), 1.10 (s, 1.1H), 1.09 (s, 3H), 2.38(m, 1H), 3.30 (m, 2H), 3.64 (s, 0.4H), 3.92 (m, 1H), 4.15 (s, 0.3H), 4.28 (s, 0.3H).

### Example 224C

### Preparation of Compound (310c)

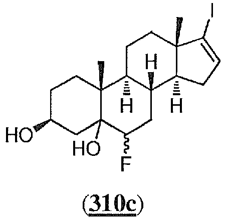

A mixture of Compound (**310b**) (1.3 g, 4.0 mmol), Et₃N (1.2 g, 11.9mmol) and N₂H₄.H₂O (3.8 g, 119.3 mmol) was stirred at reflux in EtOH (50 mL) for 3 h, then was concentrated to give a crude intermediate. The intermediate residue and Et₃N (0.8 g, 7.68 mmol) were dissolved in THF (30 mL) and DCM (30 mL), followed by adding a solution of I₂ (1.5 g, 5.8 mmol) in THF (5 mL) at 0 °C. The mixture was concentrated, dissolved in ethyl acetate (100 mL), washed by water, Na₂SO₃ aq, water, brine, and dried over Na₂SO₄. The crude product (**51c**) (1.4 g) was used for next step of reaction without further purification.

### Example 224D

### Preparation of Compound (310d)

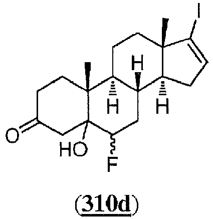

A mixture of Compound (**310c**) (1.1 g, 2.6 mmol) in acetone (30 mL) and Jones' reagent (2.0 mL, 2.67 M, 5.2 mmol) was stirred at 0 °C for 30 minutes, then water was added, extracted with ethyl acetate (100 mL x 3). The combined organic phase was washed with water, brine, and dried over Na₂SO₄. The crude product was purified by FCC (petroleum ether/ethyl acetate = 10/1∼5/1, v/v) to afford the title Compound (**310d**) (0.18 g, 13%) as a white solid. ¹H NMR (CDCl₃, 400 MHz) major characteristic peaks: *δ* (ppm) 0.78 (s, 3H), 1.31 (s, 3H), 4.21 (m, 0.5H), 4.33 (m, 0.5H), 6.13(m, 1H).

### Example 224E

### Preparation of Compound (310e)

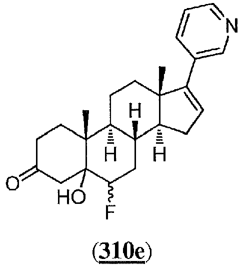

A mixture of Compound (**310d**) (180 mg, 0.42 mmol), pyridin-3-ylboronic acid (77 mg, 0.62 mmol), K₂CO₃ (290 mg, 2.10 mmol) and Pd(PPh₃)₂Cl₂ (31 mg, 0.04 mmol) in dioxane (20 mL) and H₂O (5 mL) was stirred at 80 °C under N₂ protection for 3 hrs. When the starting material was consumed, the reaction solution was cooled to room temperature and water was added. The mixture was extracted by ethyl acetate (100 mL x 3). The combined organic phase was washed with water, brine, and dried over Na₂SO₄. The crude product was purified by FCC (petroleum ether/ethyl acetate = 3/2, v/v) to afford the title compound (**310e**) (80 mg, 50 %) as a yellow solid. LCMS(m/z): 384 (M+1); ¹H NMR (CDCl₃, 400 MHz) major characteristic peaks: *δ* (ppm) 1.06 (s, 3H), 1.33 (s, 3H), 3.17 (m, 1H), 4.25 (m, 0.5H), 4.37 (m, 0.5H), 6.00 (m, 1H), 7.23 (m, 1H), 7.65 (d, *J* = 8.0, 1H), 8.45 (d, *J* = 4.0 Hz, 1H), 8.61 (s, 1H).

### Example 224F

### Preparation of Compound (310)

SOCl₂ (199 mg, 1.67 mmol) was added to a solution of compound (**310e**) (160mg, 0.42 mmol) in pyridine (5 mL) at 0 °C. it was stirred for 2 hrs when the starting materials were consumed. The reaction solution was concentrated and diluted with water (50 mL). The mixture was extracted with ethyl acetate (100 mL x 3). The combined organic phase was washed with water, brine, and dried over Na₂SO₄. The crude product was purified by prep-TLC (petroleum ether/ethyl acetate = 3/2, v/v) to afford Compound (**310**) (40 mg, 26%) as a white solid. LCMS(m/z): 366 (M+1); ¹H NMR (CDCl₃, 400 MHz) major characteristic peaks: *δ* (ppm) 1.09 (s, 3H), 1.36 (s, 3H), 4.98 (m, 0.5H), 5.11 (m, 0.5H), 5.90 (m, 1H), 6.01 (m, 1H), 7.23 (dd, *J* =8.0,4.0 Hz, 1H), 7.65 (d, *J* = 8.0 Hz, 1H), 8.48 (d, *J* = 4.0 Hz, 1H), 8.63 (s, 1H).

### Example 225

### Preparation of Compound (311)

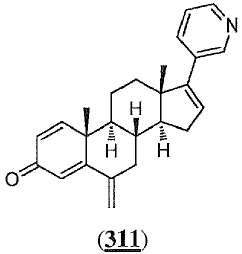

### Example 225A

### Preparation of Compound (311a)

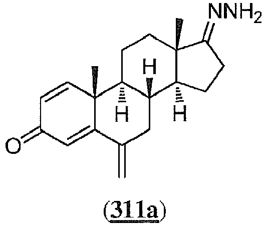

To a solution of Compound (**309a**) (5 g, 17 mmol) in ethanol (50 mL) was added N₂H₄ H₂O (2.1 g, 34 mmol), then the mixture was stirred at 0 °C for 8 h and room temperature overnight. The reaction mixture was diluted with water (150 mL), extracted with DCM (3 x 100 mL). The organic layer was washed with water (2 x 100 mL), brine (100 mL), dried over Na₂SO₄, concentrated, and purified by column chromatography (petroleum ether/ethyl acetate = 2/1, then DCM/MeOH = 100/1, v/v) to get the (**311a**) (1.8 g, 35 %) as a yellow solid. LCMS:m/z: 311 (M+1); ¹H NMR (CDCl3, 400 MHz) peaks: *δ* (ppm) 0.93 (s, 3H), 1.17 (s, 3H), 4.81 (m, 2H), 4.98 (m, 1H), 5.04 (m, 1H), 6.16 (d, *J* = 2.0 Hz, 1H), 6.25 (dd, *J* = 2.0, 10.0 Hz, 1H), 7.10 (d, *J* = 10.0 Hz, 1H).

### Example 225B

### Preparation of Compound (311b)

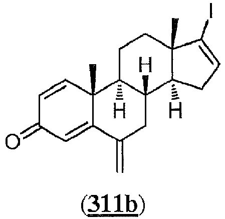

To a solution of Compound (**311a**) (1.6 g, 5.2 mmol) in DCM (30 mL) was added Et₃N (780 mg, 7.7 mmol) at 0 °C, then a solution of I₂ (1.57 g, 6.2 mmol) in THF (20 mL) was added dropwise. The mixture was stirred at room temperature for 2 hrs, washed with sat.Na₂SO₃, extracted with DCM (2 x 100 mL). The organic layer was washed with brine (100 mL), dried over Na₂SO₄ and concentrated. The crude product was purified by column chromatography (petroleum ether/ethyl acetate = 5/1, v/v) to give Compound (**311b**) (230 mg, 11.5%) as a yellow solid. ¹H NMR (CDCl3, 400 MHz) peaks: *δ* (ppm) 0.81 (s, 3H), 1.17 (s, 3H), 4.98 (m, 1H), 5.05 (m, 1H), 6.14 (m, 1H), 6.16 (d, *J* = 2.0 Hz, 1H), 6.25 (dd, *J* = 2.0, 10.0 Hz, 1H), 7.07 (d, *J* = 10.0 Hz, 1H)

### Example 225C

### Preparation of Compound (311)

To a solution of Compound (**311b**) (240 mg, 0.59 mmol) in dioxane (30 mL) was added pyridin-3-ylboronic acid (145 mg, 1.18 mmol), potassium carbonate (407 mg, 2.59 mmol) in water (2 mL), and bis(triphenylphosphine) palladium chloride (41 mg). The mixture was thoroughly degassed, and heated under nitrogen at 80 °C for 1 hour. After being filtered through a pad of Celite, the crude product was purified by prep-TLC (ethyl acetate in petroleum, 50% v/v) to give Compound (**311**) (130 mg, 62%) as a white solid. LCMS: m/z: 358 (M+1); ¹H NMR (CDCl₃, 400 MHz) peaks: *δ* (ppm) 1.10 (s, 3H), 1.21 (s, 3H), 5.01 (m, 2H), 5.06 (m, 1H), 6.01 (m, 1H), 6.18 (d, *J* = 2.0 Hz, 1H), 6.27 (dd, *J* = 2.0, 10.0 Hz, 1H), 7.09 (d, *J* = 10.0 Hz, 1H), 7.25 (m, 1H), 7.64 (m, 1H), 8.49 (d, *J* = 4.4 Hz, 1H), 8.63 (s, 1H).

### Example 226

### Preparation of Compound (312)

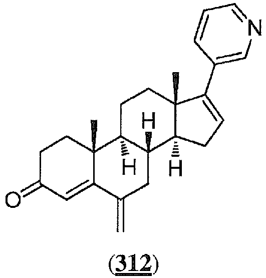

### Example 226A

### Preparation of Compound (312a)

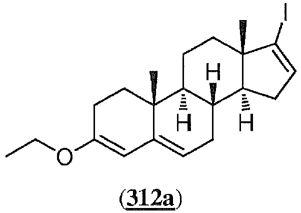

To a solution of (10R,13S)-17-iodo-10,13-dimethyl-6,7,8,9,10,11,12,13,14,15-decahydro-1H-cyclopenta[a]phenanthren-3(2H)-one (6.4 g, 16.3 mmol) in ethanol (30 mL) was added triethoxymethane (4.1 mL, 24.5 mmol) and *p*-TsOH.H₂O (309 mg, 1.63 mmol). The mixture was stirred at 40 °C for 1 hour. The mixture was cooled to room temperature and TEA was added to neutralize the mixture. After filtration, the crude product was washed with petroleum ether. This gave Compound (**312a**) (5.0 g, 80%) as a white solid. ¹H NMR (CDCl₃, 400 MHz) major characteristic peaks: *δ* (ppm) 0.78 (s, 3H), 1.00 (s, 3H), 1.31 (t, *J* = 6.8 Hz, 3H), 3.78 (q, J= 6.8 Hz, 2H), 5.13 (s, 1H), 5.21 (m, 1H), 6.15 (m, 1H).

### Example 226B

### Preparation of Compound (312b)

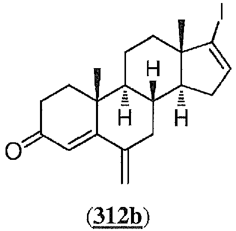

To a solution of Compound (**312a**) (5.0 g, 11.8 mmol) in ethanol (30 mL) was added p-TsOH.H₂O (224 mg, 1.18 mmol), N-methylbenzenamine (2 mL) and 37% aq. HCHO solution (2.24 mL). The mixture was stirred at 40 °C for 2 hrs. The mixture was cooled to room temperature and con.HCl (20 mL) was added to acidify the mixture. It was stirred for additional 1 hr at room temperature and diluted with (150 mL). The mixture was extracted with DCM (100 mL x 3). The combined organic layers were washed with water, brine, and dried over Na₂SO₄. The crude was purified by silica gel column (ethyl acetate in petroleum, 10% v/v) to afford Compound (**312b**) (1.94 g, 32%). LC-MS (m/z): 409 [M+H]⁺; ¹H NMR (CDCl₃, 400 MHz) major characteristic peaks: *δ* (ppm) 0.77 (s, 3H), 1.11 (s, 3H), 4.96 (t, *J* = 1.6 Hz, 1H), 5.09 (t, *J* = 1.6 Hz, 1H), 5.92 (s, 1H), 6.15 (m, 1H).

### Example 226C

### Preparation of Compound (312)

To a solution of Compound (**312b**) (200 mg, 0.49 mmol) in 1,4-dioxane (8 mL) was added pyridin-3-ylboronic acid (120 mg, 0.98 mmol), potassium carbonate (304 mg, 2.21 mmol) in water (2 mL), and Pd(dppf)Cl₂ (36 mg). The mixture was thoroughly degassed, and heated under nitrogen and heated to reflux for 2 hrs. The mixture was extracted with DCM (100 mL x 3). The organic layers were combined, washed with water, brine, and dried over Na₂SO₄. The crude product was purified by prep-TLC (ethyl acetate in petroleum, 30% v/v) to afford Compound (**312**) (64 mg, 36%) as a yellow solid. LC-MS (m/z): 360 [M+H]⁺; ¹H NMR (CDCl₃, 400 MHz) major characteristic peaks: δ (ppm) 1.06 (s, 3H), 1.15 (s, 3H), 4.99 (t, *J* = 1.6 Hz, 1H), 5.10 (t, *J* = 1.6 Hz, 1H), 5.94 (s, 1H), 6.00 (m, 1H), 7.23(m, 1H), 7.65 (m, 1H), 8.48 (dd, *J* = 1.6, 4.8 Hz, 1H), 8.61 (d, *J* = 2.0 Hz, 1H).

### Example 227

### Preparation of Compound (313)

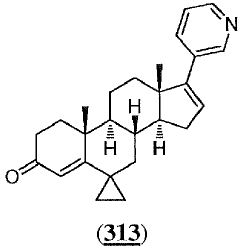

### Example 227A

### Preparation of Compound (313a)

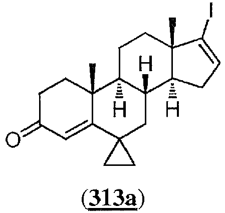

NaH (60%, 98 mg, 2.45 mmol) was added slowly to a solution of tertmethylsulfoxonium (323 mg, 1.47 mmol) in dry DMSO (10 mL) and the mixture was stirred at room temperature for 1 hour. Then a solution of Compound (**312b**) (500 mg, 1.23 mmol) in THF (5 mL) was added to above mixture under N₂. The mixture was stirred at room temperature for 2 hrs. When reaction completed, the mixture was poured into ice-water (100 mL) and extracted with DCM (100 mL x 3). The organic layers were combined and washed with water, brine, and dried over Na₂SO₄. The crude product was purified by column chromatography (ethyl acetate in petroleum ether, 10% v/v) to get Compound (**313a**) (280 mg, 54%) as a yellow solid. LC-MS (m/z): 423 [M+H]⁺; ¹H NMR (CDCl₃, 400 MHz) major characteristic peaks: *δ* (ppm) 0.81 (s, 3H), 1.28 (s, 3H), 5.64 (s, 1H), 6.13 (m, 1H).

### Example 227B

### Preparation of Compound (313)

To a solution of Compound (**313a**) (280 mg, 0.66 mmol) in 1,4-dioxane (16 mL) was added pyridin-3-ylboronic acid (139 mg, 1.13 mmol), potassium carbonate (410 mg, 2.97 mmol) in water (2 mL), and Pd(dppf)Cl₂ (51 mg). The mixture was thoroughly degassed, and heated under nitrogen and heated to reflux for 2 hour. The mixture was extracted with DCM (100 mL x 3). The organic layers were combined, washed with water, brine, and dried over Na₂SO₄. The crude product was purified by prep-TLC (ethyl acetate in petroleum, 30% v/v) to get compound **(313**) (6 mg, 3%) as a yellow solid. LC-MS (m/z): 374 [M+H]⁺; ¹H NMR (CDCl₃, 400 MHz) major characteristic peaks: *δ* (ppm) 0.46 (m, 2H), 0.86 (m, 2H), 1.09 (s, 3H), 1.31(s, 3H), 5.66 (s, 1H), 5.98 (m, 1H), 7.23(m, 1H), 7.65 (m, 1H), 8.47 (d, *J* = 3.6 Hz, 1H), 8.62 (s, 1H).

### Example 228

### Preparation of Compound (314)

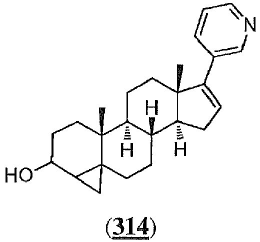

### Example 228A

### Preparation of Compound (314a)

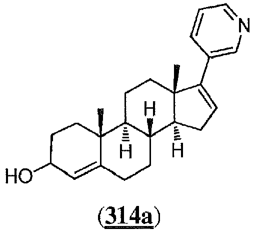

To a solution of (**50c**) (347 mg, 1 mmol) in MeOH (29 mL) was added NaBH₄ (42 mg, 1.1mmol) and CeCl₃ (24.7 mg, 0.1 mmol) at 25 °C under N₂. The reaction mixture was stirred for 1 hour at 25 °C, then saturated aq. NaHCO₃ (10 mL) was added to quench the reaction, followed by water (15 mL), extracted with dichloromethane (2 x 45 mL). The combined organic layers were dried over anhydrous Na₂SO₄, evaporated to obtain a crude product, which was purified by column chromatography (petroleum ether/ethyl acetate=3/1-1/1, v/v) to afford Compound **(314a)** (307 mg, yield: 88%) as a white solid. LC-MS (ESI) m/z: 350 (M+H)⁺; ¹H-NMR (400 MHz, DMSO-d₆) *δ* (ppm) 0.97 (s, 3H), 1.01 (s, 3H), 3.90-3.93 (m, 1H), 4.55-4.57 (d, *J* = 5.6 Hz, 1H), 5.21 (s, 1H), 6.09 (s, 1H), 7.31-7.35 (dd, *J* = 4.8, 8.0 Hz, 1H), 7.74-7.76 (dd, *J* =2.0, 5.6 Hz, 1H), 8.43-8.44 (m, 1H), 8.57-8.58 (m, 1H).

### Example 228B

### Preparation of Compound (314)

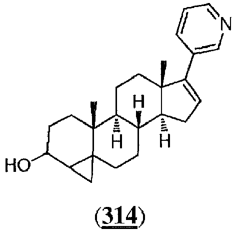

To a solution of (**314a**) (100 mg, 0.29 mmol) and Zn (active, Zn dust was washed with diluted HCl, ether, dried under N₂) (372 mg, 5.73 mmol) in dry DME (7 mL) was added a small crystal of iodine, followed by CH₂l₂ (0.17 mL, 2.18 mmol) at 25 °C under N₂. The reaction mixture was stirred for 2 hrs at 96 °C, then saturated aq. NaHCO₃ (20 mL) was added to quench the reaction. The mixture was extracted with ethyl acetate (3 x 20 mL). The combined organic layers were dried over anhydrous Na₂SO₄ and evaporated to obtain a crude product, which purified by prep-HPLC to afford Compound (**314**) (20.6 mg, 20%) as a white solid. LC-MS (ESI) m/z: 364 (M+H)⁺; ¹H-NMR (400 MHz, CDCl₃) *δ* (ppm) 0.09-0.13 (m, 1H), 1.61-1.65 (m, 1H), 1.02 (s, 3H), 1.03 (s, 3H), 4.35-4.39 (t, J= 6.8 Hz, 1H), 5.99 (s, 1H), 7.20-7.23 (m, 1H), 7.63-7.65 (d, J= 7.6 Hz, 1H), 8.45-8.46 (m, 1H), 8.62 (s, 1H).

### Example 229

### Preparation of Compound (315)

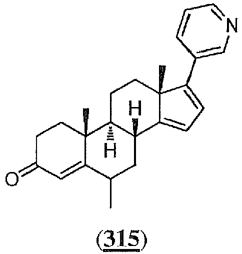

### Example 229A

### Preparation of Compound (315a)

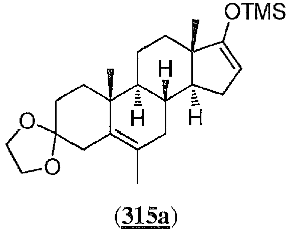

To a solution of (*i*-Pr)₂NH (2.1 mL) in THF (40 mL) cooled to -78 °C was added a solution of BuLi (2.5 M in hexane, 5.9 mL), the mixture was stirred at -78 °C for 15 min. A solution of Compound (**309e**) (1.7 g, 4.9 mmol) in THF (10 mL) was added dropwise to the solution over 30 min, and the reaction mixture was stirred at -78 °C for additional 15 min. Then Et₃N (2.7 mL) and TMSCl (2.05 mL) were added. The mixture was warmed to room temperature, stirred for additional 25 min, and then quenched with sat.NaHCO₃. The aqueous layer was extracted with ethyl acetate (2 x 100 mL) and the combined organic layers were dried over Na₂SO₄ and concentrated to give a crude product Compound (**315a**) (2.3 g, crude), which was used in next step without further purification.

### Example 229B

### Preparation of Compound (315b)

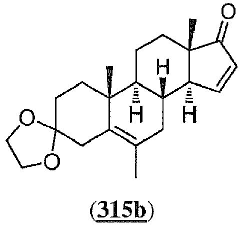

A mixture of Compound (**315a**) (2.3 g, 5.5 mmol) and Pd(OAc)₂ (1.24 g, 5.5 mmol) in DCM (30 mL) and MeCN (10 mL) was stirred at 35°C for 2 hrs, filtered and the filtrate was concentrated to give a crude product. The crude product was purified by column chromatography (petroleum ether/ethyl acetate = 5/1, v/v) to afford Compound (**315b**) (1.8 g, 75%, two steps) as a white solid. LCMS: m/z: 343 (M+1); ¹H NMR (CDCl3, 400 MHz) peaks: *δ* (ppm) 1.08 (s, 6H), 1.68 (s, 3H), 3.98 (m, 4H), 6.05 (m, 1H). 7.52 (m, 1H).

### Example 229C

### Preparation of Compound (315c)

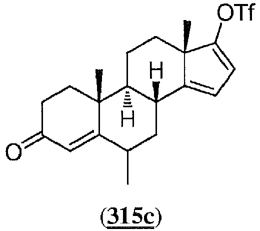

To a solution of Compound (**315b**) (1.5 g, 4.4 mmol), and imidazole (1.1 g, 13.1 mmol) in DCM (30 mL) was added Tf₂O (1.84 g, 6.6 mmol) dropwise at 0 °C. Then the mixture was stirred at room temperature for 1 hr and diluted with DCM (100 mL). The organic layer was washed with water (50 mL x 2), brine (100 mL), dried over Na₂SO₄, concentrated, and purified by column chromatography (petroleum ether/ethyl acetate = 5/1, v/v) to give Compound (**315c**) (400 mg, 20%) as a colorless oil. ¹H NMR (CDC13, 400 MHz) peaks: *δ* (ppm) 1.15 (d, *J* = 6.4 Hz, 3H), 1.18 (s, 3H), 1.29 (s, 3H), 5.78 (m, 1H), 5.83 (d, *J* = 1.2 Hz, 1H), 6.09 (d, *J* = 2.4Hz, 1H).

### Example 229D

### Preparation of Compound (315)

To a solution of Compound (**315c**) (500 mg, 1.0 mmol) in dioxane (30 mL) was added pyridin-3-ylboronic acid (259 mg, 2.1 mmol), potassium carbonate (727 mg, 5.3 mmol) in water (2 mL), and bis(triphenylphosphine) palladium chloride (74 mg). The mixture was thoroughly degassed, and heated under nitrogen at 80 °C for 1 hour. After being filtered through a pad of Celite, the crude product was purified by prep-TLC (ethyl acetate in petroleum, 50% v/v) to give Compound (**315**) (106 mg, 28%) as a yellow solid. LCMS: m/z: 360 (M+1); ¹H NMR (CDCl3, 400 MHz) peaks: *δ* (ppm) 1.17 (d, *J* = 6.0 Hz, 3H), 1.22 (s, 3H), 1.32 (s, 3H), 5.85 (d, *J* = 1.2 Hz, 1H), 6.00 (m, 1H), 6.79 (d, *J* = 2.4Hz, 1H), 7.24 (m,1H), 7.77 (m, 1H), 8.43 (m, 1H), 8.75(d, *J* = 3.0 Hz, 1H).

### Example 230

### Preparation of Compound (316)

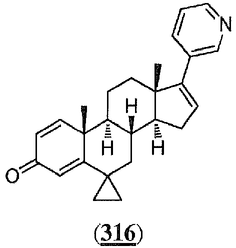

### Example 230A

### Preparation of Compound (316a)

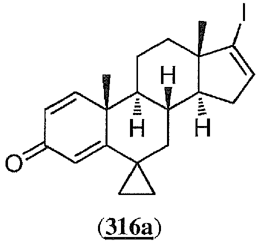

To a solution of Compound (**313a**) (1.25 g, 3.0 mmol) in 30 mL of toluene and 5 mL of acetic acid was added MnO₂ (1.29 g, 15 mmol) and DDQ (672 mg, 3.0 mmol) under N₂. The mixture was stirred and heated to reflux for 5 hrs. The mixture wea cooled to room temperature and filtered. The filtrate was extracted with ethyl acetate (100 mLx 3), washed with water, brine, and dried over Na₂SO₄. After removal of the solvents, the residues were purified by silica gel column (ethyl acetate in petroleum, 10% v/v) to afford Compound (**316a**) (822 mg, 66%) as a yellow solid. LC-MS (m/z): 421 [M+H]⁺; ¹H NMR (CDCl₃, 400 MHz) major characteristic peaks: δ (ppm) 0.43 (m, 1H), 0.56 (m, 1H), 0.83 (s, 3H), 1.34(s, 3H), 6.04 (m, 1H), 6.12 (m, 1H), 8.47 (dd, *J* = 2.0 Hz, 10.0 Hz, 1H), 8.47 (d, *J* = 10.0 Hz, 1H).

### Example 230B

### Preparation of Compound (316)

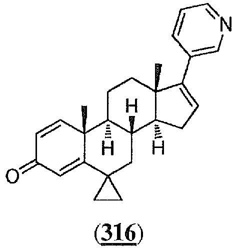

To a solution of Compound (**316a**) (300 mg, 0.71 mmol) in 1,4-dioxane (10 mL) was added pyridin-3-ylboronic acid (176 mg, 1.42 mmol), potassium carbonate (441 mg, 3.2 mmol) in water (2 mL), and Pd(dppf)Cl₂ (52 mg). The mixture was thoroughly degassed, and heated under nitrogen and heated to reflux for 2 hrs. The mixture was extracted with DCM (50 mL x 3). The organic layers were combined, washed with water, brine, and dried over Na₂SO₄. After the revomal of the solvents, the residue was purified by prep-TLC (ethyl acetate in petroleum, 30% v/v) to afford Compound (**316**) (160 mg, 60%) as a yellow solid. LC-MS (m/z): 372 [M+H]⁺; ¹H NMR (CDCl₃, 400 MHz) major characteristic peaks: δ (ppm) 0.45 (m, 1H), 0.58 (m, 1H), 0.90 (m, 1H), 1.06 (m, 1H), 1.12 (s, 3H), 1.37 (s, 3H), 5.98 (m, 1H), 6.05 (d, *J* = 2.0 Hz, 1H), 6.24 (dd, *J* = 2.0 Hz, 10.0 Hz, 1H), 7.07 (d, *J* = 10.0 Hz, 1H), 7.23(m, 1H), 7.64 (m, 1H), 8.48 (dd, *J* = 1.6 Hz, 4.4 Hz, 1H), 8.61 (d, *J* = 1.6 Hz, 1H).

### Example 231

### Preparation of Compound (317)

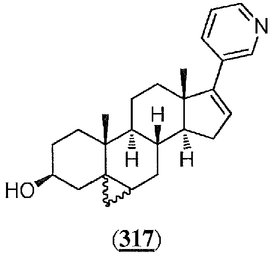

### Example 231A

### Preparation of Compound (317a)

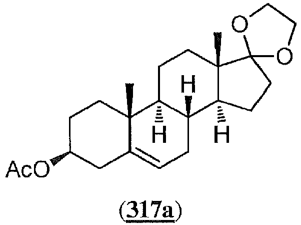

To a stirring solution of (**267a**) (4.0 g, 12 mmol) in DCM (40 mL) at 0 °C was added Et₃N (3.65 g, 36 mmol), DMAP (146 mg, 1.2 mmol), Ac₂O (1.83 g, 18 mmol) consecutively. The reaction mixture was warmed to room temperature and stirred for 6 hrs. The solvent was removed under reduced pressure and the crude product was purified by flash chromatography (petroleum ether/ ethyl acetate = 1/1, v/v) to give desired product (**317a**) (3.0 g, 67%) as a white solid. LC-MS (ESI) m/z: 375 (M+1)⁺.

### Example 231B

### Preparation of Compound (317b)

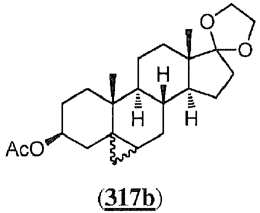

To a solution of (**317a)** (3.3 g, 8.89 mmol) in toluene (30 mL) under N₂ atmophere at-10 °C was added CH₂I₂ (28.57 g, 106.68 mmol) and ZnEt₂ (1.0 M , 53.3 mL, 53.3 mmol). The reaction mixture was allowed to warm to room temperature and stirred for 24 hrs. To the mixture was added a sat. NaHCO₃ solution. The aqueous layer was extracted with ethyl acetate (100 mL x 5). The combined organic extracts were dried over Na₂SO₄ and concentrated under reduced pressure to give a crude product (include compound (**317a**)). The crude product was purified by the following method to get pure compound (**317b**).

To a stirring solution of crude product (3.5 g) in DCM (100 mL) was added NaHCO₃ (2.35 g, 28 mmol) and *m*-CPBA (5.9 g, 14 mmol) at 0 °C. The reaction mixture was warmed to room temperature and stirred for 12 hrs before water (100 mL) was added. The aqueous layer was extracted with ethyl acetate (100 mL x 5). The combined organic extracts were dried over Na₂SO₄ and concentrated in vacuum. The residue was purified by flash chromatography (petroleum ether/ethyl acetate = 5/1, v/v) to afford Compound (**317b**) (690 mg, 20%). LC-MS (ESI) m/z: 389 (M+1)⁺.

### Example 231C

### Preparation of Compound (317c)

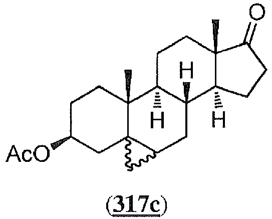

To a solution of (**317b**) (2 g, 5.15 mmol) in THF (50 mL) at 0 °C was added diluted aq. HCl solution (2N, 1.5 mL) dropwise. The resulting mixture was stirred at room temperature for 12 hrs. Then saturated NaHCO₃ solution was added to the mixture. The aqueous layer was extracted were dried over Na₂SO₄ and concentrated under reduced pressure. The residue was purified by column chromatography (petroleum ether/ethyl acetate = 10/ 1, v/v) to afford desired (**317c**) (1.7 g, 98%) as colourless oil. LC-MS (ESI) m/z: 345 (M+1)⁺.

### Example 231D

### Preparation of Compound (317d)

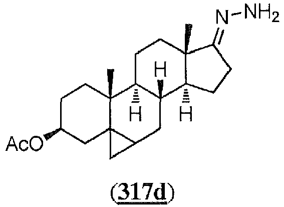

To a solution of **(317c)** (1.6 g, 4.64 mmol) in EtOH (20 mL) was addedhydrazine (1.16 g, 23.2 mmol). The reaction mixture was heated to reflux and stirred for 12 hrs. Then the solvent was removed under reduced pressure. The residue was purified by column chromatography (petroleum ether/ethyl acetate = 3/1, v/v) to give desired product (**317d**) (1.6 g, 96 %) as a colourless oil. LC-MS (ESI) m/z: 359 (M+1)⁺.

### Example 231E

### Preparation of Compound (317e)

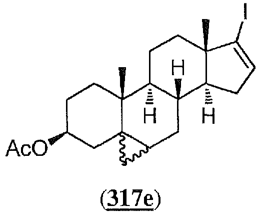

To a solution of (**317d**) (1.6 g, 4.47 mmol) in DCM (30 mL) at 0 °C under nitrogen atmosphere were added Et₃N (9 g, 89.4 mmol) and I₂ (2.8 g, 11.17 mmol). The reaction mixture was warmed to room temperature and stirred for 12 hrs. Then the solvent was removed under reduced pressure. The residue was purified by column chromatography (hexane/ ethyl acetate = 10/1, v/v) to give desired product (**317e**) (800 mg, 40 %) as a yellow oil. LC-MS (ESI) m/z: 455 (M+1)⁺.

### Example 231F

### Preparation of Compound (317f)

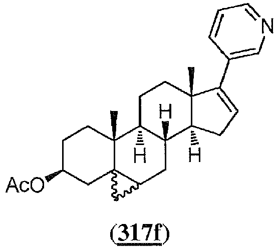

To a solution of (**317e**) (800 mg, 1.76 mmol) in DME (30 mL) under N₂ atmosphere were added pyridin-3-ylboronic acid (325 mg, 2.64 mmol), PdCl₂(dppf) (128.7 mg, 0.176 mmol) and K₃PO₄ (1.86 g, 8.8 mmol). The reaction mixture was heated to reflux for 12 hrs. Then the solvent was removed under reduced pressure and the residue was purified by column chromatography (hexane/ethyl acetate = 2/1, v/v) to give desired product (**317f**) (285 mg, 40 %). LC-MS (ESI) m/z: 405 (M+1)⁺.

### Example 231G

### Preparation of Compound (317)

To a solution of (**317f**) (100 mg, 0.24 mmol) in MeOH (3 mL) was added KOH (34.6 mg, 0.61 mmol). The reaction mixture was stirred for 12 hrs and then quenched with sat. NH₄Cl solution (10 mL). The aqueous layer was extracted with ethyl acetate (20 mL x 5). The combined organic extracts were dried over Na₂SO₄ and concentrated under reduced pressure. The residue was purified by column chromatography (hexane/ ethyl acetate = 1/ 2, v/v) to afford the desired product (**317**) (60 mg, 68%) as a white solid. LC-MS (ESI) m/z: 364 (M+1)⁺; ¹H-NMR (400 MHz, CDCl₃) *δ* (ppm): 8.59 (s, 1H), 8.43 (s, 1H), 7.62 (d, 1H), 7.22-7.20 (m, 1H), 5.96-5.95 (m, 1H), 3.88,3.75 (m, 1H, α+β diastereomer), 1.15,0.93 (s, 3H, α+βdiastereomer), 0.98,0.97 (s, 3H, α+βdiastereomer), 0.40-0.36 (m, 1H, α+βdiastereomer), 0.21-0.17,0.01-0.00 (m, 1H, α+βdiastereomer).

### Example 232

### Preparation of Compound (318)

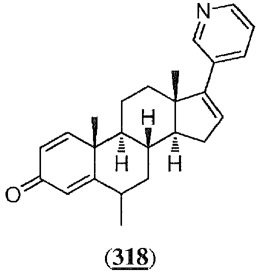

### Example 232A

### Preparation of Compound (318a)

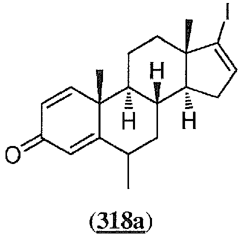

A suspension of Compound (**309h**) (2.95 g, 7.20 mmol), DDQ (1.80 g, 7.92 mmol) and MnO₂ (2.91 g, 35.98 mmol) in toluene/AcOH (30/4.5 mL, v/v) was stirred at 90 °C for 3 hrs. Then it was filtered to remove MnO₂. The filtrate was diluted with ethyl acetate (150 mL) and washed with water and brine, dried and purified by silica chromatography (ethyl acetate/ petroleum ether = 1/10, v/v) to obtain Compound (**318a**) (1.80 g, 64%) as a white solid. LCMS (m/z): 409 [M+H]⁺; ¹H NMR (CDCl₃, 400 MHz) major characteristic peaks: *δ* (ppm) 0.81 (s, 3H), 1.14 (d, *J* = 6.0 Hz, 3H), 1.26 (s, 3H), 6.10-6.13 (m, 2H), 6.25 (dd, *J* = 2.0, 11.6 Hz, 1H), 7.05 (d, *J* = 11.6 Hz, 1H).

### Example 232B

### Preparation of Compound (318)

To a solution of Compound (**318a**) (1.80 g, 4.412 mmol) in 1,4-dioxane (30 mL) was added pyridin-3-ylboronic acid (814 mg, 6.618 mmol), potassium carbonate (1.83 g, 13.24 mmol) in water (3 mL), and Pd(PPh₃)Cl₂ (310 mg, 0.44 mmol). The mixture was thoroughly degassed and heated under nitrogen and heated to 85 oC for 3 hour. The mixture was extracted with ethyl acetate (50 mL x 3). The organic layers were combined, washed with water, brine, dried over Na₂SO₄, concentrated, and purified by prep-HPLC to afford compound **(318)** (800 mg, 45%) as a white solid. LCMS (m/z): 260 [M+H]⁺; ¹H NMR (CDCl₃, 400 MHz) major characteristic peaks: *δ* (ppm) 1.09 (s, 3H), 1.17 (d, *J* = 6.4 Hz, 3H), 1.29 (s, 3H), 5.99 (s, 1H), 6.12 (s, 1H), 6.20-6.27 (m, 1H), 7.06 (d, *J*= 8.0 Hz, 1H), 7.22-7.25 (m, 1H), 7.62-7.66 (m, 1H), 8.46 (s, 1H), 8.60 (s, 1H).

### Example 233

### Preparation of Compound (319)

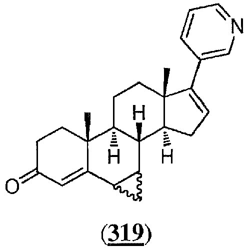

### Example 233A

### Preparation of Compound (319a)

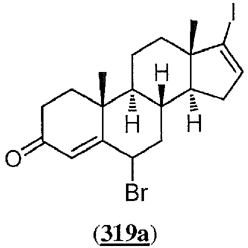

To a solution of Compound (**312a**) (1.54 g, 3.6 mmol) and NaOAc (298 mg, 3.6 mmol) in 96% acetone (30 mL) was added NBS (833 mg, 4.68 mmol), and the mixture was stirred at rt for 1 hr. The mixture was concentrated, and the residue was diluted with water (100 mL), extracted with ethyl acetate (200 mL), concentrated and purified by silica-gel column chromatography eluted with (ethyl acetate/ petroleum ether = 1/5, v/v) to obtain Compound (**312a**) (1.2 g, 65%) as a white solid. ¹H NMR (CDCl₃, 400 MHz) major characteristic peaks: *δ* (ppm) 0.85 (s, 3H), 1.57 (s, 3H), 4.99-5.00 (m, 1H), 5.92 (s, 1H), 6.15-6.16 (m, 1H).

### Example 233B

### Preparation of Compound (319b)

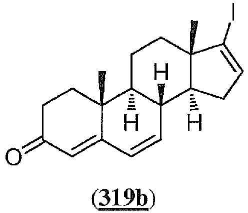

A mixture of Compound (**319a**) (720 mg, 1.515 mmol) KF (176 mg, 3.03 mmol) in DMF (15 mL) was stirred at 150 °C for 2 hrs. The mixture was cooled down to room temperature, diluted with water (100 mL), extracted with ethyl acetate (100 mL), washed with water, concentrated, and purified by silica-gel column chromatography eluted with (ethyl acetate/ petroleum ether = 1/5, v/v) to obtain Compound (**319b**) (543 mg, 73%) as a light yellow solid. LCMS (m/z): 395.1 [M+H]⁺; ¹H NMR (CDCl₃, 400 MHz) major characteristic peaks: *δ* (ppm) 0.83 (s, 3H), 1.14 (s, 3H), 5.70 (s, 1H), 6.13-6.15 (m, 2H), 6.17-6.18 (m, 1H).

### Example 233C

### Preparation of Compound (319c)

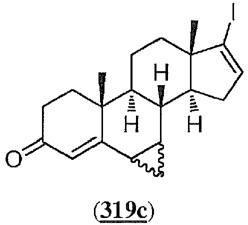

A mixture of trimethylsulfoxonium iodide (370 mg, 1.68 mmol), NaH (90 mg, 2.25 mmol) in DMSO (8 mL) was stirred at room temperature for 1 hr. Compound **(319b)** (443 mg, 1.12 mmol) in THF (1 mL) was added,and the mixture was stirred at room temperature overnight. The reaction mixture was diluted with water (100 mL), extracted with ethyl acetate (100 mL), washed with water, concentrated, and purified by silica-gel column chromatography eluted with (ethyl acetate/ petroleum ether = 1/8, v/v) to afford Compound (**319c**) (218 mg, 48%) as a light yellow solid. LCMS (m/z): 409.1 [M+H]⁺
¹H NMR (CDCl₃, 400 MHz) major characteristic peaks: *δ* (ppm) 0.76 (s, 2H), 0.81 (s, 1H), 1.10 (s, 2H), 1.15 (s, 1H), 5.95 (s, 0.33H), 6.02 (s, 0.66H), 6.17-6.18 (m, 0.33H), 6.19-6.20 (m, 0.66H).

### Example 233D

### Preparation of Compound (319)

A mixture of Compound (**319c**) (1.22 g, 2.99 mmol), pyridin-3-ylboronic acid (735 mg, 5.98 mmol), Pd(PPh₃)₂Cl₂ (210 mg, 0.299 mmol), K₂CO₃ (2.06 g, 14.95 mmol) in dioxane (20 mL) and water (4 mL) was stirred at 80 °C for 2 hrs. The reactionmixture was diluted with water (200 mL), extracted with ethyl acetate (200 mL), washed with water, concentrated, and purified by silica-gel column chromatography eluted with (ethyl acetate/ petroleum ether = 1/2, v/v) to obtain (**20**) (500 mg, 47%) as a pale yellow solid. LCMS (m/z): 360.2 [M+H]⁺; ¹H NMR (CDCl₃, 400 MHz) major characteristic peaks: *δ* (ppm) 1.04 (s, 2H), 1.10 (s, 1H), 1.13 (s, 2H), 1.19 (s, 1H), 5.96 (s, 0.33H), 6.03-6.06 (m, 1.66H), 7.23-7.25 (m, 1H), 7.65-7.68 (m, 1H), 8.47-8.49 (m, 1H), 8.62-8.64 (m, 1H).

### Example 234

### Preparation of Compound (320)

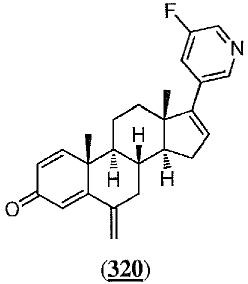

To a solution of compound (**311b**) (820 mg, 2.0 mmol) in 1,4-dioxane (20 mL) was added 5-fluoropyridin-3-ylboronic acid (564 mg, 4.0 mmol), potassium carbonate (1.25 g, 9.0 mmol) in water (5 mL), and Pd(dppf)Cl₂ (146 mg). The mixture was thoroughly degassed, and heated under nitrogen and heated to reflux for 2 hour. The mixture was extracted with ethyl acetate (100 mL x 3). The organic layers were combined, washed with water, brine, and dried over Na₂SO₄, concentrated, purified by column chromatography (ethyl acetate in petroleum, 30% v/v) to afford Compound (**320**) (320 mg, 42%) as a white solid. LC-MS (m/z): 376 [M+H]⁺; ¹H NMR (CDCl₃, 400 MHz) major characteristic peaks: *δ* (ppm) 1.09 (s, 3H), 1.20 (s, 3H), 5.00 (t, *J* = 1.6 Hz, 1H), 5.07 (t, *J* = 1.6 Hz, 1H), 6.07 (m, 1H), 6.18 (s, 1H), 6.26 (dd, *J* = 2.0 Hz, 10.4 Hz, 1H), 7.07 (d, *J*= 10.4 Hz, 1H), 7.36 (td, *J*= 2.0 Hz, 10.0 Hz, 1H), 8.35 (d, *J*= 2.0 Hz, 1H), 8.44 (s, 1H).

### Example 235

### Preparation of Compound (321), (322), and (323)

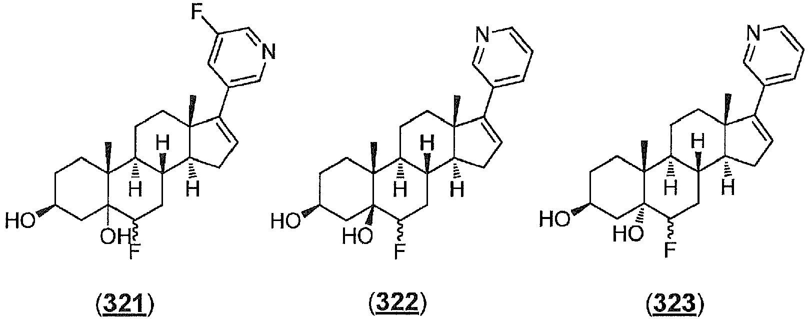

A mixture of Compound (**310c**) (360 mg, 0.83 mmol), 5-fluoropyridin-3-ylboronic acid (175 mg, 1.24 mmol), K₂CO₃ (573 mg, 4.15 mmol) and Pd(PPh₃)₂Cl₂ (62 mg, 0.08 mmol) in dioxane (20 mL) and H₂O (5 mL) was stirred at 80 °C under N₂ protection for 3 hrs. When the starting material was consumed, the reaction solution was cooled to room temperature, diluted with water, and extracted with ethyl acetate (100 mL x 3). The combined organic phase was washed with water, brine, and dried over Na₂SO₄. The crude product was purified by prep-TLC (petroleum ether/ethyl acetate = 1/1, v/v) to afford the title Compound (**321**) (65 mg, 18 %) as a white solid. LCMS (m/z): 404 (M+1). ¹H NMR (MeOD, 400 MHz) major characteristic peaks: *δ* (ppm) 1.06 (s, 2.4H), 1.07 (s, 0.6H), 1.15 (s, 0.6H), 1.22 (s, 2.4H), 3.69 (m, 0.8H), 3.95 (m, 0.8H), 4.04 (m, 0.2H), 4.22-4.34 (m, 0.2H), 6.11 (m, 1H), 7.47 (d, *J* = 9.6, 1H), 8.27 (d, *J* = 2.8 Hz, 1H), 8.39 (s, 1H).

Using a synthetic procedure and conditions similar to the preparation of compound (**321**), replacing 5-fluoropyridin-3-ylboronic acid with pyridin-3-ylboronic acid Compound (**322**) and (**323**) were isolated (1:1 ration) as a white solids.

Compound (**322**): LC-MS (m/z): 386.2 (M+1); ¹H NMR (CD₃OD, 400 MHz) major characteristic peaks: *δ* (ppm) 1.07 (s, 3H), 1.23 (s, 3H), 3.67 (s, 1H), 3.89 (m, 1H), 6.08 (s, 1H), 7.36-7.39 (m, 1H), 7.84 (d, *J*= 8.0 Hz, 1H), 8.38 (d, *J* = 4.4 Hz, 1H), 8.54 (s, 1H).

Compound (**323**): LC-MS (m/z): 386.2 (M+1); 1H NMR (CD3OD, 400 MHz) major characteristic peaks: *δ* (ppm) 1.07 (s, 3H), 1.56 (d, *J* = 4.0 Hz,3H), 3.98-4.08 (m, 1H), 4.20 (s, 0.5H), 4.32 (s, 0.5H), 6.08 (s, 1H), 7.36-7.39 (m, 1H), 7.84 (d, *J* = 8.0 Hz, 1H), 8.39 (d, *J* = 4.4 Hz, 1H), 8.53 (s, 1H).

### Example 236

### Preparation of Compound (324)

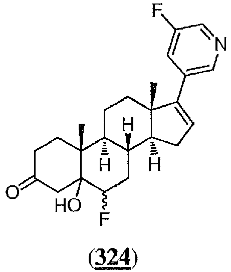

A solution of DMP (57 mg, 0.13 mmol) in DCM (5 mL) was added to a solution of Compound (**323**) (45 mg, 0.11 mmol) in DCM (10 mL) at room temperature and stirred for 2 hrs. The mixture was concentrated, and was purified by prep-TLC (petroleum ether/ethyl acetate = 2/1, v/v) to afford Compound **(324)** (7 mg, 20%) as a white solid. LCMS (m/z): 402 (M+1); ¹H NMR (CDCl₃, 400 MHz) major characteristic peaks: *δ* (ppm) 0.88 (s, 3H), 1.00 (s, 3H), 2.73 (m, 1H), 3.96 (m, 1H), 6.05 (m, 1H), 7.34 (d, *J*= 10.0 Hz, 1H), 8.35 (s, 1H), 8.44 (s, 1H).

### Example 237

### Preparation of Compound (325)

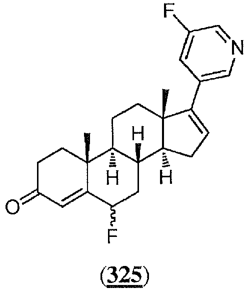

Concentrated HCl (553 mg, 5.6 mmol) was added to a solution of Compound **(324)** (450 mg, 1.13 mmol) and MgSO₄ (1.2 g, 9.7 mmol) in CHCl₃ (30 mL) at 0 °C and stirred for 30 minutes. When the starting material was consumed, the reaction solution was concentrated, saturated NaHCO₃ solution (50 mL) was added. It was then extracted with ethyl acetate (100 mL x 3) and the combined organic phases were washed with water, brine, and dried over Na₂SO₄, concentrated to dryness to give a crude product. The crude product was purified by prep-HPLC to afford the title compound (**325**) (15 mg, 5%) as a white solid. LCMS(m/z): 384 (M+1); ¹H NMR (CDCl₃, 400 MHz) major characteristic peaks: *δ* (ppm) 1.10 (s, 3H), 1.36 (s, 3H), 4.99 (m, 0.5H), 5.11 (m, 0.5H), 5.90 (m, 1H), 6.07 (m, 1H), 7.35 (d, *J* = 10.0 Hz, 1H), 8.34(d, *J* = 2.0 Hz, 1H), 8.45 (s, 1H).

### Example 238

### Preparation of Compound (326), (327), (328), and (329)

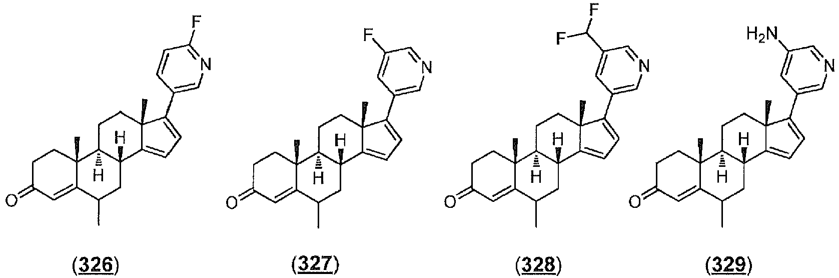

To a solution of Compound (**315c**) (500 mg, 1.16 mmol) in dioxane (20 mL) was added 6-fluoropyridin-3-ylboronic acid (300 mg, 2.13 mmol), potassium carbonate (801 mg, 5.8 mmol) in water (2 mL), and bis(triphenylphosphine) palladium chloride (81 mg). The mixture was thoroughly degassed, and heated under nitrogen at 80 °C for 1 hour. After being filtered through a pad of Celite, the crude product was purified by prep-TLC (ethyl acetate in petroleum, 20% v/v) to give Compound (**326**) (259 mg, 59 %) as a white solid. LCMS: m/z: 378 (M+1). ¹H NMR (CDCl₃, 400 MHz) peaks: *δ* (ppm) 1.17 (d, *J* = 6.8 Hz, 3H), 1.20 (s, 3H), 1.32 (s, 3H), 5.84 (d, *J* = 1.2 Hz, 1H), 5.99 (d, *J* = 2.0 Hz, 1H), 6.69 (d, *J* = 2.4 Hz, 1H), 6.89 (m, 1H), 7.87 (m, 1H), 8.29 (d, *J* = 2.8 Hz, 1H).

Using a synthetic procedure and conditions similar to the preparation of compound (**326**), replacing 6-fluoropyridin-3-ylboronic acid with various pyridin-3-ylboronic acids Compounds (**327 -** **329**) were prepared.

Compound (**327**) yield 42%: LCMS: m/z: 378(M+1). ¹H NMR (CDCl₃, 400 MHz) peaks *δ* (ppm): 1.17 (d, *J* = 6.0 Hz, 3H), 1.23 (s, 3H), 1.33 (s, 3H), 5.85 (d, *J* = 1.2 Hz, 1H), 6.02 (s, 1H), 6.85 (d, *J*= 1.2 Hz, 1H), 7.46 (m, 1H), 8.30 (m, 1H), 8.58 (s, 1H).

Compound (**328**) yield 35%: LC-MS: m/z: 410(M+1). ¹H NMR (CDCl₃, 400 MHz) peaks: *δ* (ppm) 1.18 (d, *J* = 6.0 Hz, 3H), 1.24 (s, 3H), 1.33 (s, 3H), 5.85 (s, 1H), 6.03 (m, 1H), 6.72 (t, *J* = 56 Hz, 1H), 6.88 (m, 1H), 7.88 (s, 1H), 8.55 (s, 1H), 8.86 (s, 1H).

Compound (**329**) yield 15%: LC-MS: m/z: 375(M+1). ¹H NMR (CDC13, 400 MHz) peaks: *δ* (ppm) 1.17 (d, *J*=6.0 Hz, 3H), 1.20 (s, 3H), 1.32 (s, 3H), 3.69 (s, 2H), 5.84 (m, 1H), 5.98 (m, 1H), 6.72 (m, 1H), 7.07 (m, 1H), 7.94 (m, 1H), 8.18 (m, 1H).

### Example 239

### Preparation of Compound (330)

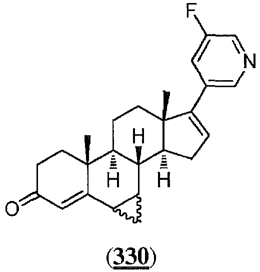

A mixture of Compound (**319c**) (376 mg, 0.92 mmol), 5-fluoropyridin-3-ylboronic acid (260 mg, 1.84 mmol), Pd(PPh₃)₂Cl₂ (65 mg, 0.092 mmol), K₂CO₃ (636 mg, 4.6 mmol) in dioxane (20 mL) and water (4 mL) was stirred at 80 °C for 2 hrs. Then the mixture was poured into water, and extracted with ethyl acetate (100 mL x 2). The extract was washed with brine, dried and evaporaed in vacuum to yield a crude product. The crude product was purified by column chromatography (ethyl acetate/ petroleum ether = from 1/10 to 1/5, to 1/3, v/v) to afford (**330**) (200 mg, 57%) as a white solid. LC-MS (ESI) m/z: 378 (M+1)⁺; ¹H-NMR (400 MHz, CDCl3): *δ* (ppm) 1.04 (s, 2H), 1.09 (s, 1H), 1.13 (s, 2H), 1.18 (s, 1H), 5.96 (s, 0.3H), 6.03 (s, 0.7H), 6.11-6.13 (m, 1H), 7.38-7.40 (m, 1H), 8.34 (s 1H), 8.46 (s, 1H).

### Example 240

### Preparation of Compound (331)

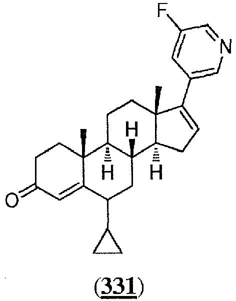

### Example 240A

### Preparation of Compound (331a)

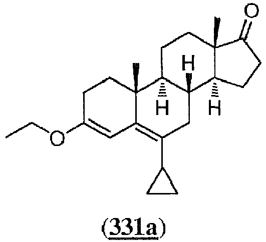

A mixrture of (**332c**) (2.50 g, 6.4 mmol), K₂CO₃ (1.75 g, 12.7 mmol), PdCl₂(PPh₃)₂ (472 mg, 0.6 mmol) and cyclopropylboronic acid (820 mg, 9.5 mmol) in dioxane (50 mL) and water (5 mL) was stirred at 100 °C overnight under N₂ protection. The reaction was cooled to room temperature and diluted with ethyl acetate (200 mL). The organic layer was washed with water and brine, dried over anhydrous Na₂SO₄. The filtrate was concentrated and purified by silica column chromatography (petroleum ether/ ethyl acetate = 80/1, v/v) to obtain Compound (**331a**) (1.24 g, 30 %) as a white solid. LCMS: 355[M+1]⁺; ¹H NMR (DMSO-d₆, 400 MHz) major characteristic peaks: *δ* (ppm) 0.38 (m, 1H), 0.58 (m, 3H), 0.82 (s, 3H), 0.89 (s, 3H), 0.91 (t, *J* = 7.2 Hz, 3H), 1.00 (d, 3H), 3.84 (t, *J* = 7.2 Hz, 3H), 5.75 (m, 1H).

### Example 240B

### Preparation of Compound 331b)

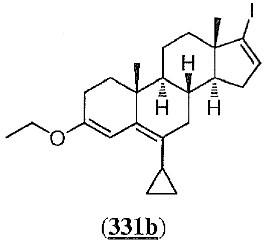

To the mixture of compound (**331a**) (300 mg, 0.85 mmol) in EtOH (15 mL) was added NH₂NH₂.H₂O (80%, 528 mg, 8.46 mmol) and stirred at 60 °C for 2 hrs. The mixture was concentrated to give a white solid. To a mixture of the obtained intermediate and Et₃N (1.04 g, 10.30 mmol) in DCM (10 mL) and THF (10 mL) was added a solution of I₂ (393 mg, 1.55 mmol) in THF (2 mL) at 0 °C, and the solution was stirred at 0 °C for 2 hrs. Then it was quenched with Na₂SO₃ solution to destroy excess I₂, and then diluted with ethyl acetate (150 mL). The organic layer was washed with water and brine, dried with anhydrous Na₂SO₄, concentrated, and purified by column chromatography (petroleum ether/ ethyl acetate = 100/1, v/v) to afford Compound (**331b**) (200 mg, 42%) as a white solid, which was used in next step without further purification.

### Example 240C

### Preparation of Compound (331c)

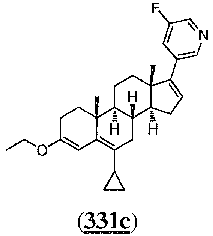

A solution of compound (**331b**) (860 mg, 1.85 mmol), K₂CO₃ (766 mg, 5.55 mmol), PdCl₂(PPh₃)₂ (137 mg, 0.19 mmol) and 5-fluoropyridin-3-ylboronic acid (391 mg, 2.78 mmol) in dioxane (30 mL) and water (5 mL) was stirred at 80 °C for 2 hrs under N₂ protection. The mixture was cooled to room temperature and diluted with water, extracted with ethyl acetate (30 mL x 3). The combined organic phases were washed with water and brine, dried over anhydrous Na₂SO₄, concentrated, and purified by column chromatography (petroleum ether/ethyl acetate = 100/1, v/v) to give Compound (**331c**) (320 mg, 40 %) as an oil. LCMS: 434[M+1]⁺.

### Example 240D

### Preparation of Compound (331)

To a solution of compound **(331c)** (320 mg, 0.74 mmol) in THF/H₂O (10/2 mL) was added 2 M HCl (5 mL) and was stirred at room temperature for 2 hrs. Then it was diluted with ethyl acetate (100 mL) and adjusted to pH 8 with saturated aq. NaHCO₃, washed with water and brine, dried with anhydrous Na₂SO₄, concentrated and purified by prep-HPLC to afford (**331**) (100 mg, 32%) as a white solid. LC-MS: (m/z) 406 [M+1]⁺; ¹H NMR (CDCl₃, 400 MHz) major characteristic peaks: *δ* (ppm) 0.05-0.19 (m, 2H), 0.57-0.70 (m, 3H), 1.06 (s, 3H), 1.17 (s, 3H), 6.08 (m, 1H), 6.29 (m, 1H), 7.37-7.41 (dt, *J* = 2.0, 8.0 Hz, 1H), 8.34 (d, *J* = 2.0 Hz, 1H), 8.45 (s, 1H).

### Example 241

### Preparation of Compounds (332), (333), and (334)

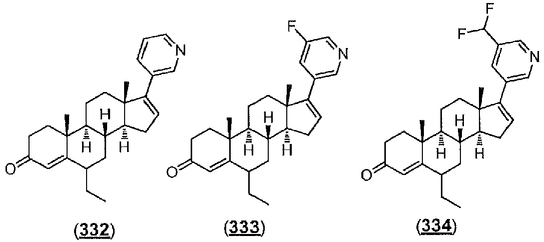

### Example 241A

### Preparation of Compound (332a)

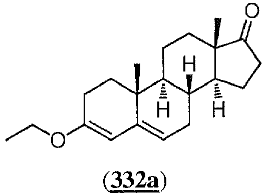

To a suspension of (10R,13S)-10,13-dimethyl-7,8,9,10,11,12,13,14,15,16-decahydro-1H-cyclopenta[a]phenanthrene-3,17(2H,6H)-dione (50.00 g, 174.60 mmol) in CH(OEt)₃ (60 mL) and EtOH (60 mL) was added Pyridine.HCl (807 mg, 7.00 mmol). The suspension was stirred at room temperature (or 40 °C) for overnight. The reaction mixture was poured into cold sat. aq. NaHCO₃ solution (100 mL). The solid was collected by filtration and dried to obtain Compound (**332a**) (57.20 g, 100%) as a white solid. LCMS: (m/z) 315 [M+1]⁺; ¹H NMR (DMSO-*d*₆, 400 MHz) major characteristic peaks: *δ* (ppm) 0.82 (s, 3H), 0.92 (s, 3H), 1.21 (t, *J* = 6.8 Hz, 3H), 3.70-3.76 (m, 2H), 5.13 (m, 1H), 5.17 (m, 1H).

### Example 241B

### Preparation of Compound (332b)

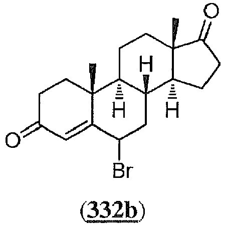

To a suspension of compound (**332a**) (28.5 g, 0.091 mol) and NaOAc (7.44 g, 0.091 mol) in acetone/H₂O (190/10 mL) was added NBS (17.82 g, 0.100 mol) at 0 °C. After the ccompletion of the addition, the mixture was stirred at room temperature for 1 hr. Then it was diluted with water (200 mL) and concentrated to remove acetone. A white solid was precipitated out. After filtration, the solid was washed with EtOH and dried to give Compound (**332b**) (31.60 g, 95%) as a white solid. LC-MS: (m/z) 365 [M+1]⁺; ¹H NMR (CDCl₃, 400 MHz) major characteristic peaks: *δ* (ppm) 0.99 (s, 3H), 1.57 (s, 3H), 5.01-5.04 (m, 1H), 5.93 (s, 1H).

### Example 241C

### Preparation of Compound (332c)

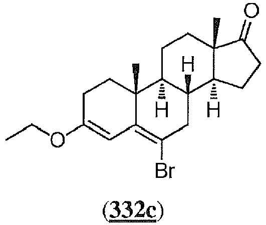

To a suspension of compound (**332b**) (51.60 g, 0.141mol) in CH(OEt)₃ (50 mL) and EtOH (50 mL) was added Pyridine.HCl (820 mg, 7.07mmol). The suspension was stirred at room temperature for 1 hr, before it was poured into cool sat. aq. NaHCO₃ solution (100 mL) to form solid. The solid was filtered and washed with EtOH. It was dried to give Compound (**332c**) (27.00 g, 49%) as a white solid. LC-MS: (m/z) 393 [M+1]⁺; ¹H NMR (DMSO-*d*₆, 400 MHz) major characteristic peaks: *δ* (ppm) 0.82 (s, 3H), 0.97 (s, 3H), 1.25 (t, *J* = 7.2 Hz, 3H), 3.77-3.83 (m, 2H), 5.49 (s, 1H).

### Example 241D

### Preparation of Compound (332d)

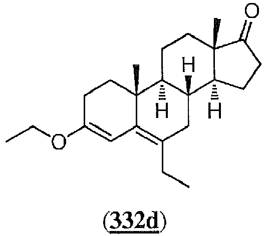

A solution of compound (**332c**) (15.00 g, 38.26 mmol), K₂CO₃ (13.20 g, 95.65 mmol), PCl₂(PPh₃)₂ (2.67 g, 3.83 mmol) and EtB(OH)₂ (3.11 g, 42.09 mmol) in dioxane/ H₂O (150/15 mL) was stirred at 100 °C for overnight under N₂. The mixture was diluted with ethyl acetate (200 mL), washed water and brine, dried with anhydrous Na₂SO₄, concentrated, purified by column chromatography (petroleum ether/ethyl acetate = 20/1, v/v) to obtain compound (**332d**) (9.20 g, 70%) as a white solid. LCMS: (m/z) 343 [M+1]⁺; ¹H NMR (DMSO-*d*₆, 400 MHz) major characteristic peaks: *δ* (ppm) 0.82 (s, 3H), 0.89 (s, 3H), 0.92 (t, *J* = 7.2 Hz, 3H), 1.23 (t, *J* = 6.8 Hz, 3H), 3.72-3.83 (m, 2H), 5.38 (s, 1H).

### Example 241E

### Preparation of Compound (332e)

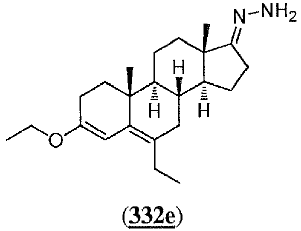

To a suspension of compound (**332d**) (6.00 g, 17.54 mmol) in EtOH (10 mL) was added NH₂NH₂ liquid (80%, 7 mL) and stirred at 60 °C for 2 hrs. The mixture was poured into ice water to form white solid. After filtration and drying, it gave Compound (**332e**) (6.50 g, 100%) as a white solid. LCMS: (m/z) 357 [M+1]⁺; ¹H NMR (DMSO-*d*₆, 400 MHz) major characteristic peaks: *δ* (ppm) 0.78 (s, 3H), 0.88 (s, 3H), 0.92 (t, J= 7.2 Hz, 3H), 1.23 (t, J = 6.8 Hz, 3H), 3.70-3.79 (m, 2H), 5.31 (s, 2H), 5.37 (s, 1H).

### Example 241F

### Preparation of Compound (332f)

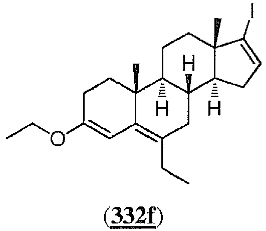

To a solution of compound (**332e**) (6.50 g, 18.26 mmol) and Et₃N (20 mL) in DCM (20 mL) was added dropwise the solution of I₂ (6.96 g, 27.39 mmol) in THF (10 mL) at 0 °C. The solution was stirred at 0 °C for 2 hrs. Then it was quenched with Na₂SO₃ solution to destroy the excess I₂. The mixture was diluted with ethyl acetate (150 mL), washed with water and brine, dried with anhydrous Na₂SO₄, concentrated, and purified by column chromatography (petroleum ether/ethyl acetate = 100/1, v/v) to obtain compound (**332f**) (4.80 g, 58%) as a white solid. LC-MS: (m/z) 453 [M+1]⁺; ¹H NMR (DMSO-*d*₆, 400 MHz) major characteristic peaks: *δ* (ppm) 0.70 (s, 3H), 0.89-0.94 (m, 6H), 1.23 (t, *J*= 6.8 Hz, 3H), 3.72-3.82 (m, 2H), 5.38 (s, 1H), 6.19 (s, 1H).

### Example 241G

### Preparation of Compounds (332g), (333g), (334g)

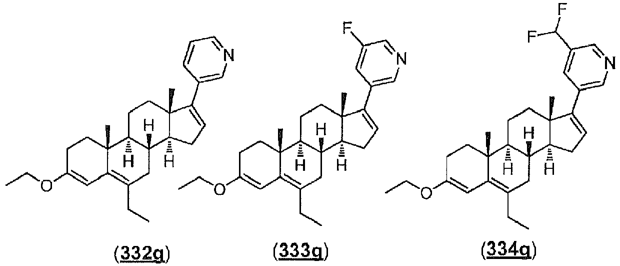

A solution of compound (**332f**) (900 mg, 1.99 mmol), K₂CO₃ (824 mg, 5.97 mmol), PCl₂(PPh₃)₂ (140 mg, 0.20 mmol) and pyridin-3-yl-3-boronic acid (368 mg, 2.99 mmol) in dioxane/ H₂O (30/3 mL) was stirred at 80 °C for 5 hrs under N₂. The mixture was diluted with ethyl acetate (100 mL), washed with water and brine, dried with anhydrous Na₂SO₄, concentrated, and purified by column chromatography (petroleum ether/ethyl acetate = 20/1, v/v) to obtain compound (**332g**) (380 mg, 49%) as a white solid. LC-MS: (m/z) 404 [M+1]⁺.

Using a synthetic procedure and conditions similar to the preparation of compound (**332g**), replacing pyridin-3-ylboronic acid with the corresponding pyridin-3-ylboronic acids Compounds (**333g**) and **(334g)** were prepared. Compound (**333g**): yield 55%. LCMS: (m/z) 421 [M+1]⁺. Compound (**334g**): yield 44%. LCMS: 454 [M+1]⁺.

### Example 241H

### Preparation of Compounds (332), (333), and (334)

To a solution of compound (**332g**) (380 mg, 0.94 mmol) in THF/H₂O (10/2 mL) was added 2 M HCl (1 mL) and stirred at room temperature for 2 h. Then it was diluted with ethyl acetate (100 mL) and adjusted to pH8 with saturated aq. NaHCO₃ washed with water and brine, dried with anhydrous Na₂SO₄, concentrated, purified with column chromatography (petroleum ether/ethyl acetate = 2/1, v/v) to obtain (**332**) (200 mg, 57%) as a white solid. LC-MS: (m/z) 376 [M+1]⁺; ¹H NMR (CDCl₃, 400 MHz) major characteristic peaks: *δ* (ppm) 0.95 (t, *J* = 7.6 Hz, 3H), 1.07 (s, 3H), 1.23 (s, 3H), 5.81 (s, 1H), 5.10 (s, 1H), 7.21-7.25 (m, 1H), 7.64 (d, *J*= 10.0 Hz, 1H), 8.47 (d, *J* = 6.4 Hz, 1H), 8.62(s, 1H).

Using a synthetic procedure and conditions similar to the preparation of compound **(332),** replacing (**332g**) with (**333g**) and (**334g**) Compounds (**333**) and (**334**) were prepared.

Compound (**333**) (yield 47%) as a white solid: LC-MS: (m/z) 394 [M+1]⁺; ¹H NMR (CDCl₃, 400 MHz) major characteristic peaks: *δ* (ppm) 0.97 (t, *J* = 7.2 Hz, 3H), 1.07 (s, 3H), 1.23 (s, 3H), 5.81 (s, 1H), 6.06 (m, 1H), 7.34-7.38 (m, 1H), 8.33 (s, 1H), 8.44(s, 1H).

Compound (**334**) (yield 48%) as a white solid: LC-MS: (m/z) 426 [M+1]⁺; ¹H NMR (CDCl₃, 400 MHz) major characteristic peaks: *δ* (ppm) 0.97 (t, *J* = 7.2 Hz, 3H), 1.09 (s, 3H), 1.23 (s, 3H), 5.81 (s, 1H), 6.11 (m, 1H), 7.72 (t, *J*= 56 Hz, 1H), 7.81 (s, 1H), 8.61 (s, 1H), 8.73 (s, 1H).

### Example 242

### Preparation of Compounds (335) and (336)

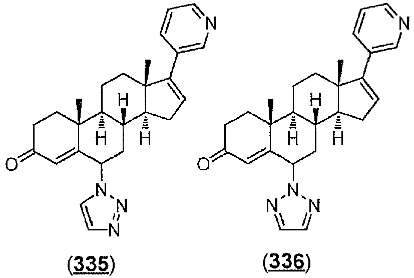

### Example 242A

### Preparation of Compounds (335a) and (336a)

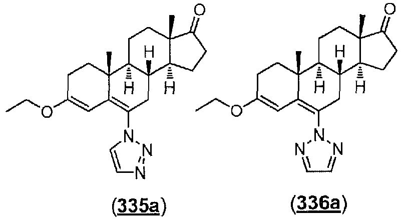

A mixture of compound (**332c**) (500mg, 1.3mmol), 1*H*-1,2,3-triazole (790 mg, 11.4mmol), K₂CO₃ (350 mg, 2.5 mmol), L-proline (88 mg, 0.8 mmol) and CuI (100 mg, 0.5 mmol) in dry DMSO (10 mL) was microwaved at 160 °C under N₂ protection for 20 minutes. The mixture was cooled to room temperature and diluted with water, extracted with ethyl acetate (50 mL x 3). The combined organic phases were washed with water, brine, dried over Na₂SO₄, concentrated to get crude product. The crude product was purified by prep-TLC (petroleum ether/ethyl acetate = 2/1, v/v) to give Compounds **(335a)** (60 mg) and **(336a)** (60 mg) as white solids.

Compound (**335a**): LC-MS(m/z): (m/z) 382 (M+1). ¹H NMR (CDCl₃, 400 MHz) major characteristic peaks: *δ* (ppm) 0.95 (s, 3H), 1.13 (s, 3H), 1.21 (t, *J* = 5.6 Hz, 3H), 3.55 (q, *J* = 5.6 Hz, 2H), 4.71 (m, 1H), 7.59 (s, 1H), 7.75 (s, 1H).

Compound (**335a**): LC-MS (m/z): (m/z) 382 (M+1). ¹H NMR (CDCl₃, 400 MHz) major characteristic peaks: *δ* (ppm) 0.94 (s, 3H), 1.14 (s, 3H), 1.23 (t, *J* = 5.6 Hz, 3H), 3.60 (q, *J* = 5.6 Hz, 2H), 4.95 (m, 1H), 7.72 (s, 2H).

### Example 242B

### Preparation of Compounds (335b) and (336b)

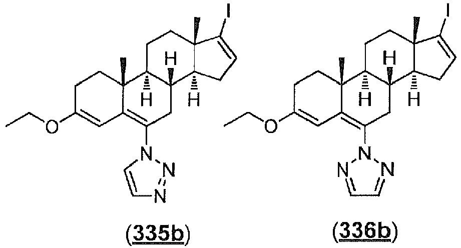

A mixture of compound (**335a**) (60 mg, 0.16 mmol) and N₂H₄.H₂O (80%, 98 mg, 1.60 mmol) in EtOH (10 mL) was stirred at 80 °C for 3 hrs. The mixture was concentrated and dried to give a white solid. The solid and Et₃N (217 mg, 2.15 mmol) were dissolved in DCM (5 mL). To this solution, a solution of I₂ (82 mg, 0.32 mmol) in THF (5 mL) was dropwise at 0 °C. It was stirred for 30 minutes and concentrated to remove the most of solvents. The residue was diluted with water, extracted with ethyl acetate (50 mL) and the organic phase was washed with water, brine, dried over Na₂SO₄, and concentrated to yield a crude product. The crude product was purified by prep-TLC (petroleum ether/ethyl acetate = 5/1, v/v) to afford Compound (**335a**) (37 mg, 33 %) as a white solid. ¹H NMR (CDCl₃, 400 MHz) major characteristic peaks: *δ* (ppm) 0.81 (s, 3H), 1.14 (s, 3H), 1.21 (t, *J*= 5.6 Hz, 3H), 3.56 (q, *J*= 5.6 Hz, 2H), 4.70 (m, 1H), 6.14 (m, 1H), 7.59 (m, 2H), 7.74 (m, 1H).

Using the similar procedure and under the same conditions, replacing (**335a**) with (**336a**), Compound (**336b**) was prepared in 38% yield. ¹H NMR (CDCl₃, 400 MHz) major characteristic peaks: *δ* (ppm) 0.81 (s, 3H), 1.15 (s, 3H), 1.22 (t, *J* = 5.6 Hz, 3H), 3.60 (q, *J* = 5.6 Hz, 2H), 4.93 (m, 1H), 6.14 (m, 1H), 7.71 (s, 2H).

### Example 242C

### Preparation of Compounds (335c) and (336c)

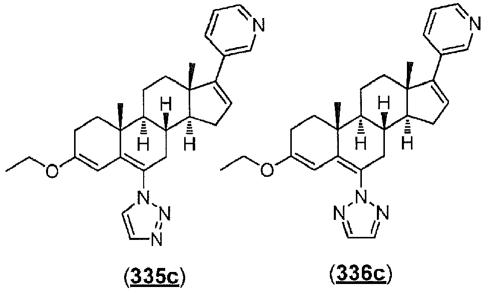

A mixture of compound **(335b)** (37 mg, 0.075 mmol), pyridin-3-ylboronic acid (14 mg, 0.11 mmol), K₂CO₃ (26 mg, 0.18 mmol), Pd(PPh₃)₂Cl₂ (6 mg, 0.007 mmol) in dioxane (10 mL) and water (2 mL) was stirred at 80 °C under N₂ protection for 2 hrs. The mixture was cooled to room temperature and diluted with water, extracted with ethyl acetate (50 mL x 3). The combined organic phases were washed with water, brine, dried over Na₂SO₄, and concentrated to give a crude product. The crude product was purified by prep-TLC (petroleum ether/ethyl acetate = 3/2, v/v) to afford Compound (**335c**) (10 mg, 30%) as a white solid. LC-MS (m/z): 443 (M+1).

Using the similar procedure and under the same conditions, replacing **(335b)** with (**336b**), Compound (**336c**) was prepared in 30% yield. LCMS (m/z): 443 (M+1).

### Example 242D

### Preparation of Compound (335) and (336)

To the mixture of compound (**335c**) (10 mg, 0.02 mmol) in THF (3 mL) was added 2 M HCl (2 mL) at room temperature. After stirred for 4 hrs, the ixture was diluted with sat. NaHCO₃ solution, extracted with ethyl acetate (50 mL x 3). The combined organic phases were washed with water, brine, dried over Na₂SO₄, and concentrated to afford a crude product. The crude product was purified by prep-TLC (ethyl acetate as eluent) to afford Compound **(335)** (6.5 mg, 69%) as a white solid. LC-MS (m/z): 415 (M+1). ¹H NMR (CDCl₃, 400 MHz) major characteristic peaks: *δ* (ppm) 1.11 (s, 3H), 1.42 (s, 3H), 4.75 (s, 1H), 5.55 (m, 1H), 6.00 (s, 1H), 7.24 (m, 1H), 7.59 (d, *J* = 0.8 Hz, 1H), 7.66 (m, 1H), 7.80 (d, *J* = 0.8 Hz, 1H), 8.49 (m, 1H), 8.63 (s, 1H).

Using the similar procedure and under the same conditions, replacing (**335c**) with (**336c**), Compound (**336**) was prepared in 60% yield. LC-MS (m/z): 415 (M+1) ¹H NMR (CDCl₃, 400 MHz) major characteristic peaks: *δ* (ppm) 1.15 (s, 3H), 1.40 (s, 3H), 4.49 (m, 1H), 5.55 (m, 1H), 6.27 (m, 1H), 7.70 (m, 3H), 8.16 (m, 1H), 8.64 (m, 1H), 8.87 (s, 1H).

### Example 243

### Preparation of Compound (337) and (338)

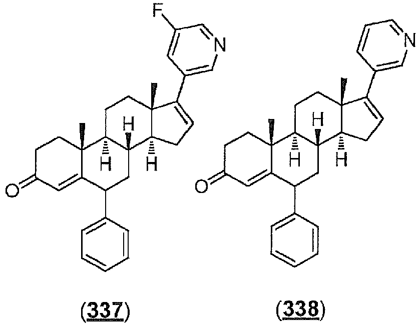

### Example 243A

### Preparation of Compound (337a)

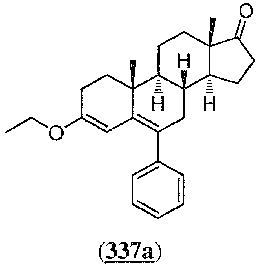

To a solution of Compound (**332c**) (7.3 g, 18.7 mmol) in 1, 4-dioxane (120 mL) was added K₂CO₃ (5.2 g, 37.4 mmol)phenylboronic acid (2.5 g, 20.5 mmol), water (40 mL), Pd(PPh₃)₂Cl₂ (656 mg, 0.935 mmol). The mixture was stirred at 100 °C for 1 hr under N₂. The mixture was diluted with EtOAc (500 mL), washed with water (300 mL x 3), brine (300 mL), dried over anhydrous Na₂SO₄, and purified by column chromatography on silica (petroleum ether/ethyl acetate = 60/1, v/v) to give Compound (**337a**) (5.8 g, 80%) as a white solid. LC-MS: (m/z) 391(M+1). ¹H NMR (CDCl₃, 400 MHz) peaks: *δ* (ppm) 0.857 (s, 3H), 1.008 (s, 3H), 1.084 (t, *J*= 6.8 Hz, 3H), 3.401 (m, 2H), 5.054 (s, 1H), 7.152 (m, 2H), 7.199 (m, 1H), 7.306 (m, 2H).

### Example 243B

### Preparation of Compound (337b)

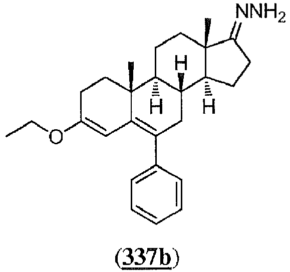

To a solution of Compound (**337a**) (3 g, 7.7 mmol) in EtOH (60 mL) was added aqueous hydrazine solution (80%, 4.8 g, 77 mmol). The mixture was stirred at 100 °C for 1hr. The mixture was concentrated to remove EtOH. It was then diluted with EtOAc (300 mL) washed with water (200 mL x 3), brine (200 mL), dried over anhydrous Na₂SO₄, and concentrated to give Compound **(337b)** (3.1 g, 100%) as a white solid. LC-MS: (m/z) 405 (M+1).

### Example 243C

### Preparation of Compound (337c)

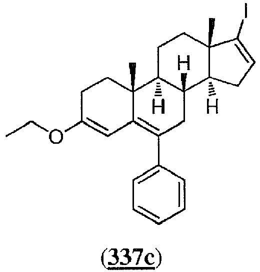

To a solution of Compound **(337b)** (3.1 g, 7.7 mmol), NEt₃ (3.9 g, 38.5 mmol) in DCM (20 mL) was added dropwise a solution of I₂ (3.9 g, 15.4 mmol) in THF (10 mL) at 0 °C. The mixture was stirred at 0 °C for 10 min. The mixture was added saturated aqueous Na₂SO₃ solution (20 mL), diluted with DCM (200 mL), washed with water (100 mL x 3), brine (100 mL), dried over anhydrous Na₂SO₄, and purified by column chromatography on silica (petroleum ether/ethyl acetate = 100/1, v/v) to give Compound (**337c**) (2.32 g, 60%) as a white solid. ¹H NMR (CDC13, 400 MHz): *δ* (ppm) 0.820 (s, 3H), 1.227 (t, *J* = 7 Hz, 3H), 1.355 (s, 3H), 3.571 (m, 1H), 3.697 (m, 2H), 5.184 (m, 1H), 6.134 (m, 1H), 7.102 (m, 2H), 7.227 (m, 1H), 7.304 (m, 2H).

### Example 243D

### Preparation of Compound (337d)

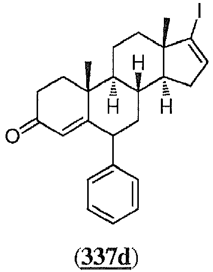

To a solution of Compound (**337c**) (2.32 g, 4.64 mmol) in THF (30 mL) was added con. aqueous HCl solution (0.5 mL). The mixture was stirred at r.t. for 2 hrs. The mixture was added saturated aqueous NaHCO₃ solution to adjust PH > 7, diluted with EtOAc (250 mL), washed with water (150 mL x 3), brine (150 mL), dried over anhydrous Na₂SO₄, and purified by column chromatography on silica (petroleum ether/ethyl acetate = 25/1, v/v) to afford Compound (**337d**) (1.8 g, 81%) as a yellow solid. LC-MS: (m/z) 473 (M+1). ¹H NMR (CDCl₃, 400 MHz): *δ* (ppm) 0.820 (s, 3H), 1.355 (s, 3H), 3.566 (m, 1H), 5.184 (m, 1H), 6.128 (m, 1H), 7.102 (m, 2H), 7.227 (m, 1H), 7.304 (m, 2H).

### Example 243E

### Preparation of Compounds (337) and (338)

To a solution of Compound (**337d**) (200 mg, 0.42 mmol) in 1, 4-dioxane (10 mL) was added K₂CO₃ (292 mg, 2.12 mmol), 5-fluoropyridin-3-ylboronic acid (77 mg, 0.63 mmol), water (1 mL), Pd(PPh₃)₂Cl₂ (28 mg, 0.04 mmol). The mixture was stirred at 80 °C for 2 hrs under N₂. The mixture was filtered to remove the brown solid. The filtrate was diluted with EtOAc (50 mL), dried over anhydrous Na₂SO₄, and purified by column chromatography (petroleum ether/ethyl acetate = 4/1, v/v) to afford (**337**) (25 mg, 14%) as a white solid. LC-MS: (m/z) 442 (M+1). ¹H NMR (CDC13, 400 MHz) peaks: *δ* (ppm) 1.107 (s, 3H), 1.385 (s, 3H), 3.605 (m, 1H), 5.197 (m, 1H), 6.061 (m, 1H), 7.124 (m, 2H), 7.260 (m, 1H), 7.318 (m, 2H), 7.373 (m, 1H), 8.347(m, 1H), 8.456(m, 1H).

Using the similar procedure and under the same conditions, replacing 5-fluoropyridin-3-ylboronic acid with pyridin-3-ylboronic acid, Compound **(338)** was prepared in 6% yield. LC-MS: (m/z) 424 (M+1). ¹H NMR (CDCl₃, 400 MHz): *δ* (ppm) 1.105 (s, 3H), 1.385 (s, 3H), 3.610 (m, 1H), 5.197 (m, 1H), 6.014 (m, 1H), 7.126 (m, 2H), 7.275 (m, 2H), 7.318 (m, 2H), 7.694 (m, 1H), 8.482 (m, 1H), 8.632 (m, 1H).

### Example 244

### Preparation of Compound (339)

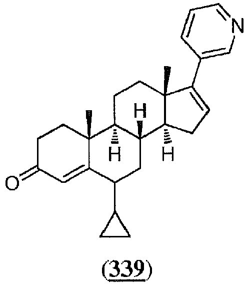

### Example 244A

### Preparation of Compound (339a)

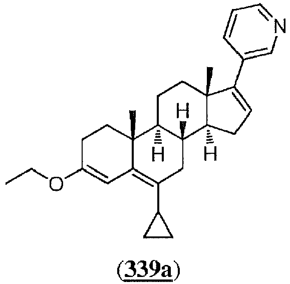

A solution of compound (**331b**) (200 mg, 0.43 mmol), K₂CO₃ (178 mg, 1.29 mmol), PdCl₂(PPh₃)₂ (32 mg, 0.04 mmol) and pyridin-3-ylboronic acid (80 mg, 0.65 mmol) in dioxane (10 mL) and water (3 mL) was stirred at 80 °C for 2 hrs under N₂ protection. Then it was cooled to room temperature, diluted with water, and extracted with ethyl acetate (30 mL x 3). The combined organic phases were washed with water and brine, dried over anhydrous Na₂SO₄, concentrated, and purified by prep-TLC (petroleum ether/ethyl acetate = 2/1, v/v) to obtain Compound (**339a**) (40 mg, 22 %) as a white solid. LC-MS: (m/z) 416 [M+1]⁺.

### Example 244B

### Preparation of Compound (339)

To a solution of compound (**339a**) (40 mg, 0.10 mmol) in THF/H₂O (10/2 mL) was added 2 M HCl (2 mL) and was stirred at room temperature for 2 hrs. Then it was diluted with ethyl acetate (100 mL) and adjusted to pH 8 with saturated NaHCO₃. The mixture then was washed with water and brine, dried with anhydrous Na₂SO₄, concentrated, and purified by prep-HPLC to obtain **(339)** (10 mg, 27%) as an colorless oil. LC-MS: (m/z) 388[M+1]⁺; ¹H NMR (CDCl₃, 400 MHz) major characteristic peaks: *δ* (ppm) 0.04-0.16 (m, 2H), 0.56-0.69 (m, 3H), 1.05 (s, 3H), 1.16 (s, 3H), 5.99 (m, 1H), 6.28 (m, 1H), 7.21-7.24 (dd, *J* = 2.4, 8.0 Hz, 1H), 7.63 (d, *J*= 8.0 Hz, 1H), 8.46(m, 1H), 8.61 (s, 1H).

### Example 245

### Preparation of Compound (340)

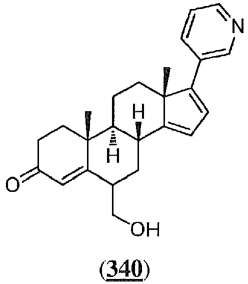

To a solution of Compound (**82**) (100 mg, 0.29 mmol) in methanol (1.5 mL) was added pyrrolidine (41 mg, 0.28 mmol). The reaction mixture was heated to reflux for 15 min. the reaction mixture was cooled to room temperature and the solid was filtered. After filtered and washed, the solid was dissolved in ethanol/toluene (4 mL/2 mL) and formaldehyde (37 percent in water, 117 mg, 1.45 mmol) was added. The reaction mixture was stirred for 30 min at room temperature. The solvent was removed in vacuum and the crude product was purified by prep-TLC (CH₂Cl₂/MeOH = 10/1, v/v) to give Compound (**340**) (20 mg, 18%) as a white solid. LC-MS (ESI): (m/z) 376 (M+H)⁺; ¹HNMR (CDCl₃, 400 MHz) major characteristic peaks: *δ* (ppm) 1.19 (s, 3H), 1.33 (s, 3H), 3.70∼3.75 (m, 1H), 3.84∼3.89 (m, 1H), 5.88 (s, 1H), 6.06∼6.07 (m, 1H), 6.79 (d, *J* = 2.4 Hz, 1H), 7.24∼7.25 (m, 1 H), 7.75∼7.78 (m, 1H), 8.42 (d, *J* = 3.6 Hz, 1H), 8.74 (s, 1H).

### Example 246

### Preparation of Compound (341) and (342)

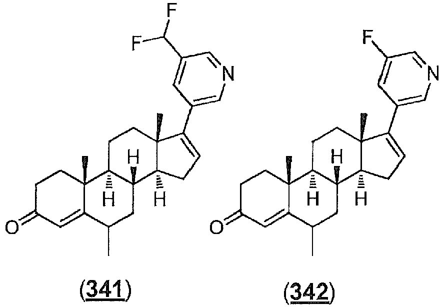

A mixture of Compound (**309h**) (615 mg, 1.5 mmol), (5-(difluoromethyl) pyridin-3-yl)boronic acid (518.8 mg, 3.0 mmol), PdCl₂(dppf) (105 mg) and K₂CO₃ (621 mg, 4.5 mmol) in dioxane (20 mL) and water (5 mL) was heated to 80 °C for 2.5 hrs. The mixture was diluted with water (20 mL), extracted with EtOAc (3 x 20 mL). The combined organic layers were washed with brine (30 mL), dried over Na₂SO₄, and concentrated to give a crude product. The crude product was purified by silica gel column chromatography (petroleum ether/ EtOAc = 3/1, v/v) to afford Compound (**341**) (280 mg, 45%) as a white solid. LC-MS: (m/z) 412 [M+1]⁺; ¹H NMR (CDCl₃, 400 MHz) major characteristic peaks: *δ* (ppm) 1.08 (s, 3H), 1.11-1.12 (d, *J*= 6.8 Hz, 3H), 1.24 (s, 3H), 5.82 (d, *J* = 1.2 Hz, 1H), 6.08-6.96 (dd, *J* = 3.2, 2.0 Hz, 1H), 6.57-6.85 (t, *J* = 55.8 Hz, 1H), 7.78 (s, 1H), 8.61 (s, 1H), 8.73 (s, 1H).

Using the similar procedure and under the same conditions, replacing (5-(difluoromethyl)pyridin-3-yl)boronic acid with 5-fluoropyridin-3-ylboronic acid, Compound (**342**) was prepared in 97% yield. LCMS: (m/z) 380(M+1). ¹H NMR (CDC13, 400 MHz) peaks: *δ* (ppm) 1.07 (s, 3H), 1.11 (d, J= 6.0 Hz, 3H), 1.24 (s, 3H), 5.82 (s, 1H), 6.08 (m, 1H), 7.39 (m, 1H), 8.35 (s, 1H), 8.45 (s, 1H).

### Example 247

### Preparation of Compound (343)

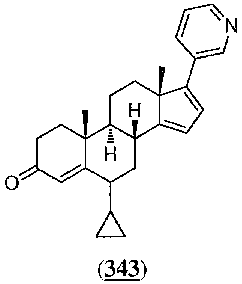

### Example 247A

### Preparation of Compound (343a)

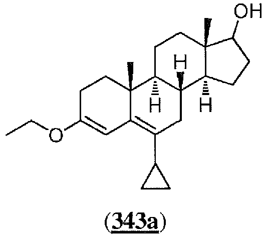

To a solution of Compound (**331a**) (1.28 g, 3.6 mmol) in THF (30 mL) and MeOH (30 mL) was added NaBH₄ (11 mg, 0.28 mmol) at 0 °C. The mixture was stirred at room temperature for 20 minutes and diluted with EtOAc (30 mL), washed with water (20 mL x 3), brine (20 mL), dried over anhydrous Na₂SO₄, and concentrated to give Compound (**343a**) (1.36 g) as a white solid.
LCMS: (m/z) 357 (M+1).

### Example 247B

### Preparation of Compound (343b)

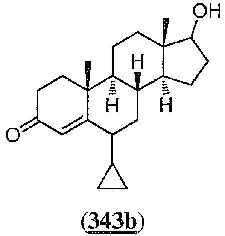

To a solution of Compound (**343a**) (1.36 g, 3.8 mmol) in THF (10 mL) was added 2M HCl (3 mL) at room temperature. The mixture was stirred at room temperature for 21 hrs. The mixture was added saturated aqueous NaHCO₃ solution to adjust pH > 7, diluted with EtOAc (30 mL), washed with water (20 mL x 3), brine (20 mL), and dried over anhydrous Na₂SO₄. After the filtration, the filtrate was concentrated to give Compound (**343b**) (1.27 g, 100%) as a white solid. LCMS: (m/z) 329 (M+1). ¹H NMR (CDCl₃, 400 MHz) peaks: *δ* (ppm) 0.008 (m, 2H), 0.62 (m, 3H), 0.78 (s, 3H), 1.12 (s, 3H), 3.65 (t, J= 4.8 Hz, 1H), 6.25 (s, 1H),.

### Example 247C

### Preparation of Compound (343c)

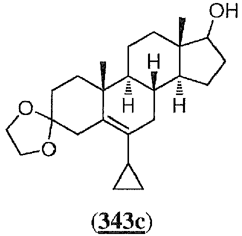

To a solution of Compound (**343b**) (1.27 g, 3.87 mmol) in THF (20 mL) was added CH(OEt)₃ (2.5 mL), p-TsOH (37 mg, 0.19 mmol), ethane-1,2-diol (4.5 mL). The mixture was stirred at room temperature for 3 hrs. The mixture was added saturated aqueous NaHCO₃ solution to adjust pH > 7, and was extracted by ethyl acetate (50 mL x 3). The combined organic phase was washed with water, brine, and dried over Na₂SO₄. After filtration the filtrate was concentrated to give a crude product, which was purified by gel column chromatography (petroleum ether/ethyl acetate = 10/1, v/v) to yield Compound (**343c**) (0.36 g, 25%) as a white solid. LC-MS: (m/z) 373 (M+1). ¹H NMR (CDCl₃, 400 MHz) peaks: *δ* (ppm) 0.30 (m, 1H), 0.51 (m, 4H), 0.66 (s, 3H), 1.03 (s, 3H), 3.11 (m, 1H), 3.62 (m, 1H), 3.96 (m, 4H).

### Example 247D

### Preparation of Compound (343d)

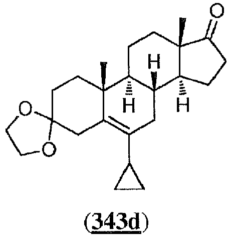

To a solution of Compound (**343c**) (0.36 g, 1.0 mmol) in DCM (10 mL) was added DMP (0.49 g, 1.2 mmol) at 0 °C. The mixture was stirred at room temperature for 1 hr. The mixture was added EtOAc (100 mL), and filtered to remove the white solid. The filtrate was concentrated and purified by prep-TLC (petroleum ether/ethyl acetate = 5/1, v/v) to afford Compound (**343d**) (0.32 g, 89%) as a white solid. LC-MS: (m/z) 371 (M+1). ¹H NMR (CDC13, 400 MHz) peaks: *δ* (ppm) 0.30 (m, 1H), 0.51 0.66 (m, 3H), 0.87 (s, 3H), 1.04 (s, 3H), 3.13 (m, 1H), 3.96 (m, 4H).

### Example 247E

### Preparation of Compound (343e)

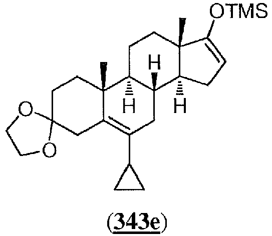

To a solution of (i-Pr)₂NH (262 mg, 2.59 mmol) in THF (10 mL) cooled to -78 °C was added a solution of n-BuLi (2.5 M) in hexane (1.1 mL, 2.59 mmol). The mixture was stirred at-78 °C for 15 min under N₂. A solution of Compound (**343d**) (0.32 g, 0.86 mmol) in THF (10 mL) was added dropwise over 20 minutes, and the mixture was stirred at -78 °C for 15 min under N₂. Then Et₃N (0.6 mL), TMSCl (0.5 mL) were added. The mixture was warmed to r.t. and stirred for 30 min under N₂. The mixture was added saturated aqueous NaHCO₃ solution (30 mL), diluted with DCM (200 mL), washed with water (150 mL x 3), brine (150 mL), dried over anhydrous Na₂SO₄, and concentrated to give Compound (**343e**) (0.35 g, 92%) as a white solid.

### Example 247F

### Preparation of Compound (343f)

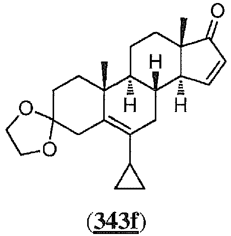

To a solution of Compound (**343e**) (0.35 g, 0.79 mmol) in DCM (12 mL) and MeCN (4 mL) was added Pd(OAc)₂ (0.18 g, 0.79 mmol). The mixture was stirred at 35 °C for 2 hrs. The mixture was filtered to remove the brown solid. The filtrate was concentrated, and purified by prep-TLC (petroleum ether/ethyl acetate = 5/1, v/v) to give Compound (**343f**) (270 mg, 93%) as a white solid. LC-MS: (m/z) 369 (M+1). ¹H NMR (CDC13, 400 MHz) peaks: *δ* (ppm) 0.35 (m, 1H), 0.54∼0.69 (m, 3H), 1.07 (s, 3H), 1.09 (s, 3H), 3.15 (m, 1H), 3.96 (m, 4H), 6.04 (m, 1H), 7.54 (m, 1H).

### Example 247G

### Preparation of Compound (343g)

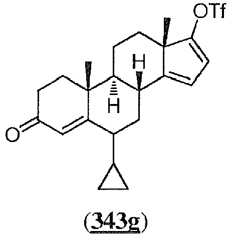

To a solution of Compound (**343f**) (270 mg, 0.73 mmol), imidazole (149 mg, 2.20 mmol) in DCM (15 mL) was added dropwise Tf₂O (412 mg, 1.46 mmol) at room temperature. The mixture was stirred at room temperature for 30 minutes. The mixture was added saturated aqueous NaHCO₃ solution to adjust pH > 7, diluted with DCM (50 mL), washed with water (50 mL x 3), brine (50 mL), dried over anhydrous Na₂SO₄, and purified by pre-TLC (petroleum ether/ethyl acetate = 5/1, v/v) to yield Compound (**343g**) (30 mg, 9%) as a colorless oil. LC-MS: (m/z) 457(M+1).

### Example 247H

### Preparation of Compound (343)

To a solution of Compound (**343g**) (30 mg, 0.07 mmol) in 1, 4-dioxane (5 mL) was added K₂CO₃ (23 mg, 0.16 mmol), pyridin-3-ylboronic acid (12 mg, 0.10 mmol), water (1 mL), Pd(PPh₃)₂Cl₂ (5 mg, 0.01 mmol). The mixture was stirred at 80 °C for 2 hrs under N₂. The mixture was filtered to remove the brown solid, diluted with DCM (50 mL), washed with water (30 mL x 3), brine (30 mL), dried over anhydrous Na2SO4, and purified by prep-TLC (petroleum ether/ethyl acetate = 2/1, v/v) to give (343) (9 mg, 36%) as a white solid. LC-MS: (m/z) 386 (M+1). ¹H NMR (CDCl3, 400 MHz) peaks: *δ* (ppm) 0.10 (m, 2H), 0.65 (m, 3H), 1.21 (s, 3H), 1.25 (s, 3H), 6.05 (m, 1H), 6.31 (s, 1H), 6.80 (d, 1H), 7.25 (dd, *J*= 8.0, 4.8 Hz, 1H), 7.77 (dt, *J*= 8.0, 2.0 Hz, 1H), 8.42 (dd, *J* = 1.6,4.8 Hz, 1H), 8.76 (d, *J*= 2.4 Hz, 1H).

### Example 248

### Preparation of Compound (344)

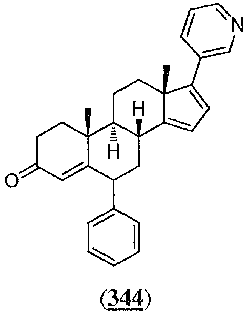

### Example 248A

### Preparation of Compound (344a)

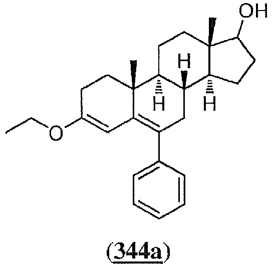

To a solution of Compound (**337a**) (100 mg, 0.26 mmol) in DCM (2 mL) was added a solution of NaBH₄ (11 mg, 0.28 mmol) in EtOH (1.5 mL) at 0 °C. The mixture was stirred at room temperature for 2 hrs and diluted with EtOAc (30 mL), washed with water (20 mL x 3), brine (20 mL), dried over anhydrous Na₂SO₄, and concentrated to yield Compound (**344a**) (100 mg, 100%) as a white solid. LC-MS: (m/z) 393 (M+1). ¹H NMR (DMSO-*d*₆, 400 MHz) peaks: *δ* (ppm) 0.697 (s, 3H), 0.983 (s, 3H), 1.079 (t, *J*= 7 Hz, 3H), 3.408 (m, 3H), 4.457 (m, 1H), 5.039 (s, 1H), 7.122 (m, 2H), 7.182 (m, 1H), 7.287 (m, 2H).

### Example 248B

### Preparation of Compound (344b)

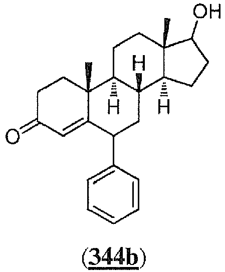

To a solution of Compound (**344a**) (100 mg, 0.255 mmol) in THF (2 mL) was added con. HCl (0.05 mL). The mixture was stirred at room temperature for 2 hrs. The mixture was added saturated aqueous NaHCO₃ solution to adjust pH > 7, diluted with EtOAc (30 mL), washed with water (20 mL x 3), brine (20 mL), dried over anhydrous Na₂SO₄, and purified by column chromatography (petroleum ether/ethyl acetate = 2/3, v/v) to give Compound (**343b**) (58 g, 62%) as a white solid.
LC-MS:m/z: 365 (M+1). ¹H NMR (CDCl₃, 400 MHz) peaks: *δ* (ppm) 0.829 (s, 3H), 1.340 (s, 3H), 3.539 (m, 1H), 3.661 (t, *J*= 8.8 Hz, 1H), 5.164 (m, 1H), 7.090 (m, 2H), 7.218 (m, 1H), 7.297 (m, 2H).

### Example 248C

### Preparation of Compound (344c)

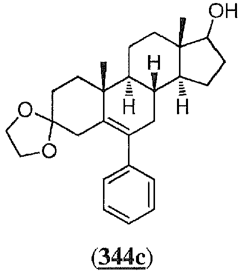

To a solution of Compound (**344b**) (2.6 g, 6.63 mmol) in THF (20 mL) was added CH(OEt)₃ (2.5 mL), p-TsOH (160 mg), ethane-1,2-diol (35 mL). The mixture was stirred at room temperature for 1 h. The mixture was added saturated aqueous NaHCO₃ solution to adjust pH > 7, filtered and dried to give Compound (**344c**) (1.56 g, 58%) as a white solid._LC-MS: (m/z) 409 (M+1). ¹H NMR (CDCl₃, 400 MHz) peaks: *δ* (ppm) 0.797 (s, 3H), 1.133 (s, 3H), 3.635 (m, 1H), 3.730 (m, 4H), 7.121 (m, 2H), 7.200 (m, 1H), 7.277 (m, 2H).

### Example 248D

### Preparation of Compound (344d)

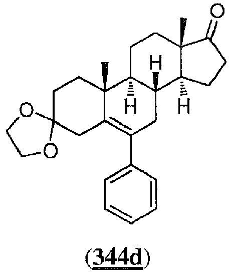

To a solution of Compound (**343c**) (1.56 g, 3.8 mmol) in DCM (30 mL) was added DMP (1.78 g, 4.2 mmol) at 0 °C. The mixture was stirred at 0 °C for 1 h. The mixture was added EtOAc (100 mL), filtered to remove the white solid. The filtrate was concentrated, purified by column chromatography (petroleum ether/ethyl acetate = 5/1, v/v) to give Compound (**344d**) (1 g, 65%) as a white solid. LC-MS: m/z: 407 (M+1). ¹H NMR (CDCl₃, 400 MHz) peaks: *δ* (ppm) 0.924 (s, 3H), 1.148 (s, 3H), 3.748 (m, 4H), 7.138 (m, 2H), 7.196 (m, 1H), 7.294 (m, 2H).

### Example 248E

### Preparation of Compound (344e)

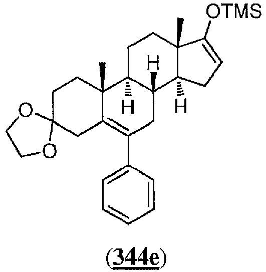

To a solution of (i-Pr)₂NH (1.1 mL, 7.8 mmol) in THF (20 mL) cooled to -78 °C was added a solution of n-BuLi (2.5 M) in hexane (3.1 mL, 7.8 mmol). The mixture was stirred at -78 °C for 15 min under N₂. A solution of Compound (**344d**) (1 g, 2.6 mmol) in THF (20 mL) was added dropwise over 20 min. The mixture was stirred at -78 °C for 15 min under N₂ before NEt₃ (1.5 mL) and TMSCl (1 mL) were added. The mixture was warmed to r.t. and stirred for 30 min under N₂. The mixture was added saturated aqueous NaHCO₃ solution (30 mL), diluted with DCM (200 mL), washed with water (150 mL x 3), brine (150 mL), dried over anhydrous Na₂SO₄, concentrated to give Compound (**344e**) (1.2 g, 100%) as a white solid.

### Example 248F

### Preparation of Compound (344f)

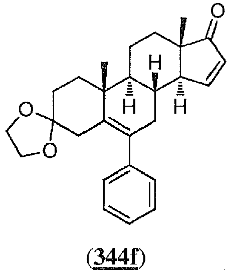

To a solution of Compound (**344e**) (1.2 g, 2.6 mmol) in DCM (30 mL) and MeCN (10 mL) was added Pd(OAc)₂ (0.5 g). The mixture was stirred at 35 °C for 1 hr. The mixture was filtered to remove the brown solid. The filtrate was concentrated, and purified by column chromatography (petroleum ether/ethyl acetate = 4/1, v/v) to give Compound (**344f**) (685 mg, 65%) as a white solid. LC-MS: (m/z) 405 (M+1). ¹H NMR (CDC13, 400 MHz) peaks: *δ* (ppm) 1.126 (s, 3H), 1.194 (s, 3H), 3.738 (m, 4H), 6.020 (m, 1H), 7.155 (m, 2H), 7.215 (m, 1H), 7.308 (m, 2H), 7.444 (m, 1H).

### Example 248G

### Preparation of Compound (344g)

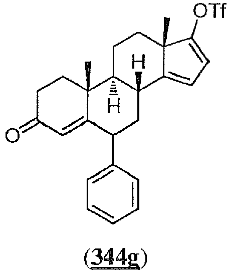

To a solution of Compound (**344f**) (100 mg, 0.25 mmol), imidazole (50 mg, 0.74 mmol) in DCM (5 mL) was added dropwise Tf₂O (105 mg, 0.37 mmol) at 0 °C. The mixture was stirred at room temperature for 2 h. The mixture was added saturated aqueous NaHCO₃ solution to adjust pH > 7, diluted with DCM (50 mL), washed with water (50 mL x 3), brine (50 mL), dried over anhydrous Na₂SO₄, and purified by column chromatography (petroleum ether/ethyl acetate = 5/1, v/v) to give Compound (**344g**) (40 mg, 33%) as a white solid. LC-MS: (m/z) 493 (M+1). ¹H NMR (CDCl3, 400 MHz) peaks: *δ* (ppm) 1.214 (s, 3H), 1.426 (s, 3H), 3.674 (m, 1H), 5.214 (m, 1H), 5.823 (m, 1H), 6.099 (m, 1H), 7.141 (m, 2H), 7.273 (m, 1H), 7.332 (m, 2H).

### Example 248H

### Preparation of Compound (344)

To a solution of Compound (**344**) (165 mg, 0.34 mmol) in 1, 4-dioxane (25 mL) was added K₂CO₃ (231 mg, 1.68 mmol), pyridin-3-ylboronic acid (63 mg, 0.51 mmol), water (3 mL), Pd(PPh₃)₂Cl₂ (24 mg, 0.034 mmol). The mixture was stirred at 80 °C for 2 hrs under N₂. The mixture was filtered to remove the brown solid, diluted with DCM (50 mL), washed with water (30 mL x 3), brine (30 mL), dried over anhydrous Na₂SO₄, and purified by column chromatography on silica (petroleum ether/ethyl acetate = 2/1, v/v) to give (**344**) (35 mg, 24%) as a white solid. LC-MS: (m/z) 422 (M+1). ¹H NMR (CDCl3, 400 MHz) peaks: *δ* (ppm) 1.255 (s, 3H), 1.469 (s, 3H), 2.739 (m, 1H), 3.731 (m, 1H), 5.229 (m, 1H), 6.055 (m, 1H), 6.809 (m, 1H), 7.173 (m, 2H), 7.281 (m, 2H), 7.344 (m, 2H), 7.792 (m, 1H), 8.436 (m, 1H), 8.770 (m, 1H).

### Example 249

### Preparation of Compound (345)

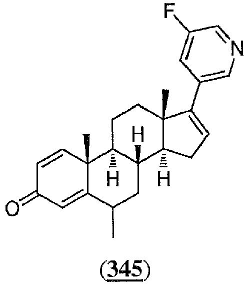

To a solution of Compound (**342**) (160 mg, 0.42 mmol) in dry CH₂Cl₂ (10 mL) was added Et₃N (0.18 mL, 1.27 mmol), followed by TMSOTf (0.1 mL, 0.51 mmol) and the mixture was stirred at ambient temperature for 3 h. Then Pd(OAc)₂ (105.3 mg, 0.51 mmol) and CH₃CN (3 mL) were added. The mixture was stirred at 40 °C for 4 hrs. After being filtered through a pad of Celite, the crude product was purified by Prep-HPLC to give Compound (**345**) (60 mg, 25%) as a white solid. LC-MS: (m/z) 378(M+1). ¹H NMR (CDCl₃, 400 MHz) peaks: *δ* (ppm) 1.10 (s, 3H), 1.17 (d, *J* = 9.0 Hz, 3H), 1.29 (s, 3H), 6.06 (m, 1H), 6.13 (s, 1H), 6.26 (d, *J* = 10 Hz, 1H), 7.06 (d, *J* = 10 Hz, 1H),7.39 (m, 1H), 7.35 (m, 1H), 8.34 (s, 1H), 8.43 (s, 1H).

### Example 250

### Preparation of Compound (346)

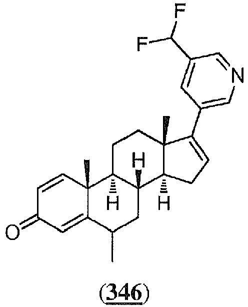

To a solution of (**341**) (102 mg, 0.25 mmol) in dry CH₂Cl₂ (2 mL) was added Et₃N (0.11 mL, 0.75 mmol) followed by trimethylsilyl trifluoromethanesulfonate (0.06 mL, 0.275 mmol) at 0 °C. The resulting mixture was stirred at 0 °C for 1 hr. Then Pd(OAc)₂ (100 mg, 0.50 mmol) and CH₃CN (6 mL) were added. The mixture was stirred at 45 °C for 4 hrs. After being filtered through a pad of Celite, the crude product was purified by prep-HPLC to afford Compound (**346**) (19.3 mg, 19%) as a white solid. LC-MS: (m/z) 410 [M+1]⁺; ¹H NMR (CDCl₃, 400 MHz) major characteristic peaks: *δ* (ppm) 1.11 (s, 3H), 1.16-1.18 (d, *J* = 6.4 Hz, 3H), 1.30 (s, 3H), 6.07-6.08 (t, *J* = 1.2 Hz, 1H), 6.13 (s, 1H), 6.25-6.28 (dd, *J* = 10, 1.2 Hz, 1H), 6.57-6.85 (t, *J*= 55.8 Hz, 1H), 7.05-7.08 (d, *J*= 10.4 Hz, 1H), 7.77 (s, 1H), 8.61 (s, 1H), 8.72 (s, 1H).

### Example 251

### Preparation of Compound (347)

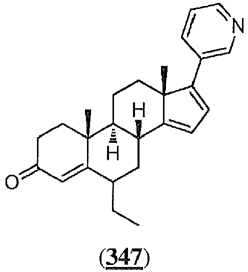

### Example 251A

### Preparation of Compound (347a)

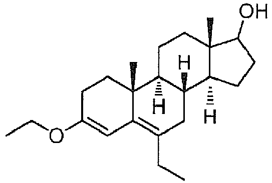

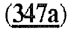

To a solution of Compound (**332d**) (4.50 g, 13.16 mmol) in THF (20 mL) and MeOH (20 mL) was added NaBH₄ (550 mg, 14.47 mmol) at 0 °C. The mixture was stirred at room temperature for 20 minutes and diluted with EtOAc (30 mL), washed with water (20 mL x 3), brine (20 mL), dried over anhydrous Na₂SO₄, and concentrated to give Compound (**347a**) (4.50 g, 100%) as a white solid. LC-MS: (m/z) 345 (M+1).

### Example 251B

### Preparation of Compound (347b)

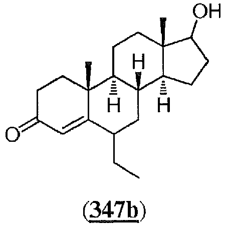

To a solution of Compound (**347a**) (4.50 g, 13.08 mmol) in THF/H2O (10/1 mL) was added 2M HCl (2 mL) at room temperature. The mixture was stirred at room temperature for 21 hrs. The mixture was added saturated aqueous NaHCO₃ solution to adjust pH > 7, diluted with EtOAc (30 mL), washed with water (20 mL x 3), brine (20 mL), and dried over anhydrous Na₂SO₄. After filtration, the filtrate was concentrated to give Compound (**347b**) (4.20 g, 100%) as a white solid.
LCMS: (m/z) 317 (M+1).

### Example 251C

### Preparation of Compound (347c)

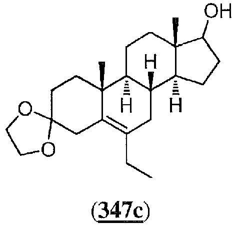

To a solution of Compound (**347b**) (4.20 g, 13.29 mmol) in THF (6 mL) was added CH(OEt)₃ (4.11 g, 27.80 mmol), p-TsOH (252 mg, 1.33 mmol), ethane-1,2-diol (6 mL). The mixture was stirred at room temperature for 3 hrs. The mixture was added saturated aqueous NaHCO₃ solution to adjust pH > 7, and extracted with ethyl acetate (100 mL) three times. The combined organic phase was washed with water, brine, dried over Na₂SO₄. After filtration, the filtrate was evaporated to dryness to give a crude product, which was purified by silica gel column chromatography, eluted with petroleum ether: ethyl acetate = 3:1 to give Compound (**347c**) (4.00 g, 84%) as a white solid. LCMS: (m/z) 360 (M+1). ¹H NMR (DMSO-*d*₆, 400 MHz) major characteristic peaks: *δ* (ppm) 0.64 (s, 3H), 0.85 (t, *J*= 7.6 Hz, 3H), 0.95 (s, 3H), 3.58-3.61 (m, 4H).

### Example 251D

### Preparation of Compound (347d)

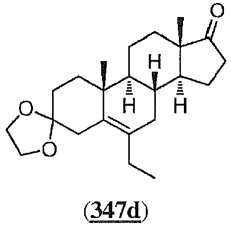

To a solution of Compound (**347c**) (4.00 g, 11.1 mmol) in DCM (30 mL) was added DMP (5.18 g, 12.2 mmol) at 0 °C. The mixture was stirred at room temperature for 1 hr. The mixture was added EtOAc (100 mL), filtered to remove the white solid. The filtrate was concentrated and purified by silica gel column chromatography, eluted with petroleum ether:ethyl acetate = 5:1 to give Compound (**347d**) (1.62 g, 41%) as a white solid. LCMS: (m/z) 359 (M+1). ¹H NMR (DMSO-*d*₆, 400 MHz) major characteristic peaks: *δ* (ppm) 0.82 (s, 3H), 0.89 (t, J= 7.6 Hz, 3H), 0.97 (s, 3H), 3.81-3.91 (m, 4H).

### Example 251E

### Preparation of Compound (347e)

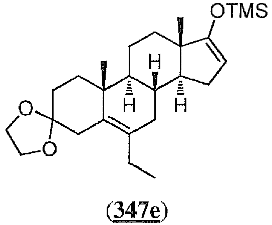

To a solution of (i-Pr)₂NH (1.92 mL, 13.56 mmol) in THF (10 mL) cooled to -78 °C was added a solution of n-BuLi (2.5 M) in hexane (5.4 mL, 13.56 mmol). The mixture was stirred at -78 °C for 15 min under N₂. A solution of Compound (**347d**) (1.62 g, 4.52 mmol) in THF (10 mL) was added dropwise over 20 minutes, and the mixture was stirred at -78 °C for 15 min under N₂ before Et₃N (2.43 mL), TMSCl (1.6 mL) were added. The mixture was warmed to r.t. and stirred for 30 min under N₂. The mixture was added saturated aqueous NaHCO₃ solution (30 mL), diluted with DCM (200 mL), washed with water (150 mL x 3), brine (150 mL), dried over anhydrous Na₂SO₄, and concentrated to give Compound (**347e**) (1.80 g, 93%) as a white solid.

### Example 251F

### Preparation of Compound (347f)

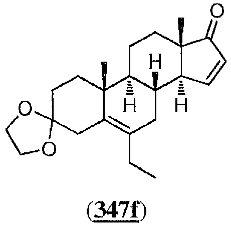

To a solution of Compound (**347e**) (1.70 g, 3.95 mmol) in THF (10 mL) and MeCN (10 mL) was added Pd(OAc)₂ (885 mg, 3.95 mmol). The mixture was stirred at 35 °C for 2 hrs. The mixture was filtered to remove the brown solid. The filtrate was concentrated, purified by silica gel column chromatography, eluted with petroleum ether:ethyl acetate = 5:1 to give Compound (**347f**) (1.30 g, 92%) as a white solid. LC-MS: (m/z) 357 (M+1). ¹H NMR (CDCl₃, 400 MHz) major characteristic peaks: *δ* (ppm) 0.96 (t, *J* = 7.2 Hz, 3H), 1.07 (s, 3H), 1.08 (s, 3H), 3.93-4.00 (m, 4H), 6.037-6.05 (m, 1H), 7.53 (d, *J* = 7.2 Hz, H).

### Example 251G

### Preparation of Compound (347g)

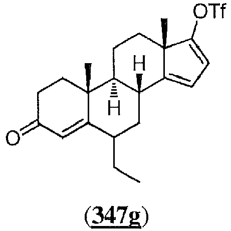

To a solution of Compound (**347f**) (1.30 g, 3.65 mmol), imidazole (745 mg, 10.96 mmol) in DCM (15 mL) was added dropwise Tf₂O (1.03 g, 3.65 mmol) at room temperature. The mixture was stirred at room temperature for 30 minutes. The mixture was added saturated aqueous NaHCO₃ solution to adjust pH > 7, diluted with DCM (50 mL), washed with water (50 mL x 3), brine (50 mL), dried over anhydrous Na₂SO₄, and purified by silica gel column chromatography, eluted with petroleum ether:ethyl acetate = 5:1 to give Compound (**347g**) (520 mg, 32%) as a colorless oil. LC-MS: (m/z) 445 (M+1).

### Example 251H

### Preparation of Compound (347)

To a solution of Compound (**347g**) (270 mg, 0.608 mmol) in 1, 4-dioxane (20 mL) was added K₂CO₃ (252 mg, 1.824 mmol), pyridin-3-ylboronic acid (90 mg, 0.730 mmol), water (1 mL), Pd(PPh₃)₂Cl₂ (42 mg, 0.061 mmol). The mixture was stirred at 80 °C for 2 hrs under N₂. The mixture was filtered to remove the brown solid, diluted with DCM (50 mL), washed with water (30 mL x 3), brine (30 mL), dried over anhydrous Na₂SO₄, and purified by silica gel column chromatography eluted with petroleum ether:ethyl acetate = 2:1 to give (**347**) (50 mg, 22%) as a white solid. LCMS: m/z: 374 (M+1). ¹H NMR (CDCl3, 400 MHz) peaks: *δ* 0.95 (t, *J* = 7.6 Hz, 3H), 1.16 (s, 3H), 1.24 (s, 3H), 5.77 (s, 1H), 5.95 (s, 1H), 6.80 (d, *J* = 2.4 Hz, 1H), 7.20-7.24 (m, 1H), 7.72 (d, *J*= 8.4 Hz, 1H), 8.36 (s, 1H), 8.69 (s, 1H).

### Example 252

### Preparation of Compound (348)

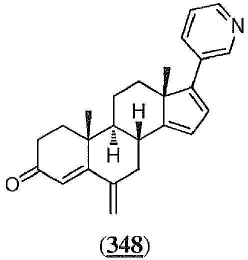

### Example 252A

### Preparation of Compound (348a)

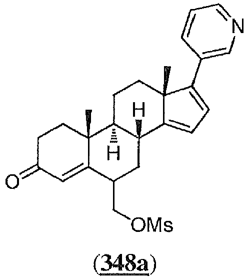

To a solution of compound (**82**) (92 mg, 0.24 mmol) in CH₂Cl₂ (10 mL) was added MsCl (54 mg, 0.48 mmol) and Et₃N (49 mg, 0.48 mmol) at 0 °C. The reaction mixture was stirred for 3 hrs at 0 °C. Then it was allowed to warm up to room temperature, washed with brine, dried over Na₂SO₄, filtered, and evaporated to give Compound (**348a**) (102 mg, 93%). It was used in the next step without further purification.

### Example 252B

### Preparation of Compound (348)

To a solution of compound (**348a**) (102 mg, 0.22 mmol) in MeOH (5 mL) was added KOH (24 mg, 0.44 mmol) and H₂O (1 mL). The reaction mixture was refluxed overnight and then evaporated to dryness. The residue was dissolved in CH₂Cl₂ (10 mL), washed with brine, dried over Na₂SO₄, filtered, and evaporated to yield a crude product, which was purified by prep-TLC (petroleum ether/ ethyl acetate = 1/1 v/v) to give Compound (**348**) (50 mg, 63%) as a yellow solid. LC-MS (ESI): (m/z) 358 (M+H)⁺; ¹HNMR (CDCl₃, 400 MHz) major characteristic peaks: *δ* (ppm) 1.19 (s, 3H), 1.33 (s, 3H), 5.06∼5.07 (m, 1H), 5.16∼5.16 (m, 1H), 5.96∼5.97 (m, 1H), 6.04∼6.05 (m, 1H), 6.80-6.81 (m, 1H), 7.27 (m, 1H), 7.77 (m, 1H), 8.44 (s, 1H), 8.77 (s, 1H).

### Example 253

### Preparation of Compound (349)

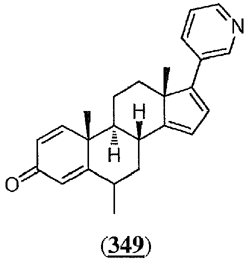

### Example 253A

### Preparation of Compound (349a)

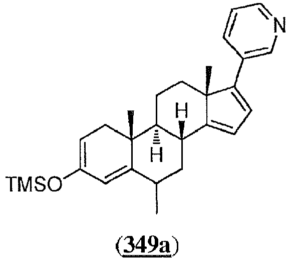

A solution of Compound (**315**) (72 mg, 0.2 mmol) in dry THF (2 mL) was added dropwise into a solution of KHMDS (0.9 M, 0.55 mL, 0.5 mmol) in dry THF (5 mL) at -78 °C under N₂. The mixture was stirred at this condition for 30 min. Then TEA (0.12 mL) and TMSCl (0.09 mL) were added. Then it was warmed up to room temperature over 30 min. the mixture was quenched with a saturated sodium bicarbonate aqueous solution and extracted with ethyl acetate (50 mL). The organic layer was washed with brine, dried over Na₂SO₄, and evaporated to give Compound (**349a**) (100 mg, 99%) as a yellow solid. The crude product was used in next step without further purification.

### Example 253B

### Preparation of Compound (349)

To a solution of Compound (**349a**) (100 mg) in dry DCM (6 mL) and CH₃CN (2 mL) was added Pd(OAc)₂ (60 mg, 0.23 mmol) under N2. The mixture was stirred at 40 °C for 3.0 hrs. After being filtered through a pad of Celite, the crude product was purified by prep-HPLC to give (**349**) (20 mg, 20%) as a white solid. LC-MS: (m/z) 358 (M+1). ¹H NMR (CDCl₃, 400 MHz) peaks: *δ* (ppm) 1.24 (m, 6H), 1.37 (s, 3H), 6.00 (s, 1H), 6.16 (s, 1H), 6.26 (m, 1H), 6.77 (m, 1H), 7.08 (m, 1H), 7.25 (m, 1H), 7.76 (m, 1H), 8.44 (m, 1H), 8.75 (s, 1H).

### Example 254

### Preparation of Compound (350)

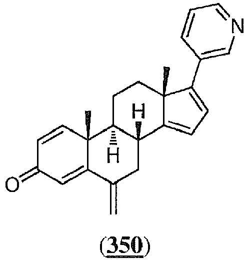

### Example 254A

### Preparation of Compound (350a)

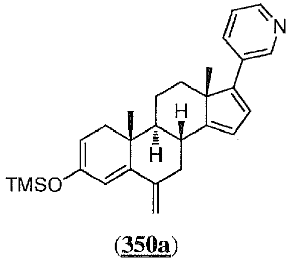

To a solution of compound (**348**) (40 mg, 0.11 mmol) in dry THF (5 mL) was added dropwise LDA (2 M, 0.1 mL) at -70 °C under nitrogen atmosphere. The reaction mixture was stirred for 1 hour at -70 °C before TEA (33 mg, 0.33 mmol) and TMSCl (35 mg, 0.32 mmol) was added. It was allowed to warm to room temperature over 30 min. the mixture was quenched with sat. aq. NaHCO₃ solution and extracted with ethyl acetate. The combined organic phases were washed with brine, dried over Na₂SO₄, filtered, and evaporated to give Compound (**350a**) (47 mg, crude), which was used in the next step without further purification.

### Example 254B

### Preparation of Compound (350)

To a solution of compound (**350a**) (47 mg, 0.11 mmol) in CH₂Cl₂ (6 mL) and MeCN (3 mL) was added Pd(OAc)₂ (28 mg, 0.12 mmol) under nitrogen atmosphere. The reaction mixture was stirred at 40 °C for 3 hrs. The solvent was removed under reduced pressure and the crude product was purified by prep-TLC (petroleum ether/ethyl acetate = 1/1, v/v) to afford (**350**) (4.0 mg, 10%) as a white solid. LC-MS (ESI): (m/z) 356 (M+H)⁺; ¹HNMR (CDCl₃, 400 MHz) major characteristic peaks: *δ* (ppm) 1.27 (s, 3H), 1.24 (s, 3H), 5.07∼5.12 (m, 2H), 6.05∼6.06 (m, 1H), 6.20∼6.21 (m, 1H), 6.25∼6.26 (m, 1H), 6.28∼6.29 (m. 1H).

### Example 255

### Preparation of Compound (351)

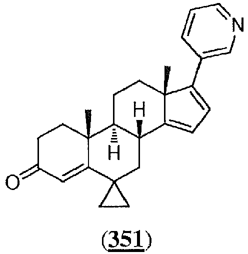

### Example 255A

### Preparation of Compound (351a)

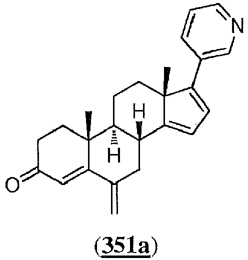

To a suspension of anhydrous sodium acetate (0.4 g) in chloroform (12 mL), formaldehyde dimethylacetal (12 mL) and phosphorus oxychloride (1.5 mL) was added Compound (**82**) (0.35 g). it was allowed to reflux for 12 hrs with stirring. After cooled to room temperature, the mixture was diluted with methylene chloride and water. A saturated NaHCO₃ solution was added dropwise to make the aqueous phase of the reaction mixture basic. The organic phase was separated, washed with water, dried over sodium sulfate, and evaporated under vacuum. The residue was purified by column chromatography (ethyl acetate/ petroleum ether = 1/1, v/v) to give Compound (**351a**) (100 mg, 30%) as a yellow solid. LC-MS (ESI): (m/z) 358(M+H)⁺.

### Example 255B

### Preparation of Compound (351)

At room temperature, trimethylsulfoxonium iodide (220 mg, 1.0 mmol) was agitated for 1.5 hrs with sodium hydride (40 mg, 1.0 mmol, 60% oil suspension) in DMSO (10 mL). Compound (**351a**) (100 mg, 0.28 mmol) was added to the solution and was stirred for 12 hrs under nitrogen at room temperature. The reaction mixture was then poured into ice water. The thus-formed precipitate was filtered off and taken up in methylene chloride. After drying and evaporation, the residue was purified by prep -HPLC to give (**351**) (52 mg, 50%) as a yellow solid. LC-MS (ESI): (m/z) 372 (M+H)⁺; ¹HNMR (CDCl₃, 400 MHz) major characteristic peaks: *δ* (ppm) 0.53 (m, 2H), 1.25 (s, 3H), 1.39 (s, 3H), 5.60 (s, 1H), 5.95 (m, 1H), 6.79 (m, 1H), 7.25 (m, 1H), 7.77 (m, 1H), 8.43 (m, 1H), 8.76 (s, 1H).

### Example 256

### Preparation of Compound (352)

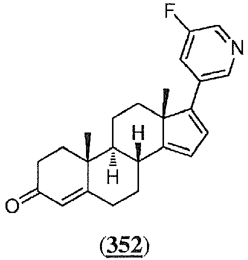

A mixture of Compound (**271**) (120 mg, 0.33 mmol) in toluene (2 mL) and butanone (5 mL) was added with 24.7% Al(Oi-Pr)₃ (400 mg, 0.5 mmol) and stirred at reflux for 3 hrs. The mixture was cooled to room temperature, poured in to ice-water, filtered through celite, washed with water (5 mL) and ethyl acetate (20 mL x 3). The filtrate was separated and the organic layer was washed with aq. NaHCO₃ solution (10 mL), brine (10 mL), concentrated, and purified by prep-HPLC to give Compound (**352**) (32 mg, 27%) as a white solid. LC-MS (ESI) m/z: 364 (M+1)⁺. ¹H-NMR (400 MHz, CDCl₃) *δ* (ppm): 1.230 (s, 3H), 1.325 (s, 3H), 5.792 (s, 1H), 6.025-6.035 (t, *J* = 2.0 Hz, 1H), 6.852-6.857 (m, 1H), 7.464-7.490 (m, 1H), 8.287 (s, 1H), 8.580 (s, 1H).

### Example 257

### Preparation of Compound (353)

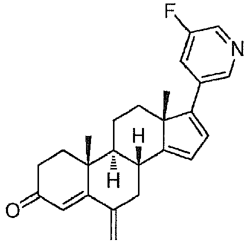

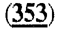

A suspension of anhydrous sodium acetate (0.5 g) in chloroform (15 mL), formaldehyde dimethylacetal (15 mL) and phosphorus oxychloride (1.9 mL) was combined with Compound (**352**) (0.49 g) and refluxed for 12 hrs under agitation. After cooled to room temperature, the mixture was diluted with methylene chloride and water. With stirring, a saturated NaHCO₃ solution was added dropwise to make the aqueous phase of the reaction mixture basic. The organic phase was separated, washed with water, dried over sodium sulfate, and evaporated under vacuum. The residue was purified by prep-HPLC to give (**353**) (150 mg, 30%) as a white solid. LC-MS (ESI): (m/z) 376 (M+H)⁺; ¹HNMR (CDCl₃, 400 MHz) major characteristic peaks: *δ*: (ppm) 1.23 (s, 3H), 1.24 (s, 3H), 5.07 (s, 1H), 5.16 (s, 1H), 5.97 (s, 1H), 6.06 (m, 1H), 6.85 (m, 1H), 7.46 (m, 1H), 8.30 (s, 1H), 8.57 (s, 1H).

### Example 258

### Preparation of Compound (354)

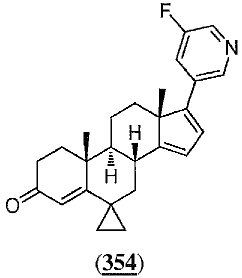

At room temperature, trimethylsulfoxonium iodide (220 mg, 1.0 mmol) was agitated for 1.5 h with sodium hydride (40 mg, 1.0 mmol, 60% oil suspension) in DMSO (10 mL). Under N₂, Compound (**353**) (130 mg, 0.35 mmol) was added to the solution and was stirred for 12 hrs at room temperature. The reaction mixture was then poured into ice water .The thus-formed precipitate was filtered off and taken up in methylene chloride. After drying and evaporation, the residue was purified by prep -HPLC to afford (**354**) (75 mg, 55%) as a yellow solid. LC-MS (ESI): (m/z) 390(M+H)⁺; ¹HNMR (CDCl₃, 400 MHz) major characteristic peaks: *δ* (ppm) 0.53 (m, 2H), 1.25 (s, 3H), 1.39 (s, 3H), 5.70 (s, 1H), 5.96 (m, 1H), 6.84 (m, 1H), 7.47 (m, 1H), 8.29 (m, 1H), 8.58 (s, 1H).

### Example 259

### Preparation of Compounds (355), (356), (357), (358), (359), and (360)

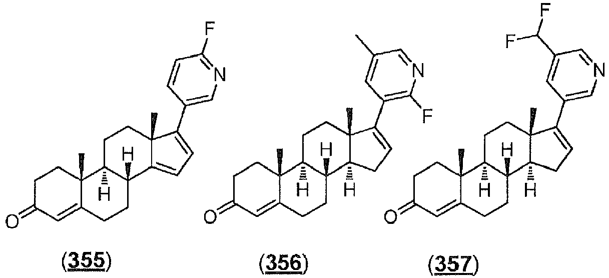

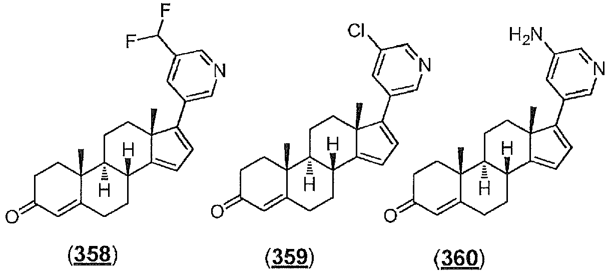

### Example 257A

### Preparation of Compounds (355a) and (360a)

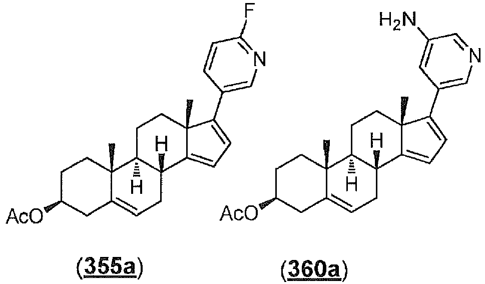

A mixture of Compound (**77**) (920 mg, 2.0 mmol), 6-fluoropyridin-3-ylboronic acid (500 mg, 3.5 mmol), Pd(PPh₃)₂Cl₂ (140 mg, 0.2 mmol), aqueous K₂CO₃ (2N, 4 mL) in dioxane (40 mL) was stirred at 80 °C for 1 hr. Then the mixture was poured into water, and extracted with ethyl acetate (100 mL x 3). The extraction was washed with brine, dried, concentrated to obtain a crude product. The crude product was purified by column chromatography (petroleum ether/ethyl acetate = 10/1, v/v) to give Compound (**355a**) (380 mg, 47%). LC-MS (ESI) m/z: 408 (M+1)⁺.

Using the similar procedure and under the same conditions, replacing 6-fluoropyridin-3-ylboronic acid with 5-aminopyridin-3-ylboronic acid, Compound (**360a**) was prepared. Compound (**360a**): Yield 100%; LC-MS (ESI) m/z: 405 (M+1)⁺.

### Example 259B

### Preparation of Compound (335b) and (360b)

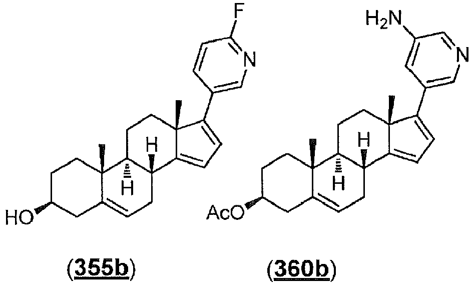

To a solution of Compound (**355a**) (380 mg, 0.93 mmol) in THF (10 mL) and water (1 mL) was added KOH (52.3 mg, 0.93 mmol). The mixture was stirred at room temperature for 1.0 hr (TLC) under N₂. The solvent was removed in vacuum. The residue was added water and extracted with DCM (50 mL x 3). The DCM phase was washed with brine, dried over Na₂SO₄, and concentrated to give Compound (**355b**) (300 mg, 90%). LC-MS (ESI) m/z: 366 (M+1)⁺.

Using the similar procedure and under the same conditions, replacing (**355a**) with (**360a**), Compounds (**360b**) weas prepared. Compound (**360b**): Yield 47%; LC-MS (ESI) m/z: 362 (M+1)⁺.

### Example 259C

### Preparation of Compounds (355), (356), (357), (358), (359), and (360)

To a solution of Compound (**355b**) (300 mg, 0.82 mmol) in toluene (10 mL) and butanone (20 mL) was added Al(O*i*-Pr)₃ (1.15 g, 1.35 mmol). The mixture was refluxed overnight under N₂. After cooled to room temperature, the mixture was poured into ice-water and extracted with ethyl acetate (50 mL x 3). The ethyl acetate phase was washed with brine, dried over Na₂SO₄, and concentrated to give a crude product. The crude product was purified by prep-TLC to afford Compound (**355**) (70 mg, 24%) as a white solid. LC-MS (ESI) m/z: 364 (M+1)⁺; ¹H NMR (CDCl₃, 400 MHz) major characteristic peaks: *δ* (ppm) 1.20 (s, 3H), 1.32 (s, 3H), 5.79 (s, 1H), 6.00 (m, 1H), 6.69 (m, 1H), 6.90 (m, 1H), 7.87 (m, 1H), 8.30 (m, 1H).

Using the similar procedure and under the same conditions, replacing (**355b**) with (**277**), (**279**), **(272),** (**273**), and (**360b**), Compounds (**356**), (**357**), (**358**), (**359**) and (**360**) were prepared.

Compound (**356**) Yield 47% as a white solid: LC-MS (m/z): 380 [M+H]⁺; ¹H NMR (CDCl₃, 400 MHz) major characteristic peaks: *δ* (ppm) 1.02 (s, 3H), 1.23 (s, 3H), 2.30 (s, 3H), 5.75 (s, 1H), 6.02 (m, 1H), 7.44 (m, 1H), 7.87 (s, 1H).

Compound (**357**) Yield 47% as a white solid: LC-MS (m/z): 398 [M+H]⁺; ¹H NMR (CDCl₃, 400 MHz) major characteristic peaks: *δ* (ppm) 1.09 (s, 3H), 1.25 (s,3H), 5.76 (s, 1H), 6.11 (m, 1H), 6.72 (t, *J*= 56 Hz, 1H), 7.81 (s, 1H), 8.61 (s, 1H), 8.73 (s, 1H).

Compound **(358)** Yield 47% as a white solid: LC-MS (m/z): 396 [M+H]⁺; ¹H NMR (CDCl₃, 400 MHz) major characteristic peaks: *δ* (ppm) 1.24 (s, 3H), 1.33 (s, 3H), 5.80 (s, 1H), 6.04 (m, 1H), 6.72 (t, *J*= 56 Hz, 1H), 6.89 (m, 1H), 7.88 (s, 1H), 8.55 (s, 1H), 8.86 (s, 1H).

Compound (**359**) Yield 58% as a white solid: LC-MS (m/z): 380.1 [M+1]⁺; ¹H NMR (CDCl₃, 400 MHz) major characteristic peaks: *δ* (ppm) 1.23 (s, 3H), 1.33 (s, 3H), 5.79 (s, 1H), 6.02-6.03 (m, 1H), 6.85 (d, *J* = 2.4 Hz, 1H), 7.73-7.75 (m, 1H), 8.38 (s, 1H), 8.63 (s, 1H).

Compound (**360**) Yield 22% as a yellow solid: LC-MS (ESI) m/z: 361 (M+1)⁺; ¹H-NMR (400 MHz, DMSO *d*₆) *δ* (ppm): 1.149 (s, 3H), 1.288 (s, 3H), 5.247 (s, 2H), 5.692 (s, 1H), 5.991 (s, 1H), 6.789-6.794 (d, *J* = 2.0 Hz, 1H). 7.063-7.068 (d, *J* = 2.0 Hz, 1H), 7.750-7.756 (d, *J* = 2.4 Hz, 1H), 7.969 (s, 1H).

### Example 260

### Preparation of Compounds (361), (362), (363), (364), (365), (366), and (367)

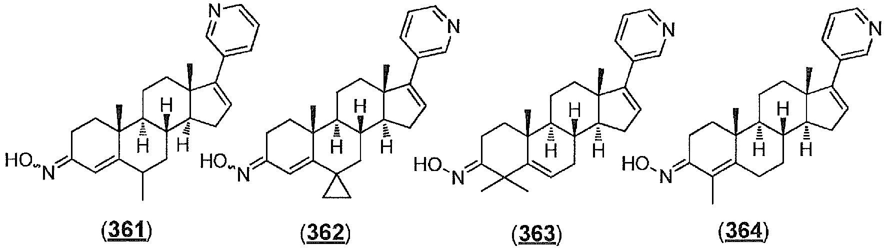

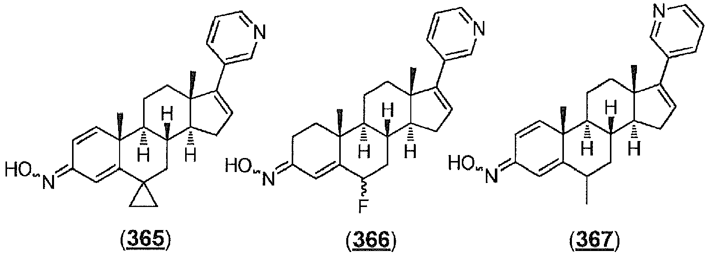

A solution of Compound (**309**) (430 mg, 1.19 mmol), NH₂OH.HCl (100 mg, 1.43 mmol) and pyridine (141 mg, 1.78 mmol) in EtOH (15 mL) was stirred at 30 °C 2 hrs. Then it was diluted with ethyl acetate (100 mL), washed with water and brine, dried with anhydrous Na₂SO₄, concentrated, and purified by prep-HPLC to obtain Compound (**361**) (300 mg, 67%) as a white solid. LC-MS: (m/z) 377 [M+1]⁺; ¹H NMR (CDCl₃, 400 MHz) major characteristic peaks: *δ* (ppm) 1.06 (s, 3H), 1.11-1.12 (m, 4H), 1.14-1.16 (m, 2H), 5.84 (s, 0.6H), 6.00 (s, 1H), 6.54 (s, 0.4H), 7.2-7.24 (m, 1H), 7.64-7.66 (m, 1H), 8.47 (s, 1H), 8.62 (s, 1H).

Using the similar procedure and under the same conditions for Compound (**361**), replacing Compound (**309**) with Compounds (**313**), (**293**), (**300**), (**316**), (**310**), and **(318),** Compounds (**362** - 367) were prepared, respectively.

Compound (**362**) (Yield, 88%) as a yellow solid: LC-MS (m/z) 389[M+H]⁺; ¹H-NMR *δ* (400 MHz, CHCl₃-*d*) major characteristic peaks: *δ* (ppm) 0.35 (m, 2H), 1.08 (s, 3H), 1.18 (s, 3H), 3.07 (m, 1H), 5.73 (s, 0.72 H), 5.98 (m, 1H), 6.48 (s, 0.28 H), 7.23 (m, 1H), 7.65 (d, *J* = 8.0 Hz, 1H), 8.46 (d, *J* = 4.0 Hz, 1H), 8.62 (s, 1H).

Compound (**363**) (Yield, 62%) as a white solid: LC-MS (ESI) m/z: 391 (M+1)⁺; ¹H-NMR (400 MHz, CDCl₃) *δ* (ppm): 0.933 (s, 3H), 1.059 (s, 3H), 1.286 (s, 3H), 1.369 (s, 3H), 5.629-5.634 (m, 1H), 6.021-6.025 (m, 1H), 7.222-7.254 (m, 1H), 7.657-7.677 (m, 1H), 8.465-8.481 (dd, *J* = 4.8, 2.0 Hz,1H), 8.636-8.640 (d, *J* = 1.6 Hz, 1H).

Compound (**364**) (Yield, 99%) as a pale white solid: LC-MS (ESI) m/z: 377 (M+H)⁺; ¹H-NMR (400 MHz, DMSO-*d*₆) *δ* (ppm) 1.03 (s, 3H), 1.05 (s, 3H), 1.78 (s, 3H), 6.11 (s, 1H), 7.32-7.36 (m, 1H), 7.75-7.77 (d, *J* = 8 Hz, 1H), 8.43-8.44 (d, *J* = 3.6 Hz, 1H), 8.58-8.59 (d, *J* = 1.6 Hz, 1H), 10.60 (s, 1H).

Compound (**365**) (Yield, 98%) as a yellow solid: LC-MS (m/z) 387 [M+H]⁺; ¹H-NMR *δ* (400 MHz, CHCl₃-*d*) major characteristic peaks: *δ* (ppm) 0.36 (m, 1H), 0.51 (m, 1H), 0.75 (m, 1H), 0.85 (m, 1H), 1.09 (s, 3H), 1.30 (s, 3H), 5.95 (s, 0.50 H), 5.97 (m, 1 H), 6.24 (dd, J= 2.0 Hz, 10.0 Hz, 0.5H), 6.24 (d, *J*= 10.0 Hz, 0.5H), 6.41 (d, *J* = 10.0 Hz, 0.5H), 6.70 (s, 0.5H),6.89 (dd, *J* = 2.0 Hz, 10.0 Hz, 0.5H), 7.23(m, 1H), 7.64 (m, 1H), 8.47 (dd, *J* = 1.6 Hz, 4.8 Hz, 1H), 8.61 (d, *J* = 1.6 Hz, 1H).

Compound (**366**) (Yield, 86%) as a white solid: LC-MS (m/z): 381 (M+1) ; ¹H NMR (CDCl₃, 400 MHz) major characteristic peaks: *δ* (ppm) 1.08 (s, 3H), 1.26 (s, 3H), 5.00 (m, 0.5H), 5.13 (m, 0.5H), 5.94 (m, 1H), 6.00 (m, 1H), 6.80 (d, 1H), 7.23 (m, 1H), 7.65 (d, *J*=8.0, 1H), 8.47 (s, 1H), 8.62 (s, 1H).

Compound (**367**) (Yield, 87%) as a white solid: LC-MS: 375 [M+1]⁺; ¹H NMR (CDCl₃, 400 MHz) major characteristic peaks: *δ* (ppm) 1.01 (s, 2.55H), 1.13 (s, 0.35), 1.14 (d, *J* = 6.8 Hz, 1.71H), 1.19 (d, *J*= 6.8 Hz, 1.61H), 1.22 (s, 2.56H), 5.96 (s, 0.4H), 5.99 (s, 0.89H), 6.16 (d, *J* = 8.4 Hz, 0.54H), 6.25 (s, *J* = 10.0 Hz, 0.50H), 6.41 (d, *J* = 10.4 Hz, 0.39H), 6.71 (s, 0.48H), 6.89 (d, *J*= 10.0 Hz, 0.42H), 7.23-7.25 (m, 1H), 7.63-7.67 (s, 1H), 8.45 (s, 1H), 8.61 (s, 1H).

### Example 261

### Preparation of Compounds (368), (369), and (370)

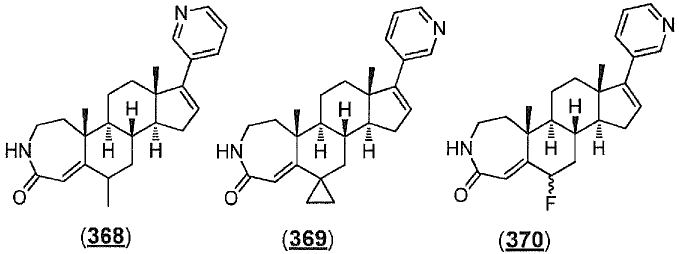

To a solution of Compound (**361**) (200 mg, 0.53 mmol) in dry THF (8 mL) was added dropwise SOCl₂ (0.5 mL) and the mixture was stirred at 30 °C for 2 hrs. Then it was adjusted to pH = 9 with saturated aq. NaHCO₃ solution and extracted with ethyl acetate (100 mL). The organic layer was washed with water and brine, dried with anhydrous Na₂SO₄, and purified by prep-HPLC to obtain Compound (**368**) (100 mg, 50%) as a white solid. LC-MS: 377 [M+1]⁺; ¹H NMR (CDCl₃, 400 MHz) major characteristic peaks: *δ* (ppm) 1.04 (s, 3H), 1.10 (d, *J* = 6.0 Hz, 3H), 1.20 (s, 3H), 3.12-3.24 (m, 2H), 5.68 (s, 1H), 5.99 (s, 1H), 6.13 (t, *J* = 4.4 Hz, 1H), 7.21-7.25 (m, 1H), 7.63-7.66 (m, 1H), 8.46 (s, 1H), 8.61 (s, 1H).

Using the similar procedure and under the same conditions for Compound (**361**), replacing Compound (**361**) with Compounds (**362**) and (**366**), Compounds (**369**) and (**367**) were prepared, respectively.

Compound (**369**) (Yield, 23%) as a white solid: LC-MS (m/z) 389 [M+H]⁺ ; ¹H-NMR *δ* (400 MHz, CHCl₃-*d*) major characteristic peaks: *δ* (ppm) 0.36 (m, 1H), 0.61 (m, 1H), 0.76 (m, 1H), 0.96 (m, 1H), 1.06 (s, 3H), 1.29 (s, 3H), 3.19 (m, 2H), 5.83 (s, 1H), 5.97 (m, 1H), 6.45 (brs, 1H), 7.23 (m, 1H), 7.63 (d, *J* = 9.6 Hz, 1H), 8.46 (d, *J* = 4.4 Hz, 1H), 8.61 (s, 1H).

Compound **(370)** (Yield, 15%) as a white solid: LC-MS(m/z): 381 (M+1); ¹H NMR (CDCl₃, 400 MHz) major characteristic peaks: *δ* (ppm) 1.012 (s, 3H), 1.31 (s, 3H), 3.34 (m, 2H), 4.97 (s, 0.5H), 5.09 (s, 0.5H), 5.97 (s, 1H), 6.30 (s, 1H), 7.59 (s, 1H), 7.76 (s, 1H), 8.22 (d, *J* = 7.6, 1H), 8.68 (s, 1H), 8.88 (s, 1H).

### Example 262

### Preparation of Compound (371)

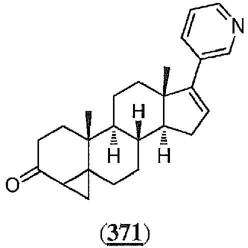

To a solution of (**314**) (135 mg, 0.37 mmol) in DCM (13 mL) was added DMP (237 mg, 0.56 mmol). The mixture was stirred at 28 °C for 3 hrs. Then it was quenched with ethanol and filtered to remove the solid. The filtrate was diluted with ethyl acetate (50 mL), washed with water and brine, dried with anhydrous Na₂SO₄, and purified by column chromatography (petroleum ether/ethyl acetate = 8/1, v/v) to obtain Compound (**371**) (60 mg, 44%) as a white solid. LC-MS (ESI) m/z: 362 (M+H)⁺.

### Preparation of Compounds (372), (373), (374), (375), (376), (377), (378), (379), (380), and (381)

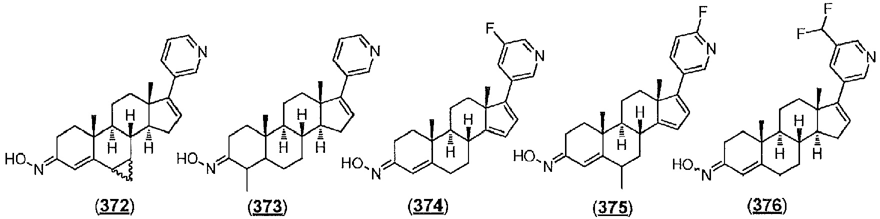

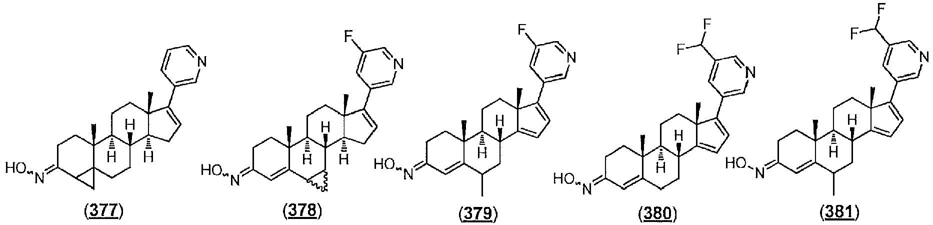

Using the similar procedure and under the same conditions described in Example 260 for the preparation of Compound (**361**), replacing Compound (**309**) with Compounds (**319**), (**303**), (**352**), (**326**), (**357**), (**371**), (**330**), (**327**), (**358**), and (**328**), Compounds (**372**) ∼ (**381**) were prepared, respectively.

Compound **(372)** (Yield 71%) as a yellow solid: LCMS (m/z): 374.9 [M+H]⁺.

Compound **(373)** (Yield 15%) as a yellow solid: LC-MS (m/z) 379 [M+H]⁺.

Compound (**374**) (Yield 37%) as a yellow solid: LC-MS (ESI) m/z: 379 (M+1)⁺; ¹H-NMR (400 MHz, CDCl₃) *δ* (ppm): 5.792 (s, 1H), 5.930 (s, 1H), 6.773-6.777 (m, 1H), 7.385-7.405 (m, 1H), 8.194-8.198 (m, 1H), 8.503 (s, 1H).

Compound **(375)** (Yield 81%) as a yellow solid: LCMS: m/z: 393 (M+1).

Compound (**376**) (Yield 74%) as a white solid: LC-MS (m/z):413 [M+H]⁺.

Compound **(377)** (Yield 99%) as a pale solid: LCMS m/z: 377 (M+H)⁺.

Compound (**378**) (Yield 80%) as a yellow solid: LC-MS (ESI) m/z: 393 (M+1)⁺.

Compound (**379**) (Yield 97%) as a white solid: LC-MS (m/z): 393 [M+H]⁺.

Compound (**380**) (Yield 92%) as a white solid: LC-MS (m/z): 411 [M+H]⁺.

Compound (**381**) (Yield 97%) as a white solid: LC-MS (m/z): 425 [M+H]⁺.

### Example 264

### Preparation of Compounds (382), (383), (384), (385), (386), (387), (388), (389), (390), and (391)

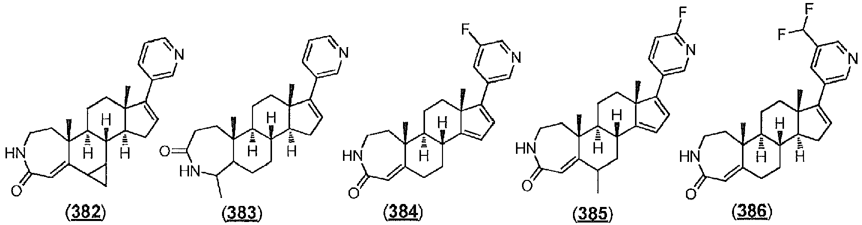

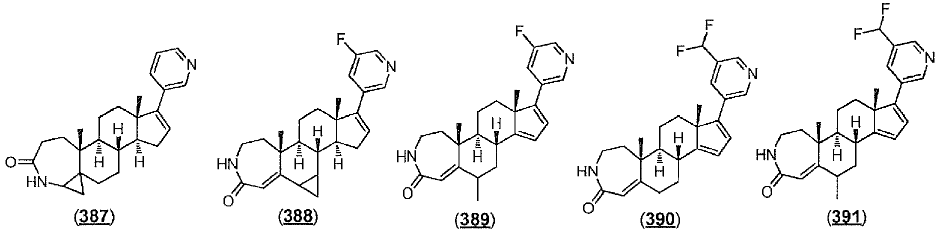

Using the similar procedure and under the same conditions described in Example 261 for the preparation of Compound (**368**), replacing Compound (**361**) with Compounds (**372**), (**373**), (**374**), (**375**), (**376**), (**377**), (**378**), (**379**), (**380**), and (**381**), Compounds (**382**) (**383**), (**384**), (**385**), (**386**), (**387**), (**388**), (**389**), (**390**), and (**391**) were prepared, respectively.

Compound (**382**) (Yield 23%) as a pale yellow solid: LC-MS (m/z): 375.2 [M+H]⁺; ¹H NMR (CDCl₃, 400 MHz) major characteristic peaks: *δ* (ppm) 0.45-0.48 (m, 0.33H), 0.55-0.58 (m, 0.66 H), 1.02 (s, 2H), 1.08 (s, 1H), 1.09 (s, 2H), 1.19 (s, 1H), 5.83-5.88 (m, 1H), 5.91 (s, 0.33H), 6.04-6.07 (m, 1.66H), 7.23-7.25 (m, 1H), 7.65-7.69 (m, 1H), 8.46-8.48 (m, 1H), 8.62-8.63 (m, 1H).

Compound (**383**) (Yield 10%) as a white solid: LC-MS (m/z): 379[M+H]⁺; ¹H NMR (CDCl₃, 400 MHz) major characteristic peaks: *δ* (ppm) 0.91 (s, 3H), 0.93 (s,3H), 1.07 (s, 3H), 2.16 (m, 1H), 2.56 (m, 1H), 3.51 (m, 1H), 5.30 (m, 1H), 5.91 (m, 1H), 7.14 (m, 1H), 7.56 (m, 1H), 8.39 (s, 1H), 8.53 (s, 1H).

Compound (**384**) (Yield 15%) as a grey solid: LC-MS (ESI) m/z: 379 (M+1)⁺; ¹H-NMR (400 MHz, DMSO-*d*₆) *δ* (ppm): 5.572 (s, 1H), 6.405 (s, 1H), 7.138-7.144 (m, 1H), 7.733-7.825 (m, 2H), 8.371-8.378 (m, 1H), 8.646 (s, 1H).

Compound (**385**) (Yield 28 %) as a white solid: LCMS m/z: 393 (M+1). ¹H NMR (CDCl3, 400 MHz) peaks: *δ* (ppm) 1.17 (m, 6H), 3.19 (m, 2H), 5.73 (s, 1H), 5.97 (m, 1H), 6.30 (m, 1H), 6.69 (d, *J* = 2.0 Hz, 1H), 6.89 (m, 1H), 7.87 (m, 1H), 8.27 (d, *J* = 2.8 Hz, 1H).

Compound (**386**) (Yield 38%) as a white solid: LC-MS (m/z): 413 [M+H]⁺; ¹H NMR (CDCl₃, 400 MHz) major characteristic peaks: *δ* (ppm) 1.05 (s, 3H), 1.20 (s,3H), 3.20 (m,2H), 5.76 (s, 1H), 6.07 (s, 1H), 6.50 (m, 1H), 6.72 (t, *J*= 56 Hz, 1H), 7.76 (m, 1H), 8.60 (s, 1H), 8.72 (s, 1H).

Compound (**387**) (Yield 32%) as a white solid: LC-MS (ESI) m/z: 377 (M+H)⁺ ; ¹H-NMR (400 MHz, CDCl₃) δ (ppm) 0.50-0.54 (m, 1H), 0.69-0.71 (m, 1H), 1.03 (s, 3H), 1.11 (s, 3H), 6.00 (s, 1H), 6.06 (s, 1H), 7.24-7.25 (m, 1H), 7.64-7.66 (d, *J*= 8 Hz, 1H), 8.47 (s, 1H), 8.62 (s, 1H).

Compound (**388**) (Yield 12%) as a yellow solid: LC-MS (ESI) m/z: 393 (M+1)⁺; ¹H-NMR (400 MHz, CDCL3): δ (ppm)1.02 (s, 2H), 1.06 (s, 1H), 1.09 (s, 2H), 1.18 (s, 1H), 3.22-3.32 (m, 2H), 5.96 (s, 0.3H), 6.03 (s, 0.7H), 6.09-6.12 (m, 1H), 6.60-6.65 (m, 1H), 7.35-7.38 (m, 1H),8.33 (s 1H), 8.45 (s, 1H).

Compound (**389**) (Yield 38%) as a white solid: LC-MS (m/z): 393[M+H]^{+ 1}H NMR (CDCl₃, 400 MHz) major characteristic peaks: *δ* (ppm) 1.17 (d, *J* = 6.0 Hz, 3H), 1.21 (s,3H), 1.29 (s,3H), 3.17 (m, 2H), 5.73 (s, 1H), 5.99 (m, 1H), 6.21 (bs, 1H), 6.85 (m, 1H), 7.46 (m, 1H), 8.28 (s, 1H), 8.57 (s, 1H).

Compound (**390**) (Yield 30%) as a gray solid: LC-MS (m/z): 411 [M+H]^{+ 1}H NMR (CDCl₃, 400 MHz) major characteristic peaks: *δ* (ppm) 1.21 (s, 3H), 1.29 (s,3H), 3.26 (m, 2H), 5.81 (s, 1H), 6.03 (m, 1H), 6.41 (s, 1H), 6.71 (m, 1H), 6.88 (m, 1H), 7.87 (s, 1H), 8.54 (s, 1H), 8.85 (s, 1H).

Compound (**391**) (Yield 29%) as a white solid: LC-MS (m/z): 425 [M+H]⁺; ¹H NMR (CDCl₃, 400 MHz) major characteristic peaks: *δ* (ppm) 1.18 (d, *J*= 6.0 Hz, 3H), 1.21 (s,3H), 1.29 (s,3H), 3.17 (m, 2H), 5.73 (s, 1H), 6.00 (m, 1H), 6.17 (bs, 1H), 6.72 (t, *J* = 56 Hz, 1H), 6.88 (m, 1H), 7.88 (s, 1H), 8.54 (s, 1H), 8.85 (s, 1H).

### Example 265

### Preparation of Compound (392), (393), and (394)

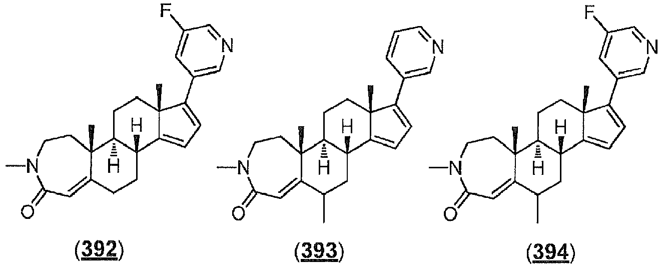

### Example 265A

### Preparation of Compound (392a)

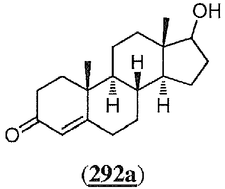

To a mixture of (10R,13S)-10,13-dimethyl-7,8,9,10,11,12,13,14,15,16-decahydro-1H-cyclopenta[a]phenanthrene-3,17(2H,6H)-dione (80 g, 0.28 mol) in methanol (1 L) was added dropwise a solution of NaBH₄ (10.64 g, 0.28 mol) in dichloromethane (400 mL) and methanol (400 mL) at 0 °C. Then the mixture was stirred at room temperature for 2 hrs. Then the resulting mixture was neutralized with acetic acid, evaporated to remove the solvent in vacuum. This gave a crude product (**392a**) (144 g, 100%) as a white solid. The crude product was directly used for next step without further purification. LC-MS (ESI) m/z: 289 (M+1)⁺; ¹H NMR (400 MHz, CDCl₃): *δ* (ppm) 0.795 (s, 3H), 1.197 (s, 3H), 3.654 (t, *J =* 8.4 Hz, 1H), 5.732 (s, 1H).

### Example 265B

### Preparation of Compound (392b) and (393b)

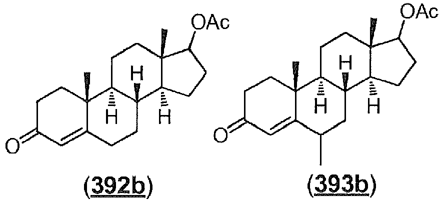

A mixture of compound (**392a**) (144 g, 0.28 mol) in Ac₂O (500 mL) was stirred at 80 °C for 2 hrs. The resulting mixture was evaporated in vacuum and the residue was purified by flash column chromatography (ethyl acetate in petroleum ether, 10% v/v) to give Compound (**392b**) (71 g, 77% in two steps) as a white solid. LC-MS (ESI) m/z: 331 (M+1)⁺; ¹H NMR (400 MHz, CDCl₃): *δ* (ppm) 0.769 (s, 3H), 1.123 (s, 3H), 1.978 (s, 3H), 4.528 (t, *J =* 8.4 Hz, 1H), 5.661 (s, 1H).

Using the similar procedure and under the same conditions for the preparation of Compound (**392b**), replacing Compound **(392a)** with a corresponding alcohol, Compound (**393b**) was prepared (yield 40%) as a white solid. LC-MS (ESI) m/z: 345 (M+1)⁺.

### Example 265C

### Preparation of Compound (392c)

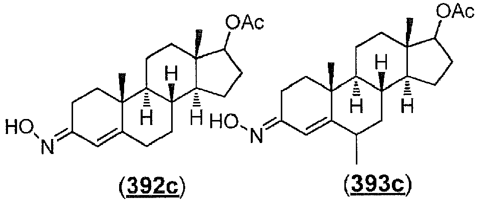

To a mixture of compound (**392b**) (33 g, 0.1 mol) in ethanol (200 mL) was added pyridine (10 mL), NH₂OH·HCl (8.34 g, 0.12 mol) and the mixture was stirred at room temperature for 16 hrs, filtered, washed with water, and dried to give Compound (**392c**) (30 g, 87%) as a white solid. The solid was directly used for next step without further purification.LC-MS (ESI) m/z: 346 (M+1)⁺.

Using the similar procedure and under the same conditions for the preparation of Compound (**392c**), replacing Compound (**392b**) with (**393b**), Compound (**393c**) was prepared (yield 100%) as a light yellow solid. LC-MS (ESI) m/z: 360 (M+1)⁺.

### Example 265D

### Preparation of Compound (392d)

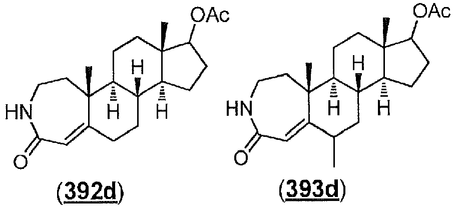

To a mixture of Compound (**392c**) (30 g, 87 mmol) in anhydrous THF (300 mL) was added SOCl₂ (7.6 mL, 0.1 mol) and the mixture was stirred at room temperature for 3 hrs. The resulting mixture was poured into ice-water (100 mL), extracted with CH₂Cl₂ (300 mL x 3). The organic layers were combined, washed with sat. NaHCO₃ (100 mL x 2) brine (100 mL), concentrated, and purified by flash column chromatography (methanol in ethyl acetate, 1% v/v) to give Compound (**392d**) (20 g, 58%) as a yellow solid. LC-MS (ESI) m/z: 346 (M+1)⁺. ¹H NMR (400 MHz, CDCl₃): *δ* (ppm) 0.838 (s, 3H), 1.103 (s, 3H), 2.062 (s, 3H), 3.184 (m, 2H), 4.613 (t, J= 8.4 Hz, 1H), 5.757 (s, 1H), 6.277 (s, 1H).

Using the similar procedure and under the same conditions for the preparation of Compound (**392d**), replacing Compound (**392c**) with (**393c**), Compound (**393d**) was prepared (yield 30%) as a yellow solid. LC-MS (ESI) m/z: 360 (M+1)⁺. ¹H-NMR (400 MHz, CDCl3) *δ* (ppm): 0.816 (s, 3H), 1.054 (d, *J=* 6.4 Hz, 3H), 1.150 (s, 3H), 2.040 (S, 3H), 3.179 (m, 2H), 4.579 (t, *J=* 8.0 Hz, 1H), 5.636 (s, 1H), 7.101 (m, 1H).

### Example 265E

### Preparation of Compound (392e)

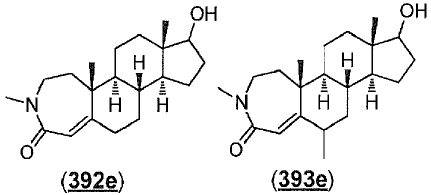

To a solution of compound (**392d**) (20 g, 58 mmol) in anhydrous DMF (150 mL) was added NaH (4.64 g, 116 mmol) and stirred at room temperature for 0.5 hr, then added CH₃I (3.5 mL, 70 mmol). The resulting mixture was stirred at 60 °C for 16 hrs, then cooled to room temperature and added with methanol (50 mL), water (50 mL) and NaOH (4.64 g, 116 mmol). The resulting mixture was stirred at room temperature for 2 hrs, and concentrate to remove methanol in vacuum. The residue was diluted with water, stirred at room temperature for 30 min, filtered. The solid was washed with water and dried to give Compound (**392e**) (13.8 g, 75%) as a yellow solid. LC-MS (ESI) m/z: 318 (M+1)⁺. ¹H NMR (400 MHz, CDCl₃): *δ* (ppm) 0.768 (s, 3H), 1.113 (s, 3H), 3.037 (m, 2H), 3.161 (m, 1H), 3.335 (m, 1H), 3.635 (t, *J=* 8.4 Hz, 1H), 5.807 (s, 1H).

Using the similar procedure and under the same conditions for the preparation of Compound **(392e),** replacing Compound (**392d**) with **(393d),** Compound **(393e)** was prepared (yield 100%). LC-MS (ESI) m/z: 332 (M+1)⁺.

### Example 265F

### Preparation of Compound (392f)

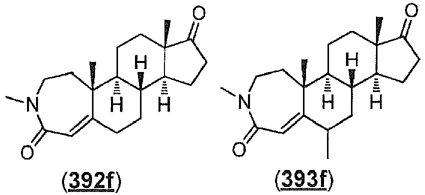

To a mixture of compound (**392e**) (2.63 g, 8.3 mmol) in CH₂Cl₂ (30 mL) was added pyridinium chlorochromate (3.3 g, 16.6 mmol) and the resulting mixture was stirred at room temperature for 3 hrs. The resulting mixture was diluted by CH₂Cl₂ (50 mL), filtered, washed by CH₂Cl₂, the filtrate was concentrated, and purified by flash column chromatography (ethyl acetate) to give Compound (**392f**) (1.5 g, 57%) as a white solid. LC-MS (ESI) m/z: 316 (M+1)⁺; ¹H NMR (400 MHz, CDCl₃): *δ* (ppm) 0.896 (s, 3H), 1.132 (s, 3H), 3.039 (s, 3H), 3.210 (m, 1H), 3.405 (m, 1H), 5.834 (s, 1H).

Using the similar procedure and under the same conditions for the preparation of Compound (**392f**), replacing Compound (**392e**) with (**393e**), Compound (**393f**) was prepared (yield 55%) as a yellow solid. LC-MS (ESI) m/z: 330 (M+1)⁺.

### Example 265G

### Preparation of Compound (392g)

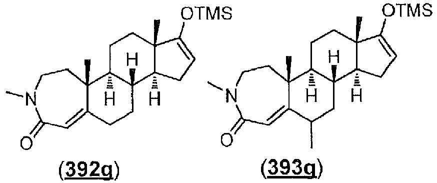

n-BuLi (2.5 M, 3.4 mL, 8.5 mmol) was added dropwise to a solution of diisopropylamine (1.27 mL, 9.44 mmol) in dried THF (10 mL) at -78 °C, stirred for 15 min, the solution of (**392f**) (1.49 g, 4.72 mmol) in THF (20 mL) was added slowly. Stirred for another 15 min, triethylamine (1.4 mL) and TMSCl (0.97 mL, 7.08 mmol) was added. The mixture was warmed to room temperature and stirred for 1 hour. The mixture was quenched with sat.NaHCO₃, extracted with ethyl acetate (100 mL x 2). The organic layer was washed with water, brine, dried and concentrated to give a crude product Compound (**392g**) (1.82 g, Crude) which used to next step without further purification. LC-MS (m/z) 388 [M+H]⁺.

Using the similar procedure and under the same conditions for the preparation of Compound (**392g**), replacing Compound (**392f**) with (**393f**). Compound (**393g)** was prepared (yield 100%). LC-MS (ESI) m/z: 402 (M+1)⁺.

### Example 265H

### Preparation of Compound (392h)

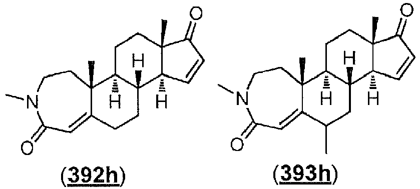

A mixture of Compound (**392g**) (1.82 g, crude) and Pd(OAc)₂ (1.17 g, 5.0 mmol) in DCM (30 mL) and MeCN (10 mL) was stirred at 35 °C for 2 hrs. The reaction mixture was filtered and the filtrate was concentrated, and purified by column chromatography (methanol in dichloromethane, 2% v/v) to give Compound (**392h**) (617 mg, 42% in two steps) as a brown solid.LC-MS (m/z) 314 [M+H]^{+ 1}H NMR (CDCl₃, 400 MHz) major characteristic peaks: *δ* (ppm) 0.90 (s, 3H), 0.98 (s, 3H), 2.85 (s, 3H), 3.02 (m, 1H), 3.23 (m, 1H), 5.67 (s, 1H), 6.05 (m, 1H), 7.51 (m, 1H).

Using the similar procedure and under the same conditions for the preparation of Compound (**392h**), replacing Compound (**392g**) with (**393g**), Compound (**393h**) was prepared (yield 100%). LC-MS (ESI) m/z: 402 (M+1)⁺.

### Example 265I

### Preparation of Compound (392i)

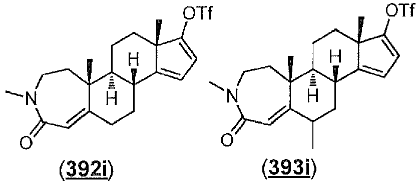

A mixture of Compoud (**392h**) (617 mg, 1.97 mmol) and triethylamine (0.35 mL, 2.52 mmol) in DCM (20 mL) was cooled to -10 °C. And Tf₂O (0.49.mL, 2.96 mmol) was added to the mixture slowly. It was stirred at the temperature for 30 min, diluted by DCM (20 mL), quenched by water (10 mL), and extracted with DCM (50 mL x 3). The organic layer was washed with Sat.NaHCO₃, water and brine, concentrated, and purified by column chromatography (methanol in dichloromethane, 1% v/v) to yield Compound (**392i**) (436 mg, 51%) as a yellow solid. LC-MS (m/z) 446 [M+H]^{+ 1}H NMR (CDCl₃, 400 MHz) major characteristic peaks: *δ* (ppm) 0.97 (s, 3H), 1.02 (s, 3H), 2.85 (s, 3H), 3.04 (m, 1H), 3.21 (m, 1H), 5.58 (m, 1H), 5.68 (m, 1H), 5.90 (m, 1H).

Using the similar procedure and under the same conditions for the preparation of Compound (**392i**), replacing Compound (**392h**) with (**393h**), Compound (**393i**) was prepared (yield 90%) as a brown oil. LC-MS (ESI) m/z: 460 (M+1)⁺.

### Example 265J

### Preparation of Compounds (392), (393), and (394)

A mixture of Compound (**392i**) (300 mg, 0.674 mmol), 5-fluoropyridin-3-ylboronic acid (142 mg, 1.011 mmol), Pd(dppf)₂Cl₂ (49 mg, 0.067 mmol) and K₂CO₃ (279 mg, 2.022 mmol) in 1,4-dioxane (20 mL) and water (2 mL) was stirred at 80 °C for 2 hrs. Filtered and the filtrate was diluted with ethyl acetate (100 mL), washed with water, brine, dried and concentrated to give the crude product. The crude product was purified by silica gel column chromatography (methanol in dichloromethane, 5% v/v) to afford Compound (**392**) (100 mg, 38%) as a white solid. LC-MS (m/z): 393 [M+H]^{+ 1}H NMR (CDCl₃, 400 MHz) major characteristic peaks: *δ* (ppm) 1.21 (s, 3H), 1.24 (s, 3H), 3.04 (s, 3H), 3.19-3.25 (m, 1H), 3.39-3.45 (m, 1H), 5.88 (s, 1H), 6.00 (s, 1H), 6.84 (s, 1H), 7.43-7.48 (m, 1H), 8.28 (s, 1H), 8.56 (s, 1H).

Using the similar procedure and under the same conditions for the preparation of Compound (**392**), replacing Compound (**392i**) with (**393i**) and 5-fluoropyridin-3-ylboronic acid with pyridin-3-ylboronic acid Compound (**393**) was prepared.

Compound (**393**) (yield 14%): LC-MS (ESI) m/z: 389 (M+1)⁺; ¹H-NMR (400 MHz, DMSO-*d*₆) *δ* (ppm): 1.070-1.086 (d, *J =* 6.4 Hz, 3H), 1.170 (s, 3H), 1.220 (s, 3H), 2.893 (s, 3H), 5.524 (s, 1H), 5.995 (s, 1H), 6.897-6.991 (d, *J=* 1.6 Hz, 1H). 7.329-7.362 (dd, *J*₁ = 8.0 Hz, *J*₂ = 4.8 Hz, 1H), 7.890-7.910 (d, *J=* 8.0 Hz, 1H), 8.374-8.388 (dd, *J*₁ = 4.4 Hz, *J*₂ = 1.2 Hz, 1H), 8.767-8.772 (d, *J=* 2.0 Hz, 1H).

Using the similar procedure and under the same conditions for the preparation of Compound (**392**), replacing Compound (**392i**) with (**393i**), Compound **(394)** was prepared.
Compound (**394**) (yield 14%) as a yellow solid: LC-MS (ESI) m/z: 407 (M+1)⁺; ¹H-NMR (400 MHz, CDCl3) *δ* (ppm): 1.162-1.178 (d, *J =* 6.4 Hz, 3H), 1.207 (s, 3H), 1.247 (s, 3H), 3.041 (s, 3H), 3.179-3.235 (m, 1H), 3.367-3.426 (m, 1H), 5.790 (s, 1H), 5.982 (s, 1H), 6.839-6.844 (d, *J* = 2.0 Hz, 1H). 7.439-7.436 (m, 1H), 8.278-8.284 (d, *J* = 2.4 Hz, 1H). 8.568 (s, 1H).

### Example 266

### Preparation of Compound (395)

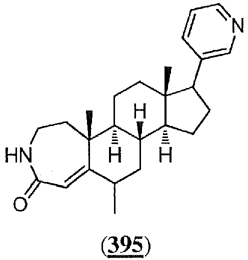

To a solution of Compound (**368**) (20 mg) in MeOH (5 mL) was added Pd/C (5 mg) and the mixture was stirred at room temperature for 2 hrs under H₂. The mixture was filtered to remove the Pd/C solid, concentrated to give a crude product, which was purified by prep-TLC (ethyl acetate) to afford (**395**) (5 mg, 25%) as a white solid. LC-MS m/z: 379 (M+1). ¹H NMR (CDCl3, 400 MHz) peaks: *δ* (ppm) 0.51 (s, 3H), 1.08 (d, *J =* 6.4, 3H), 1.15 (s, 3H), 2.67 (t, *J =* 9.6, 1H), 3.18 (m, 2H), 5.67 (s, 1H), 6.30 (s, 1H), 7.22 (m, 1H), 7.52 (d, *J =* 8.0, 1H), 8.45 (s, 2H).

### Example 267

### Preparation of Compound (396)

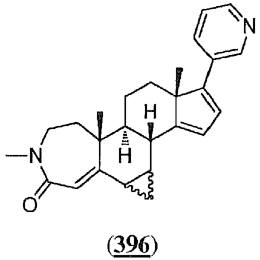

### Example 267A

### Preparation of Compound (396a)

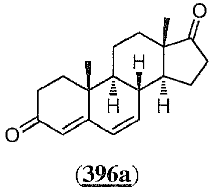

To a solution of Compound (**332b**) (15 g, 41 mmol) in DMF (150 mL) was added anhydrous KF (4.8 g, 82 mmol) and the mixture was stirred at 150 °C for 2 hrs. After cooled to room temperature, the mixture was poured into water (600 mL) and filtered. The filtered cake was disolved in DCM (300 mL) and washed with water (200 mL x 3), brine (200 mL), dried over anhydrous Na₂SO₄, and concentrated to give Compound (**396a**) (10 g, 86%) as a brown oil. LCMS: m/z: 285 (M+1); ¹H NMR (CDCl₃, 400 MHz) peaks: *δ* (ppm) 0.975 (s, 3H), 1.148 (s, 3H), 5.706 (s, 1H), 6.190 (s, 2H).

### Example 267B

### Preparation of Compound (396b)

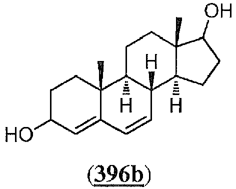

To a solution of Compound (**396a**) (10 g, 35.2 mmol) in DCM (150 mL) and EtOH (100 mL) was added NaBH₄ (1.47g, 38.7 mmol) at 0 °C. The mixture was stirred at room temperature for 2 hrs. The mixture was diluted with DCM (500 mL) and washed with water (300 mL x 3), brine (300 mL), dried over anhydrous Na₂SO₄, and concentrated to give Compound (**396b**) (9.1 g, 90%) as a yellow solid. LCMS: m/z: 289 (M+1).

### Example 267C

### Preparation of Compound (396c)

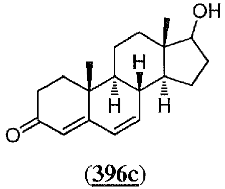

To a solution of Compound (**396b**) (24.8 g, 86.1 mmol) in DCM (150 mL) was added MnO₂ (37.5 g, 431 mmol). The mixture was stirred at room temperature for 4 hrs. The mixture was filtered to remove MnO₂, washed with a mixture solvents of DCM/MeOH (200 mL, 10/1 v/v). The organic layer was concentrated to yield Compound (**396c**) (22.2 g, 90%) as a brown solid. LC-MS: m/z: 287 (M+1). ¹H NMR (CDCl₃, 400 MHz) peaks: *δ* (ppm) 0.846 (s, 3H), 1.126 (s, 3H), 3.678 (t, J = 8.4 Hz, 1H), 5.678 (s, 1H), 6.106 (s, 2H).

### Example 267D

### Preparation of Compound (396d)

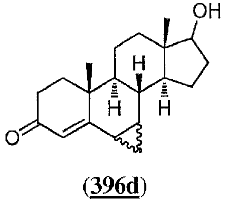

To a solution of Trimethyl sulfoxonium iodide (24.9 g, 113.3 mmol) in DMSO (250 mL) was added NaH (60%, 5.65 g, 141.3 mmol) at 0 °C. The mixture was stirred at room temperature for 1 hr, then was added Compound (**396c**) (16.2 g, 56.6 mmol). The mixture was stirred at 40 °C overnight. After cooled to room temperature, the mixture was poured into ice-water (500 mL), and extracted with DCM (200 mL x 4). The organic phases were combined and washed with water (500 mL x 3), brine (500 mL), dried over anhydrous Na₂SO₄, and concentrated to give Compound (**396d**) (16 g, 94%) as a yellow solid. LC-MS: m/z: 301 (M+1). ¹H NMR (CDCl₃, 400 MHz) peaks: *δ* (ppm) 0.775 (s, 3H), 1.085 (s, 3H), 3.701 (t, *J =* 7.2 Hz, 1H), 6.004 (s, 1H).

### Example 267E

### Preparation of Compound (396)

Following the similar procedures and under the same conditions described in Example 263D, Example 263E, Example 263F, Example 263G, Example 263H, Example 263I, and Example 263J for the preparation of Compound (**392**) and using Compound (**396d**), Compound (**396**) was prepared as a pale yellow solid. LC-MS: m/z: 387 (M+1). ¹H NMR (CDCl3, 400 MHz) peaks: *δ* (ppm) 0.538 (m, 1H), 0.868 (m, 2H), 1.118 (s, 1.5H), 1.190 (s, 1.5H), 1.200 (s, 1.5H), 1.225 (s, 1.5H), 3.043 (m, 3H), 3.212 (m, 1H), 3.471 (m, 1H), 5.881 (s, 0.1H), 5.994 (s, 0.1H), 6.033 (s, 0.5H), 6.178 (s, 0.5H), 6.266 (m, 1H), 6.790 (m, 1H), 7.254 (s, 1H), 7.759 (m, 1H), 8.432 (s, 1H), 8.759 (s, 1H).

### Example 268

### Preparation of Compounds (397), (398), and (399)

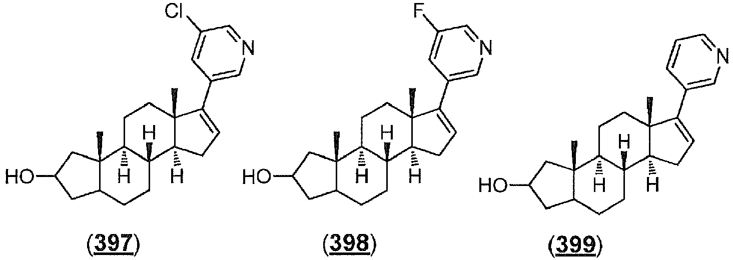

### Example 268A

### Preparation of Compound (397a)

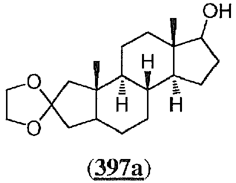

A solution of (3aS,5aS)-6-hydroxy-3a,5a-dimethyltetradecahydrodicyclopenta[a,f]naphthalen-2(1H)-one (3.5 g, 0.01 mol), CH(OEt)₃ (4 mL), ethane-1,2-diol (4 mL) andp-TsOH (308 mg, 1.62 mmol) in THF (10 mL) was stirred at room temperature for 2 hrs. Then it was adjusted to pH = 8 with Et₃N and diluted with ethyl acetate (200 mL). The organic layers were washed with water and brine, dried with anhydrous Na₂SO₄, and evaporated to obtain compound **(397a)** (3.4 g, 83%) as a white solid. LCMS: 321 [M+1]⁺; ¹H NMR (CDCl₃, 400 MHz) major characteristic peaks: *δ* (ppm) 0.726 (s, 3H), 0.857 (s, 3H), 3.626 (t, *J=* 8.8 Hz, 1H), 3.758∼3.923 (m, 4H).

### Example 268B

### Preparation of Compound (397b)

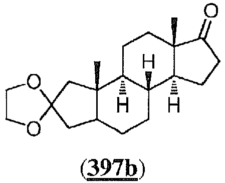

To a solution of compound **(397a)** (2.10 g, 6.07 mmol) in DCM (20 mL) was added DMP (3.09 g, 7.28 mmol) as 0 °C. Then the mixture was stirred at this temperature for 1 hr. Then it was quenched with ethanol and filtered to remove solid. The filtrate was diluted with ethyl acetate (150 mL), washed with water and brine, dried with anhydrous Na₂SO₄, and purified by column chromatography (petroleum ether/ethyl acetate = 8/1, v/v) to obtain Compound (**397b**) (1.87 g, 90%) as a white solid.
LC-MS (m/z): 319 [M+1]⁺.

### Example 268C

### Preparation of Compound (397c)

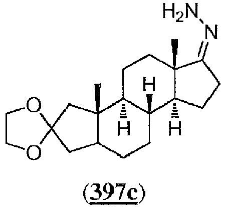

To a suspension of compound (**397b**) (1.87 g, 5.44 mmol) in EtOH (20 mL) was added NH₂NH₂ aqueous (80%, 8 mL) and stirred at room temperature for 2 hrs. The mixture was poured into ice water to form white solid. It was filtered and dried to yield Compound (**397c**) (1.60 g, 82%) as a white solid. LC-MS: (m/z) 333 [M+1]⁺.

### Example 268D

### Preparation of Compound (397d)

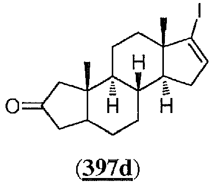

To a solution of compound (**397c**) (1.60 g, 4.47 mmol) and Et₃N (1 mL) in DCM (10 mL) was added dropwise the solution of I₂ (1.70 g, 6.70 mmol) in THF (2 mL) at 0 °C. The solution was stirred at 0 °C for 2 hrs. Then it was quenched with Na₂SO₃ solution to destroy excess I₂, diluted with ethyl acetate (150 mL), washed with water and brine, dried with anhydrous Na₂SO₄, and purified by column chromatography (petroleum ether/ ethyl acetate = 15/1, v/v) to afford Compound (**397d**) (1.40 g, 69%) as a yellow solid. LCMS: (m/z) 385 [M+1] ⁺; ¹H NMR (CDCl₃, 400 MHz) major characteristic peaks: *δ* (ppm) 0.68 (s, 3H), 0.80 (s, 3H), 6.03 (m, 1H).

### Example 268E

### Preparation of Compound (227e), (398e), and (399e)

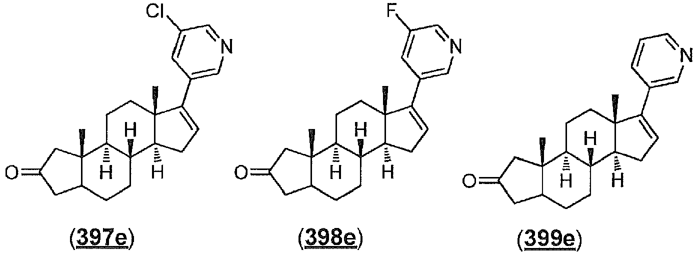

An suspension of compound (**397d**) (520 mg, 1.27 mmol), 5-chloropyridin-3-ylboronic acid (234 mg, 1.90 mmol), PdCl₂(PPh₃)₂ (91 mg, 0.13 mmol) and K₂CO₃ (512 mg, 3.71 mmol) in dioxane/H2O (20/4 mL) was stirred at 80 °C for overnight. Then it was diluted with ethyl acetate (150 mL), washed with water and brine, dried with anhydrous Na₂SO₄, concentrated to yield a crude product. The crude product was purified by prep-HPLC to obtain compound Compound (**397e**) (450 mg, 82%) as a white solid. LC-MS: (m/z) 370 [M+1]⁺.

Using the similar procedure and under the same conditions for the preparation of Compound (**397e**), replacing 5-chloropyridin-3-ylboronic acid with 5-fluoropyridin-3-ylboronic acid and pyridin-3-ylboronic acid, Compound (**398e**) and (**399e**) were prepared. Compound (**398e**) Yield 85% as a yellow solid: LC-MS (ESI) m/z: 354 (M+1)⁺. Compound (**398e**) Yield 68% as a yellow solid: LC-MS (ESI) m/z: 336 (M+1)⁺.

### Example 268F

### Preparation of Compounds (397), (398), and (399)

To a solution of compound (**397e**) (335 mg, 1 mmol) in MeOH (15 mL) was added NaBH₄ (38 mg, 1 mmol) at room temperature under N₂. The reaction mixture was stirred at room temperature for 1 hr. Then the reaction was quenched with sat NaHCO₃ (1.5 mL), and dilute with water (15 mL), extracted with Dichloromethane (2 x 15 mL). The combined organic layers were washed with brine (25 mL) and dried over Na₂SO₄, and evaporated to afford a crude product, which was purified by column chromatography (petroleum ether/ethyl acetate =12/1 to 7/1, v/v) to give Compound (**397**) (120 mg, 45%) as a white solid. LC-MS (m/z): 372 [M+1] ¹H NMR (CDCl₃, 400 MHz) major characteristic peaks: *δ* (ppm) 0.96 (s, 3H), 1.01 (s, 3H), 4.42 (m, 1H), 6.02 (m, 1H), 7.62 (m, 1H), 8.42 (s, 1H), 8.62 (s, 1H).

Using the similar procedure and under the same conditions for the preparation of Compound (**397**), replacing (**397e**) with (**398e**) and (**399e**), Compound (**398**) and (**399**) were prepared. Compound (**398**) Yield 30% as a white solid: LC-MS (m/z): 356 [M+H]⁺; ¹H NMR (CDCl₃, 400 MHz) major characteristic peaks: *δ* (ppm) 0.96 (s, 3H), 1.02 (s, 3H), 4.43 (m, 1H), 6.03 (m, 1H), 7.36 (m, 1H), 8.32 (m, 1H), 8.44 (s, 1H).

Compound (**399**) Yield 36% as a white solid: LC-MS (m/z): 338 [M+H]⁺; ¹H NMR (CDCl₃, 400 MHz) major characteristic peaks: *δ* (ppm) 0.96 (s, 3H), 1.02 (s, 3H), 4.41 (m, 1H), 5.97 (m, 1H), 7.24 (m, 1H), 7.64 (m, 1H), 8.42 (s, 1H), 8.61 (s, 1H).

### Example 269

### Preparation of Compound (400)

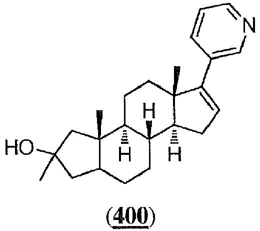

To a solution of Compound (**399e**) (384 mg, 1 mmol) in THF (20 mL) was added MeMgBr/THF solution (3M, 3 mL) at 0 °C. The mixture was stirred at room temperature about 2 hrs. The reaction mixture was quenched with aq. NH₄Cl solution and extraceted with ethyl acetate (50 mL x 3). The combined organic layers were dried, evaporated, and purified by prep-HPLC to give Compound (**400**) (60 mg) as a white solid. LC-MS (m/z): 352 [M+H]^{+ 1}H NMR (CDCl₃, 400 MHz) major characteristic peaks: *δ* (ppm) 0.99 (s, 3H), 1.02 (s, 3H), 1.35 (s, 3H), 5.97 (s, 1H), 7.22 (m, 1H), 7.65 (m, 1H), 8.46 (m, 1H), 8.61 (s, 1H).

### Example 270

### Preparation of Compound (401)

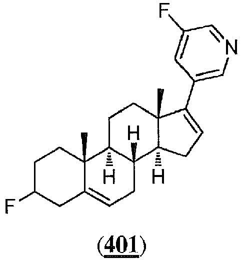

To a solution of compound DAST (0.1 mL, 0.82 mmol) in dry dichloromethane (5 mL) was added a solution of Compound (**274**) (200 mg, 0.54 mmol) in dry dichloromethane (5 mL) under N₂ at -78 °C. The mixture was stirred and warmed to room temperature (about 30 °C) for 2 hrs. The reaction mixture was cooled down and poured into ice-water (100 mL). It was extracted with ethyl acetate (100 mL x 3), washed with water, brine, and dried over Na₂SO₄, and concentrated to yield a crude product. The crude product was purified by silica gel column chromatography (ethyl acetate in petroleum, 10% v/v) to afford Compound (**401**) (109 mg, 54%) as a white solid. LC-MS (m/z): 370 [M+H]⁺; ¹H NMR (CDCl₃, 400 MHz) major characteristic peaks: *δ* (ppm) 1.05 (s, 3H), 1.09 (s, 3H), 4.46 (m, 1H), 5.43 (m, 1H), 6.07 (m, 1H), 7.37 (td, *J =* 2.0 Hz, 10.0 Hz, 1H), 8.32 (d, *J* = 2.0 Hz, 1H), 8.45 (s, 1H).

### Example 271

### Preparation of Compound (402), (403), (404), (405), (406), and (407)

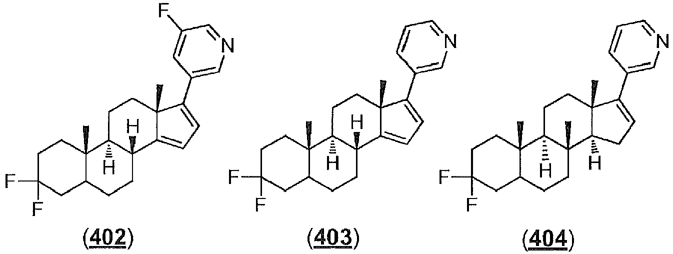

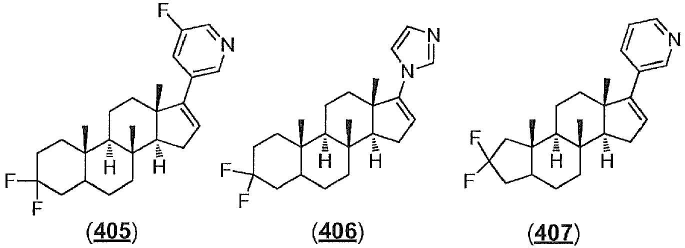

### Example 271A

### Preparation of Compound (402a)

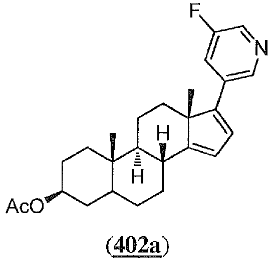

To a solution of Compound (**161**) (1.0 g, 2.2 mmol) in 1,4-dioxane (10 mL) and H₂O (2 mL) was added 5-fluoropyridin-3-ylboronic acid (451 mg, 3.2 mmol), K₂CO₃ (895 mg, 6.48 mmol) and Pd(PPh₃)₂Cl₂ (151 mg, 0.22 mmol) under N₂. The mixture was stirred at 80°C for 2-3 hrs and then quenched with NH₄Cl aq. (15 mL). It was extracted with EtOAc (3 x 30 mL), washed with brine (15 mL), dried over Na₂SO₄, and concentrated to give a crude product. The crude product was purified by column chromatography (petroleum ether/ethyl acetate = 15/1-5/1, v/v) to afford Compound (**402a**) (630 mg, 70%) as a white solid. LC-MS: (m/z) 410.2 (M+1)⁺. ¹H-NMR (400 MHz, CDCl₃) : *δ* (ppm) 0.969 (s, 3H), 1.174 (s, 3H), 2.034 (s, 3H), 2.365 (m, 2H), 4.698 (m, 2H), 5.997 (s, 1H), 6.849-6.855 (d, *J* = 2.4 Hz, 1H), 6.745 (m, 1H), 8.258-8.265 (d, *J=* 2.8 Hz, 1H), 8.574 (s, 1H).

### Example 271B

### Preparation of Compound (402b)

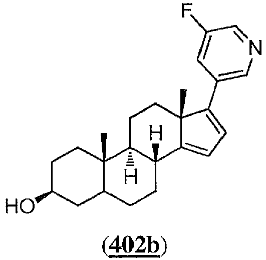

To a stirred solution of Compound (**402a**) (630 mg, 1.54 mmol) in MeOH (15 mL) was added 3M NaOH aq. (2mL), then stirred for 2 hrs at 70 °C. It was then quenched with sat. aq. NH₄Cl, extracted with CH₂Cl₂ (3 x 15 mL). The organic phase was washed with brine (2 x 15 mL), dried over Na₂SO₄, and evaporated under reduced pressure. Purification by column chromatography (petroleum ether/ethyl acetate = 7/1-5/1, v/v) gave Compound (**402b**) (530 mg, 93%) as a white solid. LC-MS: (m/z) 368.2 (M+1)⁺. ¹H-NMR (400 MHz, CDCl₃): *δ* (ppm) 0.969 (s, 3H), 1.174 (s, 3H), 2.365 (m, 2H), 4.698 (m, 2H), 5.997 (s, 1H), 6.849-6.855 (d, *J* = 2.4 Hz, 1H), 6.745 (m, 1H), 8.258-8.265 (d, *J* = 2.8 Hz, 1H), 8.574 (s, 1H).

### Example 271C

### Preparation of Compound (402c)

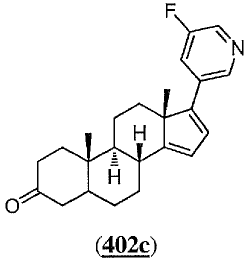

To a stirred solution of Compound (**402b**) (530 mg, 1.52 mmol) in CH₂Cl₂ (15 mL) was added DMP (773mg, 1.82mmol). The mixture was then stirred for 2 hrs at r.t. before quenched with sat. aq. Na₂SO₃. It was extracted with CH₂Cl₂ (3 x 15 mL) and organic phase was washed with brine (2 x 15 mL), dried over Na₂SO₄, and evaporated under reduced pressure. Purification by column chromatography (petroleum ether/ethyl acetate = 15/1-12/1, v/v) afforded Compound (**442c**) (390 mg, 70%) as a white solid. LC-MS: (m/z) 366.2 (M+1)⁺. ¹H-NMR (400 MHz, CDCl₃): *δ* (ppm) 0.969 (s, 3H), 1.174 (s, 3H), 5.997 (s, 1H), 6.849-6.855 (d, *J =* 2.4 Hz, 1H), 6.745 (m, 1H), 8.258-8.265 (d, *J=* 2.8 Hz, 1H), 8.574 (s, 1H).

### Example 271D

### Preparation of Compounds (402), (403), (404), (405), (406), and (407)

To a stirred solution of Compound (**402c**) (390 mg, 1.07 mmol) in CH₂Cl₂ (15 mL) was added DAST (518mg, 3.2mmol) at 0 °C. The reaction mixture was stirred for 12 hrs at r.t. before quenched with ice water. The mixture was then extracted with CH₂Cl₂ (3 x 15 mL) and organic phase was washed with brine (2 x 15 mL), dried over Na₂SO₄, and evaporated under reduced pressure. Purification by column chromatography (petroleum ether/ethyl acetate = 15/1-12/1, v/v) afforded Compound (**402**) (103 mg, 27%) as a white solid. LCMS: (m/z) 388.1 (M+1)⁺. ¹H-NMR (400 MHz, CDCl₃): *δ* (ppm) 0.974 (s, 3H), 1.181 (s, 3H), 2.350 (m, 2H), 6.011 (s, 1H), 6.855-6.861 (d, *J =* 2.4 Hz, 1H), 6.745 (m, 1H), 8.267-8.274 (d, *J =* 2.8 Hz, 1H), 8.575 (s, 1H).

Using the similar procedure and under the same conditions for the preparation of Compound (**402**), replacing (**402c**) with the corresponding ketones, Compounds (**403**) ∼ (**407**) were prepared.

Compound (**403**) (Yield 47%) as a white solid: LCMS: m/z: 370 (M+1). ¹H NMR (CDCl₃, 400 MHz): *δ* (ppm) 0.97 (s, 3H), 1.17 (s, 3H), 5.99 (t, *J =* 2 Hz, 1H), 6.79 (d, *J* = 2.4 Hz, 1H), 7.23 (m, 1H), 7.76 (m, 1H), 8.41 (m, 1H), 8.75 (d, *J* = 1.6 Hz, 1H).

Compound (**404**) (Yield 30%) as a white solid: LC-MS (ESI) m/z: 372 (M+1)⁺; ¹H-NMR (400 MHz, CDC13): *δ* (ppm) 0.88 (s, 3H), 1.01 (s, 3H), 5.97-5.98 (m, 1H), 7.20-7.23 (m, 1H), 7.63-7.65 (m, 1H), 8.46 (s, 1H), 8.61 (s, 1H).

Compound (**405**) (Yield 35%) as a white solid: LC-MS (ESI) m/z: 390 (M+1)⁺; ¹H-NMR (400 MHz, CDC13): *δ* (ppm) 0.85 (s, 3H), 0.98 (s, 3H), 6.01-6.02 (m, 1H), 7.32-7.35 (m, 1H), 8.29-8.30 (m, 1H), 8.40 (s, 1H).

Compound (**406**) (Yield 19%) as a white solid: CMS (m/z): 361.0 [M+H]⁺. H NMR (CDCl₃, 400 MHz) major characteristic peaks: *δ* (ppm) 0.88 (s, 3H), 0.97 (s, 3H), 5.67-5.68 (m, 1H), 7.04 (s, 1H), 7.09 (s, 1H), 7.63 (s, 1H).

Compound (**407**) (Yield 2%) as a white solid: LC-MS (m/z): 358 [M+H]^{+ 1}H NMR (CDCl₃, 400 MHz) major characteristic peaks: *δ* (ppm) 0.92 (s, 3H), 1.02 (s, 3H), 5.97 (m, 1H), 7.26 (m, 1H), 7.65 (m, 1H), 8.56, 8.67 (s, 2H).

### Example 272

### Preparation of Compound (408) and (409)

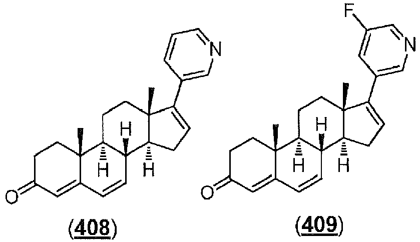

A mixture of (10R,13S)-17-iodo-10,13-dimethyl-8,9,10,11,12,13,14,15-octahydro-1H-cyclopenta[a]phenanthren-3(2H)-one (200 mg, 0.508 mmol), pyridin-3-ylboronic acid (125 mg, 1.015 mmol), Pd(PPh₃)₂Cl₂ (36 mg, 0.0508 mmol), K₂CO₃ (351 g, 2.54 mmol) in dioxane (5 mL) and water (1 mL) was stirred at 80 °C for 2 hrs. The reaction mixture was diluted with water (100 mL), extracted with ethyl acetate (100 mL), washed with water, concentrated, and purified by prep-HPLC to obtain (**408**) (33 mg, 18%) as a white solid. LC-MS (m/z): 346.2 [M+H]⁺; ¹H NMR (CDCl₃, 400 MHz) major characteristic peaks: *δ* (ppm) 1.10 (s, 3H), 1.17 (s, 3H), 5.71 (s, 1H), 6.02-6.03 (m, 1H), 6.15-6.22 (m,

Using the similar procedure and under the same conditions for the preparation of Compound (**408**), replacing pyridin-3-ylboronic acid with 5-fluoropyridin-3-ylboronic acid Compound (**409**) was prepared.

Compound **(409)** (Yield 30%) as a white solid: LC-MS (ESI) m/z: 364 (M+1)⁺; ¹H-NMR (400 MHz, CDCl₃): *δ* (ppm) 1.08 (s, 3H), 1.14 (s, 3H), 6.57 (s, 1H), 6.08-6.09 (m, 1H), 6.18 (s, 2H), 7.35-7.38 (m, 1H) 8.35 (s 1H), 8.45 (s, 1H).

### Example 273

### Preparation of Compound (410) and (411)

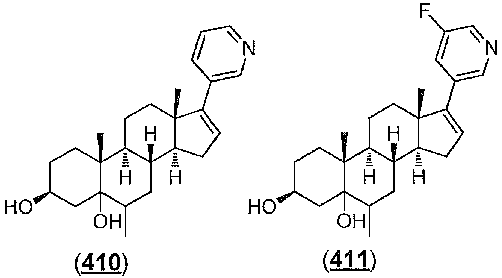

### Example 273A

### Preparation of Compound (4101a)

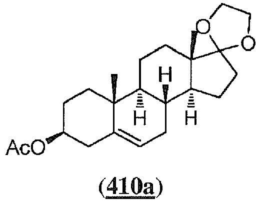

To a solution of (3S,10R,13S)-10,13-dimethyl-17-oxo-2,3,4,7,8,9,10,11,12,13,14,15,16,17-tetradecahydro-1H-cyclopenta[a]phenanthren-3-yl acetate (20 g, 60.6 mmol) in THF (100 mL) was added *p*-TsOH (1.15 g, 6.06 mmol), CH(OEt)₃ (27 g, 182 mmol), ethane-1,2-diol (100 mL). The mixture was stirred at room temperature for 1 hr, quenched with NEt₃ (adjusted to pH = 9). It was concentrated and poured into ice-water. The solid was collected by filtration and then dried to give Compound (**410a**) (22.2 g, 98%) as a white solid. LCMS (m/z): 375 (M+1)⁺; ¹H-NMR (400 MHz, CDCl₃): *δ* (ppm) 0.862 (s, 3H), 1.024 (s, 3H), 2.031 (s, 3H), 3.858 (m, 4H), 4.603 (m, 1H), 5.370 (m, 1H).

### Example 273B

### Preparation of Compound (410b)

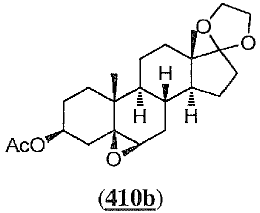

To a solution of Compound (**410a**) (5 g, 13.4 mmol) in DCM (80 mL) was added KMnO₄ (6 g), Fe₂(SO₄)₃ (2.5 g), H₂O (10 mL), t-BuOH (10 mL). The mixture was stirred at room temperature overnight. The mixture was filtered to remove brown solid, diluted with H₂O (200 mL), extracted with DCM (2 x 200 mL). The organic phase was combined and washed with water (3 x 100 mL), brine (100 mL), dried over Na₂SO₄ and concentrated to yield Compound (**410b**) (2.17 g, 42%) as a white solid. ¹H-NMR (400 MHz, CDCl₃): *δ* (ppm) 0.19 (s, 3H), 1.011 (s, 3H), 2.028 (s, 3H), 3.081 (m, 4H), 3.834 (m, 4H), 4.742 (m, 1H).

### Example 273C

### Preparation of Compound (410c)

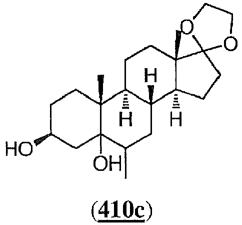

To a solution of (**410b**) (6.9 g , 19.8 mmol) and CuI (11.3 g, 59.4 mmol) in anhydrous THF (250 mL) under N₂ atmosphere at 0 °C was added MeMgCl (3 M in ethyl ether, 132 mL, 396 mmol) dropwise. The reaction mixture was stirred at 0 °C for 0.5 hr and then heated to 50 °C (oil bath) for 5-6 hrs before a sat. NH₄Cl solution (300 mL) was added. The aqueous layer was extracted with ethyl acetate (400 mL x 5) and the combined organic extracts were washed with water, dried, and concentrated to give a crude product. The crude product was purified by flash chromatography (hexane/ ethyl acetate = 2/1, v/v) to afford Compound (**410c**) (5 g, 69%) as a white solid. LC-MS (m/z): 365 (M+1)⁺. ¹H-NMR (400 MHz, CDCl₃): *δ* (ppm) 0.86 (s, 3H), 1.02-1.04 (d, *J =* 7.2 Hz, 3H), 1.06 (s, 3H), 3.83-3.94 (m, 4H), 4.11 (m, 1H).

### Example 273D

### Preparation of Compound (410d)

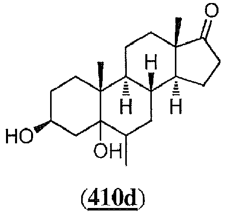

To a stirring solution of Compound (**410c**) (5 g, 13.7 mmol) in THF (150 mL) at 0 °C was added HClO₄ (138 mg, 1.37 mmol). The reaction mixture was warmed to room temperature and stirred for 3 hrs before a sat. NaHCO₃ solution (100 mL) was added. The aqueous layer was extracted with ethyl acetate (200 mL x 5) and the combined organic extracts were concentrated under reduced pressure to give a crude product. The crude product was washed with ethyl acetate to afford Compound (**410d**) (1.8 g, 41% yield) as a white solid. LC-MS (m/z): 321 (M+1)⁺. ¹H-NMR (400 MHz, CDCl₃): *δ* (ppm) 0.89 (s, 3H), 1.07-1.08 (d, *J* = 7.2 Hz, 3H), 1.08 (s, 3H), 4.14 (m, 1H).

### Example 273E

### Preparation of Compound (410e)

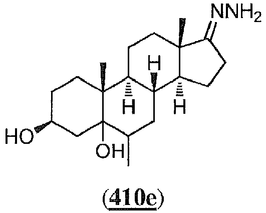

To a solution of Compound (**410d**) (1.8 g, 5.6 mmol) in ethanol (50 mL) was added N₂H₄ H₂O (2.1 g, 56 mmol), then the mixture was stirred at room temperature overnight, then to the reaction mixture was added water. The solid was collected by filtration to obtain (**61e**) (920 mg, 51%) as a white solid. LC-MS (m/z): 335 (M+1).

### Example 273F

### Preparation of Compound (410f)

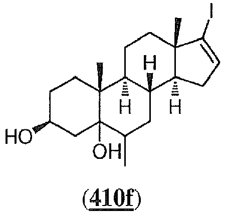

To a solution of Compound (**410e**) (920 mg, 2.7mmol) in DCM (20 mL) was added Et₃N (416 mg, 4.13 mmol) at 0°C, then to the mixture was added I₂ (838 mg, 3.3 mmol) in THF (10 mL) dropwised, then the mixture was stirred at room temperature for 2 hrs, then washed with sat.Na₂SO₃, extracted with DCM (2 x 100 mL). The organic layer was washed with brine (100 mL), dried over Na₂SO₄, and concentrated. The crude product was purified by column chromatography (petroleum ether/ethyl acetate = 5/1, v/v) to give Compound (**410f**) (500 mg, 42%) as a yellow solid. ¹H NMR (CDCl₃, 400 MHz): *δ* (ppm) 0.75 (s, 3H), 1.06 (d, *J =* 7.6 Hz, 3H), 1.09 (s, 3H), 4.13 (m, 1H), 6.12 (m, 1H).

### Example 273G

### Preparation of Compounds (410) and (411)

To a solution of Compound (**410f**) (250 mg, 0.58 mmol) in dioxane (20 mL) was added pyridin-3-ylboronic acid (143 mg, 1.16 mmol), potassium carbonate (400 mg, 2.9 mmol) in water (2 mL), and bis(triphenylphosphine) palladium chloride (41mg). The mixture was thoroughly degassed, and heated under nitrogen at 80 °C for 1 hour. After being filtered through a pad of Celite, the crude product was purified by prep-TLC (ethyl acetate in petroleum, 50% v/v) to give Compound (**410**) (35 mg, 16%) as a pale yellow solid. LC-MS (m/z): 382 (M+1). ¹H NMR (CDCl₃, 400 MHz) characteristic peaks: *δ* (ppm) 1.03 (s, 3H), 1.09 (d, *J =* 7.6 Hz, 3H), 1.12 (s, 3H), 4.14 (m, 1H), 5.98 (m, 1H), 7.21 (m, 1H), 7.64 (m, 1H), 8.45 (m, 1H), 8.61 (d, *J* = 2.0 Hz, 1H).

Using the similar procedure and under the same conditions for the preparation of Compound (**410**), replacing pyridin-3-ylboronic acid with 5-fluoropyridin-3-ylboronic acid Compound (**411**) was prepared.

Compound (**410**) (Yield, 29 %) as a white solid: LCMS (m/z): 400 (M+1). ¹H NMR (CDCl₃, 400 MHz) characteristic peaks: *δ* (ppm) 1.04 (s, 3H), 1.10 (d, *J =* 7.6 Hz, 3H), 1.12 (s, 3H), 4.14 (m, 1H), 6.05 (m, 1H), 7.37 (m, 1H), 8.32 (d, *J* = 2.8 Hz, 1H), 8.44 (d, *J* =1.6 Hz, 1H).

### Example 274

### Preparation of Compound (412)

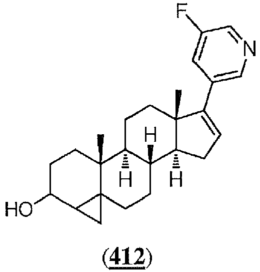

### Example 274A

### Preparation of Compound (412a)

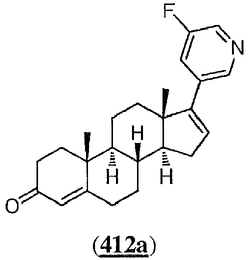

The suspension of (10R,13S)-17-iodo-10,13-dimethyl-6,7,8,9,10,11,12,13,14,15-decahydro-1H-cyclopenta[a]phenanthren-3(2H)-one (2.1 g, 5.3 mmol), 5-fluoropyridin-3-ylboronic acid (1.7 g, 11.7 mmol), PdCl₂(PPh₃)₂ (186 mg, 0.27 mmol) and K₂CO₃ (2.93 g, 21.2 mmol) in dioxane/H₂O (20/10 mL) was stirred at 80 °C overnight. Then it was diluted with ethyl acetate (200 mL), washed with water and brine, dried with anhydrous Na₂SO₄, and purified by column chromatography, eluted with (petroleum ether/ ethyl acetate = 3/1 to 1/1, v/v) to obtain Compound (**412a**) (0.9 g, 46%) as a yellow solid. LC-MS (ESI) m/z: 366 (M+H)⁺

### Example 274B

### Preparation of Compound (412b)

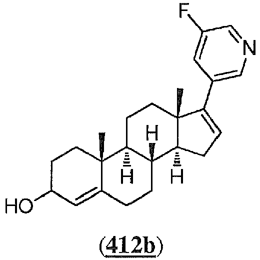

To a solution of (**412a**) (885 mg, 2.42 mmol) in MeOH (70 mL) was added NaBH₄ (101 mg, 2.66 mmol) and CeCl₃ (60 mg, 0.24 mmol) at 25 °C under N₂. The reaction mixture was stirred for 1 hour at 25 °C, then 30 mL of saturated NaHCO₃ was added to quench the reaction. The mixture was diluted with water (24 mL), extracted by dichloromethane (3 x 150 mL). The combined organic layers were dried with anhydrous Na₂SO₄, and evaporated to obtain a crude product, which was purified by column chromatography, eluted with (petroleum ether/ ethyl acetate = 3/1 to 1/1, v/v) to afford Compound **(412b)** (850 mg, 96%) as a white solid. LC-MS (ESI) m/z: 350 (M+H)⁺; ¹H-NMR (400 MHz, DMSO-*d*₆) δ (ppm) 0.97 (s, 3H), 1.01 (s, 3H), 3.90-3.93 (m, 1H), 4.55-4.57 (d, *J=* 5.6 Hz, 1H), 5.21 (s, 1H), 6.09 (s, 1H), 7.31-7.35 (dd, *J=* 4.8,, 8.0 Hz, 1H), 7.74-7.76 (dd, *J*=2.0, 5.6 Hz, 1H), 8.43-8.44 (m, 1H), 8.57-8.58 (m, 1H).

### Example 274C

### Preparation of Compound (412)

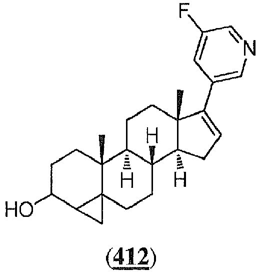

To a solution of (**412b**) (100 mg, 0.27 mmol) and Zn (active) (450 mg, 6.81 mmol) in dry DME (7 mL) was added a small crystal of iodine, followed by CH₂I₂ (0.17 mL, 2.04 mmol) at 25 °C under N₂. The reaction mixture was stirred for 2 hrs at 96 °C, then saturated NaHCO₃ (20 mL) was added to quench the reaction. The mixture was extracted with ethyl acetate (3 x 20 mL). The combined organic layers were dried by anhydrous Na₂SO₄, and evaporated to obtain a crude product, which was purified by prep-HPLC to afford Compound (**412**) (20.6 mg, 20%) as a white solid. LC-MS (ESI) m/z: 382 (M+H)⁺; ¹H-NMR (400 MHz, CDCl₃) δ (ppm) 0.09-0.13 (m, 1H), 0.61-0.63 (m, 1H), 1.02 (s, 3H), 1.03 (s, 3H), 4.35-4.39 (t, *J =* 6.8 Hz, 1H), 6.05 (s, 1H), 7.34-7.37 (d, *J =* 10.4 Hz, 1H), 8.32 (s, 1H), 8.44 (s, 1H).

### Example 275

### Preparation of Compound (413) and (414)

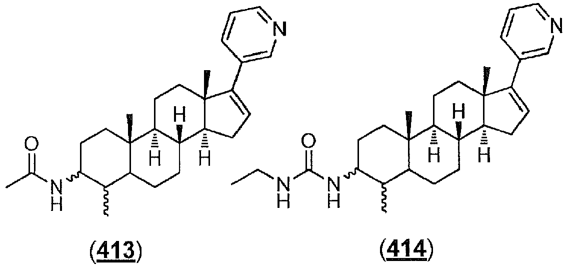

### Example 275A

### Preparation of Compound (413a)

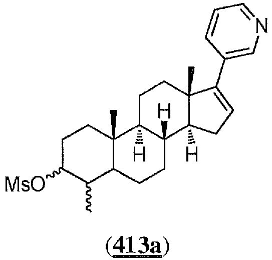

To a solution of Compound (**307**) (1.4 g, 3.83 mmol) and DIPEA (0.99 g, 7.66 mmol) in DCM (28 mL) was added MsCl (0.53 g, 4.6 mmol), and the mixture was stirred at rt for 1 hr. the mixture was then diluted with water, extracted with ethyl acetate (200 mL), washed with water (100 mL) and brine (100 mL), and concentrated to give Compound (**413a**) (2.7 g, crude) as a yellow oil. LC-MS (m/z): 443.9 [M+H]⁺.

### Example 275B

### Preparation of Compound (413b)

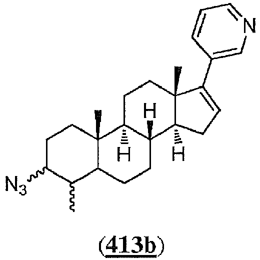

A mixture of Compound (**413a**) (2.7 g, 6.09 mmol), NaN₃ (594 mg, 9.13 mmol) in DMF (50 mL) was stirred at 60 °C overnight It was diluted with ethyl acetate (200 mL), washed with water (100 mL x 3), brine (100 mL), and concentrated to obtain Compound (**413b**) (2.3 g, crude) as a yellow oil. LC-MS (m/z): 365.3 [M+H]⁺.

### Example 275C

### Preparation of Compound (413c)

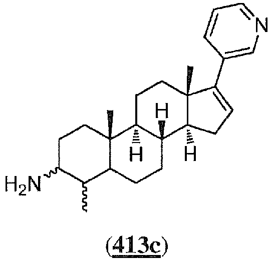

A mixture of Compound (**413b**) (2.3 g, 5.89 mmol), PPh₃ (7.7 g, 29.45 mmol) in MeOH (50 mL) and THF (30 mL) was stirred at rt for 5 hrs. It was diluted with water (200 mL), adjusted pH = 2 with HCl, washed with ethyl acetate (100 mL), then adjusted pH >10 with NaOH solution, extracted with DCM. The DCM layer was dried over Na₂SO₄, concentrated, and purified by prep-HPLC to give Compound (**413c**) (250 mg, 18%) as a light yellow solid. LC-MS (m/z): 365.0 [M+H]⁺.

### Example 275D

### Preparation of Compound (413)

A mixture of Compound (**413c**) (120 mg, 0.33 mmol) and Ac₂O (2 mL) was stirred at rt for 3 hrs. The reaction was diluted with ethyl acetate (100 mL), washed with water (100 mL), saturated NaHCO₃ (50 mL) solution and brine (100 mL), concentrated, and purified by prep-HPLC to obtain Compound (**413**) (57 mg, 43%) as a white solid. LC-MS (m/z): 407.3 [M+H]⁺; ¹H NMR (CDCl₃, 400 MHz) major characteristic peaks: *δ* (ppm) 0.83-0.90 (m, 6H), 1.00-1.03 (m, 3H), 1.98-2.01 (m, 3H), 3.45-3.57 (m, 0.3H), 4.09-4.17 (m, 0.7H), 5.19-5.28 (m, 0.3 H), 5.58-5.60 (m, 0.7H), 5.97-5.99 (m, 1H), 7.20-7.24 (m, 1H), 7.63-7.65 (m, 1H), 8.45-8.46 (m, 1H), 8.61-8.62 (m, 1H).

### Example 275E

### Preparation of Compound (414)

A mixture of Compound (**413c**) (120 mg, 0.33 mmol) and isocyanatoethane (28 mg, 0.395 mmol) in THF (2 mL) was stirred at rt overnight. The reaction was diluted with ethyl acetate (100 mL), washed with water (100 mL) and brine (100 mL), concentrated, and purified by prep-HPLC to afford Compound (**414**) (68 mg, 47%) as a white solid. LC-MS (m/z): 436.4 [M+H]⁺; ¹H NMR (CDCl₃, 400 MHz) major characteristic peaks: *δ* (ppm) 0.85-0.95 (m, 6H), 1.00-1.01 (m, 3H), 1.12-1.16 (m, 3H), 3.18-3.24 (m, 2H), 3.82-4.52 (m, 2H), 5.98-5.99 (m, 1H), 7.22-7.25 (m, 1H), 7.65-7.67 (m, 1H), 8.45-8.46 (m, 1H), 8.61-8.62 (m, 1H).

### Example 276

### Preparation of Compound (415)

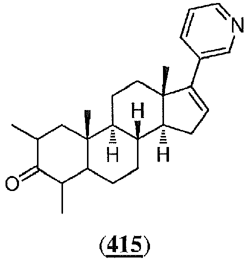

### Example 276A

### Preparation of Compound (415a)

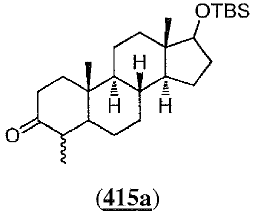

To a solution of Compound (**303b**) (1.5 g, 4.93 mmol) in THF (100 mL) was added imidazole (1.01 g, 14.79 mmol), TBSCl (1.11 g, 7.40 mmol). The mixture was stirred at room temperature for 12 hrs. It was diluted with NH₄Cl aq. (20 mL), extracted with CH₂Cl₂ (3 x 30 mL). The organic phase was washed with brine (2 x 20 mL), dried over Na₂SO₄, concentrated, and purified by column chromatography (petroleum ether, then petroleum ether/ ethyl acetate =100/1, v/v) to give Compound (**415a**') (1.89 g, 89%) as a mixture of isomers.

To a solution of NaOEt in EtOH was added Compound (**5a'**) (1.2 g, 2.87 mmol) in EtOH (20 mL). It was stirred for 2 hrs at 85 °C before cooled to room temperature. It was diluted with aq. NH₄Cl, extracted with EtOAc (3 x 30 mL). The organic layer phase was washed with brine (2 x 20 mL), dried over Na₂SO₄, concentrated, and purified by column chromatography (petroleum ether, then petroleum ether:ethyl acetate = 75/1, v/v) to give Compound (**5a**) (1.2 g, 99%) as a white solid LCMS: m/z: 419,3 (M+1)⁺; ¹H-NMR (400 MHz, CDCl₃): *δ* (ppm) 0.007 (s, 6H), 0.870 (s, 9H), 0.911 (s, 3H), 1.114 (d, *J* = 8.0Hz, 3H), 1.252 (s, 3H), 2.610 (m, 1H), 3.533 (t, *J=* 8.0Hz, 1H).

### Example 276B

### Preparation of Compound (415b)

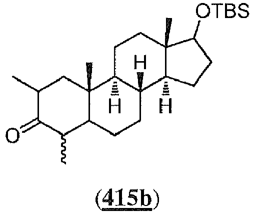

A solution of (*i*-Pr)₂NH (0.28 mL, 2.0 mmol) in dry THF was stirred under argon at 0°C and a 2.5M solution of n-BuLi in hexanes(0.8 mL, 2.0 mmol) was added dropwise. After 30 min, Compound (**415a**) (210 mg, 0.5 mmol) dissolved in dry THF was added dropwise in the resulting lithium diisopropylamine solution. This mixture was allowed to stir for 1 hr at -78 °C, and then methyl iodide (93 µL, 1.5 mmol) was added dropwise. The reaction mixture was slowly heated to room temperature and stirred for 12 hrs and quenched with water. The mixture was extracted with EtOAc (3 x 30 mL) and organic phase was washed with brine (2 x 20 mL), dried over Na₂SO₄, and evaporated under reduced pressure. Purification by column chromatography (petroleum ether, then petroleum ether/ethyl acetate = 100/1, v/v) afforded Compound (**415b**) (91 mg, 42%) as a white solid. LC-MS: m/z: 432.3 (M+1)⁺. ¹H-NMR (400 MHz, CDCl₃): *δ* (ppm) 0.007 (s, 6H), 0.723 (m, 3H), 0.871(m, 9H), 0.961 (m, 6H), 1.252 (s, 3H), 2.238-2.505 (m, 2H), 3.552 (m, 1H).

### Example 276C

### Preparation of Compound (415c)

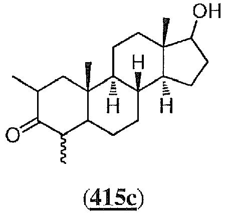

To a stirred solution of Compound (**415b**) (58 mg, 0.13 mmol) in CH₂Cl₂ (5 mL) was added TFA (50 µL, then stirred for 4 h and quenched with sat. aq. NaHCO₃ until pH7∼8. The misture was extracted with EtOAc. (3 x 5 mL) and organic phase was washed with brine (2 x 4 mL), dried over Na₂SO₄ and evaporated under reduced pressure. Purification by column chromatography (petroleum ether/ethyl acetate = 10/1-8/1, v/v) gave Compound (**415c**) (20 mg, 61%) as a white solid. LC-MS:m/z:319.3(M+1)⁺. ¹H-NMR (400 MHz, CDCl₃): *δ* (ppm) 0.690 (s, 3H), 0.879-0.895 (d, *J =* 6.4 Hz, 3H), 0.917-0.933 (d, *J* = 6.4 Hz, 3H), 1.062 (s, 3H), 1.957-2.025 (m, 2H), 2.242-2.319 (m, 1H), 2.317-2.423 (m, 1H), 3.542-3.585 (m, 1H).

### Example 276D

### Preparation of Compound (415d)

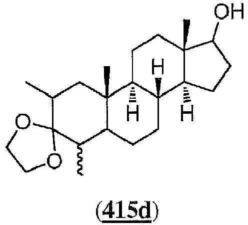

To a solution of Compound (**415c**) (540 mg, 1.7 mmol) in THF (10 mL) was added CH(OEt)₃ (755 mg, 5.1 mmol), ethylene glycol (10 mL), *p*-TsOH (33 mg, 0.16 mmol). Then the mixture was stirred for 7 hrs at 80 °C and quenched with Et₃N (1 mL). The mixture was diluted with EtOAc (15 mL) and washed with water (2 x 10 mL), sat. aq. NaHCO₃ (2 x 10 mL), brine (10 mL), dried over Na₂SO₄, and concentrated to afford Compound **(415d)** (523 mg, 85%) as a white solid. LCMS: m/z: 463.3(M+1)⁺. ¹H-NMR (400 MHz, CDCl₃) : *δ* (ppm) 0.725 (s, 3H), 0.776-0.793 (d, *J* = 6.8 Hz, 3H), 0.826-0.842 (d, *J* = 6.4 Hz, 3H), 1.990-2.104 (m, 1H), 3.600-3.643 (t, *J*= 8.8 Hz, 1H), 3.989-4.069(m, 4H).

### Example 276E

### Preparation of Compound (415e)

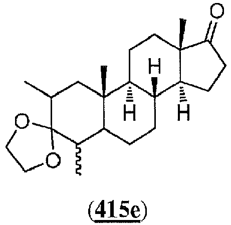

To a solution of Compound (**415d**) (1.5 g, 4.14 mmol) in CH₂Cl₂ (40 mL) was added DMP (2.6 g, 6.21 mmol) in room temperature, then stirred for 1 hr. It was filtered through celite, and concentrated to yield a crude product (**415e**) (1.5 g) as a white solid, which was used in next step without further purification. LCMS: m/z:361.3(M+1)⁺.

### Example 276F

### Preparation of Compound (415f)

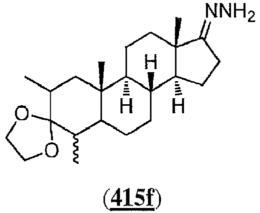

To a solution of Compound (**415e**) (1.5 g, 4.2mmol) in EtOH (42 mL) was added NH₂NH₂·H₂O (395 mg, 6.3 mmol). Stirred for 1 hr at 80°C, it was evaporated under reduced pressure, to remove solvents. It was diluted with NH₄Cl aq. (20 mL), extracted with EtOAc (3 x 30 mL). The organic phase was washed by brine, dried over NaSO₄, and concentrated to give a crude product **(415f)** (1.5 g) as a white solid, which was used in next step whithout further purification. LCMS: m/z: 475.3(M+1)⁺.

### Example 276G

### Preparation of Compound (415g)

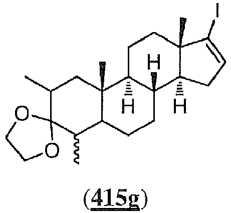

To a stirred of Compound (**415f**) (2.0 g, 5.9 mmol) in THF (25 mL) and CH₂Cl₂ (25 mL) was added Et₃N (1.2 g, 11.8 mmol) at 0 °C, then added the solution of I₂ (2.25 g, 8.87 mmol) in THF dropwise at 0°C, then stirred at same temperature for 1 hr. Na₂S₂O₃ aq. (30 mL) was added to quench the reaction and the crude product was extracted with DCM (3 x 30 mL). The organic phase was washed with brine (15 mL), dried over Na₂SO₄, concentrated, and purified by column chromatogrpahy (petroleum ether/ ethyl acetate = 150/1, v/v) to give Compound (**415g**) (836 mg, 43% yield for 3 steps) as a white solid. ¹H-NMR (400 MHz, CDCl₃) : *δ* 0.719-0.736 (d, *J* = 6.8Hz, 3H), 0.761-0.777 (d, *J* = 6.4 Hz, 3H), 1.041 (s, 3H), 1.302 (s, 3H), 2.125-2.189 (m, 2H), 3.916-4.005 (m, 4H), 6.41 (m,1H).

### Example 276H

### Preparation of Compound (415h)

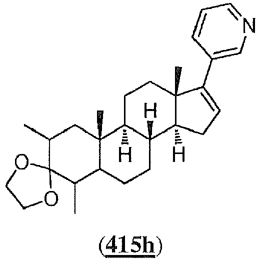

To a solution of Compound (**415g**) (1.5 g, 3.2 mmol) in 1,4-dioxane (28 mL) and H₂O (7 mL) was added 3-pyridineboronic acid (588 mg, 4.78 mmol), K₂CO₃(1.7 g,12.8 mmol) and Pd(PPh₃)₂Cl₂ (224 mg, 0.32 mmol) at N₂ atmosphere. Stirred at 80°C for 2-3 hrs, NH₄Cl aq. (15 mL) was added. It was extracted with EtOAc (3 x 30 mL), washed with brine (15 mL), dried over Na₂SO₄, and concentrated. The crude product was purified by column chromatography (petroleum ether/ethyl acetate = 30/1-15/1, v/v) to give a part of Compound (**415h**) (538 mg, 42%, contained isomers) as a white solid and pure Compound (**415h**) (86 mg) as a white solid. LCMS: m/z: 422.3(M+1)⁺.
¹H-NMR (400 MHz, CDCl₃) : *δ* (ppm) 0.719-0.736 (d, *J* = 6.8Hz, 3H), 0.761-0.777 (d, *J* = 6.4Hz, 3H), 0.822 (s, 3H), 0.923 (s, 3H), 2.125-2.189 (m, 2H), 3.916-4.005 (m, 4H), 5.893 (m,1H), 7.108-7.141 (m, 1H), 7.548-7.572 (m, 1H), 8.360-8.375 (m, 1H), 8.534 (m, 1H).

### Example 276I

### Preparation of Compound (415)

To a solution of Compound (**415h**) (86 mg, 0.2 mmol) in THF (2 mL) was added concentrated HCl (0.2 mL) and it was stirred at room temperature for 5 hrs before quenched with sat. aq. NaHCO₃ (5 mL). The mixture was extracted with EtOAc (3 x 8 mL), dried over Na₂SO₄, concentrated, and purified by column chromatography (petroleum ether/ethyl acetate = 30/1-15/1, v/v) to afford Compound **(415)** (64 mg, 85%) as a white solid. LC-MS: m/z: 378.3(M+I)⁺. ¹H-NMR (400 MHz, CDCl₃) : *δ* (ppm) 0.965-0.981 (d, *J* = 6.4 Hz, 3H), 1.011-0.996 (d, *J* = 6.0 Hz, 3H), 1.029 (s, 3H), 1.174 (s, 3H), 5.581 (m, 1H), 7.229 (m, 1H), 7.753 (m, 1H), 8.463 (s, 1H), 8.615 (s, 1H).

### Example 277

### Preparation of Compound (416)

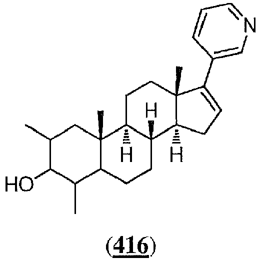

To a solution of Compound **(415)** (483 mg, 1.28 mmol, crude) in MeOH (22 mL) was added NaBH₄ (73 mg, 1.92 mmol) at room temperature and stirred for 1 hr. The mixture was quenched with sat. aq. NH₄Cl (10 mL) and extracted with EtOAc (3 x 8 mL). The organic phase was washed with brine, dried over Na₂SO₄, concentrated, and purified by prep-TLC (petroleum ether/ethyl acetate = 5/1, v/v) to give Compound **(416)** (20 mg) as a white solid. LCMS: m/z: 380.3(M+1)⁺. ¹H-NMR (400 MHz, CDCl₃) : *δ* 0.906-0.923 (d, *J*= 6.8 Hz, 3H), 0.961-0.979 (d, *J* = 7.2 Hz, 3H), 0.996 (s, 3H), 1.003 (s, 3H), 3.552 (s, 1H), 5.568 (m, 1H), 7.216 (m, 1H), 7.646 (m, 1H), 8.444 (m, 1H), 8.602 (m, 1H).

### Example 278

### Preparation of Compounds (417) and (418)

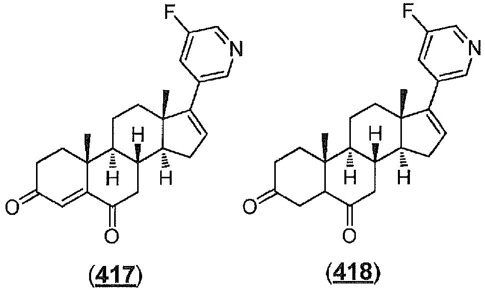

### Example 278A

### Preparation of Compound (417a)

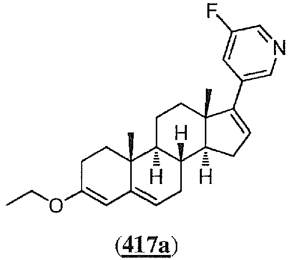

To a solution of compound **(312a)** (3.0 g, 7.07 mmol) in 1,4-dioxane (30 mL) was added 5-fluoropyridin-3-ylboronic acid (1.50 g, 10.6 mmol), potassium carbonate (4.39 g, 31.8 mmol) in water (6 mL), and Pd(dppf)Cl₂ (518 mg). The mixture was thoroughly degassed, and heated under nitrogen and heated to reflux for 2 hrs. The mixture was extracted with ethyl acetate (100 mL x 3). The organic layers were combined, washed with water, brine, and dried over Na₂SO₄. The crude product was purified by column chromatography (ethyl acetate in petroleum, 20% v/v) to yield Compound (**417a**) (1.62 g, 58%) as a yellow solid. LC-MS (m/z): 394 [M+H]^{+ 1}H NMR (DMSO-*d*₆, 400 MHz) major characteristic peaks: *δ* (ppm) 0.96 (s, 3H), 1.04 (s, 3H), 1.22 (t, *J* = 7.2 Hz, 3H), 3.73 (m, 2H), 5.14 (s, 1H), 5.18 (m, 1H), 6.27 (m, 1H), 7.68 (td, *J* = 2.0 Hz, 10.4 Hz, 1H), 8.45 (d, *J* = 2.0 Hz, 1H), 8.50 (s, 1H).

### Example 278B

### Preparation of Compound (417b)

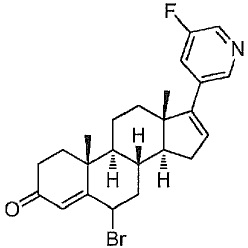

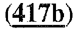

To a suspension of compound (**417a**) (1.62 g, 4.12 mmol) and NaOAc (338 mg, 4.12 mmol) in 30 mL of 96% aq. acetone, NBS (954 mg, 5.36 mmol) was slowly added. After stirring for 30 min at 30 °C, the solution was concentrated under reduced pressure. The residue was extracted with CH₂Cl₂ (100 mL x 3), washed with water, brine, and dried over Na₂SO₄. The crude product was purified with silica gel column (ethyl acetate/petroleum = 1/3, v/v) to yield Compound (**417b**) (853 mg, 46%) as a white solid. LC-MS (m/z): 444 [M+H]⁺, 446 [M+2+H]^{+ 1}H NMR (DMSO-*d*₆, 400 MHz) major characteristic peaks: *δ* (ppm) 1.10 (s, 3H), 1.52 (s, 3H), 5.41(m, 1H), 5.98 (s, 1H), 6.28 (m, 1H), 7.69 (td, *J* = 2.0 Hz, 10.0 Hz, 1H), 8.47 (d, *J* = 2.0 Hz, 1H), 8.51 (s, 1H).

### Example 278C

### Preparation of Compound (417c)

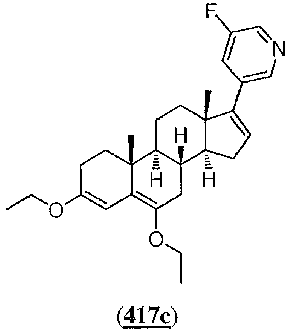

To a suspension of compound (**417b**) (850 mg, 1.92 mmol) in 5 mL of ethanol was added triethyl orthoformate (1 mL) and sulfosalicylic acid (12 mg) under N_{2.} After stirred at 40 °C for 30 min, Et₃N was added to adjust pH to 8. The mixture was extracted with CH₂Cl₂ (100 mL x 3), washed with water, brine, and dried over Na₂SO₄. The crude product was purified with neutralized Al₂O₃ column (ethyl acetate in petroleum, 10% v/v) to give Compound (**417c**) (540 mg, 64%) as a yellow solid. LC-MS (m/z): 438 [M+H]⁺ ; ¹H NMR (CDCl₃, 400 MHz) major characteristic peaks: *δ* (ppm)1.01 (s, 3H), 1.07 (s, 3H), 1.27 (t, *J* = 6.8 Hz, 3H), 1.33 (t, *J* = 6.8 Hz, 3H), 3.79 (m, 2H), 3.84 (m, 2H), 5.65 (s, 1H), 6.08 (m, 1H), 7.38 (td, J = 2.4 Hz, 12 Hz, 1H), 8.34 (d, *J =* 2.4 Hz, 1H), 8.45 (s, 1H).

### Example 278D

### Preparation of Compounds (417) and (418)

To a suspension of compound (**417c**) (200 mg, 0.46 mmol) in THF (10 mL) was added con. HCl (0.5 mL) under N₂. After stirred at room temperature for 30 min, sat. aq. NaHCO₃ was added to adjust pH to 8. The mixture was extracted with ethyl acetate (50 mL x 3), washed with water, brine, and dried over Na₂SO₄. The crude product was purified by prep-TLC (ethyl acetate/petroleum = 1/3, v/v) to afford Compound **(417)** (9 mg, 5%) and (**418**) (12 mg, 7%).

Compound (**417**): LC-MS (m/z): 380 [M+H]^{+ 1}H NMR (CDCl₃, 400 MHz) major characteristic peaks: *δ* (ppm) 1.09 (s, 3H), 1.24 (s, 3H), 6.08 (m, 1H), 6.22 (s, 1H), 7.37 (td, *J* = 2.0 Hz, 10.0 Hz, 1H), 8.36 (d, *J*= 2.0 Hz, 1H), 8.45 (s, 1H).

Compound (**418**): LC-MS (m/z): 382[M+H]^{+ 1}H NMR (CDCl₃, 400 MHz) major characteristic peaks: *δ* (ppm) 1.03 (s, 3H), 1.05 (s, 3H), 6.07 (m, 1H), 7.37 (td, *J* = 2.0 Hz, 9.6 Hz, 1H), 8.36 (d, *J* = 2.0 Hz, 1H), 8.45 (s, 1H).

### Example 279

### Preparation of Compound (419)

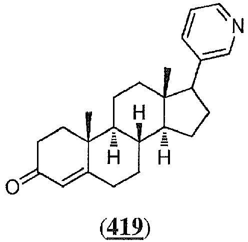

To a mixture of Compound (**50**) (90 mg, 0.26 mmol) in methanol (5 mL) was added Pd/C (9 mg) and the mixture was stirred at room temperature for 8 hrs under H₂. The mixture was filtered and the filtrate was washed with methanol (10 mL). It was concentrated and purified by prep-TLC (ethyl acetate in petroleum ether, 50% v/v) to give Compound (**419**) (40 mg, 44%) as a white oil. LC-MS (ESI) m/z: 350 (M+1)⁺; ¹H-NMR (400 MHz, CDCl₃) δ (ppm): 0.480 (s, 3H), 1.115 (s, 3H), 2.772-2.819 (t, *J* = 9.6 Hz, 1H), 5.686 (s, 1H), 7.738 (s, 1H), 8.086-8.104 (d, *J* = 7.2 Hz, 1H), 8.667 (s, 2H ).

### Example 280

### Preparation of Compound (420)

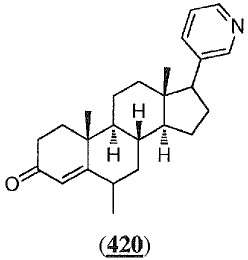

### Example 280A

### Preparation of Compound (420a)

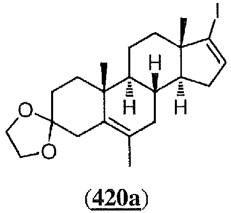

To a solution of Compound (**309e**) (1.9 g, 5.5 mmol) in EtOH (10 mL) was added N₂H₄ H₂O (2.2 g, 55 mmol), then the mixture was stirred at 50 °C for 2 hrs. The mixture was cooled to room temperature, evaporated to remove solvents. To the mixture water was added to precipitate crude intermediate. After filtration, the solid was dried and used for next step without further purification.

To a mixture of above intermediate (1.9 g, 5.3 mmol) and Et₃N (803 mg, 7.9 mmol) in DCM (10 mL) was added I₂ (1.6 g, 6.4 mmol) in THF (2 mL) dropwise at 0 °C, then the mixture was stirred at room temperature for 2 hrs, then added sat.Na₂S₂O₃, extracted with DCM (2 x 50 mL), washed with brine (50 mL), and dried over Na₂SO₄. The crude product was purified by column chromatography (petroleum ether/ethyl acetate = 10/1, v/v) to obtain Compound (**420a**) (1.13 g, 47%) as a green solid.

### Example 280B

### Preparation of Compound (420b)

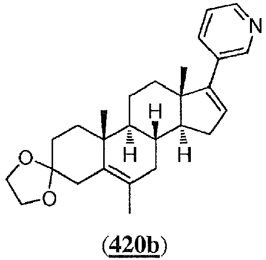

A mixture of Compound (**420a**) (1 g, 2.2 mmol), pyridin-3-ylboronic acid (542 mg, 4.4 mmol), K₂CO₃ (1.5 g, 11 mmol) in H₂O (2 mL), Pd(PPh₃)₂Cl₂ (154 mg, 0.22 mmol) in dioxane (10 mL) was stirred at 80 °C for 2 hrs under N₂, then cooled to room temperature. The mixture was diluted with water, extracted with DCM (2 x 50 mL), washed with brine (50 mL), dried over Na₂SO₄ and the crude product was purified by column chromatography (petroleum ether/ethyl acetate = 5/1, v/v) to give Compound (**420b**) (400 mg, 45%) as a white solid. LC-MS: m/z: 406 (M+1). ¹H NMR (CDCl3, 400 NHz): *δ* (ppm) 1.04 (s, 3H), 1.07 (s, 3H), 1.66 (s, 3H), 3.97(m, 4H), 6.01 (m, 1H), 7.23 (m, 1H), 7.67 (m, 1H), 8.45 (m, 1H), 8.62 (d, *J*= 1.6 Hz, 1H).

### Example 280C

### Preparation of Compound (420c)

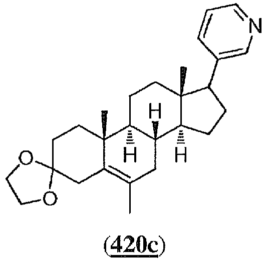

To a solution of Compound (**420b**) (400 mg, 0.96 mmol) in MeOH (10 mL) was added Pd/C (100 mg) under H₂, then the mixture was stirred at room temperature overnight. The mixture was filtered and the filtrate was evaporated to give Compound (**420c**) (360 mg, 90%) as a white solid. LC-MS (m/z): 408 (M+1). ¹H NMR (CDCl₃, 400 MHz) peaks: *δ* (ppm) 0.49 (s, 3H), 1.00 (s, 3H), 1.66 (s, 3H), 2.68 (m, 2H), 3.97 (m, 4H), 7.21 (m, 1H), 7.52 (m, 1H), 8.44 (m, 2H).

### Example 280D

### Preparation of Compound (420)

To a solution of Compound (**420c**) (200 mg, 0.49 mmol) in THF (5 mL) was added con.HCl (3 drops) at 0°C, then the mixture was stirred at room temperature for 1 hr. The mixture was then diluted with ethyl acetate (20 mL), neutralized with sat.NaHCO₃, then extracted with ethyl acetate (2 x 50 mL), washed with brine (50 mL), dried over Na₂SO₄. The organic layer was evaporated and the residue was purified by prep-TLC (DCM / MeOH = 20/1, v/v) to give Compound (**420**) (120 mg, 67 %) as a white solid. LC-MS (m/z): 364 (M+1). ¹H NMR (CDCl3, 400 MHz): *δ* (ppm) 0.53 (s, 3H), 1.10 (d, *J* = 6.4 Hz, 3H), 1.18 (s, 3H), 3.67 (t, *J* = 9.6 Hz, 1H), 5.81 (s, 1H), 7.24 (m, 1H), 7.53 (d, *J*= 8.0 Hz, 1H), 8.46 (s, 2H).

### Example 281

### Preparation of Compound (421)

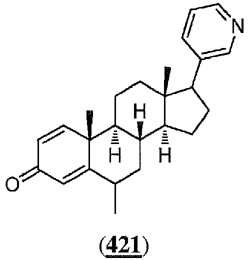

The mixture of Compound (**318**) (200 mg, 0.46 mmol), Pd(AcO)₂ (103 mg, 0.46 mmol) in DCM (9 mL) and MeCN (3 mL) was stirred at 35 °C for 2 hrs, then cooled to room temperature, filtered , the filtrarte was evaporated and purified by prep-HPLC to give **(421)** (16 mg, 10%) as a white solid. LC-MS: (m/z): 362 (M+1). ¹H NMR (CDCl3, 400 MHz) peaks: *δ* (ppm) 0.56 (s, 3H), 1.14 (d, *J* = 6.0 Hz, 3H), 1.23 (s, 3H), 2.57 (m, 1H), 2.68 (t, *J* = 10.0 Hz, 1H), 6.11 (t, *J* = 2.0 Hz, 1H), 6.25 (dd, *J*₁,₂=10.4, 2.0 Hz, 1H), 7.05 (d, *J* = 10.4 Hz, 1H), 7.24 (m, 1H), 7.54 (m, 1H), 8.46 (m, 2H).

### Example 282

### Preparation of Compound (422)

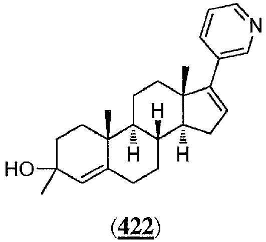

A mixture of Compound (**50c**) (175 mg, 0.5 mmol) in anhydrous THF (4 mL) was cooled to -70 °C and added with a solution of CH₃Li^{·}LiBr in ether (1.5 M, 0.4 mL) slowly and then stirred at room temperature for 1 hr. It was allowed to warm up to 0 °C, quenched by water (1 mL), and extracted with ether acetate (10 mL x 3). The organic phases were separated and washed with brine (5 mL), concentrated ,and purified by prep-HPLC to give Compound (**422**) (15 mg, 8%) as a white solid. LC-MS (ESI) m/z: 364 (M+1)⁺; ¹H-NMR (400 MHz, CDCl₃) *δ* (ppm): 1.050-1.110 (m, 6H), 1.278 (s, 3H), 5.233-5.310 (m, 1H), 5.994 (s, 1H), 7.243-7.258 (m, 1H), 7.651-7.670 (m, 1H), 8.472 (s, 1H), 8.629 (s, 1H).

### Example 283

### Preparation of Compound (423)

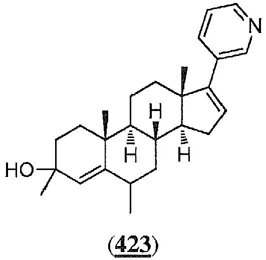

A mixture of Compound (**309**) (180 mg, 0.5 mmol) in anhydrous THF (4 mL) was cooled to -70 °C and added with a solution of CH₃Li·LiBr in ether (1.5 M, 0.4 mL, 0.6 mmol) slowly, then stirred at room temperature for 16 hrs, cooled to 0 °C, and quenched by water (1 mL), extracted by ether acetate (10 mL x 3). The organic phases were separated and washed with brine (5 mL), concentrated, and purified by prep-HPLC to give Compound (**423**) (16 mg, 8%) as a white solid. LC-MS (ESI) m/z: 378 (M+1)⁺; ¹H-NMR (400 MHz, CDCl₃) *δ* (ppm): 1.015 (s, 6H), 1.037-1.092 (m, 2H), 1.255-1.273 (m, 2H), 5.201 (S, 1H), 5.994 (s, 1H), 7.237-7.243 (m, 1H), 7.656-7.676 (m, 1H), 8.459-8.468 (m, 1H), 8.619 (s, 1H).

### Example 284

### Preparation of Compounds (424) and (425)

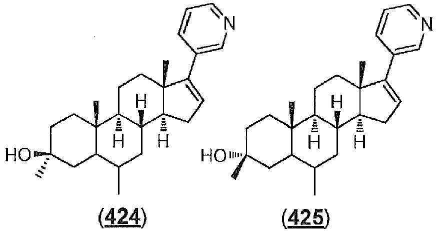

### Example 284A

### Preparation of Compound (424a)

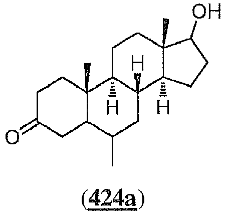

To a solution of Compound (**309c**) (2 g, 6.6 mmol) in EtOH (40 mL) was added Pd/C (10%, 240 mg) and the mixture was stirred under hydrogen (1 atm) at room temperature overnight. The mixture was filtered to remove Pd/C and the filtrate was evaporated to give Compound (**424a**) (1.95 g, 97%) as a white solid. LCMS: m/z: 305(M+1); ¹H NMR (CDC13, 400 MHz): *δ* (ppm) 0.762 (s, 3H), 0.776 (d, *J* = 6.8 Hz, 3H), 1.025 (s, 3H), 3.620 (m, 1H).

### Example 284B

### Preparation of Compound (424b)

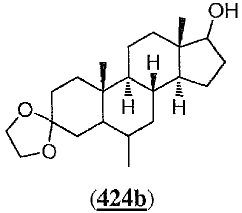

A solution of Compound (**424a**) (250 mg, 0.82 mmol), *p*-TsOH (16 mg, 0.08 mmol), CH(OEt)₃ (364 mg, 2.46 mmol) and ethane-1,2-diol (2 mL) in THF (2 mL) was stirred at room temperature for 1 hr. The solution was diluted with EtOAc (20 mL) and washed with saturated aqueous NaHCO₃ solution (20 mL x 2), water (20 mL x 3) and brine (20 mL), dried over anhydrous Na₂SO₄, and purified by column chromatography (petroleum ether/ethyl acetate = 6/1, v/v) to give Compound (**424b**) (175 mg, 61%) as a light oil. LCMS: m/z: 349 (M+1); ¹H NMR (CDCl3, 400 MHz): *δ* (ppm) 0.722 (s, 2H), 0.732 (s, 1H), 0.788 (d, *J* = 6.4 Hz, 3H), 0.828 (s, 1H), 0.946 (s, 2H), 3.635 (m, 1H), 3.939 (s, 4H).

### Example 284C

### Preparation of Compound (424c)

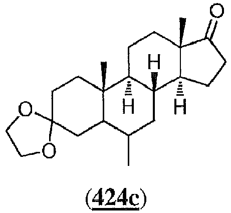

To a solution of Compound (**424b**) (175 mg, 0.5 mmol) in DCM (3 mL), was added DMP (233 mg, 0.55 mmol) at 0 °C. The mixture was stirred at 0 °C for 1 hr and filtered to remove the white solid salt. The filtrate was diluted with DCM (20 mL), washed with water (20 mL x 3), brine (20 mL), dried over anhydrous Na₂SO₄, and concentrated to give Compound (**424c**) (200 mg, crude product) as a yellow oil. LCMS: m/z: 347 (M+1); ¹H NMR (CDCl3, 400 MHz) peaks: *δ* (ppm) 0.775 (s, 1H), 0.781 (s, 3H), 0.894 (s, 2H), 1.187 (s, 3H), 3.871 (s, 4H).

### Example 284D

### Preparation of Compound (424d)

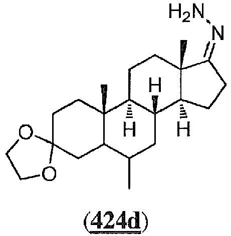

To a solution of Compound (**424c**) (1.7 g, 4.9 mmol) in EtOH (30 mL) was added N₂H₄ • H₂O (80%, 3.06 g, 49 mmol). The mixture was stirred at 80 °C for 1 hr. The mixture was diluted with EtOAc (300 mL), washed with water (200 mL x 3) and brine (200 mL), dried over anhydrous Na₂SO₄, and concentrated to afford Compound (**424d**) (1.53 g, 87%) as a white solid. LCMS: m/z: 361 (M+1).

### Example 284E

### Preparation of Compound (424e)

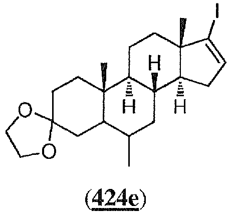

To a solution of Compound (**424d**) (1.53 g, 4.24 mmol) and NEt₃ (0.64 g, 6.37 mmol) in DCM (10 mL) was added dropwise a solution of I₂ (1.3 g, 5.1 mmol) in THF (5 mL) at 0 °C. The mixture was stirred at 0 °C for 1 hr, quenched with saturated aqueous NaHSO₃ solution, extracted with DCM (100 mL x 3). The organic phases were combined, washed with water (100 mL x 3) and brine (100 mL), and purified by column chromatography (petroleum ether/ethyl acetate = 80/1, v/v) to give Compound (**424e**) (1.8 g, 93%) as a yellow oil. LC-MS (m/z): no signal; ¹H NMR (CDC13, 400MHz): *δ* (ppm) 0.807 (d, *J* = 6.4 Hz, 4H), 0.964 (s, 2H), 1.259 (s, 3H), 3.941 (s, 4H), 6.113 (m, 1H).

### Example 284F

### Preparation of Compound (424f)

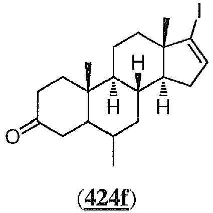

To a solution of Compound (**424e**) (1.8 g, 3.9 mmol) in THF (20 mL) was added HCl (2N, 2 mL) at 0°C. The mixture was stirred at room temperature for 4 hrs, then con. HCl (1 mL) was added in and stirred at room temperature overnight. The mixture was diluted with EtOAc (100 mL), washed with water (100 mL x 3), saturated aqueous NaHCO₃ solution (100 mL), brine (100 mL), dried over anhydrous Na₂SO₄ and purified by column chromatography (petroleum ether/ethyl acetate = 80/1, v/v) to give (**424f**) (620 mg, 39%) as a brown oil. LC-MS (m/z): 413 (M+1); ¹HNMR (CDCl₃, 400MHz): *δ* (ppm) 0.748 (s, 3H), 0.797 (d, *J* = 6.8 Hz, 3H), 1.043 (s, 3H), 6.132 (m, 1H).

### Example 284G

### Preparation of Compound (424g)

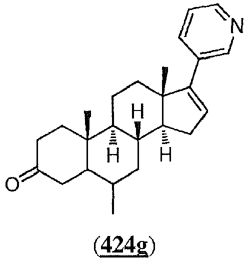

To a solution of Compound (**424f**) (620 mg, 1.5 mmol) in 1,4-dioxane (20 mL) was added a solution of K₂CO₃ (1 g, 7.5 mmol) in water (2 mL), pyridin-3-ylboronic acid (277 mg, 2.25 mmol), Pd(PPh₃)₂Cl₂ (105 mg, 0.15 mmol). The mixture was stirred at 80 °C for 2 hrs under N₂. The mixture was diluted with water (100 mL), extracted with DCM (100 mL x 3). The organic phases were combined, washed with water (100 mL x 3), brine (100 mL), dried over anhydrous Na₂SO₄, and purified by column chromatography (petroleum ether/ethyl acetate = 5/1, v/v) to give Compound (**424g**) (272 mg, 50%) as a yellow oil. LC-MS (m/z): 364 (M+1).

### Example 284H

### Preparation of Compounds (424) and (425)

To a solution of Compound (**424g**) (272 mg, 0.75 mmol) in anhydrous THF (10 mL) was added MeMgBr (3M, 0.75 mL) at 0 °C under N₂. The mixture was stirred at 0 °C for 2 hrs under N₂. The mixture was diluted with saturated aqueous NH₄Cl solution (50 mL), extracted with EtOAc (50 mL x 3). The organic phases were combined and washed with water (50 mL x 3), brine (50 mL), dried over anhydrous Na₂SO₄, and purified by prep-TLC (ethyl acetate/petroleum ether = 1/3, v/v, twice) to give Compounds (**424**) (27 mg, 9%) as a white solid and (**425**) (17 mg, 6%) as a white solid. Compound (**424**) LC-MS (m/z): 380 (M+1); ¹HNMR (CDCl₃, 400MHz): *δ* (ppm) 0.831 (d, *J* = 7.2 Hz, 3H), 0.996 (s, 3H), 1.011 (s, 3H), 1.229 (s, 3H), 5.976 (m, 1H), 7.212 (m, 1H), 7.637 (m, 1H), 8.452 (m, 1H), 8.611 (m, 1H). Compound (**425**) LC-MS (m/z): 380 (M+1); ¹HNMR (CDCl₃, 400MHz): *δ* (ppm) 0.836 (d, *J* = 6.4 Hz, 3H), 0.986 (s, 3H), 0.989 (s, 3H), 1.252 (s, 3H), 5.974 (m, 1H), 7.214 (m, 1H), 7.640 (m, 1H), 8.453 (m, 1H), 8.613(m, 1H).

### Example 285

### Preparation of Compound (426)

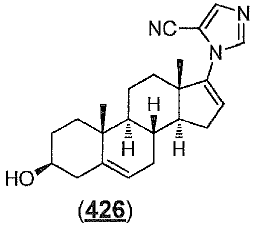

### Example 285A

### Preparation of Compound (426a)

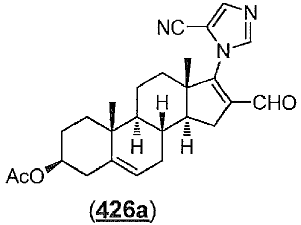

To a solution of Compound (**2**) (2.0 g, 5.3 mmol) in dry DMF (20 mL) was added 1H-imidazole-4-carbonitrile (0.5 g, 8.0 mmol), K₂CO₃ (2.3 g, 16 mmol) under N₂ and stirred at 80 °C for 3 hrs. After cooling to room temperature, the reaction mixture was poured into ice-water (250 mL), and the resulting white precipitate was filtered, washed with water, dried, evaporated to dryness to give Compound (**426a**) (1.2 g, 52%) as a yellow solid. LC-MS (m/z): (M +H)⁺ = 434, ¹H NMR (CDCl₃, 400 MHz) major characteristic peaks: *δ* (ppm) 1.07(s, 6H), 2.04 (s, 3H), 4.60 (m, 1H), 5.41 (m, 1H), 7.62 (s, 1H), 7.64 (s, 1H), 9.60 (s, 1H).

### Example 285B

### Preparation of Compound (426b)

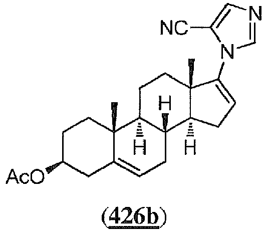

A solution of Compound (**426a**) (0.6 g, 1.4 mmol) in dry benzonitrile (20 mL) was refluxed in the presence of 10% palladium on activated charcoal (0.3 g) for 12 hrs. After cooling to room temperature, the catalyst was removed by filtration through a Celite pad. The filtrate was evaporated to give a crude Compound (**426a**) (0.6 g) as a yellow solid. LCMS: (M +H)⁺ = 405.

### Example 285C

### Preparation of Compound (426)

To a solution of Compound (**426b**) (0.2 g, 0.5 mmol) in methanol (2 mL) and THF (6 mL) was added NaOH (20 mg, 0.5 mmol) and stirred at room temperature for 1 hr. After reaction the mixture was poured into ice-water (100 mL) and extracted with DCM (100 mL x 2). The organic phase was washed with brine (50 mL x 2) and dried over Na₂SO₄, concentrated and purified by prep-TLC to give compound (**426**) (75 mg, 42%) as a white solid. LC-MS (m/z): 364 (M+H)⁺; ¹H NMR (CDCl3, 400 MHz) major characteristic peaks: *δ* (ppm) 1.00 (s, 3H), 1.06 (s, 3H), 3.54 (m, 1H), 5.38 (m, 1H), 5.83 (m, 1H), 7.56 (s, 1H), 7.61 (s, 1H).

### Example 286

### Preparation of Compound (427)

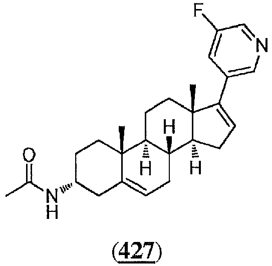

A mixture of compound (**231a**) (420 mg, 1 mmol), 5-fluoropyridin-3-ylboronic acid (282 mg, 2 mmol), Pd(PPh₃)₂Cl₂ (7 mg, 0.1 mmol), K₂CO₃ (690 mg, 5 mmol) in dioxane (6 mL) and H₂O (1 mL) was stirred at 80 °C overnight. Then the mixture was poured into water, and extracted with ethyl acetate (100 mL x 3). The extract was washed with brine, dried, concentrated to yield a crude product. The crude product was purified by prep-TLC (DCM/MeOH = 20/1, v/v) to get **(427)** (90 mg, 22%) as a white solid. LC-MS (ESI) m/z: 409 (M+H)⁺; ¹H-NMR (400 MHz, CDCL3): *δ* (ppm) 1.05 (s, 3H), 1.08 (s, 3H), 1.97 (s, 3H), 4.16 (m, 1H), 5.42 (dd, 1H), 5.50 (dd, 1H), 6.08 (s, 1H), 7.38 (m, 1H), 8.34 (s, 1H), 8.46 (s, 1H).

### Example 287

### Preparation of Compound (428) and (429)

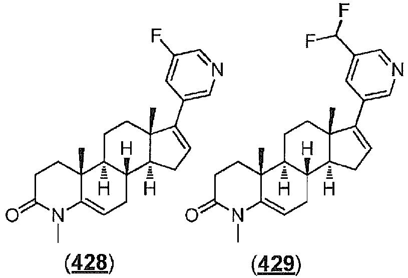

To a solution of Compound (**1**) (300 mg, 0.73 mmol) in 1,4-dioxane (10 mL) was added a solution of K₂CO₃ (504 mg, 3.65 mmol) in water (1 mL), 5-fluoropyridin-3-ylboronic acid (150 mg, 1.1 mmol), Pd(PPh₃)₂Cl₂ (51 mg, 0.073 mmol). The mixture was stirred at 80 °C for 2 hrs under N₂. The mixture was diluted with water (100 mL), extracted with DCM (75 mL x 3). The organic phases were combined and washed with water (100 mL x 3), brine (100 mL), dried over anhydrous Na₂SO₄, and purified by column chromatograp (petroleum ether/ethyl acetate = 3/1, v/v) to afford a crude product. The crude product was washed with cold Et₂O to give (**428**) (50 mg, 18%) as a white solid. LC-MS (m/z): 381 (M+1); ¹HNMR (CDCl₃, 400MHz): *δ* (ppm) 1.006 (s, 3H), 1.046 (s, 3H), 3.076 (s, 3H), 4.998 (m, 1H), 6.102 (m, 1H), 7.294 (m, 1H), 8.269 (d, *J* = 2.8 Hz, 1H), 8.384 (s, 1H).

Using a similar procedure and under the same conditions for the preparation of Compound (**428**), replacing 5-fluoropyridin-3-ylboronic acid with (5-(difluoromethyl)pyridin-3-yl)boronic acid, Compound (**429**) was prepared.
Compound (**429**) (Yield 19%) as a white solid: LC-MS (m/z): 413 (M+1); ¹H NMR (CDCl₃, 400MHz): *δ* (ppm) 1.089 (s, 3H), 1.118 (s, 3H), 3.147 (s, 3H), 5.073 (m, 1H), 6.109 (m, 1H), 6.717 (t, J= 56 Hz, 1H), 7.805 (s, 1H), 8.611 (s, 1H), 8.737 (s, 1H).

### Example 288

### Preparation of Compound (430)

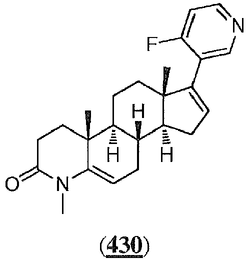

### Example 288A

### Preparation of Compound (430a)

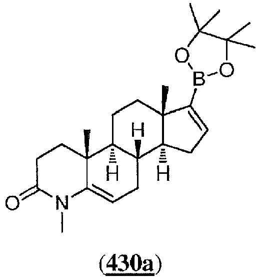

A mixture of Compound (**1**) (2.2 g, 5.3 mmol), 4,4,4',4',5,5,5',5'-octamethyl-2,2'-bi(1,3,2-dioxaborolane) (2.0 g, 8.02 mmol), AcOK (1.57 g, 16 mmol) and Pd(dppf)₂Cl₂ (117 mg, 0.16 mmol) in anhydrous DMSO (50 mL) under N₂ was stirred at 80°C for 2 hrs. After cooled down to room temperature, the mixture was diluted with water (100 mL), extracted with ethyl acetate (50 mL x 2). The organic phase was washed with water (50 mL x 3), btine (50 mL), dried over Na₂SO₄, and purified by column chromatography (petroleum ether/ethyl acetate = 5/1, v/v) to afford Compound (**430a**) (351 mg, 16%) as a white solid. LC-MS (m/z): 412 (M+1)⁺;

### Example 288B

### Preparation of Compound (430)

To a solution of Compound (**430a**) (200 mg, 0.49 mmol) in dioxane (10 mL) was added 3-bromo-4-fluoropyridine (103 mg, 0.58 mmol), potassium carbonate (134 mg, 0.97 mmol) in water (2 mL), and bis(triphenylphosphine) palladium chloride (34 mg). The mixture was thoroughly degassed, and heated under nitrogen at 80 °C for 2 hrs. After being filtered through a pad of Celite, the crude product was purified by prep-TLC (ethyl acetate in petroleum, 50% v/v) to give Compound (**430**) (16 mg, 9%) as a pale yellow solid. LC-MS (m/z): 381 (M+1). ¹H NMR (CDCl3, 400 MHz): *δ* (ppm) 1.03 (s, 3H), 1.11 (s, 3H), 3.15 (s, 3H), 5.08 (m, 1H), 6.04 (m, 1H), 7.04 (m, 1H), 8.47 (m, 2H).

### Example 289

### Preparation of Compounds (431) and (432)

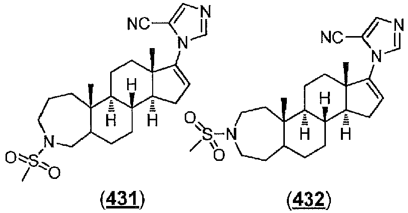

### Example 289A

### Preparation of Compound (431a)

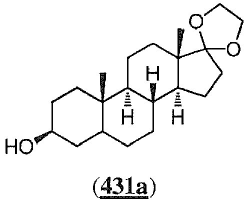

To a solution of (3S,10S,13S)-3-hydroxy-10,13-dimethyltetradecahydro-1H-cyclopenta[a]phenanthren-17(2H)-one (10.0 g, 34.5 mmol) in ethane-1,2-diol (60 mL) and THF (60 mL) was added triethoxymethane (15.3 g, 104 mmol) and p-TsOH (656 mg) under N₂. The mixture was stirred at room temperature for 2 hrs. After the completion of the reaction as indicated by TLC, the mixture was adjusted pH to 8 with saturated aqueous NaHCO_{3.} The solvent was evaporated, and the residue was diluted with water (100 mL). The resulting precipitates was collected by filtration and washed with water, and dried in vacuo to give Compound (**431a**) (11.5 g, 99%) as a white solid. LC-MS (m/z): 317 [M-OH]^{+ 1}H-NMR (400 MHz, CHCl₃-*d*) major characteristic peaks: *δ* (ppm) 0.81 (s, 3H), 0.84 (s, 3H), 3.59 (m, 1H), 3.89 (m, 4H).

### Example 289B

### Preparation of Compound (431b)

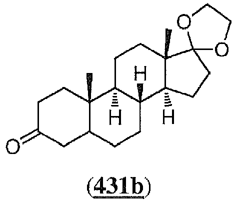

To a solution of Compound (**431a**) (2.0 g, 6.0 mmol) in ethyl acetate (50 mL) was added IBX (3.35 g, 12 mmol) under N₂. The mixture was stirred and heated to reflux overnight. After reaction, the mixture was filtrated. The filtrate was evaporated, and the residue was washed with petroleum ether to give Compound (**431b**) (1.82 g, 93%) as a white solid. LC-MS (m/z): 333 [M+H]⁺; ¹H-NMR (400 MHz, CHCl₃-*d*) major characteristic peaks: *δ* (ppm) 0.85 (s, 3H), 0.90 (s, 3H), 3.89 (m, 4H).

### Example 289C

### Preparation of Compound (431c)

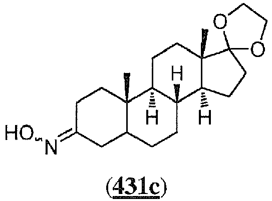

To a solution of Compound (**431b**) (3.0 g, 9 mmol) in ethanol (50 mL) was added pyridine (4 mL) and Hydroxylamine hydrochlororide (1.25 g, 18 mmol) under N₂. The mixture was stirred at room temperature for 1 hr. The mixture was poured into ice-water (100 mL) and stirred 30 min. to give a white precipitate, wich was collected by filtration and dried to yield Compound (**431c**) (2.4 g, 80%) as a white solid. LC-MS (m/z): 348 [M+H]⁺.

### Example 289D

### Preparation of Compounds (431d) and (432d)

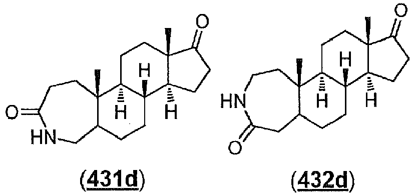

To a solution of Compound (**431c**) (1.0 g, 2.9 mmol) in dry THF (8 mL) was added dropwise a solution of SOCl₂ (3 mL) in THF (2 mL) at 0 °C. The mixture was stirred at room temperature for 30 min. After reaction, the mixture was poured into ice-water (200 mL) and adjusted PH to 8 with NH₃.H₂O. The mixture was extracted with DCM (100 mL x 2), washed with water, brine, and dried over Na₂SO₄. The crude product was purified by column chromatography (ethyl acetate in petroleum, 50% v/v) to give a mixture of (**431d**) and (**432d**) (400 mg, 46% in two steps) as a yellow solid LC-MS (m/z): 302 [M+H]^{+ 1}H-NMR (400 MHz, CHCl₃-*d*) major characteristic peaks: *δ* (ppm) 0.86 (s, 3H), 0.95 (s, 3H), .5.88 (brs, 0.5H), 6.03 (brs, 0.5H).

### Example 289E

### Preparation of Compound (431e) and (432e)

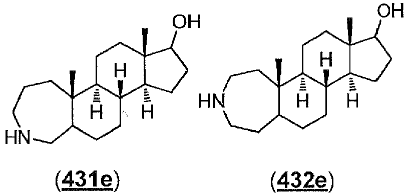

To a solution of mixture (**431d**) and (**432d**) (2.5 g, 8.25 mmol) in dry THF (50 mL) was added LiAlH₄ (1.25 g, 33 mmol) carefully under N₂ at 0 °C. Then the mixture was heated to reflux and stirred overnight. The mixture was quenched with water (1.25 mL), 15% aqueous NaOH solution (1.25 mL), and water (3.75 mL). The mixture was filtered and extracted with ethyl acetate (100 mL x 3), washed with water, brine and dried over Na₂SO₄. Removal of solvents yielded a residue as a yellow solid (1.61 g, 67%), which was used to next step without further purification. LC-MS (m/z): 292 [M+H]^{+ 1}H-NMR (400 MHz, CHCl₃-*d*) major characteristic peaks: *δ* (ppm) 0.74 (s, 3H), 0.83 (s, 3H), 3.63 (t, *J*= 8.4 Hz, 1H).

### Example 289F

### Preparation of Compounds (431f) and (432f)

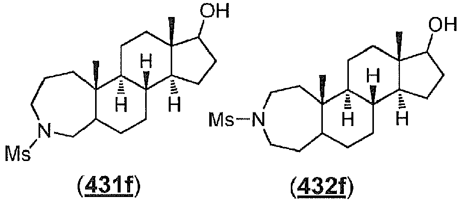

To a solution of Compound (**431e**) and (**432e**) (1.61 g, 5.53 mmol) in DCM (20 mL) was added DIPEA (1 mL), and methanesulfonyl chloride (0.6 mL) under N₂ at 0 °C. The mixture was stirred at 0 °C for 2 hrs. After reaction, the mixture was quenched with aq. NaHCO₃ and extracted with DCM (100 mL x 3), washed with water, brine and dried over Na₂SO₄. Removal of the solvents yielded a mixture of (**431f**) and (**432f**) as a yellow solid (2.03 g, 97%), which was used to next step without purification. LC-MS (m/z): 370 [M+H]⁺.

### Example 289G

### Preparation of Compounds (431g) and (432g)

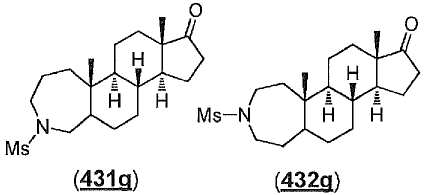

To a solution of Compounds (**431f**) and (**432f**) (2.03 g, 5.5 mmol) in DCM (12 mL) was added DMP (2.8 g, 6.6 mmol) under N₂. The mixture was stirred at room temperature for 3 hrs. After reaction, the mixture was diluted with ethyl acetate (100 mL), and filtrated. The crude product was purified by column chromatography (ethyl acetate in petroleum, 33% v/v) to give a mixture of (**431s**) and (**432g**) (700 mg, 35% in three steps) as a yellow solid. LC-MS (m/z): 368 [M+H]^{+ 1}H-NMR (400 MHz, CHCl₃-*d*) major characteristic peaks: *δ* (ppm)0.77 (s, 3H), 0.79 (s, 3H), 2.73 (s, 3H), 2.93 (m, 1H), 3.26 (m, 3H).

### Example 289H

### Preparation of Compounds (431h) and (432h)

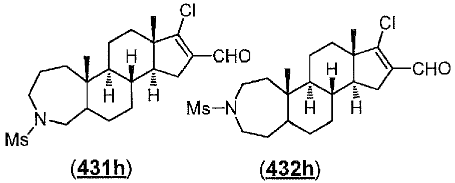

imethylformamide (3 mL, 38 mmol) was added into a cold solution of phosphorus oxychloride (3 mL, 33 mmol) in chloroform (8 mL) in ice/water bath, followed by a solution of (**431g**) and (**432g**) (667 mg, 1.82 mmol) in chloroform (12 mL) added dropwise into the reaction flask. After the addition, the mixture was warmed to 40 °C and stirred under N₂ for 2 hrs. It was poured into ice-water (200 mL), followed by extraction with ethyl acetate (100 mL). The combined extracts were washed with water, brine and dried over Na₂SO₄. The crude product was purified by column chromatography (ethyl acetate in petroleum, 33% v/v) to give a mixture of (**431g**) and (**432g**) (364 mg, 48%) as a white solid. LC-MS (m/z): 414[M+H]⁺.

### Example 289I

### Preparation of Compounds (431i) and (432i)

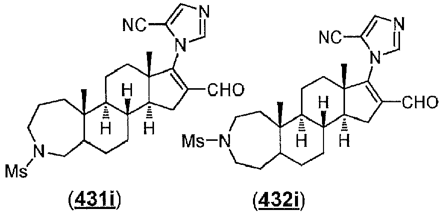

To a solution of Compounds (**431h**) and (**432h**) (357 mg, 0.86 mmol) in dry DMF (16 mL) were added K₂CO₃ (358 mg, 2.59 mmol) and 1H-imidazole-5-carbonitrile (121 mg, 1.3 mmol) under N₂. The mixture was stirred and heated to 40 °C for 3 hrs. The mixture was cooled to room temperature, and poured into 200 mL of water.The mixture was extracted with DCM (50 mL x 3), washed with water, brine and dried over Na₂SO₄. The crude product was purified by column chromatography (methanol in dichloromethane, 1% v/v) to give a mixture of (**431i**) and (**432i**) (410 mg, 99%) as a white solid. LC-MS (m/z): 471 [M+H]⁺; ¹H-NMR (400 MHz, CHCl₃-*d*) major characteristic peaks: *δ* (ppm) 0.80 (s, 1.5H), 0.81 (s, 1.5H), 0.96 (s, 3H), 2.73 (s, 3H), 7.57 (s, 2H), 9.62 (s, 1H).

### Example 289J

### Preparation of Compounds (431) and (432)

To a solution of Compounds (**431i**) and (**432i**) (400 mg, 0.85 mmol) in dry PhCN (12 mL) was added 10 % Pd/C (200 mg) under N₂. The mixture was stirred and heated to reflux for 5 hrs. After cooling to room temperature, the catalyst was removed by filtration through a Celite pad. The filtrate was evaporated, and the residue was purified by prep-HPLC to afford Compound (**431**) (70 mg, 21%) as a white solid and (**432**) (49 mg, 15%) as a white solid.

Compound (**431**): LC-MS (m/z): 443 [M+H]⁺; ¹H-NMR (400 MHz, CHCl₃-*d*) major characteristic peaks: *δ* (ppm) 0.87 (s, 3H), 0.96 (s, 3H), 2.80 (s, 3H), 3.01 (d, *J*= 14.0 Hz, 1H), 3.25 (d, *J*= 14.0 Hz, 1H), 3.35 (m, 2H), 5.82 (m, 1H), 7.55 (s, 1H), 7.61 (s, 1H).

Compound (**432**): LC-MS (m/z): 443 [M+H] ⁺; ¹H-NMR (400 MHz, CHCl₃-*d*) major characteristic peaks: *δ* (ppm) 0.88 (s, 3H), 0.96 (s, 3H), 2.80 (s, 3H), 3.13 (t, *J*= 12.0 Hz, 1H), 3.38 (m, 3H), 3.35 (m, 2H), 5.82 (m, 1H), 7.55 (s, 1H), 7.60 (s, 1H).

### Example 290

### Preparation of Compound (433)

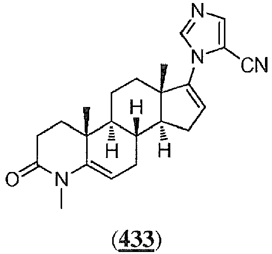

A mixture of compound (**1**) (205 mg, 0.5 mmol), 1H-imidazole-5-carbonitrile (46 mg, 0.5 mmol), L-proline (35 mg, 0.3 mmol), CuI (86 mg, 0.45 mmol), K₂CO₃ (138 mg, 1 mmol) in DMSO (6 mL) was stirred at 130 °C for 2 hrs. the mixture was cooled to room temperature, quenched with water (5 mL), and extracted with CH₂Cl₂ (20 mL x 3). The organic phases were combined and washed with NaHCO₃ solution (20 mL), brine (20 mL), concentrated, and purified by prep-HPLC to give Compound (**433**) (30 mg, 16%) as a yellow solid. LC-MS (ESI) m/z: 377 (M+1)⁺. ¹H-NMR (400 MHz, CDCl₃) *δ* (ppm): 1.041 (s, 3H), 1.121 (s, 3H), 3.154 (s, 3H), 5.069-5.083 (m, 1H), 5.870-5.882 (dd, *J* = 3.2, 1.2 Hz, 1H), 7.274-7.576 (m, 1H), 7.632-7.635 (m, 1H).

### Example 291

### Preparation of Compound (434)

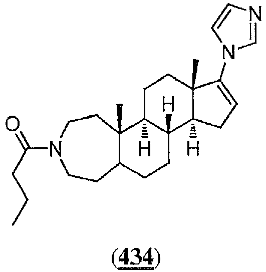

### Example 291A

### Preparation of Compound (434a)

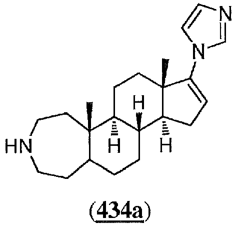

LiAlH₄ (138 mg, 3.6 mmol) was added to a solution of Compound (**135**) (320 mg, 0.9 mmol) in THF (20 mL) at 0 °C , and the mixture was stirred for 1 hr at 50 °C. After being cooled to room temperature, water (1.3 mL) and 15 % aq. NaOH solution (0.3 mL) were added to the reaction mixture. Then the mixture was stirred for 30 mins. It was then filtered and the filtrate was concentrated to give a crude product (**434a**) (360 mg) as an oil, which was used for next step reaction without further purification. LC-MS (m/z): 340(M+1)⁺.

### Example 291B

### Preparation of Compound (434)

To a mixture of Compound (**434a**) (180 mg) and Et₃N (214 mg, 2.12 mmol) in THF (10 mL) was added butyryl chloride (170 mg, 1.59 mmol) at 0 °C. The mixture was stirred at this temperature for 2 hrs. MeOH (1 mL) was added to quench the reaction. It was filtered to give a crude product. The crude product was purified by prep-HPLC to afford Compound (**434**) (28 mg, 13%) as an oil. LC-MS (m/z): 410(M+1)⁺; ¹H NMR (CDCl₃, 400 MHz) major characteristic peaks: *δ* (ppm) 0.83-0.85 (m, 4H), 0.95-0.99 (m, 7H), 3.06-3.91 (m, 4H), 5.66 (m, 1 H), 7.03 (s, 1H), 7.08 (s, 1H), 7.62 (s, 1H).

### Example 292

### Preparation of Compound (435) and (436)

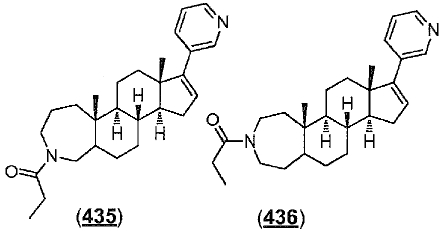

### Example 292A

### Preparation of Compound (435a)

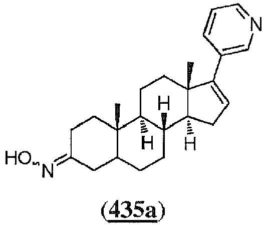

A solution of (10S,13S)-10,13-dimethyl-17-(pyridin-3-yl)-4,5,6,7,8,9,10,11,12,13,14,15-dodecahydro-1H-cyclopenta[a]phenanthren-3(2H)-one (1.1 g, 3.2 mmol), Hydroxylamine hydrochloride 0.26 g, 3.8 mmol) and pyridine(0.37 g, 4.7 mmol) in EtOH (20 mL) was stirred at room temperature overnight. The mixture was poured into ice-water, stirred for 0.5 hr. and then filtered. The filtered cake was washed with H2O and dried to give Compound (**435a**) (1.0 g, 81%) as a white solid. LC-MS: (m/z) 365 [M+1] ⁺.

### Example 292B

### Preparation of Compound (435b) and (436b)

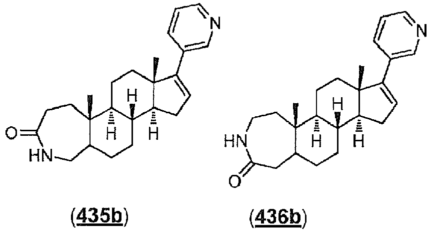

A solution of Compound (**435a**) (1.0 g, 2.7 mmol), SOCl₂ (1 mL) and THF (20 mL) was stirred at room temperature for 1 hr. The mixture was poured into ice-water, then adjusted pH to 10-12 with aq. NaOH (1 N), and extracted with DCM (50mL x 3). The organic phase was washed with brine, dried over Na2SO4, and purified by column chromatography (ethyl acetate in petroleum ether, 50% v/v) to give a mixture of Compounds (**435b**) and (**436b**) (560 mg, 56%) as a yellow solid. LC-MS: (m/z) 365 [M+1]⁺.

### Example 292C

### Preparation of Compounds (435c) and (436c)

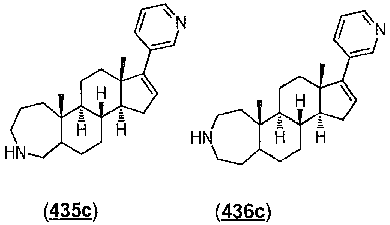

To a solution of Compounds (**435b**) and (**436b**) (0,56 g, 1.5 mmol) in THF (20 mL) was added A1LiH₄ (116 mg, 3.0 mmol) at 0 °C. The mixture was heated to 50°C and stirred for 2 hrs. Then it was cooled to room temperature and ice-water (20 mL) was added slowly to the reaction mixture. It was extracted with DCM (50 mL x 3) and combined organic layers were washed with brine (30 mL x 3), dried over Na₂SO₄, and concentrated to yield a crude product, which was purified by column chromatography (ethyl acetate in petroleum ether, 50% v/v) to afford a mixture of (**435c**) and (**436c**) (220 mg, 41%) as a yellow oil. LC-MS: (m/z) 351 [M+1]⁺.

### Example 292D

### Preparation of Compounds (435) and (436)

To a solution of Compound (**435c**) and (**436c**) (0.22 g, 0.63 mmol) and Et₃N (127 mg, 1.2 mmol) was in DCM (20 mL) was added propionyl chloride (86 mg, 0.94 mmol) at 0 °C. The mixture was stirred at room temperature for 2 hrs and diluted with DCM (40 mL). Then it was washed with water (30 mL x 3), brine (30 mL x 3), dried over Na₂SO₄, and concentrated to yield a crude product, which was purified by column chromatography (ethyl acetate in petroleum ether, 25% v/v, 0.1% NH₄OH) to obtained a mixture of crude product. The mixture was further purified by prep-TLC (ethyl acetate in petroleum ether, 16% v/v, 0.1 % NH₄OH) to afford (**435**) (27 mg) as a white solid and a crude product (**436**), which was subjected further purification using prep-TLC to give a pure form (26 mg) as a white solid.

Compound (**435**): LC-MS: (m/z) 407 [M+1]⁺; ¹H NMR (CDCl₃, 400 MHz) major characteristic peaks: *δ* (ppm) 0.87 (d, *J*= 5.2 Hz, 3H), 1.01 (s, 3H), 1.16 (m, 3H), 2.40 (m, 0.5H), 2.81 (m, 0.5H), 3.18 (t, 1H), 3.33 (m, 0.5 H), 3.41 (t, 1H), 3.66 (m, 0.5H), 3.90 (d, 0.5H), 5.98 (s, 1H), 7.22 (m, 1H), 7.64 (d, *J*= 8.0 Hz, 1H), 8.45 (s, 1 H), 8.61 (s, 1H).

Compound (**436**): LC-MS: (m/z) 407 [M+1]⁺; ¹H NMR (CDCl₃, 400 MHz) major characteristic peaks: *δ* (ppm) 0.78 (d, *J*= 6.4 Hz, 3H), 0.93 (s, 3H), 1.07 (m, 3H), 3.00 (m, 0.5H), 3.20 (m, 0.5H), 3.39 (m, 2H), 3.83 (m, 0.5 H), 5.91 (s, 1H), 7.14 (m, 1 H), 7.56 (d, J= 8.0 Hz, 1H), 8.38 (s, 1 H), 8.54 (s, 1 H).

### Example 293

### Preparation of Compound (437)

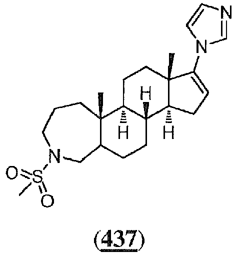

### Example 293A

### Preparation of Compound (437a)

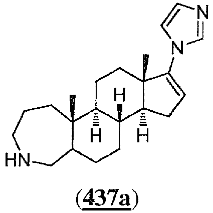

To a suspension of LiAlH₄ (100 mg, 2.56 mmol, 3 eq) in anhydrous THF (8 mL) was added the solution of Compound (**136**) (301 mg, 0.851 mmol) in anhydrous THF (2 mL) dropwise at 0 °C. The mixture was stirred at 60 °C for 2 hrs before water (0.1 mL), 15% aq. NaOH solution (0.1 mL) and water (0.3 mL) was added slowly with stirring. The mixture was then filtered and the filtrate was concentrated to give a crude Compound (**437a**) (300 mg, 100%) as a yellow oil. LC-MS (m/z): 340.3 [M+H]⁺.

### Example 293B

### Preparation of Compound (437)

To a solution of Compound (**437a**) (150 mg, 0.442 mmol) and DIPEA (171 mg, 1.326 mmol) in DCM (3 mL) was added MsCl (101 mg, 0.884 mmol) dropwise at 0 °C. The mixture was stirred at rt for 2 hrs. The mixture was concentrated and the residue was purified by prep-HPLC to afford Compound **(437)** (26 mg, 14%) as a white solid. LC-MS (m/z): 418.0 [M+H]^{+ 1}H NMR (CDCl3, 400 MHz) major characteristic peaks: *δ* (ppm) 0.87 (s, 3H), 0.96 (s, 3H), 2.80 (s, 3H), 3.00-3.06 (m, 1H), 3.23-3.27 (m, 1H), 3.34-3.38 (m, 2H), 5.66-5.68 (m, 1H), 7.04 (s, 1H), 7.08 (s, 1H), 7.62 (s, 1H).

### Example 294

### Preparation of Compound (438)

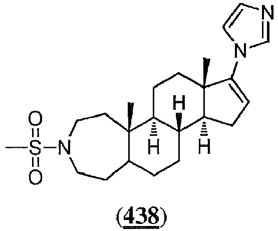

To a solution of Compound (**434a**) (180 mg, 0.53 mmol) and DIPEA (205 mg, 1.59 mmol) in DCM (4 mL) was added MsCl (121 mg, 1.06 mmol) dropwise at 0 °C. The mixture was stirred at room temperature for 2 hrs. The mixture was concentrated and the residue was purified by prep-HPLC to obtain (**438**) (39 mg, 18%) as a white solid. LC-MS (m/z): 418.0 [M+H]^{+ 1}H NMR (CDCl₃, 400 MHz) major characteristic peaks: *δ* (ppm) 0.88 (s, 3H), 0.96 (s, 3H), 2.79 (s, 3H), 3.10-3.17 (m, 1H), 3.30-3.48 (m, 3H), 5.66-5.67 (m, 1H), 7.04 (s, 1H), 7.08 (s, 1H), 7.61 (s, 1H).

### Example 295

### Preparation of Compound (439)

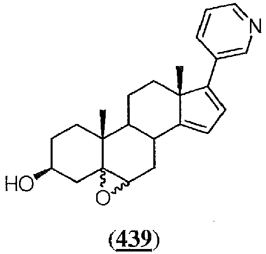

To a solution of Compound (**79**) (347 mg, 1 mmol) in AcOH (5 mL) was added H₂O₂ (4 mL). The mixture was stirred at room temperature for about 4 hrs and diluted with water and subsequently extracted with EtOAc (10 mL x 3). The organic layer was washed with Na₂SO₃ solution, water and brine, and then dried over anhydrous sodium sulfate. After removal of the solvents, the residue was purified with prep-HPLC to afford Compound (**439**) (11 mg) as a gray gum. LC-MS (m/z): 364[M+H]⁺; ¹H NMR (CDCl₃, 400 MHz) major characteristic peaks: *δ* (ppm) 1.05 (s, 3H) 1.14 (s, 3H), 2.97 (s, 1H), 3.66 (m, 1H), 5.98 (s, 1H), 6.80 (s, 1H), 7.28 (m, 1H), 7.86 (m, 1H), 8.21 (m, 1H), 8.56 (s, 1H).

### Example 296

### Preparation of Compound (440)

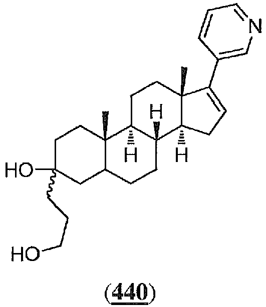

### Example 296A

### Preparation of Compound (440a)

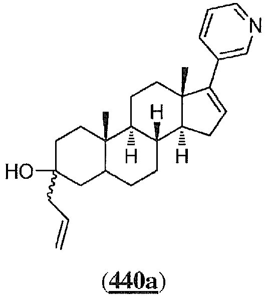

To a solution of (10S,13S)-10,13-dimethyl-17-(pyridin-3-yl)-4,5,6,7,8,9,10,11,12,13,14,15-dodecahydro-1H-cyclopenta[a]phenanthren-3(2H)-one (2 g, 5.7 mmol) in dry THF (50 mL) was added allylmagnesium bromide (1 M, 17.2 mL) at room temperature, and the mixture was stirred at room temperature overnight. The mixture was quenched with sat. NH₄Cl (50 mL) and diluted with ethyl acetate (50 mL). It was extracted with ethyl acetate (2 x 50 mL), the combined organic layers were washed with water, brine, dried, concentrated, and purified by column chromatography (petroleum ether/ethyl acetate=5/1, v/v) to give Compound (440a) (900 mg, 41%) as a white solid. LC-MS (m/z): 392 (M+1). ¹H NMR (CDCl3, 400 MHz): *δ* (ppm) 0.80 (s, 2H), 0.89 (s, 2H), 1.01 (s, 3H), 5.16 (m, 2H), 5.89 (m, 1H), 5.97 (m, 1H), 7.21 (m, 1H), 7.63 (m, 1H), 8.45 (d, *J* = 4.8 Hz, 1H), 8.61(m,1H).

### Example 296B

### Preparation of Compound (440)

To a solution of Compound (**440a**) (330 mg, 0.84 mmol) in dry THF (10 mL) was added 9-BBN (12 mL) at room temperature, then the mixture was heated and stirred at reflux overnight. To this mixture was added NaOH (3 M, 1.36 mL) and H₂O₂ (0.7 mL) at room temperature and then the mixture was stirred at 40 °C for 3 hrs. It was cooled to room temperature, diluted with ethyl acetate (50 mL) and water (100 mL), and extracted with ethyl acetate (2 x 100 mL). The combined organic layers were washed with water, brine, dried, concentrated, and purified by column chromatography (petroleum ether/ethyl acetate = 1/1, v/v) to give Compound (**440**) (120 mg, 35%) as a white solid. LC-MS (m/z): 410 (M+1). ¹H NMR (CDCl3, 400 MHz): *δ* (ppm) 0.80 (s, 1H), 0.89 (s, 2H), 1.00 (s, 3H), 3.68 (m, 2H), 5.98 (m, 1H), 7.21 (m, 1H), 7.63 (m, 1H), 8.44 (m, 1H), 8.61 (d, *J* = 2.0 Hz, 1H).

### Additional compounds

**Compound (441):** 1-((5a*R*,5b*S*,7a*S*,10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)propan-1-one. ¹H-NMR (400 MHz, CDCl₃) δ (ppm) 5.63 (s, 1H), 7.00-7.04 (d, 2H), 7.66 (s, 1H); LC-MS m/z: 395.9(M+1)⁺.

**Compound (442):** (5a*R*,5b*S*,7a*S*,10a*S*,10b*R*,12a*R*)-methyl 8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepine-2(1*H*)-carboxylate. ¹H-NMR (400 MHz, CDCl₃) δ (ppm): 3.69 (s, 3H), 5.69 (s, 1H), 7.04-7.08 (d, 2H), 7.62 (s, 1H); LC-MS m/z: 397.9(M+1)⁺.

**Compound (443):** 1-((5a*R*,5b*S*,7a*S*,10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)ethanone. ¹H-NMR (400 MHz, CDCl₃) δ (ppm): 5.66 (s, 1H), 7.03-7.08 (d, 2H), 7.62 (s, 1H).

**Compound (444):** (9a*R*,11a*S*)-5,9a,11a-trimethyl-1-(4-methylpyridin-3-yl)-3b,4,5,8,9,9a,9b,10,11,11a-decahydro-3*H*-cyclopenta[i]phenanthridin-7(3a*H*)-one. ¹H NMR (CDCl3, 400 MHz) major characteristic peaks: δ (ppm) 0.98 (s, 3 H), 1.30 (s, 3 H), 2.29 (s, 3 H), 2.83 (s, 3 H), 2.96 (t, *J* = 11.6 Hz, 1 H), 3.35 (m, 1 H), 5.09 (s, 1 H), 5.68 (s, 1 H), 7.14 (d, *J* = 4.8 Hz, 1 H), 8.28 (s, 1H), 8.35 (t, *J* = 4.8 Hz, 1 H).

### Biological Studies

### Biological Example 1: Human and murine C_{17,20}-lyase biochemical assays:

Recombinant human C_{17,20}-lyase (hLyase) is expressed in baculovirus-infected Sf9 cells and hLyase enriched microsomes are prepared from cultures as described (Barnes H. J.; Jenlins, C. M.; Waterman, M. R. Archives of Biochemistry and Biophysics 1994, 315(2), 489-494). Recombinant murine C_{17,20}-lyase (mLyase) is prepared in a similar manner. hLyase and mLyase preparations are titrated using assay conditions to determine protein concentrations to be used for assays. Both mLyase and hLyase assays are run in an identical manner except that cytochrome b5 is omitted in the murine assay.

Test compound solutions (20 mM in DMSO) are diluted 1:4 with DMSO and put into the top well of a 96-well mother plate. These solutions are then diluted serially in six steps (1:4 each step) with DMSO to obtain 800 µM to 51.2 nM concentrations on a mother plate (columns 3-12) for subsequent use in the assay. These compound solutions are further diluted twenty-fold in water to obtain a daughter plate containing compound concentrations ranging from 40 µM to 2.56 nM in 5% DMSO. The first 2 columns (of wells) on each 96-well mother plate are used for the DHEA standard curve. DHEA standards are serially diluted (in half-logs) in DMSO to obtain 400 µM to 120 nM standards, then diluted (1:19) in water to obtain 20 µM to 6 nM solutions in 5% DMSO on the daughter plate. These 5% DMSO solutions (5 µL each) from the daughter plate are transferred to the SPA assay plate prior to adding the reaction mixture.

To prepare the reaction mixture, clear-bottomed opaque 96-well assay plates are loaded with 50 µL of assay buffer (50 mM Na₃PO₄, pH 7.5), 5 mL of the diluted compounds (or standards), and 30 mL of substrate solutions (7 mM NADPH, 3.35 µM 17-OH-pregnenolone, 3.35 µg/mL human cytochrome b5 in 50 mM Na₃PO₄). Reactions are initiated with the addition of hLyase or mLyase in assay buffer (10 µL). Enzymatic reactions are incubated at room temperature for 2 hours with gentle agitation. Reactions are terminated with the addition of 5 µL of 1 mM (50 µM final concentration) YM116, a potent C_{17,20}-lyase inhibitor.

The concentration of DHEA generated by hLyase (or mLyase) is determined by radioimmunoassay (RIA). RIA will utilize a ³H-DHEA (0.08 µCi) tracer in 50 µL of scintillation proximity assay (SPA) buffer (100 mM Tris-HCl, pH 7.5, 50 mM NaCl, 0.5% BSA, 0.2% Tween 20) which is added to each well. DHEA antiserum from rabbit (50 µL) with anti-rabbit SPA beads in SPA buffer is added to all wells. Mixtures are allowed to equilibrate with gentle agitation for 1 hour followed by overnight equilibration with no agitation. H-DHEA bound to the SPA beads is determined by scintillation counting with a Wallac microbeta counter. The concentration of DHEA generated is calculated from raw data (CPM) and the standard curve. The concentration of DHEA formed in the presence of test compounds is then expressed as a percent inhibition compared to the DHEA concentration in the absence of test compounds: [1-(nM DHEA formed in the presence of test compound/nM DHEA formed in the absence of test compounds)]x100. Determination of IC₅₀ for each compound will be performed using the Analyze 5 program.

### Biological Example 2: Human C_{17,20}-lyase cellular assay:

Human HEK 293-lyase stable transfectant cells are seeded in a 96-well plate at 10,000 cells/well/100 µL in DMEM plus 10% FBS (supplemented with 1% glutamine, 0.8 mg/mL G418) and allowed to attach overnight. The next day, the media is removed from the cell plate and replaced with 100 µL RPMI without phenol red. Test compounds, DMSO vehicle, or DHEA standards of 5 mL each are added to the cell plate and incubated for 10 min. at room temperature. The reaction is initiated with 10 µL of 5 µM 17-OH-pregnenolone added to all the wells of the cell plate, then incubated for 1 hour at 37 °C. Following the incubation, 90 µL of media (containing DHEA product) is removed from the cell plate and transferred to the SPA assay plate. The subsequent SPA procedure for the detection of DHEA product is performed in the same manner as described for the enzyme assay (see above). The mother plate of test compounds is also prepared in the same manner as the enzyme assay.

Reagents (including catalog #) for the SPA assay are obtained from the following sources: ³H-DHEA: NEN (NET814), Anti-DHEA: Endocrine Sciences (D7-421), Anti-Rabbit SPA Beads: Amersham (RPNQ 0016), 17-OH-pregnenolone: Steraloids (Q4710), NADPH: Sigma (N1630), Cytochrome b5: Panvera (P2252), DHEA (500 µM stock in 100% EtOH), BSA: Sigma (A9647).

### Biological Example 3: Evaluation of a compound having the structure of Formula (I), (II) or (III) as Inhibitors of Testicular Human and Rat 17α-hydroxylase/C_{17,20}-lyase (17α-lyase) in vitro

The potency as inhibitors of P450₁₇α of the compounds described herein are evaluated in human and rat testicular microsomes.

Human testicular microsomes are prepared from human testes (obtained from untreated prostatic cancer patients undergoing orchidectomy), as described in Li et al., The Prostate, 26:140-150 (1995).

Rat testicular microsomes are prepared from the testes of adult Sprague-Dawley rats, as described by Li et al., J. Med. Chem., 39:4335-4339 (1996).

The microsomes are stored at -70 °C until assayed. Just before use, the thawed microsomes are diluted with 0.1 M phosphate buffer (pH 7.4) to appropriate concentrations.

The protein concentration of the microsomes used in the assay are determined by the method of Lowry et al., J. Biol. Chem., 193:265-275 (1951).

The enzyme reaction (activity) is monitored by determination of the release of C³H³COOH from [21-³H³]-17α-hydroxypregnenolone during cleavage of the C-21 side-chain in the conversion to dehydroepiandrosterone (DHEA) as described by Njar et al., Steroids, 62:468-473 (1997). This assay measures only the lyase activity of the P450₁₇α enzyme. This assay is comparable to the HPLC assay procedure (which utilizes [7-³ H]-pregnenolone as substrate), and measures both the hydroxylase and lyase activities of the enzyme.

IC₅₀ values for inhibitors are calculated from the linear regression line in the plot of logit of lyase activity versus log of inhibitor concentration. Kᵢ values are also determined from assays as described by Njar *et al.*, (1997), *supra.* Each inhibitor is examined at three concentrations. Data from the various assays are used to obtain Lineweaver-Burk plots and from replots of slopes versus inhibitor concentration, Kᵢ values are obtained and the Kₘ for 17α-hydroxypregnenolone (substrate) is also determined.

Human C_{17,20}-lyase enzymatic assays were conducted in 200 µL volume in Eppendorf tubes, using microsomal fraction from human testis (Celsis Cat #S00110) as the enzyme source. Total protein concentration of the microsomal fraction is estimated to be 20 mg/ml. Prior to adding the microsomal fractions, reaction mixtures containing 50 mM NaPO₄ buffer (pH 7.4), 1 mM MgCl₂, 0.1 mM EDTA, 0.1 mM dithiothreitol, 0.5 mM NADPH, 4 µM 17α-hydroxypregnenolone, 1 µL of [21-³H]-17α-hydroxypregnenolone (American Radiolabeled Chemicals, ART #1663, Specific activity = 50-60 Ci/mmol), and the appropriate testing compounds were incubated for 5 minutes in a 37 °C shaking water bath (150 rpm). Following the 5-minute pre-incubation, 5µL of human testis microsome was added to each of the reaction mixtures (except for the Negative Controls, which received 5µL of H₂O). After 30-minute incubation at 37°C in shaking water bath (150 rpm), reactions were stopped by addition of 200 µL of cold chloroform and vigorous shaking for 30 minutes. Tubes were centrifuged at 1,500x g for 15 minutes at 4 °C, and the aqueous phase was transferred to fresh Eppendorf tubes. Forty microliters (40 µL) of 8.5% charcoal (Sigma Cat # C6241) suspension was added to each tube, mixed well and incubated at 4 °C for 30 minutes. Tubes were centrifuged at 1,500x g for 15 minutes at 4°C, and 100 µL upper layer from each tube was transferred into each well of a 96-well isoplate (PerkinElmer Cat # 6005040). Finally, 100µL of Optiphase supermix scintillation fluid (PerkinElmer Cat # 1200430) was added to each well, mixed by pippeting up and down 3 times. Radioactivity was measured with MicroBeta Trilux Counter using tritium program. All testing compounds were dissolved and diluted in methanol. Two microliters (2 µL) of the properly diluted test compound was added to each reaction to reach the desired concentration. In Negative control (no enzyme activity) and Activity control (100% enzyme activity), 2µL of methanol was added. Each data point was tested in duplicate. Inhibition of human C_{17,20}-lyase activity was calculated either by inhibition rate at 100nM concentration and the inhibition rate was calculated as following;

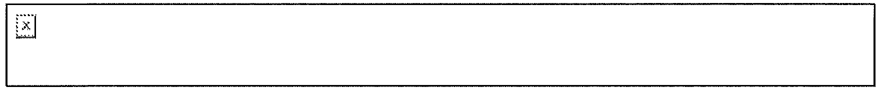

or by IC₅₀ value which was generated using Prism software under "non-linear regression analysis".
Most of the compounds tested have an inhibition rate over 50% when tested at 100 nM concentration.

### Biological Example 4: CYP17 Enzymatic Assay (IC₅₀)

The assays were performed in 96-well plates. Typical assays were carried out in total 100 µL volume that included test compounds at designated concentrations, 1X reaction buffer, 0.5 mM NADPH, 1 µM 17α-hydroxypregnenolone, and 2.5 µL of diluted human testicular microsome (Celsis. Cat #S00110). Reaction without test compound served as positive control. Reactions were allowed to incubate at 37 °C for 90 minutes with gentle shaking. The reaction was stopped by directly applying the enzymatic reaction mixture to ELISA plate for evaluation of DHEA concentration according to manufacturer's protocol (IBL America). A standard curve (OD450 vs. DHEA concentration) was generated using purified DHEA. The amount of DHEA in the negative control (reaction that did not include 17α-hydroxypregnenolone) was subtracted from total DHEA concentration and the amount of de novo synthesized DHEA was plotted vs. the concentration of the test compounds. The inhibition curve was generated using GraphPad Prism software under "non-linear regression analysis" and IC₅₀ value was calculated as compound concentrations that resulted in 50% inhibition of the positive control value.

### Biological Example 5: CYP17 Enzymatic Assay (% Inhibition at 10 nM), version 1

The assays were performed in 96-well plates. Typical assays were carried out in total 100 µL volume that included test compounds at 10 nM concentration, 1X reaction buffer, 0.5 mM NADPH, 1 µM 17α-hydroxypregnenolone, and 2.5 µL of diluted human testicular microsome (Celsis. Cat #S00110). Reaction without test compound served as positive control. Reactions were allowed to incubate at 37 °C for 90 minutes with gentle shaking. The reaction was stopped by directly applying the enzymatic reaction mixture to ELISA plate for evaluation of DHEA concentration according to manufacturer's protocol (IBL America). A standard curve (OD450 vs. DHEA concentration) was generated using purified DHEA. Amount of DHEA in the negative control (reaction that does not include 17α-hydroxypregnenolone) was subtracted from total DHEA. Percent inhibition (inhibition %) of the compound was calculated using normalized DHEA concentration based on following formula: 100-([DHEA] of compound/[DHEA] of positive control *100).

### Biological Example 6: CYP17 Enzymatic Assay (% Inhibition at 10 nM), version 2

Human C_{17,20}-lyase enzymatic assays were conducted in 200 µL volume in Eppendorf tubes, using microsomal fraction from human testis (Celsis Cat #S00110) as the enzyme source. Total protein concentration of the microsomal fraction is estimated to be 20 mg/ml. Prior to adding the microsomal fractions, reaction mixtures containing 50 mM NaPO₄ buffer (pH 7.4), 1 mM MgCl₂, 0.1 mM EDTA, 0.1 mM dithiothreitol, 0.5 mM NADPH, 4 µM 17α-hydroxypregnenolone, 1 µL of [21-³H]-17α-hydroxypregnenolone (American Radiolabeled Chemicals, ART #1663, Specific activity = 50-60 Ci/mmol), and the appropriate testing compounds were incubated for 5 minutes in a 37 °C shaking water bath (150 rpm). Following the 5-minute pre-incubation, 5 µL of human testis microsome was added to each of the reaction mixtures (except for the Negative Controls, which received 5µL of H₂O). After 30-minute incubation at 37 °C in shaking water bath (150 rpm), reactions were stopped by addition of 200 µL of cold chloroform and vigorous shaking for 30 minutes. Tubes were centrifuged at 1,500x g for 15 minutes at 4 °C, and the aqueous phase was transferred to fresh Eppendorf tubes. Forty microliters (40 µL) of 8.5% charcoal (Sigma Cat # C6241) suspension was added to each tube, mixed well and incubated at 4 °C for 30 minutes. Tubes were centrifuged at 1,500x g for 15 minutes at 4°C, and 100 µL upper layer from each tube was transferred into each well of a 96-well isoplate (PerkinElmer Cat # 6005040). Finally, 100 µL of Optiphase supermix scintillation fluid (PerkinElmer Cat # 1200430) was added to each well, mixed by pippeting up and down 3 times. Radioactivity was measured with MicroBeta Trilux Counter using tritium program.

All testing compounds were dissolved and diluted in methanol. Two microliters (2 µL) of the properly diluted test compound was added to each reaction to reach the desired concentration. In Negative control (no enzyme activity) and Activity control (100% enzyme activity), 2µL of methanol was added. Each data point was tested in duplicate. For initial screening, each test compound was tested at 100 nM, and the inhibition rate was calculated as following:

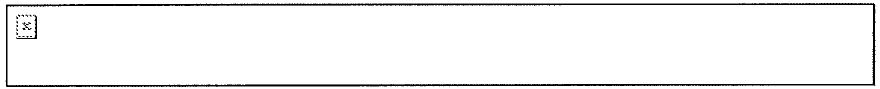

For compounds of further interest, IC₅₀ value was generated by measuring the inhibition rate at 5 concentrations and using Prism software under "non-linear regression analysis."

### Biological Example 7: CYP17 Cellular Assay (IC₅₀)

The assays were performed in 96-well plates. Typically H295R cells were seeded at 4.5X 10⁴ cells/well in 100 µL Growth Medium containing DMEM/F-12 (Gibco/Invitrogen; 11330-032), 2.5%Nu-Serum (BD Bioscience; 355100) and ITS (BD Bioscience; 354352), and cultured at 37°C, 5% CO₂ for 24hr. The next day, media were carefully removed and cells were washed twice with 200 µL 1xHBSS++. Test compounds were then added to the cells at 100, 10, 1, 0.1 nM final concentrations in 125 µL volume per well in serum-free media containing DMEM/F-12 and ITS+ Premix (BD Bioscience; 354352) with 0.1% methanol. Serum-free media with 0.1% methanol without test compound was used as control. Cells were incubated with compounds at 37 °C for additional 24 hours, and the cultured media from each compound treated well was harvested for measurement of testosterone concentration by ELISA according to manufacturer's instruction (Assay Design). Testosterone concentration of each sample was plotted vs. the concentration of the test compounds. Inhibition curve was generated using GraphPad Prism software under "non-linear regression analysis" and IC₅₀ value was calculated as compound concentrations that resulted in 50% inhibition of the positive control testosterone value.

### Biological Example 8a: Rat Liver Microsome Stability Assay and Biological Example 8b: Human Liver Microsome Stability Assay

Each test compound (1.5 µM, 30 µL) was incubated with rat (BD Gentest, Cat No. 452501) or human (BD Gentest, Cat No. 452161) liver microsomes (0.75 mg/mL, 0.1 M potassium phosphate buffer, 1.0 mM EDTA, pH 7.4) in 96 well plates. The reaction was started with addition of excess NADPH (5 mg/mL, 15 µL in 0.1 M potassium phosphate buffer, 1.0 mM EDTA, pH 7.4), the total volume is 45 µL (compound final concentration was 1 µM). The incubations were terminated at time zero and at 5, 15, 30, and 60 minutes by addition of 135 µL/excess ACN containing internal standard (IS). After quenching, the reaction mixtures was centrifuged (Eppendorf Centrifuge 5810R) at 3220 x g for 10 min (4 °C). 50 µL of the supernatant was transferred from each well into a 96-well sample plate containing 50 µL of ultrapure water (Millipore) for LC-MS/MS analysis. The half-life T1/2=0.693/K (K is the rate constant from a plot of log [concentration] vs. incubation time) was calculated. The rate of intrinsic clearance (CLi) of each compound was then calculated using the following formula: Clint = (0.693/T1/2) x (1/(microsomal protein concentration (0.5 mg/mL)) x Scaling Factors (1254.2 for human and 1792 for rat). Ketanserin was used as a positive control to confirm acceptable assay performance.

### Biological Example 9: CYP11B Enzymatic Assay (% Inhibition at 100 nM)

Adrenals excised from Sprague Dawley rats were homogenized, and mitochondria fraction were prepared by a series of centrifugation as described in Shuji Ohno, et al. J. of Steroid Biochemistry & Molecular Biology 2002, 80, 355-363. Prepared mitochondria protein was washed and resuspended in Buffer B (0.21 M mannitol, 70 mM sucrose, 5 mM MgCl2, 0.5 mM CaCl₂, 20 mM Tris-HCl (pH 7.4) and 1 mM DTT, 0.1 M PMSF). The protein concentration was determined by BCA assay and stored as 10 µL aliquots at -80 °C.

The rat Cyp11B enzyme assay was performed in 96-well plates. Typical assays were carried out in total 50 µL volume that included test compounds at 100 nM concentration, 1X Buffer B, 0.5 mM NADPH, 0.1 µM 11-deoxycortisol, and 2 µL of prepared rat adrenal mitochondria (added last). Reaction without test compound served as positive control and reaction without NADPH served as negative control. Reactions were allowed to incubate at 37 °C for 60 min with gentle shaking. The reaction was stopped by directly applying the enzymatic reaction mixture (1:5 diluted in ELISA assay buffer) to ELISA plate for evaluation of cortisol concentration according to manufacturer's protocol (Assay Design).

The mean absorbance value for each sample was used to determine the corresponding concentration from the standard curve. Amount of cortisol in the negative control (reaction that dis not include NADPH) was subtracted from total cortisol. Percent inhibition (inhibition %) of the compound was calculated using normalized cortisol concentration based on following formula: 100-([Cortisol] of compound/[Cortisol] of positive control * 100).

### Biological Example 10: CYP21-HEK293 Enzymatic Assay (% Inhibition at 100 nM)

Proceeding using methods similar to those described in Biological Example 11, the percent inhibition at 100 nM was measured.

### Biological Example 11: CYP21-HEK293 Enzymatic Assay (IC₅₀)

This assay was used to determine compound activity against Cyp21 enzyme. Human HEK 293-Cyp21 stable transfected cells (maintained in complete growth media DMEM + 10% FBS, supplemented with 125ug/mL Hygromycin) were seeded in a 96-well plate at 20,000 cells/well/100 uL in DMEM plus 10% FBS and allowed to attach overnight. The next day, 20 µL medium was carefully removed and 10 µL of 10x pre-made compounds in DMEM+10%FBS+10% methanol was transfered to the wells to final concentrations of 1000, 100, 10, 1, or 0.1 nM. Mock treatment was 1% methanol in growth media and the cells were left at 37 °C, 5% CO₂ for 5hr. After the incubation, 10 µL of substrate progesterone (Sigma) was added to the wells to make a final concentration of 10 µM. The cells were incubated the cells at 37 °C for 20hr. At the end of incubation, 90 µL of conditioned media was removed to measure DOC level by ELISA.

Preparation of the DOC ELISA: 100 µL of coating buffer (0.025M PBS pH 7.4) containing 200 ng/mL DOC-3-carboxymethyloxime-BSA (Steraloids.inc, Cat#3469-000) was added to each well of a 96-well plate and incubated overnight at 4 °C.The next day, the plates were washed twice with 250 µl of wash solution (0.015M PBS pH 7.4 containing 0.05% Tween 20). After being washed, the wells were blocked with 250 µL blocking buffer (3% BSA in 0.025M PBS, pH 7.4) at 37 °C for 2h. The plates were washed again 3 times as before, and then 90 µL of sample /standards (prepared in complete growth media) was added into the appropriate wells followed by 10 µL of antibody solution (1:450 rabbit anti-DOC in 0.6% BSA/PBS). The plates were incubated at room temperature for 2h on a shaker. The contents of the wells were removed and the plate was washed 4 times. After the final wash, 100 µL of secondary antibody (1:12000 HRP-goat-anti-rabbit IgG conjugate 0.6% BSA/PBS) was added into each well. The plate was incubated at 30 °C for 45 min. The plate was washed again 4 times and 100 µL of TMB substrate solution was added to each well. The plate was incubated at room temperature for 10 min. 100 µL of stop solution was added to each well and the optical density was read at 450nm.

The mean absorbance value for each sample was used to determine the corresponding concentration from the standard curve. Data was analyzed with "log (antagonist) vs response variable slope" under "non-linear regression analysis" using Prism5 software. IC50s were calculated as compound concentration that resulted in 50% inhibition of the control value.

### Biological Example 12: CYP17 Enzymatic Assay (% Inhibition at 1 nM)

The assays were performed in 96-well plates. Typical assays were carried out in total 100 µL volume that included test compounds at 1 nM concentration, 1X reaction buffer, 0.5 mM NADPH, 1 µM 17α-hydroxypregnenolone, and 2.5 µL of human testicular microsome (Celsis. Cat #S00110). Reaction without test compound served as a positive control. Reactions were allowed to incubate at 37 °C for 90 min with gentle shaking. The reaction was stopped by directly applying the enzymatic reaction mixture to ELISA plate for evaluation of DHEA concentration according to manufacturer's protocol (IBL America). A standard curve (OD450 vs. DHEA concentration) was generated using purified DHEA. Amount of DHEA in the negative control (reaction that does not include 17α-hydroxypregnenolone) was subtracted from total DHEA. Percent inhibition (inhibition %) of the compound was calculated using normalized DHEA concentration based on following formula: 100-([DHEA] of compound/[DHEA] of positive control *100).

### Tables 2 and 3: Biological Data

In the following table, data for compounds are given. For the assay described in Biological Example **4,** A means the compound had an IC₅₀ of less than or equal to 11 nM and A1 means an IC₅₀ of greater than 11 nM but less than or equal to 25 nM. For the assay described in Biological Example **5,** C means the compound had a percent inhibition of greater than or equal to 80% when tested at 10 nM; D means the compound had a percent inhibition of greater than or equal to 50% but less than 80%; and E means the compound had a percent inhibition of greater than or equal to 1% but less than 50%. For the assay described in Biological Example **6,** F means the compound had a percent inhibition of greater than or equal to 38% and G means the compound had a percent inhibition of less than 38% but greater than or equal to 3%. For the assay described in Biological Example 7, H means the compound had an IC₅₀ of less than or equal to 15 nM and L means the compound has an IC₅₀ of greater than 15 nM but less than or equal to 100 nM. For the assay Biological Example **8a, J** means the compound had a half life of greater than or equal to 1 minute but less than 10 minutes and K means the compound had a half life of greater than or equal to 10 minutes but less than 60 minutes. For the assay Biological Example **8b,** R means the compound had a half life of greater than or equal to 1 minute but less than 50 minutes and S means the compound had a half life of greater than or equal to 50 minutes. For the assay described in Biological Example **9,** T means the compound had a percent inhibition of less than 32% when tested at 100 nM and U means the compound had a percent inhibition of greater than or equal to 32%. For the assay described in Biological Example **10,** V means the compound had a percent inhibition of less than 30% when tested at 100 nM and W means the compound had a percent inhibition of greater than or equal to 30%. For the assay described in Biological Example **11,** Z means the compound had an IC₅₀ of less than or equal to 10 nM; Y means the compound has an IC₅₀ of greater than 10 nM but less than or equal to 100 nM; and X means the compound has an IC₅₀ of greater than 100 nM. For the assay described in Biological Example **12,** M means the compound had a percent inhibition of greater than or equal to 50% when tested at 1 nM; N means the compound had a percent inhibition of greater than or equal to 10% but less than 50%; and P means the compound had a percent inhibition of less than 10%. For all assays, "nt" means the compound was not tested in the assay and "na" means the compound was not active under the conditions tested.

**Table 2.**

| **Cmpd No.** | **Name** | **Biological Example No.** | | | | | | |
|---|---|---|---|---|---|---|---|---|
| | | **4** | **5** | **6** | **7** | **8a/ 8b** | **9** | **10** |
| 112 | (1*S*,2*S*,8*S*,12*S*,16*S*,11*R*)-2,16-dimethyl-15-(3-pyridyl)-5-oxatetracyclo[9.7.0.0^{2,8}.0^{12,16}]octadec-14-en-6-one | A | nt | F | nt | K/ R | nt | nt |
| 113 | (1*S*,12*S*,16*S*,2*R*,8*R*,11*R*)-2,16-dimethyl-15-(3-pyridyl)-6-oxatetracyclo[9.7.0.0^{2,8}.0^{12,16}]octadec-14-en-5-one | A | nt | F | nt | K/ R | nt | nt |
| 117 | (1*S*,12*S*,16*S*,2*R*,8*R*,11*R*)-15-(5-ethoxy(3-pyridyl))-2,16-dimethyl-6-oxatetracyclo[9.7.0.0^{2,8}.0^{12,11}octadec-14-en-5-one | nt | nt | G | nt | nt | nt | nt |
| 115 | (1*S*,12*S*,16*S*,2*R*,8*R*,11*R*)-15-(5-methoxy(3-pyridyl))-2,16-dimethyl-6-oxatetracyclo[9.7.0.0^{2,8}.0^{12,16}]octadec-14-en-5-one | nt | nt | F | nt | nt | nt | nt |
| 34 | (11a*R*,13a*S*)-11a,13a-dimethyl-1-(pyridin-3-yl)-3b,4,5,8,11,11a,11b,12,13,13a-decahydro-3*H*-azepino[2,1-a]cyclopenta[f]isoquinolin-7(3a*H*)-one | nt | nt | F | nt | J | nt | nt |
| 35 | (11a*R*,13a*S*)-11a,13a-dimethyl-1-(pyridin-3-yl)-3b,4,5,10,11,11a,11b,12,13,13a-decahydro-3*H*-azepino[2,1-a]cyclopenta[f]isoquinolin-7(3a*H*)-one | nt | nt | F | nt | nt | nt | nt |
| 119 | (5a*R*,5b*S*,7a*S*,10a*S*,10b*R*,12a*R*)-5a,7a-dimethyl-8-(5-methylpyridin-3-yl)-5,5a,5b,6,7,7a,10,10a,10b,11,12,12a-dodecahydro-1*H-*cyclopenta[5,6]naphtho[2,1-c]oxepin-3(4*H*)-one | nt | E | nt | nt | J | nt | nt |
| 36 | (11a*R*,13a*S*)-9-hydroxy-11a,13a-dimethyl-1-(pyridin-3-yl)-3b,4,5,8,9,10,11,11a,11b,12,13,13a-dodecahydro-3*H*-azepino[2,1-a]cyclopenta[f]isoquinolin-7(3a*H*)-one | nt | nt | nt | nt | nt | nt | nt |
| 37 | (11a*R*,13a*S*)-11a,13a-dimethyl-1-(pyridin-3-yl)-4,5,8,10,11,11a,11b,12,13,13a-decahydro-3*H*-azepino[2,1-a]cyclopenta[f]isoquinoline-7,9(3a*H*,3b*H*)-dione | nt | nt | nt | nt | nt | nt | nt |
| 38 | (11a*R*,13a*S*)-11a,13a-dimethyl-1-(pyridin-3-yl)-3b,4,5,8,9,10,11,11a,11b,12,13,13a-dodecahydro-3*H*-azepino[2,1-a]cyclopenta[f]isoquinolin-7(3a*H*)-one | A | D | nt | nt | nt | na | V |
| 39 | (11a*R*,13a*S*)-11a,13a-dimethyl-1-(5-methylpyridin-3-yl)-3b,4,5,8,9,10,11,11a,11b,12,13,13a-dodecahydro-3*H*-azepino[2,1-a]cyclopenta[f]isoquinolin-7(3a*H*)-one | A | D | nt | nt | J | nt | nt |
| 40 | (11a*R*,13a*S*)-1-(5-methoxypyridin-3-yl)-11a,13a-dimethyl-3b,4,5,8,9,10,11,11a,11b,12,13,13a-dodecahydro-3*H*-azepino[2,1-a]cyclopenta[f]isoquinolin-7(3a*H*)-one | A | C | nt | nt | J | nt | nt |
| 41 | (11a*R*,13a*S*)-1-(5-ethylpyridin-3-yl)-11a,13a-dimethyl-3b,4,5,8,9,10,11,11a,11b,12,13,13a-dodecahydro-3*H*-azepino[2,1-a]cyclopenta[f]isoquinolin-7(3a*H*)-one | A | c | nt | nt | J | nt | nt |
| 101 | (6a*S*,6b*S*,8a*S*,11a*S*,11b*R*,13a*S*)-6a,8a-dimethyl-9-(pyridin-3-yl)-4,5,6,6a,6b,7,8,8a,11,11a,11b,12,13,13a-tetradecahydro-1*H-*cyclopenta[5,6]naphtho[1,2-d]azocin-3(2*H*)-one | nt | D | nt | nt | nt | nt | nt |
| 44 | (11a*R*,13a*S*)-11a,13a-dimethyl-1-(pyridin-3-yl)-3a,3b,4,5,7,8,9,10,11,11a,11b,12,13,13a-tetradecahydro-3*H-*azepino[2,1-a]cyclopenta[f]isoquinoline | nt | E | nt | nt | nt | na | na |
| 42 | (11a*R*,13a*S*)-11a,13a-dimethyl-1-(5-propoxypyridin-3-yl)-3b,4,5,8,9,10,11,11a,11b,12,13,13a-dodecahydro-3*H*-azepino[2,1-a]cyclopenta[f]isoquinolin-7(3a*H*)-one | A | D | nt | nt | nt | nt | nt |
| 120 | 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)pyridine | A | C | nt | nt | J | nt | nt |
| 123 | 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-methoxypyridine | A | D | nt | nt | J | nt | nt |
| 121 | 3-((5a*R*,5b*S*,7a*S*,10a*S*,10b*R*,12a*S*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)pyridine | A | C | nt | nt | J | nt | nt |
| 53 | (5a*R*,7a*S*)-8-(5-methoxypyridin-3-yl)-5a,7a-dimethyl-4,5,5a,5b,6,7,7a,8,9,10,10a,10b,11,12-tetradecahydrocyclopenta[5,6]naphtho[2,1-c]azepin-3(2*H*)-one | A | D | nt | nt | nt | nt | nt |
| 45 | (11a*R*,13a*S*)-11a,13a-dimethyl-1-(5-methylpyridin-3-yl)-3a,3b,4,5,7,8,9,10,11,11a,11b,12,13,13a-tetradecahydro-3*H-*azepino[2,1-a]cyclopenta[f]isoquinoline | nt | E | nt | nt | nt | nt | nt |
| 43 | (11aR,13a*S*)-11a,13a-dimethyl-1-(5-vinylpyridin-3-yl)-3b,4,5,8,9,10,11,11a,11b,12,13,13a-dodecahydro-3*H*-azepino[2,1-a]cyclopenta[f]isoquinolin-7(3a*H*)-one | A | D | nt | nt | nt | nt | nt |
| 46 | (11a*R*,13a*S*)-11a,13a-dimethyl-1-(5-propoxypyridin-3-yl)-3a,3b,4,5,7,8,9,10,11,11a,11b,12,13,13a-tetradecahydro-3*H-*azepino[2,1-a]cyclopenta[f]isoquinoline | nt | D | nt | nt | K | nt | nt |
| 47 | (11a*R*,13a*S*)-1-(5-methoxypyridin-3-yl)-11a,13a-dimethyl-3a,3b,4,5,7,8,9,10,11,11a,11b,12,13,13a-tetradecahydro-3*H-*azepino[2,1-a]cyclopenta[f]isoquinoline | nt | E | nt | nt | nt | nt | nt |
| 48 | (11a*R*,13a*S*)-1-(5-ethylpyridin-3-yl)-11a,13a-dimethyl-3a,3b,4,5,7,8,9,10,11,11a,11b,12,13,13a-tetradecahydro-3*H-*azepino[2,1-a]cyclopenta[f]isoquinoline | nt | E | nt | nt | J | nt | nt |
| 142 | 10a,12a-dimethyl-1-(pyrazin-2-yl)-3,3a,3b,4,8,9,10,10a,10b,11,12,12a-dodecahydrocyclohepta[a]cyclopenta[f]naphthalen-7(6*H*)-one | nt | c | nt | nt | nt/ R | nt | w |
| 143 | (5a*R*,7a*S*)-5a,7a-dimethyl-8-(5-propoxypyridin-3-yl)-4,5,5a,5b,6,7,7a,10,10a,10b,11,12-dodecahydrocyclopenta[5,6]naphtho[2,1-c]azepin-3(2*H*)-one | A | D | nt | nt | nt | nt | V |
| 144 | (5a*R*,7a*S*)-2,5a,7a-trimethyl-8-(5-propoxypyridin-3-yl)-4,5,5a,5b,6,7,7a,10,10a,10b,11,12-dodecahydrocyclopenta[5,6]naphtho[2,1-c]azepin-3(2*H*)-one | nt | D | nt | nt | nt | nt | nt |
| 145 | (5a*R*,7a*S*)-8-(5-ethylpyridin-3-yl)-2,5a,7a-trimethyl-4,5,5a,5b,6,7,7a,10,10a,10b,11,12-dodecahydrocyclopenta[5,6]naphtho[2,1-c]azepin-3(2*H*)-one | nt | D | nt | nt | nt | nt | W |
| 122 | 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-methoxypyridine | A | D | nt | nt | J | nt | nt |
| 98 | (5a*R*,5b*S*,7a*S*,10a*S*,10b*S*)-1,5a,7a-trimethyl-8-(pyridin-3-yl)-5,5a,5b,6,7,7a,10,10a,10b,11,12,12a-dodecahydro-1*H-*cyclopenta[5,6]naphtho[2,1-e][1,4]oxazepin-2(3*H*)-one | A | D | nt | nt | J | nt | nt |
| 99 | (5a*R*,5b*S*,7a*S*,10a*S*,10b*S*)-8-(5-ethoxypyridin-3-yl)-1,5a,7a-trimethyl-5,5a,5b,6,7,7a,10,10a,10b,11,12,12a-dodecahydro-1*H-*cyclopenta[5,6]naphtho[2,1-e][1,4]oxazepin-2(3*H*)-one | nt | E | nt | nt | nt | nt | nt |
| 90 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)pyridine | nt | E | nt | nt | nt | nt | nt |
| 91 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-methylpyridine | nt | E | nt | nt | nt | nt | nt |
| 92 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-methoxypyridine | nt | D | nt | nt | nt | nt | nt |
| 445 | (5a*R*,5b*S*,7a*S*,10a*S*,10b*S*)-1,5a,7a-trimethyl-8-(5-methylpyridin-3-yl)-5,5a,5b,6,7,7a,10,10a,10b,11,12,12a-dodecahydro-1*H-*cyclopenta[5,6]naphtho[2,1-e][1,4]oxazepin-2(3*H*)-one | nt | E | nt | nt | nt | nt | nt |
| 127 | 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-methylpyridine | A | D | nt | nt | J | nt | nt |
| 126 | 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-methylpyridine | A | D | nt | nt | nt | nt | nt |
| 93 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-ethylpyridine | nt | E | nt | nt | nt | nt | nt |
| 94 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-ethoxypyridine | nt | E | nt | nt | nt | nt | nt |
| 446 | (5a*R*,7a*S*)-2,5a,7a-trimethyl-8-(5-methylpyridin-3-yl)-2,3,4,5,5a,5b,6,7,7a,10,10a,10b,11,12-tetradecahydrocyclopenta[5,6]naphtho[2,1-c]azepine | nt | E | nt | nt | nt | nt | nt |
| 146 | (5a*R*,7a*S*)-8-(1*H*-imidaxol-1-yl)-2,5a,7a-trimethyl-4,5,5a,5b,6,7,7a,10,10a,10b,11,12-dodecahydrocyclopenta[5,6]naphtho[2,1-c]azepin-3(2*H*)-one | A | C | nt | nt | K/ R | U | na |
| 147 | (5a*R*,7a*S*)-2,5a,7a-trimethyl-8-(pyridin-3-yl)-2,3,4,5,5a,5b,6,7,7a,10,10a,10b,11,12-tetradecahydrocyclopenta[5,6]naphtho[2,1-c]azepine | nt | E | nt | nt | nt | na | na |
| 148 | (5a*R*,7a*S*)-8-(5-methoxypyridin-3-yl)-2,5a,7a-trimethyl-2,3,4,5,5a,5b,6,7,7a,10,10a,10b,11,12-tetradecahydrocyclopenta[5,6]naphtho[2,1-c]azepine | nt | E | nt | nt | nt | nt | nt |
| 149 | (5a*R*,7a*S*)-2,5a,7a-trimethyl-8-(5-propoxypyridin-3-yl)-2,3,4,5,5a,5b,6,7,7a,10,10a,10b,11,12-tetradecahydrocyclopenta[5,6]naphtho[2,1-c]azepine | nt | E | nt | nt | nt | nt | nt |
| 125 | 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-ethoxypyridine | A | D | nt | nt | J | nt | nt |
| 124 | 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-ethoxypyridine | nt | D | nt | nt | nt | nt | nt |
| 95 | 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-4-methylpyridine | nt | E | nt | nt | nt | nt | nt |
| 96 | 4-chloro-3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)pyridine | nt | E | nt | nt | nt | nt | nt |
| 150 | (5a*R*,7a*S*)-8-(5-ethylpyridin-3-yl)-2,5a,7a-trimethyl-2,3,4,5,5a,5b,6,7,7a,10,10a,10b,11,12-tetradecahydrocyclopenta[5,6]naphtho[2,1-c]azepine | nt | E | nt | nt | nt | nt | nt |
| 151 | (5a*R*,7a*S*)-8-(1*H*-imidazol-1-yl)-2,5a,7a-trimethyl-2,3,4,5,5a,5b,6,7,7a,10,10a,10b,11,12-tetradecahydrocyclopenta[5,6]naphtho[2,1-c]azepine | nt | E | nt | nt | nt | T | na |
| 97 | 1-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-1*H-*imidazole | A | D | nt | nt | K | nt | nt |
| 152 | (5a*R*,7a*S*)-8-(1*H*-imidazol-1-yl)-5a,7a-dimethyl-4,5,5a,5b,6,7,7a,8,9,10,10a,10b,11,12-tetradecahydrocyclopenta[5,6]naphtho[2,1-c]azepin-3(2*H*)-one | A | C | nt | nt | K/S | nt | na |
| 62 | (5a*R*,7a*S*)-5a,7a-dimethyl-8-(1*H*-1,2,4-triazol-1-yl)-4,5,5a,5b,6,7,7a,10,10a,10b,11,12-dodecahydrocyclopenta[5,6]naphtho[2,1-c]azepin-3(2*H*)-one | A | C | nt | nt | J | T | W |
| 153 | (5a*R*,7a*S*)-8-(1*H*-benzo[d]imidazol-1-yl)-5a,7a-dimethyl-4,5,5a,5b,6,7,7a,10,10a,10b,11,12-dodecahydrocyclopenta[5,6]naphtho[2,1-c]azepin-3(2*H*)-one | nt | E | nt | nt | nt | nt | nt |
| 154 | (3a*R*,7a*S*)-5a,7a-dimethyl-8-(4-methyl-1*H*-imidazol-1-yl)-4,5,5a,5b,6,7,7a,10,10a,10b,11,12-dodecahydrocyclopenta[5,6]naphtho[2,1-c]azepin-3(2*H*)-one | A | D | nt | nt | K | nt | nt |
| 155 | (5a*R*,7a*S*)-8-(1*H*-benzo[d]imidazol-1-yl)-2,5a,7a-trimethyl-4,5,5a,5b,6,7,7a,10,10a,10b,11,12-dodecahydrocyclopenta[5,6]naphtho[2,1-c]azepin-3(2*H*)-one | nt | E | nt | nt | nt | nt | nt |
| 59 | (5a*R*,7a*S*)-5a,7a-dimethyl-8-(1*H*-1,2,3-triazol-1-yl)-4,5,5a,5b,6,7,7a,10,10a,10b,11,12-dodecahydrocyclopenta[5,6]naphtho[2,1-c]azepin-3(2*H*)-one | nt | C | nt | nt | J/S | na | V |
| 64 | 3-((10*R*,13*S*)-10,13-dimethyl-2,3,4,7,8,9,10,11,12,13,14,15-dodecahydro-1*H*-cyclopenta[a]phenanthren-17-yl)-5-ethylpyridine | nt | E | nt | nt | nt | nt | nt |
| 60 | (5a*R*,7a*S*)-5a,7a-dimethyl-8-(pyrimidin-5-yl)-4,5,5a,5b,6,7,7a,10,10a,10b,11,12-dodecahydrocyclopenta[5,6]naphtho[2,1-c]azepin-3(2*H*)-one | nt | D | nt | nt | J | nt | W |
| 65 | 3-((10*R*,13*S*)-10,13-dimethyl-2,3,4,7,8,9,10,11,12,13,14,15-dodecahydro-1*H*-cyclopenta[a]phenanthren-17-yl)-5-methylpyridine | nt | D | nt | nt | nt | nt | nt |
| 66 | 3-((10*R*,13*S*)-10,13-dimethyl-2,3,4,7,8,9,10,11,12,13,14,15-dodecahydro-1*H*-cyclopenta[a]phenanthren-17-yl)-5-methoxypyridine | nt | D | nt | nt | nt | nt | nt |
| 67 | 3-((10*R*,13*S*)-10,13-dimethyl-2,3,4,7,8,9,10,11,12,13,14,15-dodecahydro-1*H*-cyclopenta[a]phenanthren-17-yl)pyridine | nt | D | nt | nt | J | nt | nt |
| 135 | (5a*S*,7a*S*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one | A | C | nt | nt | K/S | nt | nt |
| 136 | (5a*R*,7a*S*)-8-(1*H*-imidazol-1-yl)-5a,7a-dimethyl-1,2,5,5a,5b,6,7,7a,10,10a,10b,11,12,12a-tetradecahydrocyclopenta[5,6]naphtho[2,1-c]azepin-3(4*H*)-one | nt | D | nt | nt | nt | nt | nt |
| 61 | (5a*R*,7a*S*)-5a,7a-dimethyl-8-(pyrazin-2-yl)-4,5,5a,5b,6,7,7a,10,10a,10b,11,12-dodecahydrocyclopenta[5,6]naphtho[2,1-c]azepin-3(2*H*)-one | nt | C | nt | nt | J | nt | w |
| 63 | (5a*R*,7a*S*)-5a,7a-dimethyl-8-(2*H*-tetrazol-2-yl)-4,5,5a,5b,6,7,7a,10,10a,10b,11,12-dodecahydrocyclopenta[5,6]naphtho[2,1-c]azepin-3(2*H*)-one | nt | C | nt | nt | J | nt | nt |
| 70 | (10*R*,13*S*)-10,13-dimethyl-17-(5-methylpyridin-3-yl)-2,3,4,5,6,7,8,9,10,11,12,13,14,15-tetradecahydro-1*H-*cyclopenta[a]phenanthren-6-ol | A | nt | nt | nt | J | nt | nt |
| 71 | (10*R*,13*S*)-10,13-dimethyl-17-(5-methylpyridin-3-yl)-2,3,4,5,7,8,9,11,12,13,14,15-dodecahydro-1*H-*cyclopenta[a]phenanthren-6(10*H*)-one | A | nt | nt | nt | J | nt | nt |
| 72 | (10*R*,13*S*)-17-(5-methoxypyridin-3-yl)-10,13-dimethyl-2,3,4,5,7,8,9,11,12,13,14,15-dodecahydro-1*H-*cyclopenta[a]phenanthren-6(10*H*)-one | A | nt | nt | nt | J | nt | nt |
| 73 | (3S,10*R*,13*S*)-17-(1*H*-imidazol-1-yl)-10,13-dimethyl-2,3,4,7,8,9,10,11,12,13-decahydro-1*H*-cyclopenta[a]phenanthren-3-yl acetate | A | nt | nt | nt | na | nt | nt |
| 137 | (5a*S*,5b*S*,7a*S*,10a*S*,10b*R*,12a*S*)-5a,7a-dimethyl-8-(1*H*-1,2,3-triazol-1-yl)-3,4,5,5a,5b,6,7,7a,10,10a,10b,11,12,12a-tetradecahydrocyclopenta[5,6]naphtho[1,2-d]azepin-2(1*H*)-one | A | C | nt | H | K/S | nt | nt |
| 138 | (5a*R*,5b*S*,7a*S*,10a*S*,10b*R*,12a*R*)-5a,7a-dimethyl-8-(1*H*-1,2,3-triazol-1-yl)-1,2,5,5a,5b,6,7,7a,10,10a,10b,11,12,12a-tetradecahydrocyclopenta[5,6]naphtho[2,1-c]azepin-3(4*H*)-one | nt | E | nt | nt | nt | nt | nt |
| 106 | (3a*S,*10a*R*,10b*S*,12a*S*)-1-(1*H*-imidazol-1-yl)-10a,12a-dimethyl-3,3a,3b,4,6,7,10,10a,10b,11,12,12a-dodecahydrocyclohepta[a]cyclopenta[f]naphthalen-8(9*H*)-one | A | C | nt | nt | nt/ R | nt | nt |
| 107 | (3a*S*,10a*R*,10b*S*,12a*S*)-1-(1*H*-imidazol-1-yl)-10a,12a-dimethyl-3,3a,3b,4,8,9,10,10a,10b,11,12,12a-dodecahydrocyclohepta[a]cyclopenta[f]naphthalen-7(6*H*)-one | A | C | nt | nt | nt/ R | nt | W |
| 131 | (3*S*,10*S*,13*S*)-17-(1*H*-imidazol-1-yl)-10,13-dimethyl-15-oxo-2,3,4,5,6,7,8,9,10,11,12,13,14,15-tetradecahydro-1*H-*cyclopenta[a]phenanthren-3-yl acetate | nt | E | nt | nt | nt | nt | nt |
| 75 | (10*R*,13*S*)-17-(1*H*-imidazol-1-yl)-10,13-dimethyl-6,7,8,9,10,11,12,13-octahydro-1*H*-cyclopenta[a]phenanthren-3(2*H*)-one | A | C | nt | nt | K/S | U | V |
| 76 | (5a*R*,7a*S*)-8-(1*H*-imidazol-1-yl)-5a,7a-dimethyl-4,5,5a,5b,6,7,7a,10b,11,12-decahydrocyclopenta[5,6]naphtho[2,1-c]azepin-3(2*H*)-one | A | C | nt | nt | K/S | na | V |
| 108 | (3a*S*,3b*S*,5a*S*,8a*S*,8b*R*,10a*S*)-6-(1*H*-imidazol-1-yl)-3a,5a-dimethyl-3,3a,3b,4,5,5a,8,8a,8b,9,10,10a-dodecahydrodicyclopenta[a,f]naphthalen-2(1*H*)-one | A | C | nt | nt | J/S | nt | nt |
| 110 | (3a*S*,5a*S*)-3a,5a-dimethyl-6-(1*H*-1,2,3-triazol-1-yl)-3,3a,3b,4,5,5a,8,8a,8b,9,10,10a-dodecahydrodicyclopenta[a,f]naphthalen-2(1*H*)-one | nt | E | nt | nt | nt | nt | nt |
| 78 | (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl acetate | nt | E | nt | nt | nt | U | nt |
| 79 | (3*S*,10*R*,13*S*)-10,13-dimethyl-17-(pyridin-3-yl)-2,3,4,7,8,9,10,11,12,13-decahydro-1*H*-cyclopenta[a]phenanthren-3-ol | A | D | nt | H | K/ R | T | V |
| 80 | (3*S*,10*R*,13*S*)-17-(5-methoxypyridin-3-yl)-10,13-dimethyl-2,3,4,7,8,9,10,11,12,13-decahydro-1*H*-cyclopenta[a]phenanthren-3-yl acetate | A | C | nt | nt | nt | nt | nt |
| 81 | (3*S*,10*R*,13*S*)-17-(5-methoxypyridin-3-yl)-10,13-dimethyl-2,3,4,7,8,9,10,11,12,13-decahydro-1*H*-cyclopenta[a]phenanthren-3-ol | A | C | nt | H | S | nt | nt |
| 82 | (10*R*,13*S*)-10,13-dimethyl-17-(pyridin-3-yl)-6,7,8,9,10,11,12,13-octahydro-1*H*-cyclopenta[a]phenanthren-3(2*H*)-one | A | C | nt | nt | J/R | na | W |
| 83 | (10*R*,13*S*)-17-(5-methoxypyridin-3-yl)-10,13-dimethyl-6,7,8,9,10,11,12,13-octahydro-1*H*-cyclopenta[a]phenanthren-3(2*H*)-one | nt | D | nt | nt | nt/ R | nt | W |
| 85 | (10*R*,13*S*)-17-(1*H*-imidazol-1-yl)-10,13-dimethyl-2,3,4,5,6,7,8,9,10,11,12,13,14,15-tetradecahydro-1*H-*cyclopenta[a]phenanthren-6-ol | A | D | nt | nt | nt/ R | nt | nt |
| 139 | (5a*S*,7a*S*)-5a,7a-dimethyl-8-(1*H*-1,2,3-triazol-1-yl)-1,2,3,4,5,5a,5b,6,7,7a,10,10a,10b,11,12,12a-hexadecahydrocyclopenta[5,6]naphtho[1,2-d]azepine | nt | E | nt | nt | nt | nt | nt |
| 140 | (5a*R*,7a*S*)-5a,7a-dimethyl-8-(1*H*-1,2,3-triazol-1-yl)-1,2,3,4,5,5a,5b,6,7,7a,10,10a,10b,11,12,12a-hexadecahydrocyclopenta[5,6]naphtho[2,1-c]azepine | nt | E | nt | nt | nt | nt | nt |
| 109 | (3a*S*,5a*S*)-6-(1*H*-imidazol-1-yl)-3a,5a-dimethyl-1,2,3,3a,3b,4,5,5a,8,8a,8b,9,10,10a-tetradecahydrodicyclopenta[a,f]naphthalen-2-ol | A | C | nt | H | K | U | W |
| 164 | (5*S*,8*R*,9*S*,10*S*,13*S*)-10,13-dimethyl-17-(pyridin-3-yl)-4,5,6,7,8,9,10,11,12,13-decahydro-1*H*-cyclopenta[a]phenanthren-3(2*H*)-one | A | C | nt | nt | nt/ R | U | W |
| 165 | (5a*S*,5b*S*,7a*S*,10b*R*,12a*S*)-5a,7a-dimethyl-8-(pyridin-3-yl)-3,4,5,5a,5b,6,7,7a,10b,11,12,12a-dodecahydrocyclopenta[5,6]naphtho[1,2-d]azepin-2(1*H*)-one | A | C | nt | nt | J/R | na | V |
| 166 | (5a*R*,5b*S*,7a*S*,10b*R*,12a*R*)-5a,7a-dimethyl-8-(pyridin-3-yl)-1,2,5,5a,5b,6,7,7a,10b,11,12,12a-dodecahydrocyclopenta[5,6]naphtho[2,1-c]azepin-3(4*H*)-one | nt | D | nt | nt | nt | nt | nt |
| 158 | (5a*R*,7a*S*)-2,5a,7a-trimethyl-8-(pyridin-3-yl)-4,5,5a,5b,6,7,7a,10b,11,12-decahydrocyclopenta[5,6]naphtho[2,1-c]azepin-3(2*H*)-one | A | C | nt | nt | nt/ R | T | W |
| 159 | (5a*R*,7a*S*)-8-(5-methoxypyridin-3-yl)-2,5a,7a-trimethyl-4,5,5a,5b,6,7,7a,10b,11,12-decahydrocyclopenta[5,6]naphtho[2,1-c]azepin-3(2*H*)-one | A | C | nt | nt | nt/ R | nt | W |
| 162 | (5*S*,8*R*,9*S*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-yl acetate | A | C | nt | nt | nt | nt | nt |
| 169 | (5*S*,8*R*,9*S*,10*S*,13*S*)-17-(1*H*-imidazol-1-yl)-10,13-dimethyl-2,3,4,5,6,7,8,9,10,11,12,13-dodecahydro-1*H-*cyclopenta[a]phenanthren-3-yl acetate | A | C | nt | nt | nt | nt | nt |
| 170 | (5*S*,8*R*,9*S*,10*S*,13*S*)-17-(1*H*-imidazol-1-yl)-10,13-dimethyl-2,3,4,5,6,7,8,9,10,11,12,13-dodecahydro-1*H-*cyclopenta[a]phenanthren-3-ol | A | C | nt | H | K/S | nt | nt |
| 171 | (5*S*,8*R*,9*S*,10*S*,13*S*)-17-(1*H*-imidazol-1-yl)-10,13-dimethyl-4,5,6,7,8,9,10,11,12,13-decahydro-1*H*-cyclopenta[a]phenanthren-3(2*H*)-one | A | C | nt | nt | nt/ R | T | V |
| 160 | (5a*R*,7a*S*)-2,5a,7a-trimethyl-8-(5-methylpyridin-3-yl)-4,5,5a,5b,6,7,7a,10b,11,12-decahydrocyclopenta[5,6]naphtho[2,1-c]azepin-3(2*H*)-one | nt | D | nt | nt | nt | nt | V |
| 176 | (3*S*,8*R*,9*S*,10*S*,13*S*)-10,13-dimethyl-17-(1*H*-1,2,3-triazol-1-yl)-2,3,4,5,6,7,8,9,10,11,12,13-dodecahydro-1*H-*cyclopenta[a]phenanthren-3-ol | na | nt | nt | na | J | nt | nt |
| 178 | (5a*S*,5b*S*,7a*S*,10b*R*)-5a,7a-dimethyl-8-(1*H*-1,2,3-triazol-1-yl)-3,4,5,5a,5b,6,7,7a,10b,11,12,12a-dodecahydrocyclopenta[5,6]naphtho[1,2-d]azepin-2(1*H*)-one | nt | E | nt | nt | nt | nt | nt |
| 179 | (5a*R*,5b*S*,7a*S*,10b*R*)-5a,7a-dimethyl-8-(1*H*-1,2,3-triazol-1-yl)-1,2,5,5a,5b,6,7,7a,10b,11,12,12a-dodecahydrocyclopenta[5,6]naphtho[2,1-c]azepin-3(4*H*)-one | nt | E | nt | nt | nt | nt | nt |
| 130 | (3*S*,5*S*,8*R*,9*S*,10*S*,13*S*,14*S*)-10,13-dimethyl-17-(1*H*-1,2,3-triazol-1-yl)-2,3,4,5,6,7,8,9,10,11,12,13,14,15-tetradecahydro-1*H-*cyclopenta[a]phenanthren-3-yl acetate | A | C | nt | H | nt | nt | nt |
| 141 | 1-((5a*R*,5b*S*,7a*S*,10a*S*,10b*R*,12a*R*)-5a,7a-dimethyl-8-(1*H*-1,2,3-triazol-1-yl)-3,4,5,5a,5b,6,7,7a,10,10a,10b,11,12,12a-tetradecahydrocyclopenta[5,6]naphtho[2,1-c]azepin-2(1*H*)-yl)ethanone | nt | na | nt | nt | nt | nt | nt |
| 111 | (3a*S*,5a*S*)-3a,5a-dimethyl-6-(1*H*-1,2,3-triazol-1-yl)-1,2,3,3a,3b,4,5,5a,8,8b,9,10,10a-tetradecahydrodicyclopenta[a,f]naphthalen-2-ol | D | A | nt | nt | nt | nt | nt |
| 50 | (5a*R*,7a*S*)-5a,7a-dimethyl-8-(pyridin-3-yl)-4,5,5a,5b,6,7,7a,10,10a,10b,11,12-dodecahydrocyclopenta[5,6]naphtho[2,1-c]azepin-3(2*H*)-one | A | C | nt | nt | J | na | V |
| 54 | (5a*R*,7a*S*)-2,5a,7a-trimethyl-8-(5-methylpyridin-3-yl)-4,5,5a,5b,6,7,7a,10,10a,10b,11,12-dodecahydrocyclopenta[5,6]naphtho[2,1-c]azepin-3(2*H*)-one | A | D | nt | nt | nt | nt | na |
| 157 | (5a*R*,7a*S*)-8-(1*H*-imidazol-1-yl)-2,5a,7a-trimethyl-4,5,5a,5b,6,7,7a,10b,11,12-decahydrocyclopenta[5,6]naphtho[2,1-c]azepin-3(2*H*)-one | nt | C | nt | H | K/S | nt | W |
| 163 | (5*S*,8*R*,9*S*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-ol | A | C | nt | nt | nt/ S | nt | nt |
| 57 | (5a*R*,7a*S*)-8-(5-ethylpyridin-3-yl)-5a,7a-dimethyl-4,5,5a,5b,6,7,7a,10,10a,10b,11,12-dodecahydrocyclopenta[5,6]naphtho[2,1-c]azepin-3(2*H*)-one | A | C | nt | nt | K | nt | V |
| 55 | (5a*R*,7a*S*)-8-(5-methoxypyridin-3-yl)-2,5a,7a-trimethyl-4,5,5a,5b,6,7,7a,10,10a,10b,11,12-dodecahydrocyclopenta[5,6]naphtho[2,1-c]azepin-3(2*H*)-one | A | D | nt | nt | J | T | na |
| 56 | (5a*R*,7a*S*)-8-(5-methoxypyridin-3-yl)-5a,7a-dimethyl-4,5,5a,5b,6,7,7a,10,10a,10b,11,12-dodecahydrocyclopenta[5,6]naphtho[2,1-c]azepin-3(2*H*)-one | A | C | nt | nt | K | T | na |
| 51 | (5a*R*,7a*S*)-5a,7a-dimethyl-8-(5-methylpyridin-3-yl)-4,5,5a,5b,6,7,7a,10,10a,10b,11,12-dodecahydrocyclopenta[5,6]naphtho[2,1-c]azepin-3(2*H*)-one | A | D | nt | nt | K | nt | W |
| 52 | (5a*R*,7a*S*)-2,5a,7a-trimethyl-8-(pyridin-3-yl)-4,5,5a,5b,6,7,7a,10,10a,10b,11,12-dodecahydrocyclopenta[5,6]naphtho[2,1-c]azepin-3(2*H*)-one | A | D | nt | nt | K | nt | w |
| 68 | (10*R*,13*S*)-10,13-dimethyl-17-(pyridin-3-yl)-2,3,4,5,6,7,8,9,10,11,12,13,14,15-tetradecahydro-1*H-*cyclopenta[a]phenanthren-6-ol | nt | nt | nt | nt | nt | nt | nt |
| 69 | (10*R*,13*S*)-10,13-dimethyl-17-(pyridin-3-yl)-2,3,4,5,7,8,9,11,12,13,14,15-dodecahydro-1*H-*cyclopenta[a]phenanthren-6(10*H*)-one | nt | nt | nt | nt | nt | nt | nt |
| 86 | (5a*R*,7a*S*)-8-(5-methoxypyridin-3-yl)-5a,7a-dimethyl-4,5,5a,5b,6,7,7a,10b,11,12-decahydrocyclopenta[5,6]naphtho[2,1-c]azepin-3(2*H*)-one | A | D | nt | nt | nt | nt | w |
| 87 | (5a*R*,7a*S*)-5a,7a-dimethyl-8-(pyridin-3-yl)-4,5,5a,5b,6,7,7a, 10b,11,12-decahydrocyclopenta[5,6]naphtho[2,1-c]azepin-3(2*H*)-one | A | D | nt | nt | nt | na | na |
| 88 | (10*R*,13*S*)-17-(1*H*-imidazol-1-yl)-10,13-dimethyl-2,3,4,5,7,8,9,11,12,13,14,15-dodecahydro-1*H-*cyclopenta[a]phenanthren-6(10*H*)-one | A | C | nt | nt | nt | T | na |
| 89 | (10*R*,13*S*)-10,13-dimethyl-17-(pyridin-3-yl)-2,3,5,6,7,8,9,11,12,13,14,15-dodecahydro-1*H-*cyclopenta[a]phenanthren-4(10*H*)-one | A | C | nt | H | nt | nt | nt |
| 156 | (5a*R*,7a*S*)-5a,7a-dimethyl-8-(1*H*-tetrazol-1-yl)-4,5,5a,5b,6,7,7a,10,10a,10b,11,12-dodecahydrocyclopenta[5,6]naphtho[2,1-c]azepin-3(2*H*)-one | nt | nt | nt | nt | nt | U | V |
| 175 | (3*S*,8*R*,9*S*,10*S*,13*S*)-10,13-dimethyl-17-(1*H*-1,2,3-triazol-1-yl)-2,3,4,5,6,7,8,9,10,11,12,13-dodecahydro-1*H-*cyclopenta[a]phenanthren-3-yl acetate | nt | nt | nt | nt | nt | nt | nt |
| 177 | (8*R*,9*S*,10*S*,13*S*)-10,13-dimethyl-17-(1*H*-1,2,3-triazol-1-yl)-4,5,6,7,8,9,10,11,12,13-decahydro-1*H*-cyclopenta[a]phenanthren-3(2*H*)-one | nt | nt | nt | nt | nt | nt | nt |
| 49 | (5a*R*,7a*S*)-8-(1*H*-imidazol-1-yl)-5a,7a-dimethyl-4,5,5a,5b,6,7,7a,10,10a,10b,11,12-dodecahydrocyclopenta[5,6]naphtho[2,1-c]azepin-3(2*H*)-one | A | C | nt | H | K/S | nt | V |

**Table 3.**

| **Cmpd No.** | **Name** | **Biological Example No.** | | | | | | | |
|---|---|---|---|---|---|---|---|---|---|
| | | **4** | **5** | **12** | **7** | **8b** | **9** | **10** | **11** |
| 183 | (4a*R*,6a*S*)-7-(1H-imidazol-1-yl)-1,4a,6a-trimethyl-4,4a,4b,5,6,6a,9,9a,9b,10-decahydro-1H-indeno[5,4-f]quinolin-2(3*H*)-one | nt | D | nt | nt | nt | U | nt | nt |
| 180 | (5a*R*,7a*S*)-1,5a,7a-trimethyl-8-(pyridin-3-yl)-3,4,5.5a,5b,6,7,7a,10,10a,10b,11-dodecahydrocyclopenta[5,6]naphtho[2,1-b]azepin-2(1*H*)-one | A | C | nt | nt | R | U | nt | nt |
| 181 | (5a*R*,7a*S*)-8-(5-methoxypyridin-3-yl)-1,5a,7a-trimethyl-3,4,5,5a,5b,6,7,7a,10,10a,10b,11-dodecahydrocyclopenta[5,6]naphtho[2,1-b]azepin-2(1*H*)-one | nt | C | nt | nt | R | U | nt | nt |
| 447 | (5a*S*,5b*S*,7a*S*,10b*R*,12a*S*)-8-(1*H*-imidazol-1-yl)-5a,7a-dimethyl-3,4,5,5a,5b,6,7,7a,10b,11,12,12a-dodecahydrocyclopenta[5,6]naphtho[1,2-d]azepin-2(1*H*)-one | A | C | nt | nt | R | na | W | nt |
| 448 | (5a*R*,5b*S*,7a*S*,10b*R*,12a*R*)-8-(1*H*-imidazol-1-yl)-5a,7a-dimethyl-1,2,5,5a,5b,6,7,7a,10b,11,12,12a-dodecahydrocyclopenta[5,6]naphtho[2,1-c]azepin-3(4*H*)-one | nt | D | nt | nt | nt | nt | nt | nt |
| 182 | (5a*R*,7a*S*)-1,5a,7a-trimethyl-8-(5-methylpyridin-3-yl)-3,4,5,5a,5b,6,7,7a,10,10a,10b,11-dodecahydrocyclopenta[5,6]naphtho[2,1-b]azepin-2(1*H*)-one | nt | D | nt | nt | nt | U | nt | nt |
| 236 | 1-((3a*R*,5a*S*)-10-methoxy-3a,5a-dimethyl-1,1a,2,3,3a,3b,4,5,5a,8,8a,8b,9,10-tetradecahydrocyclopenta[a]cyclopropa[2,3]cyclopenta[1,2-f]naphthalen-6-yl)-1*H*-imidazole | A | C | N | nt | nt | nt | nt | nt |
| 449 | 3-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)pyridine | nt | E | nt | nt | nt | nt | nt | nt |
| 450 | 3-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-5-methylpyridine | nt | E | nt | nt | nt | nt | nt | nt |
| 451 | (3*S*,5*S*,8*R*,9*S*,10*S*,13*S*,14*S*)-10,13-dimethyl-17-(1*H*-1,2,3-triazol-1-yl)-2,3,4,5,6,7,8,9,10,11,12,13,14,15-tetradecahydro-1*H*-cyclopenta[a]phenanthren-3-ol | A | C | M | nt | nt | nt | nt | nt |
| 452 | 1-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-1H-1,2,3-triazole | nt | E | nt | nt | nt | nt | nt | nt |
| 184 | (5a*R*,7a*S*)-3,5a,7a-trimethyl-8-(pyrazin-2-yl)-3,4,5,5a,5b,6,7,7a,11,12-decahydrocyclopenta[5,6]naphtho[1,2-d]azepin-2(10b*H*)-one | A | C | N | nt | nt | nt | w | nt |
| 185 | (3a*R*,5a*S*)-1,3a,5a-trimethyl-6-(pyridin-3-yl)-3,3a,3b,4,5,5a,8,8a,8b,9-decahydroindeno[5,4-e]indol-2(1*H*)-one | A | nt | M | nt | nt | U | nt | nt |
| 186 | (3a*R*,5a*S*)-6-(1*H*-imidazol-1-yl)-1,3a,5a-trimethyl-3,3a,3b,4,5,5a,8,8a,8b,9-decahydroindeno[5,4-e]indol-2(1*H*)-one | nt | nt | N | nt | nt | U | nt | nt |
| 453 | 3-((10*R*,13*S*)-3-methoxy-10,13-dimethyl-2,3,4,7,8,9,10,11,12,13,14,15-dodecahydro-1*H-*cyclopenta[a]phenanthren-17-yl)pyridine | nt | nt | P | nt | nt | nt | nt | nt |
| 454 | 3-((10*R*,13*S*)-3-methoxy-10,13-dimethyl-2,3,4,7,8,9,10,11,12,13,14,15-dodecahydro-1*H-*cyclopenta[a]phenanthren-17-yl)-5-methylpyridine | nt | nt | P | nt | nt | nt | nt | nt |
| 187 | (3a*R*,5a*S*)-6-(5-methoxypyridin-3-yl)-1,3a,5a-trimethyl-3,3a,3b,4,5,5a,8,8a,8b,9-decahydroindeno[5,4-e]indol-2(1*H*)-one | A | nt | M | nt | R | T | nt | nt |
| 188 | (3a*R*,5a*S*)-1,3a,5a-trimethyl-6-(5-methylpyridin-3-yl)-3,3a,3b,4,5,5a,8,8a,8b,9-decahydroindeno[5,4-e]indol-2(1*H*)-one | nt | nt | N | nt | nt | T | nt | nt |
| 455 | 3-ethyl-5-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H*-cyclopenta[a]phenanthren-17-yl)pyridine | nt | nt | N | nt | nt | nt | nt | nt |
| 456 | 3-methoxy-5-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H*-cyclopenta[a]phenanthren-17-yl)pyridine | nt | nt | N | nt | nt | nt | nt | nt |
| 457 | 3-ethoxy-5-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H*-cyclopenta[a]phenanthren-17-yl)pyridine | nt | nt | N | nt | nt | nt | nt | nt |
| 458 | 3-((3*S*,5*S*,8*R*,9*S*, 10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-4-methylpyridine | nt | nt | N | nt | nt | nt | nt | nt |
| 210 | (10*S*,13*S*)-N,10,13-trimethyl-17-(5-methylpyridin-3-yl)-2,3,4,5,6,7,8,9,10,11,12,13,14,15-tetradecahydro-1*H-*cyclopenta[a]phenanthren-3-amine | nt | nt | N | nt | nt | nt | nt | nt |
| 459 | 1-((3*S*,10*R*,13*S*)-3-methoxy-10,13-dimethyl-2,3,4,7,8,9,10,11,12,13,14,15-dodecahydro-1*H-*cyclopenta[a]phenanthren-17-yl)-1*H*-imidazole | nt | nt | N | nt | nt | nt | nt | nt |
| 211 | (10*S*,13*S*)-N,10,13-trimethyl-17-(pyridin-3-yl)-2,3,4,5,6,7,8,9,10,11,12,13,14,15-tetradecahydro-1*H-*cyclopenta[a]phenanthren-3-amine | nt | nt | N | nt | nt | nt | nt | nt |
| 189 | (3a*R*,5a*S*)-6-(5-ethoxypyridin-3-yl)-1,3a,5a-trimethyl-3,3a,3b,4,5,5a,8,8a,8b,9-decahydroindeno[5,4-e]indol-2(1*H*)-one | nt | nt | N | nt | nt | U | nt | nt |
| 212 | *N*-((10*S*,13*S*)-10,13-dimethyl-17-(5-methylpyridin-3-yl)-2,3,4,5,6,7,8,9,10,11,12,13,14,15-tetradecahydro-1*H-*cyclopenta[a]phenanthren-3-yl)-*N*-methylacetamide | nt | nt | N | nt | nt | nt | nt | nt |
| 214 | (3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-amine | nt | nt | N | nt | nt | nt | nt | nt |
| 213 | *N*-((10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)-*N*-methylacetamide | nt | nt | N | nt | nt | nt | nt | nt |
| 215 | *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)acetamide | A | nt | M | nt | R | nt | nt | nt |
| 460 | (5a*S*,5b*S*,7a*S,*10b*R*)-5a,7a-dimethyl-8-(5-methylpyridin-3-yl)-3,4,5,5a,5b,6,7,7a,10b,11,12,12a-dodecahydrocyclopenta[5,6]naphtho[1,2-d]azepin-2(1*H*)-one | A | nt | M | nt | nt | nt | nt | nt |
| 461 | (5a*R*,5b*S*,7a*S*,10b*R*)-5a,7a-dimethyl-8-(5-methylpyridin-3-yl)-1,2,5,5a,5b,6,7,7a,10b,11,12,12a-dodecahydrocyclopenta[5,6]naphtho[2,1-c]azepin-3(4*H*)-one | nt | nt | N | nt | nt | nt | nt | nt |
| 214b | 3-((3*R*,10*S*,13*S*)-3-azido-10,13-dimethyl-2,3,4,5,6,7,8,9,10,11,12,13,14,15-tetradecahydro-1*H-*cyclopenta[a]phenanthren-17-yl)pyridine | nt | nt | N | nt | nt | nt | nt | nt |
| 190 | (3*S*, 10*R*,13*S*)-10,13-dimethyl-17-(4-methylpyridin-3-yl)-2,3,4,7,8,9,10,11,12,13-decahydro-1*H-*cyclopenta[a]phenanthren-3-ol | nt | nt | M | L | R | nt | nt | nt |
| 191 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl isobutyrate | nt | nt | N | nt | nt | nt | nt | nt |
| 195' | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 2-(dimethylamino)acetate | nt | nt | M | L | nt | nt | nt | nt |
| 444 | (9a*R*,11a*S*)-5,9a,11a-trimethyl-1-(4-methylpyridin-3-yl)-3b,4,5,8,9,9a,9b,10,11,11a-decahydro-3*H-*cyclopenta[i]phenanthridin-7(3a*H*)-one | nt | nt | N | H | nt | U | nt | nt |
| 216 | *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)isobutyramide | nt | nt | M | H | R | nt | nt | nt |
| 217 | *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)-2,2,2-trifluoroacetamide | nt | nt | N | nt | nt | nt | nt | nt |
| 462 | (5a*S*,5b*S*,7a*S*,10a*S*,10b*R*,12a*S*)-5a,7a-dimethyl-8-(4-methylpyridin-3-yl)-3,4,5,5a,5b,6,7,7a,10,10a,10b,11,12,12a-tetradecahydrocyclopenta[5,6]naphtho[1,2-d]azepin-2(1*H*)-one | nt | nt | N | H | nt | T | na | nt |
| 192 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl pivalate | nt | nt | N | nt | nt | nt | nt | nt |
| 223 | N-((3R, 10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)acetamide | A | nt | M | H | R | nt | nt | nt |
| 21 | N-((3*R*, 10*S*,13*S*)-10,13-dimethyl-17-(4-methylpyridin-3-yl)-2,3,4,5,6,7,8,9,10,11,12,13,14,15-tetradecahydro-1*H-*cyclopenta[a]phenanthren-3-yl)acetamide | nt | nt | M | H | R | nt | nt | nt |
| 243 | *N*-((3*R*,10*R*,13*S*)-10,13-dimethyl-17-(pyridin-3-yl)-2,3,4,7,8,9,10,11,12,13-decahydro-1*H-*cyclopenta[a]phenanthren-3-yl)acetamide | nt | nt | M | H | R | nt | nt | nt |
| 237 | *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)acetamide | A | nt | M | H | s | U | na | nt |
| 248 | (3a*R*,5a*S*)-6-(1*H*-imidazol-1-yl)-*N*,3a,5a-trimethyl-1,1a,2,3,3a,3b,4,5,5a,8,8a,8b,9,10-tetradecahydrocyclopenta[a]cyclopropa[2,3]cyclopenta[1,2-f]naphthalen-10-amine | nt | nt | na | nt | nt | nt | nt | nt |
| 222 | *N*-((3*R*,10*S*,13*S*)-17-(5-methoxypyridin-3-yl)-10,13-dimethyl-2,3,4,5,6,7,8,9,10,11,12,13,14,15-tetradecahydro-1*H-*cyclopenta[a]phenanthren-3-yl)acetamide | nt | nt | M | nt | R | nt | nt | nt |
| 463 | (5a*S*,7a*S*)-5a,7a-dimethyl-8-(4-methylpyridin-3-yl)-3,4,5,5a,5b,6,7,7a,10b,11,12,12a-dodecahydrocyclopenta[5,6]naphtho[1,2-d]azepin-2(1*H*)-one | nt | nt | N | nt | nt | nt | nt | nt |
| 464 | (5a*R*,7a*S*)-5a,7a-dimethyl-8-(4-methylpyridin-3-yl)-1,2,5,5a,5b,6,7,7a,10b,11,12,12a-dodecahydrocyclopenta[5,6]naphtho[2,1-c]azepin-3(4*H*)-one | nt | nt | N | nt | nt | nt | nt | nt |
| 465 | (3*S*, 10*S*,13*S*)-17-(4-ethylpyridin-3-yl)-10,13-dimethyl-2,3,4,5,6,7,8,9,10,11,12,13-dodecahydro-1*H-*cyclopenta[a]phenanthren-3-ol | nt | nt | p | nt | nt | nt | nt | nt |
| 466 | (3*S*,10*S*,13*S*)-10,13-dimethyl-17-(4-methylpyridin-3-yl)-2,3,4,5,6,7,8,9,10,11,12,13-dodecahydro-1*H-*cyclopenta[a]phenanthren-3-yl acetate | nt | nt | N | nt | nt | nt | nt | nt |
| 219 | methyl ((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)carbamate | nt | nt | M | nt | R | nt | nt | nt |
| 244 | *N*-((3*R*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-yl)acetamide | nt | nt | M | nt | R | nt | nt | nt |
| 220 | *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)cyclopropanecarboxamide | A | nt | M | nt | R | nt | nt | nt |
| 245 | *N*-((3*R*,10*R*,13*S*)-17-(1*H*-imidazol-1-yl)-10,13-dimethyl-2,3,4,7,8,9,10,11,12,13-decahydro-1*H-*cyclopenta[a]phenanthren-3-yl)acetamide | nt | nt | N | nt | nt | T | na | nt |
| 238 | *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)cyclopropanecarboxamide | A | nt | M | nt | R | U | na | nt |
| 224 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclopropanecarboxamide | A | nt | M | nt | R | nt | nt | nt |
| 467 | (5a*S*,7a*S*)-8-(4-ethylpyridin-3-yl)-5a,7a-dimethyl-3,4,5,5a,5b,6,7,7a,10b,11,12,12a-dodecahydrocyclopenta[5,6]naphtho[1,2-d]azepin-2(1*H*)-one | nt | nt | N | nt | nt | nt | nt | nt |
| 468 | (5a*R*,7a*S*)-8-(4-ethylpyridin-3-yl)-5a,7a-dimethyl-1,2,5,5a,5b,6,7,7a,10b,11,12,12a-dodecahydrocyclopenta[5,6]naphtho[2,1-c]azepin-3(4*H*)-one | nt | nt | N | nt | nt | nt | nt | nt |
| 239 | methyl ((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)carbamate | A | nt | M | nt | nt | T | na | nt |
| 195 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 2-methylbenzoate | nt | nt | N | nt | nt | nt | nt | nt |
| 225 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)methanesulfonamide | nt | nt | N | nt | nt | nt | nt | nt |
| 226 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclobutanecarboxamide | A | nt | M | nt | nt | nt | nt | nt |
| 240 | *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)cyclobutanecarboxamide | A | nt | M | nt | R | nt | nt | nt |
| 199 | (3*R*, 10*R*,13*S*)-10,13-dimethyl-17-(pyridin-3-yl)-2,3,4,7,8,9,10,11,12,13-decahydro-1*H-*cyclopenta[a]phenanthren-3-ol | A | nt | M | nt | R | nt | nt | nt |
| 441 | 1-((5a*R*,5b*S*,7a*S*, 10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)propan-1-one | A | nt | N | nt | nt | U | nt | Y |
| 442 | (5a*R*,5b*S*,7a*S*,10a*S*,10b*R*,12a*R*)-methyl8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepine-2(1*H*)-carboxylate | nt | nt | N | nt | nt | U | nt | X |
| 469 | (5a*S*,5b*S*,7a*S*,10a*S*,10b*R*,12a*S*)-8-(4-ethylpyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one | nt | nt | N | nt | nt | nt | nt | nt |
| 470 | (5a*R*,5b*S*,7a*S*,10a*S*,10b*R*,12a*R*)-8-(4-ethylpyridin-3-yl)-5a,7a-dimethyl-1,2,5,5a,5b,6,7,7a,10,10a,10b,11,12,12a-tetradecahydrocyclopenta[5,6]naphtho[2,1-c]azepin-3(4*H*)-one | nt | nt | na | nt | nt | nt | nt | nt |
| 200 | (3a*R*,5a*S*)-3a,5a-dimethyl-6-(pyridin-3-yl)-1,1a,2,3,3a,3b,4,5,5a,8b,9,10-dodecahydrocyclopenta[a]cyclopropa[2,3]cyclopenta[1,2-f]naphthalen-10-ol | A | nt | M | nt | R | nt | nt | nt |
| 227 | *N*-((3*R*,10*R*,13*S*)-10,13-dimethyl-17-(5-methylpyridin-3-yl)-2,3,4,7,8,9,10,11,12,13,14,15-dodecahydro-1*H-*cyclopenta[a]phenanthren-3-yl)cyclopropanecarboxamide | A | nt | M | nt | nt | nt | nt | nt |
| 228 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclopropanesulfonamide | nt | nt | N | nt | nt | nt | nt | na |
| 196 | (3*S*, 10*R*,13*S*)-10,13-dimethyl-17-(pyridin-3-yl)-2,3,4,7,8,9,10,11,12,13-decahydro-1*H-*cyclopenta[a]phenanthren-3-yl2,6-dimethylbenzoate | nt | nt | na | nt | nt | nt | nt | na |
| 197 | 3-((10*R*,13*S*)-10,13-dimethyl-2,7,8,9,10,11,12,13-octahydro-1*H*-cyclopenta[a]phenanthren-17-yl)pyridine | nt | nt | N | nt | nt | nt | nt | na |
| 198 | (3*R*, 10*R*,13*S*)-10,13-dimethyl-17-(pyridin-3-yl)-2,3,4,7,8,9,10,11,12,13-decahydro-1*H-*cyclopenta[a]phenanthren-3-yl 2,6-dimethylbenzoate | nt | nt | na | nt | nt | nt | nt | na |
| 229 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)thiazole-2-carboxamide | nt | nt | N | nt | nt | nt | nt | nt |
| 230 | *N*-((3*R*,10*R*,13*S*)-17-(5-methoxypyridin-3-yl)-10,13-dimethyl-2,3,4,7,8,9,10,11,12,13,14,15-dodecahydro-1*H-*cyclopenta[a]phenanthren-3-yl)cyclopropanecarboxamide | A | nt | M | nt | R | nt | nt | nt |
| 250 | (3a*R*,5a*S*)-3a,5a-dimethyl-6-(pyridin-3-yl)-1a,2,3,3a,3b,4,5,5a,8b,9-decahydrocyclopenta[a]cyclopropa[2,3]cyclopenta[1,2-f]naphthalen-10(1*H*)-one | nt | nt | M | nt | nt | nt | nt | nt |
| 231 | *N*-((3*R*,10*R*,13*S*)-10,13-dimethyl-17-(5-(trifluoromethyl)pyridin-3-yl)-2,3,4,7,8,9,10,11,12,13,14,15-dodecahydro-1*H*-cyclopenta[a]phenanthren-3-yl)acetamide | nt | nt | N | nt | R | nt | nt | nt |
| 232 | 1-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)-3-ethylurea | nt | nt | M | nt | R | nt | nt | nt |
| 241 | *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)thiazole-2-carboxamide | nt | nt | N | nt | nt | nt | nt | nt |
| 201 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 2-(tert-butyl)benzoate | nt | nt | na | nt | nt | nt | nt | nt |
| 202 | (3*S*, 10*R*,13*S*)-10,13-dimethyl-17-(pyridin-3-yl)-2,3,4,7,8,9,10,11,12,13-decahydro-1*H-*cyclopenta[a]phenanthren-3-yl ethylcarbamate | nt | nt | P | nt | nt | nt | nt | nt |
| 246 | *N*-((3*R*,10*R*,13*S*)-17-(1*H*-imidazol-1-yl)-10,13-dimethyl-2,3,4,7,8,9,10,11,12,13-decahydro-1*H-*cyclopenta[a]phenanthren-3-yl)cyclopropanecarboxamide | nt | nt | N | nt | nt | U | na | nt |
| 242 | 1-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)-3-ethylurea | nt | nt | M | nt | R | U | V | nt |
| 233 | 1-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)-3-ethylthiourea | nt | nt | N | nt | nt | nt | nt | nt |
| 234 | 1-ethyl-3-((10*R*,13*S*)-17-(5-methoxypyridin-3-yl)-10,13-dimethyl-2,3,4,7,8,9,10,11,12,13,14,15-dodecahydro-1*H-*cyclopenta[a]phenanthren-3-yl)urea | nt | nt | N | nt | nt | nt | nt | nt |
| 251 | *N*-((3a*R*,5a*S*)-3a,5a-dimethyl-6-(pyridin-3-yl)-1,1a,2,3,3a,3b,4,5,5a,8,8a,8b,9,10-tetradecahydrocyclopenta[a]cyclopropa[2,3]cyclopenta[1,2-f]naphthalen-10-yl)thiazol-2-amine | nt | nt | N | nt | nt | nt | nt | nt |
| 247 | 1-((3*R*,10*S*,13*S*)-17-(1*H*-imidazol-1-yl)-10,13-dimethyl-2,3,4,5,6,7,8,9,10,11,12,13,14,15-tetradecahydro-1*H-*cyclopenta[a]phenanthren-3-yl)-3-ethylurea | nt | nt | M | nt | nt | nt | nt | nt |
| 288 | (10*R*,13*S*)-10,13-dimethyl-17-(pyridin-3-yl)-6,7,8,9,10,11,12,13-octahydro-1*H*-cyclopenta[a]phenanthren-3(2*H*)-one oxime | A | nt | M | nt | R | nt | V | nt |
| 203 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl tert-butylcarbamate | nt | nt | N | nt | nt | nt | nt | nt |
| 287 | (10*R*,13*S*)-10,13-dimethyl-17-(pyridin-3-yl)-6,7,8,9,10,11,12,13-octahydro-1*H*-cyclopenta[a]phenanthren-3(2*H*)-one O-methyl oxime | A | nt | N | nt | S | nt | W | X |
| 439 | (3*S*,9a*S*,11b*R*)-9a11b-dimethyl-9-(pyridin-3-yl)-1,2,3,4,5a,6,6a,9a,10,11,11a,11b-dodecahydrocyclopenta[1,2]phenanthro[8a,9-b]oxiren-3-ol | nt | nt | M | nt | nt | nt | nt | nt |
| 471 | (10*R*,13*S*)-10,13-dimethyl-17-(pyrimidin-5-yl)-6,7,8,9,10,11,12,13-octahydro-1*H*-cyclopenta[a]phenanthren-3(2*H*)-one | A | nt | M | nt | R | nt | nt | Y |
| 472 | *3-*((3*S,* 10*S*,13*S*)-3-hydroxy-10,13-dimethylhexadecahydro-1*H*-cyclopenta[a]phenanthren-17-yl)pyridine 1-oxide | nt | nt | N | nt | nt | nt | nt | nt |
| 235 | *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)butyramide | A | nt | M | nt | R | nt | nt | nt |
| 419 | (10*R*,13*S*)-10,13-dimethyl-17-(pyridin-3-yl)-6,7,8,9,10,11,12,13,14,15,16,17-dodecahydro-1*H-*cyclopenta[a]phenanthren-3(2*H*)-one | nt | nt | N | nt | nt | nt | W | Y |
| 252 | (3*S*,10*S*,13*S*)-3-cyclopropyl-10,13-dimethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 253 | (3*R*,10*S*,13*S*)-3,10,13-trimethyl-17-(pyridin-3-yl)-2,3,4,5,6,7,8,9,10,11,12,13,14,15-tetradecahydro-1*H-*cyclopenta[a]phenanthren-3-ol | nt | nt | P | nt | nt | nt | nt | nt |
| 254 | (3*S*,10*S*,13*S*)-3,10,13-trimethyl-17-(pyridin-3-yl)-2,3,4,5,6,7,8,9,10,11,12,13,14,15-tetradecahydro-1*H-*cyclopenta[a]phenanthren-3-ol | A | nt | M | nt | R | nt | nt | nt |
| 255 | (3*R*, 10*S*,13*S*)-3-ethyl-10,13-dimethyl-17-(pyridin-3-yl)-2,3,4,5,6,7,8,9,10,11,12,13,14,15-tetradecahydro-1*H-*cyclopenta[a]phenanthren-3-ol | nt | nt | P | nt | nt | nt | nt | nt |
| 256 | (3*S*,10*S*,13*S*)-3-ethyl-10,13-dimethyl-17-(pyridin-3-yl)-2,3,4,5,6,7,8,9,10,11,12,13,14,15-tetradecahydro-1*H-*cyclopenta[a]phenanthren-3-ol | na | nt | N | nt | R | nt | nt | nt |
| 293 | (10*R*,13*S*)-4,4,10,13-tetramethyl-17-(pyridin-3-yl)-4,7,8,9,10,11,12,13,14,15-decahydro-1*H-*cyclopenta[a]phenanthren-3(2*H*)-one | nt | nt | P | nt | nt | nt | nt | nt |
| 294 | (10*R*,13*S*)-4,4,10,13-tetramethyl-17-(pyridin-3-yl)-2,3,4,7,8,9,10,11,12,13,14,15-dodecahydro-1*H-*cyclopenta[a]phenanthren-3-ol | nt | nt | P | nt | nt | nt | nt | nt |
| 473 | (3*S*,10*S*,13*S*)-10,13-dimethyl-17-(pyridin-3-yl)hexadecahydro-1*H*-cyclopenta[a]phenanthren-3-ol | nt | nt | P | nt | nt | nt | nt | nt |
| 443 | 1-((5a*R*,5b*S*,7a*S*,10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)ethanone | nt | nt | N | nt | nt | U | nt | nt |
| 259 | (3*S*,10*S*,13*S*)-3,10,13-trimethyl-17-(pyridin-3-yl)-2,3,4,5,6,7,8,9,10,11,12,13-dodecahydro-1*H-*cyclopenta[a]phenanthren-3-ol | A | nt | N | nt | R | nt | nt | nt |
| 260 | (3*R*,10*S*,13*S*)-3,10,13-trimethyl-17-(pyridin-3-yl)-2,3,4,5,6,7,8,9,10,11,12,13-dodecahydro-1*H-*cyclopenta[a]phenanthren-3-ol | nt | nt | P | nt | nt | nt | nt | nt |
| 298 | (10*R*,13*S*)-4,4,10,13-tetramethyl-17-(pyridin-3-yl)-2,3,4,7,8,9,10,11,12,13-decahydro-1*H-*cyclopenta[a]phenanthren-3-ol | na | nt | N | nt | R | nt | nt | nt |
| 289 | (10*R*,13*S*)-10,13-dimethyl-17-(pyridin-3-yl)-8,9,10,11,12,13-hexahydro-1*H*-cyclopenta[a]phenanthren-3(2*H*)-one | A | nt | M | nt | R | nt | nt | Y |
| 255 | (3*S*,10*S*,13*S*)-3-ethyl-10,13-dimethyl-17-(pyridin-3-yl)-2,3,4,5,6,7,8,9,10,11,12,13-dodecahydro-1*H-*cyclopenta[a]phenanthren-3-ol | nt | nt | N | nt | nt | nt | nt | nt |
| 256 | (3*R*, 10*S*,13*S*)-3-ethyl-10,13-dimethyl-17-(pyridin-3-yl)-2,3,4,5,6,7,8,9,10,11,12,13-dodecahydro-1*H-*cyclopenta[a]phenanthren-3-ol | nt | nt | P | nt | nt | nt | nt | nt |
| 474 | (3*S*,10*S*,13*S*)-3-ethynyl-10,13-dimethyl-17-(pyridin-3-yl)-2,3,4,5,6,7,8,9,10,11,12,13,14,15-tetradecahydro-1*H-*cyclopenta[a]phenanthren-3-ol | na | nt | N | nt | S | nt | nt | nt |
| 257 | (3*R*, 10*S*,13*S*)-3-ethynyl-10,13-dimethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 291 | (10*R*,13*S*)-10,13-dimethyl-17-(pyrazin-2-yl)-6,7,8,9,10,11,12,13-octahydro-1*H*-cyclopenta[a]phenanthren-3(2*H*)-one | nt | nt | M | nt | R | nt | nt | X |
| 434 | 1-((5a*S*,7a*S*)-8-(1*H*-imidazol-1-yl)-5a,7a-dimethyl-1,2,5,5a,5b,6,7,7a,10,10a,10b,11,12,12a-tetradecahydrocyclopenta[5,6]naphtho[1,2-d]azepin-3(4*H*)-yl)butan-1-one | A | nt | M | nt | nt | T | nt | na |
| 435 | 1-((5a*R*,7a*S*)-5a,7a-dimethyl-8-(pyridin-3-yl)-3,4,5,5a,5b,6,7,7a,10,10a,10b,11,12,12a-tetradecahydrocyclopenta[5,6]naphtho[2,1-c]azepin-2(1*H*)-yl)propan-1-one | A | nt | N | nt | nt | U | nt | na |
| 436 | 1-((5a*S*,7a*S*)-5a,7a-dimethyl-8-(pyridin-3-yl)-1,2,5,5a,5b,6,7,7a,10,10a,10b,11,12,12a-tetradecahydrocyclopenta[5,6]naphtho[1,2-d]azepin-3(4*H*)-yl)propan-1-one | A | nt | N | nt | nt | T | nt | na |
| 309 | (10*R*,13*S*)-6,10,13-trimethyl-17-(pyridin-3-yl)-6,7,8,9,10,11,12,13,14,15-decahydro-1*H-*cyclopenta[a]phenanthren-3(2*H*)-one | A | nt | M | H | R | nt | nt | Y |
| 290 | (10*R*,13*S*)-10,13-dimethyl-17-(pyridin-3-yl)-6,7,8,9,10,11,12,13-octahydro-3*H*-cyclopenta[a]phenanthren-3-one | A | nt | M | H | R | nt | nt | Y |
| 296 | (10*R*,13*S*)-4,10,13-trimethyl-17-(pyridin-3-yl)-6,7,8,9,10,11,12,13-octahydro-1*H*-cyclopenta[a]phenanthren-3(2*H*)-one | nt | nt | M | nt | nt | nt | nt | X |
| 311 | (10*R*,13*S*)-10,13-dimethyl-6-methylene-17-(pyridin-3-yl)-6,7,8,9,10,11,12,13,14,15-decahydro-3*H-*cyclopenta[a]phenanthren-3-one | A | nt | M | H | R | nt | nt | Z |
| 299 | (10*R*,13*S*)-4,10,13-trimethyl-17-(pyridin-3-yl)-2,3,6,7,8,9,10,11,12,13-decahydro-1*H-*cyclopenta[a]phenanthren-3-ol | A | nt | na | nt | nt | nt | nt | nt |
| 312 | (10*R*,13*S*)-10,13-dimethyl-6-methylene-17-(pyridin-3-yl)-6,7,8,9,10,11,12,13,14,15-decahydro-1*H-*cyclopenta[a]phenanthren-3(2*H*)-one | A | nt | M | H | R | nt | nt | Y |
| 297 | (10*R*,13*S*)-4-hydroxy-10,13-dimethyl-17-(pyridin-3-yl)-8,9,10,11,12,13-hexahydro-1*H*-cyclopenta[a]phenanthren-3(2*H*)-one | nt | nt | M | nt | R | nt | nt | X |
| 301 | (10*R*,13*S*)4-hydroxy-10,13-dimethyl-17-(pyridin-3-yl)-8,9,10,11,12,13,14,15-octahydro-1*H-*cyclopenta[a]phenanthren-3(2*H*)-one | nt | nt | M | nt | R | nt | nt | na |
| 313 | (10*R*,13*S*)-10,13-dimethyl-17-(pyridin-3-yl)-1,7,8,9,10,11,12,13,14,15-decahydrospiro[cyclopenta[a]phenanthrene-6,1'-cyclopropan]-3(2*H*)-one | A | nt | M | H | R | nt | nt | Z |
| 314 | (4a*R*,6a*S*)-4a,6a-dimethyl-7-(pyridin-3-yl)-1a,2,3,4,4a,4b,5,6,6a,9,9a,9b,10,11-tetradecahydro-1*H-*cyclopenta[a]cyclopropa[j]phenanthren-2-ol | A | nt | M | H | nt | nt | nt | nt |
| 361 | (10*R*,13*S*)-6,10,13-trimethyl-17-(pyridin-3-yl)-6,7,8,9,10,11,12,13,14,15-decahydro-1*H-*cyclopenta[a]phenanthren-3(2*H*)-one oxime | nt | nt | N | nt | R | nt | nt | X |
| 362 | (6c*R*,8a*S*)-10,13-dimethyl-17-(pyridin-3-yl)-1,7,8,9,10,11,12,13,14,15-decahydrospiro[cyclopenta[a]phenanthrene-6,1'-cyclopropan]-3(2*H*)-one oxime | nt | nt | N | nt | nt | nt | nt | X |
| 363 | (10*R*,13*S*,E)-4,4,10,13-tetramethyl-17-(pyridin-3-yl)-4,7,8,9,10,11,12,13,14,15-decahydro-1*H-*cyclopenta[a]phenanthren-3(2*H*)-one oxime | nt | nt | P | nt | nt | nt | nt | na |
| 368 | (5a*R*,7a*S*)-5a,7a,12-trimethyl-8-(pyridin-3-yl)-3,4,5,5a,5b,6,7,7a,10,10a,11,12-dodecahydrocyclopenta[5,6]naphtho[1,2-d]azepin-2(10b*H*)-one | A | nt | M | H | R | nt | nt | Z |
| 369 | (5a*R*,7a*S*)-5a,7a-dimethyl-8-(pyridin-3-yl)-3,4,5,5a,5b,6,7,7a,10,10a,10b,11-dodecahydro-2*H-*spiro[cyclopenta[5,6]naphtho[1,2-d]azepine-12,1'-cyclopropan]-2-one | A | nt | N | H | R | nt | nt | Z |
| 300 | (10*R*,13*S*)-4,10,13-trimethyl-17-(pyridin-3-yl)-6,7,8,9,10,11,12,13,14,15-decahydro-1*H-*cyclopenta[a]phenanthren-3(2*H*)-one | A | nt | M | H | nt | nt | nt | na |
| 204 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl pyridin-3-ylcarbamate | nt | nt | N | nt | nt | nt | nt | nt |
| 315 | (10*R*,13*S*)-6,10,13-trimethyl-17-(pyridin-3-yl)-6,7,8,9,10,11,12,13-octahydro-1*H*-cyclopenta[a]phenanthren-3(2*H*)-one | A | nt | M | nt | R | nt | nt | Z |
| 274 | (3*S*,10*R*,13*S*)-17-(5-fluoropyridin-3-yl)-10,13-dimethyl-2,3,4,7,8,9,10,11,12,13,14,15-dodecahydro-1*H-*cyclopenta[a]phenanthren-3-ol | A | nt | M | nt | s | nt | nt | nt |
| 302 | (10*R*,13*S*)-4,10,13-trimethyl-17-(pyridin-3-yl)-2,3,6,7,8,9,10,11,12,13,14,15-dodecahydro-1*H-*cyclopenta[a]phenanthren-3-ol | A | nt | N | nt | nt | nt | nt | nt |
| 364 | (10*R*,13*S*,E)-4,10,13-trimethyl-17-(pyridin-3-yl)-6,7,8,9,10,11,12,13,14,15-decahydro-1*H-*cyclopenta[a]phenanthren-3(2*H*)-one oxime | nt | nt | N | nt | nt | nt | nt | nt |
| 275 | 5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)nicotinonitrile | nt | nt | N | nt | nt | nt | nt | nt |
| 322 | (3*S*,5*S*,10*R*,13*S*)-6-fluoro-10,13-dimethyl-17-(pyridin-3-yl)-2,3,4,5,6,7,8,9,10,11,12,13,14,15-tetradecahydro-1*H-*cyclopenta[a]phenanthrene-3,5-diol | A | nt | M | nt | R | nt | nt | nt |
| 323 | (3*S*,5*R*,10*R*,13*S*)-6-fluoro-10,13-dimethyl-17-(pyridin-3-yl)-2,3,4,5,6,7,8,9,10,11,12,13,14,15-tetradecahydro-1*H-*cyclopenta[a]phenanthrene-3,5-diol | A | nt | M | nt | R | nt | nt | nt |
| 276 | (3*S*,10*R*,13*S*)-17-(6-fluoropyridin-3-yl)-10,13-dimethyl-2,3,4,7,8,9,10,11,12,13,14,15-dodecahydro-1*H-*cyclopenta[a]phenanthren-3-ol | na | nt | N | nt | R | nt | nt | nt |
| 205 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 4-methylnicotinate | nt | nt | N | nt | nt | nt | nt | nt |
| 316 | (10*R*,13*S*)-10,13-dimethyl-17-(pyridin-3-yl)-7,8,9,11,12,13,14,15-octahydrospiro[cyclopenta[a]phenanthrene-6,1'-cyclopropan]-3(10*H*)-one | A | nt | N | H | R | nt | nt | Z |
| 310e | (10*R*,13*S*)-6-fluoro-5-hydroxy-10,13-dimethyl-17-(pyridin-3-yl)-4,5,6,7,8,9,10,11,12,13,14,15-dodecahydro-1*H-*cyclopenta[a]phenanthren-3(2*H*)-one | A | nt | M | nt | nt | nt | nt | nt |
| 422 | (10*R*,13*S*)-3,10,13-trimethyl-17-(pyridin-3-yl)-2,3,6,7,8,9,10,11,12,13,14,15-dodecahydro-1*H-*cyclopenta[a]phenanthren-3-ol | nt | nt | N | nt | nt | nt | nt | nt |
| 206 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 3-hydroxy-2-(hydroxymethyl)-2-methylpropanoate | nt | nt | P | nt | nt | nt | nt | nt |
| 365 | (6c*R*,8a*S*)-10,13-dimethyl-17-(pyridin-3-yl)-7,8,9,11,12,13,14,15-octahydrospiro[cyclopenta[a]phenanthrene-6,1'-cyclopropan]-3(10*H*)-one oxime | nt | nt | N | nt | nt | nt | nt | X |
| 317 | (3a*S*,5b*R*,8*S*)-3a,5b-dimethyl-3-(pyridin-3-yl)-3a,4,5,5a,5b,6,7,8,9,10,10a,11,11a,11b-tetradecahydro-1*H-*cyclopenta[a]cyclopropa[k]phenanthren-8-ol | A | nt | M | H | R | nt | nt | nt |
| 408 | (10*R*,13*S*)-10,13-dimethyl-17-(pyridin-3-yl)-8,9,10,11,12,13,14,15-octahydro-1*H-*cyclopenta[a]phenanthren-3(2H)-one | nt | nt | M | nt | R | nt | nt | X |
| 366 | (10*R*,13*S*)-6-fluoro-10,13-dimethyl-17-(pyridin-3-yl)-6,7,8,9,10,11,12,13,14,15-decahydro-1*H-*cyclopenta[a]phenanthren-3(2*H*)-one oxime | nt | nt | N | nt | nt | nt | nt | na |
| 318 | (10*R*,13*S*)-6,10,13-trimethyl-17-(pyridin-3-yl)-6,7,8,9,10,11,12,13,14,15-decahydro-3*H-*cyclopenta[a]phenanthren-3-one | nt | nt | M | H | R | nt | nt | Z |
| 319 | (4a*R*,6a*S*)-4a,6a-dimethyl-7-(pyridin-3-yl)-4,4a,4b,5,6,6a,9,9a,9b,9c,10,10a-dodecahydrocyclopenta[a]cyclopropa[l]phenanthrea-2(3*H*)-one | nt | nt | M | nt | R | nt | nt | X |
| 475 | 3-((10*S*,13*S*)-10,13-dimethyl-1,2,4,5,6,7,8,9,10,11,12,13,14,15-tetradecahydrospiro[cyclopenta[a]phenanthrene-3,2'-oxiran]-17-yl)pyridine | nt | nt | N | nt | nt | nt | nt | nt |
| 423 | (10*R*,13*S*)-3,6,10,13-tetramethyl-17-(pyridin-3-yl)-2,3,6,7,8,9,10,11,12,13,14,15-dodecahydro-1*H-*cyclopenta[a]phenanthren-3-ol | nt | nt | N | nt | nt | nt | nt | nt |
| 207 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl 1-methylpiperidine-4-carboxylate | nt | nt | N | nt | nt | nt | nt | nt |
| 424 | (3*R*,10*S*,13*S*-3,6,10,13-tetramethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 425 | (3*S*, 10*S*,13*S*)-3,6,10,13-tetramethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 370 | (5a*R*,7a*S*)-12-fluoro-5a,7a-dimethyl-8-(pyridin-3-yl)-3,4,5,5a,5b,6,7,7a,10,10a,11,12-dodecahydrocyclopenta[5,6]naphtho[1,2-d]azepin-2(10b*H*)-one | A | nt | M | nt | R | nt | nt | na |
| 303 | (10*R*,13*S*)-4,10,13-trimethyl-17-(pyridin-3-yl)-4,5,6,7,8,9,10,11,12,13,14,15-dodecahydro-1*H-*cyclopenta[a]phenanthren-3(2*H*)-one | nt | nt | N | nt | nt | nt | nt | na |
| 367 | (10*R*,13*S*)-6,10,13-trimethyl-17-(pyridin-3-yl)-6,7,8,9,10,11,12,13,14,15-decahydro-3*H-*cyclopenta[a]phenanthren-3-one oxime | nt | nt | N | nt | nt | nt | nt | na |
| 426 | 1-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)-1*H*-imidazole-5-carbonitrile | nt | nt | N | nt | nt | U | nt | nt |
| 476 | methyl 1-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)-1*H*-imidazole-5-carboxylate | nt | nt | N | nt | nt | na | nt | nt |
| 320 | (10*R*,13*S*)-17-(5-fluoropyridin-3-yl)-10,13-dimethyl-6-methylene-6,7,8,9,10,11,12,13,14,15-decahydro-3*H-*cyclopenta[a]phenanthren-3-one | A | nt | nt | H | R | nt | nt | X |
| 440 | (10*S*,13*S*)-3-(3-hydroxypropyl)-10,13-dimethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 304 | (10*R*,13*S*)-4,10,13-trimethyl-17-(pyridin-3-yl)-2,3,4,5,6,7,8,9,10,11,12,13,14,15-tetradecahydro-1*H-*cyclopenta[a]phenanthren-3-ol | A | nt | N | H | nt | nt | nt | nt |
| 382 | (5a*R*,7a*S*)-5a,7a-dimethyl-8-(pyridin-3-yl)-3,4,5,5a,5b,6,7,7a,10,10a,10b,10c,11,11a-tetradecahydro-2*H-*cyclopenta[5,6]cyclopropa[3,4]naphtho[1,2-d]azepin-2-one | A | nt | M | nt | R | nt | nt | X |
| 401 | 3-fluoro-5-((10*R*,13*S*)-3-fluoro-10,13-dimethyl-2,3,4,7,8,9,10,11,12,13,14,15-dodecahydro-1*H-*cyclopenta[a]phenanthren-17-yl)pyridine | A | nt | nt | H | S | nt | nt | nt |
| 271 | (3*S*,10*R*,13*S*)-17-(5-fluoropyridin-3-yl)-10,13-dimethyl-2,3,4,7,8,9,10,11,12,13-decahydro-1*H-*cyclopenta[a]phenanthren-3-ol | A | nt | M | H | R | nt | nt | nt |
| 384 | (5a*R*,7a*S*)-1,5a,7a-trimethyl-8-(pyridin-3-yl)-1,2,5,5a,5b,6,7,7a,10,10a,10b,11,12,12a-tetradecahydrocyclopenta[5,6]naphtho[2,1-c]azepin-3(4*H*)-one | nt | nt | N | nt | nt | nt | nt | na |
| 321 | (3*S,*10*R,*13*S*)-6-fluoro-17-(5-fluoropyridin-3-yl)-10,13-dimethyl-2,3,4,5,6,7,8,9,10,11,12,13,14,15-tetradecahydro-1*H*-cyclopenta[a]phenanthrene-3,5-diol | A | nt | M | H | R | nt | nt | nt |
| 263 | (3*R*,10*S*,13*S*)-17-(5-fluoropyridin-3-yl)-3,10,13-trimethyl-2,3,4,5,6,7,8,9,10,11,12,13,14,15-tetradecahydro-1*H-*cyclopenta[a]phenanthren-3-ol | A1 | nt | nt | nt | nt | nt | nt | nt |
| 264 | (3*S,*10*S,*13*S*)-17-(5-fluoropyridin-3-yl)-3,10,13-trimethyl-2,3,4,5,6,7,8,9,10,11,12,13,14,15-tetradecahydro-1*H-*cyclopenta[a]phenanthren-3-ol | A | nt | nt | H | R | nt | nt | nt |
| 477 | (10*S*,13*S*)-10,13-dimethyl-17-(pyridin-3-yl)-1,2,4,5,6,7,8,9,10,11,12,13,14,15-tetradecahydro-3'*H-*spiro[cyclopenta[a]phenanthrene-3,2'-furan]-5'(4'*H*)-one | nt | nt | P | nt | nt | nt | nt | nt |
| 427 | *N*-((3*R*, 10*R*,13*S*)-17-(5-fluoropyridin-3-yl)-10,13-dimethyl-2,3,4,7,8,9,10,11,12,13,14,15-dodecahydro-1*H-*cyclopenta[a]phenanthren-3-yl)acetamide | A | nt | M | nt | R | nt | nt | nt |
| 478 | 1-ethyl-3-((3*R*,10*R*,13*S*)-17-(5-fluoropyridin-3-yl)-10,13-dimethyl-2,3,4,7,8,9,10,11,12,13,14,15-dodecahydro-1*H-*cyclopenta[a]phenanthren-3-yl)urea | nt | nt | N | nt | nt | nt | nt | nt |
| 352 | (10*R*,13*S*)-17-(5-fluoropyridin-3-yl)-10,13-dimethyl-6,7,8,9,10,11,12,13-octahydro-1*H*-cyclopenta[a]phenanthren-3(2*H*)-one | A | nt | M | H | R | nt | nt | Y |
| 324 | (10*R*,13*S*)-6-fluoro-17-(5-fluoropyridin-3-yl)-5-hydroxy-10,13-dimethyl-4,5,6,7,8,9,10,11,12,13,14,15-dodecahydro-1*H*-cyclopenta[a]phenanthren-3(2*H*)-one | A | nt | M | nt | nt | nt | nt | nt |
| 277 | (3*S*,10*R*, 13*S*)-17-(2-fluoro-5-methylpyridin-3-yl)-10,13-dimethyl-2,3,4,7,8,9,10,11,12,13,14,15-dodecahydro-1*H-*cyclopenta[a]phenanthren-3-ol | nt | nt | N | nt | nt | nt | nt | nt |
| 278 | (3*S*,10*R*,13*S*)-17-(6-fluoro-5-methylpyridin-3-yl)-10,13-dimethyl-2,3,4,7,8,9,10,11,12,13,14,15-dodecahydro-1*H-*cyclopenta[a]phenanthren-3-ol | nt | nt | P | nt | nt | nt | nt | nt |
| 355 | (10*R*,13*S*)-17-(6-fluoropyridin-3-yl)-10,13-dimethyl-6,7,8,9,10,11,12,13-octahydro-1*H*-cyclopenta[a]phenanthren-3(2*H*)-one | nt | nt | N | nt | nt | nt | nt | na |
| 325 | (10*R*,13*S*)-6-fluoro-17-(5-fluoropyridin-3-yl)-10,13-dimethyl-6,7,8,9,10,11,12,13,14,15-decahydro-1*H-*cyclopenta[a]phenanthren-3(2*H*)-one | A | nt | M | nt | R | nt | nt | na |
| 356 | (10*R*,13*S*)-17-(2-fluoro-5-methylpyridin-3-yl)-10,13-dimethyl-6,7,8,9,10,11,12,13,14,15-decahydro-1*H-*cyclopenta[a]phenanthren-3(2*H*)-one | nt | nt | N | nt | nt | nt | nt | na |
| 326 | (10*R*,13*S*)-17-(6-fluoropyridin-3-yl)-6,10,13-trimethyl-6,7,8,9,10,11,12,13-octahydro-1*H*-cyclopenta[a]phenanthren-3(2*H*)-one | nt | nt | N | nt | nt | nt | nt | na |
| 428 | (4a*R*,6a*S*)-7-(5-fluoropyridin-3-yl)-1,4a,6a-trimethyl-4,4a,4b,5,6,6a,9,9a,9b,10-decahydro-1*H*-indeno[5,4-f]quinolin-2(3*H*)-one | nt | nt | N | nt | nt | U | nt | nt |
| 384 | (5a*R*,7a*S*)-8-(5-fluoropyridin-3-yl)-5a,7a-dimethyl-3,4,5,5a,5b,6,7,7a,11,12-decahydrocyclopenta[5,6]naphtho[1,2-d]azepin-2(10b*H*)-one | nt | nt | N | H | R | nt | nt | X |
| 279 | (3*S*,10*R*,13*S*)-17-(5-(difluoromethyl)pyridin-3-yl)-10,13-dimethyl-2,3,4,7,8,9,10,11,12,13,14,15-dodecahydro-1*H-*cyclopenta[a]phenanthren-3-ol | A | nt | M | H | s | nt | nt | nt |
| 385 | (5a*R*,7a*S*)-8-(6-fluoropyridin-3-yl)-5a,7a,12-trimethyl-3,4,5,5a,5b,6,7,7a,11,12-decahydrocyclopenta[5,6]naphtho[1,2-d]azepin-2(10b*H*)-one | nt | nt | N | nt | nt | nt | nt | na |
| 285 | (3*S*,10*R*,13*S*)-17-(4-fluoropyridin-3-yl)-10,13-dimethyl-2,3,4,7,8,9,10,11,12,13,14,15-dodecahydro-1*H-*cyclopenta[a]phenanthren-3-ol | A | nt | M | nt | R | nt | nt | nt |
| 285 | (3*S*,10*R,*13*S*)-17-(2-fluoropyridin-3-yl)-10,13-dimethyl-2,3,4,7,8,9,10,11,12,13,14,15-dodecahydro-1*H-*cyclopenta[a]phenanthren-3-ol | nt | nt | na | nt | nt | nt | nt | nt |
| 479 | (10*R*,13*S*)-17-(5-(difluoromethyl)pyridin-3-yl)-10,13-dimethyl-6,7,8,9,10,11,12,13,14,15-decahydro-1*H-*cyclopenta[a]phenanthren-3(2*H*)-one | nt | nt | N | nt | nt | nt | nt | na |
| 330 | (4a*R*,6a*S*)-7-(5-fluoropyridin-3-yl)-4a,6a-dimethyl-4,4a,4b,5,6,6a,9,9a,9b,9c,10,10a-dodecahydrocyclopenta[a]cyclopropa[1]phenanthren-2(3*H*)-one | A | nt | M | H | R | nt | nt | nt |
| 409 | (10*R*,13*S*)-17-(5-fluoropyridin-3-yl)-10,13-dimethyl-8,9,10,11,12,13,14,15-octahydro-1*H-*cyclopenta[a]phenanthren-3(2*H*)-one | A | nt | M | H | R | nt | nt | nt |
| 272 | (3*S*,10*R*,13*S*)-17-(5-(difluoromethyl)pyridin-3-yl)-10,13-dimethyl-2,3,4,7,8,9,10,11,12,13-decahydro-1*H-*cyclopenta[a]phenanthren-3-ol | nt | nt | P | L | nt | nt | nt | nt |
| 386 | (5a*R*,7a*S*)-8-(5-(difluoromethyl)pyridin-3-yl)-5a,7a-dimethyl-3,4,5,5a,5b,6,7,7a,10,10a,11,12-dodecahydrocyclopenta[5,6]naphtho[1,2-d]azepin-2(10b*H*)-one | A | nt | M | H | R | nt | nt | nt |
| 410 | (3*S*,10*R*,13*S*)-6,10,13-trimethyl-17-(pyridin-3-yl)-2,3,4,5,6,7,8,9,10,11,12,13,14,15-tetradecahydro-1*H-*cyclopenta[a]phenanthrene-3,5-diol | A | nt | M | nt | nt | nt | nt | nt |
| 411 | (10*R*,13*S*)-17-(5-(difluoromethyl)pyridin-3-yl)-10,13-dimethyl-6,7,8,9,10,11,12,13-octahydro-1*H-*cyclopenta[a]phenanthren-3(2*H*)-one | nt | nt | N | nt | nt | nt | nt | na |
| 327 | (10*R*,13*S*)-17-(5-fluoropyridin-3-yl)-6,10,13-trimethyl-6,7,8,9,10,11,12,13-octahydro-1*H*-cyclopenta[a]phenanthren-3(2*H*)-one | nt | nt | N | H | R | nt | nt | Z |
| 387 | (5a*R*,7a*S*)-5a,7a-dimethyl-8-(pyridin-3-yl)-1a,2,4,5,5a,5b,6,7,7a,10,10a,10b,11,12-tetradecahydrocyclopenta[5,6]naphtho[2,1-c]cyclopropa[b]azepin-3(1*H*)-one | nt | nt | P | nt | nt | nt | nt | na |
| 412 | (4a*R*,6a*S*)-7-(5-fluoropyridin-3-yl)-4a,6a-dimethyl-1a,2,3,4,4a,4b,5,6,6a,9,9a,9b,10,11-tetradecahydro-1*H-*cyclopenta[a]cyclopropa[j]phenanthren-2-ol | nt | nt | N | nt | nt | nt | nt | nt |
| 388 | (5a*R*,7a*S*)-8-(5-fluoropyridin-3-yl)-5a,7a-dimethyl-3,4,5,5a,5b,6,7,7a,10,10a,10b,10c,11,11a-tetradecahydro-2*H-*cyclopenta[5,6]cyclopropa[3,4]naphtho[1,2-d]azepin-2-one | A | nt | M | H | R | nt | nt | na |
| 413 | *N*-((10*R*,13*S*)4,10,13-trimethyl-17-(pyridin-3-yl)-2,3,4,5,6,7,8,9,10,11,12,13,14,15-tetradecahydro-1*H-*cyclopenta[a]phenanthren-3-yl)acetamide | nt | nt | P | nt | nt | nt | nt | nt |
| 414 | 1-ethyl-3-((10*R*,13*S*)-4,10,13-trimethyl-17-(pyridin-3-yl)-2,3,4,5,6,7,8,9,10,11,12,13,14,15-tetradecahydro-1*H-*cyclopenta[a]phenanthren-3-yl)urea | nt | nt | P | nt | nt | nt | nt | nt |
| 429 | (4a*R*,6a*S*)-7-(5-(difluoromethyl)pyridin-3-yl)-1,4a,6a-trimethyl-4,4a,4b,5,6,6a,9,9a,9b,10-decahydro-1*H-*indeno[5,4-f]quinolin-2(3*H*)-one | nt | nt | P | nt | nt | nt | nt | nt |
| 480 | *N*-((3R,10*R*,13*S*)-17-(5-(difluoromethyl)pyridin-3-yl)-10,13-dimethyl-2,3,4,7,8,9,10,11,12,13,14,15-dodecahydro-1*H-*cyclopenta[a]phenanthren-3-yl)acetamide | A | nt | M | nt | R | nt | nt | nt |
| 415 | (10*R*,13*S*)-2,4,10,13-tetramethyl-17-(pyridin-3-yl)-4,5,6,7,8,9,10,11,12,13,14,15-dodecahydro-1*H-*cyclopenta[a]phenanthren-3(2*H*)-one | nt | nt | na | nt | nt | nt | nt | nt |
| 389 | (5a*R*,7a*S*)-8-(5-fluoropyridin-3-yl)-5a,7a,12-trimethyl-3,4,5,5a,5b,6,7,7a,11,12-decahydrocyclopenta[5,6]naphtho[1,2-d]azepin-2(10b*H*)-one | nt | nt | N | nt | R | nt | nt | Y |
| 416 | (10*R*,13*S*)-2,4,10,13-tetramethyl-17-(pyridin-3-yl)-2,3,4,5,6,7,8,9,10,11,12,13,14,15-tetradecahydro-1*H-*cyclopenta[a]phenanthren-3-ol | nt | nt | N | nt | nt | nt | nt | nt |
| 430 | (4a*R*,6a*S*)-7-(4-fluoropyridin-3-yl)-1,4a,6a-trimethyl-4,4a,4b,5,6,6a,9,9a,9b,10-decahydro-1*H*-indeno[5,4-f]quinolin-2(3H)-one | nt | nt | N | nt | nt | nt | nt | nt |
| 282 | 1,1,1-trifluoro-*N*-(5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)pyridin-3-yl)methanesulfonamide | nt | nt | P | nt | nt | nt | nt | nt |
| 283 | (3*S*,10*R*,13*S*)-17-(5-aminopyridin-3-yl)-10,13-dimethyl-2,3,4,7,8,9,10,11,12,13,14,15-dodecahydro-1*H-*cyclopenta[a]phenanthren-3-ol | A | nt | M | nt | s | nt | nt | nt |
| 331 | (10*R*,13*S*)-6-cyclopropyl-17-(5-fluoropyridin-3-yl)-10,13-dimethyl-6,7,8,9,10,11,12,13,14,15-decahydro-1*H-*cyclopenta[a]phenanthren-3(2*H*)-one | nt | nt | N | nt | nt | nt | nt | na |
| 390 | (5a*R*,7a*S*)-8-(5-(difluoromethyl)pyridin-3-yl)-5a,7a-dimethyl-3,4,5,5a5b,6,7,7a,11,12-decahydrocyclopenta[5,6]naphtho[1,2-d]azepin-2(10b*H)*-one | A | nt | M | nt | nt | nt | nt | na |
| 280 | (3S,10*R*,13*S*)-17-(5-chloropyridin-3-yl)-10,13-dimethyl-2,3,4,7,8,9,10,11,12,13,14,15-dodecahydro-1*H-*cyclopenta[a]phenanthren-3-ol | A | nt | M | nt | R | nt | nt | nt |
| 333 | (10*R*,13*S*)-6-ethyl-17-(5-fluoropyridin-3-yl)-10,13-dimethyl-6,7,8,9,10,11,12,13,14,15-decahydro-1*H-*cyclopenta[a]phenanthren-3(2*H*)-one | nt | nt | na | nt | nt | nt | nt | na |
| 332 | (10*R*,13,*S*)-6-ethyl-10,13-dimethyl-17-(pyridin-3-yl)-6,7,8,9,10,11,12,13,14,15-decahydro-1*H-*cyclopenta[a]phenanthren-3(2*H*)-one | nt | nt | N | nt | nt | nt | nt | Y |
| 334 | (10*R*,13*S*)-17-(5-(difluoromethyl)pyridin-3-yl)-6-ethyl-10,13-dimethyl-6,7,8,9,10,11,12,13,14,15-decahydro-1*H-*cyclopenta[a]phenanthren-3(2*H*)-one | nt | nt | na | nt | nt | nt | nt | na |
| 328 | (10*R*,13*S*)-17-(5-(difluoromethyl)pyridin-3-yl)-6,10,13-trimethyl-6,7,8,9,10,11,12,13-octahydro-1*H-*cyclopenta[a]phenanthren-3(2*H*)-one | nt | nt | P | nt | nt | nt | nt | na |
| 335 | (10*R*,13*S*)-10,13-dimethyl-17-(pyridin-3-yl)-6-(1*H*-1,2,3-triazol-1-yl)-6,7,8,9,10,11,12,13,14,15-decahydro-1*H-*cyclopenta[a]phenanthren-3(2*H*)-one | nt | nt | N | nt | nt | nt | nt | na |
| 305 | (10*R,*13*S*)-17-(5-fluoropyridin-3-yl)-4,10,13-trimethyl-2,3,4,5,6,7,8,9,10,11,12,13,14,15-tetradecahydro-1*H-*cyclopenta[a]phenanthren-3-ol | nt | nt | P | nt | nt | nt | nt | nt |
| 273 | (3*S,*10*R,*13*S*)-17-(5-chloropyridin-3-yl)-10,13-dimethyl-2,3,4,7,8,9,10,11,12,13-decahydro-1*H-*cyclopenta[a]phenanthren-3-ol | nt | nt | N | nt | nt | nt | nt | nt |
| 391 | (5a*R*,7a*S*)-8-(5-(difluoromethyl)pyridin-3-yl)-5a,7a,12-trimethyl-3,4,5,5a,5b,6,7,7a,11,12-decahydrocyclopenta[5,6]naphtho[1,2-d]azepin-2(10b*H*)-one | nt | nt | P | nt | nt | nt | nt | na |
| 307 | (3*S*,4*S*,10*R*,13*S*)-17-(5-(difluoromethyl)pyridin-3-yl)-4,10,13-trimethyl-2,3,4,5,6,7,8,9,10,11,12,13,14,15-tetradecahydro-1*H*-cyclopenta[a]phenanthren-3-ol | nt | nt | N | nt | nt | nt | nt | nt |
| 306 | (10*R*,13*S*)-17-(5-(difluoromethyl)pyndin-3-yl)-4,10,13-trimethyl-2,3,4,5,6,7,8,9,10,11,12,13,14,15-tetradecahydro-1*H*-cyclopenta[a]phenanthren-3-ol | nt | nt | P | nt | nt | nt | nt | nt |
| 308 | (3*R*,4*S*,10*R*,13*S*)-17-(5-(difluoromethyl)pyridin-3-yl)-4,10,13-trimethyl-2,3,4,5,6,7,8,9,10,11,12,13,14,15-tetradecahydro-1*H*-cyclopenta[a]phenanthren-3-ol | nt | nt | P | nt | nt | nt | nt | nt |
| 336 | (10*R*,13*S*)-10,13-dimethyl-17-(pyridin-3-yl)-6-(2*H*-1,2,3-triazol-2-yl)-6,7,8,9,10,11,12,13,14,15-decahydro-1*H-*cyclopenta[a]phenanthren-3(2*H*)-one | nt | nt | na | nt | nt | nt | nt | na |
| 433 | 1-((4a*R*,6a*S*)-1,4a,6a-trimethyl-2-oxo-2,3,4,4a,4b,5,6,6a,9,9a,9b,10-dodecahydro-1*H*-indeno[5,4-f]quinolin-7-yl)-1*H*-imidazole-5-carbonitrile | nt | nt | P | nt | nt | T | nt | nt |
| 337 | (10*R*,13*S*)-17-(5-fluoropyridin-3-yl)-10,13-dimethyl-6-phenyl-6,7,8,9,10,11,12,13,14,15-decahydro-1*H-*cyclopenta[a]phenanthren-3(2*H*)-one | nt | nt | na | nt | nt | nt | nt | na |
| 339 | (10*R*,13*S*)-6-cyclopropyl-10,13-dimethyl-17-(pyridin-3-yl)-6,7,8,9,10,11,12,13,14,15-decahydro-1*H-*cyclopenta[a]phenanthren-3(2*H*)-one | nt | nt | P | nt | nt | nt | nt | na |
| 340 | (10*R*,13*S*)-6-(hydroxymethyl)-10,13-dimethyl-17-(pyridin-3-yl)-6,7,8,9,10,11,12,13-octahydro-1*H-*cyclopenta[a]phenanthren-3(2*H*)-one | A | nt | M | nt | nt | nt | nt | X |
| 338 | (10*R*,13*S*)-10,13-dimethyl-6-phenyl-17-(pyridin-3-yl)-6,7,8,9,10,11,12,13,14,15-decahydro-1*H* cyclopenta[a]phenanthren-3(2*H*)-one | nt | nt | P | nt | nt | nt | nt | na |
| 398 | (3a*S*,5a*S*)-6-(5-fluoropyridin-3-yl)-3a,5a-dimethyl-1,2,3,3a,3b,4,5,5a,8,8a,8b,9,10,10a-tetradecahydrodicyclopenta[a,f]naphthalen-2-ol | A | nt | N | H | S | nt | nt | nt |
| 399 | (3a*S*,5a*S*)-3a,5a-dimethyl-6-(pyridin-3-yl)-1,2,3,3a,3b,4,5,5a,8,8a,8b,9,10,10a-tetradecahydrodicyclopenta[a,f]naphthlen-2-ol | A | nt | M | H | R | nt | nt | nt |
| 284 | *N*-(5-((3*S*,10*R*,13*S*)-3-hydroxy-10,13-dimethyl-2,3,4,7,8,9,10,11,12,13,14,15-dodecahydro-1H-cyclopenta[a]phenanthren-17-yl)pyridin-3-yl)methanesulfonamide | nt | nt | P | nt | nt | nt | nt | nt |
| 397 | (3a*S*,5a*S*)-6-(5-chloropyridin-3-yl)-3a,5a-dimethyl-1,2,3,3a,3b,4,5,5a,8,8a,8b,9,10,10a-tetradecahydrodicyclopenta[a,f]naphthalen-2-ol | nt | nt | N | nt | nt | nt | nt | nt |
| 265 | (3*S*,10*S*,13*S*)-17-(5-chloropyridin-3-yl)-3,10,13-trimethyl-2,3,4,5,6,7,8,9,10,11,12,13,14,15-tetradecahydro-1*H-*cyclopenta[a]phenanthren-3-ol | nt | nt | na | nt | nt | nt | nt | nt |
| 266 | (3*R*,10*S*,13*S*)-17-(5-chloropyridin-3-yl)-3,10,13-trimethyl-2,3,4,5,6,7,8,9,10,11,12,13,14,15-tetradecahydro-1*H-*cyclopenta[a]phenanthren-3-ol | nt | nt | N | nt | nt | nt | nt | nt |
| 341 | (10*R*,13*S*)-17-(5-(difluoromethyl)pyridin-3-yl)-6,10,13-trimethyl-6,7, 8,9,10,11,12,13,14,15-decahydro-1*H-*cyclopenta[a]phenanthren-3(2*H*)-one | nt | nt | N | nt | nt | nt | nt | na |
| 342 | (10*R*,13*S*)-17-(5-fluoropyridin-3-yl)-6,10,13-trimethyl-6,7,8,9,10,11,12,13,14,15-decahydro-1*H-*cyclopenta[a]phenanthren-3(2*H*)-one | A | nt | M | H | nt | nt | nt | X |
| 481 | (5a*S*,7a*S*)-8-(5-fluoropyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one | nt | nt | N | nt | nt | nt | nt | nt |
| 343 | (10*R*,13*S*)-6-cyclopropyl-10,13-dimethyl-17-(pyridin-3-yl)-6,7,8,9,10,11,12,13-octahydro-1*H*-cyclopenta[a]phenanthren-3(2*H*)-one | nt | nt | N | nt | nt | nt | nt | X |
| 344 | (10*R*,13*S*)-10,13-dimethyl-6-phenyl-17-(pyridin-3-yl)-6,7,8,9,10,11,12,13-octahydro-1*H*-cyclopenta[a]phenanthren-3(2*H*)-one | nt | nt | N | nt | nt | nt | nt | na |
| 359 | (10*R*,13*S*)-17-(5-chloropyridin-3-yl)-10,13-dimethyl-6,7,8,9,10,11,12,13-octahydro-1*H*-cyclopenta[a]phenanthren-3(2*H*)-one | nt | nt | N | nt | nt | nt | nt | na |
| 345 | (10*R*,13*S*)-17-(5-fluoropyridin-3-yl)-6,10,13-trimethyl-6,7,8,9,10,11,12,13,14,15-decahydro-3*H-*cyclopenta[a]phenanthren-3-one | A | nt | M | nt | nt | nt | nt | Y |
| 346 | (10*R-*13*S*)-17-(5-(difluoromethyl)pyridin-3-yl)-6,10,13-trimethyl-6,7,8,9,10,11,12,13,14,15-decahydro-3*H-*cyclopenta[a]phenanthren-3-one | nt | nt | N | nt | nt | nt | nt | na |
| 482 | (10*R*,13*S*)-6-ethyl-10,13-dimethyl-17-(pyridin-3-yl)-6,7,8,9,10,11,12,13-octahydro-1*H*-cyclopenta[a]phenanthren-3(2*H*)-one | A | nt | N | nt | R | nt | nt | Y |
| 417 | (10*R*,13*S*)-17-(5-fluoropyridin-3-yl)-10,13-dimethyl-7,8,9,11,12,13,14,15-octahydro-1*H-*cyclopenta[a]phenanthrene-3,6(2*H*,10*H*)-dione | A | nt | M | nt | nt | nt | nt | na |
| 418 | (10*R*,13*S*)-17-(5-fluoropyridin-3-yl)-10,13-dimethyl-4,5,7,8,9,11,12,13,14,15-decahydro-1*H-*cyclopenta[a]phenanthrene-3,6(2*H*,10*H*)-dione | A | nt | M | nt | nt | nt | nt | na |
| 483 | (5a*S*,7a*S*)-8-(5-(difluoromethyl)pyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one | nt | nt | M | nt | nt | nt | nt | nt |
| 437 | (5a*R*,7a*S*)-8-(1*H*-imidazol-1-yl)-5a,7a-dimethyl-2-(methylsulfonyl)-1,2,3,4,5,5a,5b,6,7,7a,10,10a,10b,11,12,12a-hexadecahydrocyclopenta[5,6]naphtho[2,1-c]azepine | na | nt | nt | nt | nt | U | | nt |
| 329 | (10*R*,13*S*)-17-(5-aminopyridin-3-yl)-6,10,13-trimethyl-6,7,8,9,10,11,12,13-octahydro-1*H*-cyclopenta[a]phenanthren-3(2*H*)-one | A | nt | nt | nt | nt | nt | V | nt |
| 360 | (10*R*,13*S*)-17-(5-aminopyridin-3-yl)-10,13-dimethyl-6,7,8,9,10,11,12,13-octahydro-1*H*-cyclopenta[a]phenanthren-3(2*H*)-one | A | nt | nt | nt | nt | nt | na | nt |
| 402 | 3-((10*S*,13*S*-3,3-difluoro-10,13-dimethyl-2,3,4,5,6,7,8,9,10,11,12,13-dodecahydro-1*H-*cyclopenta[a]phenanthren-17-yl)-5-fluoropyridine | na | nt | nt | nt | nt | nt | nt | nt |
| 403 | 3-((10*S*,13*S*)-3,3-difluoro-10,13-dimethyl-2,3,4,5,6,7,8,9,10,11,12,13-dodecahydro-1*H-*cyclopenta[a]phenanthren-17-yl)pyridine | A | nt | nt | nt | nt | nt | nt | nt |
| 404 | 3-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)pyridine | A | nt | nt | nt | nt | nt | nt | nt |
| 405 | 3-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)-5-fluoropyridine | na | nt | nt | nt | nt | nt | nt | nt |
| 392 | (5a*R*,7a*S*)-8-(5-fluoropyridin-3-yl)-3,5a,7a-trimethyl-3,4,5,5a,5b,6,7,7a,11,12-decahydrocyclopenta[5,6]naphtho[1,2-d]azepin-2(10b*H*)-one | nt | nt | nt | nt | nt | nt | nt | X |
| 348 | (10*R*,13*S*)-10,13-dimethyl-6-methylene-17-(pyridin-3-yl)-6,7,8,9,10,11,12,13-octahydro-1*H*-cyclopenta[a]phenanthren-3(2*H*)-one | nt | nt | nt | nt | nt | nt | nt | Y |
| 349 | (10*R*,13*S*)-6,10,13-trimethyl-17-(pyridin-3-yl)-6,7,8,9,10,11,12,13-octahydro-3*H*-cyclopenta[a]phenanthren-3-one | nt | C | M | nt | R | nt | nt | z |
| 407 | 3-((8a*S*,10a*S*)-7,7-difluoro-8a,10a-dimethyl-3,3a,3b,4,5,5a,6,7,8,8a,8b,9,10,10a-tetradecahydrodicyclopenta[a,f]naphthalen-1-yl)pyridine | nt | nt | N | nt | nt | nt | nt | nt |
| 400 | (3a*S*,5a*S*)-2,3a,5a-trimethyl-6-(pyridin-3-yl)-1,2,3,3a,3b,4,5,5a,8,8a,8b,9,10,10a-tetradecahydrodicyclopenta[a,f]naphthalen-2-ol | nt | nt | N | nt | nt | nt | nt | nt |
| 484 | (5a*R*,5b*S*,7a*S*,10a*S*,10b*R*,12a*R*)-5a,7a-dimethyl-8-(4-methylpyridin-3-yl)-1,2,5,5a,5b,6,7,7a,10,10a,10b,11,12,12a-tetradecahydrocyclopenta[5,6]naphtho[2,1-c]azepin-3(4*H*)-one | nt | nt | N | nt | nt | nt | nt | nt |
| 193 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl propionate | nt | nt | N | nt | nt | nt | nt | nt |
| 218 | *N-*((3*R,*10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)pivalamide | nt | nt | N | nt | nt | nt | nt | nt |
| 485 | (3*S*,10*S*,13*S*)-10,13-dimethyl-17-(4-methylpyridin-3-yl)-2,3,4,5,6,7,8,9,10,11,12,13-dodecahydro-1*H-*cyclopenta[a]phenanthren-3-ol | nt | nt | N | nt | nt | nt | nt | nt |
| 486 | (10*S*,13*S*)-10,13-dimethyl-17-(4-methylpyridin-3-yl)-4,5,6,7,8,9,10,11,12,13-decahydro-1*H-*cyclopenta[a]phenanthren-3(2*H*)-one | nt | nt | N | nt | nt | nt | nt | nt |
| 487 | (3*S*,10*S*,13*S*)-17-(4-ethylpyridin-3-yl)-10,13-dimethyl-2,3,4,5,6,7,8,9,10,11,12,13-dodecahydro-1*H* cyclopenta[a]phenanthren-3-yl acetate | nt | nt | N | nt | nt | nt | nt | nt |
| 488 | (10*S*,13*S*)-17-(4-ethylpyridin-3-yl)-10,13-dimethyl-4,5,6,7,8,9,10,11,12,13-decahydro-1*H-*cyclopenta[a]phenanthren-3(2*H*)-one | nt | nt | N | nt | nt | nt | nt | nt |
| 194 | (3*S*,10*R*,13*S*)-10,13-dimethyl-17-(pyridin-3-yl)-2,3,4,7,8,9,10,11,12,13-decahydro-1*H-*cyclopenta[a]phenanthren-3-yl benzoate | nt | nt | N | nt | nt | nt | nt | nt |
| 281 | *N-*(5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)pyridin-3-yl)acetamide | nt | nt | P | nt | nt | nt | nt | nt |
| 411 | (3*S*,10*R*,13*S*)-17-(5-fluoropyridin-3-yl)-6,10,13-trimethyl-2,3,4,5,6,7,8,9,10,11,12,13,14,15-tetradecahydro-1*H-*cyclopenta[a]phenanthrene-3,5-diol | nt | nt | N | nt | nt | nt | nt | nt |
| 267 | (3*R*,10*R*,13*S*)-3,10,13-trimethyl-17-(pyridin-3-yl)-2,3,4,7,8,9,10,11,12,13,14,15-dodecahydro-1*H-*cyclopenta[a]phenanthren-3-ol | nt | nt | M | nt | nt | nt | nt | nt |
| 268 | (3*S*,10*R*,13*S*)-3,10,13-trimethyl-17-(pyridin-3-yl)-2,3,4,7,8,9,10,11,12,13,14,15-dodecahydro-1*H-*cyclopenta[a]phenanthren-3-ol | nt | nt | M | nt | nt | nt | nt | nt |
| 269 | (3*R*,10*R*,13*S*)-17-(5-fluoropyridin-3-yl)-3,10,13-trimethyl-2,3,4,7,8,9,10,11,12,13,14,15-dodecahydro-1*H-*cyclopenta[a]phenanthren-3-ol | nt | nt | M | nt | s | nt | nt | nt |
| 270 | (3*S-*10*R*,13*S*)-17-(5-fluoropyridin-3-yl)-3,10,13-trimethyl-2,3,4,7,8,9,10,11,12,13,14,15-dodecahydro-1*H-*cyclopenta[a]phenanthren-3-ol | nt | nt | N | nt | nt | nt | nt | nt |
| 406 | 1-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)-1*H*-imidazole | A | nt | nt | nt | nt | nt | nt | nt |
| 489 | (5a*S*,7a*S*)-8-(1*H*-imidazol-1-yl)-5a,7a-dimethyl-3-(methylsulfonyl)-1,2,3,4,5,5a,5b,6,7,7a,10b,11,12,12a-tetradecahydrocyclopenta[5,6]naphtho[1,2-d]azepine | nt | nt | nt | nt | nt | nt | nt | na |
| 350 | (10*R*,13*S*)-10,13-dimethyl-6-methylene-17-(pyridin-3-yl)-6,7,8,9,10,11,12,13-octahydro-3*H*-cyclopenta[a]phenanthren-3-one | nt | nt | nt | nt | R | nt | nt | Z |
| 208 | (3*S*,10*R*,13*S*)-17-(5-fluoropyridin-3-yl)-10,13-dimethyl-2,3,4,7,8,9,10,11,12,13-decahydro-1*H-*cyclopenta[a]phenanthren-3-yl 1-methylpiperidine-4-carboxylate | nt | nt | nt | nt | nt | nt | nt | nt |
| 393 | (5a*R*,7a*S*)-3,5a,7a,12-tetramethyl-8-(pyridin-3-yl)-3,4,5,5a,5b,6,7,7a,11,12-decahydrocyclopenta[5,6]naphtho[1,2-d]azepin-2(10b*H*)-one | nt | nt | nt | nt | nt | nt | nt | Y |
| 395 | (5a*R*,7a*S*)-5a,7a,12-trimethyl-8-(pyridin-3-yl)-3,4,5,5a,5b,6,7,7a,8,9,10,10a,11,12-tetradecahydrocyclopenta[5,6]naphtho[1,2-d]azepin-2(10b*H*)-one | nt | nt | nt | nt | R | nt | nt | Z |
| 394 | (5a*R*,7a*S*)-8-(5-fluoropyridin-3-yl)-3,5a,7a,12-tetramethyl-3,4,5,5a,5b,6,7,7a,11,12-decahydrocyclopenta[5,6]naphtho[1,2-d]azepin-2(10b*H*)-one | nt | nt | nt | nt | nt | nt | nt | x |
| 438 | (5a*S*,7a*S*)-8-(1*H*-imidazol-1-yl)-5a,7a-dimethyl-3-(methylsulfonyl)-1,2,3,4,5,5a,5b,6,7,7a,10,10a,10b,11,12,12a-hexadecahydrocyclopenta[5,6]naphtho[1,2-d]azepine | nt | nt | nt | nt | nt | nt | nt | na |
| 420 | (10*R*,13*S*)-6,10,13-trimethyl-17-(pyridin-3-yl)-6,7,8,9,10,11,12,13,14,15,16,17-dodecahydro-1*H-*cyclopenta[a]phenanthren-3(2*H*)-one | nt | nt | nt | nt | R | nt | nt | Z |
| 351 | (10*R*,13*S*)-10,13-dimethyl-17-(pyridin-3-yl)-1,7,8,9,10,11,12,13-octahydrospiro[cyclopenta[a]phenanthrene-6,1'-cyclopropan]-3(2*H*)-one | nt | nt | nt | nt | nt | nt | nt | z |
| 421 | (10*R*,13*S*)-6,10,13-trimethyl-17-(pyridin-3-yl)-6,7,8,9,10,11,12,13,14,15,16,17-dodecahydro-3*H-*cyclopenta[a]phenanthren-3-one | A1 | nt | nt | nt | R | nt | nt | z |
| 353 | (10*R*,13*S*)-17-(5-fluoropyridin-3-yl)-10,13-dimethyl-6-methylene-6,7,8,9,10,11,12,13-octahydro-1*H-*cyclopenta[a]phenanthren-3(2*H*)-one | nt | nt | nt | nt | nt | nt | nt | Y |
| 354 | (10*R*,13*S*)-17-(5-fluoropyridin-3-yl)-10,13-dimethyl-1,7,8,9,10,11,12,13-octahydrospiro[cyclopenta[a]phenanthrene-6,1'-cyclopropan]-3(2*H*)-one | nt | nt | nt | nt | nt | nt | nt | Y |
| 396 | (5a*R*,7a*S*)-3,5a,7a-trimethyl-8-(pyridin-3-yl)-3,4,5,5a,5b,6,7,7a,10b,10c,11,11a-dodecahydro-2*H-*cyclopenta[5,6]cyclopropa[3,4]naphtho[1,2-d]azepin-2-one | nt | nt | nt | nt | nt | nt | nt | Y |
| 431 | 1-((5a*R,*7a*S*)-5a,7a-dimethyl-2-(methylsulfonyl)-1,2,3,4,5,5a,5b,6,7,7a,10,10a,10b,11,12,12a-hexadecahydrocyclopenta[5,6]naphtho[2,1-c]azepin-8-yl)-1*H-*imidazole-5-carbonitrile | nt | nt | nt | nt | nt | nt | nt | na |
| 432 | 1-((5a*S*,7a*S*)-5a,7a-dimethyl-3-(methylsulfonyl)-1,2,3,4,5,5a,5b,6,7,7a,10,10a,10b,11,12,12a-hexadecahydrocyclopenta[5,6]naphtho[1,2-d]azepin-8-yl)-1*H*-imidazole-5-carbonitrile | nt | nt | nt | nt | nt | nt | nt | na |
| 490 | (10*R*,13*S*)-17-(5-fluoropyridin-3-yl)-10,13-dimethyl-7,8,9,11,12,13-hexahydrospiro[cyclopenta[a]phenanthrene-6,1'-cyclopropan]-3(10*H*)-one | nt | nt | nt | nt | nt | nt | nt | nt |

**Table 4. Compounds**

| **Cmpd No.** | **Name** | **Biological Example No.** | | | | | | | |
|---|---|---|---|---|---|---|---|---|---|
| | | **4** | **5** | **12** | **7** | **8b** | **9** | **10** | **11** |
| A | (3*S*,10*R*,13*S*)-17-(1*H*-imidazol-1-yl)-10,13-dimethyl-2,3,4,7,8,9,10,11,12,13-decahydro-1*H-*cyclopenta[a]phenanthren-3-ol | A | C | nt | nt | S | nt | nt | nt |
| B | (10*R*,13*S*)-10,13-dimethyl-17-(pyridin-3-yl)-6,7,8,9,10,11,12,13,14,15-decahydro-1*H-*cyclopenta[a]phenanthren-3(2*H*)-one | nt | nt | M | nt | nt | nt | W | nt |
| C | (3S,8R,9S,10R,13S,14S)-10,13-dimethyl-17-(pyridin-3-yl)-2,3,4,7,8,9,10,11,12,13,14,15-dodecahydro-1H-cyclopenta[a]phenanthren-3-ol | A | C | M | H | R | T | | |
| D | (10R,13S)-6-fluoro-10,13-dimethyl-17-(pyridin-3-yl)-6,7,8,9,10,11,12,13,14,15-decahydro-1H-cyclopenta[a]phenanthren-3(2H)-one | A | nt | M | H | R | nt | nt | na |

### Biological Example 13: In vivo Antitumor Studies (LAPC-4 Prostate Cancer Xenografts)

All animal studies are performed according to the guidelines and approval of the Animal Care Committee of the testing facility.

Male sever combined immunodeficient (SCID) mice 4-6 weeks of age are purchased, for example, from the National Cancer Institute-Frederick Cancer Research and Development Center and housed in a pathogen-free environment under controlled conditions of light and humidity and allowed free access to food and water. Tumors are developed from LAPC-4 cells inoculated subcutaneously (s.c.) in the mice. LAPC-4 cells are grown in IMEM with 15% FBS plus 1% PS and 10 nm DHT until 80% confluent. Cells are scraped into DPBS, collected by centrifugation, and resuspended in Matrigel (10 mg/ml) at 3 x 10⁷ cells/ml. Mice are injected s.c. with 100 µl of the cell suspension at one site on each flank. Tumors will be measured weekly with calipers, and tumor volumes will be calculated by the formula: 4/3Π x r₁² x r₂ (r₁<r₂).

LAPC-4 tumors are allowed to grow for 8-10 weeks following inoculation. Groups of 5 mice with comparable total tumor volumes are either castrated under methoxyfluorane anesthesia or treated with a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) (about 0.15 mmol/kg once-daily and 0.15 mmol/kg twice-daily). A compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) is prepared at about 17 mg/ml in about a 0.3% solution of hydroxypropyl cellulose in saline, and mice receive s.c. injections daily. Control and castrated mice are treated with vehicle only. Tumors are measured weekly for the 4 weeks of treatment and tumor volumes are calculated. At the end of the treatment period, the mice are sacrificed under halothane anesthesia; the tumors are excised, weighed and stored at -80 °C. The mice arealso weighed weekly and monitored for general health status and signs of possible toxicity due to treatment.

### Human Clinical Trial of the Safety and Efficacy of Compounds of Formulas (I) - (III)

Objective: To evaluate the safety, pharmacokinetics, pharmacodynamics, and anti-tumor activities of an oral CYP11B, CYP17, and/or CYP21 inhibitor, a compound having the structure of Formula (I), (II), (III), (IV), (V), (VI), (VII), (VIII), (IA), (IB), (IC), (ID), (IE), (IF), (IG), (IIA), (IIB), (IIC), (IIID), (IIIA), (IIIB), (IIIC), (IIID), (IVA), (IVB), (IVC), (IVD), (IVE), (VA), (VB), (VC) or (VD) administered to patients with hormone refractory prostate cancer (HRPC).

Patients: Eligible subjects will be men 18 years and older.

Inclusion criteria for Phase I will include:
- Histologically confirmed adenocarcinoma of the prostate;
- No prior therapy with chemotherapy for prostate cancer;
- Ongoing gonadal androgen deprivation therapy with LHRH analogues or orchiectomy. Patients, who have not had an orchiectomy will be maintained on effective LHRH analogue therapy for the duration of the trial;
- Testosterone < 50 ng/dL;
- Progressive disease after androgen deprivation: PSA evidence for progressive prostate cancer consists of a PSA level of at least 5 ng/ml which has risen on at least 2 successive occasions, at least 2 weeks apart. If the confirmatory PSA value is less than the screening PSA value, then an additional test for rising PSA will be required to document progression;
- Antiandrogen Withdrawal Patients who are receiving an antiandrogen as part of primary androgen ablation must demonstrate disease progression following discontinuation of antiandrogen. Disease progression after antiandrogen withdrawal is defined as 2 consecutive rising PSA values, obtained at least 2 weeks apart, or documented osseous or soft tissue progression.
   o For patients receiving flutamide, at least one of the PSA values must be obtained 4 weeks or more after flutamide discontinuation;
   o For patients receiving bicalutamide or nilutamide, at least one of the PSA values must be obtained 6 weeks or more after antiandrogen discontinuation;
- ECOG Performance Status 0-1;
- Serum Creatinine ≤ 1.5 x ULN;
- K+ more than or equal to 3.5mmol/L;
- Bilirubin ≤1.5xULN;
- AST and ALT ≤ 2.5 x ULN;
- Systolic blood pressure < 160 mmHg and diastolic blood pressure < 110mmHg documented on at least 3 different days;
- Baseline ACTH stimulation test demonstrating a peak cortisol >18 µg/dL; and
- Life expectancy of more than or equal to 12 weeks.

Exclusion criteria for Phase I will include:
- Therapy with other hormonal therapy, including any dose of megestrol acetate (Megace), finasteride (Proscar), dutasteride (Avodart) any herbal product known to decrease PSA levels (e.g., Saw Palmetto and PC-SPES), or any systemic corticosteroid within 4 weeks prior to first dose of study drug;
- Initiation of bisphosphonate therapy within 4 weeks prior to first dose of study drug. Patients on stable doses of bisphosphonates that show subsequent tumor progression may continue on the medication; however, patients will not be allowed to initiate bisphosphonate therapy during the study;
- Therapy with supplements or complementary medicines/botanicals within 4 weeks of first dose of study drug, except for any combination of the following:
   o conventional multivitamin supplements;
   o selenium;
   o lycopene;
   o soy supplements;
- Prior radiation therapy completed < 4 weeks prior to enrollment;
- Prior chemotherapy for hormone refractory prostate cancer;
- Hemoglobin less than or equal to 9.0 g/dL;
- ANC less than or equal to 1.5 x 109/L;
- Platelets less than or equal to 100 x 109/L;
- Any "currently active" second malignancy, other than non-melanoma skin cancer. Patients will not considered to have a "currently active" malignancy, if they have completed therapy and are considered by their physician to be at least less than 30% risk of relapse over next 3 months;
- Systolic blood pressure more than or equal to 160 mmHg or diastolic blood pressure more than or equal to 110 mmHg measured on at least 2 occasions;
- Serum K+ <3.5 mmoL/L;
- NYHA Class III or IV Congestive Heart Failure;
- Myocardial infarction within the 6 months prior to the first dose of study drug;
- Serious intercurrent infections or nonmalignant medical illnesses that are uncontrolled;
- Active psychiatric illnesses/social situations that would limit compliance with protocol requirements; and
- Active or uncontrolled autoimmune disease that may require corticosteroid therapy during study.

Inclusion criteria for Phase II will include the same criteria for Phase I with the following additions:
- Neoadjuvant or adjuvant chemotherapy is only allowed if the last dose is > 1 year from Cycle 1 Day 1;
- Target or Non-Target abnormalities must be present either on screening bone scan, CT or MRI; and
- No prior treatment with ketoconazole for the management of androgen independent prostate cancer.

Exclusion criteria for Phase II will include the same criteria as Phase I with the following addition:
- Abnormal electrocardiogram, including any finding which would interfere with assessment of intervals (patients with long QT syndrome, bundle branch blocks or hemiblocks will be prohibited).

Study Design: This will be a Phase I/II, non-randomized, open label dose escalation, single group assignment clinical trial of an oral compound of Formulas (I) - (III).

Primary Outcome Measures: Phase I: To determine maximum tolerated dose of a compound having the structure of Formula (I), (II) or (III) administered orally on a continuous once-daily schedule in patients with HRPC. Phase II: To assess proportion of patients achieving a >50% PSA decline during therapy with concurrent prednisone.

Secondary Outcome Measures: Phase I: 1. Safety/tolerability; 2. Pharmacokinetics; 3. Pharmacodynamics; 4. Need for steroids; 5. Preliminary anti-tumor activities. Phase II: 1. To assess safety and tolerability of a compound having the structure of Formula (I), (II) or (III) with concurrent prednisone; 2. Additional parameters for anti-tumor activity and clinical benefits.

Arms: Experimental-Phase I: A compound having the structure of Formula (I), (II) or (III); Phase II: A compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) and (VD) and prednisone.

Assigned Interventions: Drug: A compound having the structure of Formula (I), (II), (III), (IV), (V), (VI), (VII), (VIII), (IA), (IB), (IC), (ID), (IE), (IF), (IG), (IIA), (IIB), (IIC), (IID), (IIA), (IIIB), (IIIC), (IIID), (IVA), (IVB), (IVC), (IVD), (IVE), (VA), (VB), (VC) or (VD)) - Phase I: Dose escalating; Phase II: 1000 mg of a compound having the structure of Formula (I), (II), (III), (IV), (V), (VI), (VII), (VIII), (IA), (IB), (IC), (ID), (IE), (IF), (IG), (IIA), (IIB), (IIC), (IID), (IIIA), (IIIB), (IIIC), (IIID), (IVA), (IVB), (IVC), (IVD), (IVE), (VA), (VB), (VC) or (VD) PO daily and 5 mg of prednisone PO bid.

The examples and embodiments described herein are for illustrative purposes only and various modifications or changes suggested to persons skilled in the art are to be included within the spirit and purview of this application and scope of the appended claims.

The invention will now be described in further detail in the following numbered paragraphs:
1. A compound of Formula IX 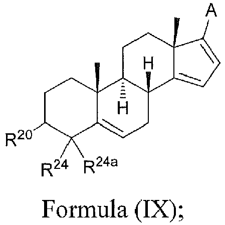 where
   A is a heteroaryl optionally substituted with 1, 2, 3, or 4 R⁴;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R²⁰ is arylcarbonyloxy, heterocycloalkylcarbonyloxy, heteroarylcarbonyloxy, -OC(O)NR²¹R^{21a}, or -OC(O)-alkylene-NR²¹R^{21a}; where the aryl, heterocycloalkyl, and heteroaryl are independently optionally substituted with one or two alkyl;
   R²¹ is hydrogen or alkyl;
   R^{21a} is hydrogen, alkyl, or heteroaryl;
   R²⁴ and R^{24a} are independently hydrogen or alkyl; and
   provided that when R²⁰ is phenylcarbonyloxy, then A is not unsubstituted pyridinyl or pyridinyl substituted with one alkyl; or
   a single stereoisomer or tautomer or mixture thereof, optionally as a pharmaceutically acceptable salt or solvate thereof.
2. A Compound of Formula X 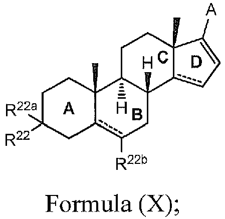 where
   one 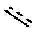 is a single bond and the other is a double bond or both are single bonds;
   A is heteroaryl optionally substituted with 1, 2, 3, or 4 R⁴;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R²² is halo or hydroxy; and R^{22a} is halo, alkyl, hydroxyalkyl, alkynyl, or cycloalkyl; or R²² and R^{22a} together with the carbon to which they are attached form heterocycloalkyl; and
   R^{22b} is hydrogen or alkyl; or
   a single stereoisomer or tautomer or mixture thereof, optionally as a pharmaceutically acceptable salt or solvate thereof.
3. A Compound of Formula XI 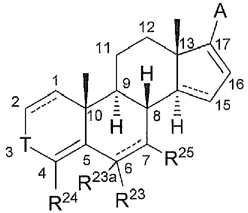 where
   each 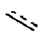 is independently a single or double bond;
   T is C(O), C(=N-OH), or C(=N-O(alkyl));
   A is a 5- or 6-membered heteroaryl optionally substituted with 1, 2, 3, or 4 R⁴;
   each R⁴ is independently halogen, cyano, hydroxy, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R²³ is hydrogen, halo, alkyl, hydroxyalkyl, cycloalkyl, phenyl, or heteroaryl and R^{23a} is hydrogen or is absent when the bond between carbons 6 and 7 is a double bond; or R²³ and R^{23a} together with the carbon to which they are attached form C=O, C=CH₂ or cycloalkyl;
   R²⁴ is hydrogen, hydroxy, or alkyl;
   R²⁵ is hydrogen; or R²³ and R²⁵ together with the carbons to which they are attached form cycloalkyl; and
   when all 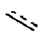 are single bonds, then
   a) one of R²³, R²⁴, and R²⁵ is not hydrogen, or
   b) R²³ and R²⁵ together with the carbons to which they are attached form cycloalkyl; and
   when A is unsubstituted pyridinyl or pyridinyl substituted with one alkyl, and R²⁴ and R²⁵ are hydrogen, then
   a) R²³ is not halo, and
   b) R²³ and R^{23a} do not form oxo; or
   a single stereoisomer or tautomer or mixture thereof, optionally as a pharmaceutically acceptable salt or solvate thereof.
4. A Compound of Formula XII 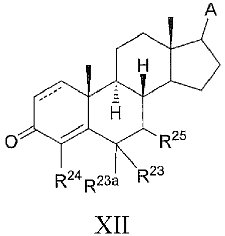 where 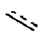 is a single bond or a double bond;
   A is a heteroaryl optionally substituted with 1, 2, 3, or 4 R⁴;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl;
   R²⁴ is hydrogen, hydroxy, or alkyl; and
   R²⁵ is hydrogen; or
   a single stereoisomer or tautomer or mixture thereof, optionally as a pharmaceutically acceptable salt or solvate thereof.
5. A Compound of Formula XIII 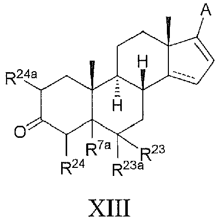 where
   A is a heteroaryl optionally substituted with 1, 2, 3, or 4 R⁴;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   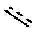 is a single bond and at least one of R²³ and R^{7a} is not hydrogen; or 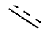 is a double bond;
   R^{7a} is hydrogen or hydroxy;
   R^{23a} is hydrogen and R²³ is halo, hydroxy, alkyl, haloalkyl, hydroxyalkyl, cycloalkyl, heterocycloalkyl, aryl, or heteroaryl; or R^{23a} and R²³ together with the carbon to which they are attached form C(O), C=CH₂, or cycloalkyl; and
   R²⁴ and R^{24a} are independently hydrogen, hydroxy, or alkyl; or
   a single stereoisomer or tautomer or mixture thereof, optionally as a pharmaceutically acceptable salt or solvate thereof.
6. A Compound of Formula XIV 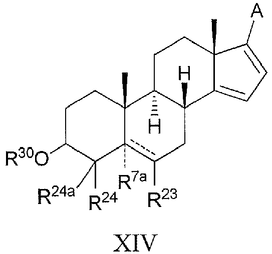 where
   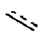 is a single bond or a double bond;
   A is a heteroaryl optionally substituted with 1, 2, 3, or 4 R⁴;
   provided that A is not unsubstituted benzimidazolyl; A is not furyl; and A is not pyridinyl optionally substituted with alkyl when both R²⁴ and R^{24a} are hydrogen;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R²³ is hydrogen or alkyl and R^{7a} is hydrogen; or R^{7a} and R²³ together with the carbons to which they are attached form oxiranyl;
   R²⁴ and R^{24a} are independently hydrogen or alkyl; and
   R³⁰ is hydrogen or alkylcarbonyl where the alkyl is optionally substituted with one or two groups independently selected from hydroxy, amino, alkylamino, and dialkylamino;
   provided that when R³⁰ is hydroxy, then A is not unsubstituted imidazolyl;
   provided that when R³⁰ is hydroxy or alkylcarbonyloxy, then A is not unsubstituted pyridinyl or pyridinyl substituted with one alkyl; or
   a single stereoisomer or tautomer or mixture thereof, optionally as a pharmaceutically acceptable salt or solvate thereof.
7. A Compound of Formula XV 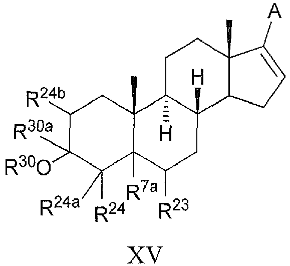 where
   A is a heteroaryl optionally substituted with 1, 2, 3, or 4 R⁴;
   provided that
   A is not unsubstituted benzimidazolyl, unsubstituted benzotriazolyl, unsubstituted triazolyl, unsubstituted imidazolyl, unsubstituted pyrimidinyl, or unsubstituted pyrazinyl;
   A is not furyl; and
   A is not pyridinyl optionally substituted with alkyl when R³⁰ and both R²⁴ and R^{7a} are hydrogen;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms;
   R²³ is hydrogen, halo, or alkyl; or R²³ and R^{7a} together with the carbons to which they are attached form cycloalkyl;
   R^{7a} is hydrogen, alkyl, or hydroxy; and R²⁴, R^{24a}, and R^{24b} are independently hydrogen or alkyl; or R²⁴ and R^{7a} together with the carbon to which they are both attached form cycloalkyl, R^{24a} is hydrogen, and R^{24b} is hydrogen or alkyl; and
   R³⁰ is hydrogen or alkyl and R^{30a} is hydrogen or R³⁰ and R^{30a} together with the carbon to which they are attached form oxiranyl or 2(3*H*)-oxo-dihydrofuranyl; or
   a single stereoisomer or tautomer or mixture thereof, optionally as a pharmaceutically acceptable salt or solvate thereof.
8. A Compound of Formula XVI 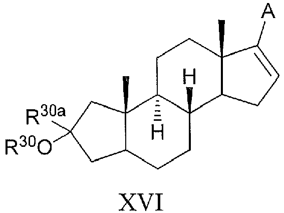 where
   A is a heteroaryl optionally substituted with 1, 2, 3, or 4 R⁴; provided that A is not pyridinyl optionally substituted with alkyl;
   each R⁴ is independently halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R_{A} and R_{B} are independently hydrogen, alkyl, haloalkyl, alkoxyalkyl, cycloalkyl, aryl, arylalkyl, heteroaryl, or heteroarylalkyl; or R_{A} and R_{B} taken together with the nitrogen atom form a 4 to 7 membered heterocyclic ring having one or two heteroatoms; R³⁰ is hydrogen, alkyl, or halo; and
   R^{30a} is hydroxy or halo; or
   a single stereoisomer or tautomer or mixture thereof, optionally as a pharmaceutically acceptable salt or solvate thereof.
9. A Compound of Formula XVII 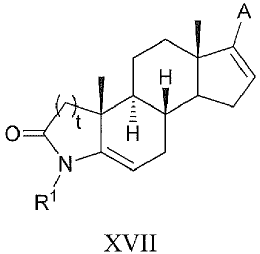 where
   t is 1 or 3;
   A is a heteroaryl optionally substituted with 1, 2, 3, or 4 R⁴;
   each R¹ is independently selected from the group consisting of hydrogen, alkyl, cycloalkyl, alkenyl, alkynyl, alkoxyalkyl, hydroxyl, and haloalkoxyalkyl;
   each R⁴, when present, is independently selected from the group consisting of halogen, cyano, hydroxyl, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a},-NR^{13a}C(O)R^{13a}, and -NR_{A}R_{B};
   R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
   each R¹³ is independently hydrogen or alkyl; and
   each R^{13a} is independently hydrogen, alkyl, haloalkyl, alkoxyalkyl, cycloalkyl, aryl, arylalkyl, heteroaryl, or heteroarylalkyl; or
   a single stereoisomer or tautomer or mixture thereof, optionally as a pharmaceutically acceptable salt or solvate thereof.
10. A compound of Formula Z 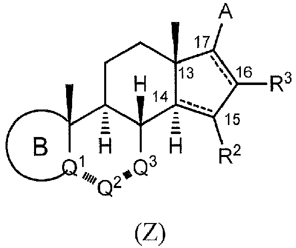 where 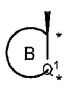 is according to one of the following formulas Z-I to Z-IX: 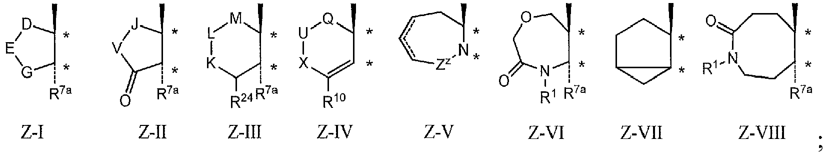 each 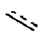 is independently a single bond or a double bond;
   Q¹'''''Q²'''''Q³ is Q¹-Q²=Q³; where Q¹ is as depicted in formulas Z-I to Z-VIII above and R^{7a} is absent or is hydrogen or hydroxy; and Q² and Q³ are CH; or
   Q¹'''''Q²'''''Q³ is Q¹=Q²-Q³; where Q¹ is as depicted in formulas Z-I to Z-VIII above and R^{7a} is absent; Q² is CH; and Q³ is CHR⁸ or C(O) where R⁸ is hydrogen; or
   Q¹'''''Q²'''''Q³ is Q¹-Q²-Q³; where Q¹ is as depicted in formulas Z-I to Z-VIII above and R^{7a} is absent or is hydrogen or hydroxy; Q² is N(H), N(alkyl), N-C(O)R¹, C(R^{7b})(R^{7c}), or O; Q³ is CHR⁸ or C=O; R^{7b} is hydrogen, alkyl, hydroxyalkyl, halo, hydroxy, cycloalkyl, heteroaryl, aryl, amino, alkylamino, dialkylamino, heteroarylamino, -OR¹, or -OC(O)R¹; R^{7c} is hydrogen; and R⁸ is hydrogen; or when
   Q² is C(R^{7b})(R^{7c}), then
   a) R^{7a}, when present, and R^{7b} together with the carbons to which they are attached form cycloalkyl or heterocycloalkyl;
   b) R^{7b} and R^{7c} together with the carbon to which they are attached form C=CH₂, cycloalkyl, or carbonyl; or
   c) R⁸ and R^{7b} together with the carbons to which they are attached form cycloalkyl;
   A is a heteroaryl optionally substituted with 1, 2, 3, or 4 R⁴;
   each R¹ is independently selected from the group consisting of hydrogen, alkyl, cycloalkyl, alkenyl, alkynyl, alkoxyalkyl, hydroxy, and haloalkoxyalkyl; where the alkyl, cycloalkyl, alkenyl, alkynyl, alkoxyalkyl, and haloalkoxyalkyl groups are optionally substituted with 1, 2, or 3 substituents independently selected from the group consisting of halogen, alkyl, alkenyl, aryl, heteroaryl, alkoxy, alkoxycarbonyl, hydroxyl, hydroxyalkyl, alkynyl, cyano, haloalkoxy, haloalkyl, nitro, -NR_{A}R_{B}, and-C(O)NR_{A}R_{B};
   R² is selected from the group consisting of hydrogen, halogen, alkyl, cycloalkyl, cyano, nitro, oxo, alkoxy, alkoxyalkyl, haloalkoxy, haloalkoxyalkyl, hydroxy, hydroxyalkyl and alkylcarbonyloxy;
   R³ is selected from the group consisting of hydrogen, halogen, alkyl, cycloalkyl, alkynyl, cyano, haloalkoxy, haloalkyl, hydroxy, hydroxyalkyl, nitro, -C(O)R_{A}, -NR_{A}R_{B}, and-C(O)NR_{A}R_{B}; and
   each R⁴, when present, is independently selected from the group consisting of halogen, cyano, hydroxy, alkoxy, alkyl, alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a},-NR^{13a}C(O)R^{13a}, and -NR_{A}R_{B};
   R_{A} and R_{B} are independently selected from the group consisting of hydrogen, alkyl, haloalkyl, alkoxyalkyl, cycloalkyl, aryl, arylalkyl, heteroaryl, and heteroarylalkyl; or R_{A} and R_{B} taken together with the nitrogen atom form a 4 to 7 membered heterocyclic ring having one or two heteroatoms;
   D is (CH₂)_{d} where d is an integer from 1 to 3;
   E is CH₂, CR¹⁴R^{14a}, O, NR¹, N-COR¹, N-S(O)₀₋₂(alkyl), or N-COOR¹;
   G is CH(CH₃), C(CH₃)₂, or (CH₂)ₑ where e is an integer from 1 to 3, provided that
   1) when E is CH₂ and d + e is 3, then A is not unsubstituted furyl or unsubstituted thienyl,
   2) when Q¹'''''Q²'''''Q³, is Q¹-Q²-Q³, E is CH₂, d + e is 3, and the bond between carbons 16 and 17 is a double bond, then a) A is not oxadiazolyl substituted with alkyl or phenyl and is not thiadiazolyl substituted with alkyl;
   V is (CH₂), O, NR¹, N-COR¹ or N-COOR¹;
   J is (CH₂)₁₋₃;
   K is C(O), NR¹, N-C(O)R¹ or N-C(O)OR¹;
   L is CH₂, C(O), NR¹, N-C(O)R¹ or N-C(O)OR¹;
   M is (CH=CH) or (CH₂)_{g} where g is an integer 2 or 3,
   provided that when g is 2, K is C(O) and L is NR¹, N-C(O)R¹ or N-C(O)OR¹; when g is 2, K is NR¹, N-C(O)R¹or N-C(O)OR¹ and L is C(O); then the bond between carbons 14 and 15 is a double bond; and
   provided that g cannot be 2 when K is NR¹, N-C(O)R¹or N-C(O)OR¹ and L is or CH₂;
   R²⁴ is hydrogen or alkyl; or when R^{7a} is present, R²⁴ and R^{7a} together with the carbons to which they are attached form cycloalkyl;
   Q is (CH₂)ᵢ where i is an integer from 1 to 3;
   U is (CH₂), CO, O, NR¹, N-COR¹or N-COOR¹; or
   Q and U together are CH=CH;
   X is CR^{11a}R^{11b}, C=O, C=NOR⁹, O, NR¹, N-COR¹or N-COOR¹
   provided that i cannot be 1 when X is CO and U is CH₂;
   provided that when X is CR^{11a}R^{11b}, R^{11b} is OR¹, then
   a) the bond between carbons 16 and 17 is a double bond;
   b) A is not unsubstituted benzimidazolyl, unsubstituted imidazolyl, or unsubstituted pyrazolyl; and
   c) A is not imidazolyl, thiazolyl, or oxazolyl, where each are substituted with amino, alkylamino, or dialkylamino;
   R⁹ is hydrogen, alkyl, or haloalkyl;
   R¹⁰ is hydrogen, hydroxy, or alkyl;
   R^{11a} is hydrogen or alkyl;
   R^{11b} is hydrogen or -OR¹;
   each R¹³ is independently hydrogen or alkyl;
   each R^{13a} is independently hydrogen, alkyl, haloalkyl, alkoxyalkyl, cycloalkyl, aryl, arylalkyl, heteroaryl, or heteroarylalkyl;
   R¹⁴ is hydrogen or alkyl;
   R^{14a} is halo, -N₃, -NR¹⁵S(O)₂R^{15a}, -NR¹⁵C(O)R^{15a}, -NR¹⁵C(O)NR^{15a}R^{15b}, -NR¹⁵C(S)NR^{15a}R^{15 b}, -NR¹⁵C(O)OR^{15a}, or -NR¹⁵R^{15a};
   provided that when R^{14a} is -NR¹⁵R^{15a}, then Q¹'''''Q²'''''Q³ is not Q¹-Q²-Q³ and A is monocyclic heteroaryl;
   each R¹⁵ and R^{15b} is independently hydrogen, alkyl, or haloalkyl;
   each R^{15a} are independently alkyl, haloalkyl, cycloalkyl, or heteroaryl where the heteroaryl is optionally substituted with one or two alkyl;
   Z^{Z} is CO or (CH₂);
   provided that when 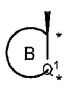 is according to formula Z-VII, and Q² is CH(OH), CH(OCH₃),
   or CH(OC(O)alkyl), then A is not unsusbstituted pyridinyl and A is not pyrazolyl optionally substituted with one R⁴;
   or a single stereoisomer or tautomer or mixture thereof, optionally as a pharmaceutically acceptable salt or solvate thereof.
11. The compound of any of paragraphs 1-10 where A is monocyclic heteroaryl optionally substituted with one or two R⁴ groups independently selected from halogen, cyano, hydroxy, alkoxy, alkyl, haloalkyl, cycloalkyl, and aryl.
12. The compound of paragraph 11 where A is pyridinyl, imidazolyl, triazolyl, pyrrolyl, pyrazolyl, pyrimidinyl, pyrazinyl, pyridazinyl, oxazolyl or thiazolyl, each of which is optionally substituted with one or two R⁴ groups independently selected from halogen, alkoxy, alkyl, and haloalkyl.
13. The compound of paragraph 11 where A is pyridinyl optionally substituted with one or two R⁴ groups independently selected from halogen, alkoxy, alkyl, and haloalkyl.
14. The compound of paragraph 11 where A is imidazolyl optionally substituted with one or two R⁴ groups independently selected from alkyl and haloalkyl.
15. The compound of paragraph 11 where A is pyrazinyl optionally substituted with one or two R⁴ groups independently selected from halogen, alkoxy, alkyl, and haloalkyl.
16. The compound of paragraph 11 where A is triazolyl optionally substituted with one or two R⁴ groups independently selected from alkyl and haloalkyl.
17. The compound of any of paragraphs 9-16 where R¹ is hydrogen or C₁-C₆ alkyl.
18. The compound of any of paragraphs 10-16 where R² is hydrogen, oxo, or C₁-C₆ alkyl.
19. The compound of any of paragraphs 10-16 where R³ is hydrogen or C₁-C₆ alkyl.
20. The compound of any of paragraphs 10-16, where both R² and R³ are hydrogen.
21. A compound selected from Table 2 and Table 3.
22. A pharmaceutical composition comprising a compound of any of paragraphs 1-21 and a pharmaceutically acceptable carrier, excipient or binder.
23. A method for treating cancer in a subject comprising administering to a subject in need thereof 1) a therapeutically effective amount of a compound of any of paragraphs 1-22 or a pharmaceutically acceptable salt or solvate thereof or 2) a pharmaceutical composition comprising a therapeutically effective amount of a compound of any of paragraphs 1-22 or a pharmaceutically acceptable salt or solvate thereof and a pharmaceutically acceptable carrier, excipient or binder.
24. The method of paragraph 23 where the cancer is selected from the group consisting of bladder cancer, brain cancer, breast cancer, cervical cancer, colorectal cancer, endometrial cancer, gastric cancer, glioblastoma, head and neck cancer, Kaposi's sarcoma, kidney cancer, leiomyosarcoma, leukemia, liver cancer, lung cancer, melanoma, multiple myeloma, Non-Hodgkin lymphoma, ovarian cancer, pancreatic cancer, papillary renal cell carcinoma, prostate cancer, renal cancer, squamous cell cancer, and/or thoracic cancer.
25. The method of paragraph 24 where the cancer is prostate cancer.
26. The method of any of paragraphs 23-25 further comprising providing to the subject an additional therapy selected from the group consisting of surgery, an anti-androgenic agent, and radiation, and a combination thereof.
27. The method of paragraph 27 where providing chemotherapy to the subject comprises administering a therapeutically effective amount of at least one anti-androgenic agent which is optionally selected from the group consisting of flutamide, nicalutamide, bicalutamide, inhibitors of 17α-hydroxylase/C17-20 lyase, luteinizing hormone-releasing hormone agonists, luteinizing hormone-releasing hormone antagonists, and/or 5α-reductase type 1 and/or type 2 and/or combinations thereof.
28. A method of inhibiting CYP11B, CYP17, and/or CYP21 enzyme comprising contacting a compound of any of paragraphs 1-21 or a pharmaceutically acceptable salt or solvate thereof with a CYP11B, CYP 17, and/or CYP21 enzyme.
29. The method of paragraph 29 where the contacting step is in vivo.
30. A method of treating an androgen-dependent disorder in a subject comprising administering to a subject in need thereof a therapeutically effective amount of a compound of any of paragraphs 1-21 or a pharmaceutically acceptable salt or solvate thereof.
31. The method of paragraph 31 where the androgen-dependent disorder is selected from the group consisting of prostate cancer, benign prostatic hyperplasia, prostatic intraepithelial neoplasia, hirsutism, acne, androgenic alopecia, and polycystic ovary syndrome.
32. The method of paragraph 32 where the androgen-dependent disorder is prostate cancer.
33. A method of treating a proliferative disease comprising administering to a subject in need thereof a therapeutically effective amount of a compound of any of paragraphs 1-21 or a pharmaceutically acceptable salt or solvate thereof.
34. The method of paragraph 34 further comprising administering a therapeutically effective amount of at least one additional agent or providing an additional therapy selected from the group consisting of a chemotherapeutic agent, a biological agent, surgery, and/or radiation therapy.
35. The method of paragraph 35 where the administration of the at least one additional agent is performed concurrently or sequentially.
36. A method of treating a disease associated with hypercortisolism comprising administering to a subject in need thereof a therapeutically effective amount of a compound having the structure of Formula (Z), IX, X, XI, XII, XIII, XIV, XV, XVI, or XVII, or a pharmaceutically acceptable salt or solvate thereof.
37. The method of paragraph 37 where the disease is Cushing's Syndrome.
38. An article of manufacture, comprising packaging material, a compound of any of paragraphs 1-21, and a label, where the compound is effective for the treatment of an androgen dependent disorder, where the compound is packaged within the packaging material, and where the label indicates that the compound, or pharmaceutically acceptable salt or solvate thereof is used for the treatment of an androgen dependent disorder.
39. Use of a compound of any one of paragraphs 1-21 or a pharmaceutically acceptable salt or solvate thereof for the manufacture of a medicament for the treatment of prostate cancer.
40. A compound of any one of paragraphs 1-21 for use in treating an androgen dependent disorder or a disease associated with hypercortisolism.

## Claims

1. A Compound of Formula XI 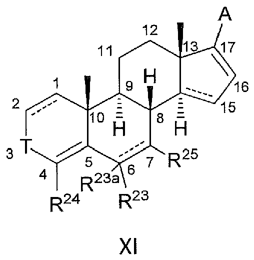 where
each 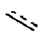 is independently a single or double bond;
T is C(O), C(=N-OH), or C(=N-O(alkyl));
A is a 5- or 6-membered heteroaryl optionally substituted with 1, 2, 3, or 4 R⁴;
each R⁴ is independently halogen, cyano, hydroxy, C₁₋₁₀-alkoxy, C₁₋₁₀-alkyl, C₂₋₁₀-alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
R²³ is hydrogen, halo, C₁₋₁₀-alkyl, C₁₋₁₀-hydroxyalkyl, cycloalkyl, phenyl, or heteroaryl and R^{23a} is hydrogen or is absent when the bond between carbons 6 and 7 is a double bond; or R²³ and R^{23a} together with the carbon to which they are attached form C=O, C=CH₂ or cycloalkyl;
R²⁴ is hydrogen, hydroxy, or C₁₋₁₀-alkyl;
R²⁵ is hydrogen; or R²³ and R²⁵ together with the carbons to which they are attached form cycloalkyl;
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a-C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; and
when all 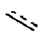 are single bonds, then
a) one of R²³, R²⁴, and R²⁵ is not hydrogen, or
b) R²³ and R²⁵ together with the carbons to which they are attached form cycloalkyl; and
when A is unsubstituted pyridinyl or pyridinyl substituted with one C₁₋₁₀-alkyl, and R²⁴ and R²⁵ are hydrogen, then
a) R²³ is not halo, and
b) R²³ and R^{23a} do not form oxo; or
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

2. A Compound of Formula XIV 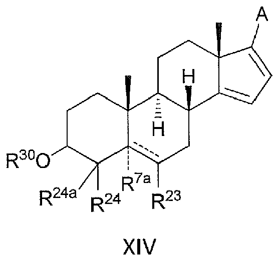 where 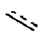 is a single bond or a double bond;
A is a heteroaryl optionally substituted with 1, 2, 3, or 4 R⁴;
provided that A is not unsubstituted benzimidazolyl; A is not furyl; and A is not pyridinyl optionally substituted with C₁₋₁₀-alkyl when both R²⁴ and R^{24a} are hydrogen;
each R⁴ is independently halogen, cyano, hydroxyl, C₁₋₁₀-alkoxy, C₁₋₁₀-alkyl, C₂₋₁₀-alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
R²³ is hydrogen or C₁₋₁₀-alkyl and R^{7a} is hydrogen; or R^{7a} and R²³ together with the carbons to which they are attached form oxiranyl;
R²⁴ and R^{24a} are independently hydrogen or C₁₋₁₀-alkyl; and
R³⁰ is hydrogen or C₁₋₁₀-alkylcarbonyl where the alkyl is optionally substituted with one or two groups independently selected from hydroxy, amino, C₁₋₁₀-alkylamino, and C₁₁₀-dialkylamino;
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a-C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently-O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; and
provided that when R³⁰ is hydrogen, then A is not unsubstituted imidazolyl;
provided that when R³⁰ is hydrogen or alkylcarbonyl, then A is not unsubstituted pyridinyl or pyridinyl substituted with one C₁₋₁₀-alkyl; or
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

3. A compound of Formula IX 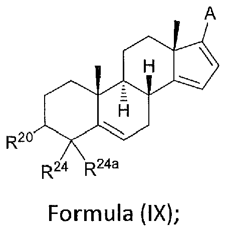 where
A is a heteroaryl optionally substituted with 1, 2, 3, or 4 R⁴;
each R⁴ is independently halogen, cyano, hydroxyl, C₁₋₁₀-alkoxy, C₁₋₁₀-alkyl; C₂₋₁₀-alkenyl, cycloalkyl, heterocycloalkyl, aryl, heteroaryl, -C(O)R^{13a}, C₁₋₁₀-alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
R²⁰ is arylcarbonyloxy, heterocycloalkylcarbonyloxy, heteroarylcarbonyloxy, -OC(O)NR²¹R^{21a}, or -OC(O)-alkylene-NR²¹R^{21a}; where the aryl, heterocycloalkyl, and heteroaryl are independently optionally substituted with one or two C₁₋₁₀-alkyl;
R²¹ is hydrogen or C₁₋₁₀-alkyl;
R^{21a} is hydrogen, C₁₋₁₀-alkyl, or heteroaryl;
R²⁴ and R^{24a} are independently hydrogen or C₁₋₁₀-alkyl; and
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a-C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; and
provided that when R²⁰ is phenylcarbonyloxy, then A is not unsubstituted pyridinyl or pyridinyl substituted with one C₁₋₁₀-alkyl; or
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

4. A Compound of Formula XII 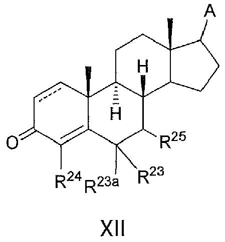 where
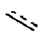 is a single bond or a double bond;
A is a heteroaryl optionally substituted with 1, 2, 3, or 4 R⁴;
each R⁴ is independently halogen, cyano, hydroxyl, C₁₋₁₀-alkoxy, C₁₋₁₀-alkyl, C₂₋₁₀-alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
R^{23a} is hydrogen and R²³ is halo, hydroxy, C₁₋₁₀-alkyl, C₁₋₁₀-haloalkyl, C₁₋₁₀-hydroxyalkyl, cycloalkyl, heterocycloalkyl, aryl, or heteroaryl; or R^{23a} and R²³ together with the carbon to which they are attached form C(O), C=CH₂, or cycloalkyl;
R²⁴ is hydrogen, hydroxy, or C₁₋₁₀-alkyl; and
R²⁵ is hydrogen;
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a - C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently-O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; or
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

5. A Compound of Formula XIII 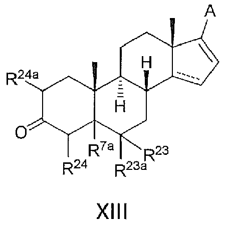 where
A is a heteroaryl optionally substituted with 1, 2, 3, or 4 R⁴;
each R⁴ is independently halogen, cyano, hydroxyl, C₁₋₁₀-alkoxy, C₁₋₁₀-lkyl, C₂₋₁₀-alkenyl, cycloalkyl, heterocycloalkyl, aryl, heteroaryl, -C(O)R^{13a}, C₁₋₁₀-alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
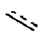 is a single bond and at least one of R²³ and R^{7a} is not hydrogen; or 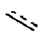 is a double bond;
R^{7a} is hydrogen or hydroxy;
R^{23a} is hydrogen and R²³ is halo, hydroxy, C₁₋₁₀-alkyl, C₁₋₁₀-haloalkyl, C₁₋₁₀- hydroxyalkyl, cycloalkyl, heterocycloalkyl, aryl, or heteroaryl; or R^{23a} and R²³ together with the carbon to which they are attached form C(O), C=CH₂, or cycloalkyl; and
R²⁴ and R^{24a} are independently hydrogen, hydroxy, or C₁₋₁₀-alkyl;
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a-C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; and
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

6. A Compound of Formula X 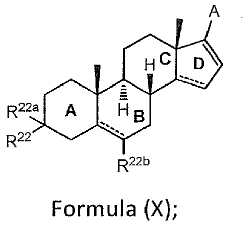 where
one 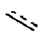 is a single bond and the other is a double bond or both are single bonds;
A is heteroaryl optionally substituted with 1, 2, 3, or 4 R⁴;
each R⁴ is independently halogen, cyano, hydroxyl, C₁₋₁₀-alkoxy, C₁₋₁₀-alkyl, C₂₋₁₀-alkenyl, cycloalkyl, heterocycloalkyl, aryl, heteroaryl, -C(O)R^{13a}, alkoxycarbonyl, -C(O)NR_{A}R_{B}, -NR¹³S(O)₂R^{13a}, -NR¹³C(O)R^{13a}, or -NR_{A}R_{B};
each R¹³ is independently hydrogen or C₁₋₁₀-alkyl;
each R^{13a} is independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl;
R_{A} and R_{B} are independently hydrogen, C₁₋₁₀-alkyl, C₁₋₁₀-haloalkyl, C₁₋₁₀-alkoxyalkyl, cycloalkyl, aryl, aryl-C₁₋₁₀-alkyl, heteroaryl, or heteroaryl-C₁₋₁₀-alkyl; or R_{A} and R_{B} taken together with the nitrogen atom form a 4 to 7 membered heterocyclic ring having one or two heteroatoms;
R²² is halo or hydroxy; and R^{22a} is halo, C₁₋₁₀-alkyl, C₁₋₁₀-hydroxyalkyl, C₂₋₁₀-alkynyl, or cycloalkyl; or R²² and R^{22a} together with the carbon to which they are attached form heterocycloalkyl; and
R^{22b} is hydrogen or C₁₋₁₀-alkyl;
where aryl means a monovalent six- to fourteen-membered, mono- or bi-carbocyclic ring, where the monocyclic ring is aromatic and at least one of the rings in the bicyclic ring is aromatic;
where cycloalkyl means a monocyclic or fused or bridged bicyclic or tricyclic, saturated or partially unsaturated (but not aromatic), monovalent hydrocarbon radical of three to ten carbon ring atoms and one or two ring carbon atoms may be replaced with a - C(O)-, -C(S)-, or -C(=NH)- group;
where heteroaryl means a monocyclic or fused or bridged bicyclic monovalent radical of 5 to 14 ring atoms containing one or more, specifically one, two, three, or four ring heteroatoms where each heteroatom is independently -O-, -S(O)ₙ- (n is 0, 1, or 2), - NH-, -N=, or N-oxide, with the remaining ring atoms being carbon, where the ring comprising a monocyclic radical is aromatic and where at least one of the fused and bridged rings comprising a bicyclic radical is aromatic and where one or two ring carbon atoms of any nonaromatic rings comprising the bicyclic radical may be replaced by a -C(O)-, -C(S)-, or -C(=NH)- group; and
where heterocycloalkyl means a saturated or partially unsaturated (but not aromatic) monovalent monocyclic group of 3 to 8 ring atoms or a saturated or partially unsaturated (but not aromatic) monovalent fused or bridged bicyclic or tricyclic group of 5 to 12 ring atoms in which one or more, specifically one, two, three, or four ring heteroatoms where each heteroatom is independently -O-, -S(O)ₙ- (n is 0, 1, or 2), -N=, -NH-, the remaining ring atoms being carbon and where one or two ring carbon atoms may be replaced by a -C(O)-, -C(S)-, or -C(=NH)- group; or
a single stereoisomer or tautomer or mixture thereof, optionally as a pharmaceutically acceptable salt or solvate thereof; and
optionally a pharmaceutical composition comprising the compound and a pharmaceutically acceptable carrier, excipient or binder.

7. The compound of any of claims 1-6 where
(a) A is monocyclic heteroaryl optionally substituted with one or two R⁴ groups independently selected from halogen, cyano, hydroxy, C₁₋₁₀-alkoxy, C₁₋₁₀-alkyl, C₁₋₁₀-haloalkyl, cycloalkyl, and aryl; or
(b) A is pyridinyl, imidazolyl, triazolyl, pyrrolyl, pyrazolyl, pyrimidinyl, pyrazinyl, pyridazinyl, oxazolyl or thiazolyl, each of which is optionally substituted with one or two R⁴ groups independently selected from halogen, C₁₋₁₀-alkoxy, C₁₋₁₀-alkyl, and C₁₋₁₀-haloalkyl; or
(c) A is pyridinyl optionally substituted with one or two R⁴ groups independently selected from halogen, C₁₋₁₀-alkoxy, C₁₋₁₀-alkyl, and C₁₋₁₀-haloalkyl; or
(d) A is imidazolyl optionally substituted with one or two R⁴ groups independently selected from C₁₋₁₀-alkyl and C₁₋₁₀-haloalkyl; or
(e) pyrazinyl optionally substituted with one or two R⁴ groups independently selected from halogen, C₁₋₁₀-alkoxy, C₁₋₁₀-alkyl, and C₁₋₁₀-haloalkyl; or
(f) A is triazolyl optionally substituted with one or two R⁴ groups independently selected from C₁₋₁₀-alkyl and C₁₋₁₀-haloalkyl; and
optionally a pharmaceutical composition comprising the compound and a pharmaceutically acceptable carrier, excipient or binder.

8. The compound of claim 7 where R¹ is hydrogen or C₁-C₆ alkyl; and optionally a pharmaceutical composition comprising the compound and a pharmaceutically acceptable carrier, excipient or binder.

9. The compound of claim 11 where R² is hydrogen, oxo, or C₁-C₆ alkyl; or where R³ is hydrogen or C₁-C₆ alkyl; or where both R² and R³ are hydrogen; and optionally a pharmaceutical composition comprising the compound and a pharmaceutically acceptable carrier, excipient or binder.

10. A compound selected from
| |
|---|
| (1*S*,2*S*,8*S*,12*S*,16*S*,11*R*)-2,16-dimethyl-15-(3-pyridyl)-5-oxatetracyclo[9.7.0.0^{2,8}.0^{12,16}]octadec-14-en-6-one; |
| (1*S,*12*S,*16*S,*2*R,*8*R,*11*R*)-2,16-dimethyl-15-(3-pyridyl)-6-oxatetracyclo[9.7.0.0^{2,8}.0^{12,16}]octadec-14-en-5-one; |
| (1*S*,12*S*,16*S*,2*R*,8*R*,11*R*)-15-(5-ethoxy(3-pyridyl))-2,16-dimethyl-6-oxatetracyclo[9.7.0.0^{2,8}.0^{12,16}]octadec-14-en-5-one; |
| (1*S*,12*S*,16*S*,2*R*,8*R*,11*R*)-15-(5-methoxy(3-pyridyl))-2,16-dimethyl-6-oxatetracyclo[9.7.0.0^{2,8}.0^{12,16}]octadec-14-en-5-one; |
| (11a*R*,13a*S*)-11a,13a-dimethyl-1-(pyridin-3-yl)-3b,4,5,8,11,11a,11b,12,13,13a-decahydro-3*H*-azepino[2,1-a]cyclopenta[f]isoquinolin-7(3a*H*)-one; |
| (11a*R*,13a*S*)-11a,13a-dimethyl-1-(pyridin-3-yl)-3b,4,5,10,11,11a,11b,12,13,13a-decahydro-3*H*-azepino[2,1-a]cyclopenta[f]isoquinolin-7(3a*H*)-one; |
| (5a*R*,5b*S*,7a*S*,10a*S*,10b*R*,12a*R*)-5a,7a-dimethyl-8-(5-methylpyridin-3-yl)-5,5a,5b,6,7,7a,10,10a,10b,11,12,12a-dodecahydro-1*H*-cyclopenta[5,6]naphtho[2,1-c]oxepin-3(4*H*)-one; |
| (11a*R*,13a*S*)-9-hydroxy-11a,13a-dimethyl-1-(pyridin-3-yl)-3b,4,5,8,9,10,11,11a,11b,12,13,13a-dodecahydro-3*H-*azepino[2,1-a]cyclopenta[f]isoquinolin-7(3a*H*)-one; |
| (11a*R*,13a*S*)-11a,13a-dimethyl-1-(pyridin-3-yl)-4,5,8,10,11,11a,11b,12,13,13a-decahydro-3*H*-azepino[2,1-a]cyclopenta[f]isoquinoline;-7,9(3a*H*,3b*H*)-dione; |
| (11a*R*,13a*S*)-11a,13a-dimethyl-1-(pyridin-3-yl)-3b,4,5,8,9,10,11,11a,11b,12,13,13a-dodecahydro-3*H*-azepino[2,1-a]cyclopenta[f]isoquinolin-7(3a*H*)-one; |
| (11a*R*,13a*S*)-11a,13a-dimethyl-1-(5-methylpyridin-3-yl)-3b,4,5,8,9,10,11,11a,11b,12,13,13a-dodecahydro-3*H-*azepino[2,1-a]cyclopenta[f]isoquinolin-7(3a*H*)-one; |
| (11a*R*,13a*S*)-1-(5-methoxypyridin-3-yl)-11a,13a-dimethyl-3b,4,5,8,9,10,11,11a,11b,12,13,13a-dodecahydro-3*H-*azepino[2,1-a]cyclopenta[f]isoquinolin-7(3a*H*)-one; |
| (11a*R*,13a*S*)-1-(5-ethylpyridin-3-yl)-11a,13a-dimethyl-3b,4,5,8,9,10,11,11a,11b,12,13,13a-dodecahydro-3*H-*azepino[2,1-a]cyclopenta[f]isoquinolin-7(3a*H*)-one; |
| (6a*S*,6b*S*,8a*S*,11a*S*,11b*R*,13a*S*)-6a,8a-dimethyl-9-(pyridin-3-yl)-4,5,6,6a,6b,7,8,8a,11,11a,11b,12,13,13a-tetradecahydro-1*H*-cyclopenta[5,6]naphtho[1,2-d]azocin-3(2*H*)-one; |
| (11a*R*,13a*S*)-11a,13a-dimethyl-1-(pyridin-3-yl)-3a,3b,4,5,7,8,9,10,11,11a,11b,12,13,13a-tetradecahydro-3*H-*azepino[2,1-a]cyclopenta[f]isoquinoline; |
| (11a*R*,13a*S*)-11a,13a-dimethyl-1-(5-propoxypyridin-3-yl)-3b,4,5,8,9,10,11,11a,11b,12,13,13a-dodecahydro-3*H-*azepino[2,1-a]cyclopenta[f]isoquinolin-7(3a*H*)-one; |
| 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)pyridine; |
| 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-methoxypyridine; |
| 3-((5a*R*,5b*S*,7a*S*,10a*S*,10b*R*,12a*S*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)pyridine; |
| (5a*R*,7a*S*)-8-(5-methoxypyridin-3-yl)-5a,7a-dimethyl-4,5,5a,5b,6,7,7a,8,9,10,10a,10b,11,12-tetradecahydrocyclopenta[5,6]naphtho[2,1-c]azepin-3(2*H*)-one; |
| (11a*R*,13a*S*)-11a,13a-dimethyl-1-(5-methylpyridin-3-yl)-3a,3b,4,5,7,8,9,10,11,11a,11b,12,13,13a-tetradecahydro-3*H*-azepino[2,1-a]cyclopenta[f]isoquinoline; |
| (11a*R*,13a*S*)-11a,13a-dimethyl-1-(5-vinylpyridin-3-yl)-3b,4,5,8,9,10,11,11a,11b,12,13,13a-dodecahydro-3*H-*azepino[2,1-a]cyclopenta[f]isoquinolin-7(3a*H*)-one; |
| (11a*R*,13a*S*)-11a,13a-dimethyl-1-(5-propoxypyridin-3-yl)-3a,3b,4,5,7,8,9,10,11,11a,11b,12,13,13a-tetradecahydro-3*H*-azepino[2,1-a]cyclopenta[f]isoquinoline; |
| (11a*R*,13a*S*)-1-(5-methoxypyridin-3-yl)-11a,13a-dimethyl-3a,3b,4,5,7,8,9,10,11,11a,11b,12,13,13a-tetradecahydro-3*H*-azepino[2,1-a]cyclopenta[f]isoquinoline; |
| (11a*R*,13a*S*)-1-(5-ethylpyridin-3-yl)-11a,13a-dimethyl-3a,3b,4,5,7,8,9,10,11,11a,11b,12,13,13a-tetradecahydro-3*H*-azepino[2,1-a]cyclopenta[f]isoquinoline; |
| 10a,12a-dimethyl-1-(pyrazin-2-yl)-3,3a,3b,4,8,9,10,10a,10b,11,12,12a-dodecahydrocyclohepta[a]cyclopenta[f]naphthalen-7(6*H*)-one; |
| (5a*R*,7a*S*)-5a,7a-dimethyl-8-(5-propoxypyridin-3-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-2,5a,7a-trimethyl-8-(5-propoxypyridin-3-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-8-(5-ethylpyridin-3-yl)-2,5a,7a-trimethyl-4,5,5a,5b,6,7,7a,10,10a,10b,11,12-dodecahydrocyclopenta[5,6]naphtho[2,1-c]azepin-3(2*H*)-one; |
| 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-methoxypyridine; |
| (5a*R*,5b*S*,7a*S*,10a*S*,10b*S*)-1,5a,7a-trimethyl-8-(pyridin-3-yl)-5,5a,5b,6,7,7a,10,10a,10b,11,12,12a-dodecahydro-1*H*-cyclopenta[5,6]naphtho[2,1-e][1,4]oxazepin-2(3*H*)-one; |
| (5a*R*,5b*S*,7a*S*,10a*S*,10b*S*)-8-(5-ethoxypyridin-3-yl)-1,5a,7a-trimethyl-5,5a,5b,6,7,7a,10,10a,10b,11,12,12a-dodecahydro-1*H*-cyclopenta[5,6]naphtho[2,1-e][1,4]oxazepin-2(3*H*)-one; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)pyridine; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-methylpyridine; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-methoxypyridine; |
| (5a*R*,5b*S*,7a*S*,10a*S*,10b*S*)-1,5a,7a-trimethyl-8-(5-methylpyridin-3-yl)-5,5a,5b,6,7,7a,10,10a,10b,11,12,12a-dodecahydro-1*H*-cyclopenta[5,6]naphtho[2,1-e][1,4]oxazepin-2(3*H*)-one; |
| 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-methylpyridine; |
| 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-methylpyridine; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-ethylpyridine; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-5-ethoxypyridine; |
| (5a*R*,7a*S*)-2,5a,7a-trimethyl-8-(5-methylpyridin-3-yl)-2,3,4,5,5a,5b,6,7,7a,10,10a,10b,11,12-tetradecahydrocyclopenta[5,6]naphtho[2,1-c]azepine; |
| (5a*R*,7a*S*)-8-(1*H*-imidazol-1-yl)-2,5a,7a-trimethyl-4,5,5a,5b,6,7,7a,10,10a,10b,11,12-dodecahydrocyclopenta[5,6]naphtho[2,1-c]azepin-3(2*H*)-one; |
| (5a*R*,7a*S*)-2,5a,7a-trimethyl-8-(pyridin-3-yl)-2,3,4,5,5a,5b,6,7,7a,10,10a,10b,11,12-tetradecahydrocyclopenta[5,6]naphtho[2,1-c]azepine; |
| (5a*R*,7a*S*)-8-(5-methoxypyridin-3-yl)-2,5a,7a-trimethyl-2,3,4,5,5a,5b,6,7,7a,10,10a,10b,11,12-tetradecahydrocyclopenta[5,6]naphtho[2,1-c]azepine; |
| (5a*R*,7a*S*)-2,5a,7a-trimethyl-8-(5-propoxypyridin-3-yl)-2,3,4,5,5a,5b,6,7,7a,10,10a,10b,11,12-tetradecahydrocyclopenta[5,6]naphtho[2,1-c]azepine; |
| 3-((5a*R*,5b*S*,7a*S*,10a*S*,10b*R*,12a*R*)-5a,7a-dimethyl-3,4,5,5a,5b,6,7,7a,10,10a,10b,11,12,12a-tetradecahydro-1*H-*cyclopenta[5,6]naphtho[2,1-c]oxepin-8-yl)-5-ethoxypyridine; |
| 3-((5a*S*,5b*S*,7a*S*,10a*S*,10b*R*,12a*S*)-5a,7a-dimethyl-2,4,5,5a,5b,6,7,7a,10,10a,10b,11,12,12a-tetradecahydro-1*H-*cyclopenta[5,6]naphtho[1,2-d]oxepin-8-yl)-5-ethoxypyridine; |
| 3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-4-methylpyridine; |
| 4-chloro-3-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)pyridine; |
| (5a*R*,7a*S*)-8-(5-ethylpyridin-3-yl)-2,5a,7a-trimethyl-2,3,4,5,5a,5b,6,7,7a,10,10a,10b,11,12-tetradecahydrocyclopenta[5,6]naphtho[2,1-c]azepine; |
| (5a*R*,7a*S*)-8-(1*H*-imidazol-1-yl)-2,5a,7a-trimethyl-2,3,4,5,5a,5b,6,7,7a,10,10a,10b,11,12-tetradecahydrocyclopenta[5,6]naphtho[2,1-c]azepine; |
| 1-((3a*S*,3b*R*,5a*R*,10a*S*,10b*S*,12a*S*)-10a,12a-dimethyl-3,3a,3b,4,5,5a,6,7,8,9,10,10a,10b,11,12,12a-hexadecahydrocyclohepta[a]cyclopenta[f]naphthalen-1-yl)-1*H*-imidazole; |
| (5a*R*,7a*S*)-8-(1*H*-imidazol-1-yl)-5a,7a-dimethyl-4,5,5a,5b,6,7,7a,8,9,10,10a,10b,11,12-tetradecahydrocyclopenta[5,6]naphtho[2,1-c]azepin-3(2*H*)-one; |
| (5a*R*,7a*S*)-5a,7a-dimethyl-8-(1*H*-1,2,4-triazol-1-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-8-(1*H*-benzo[d]imidazol-1-yl)-5a,7a-dimethyl-4,5,5a,Sb,6,7,7a,10,10a,10b,11,12-dodecahydrocyclopenta[5,6]naphtho[2,1-c]azepin-3(2*H*)-one; |
| (5a*R*,7a*S*)-5a,7a-dimethyl-8-(4-methyl-1*H*-imidazol-1-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-8-(1*H*-benzo[d]imidazol-1-yl)-2,5a,7a-trimethyl-4,5,5a,5b,6,7,7a,10,10a,10b,11,12-dodecahydrocyclopenta[5,6]naphtho[2,1-c]azepin-3(2*H*)-one; |
| (5a*R*,7a*S*)-5a,7a-dimethyl-8-(1*H*-1,2,3-triazol-1-yl)-4,5,5a,5b,6,7,7a,10,10a,10b,11,12-dodecahydrocyclopenta[5,6]naphtho[2,1-c]azepin-3(2*H*)-one; |
| 3-((10*R*,13*S*)-10,13-dimethyl-2,3,4,7,8,9,10,11,12,13,14,15-dodecahydro-1*H*-cyclopenta[a]phenanthren-17-yl)-5-ethylpyridine; |
| (5a*R*,7a*S*)-5a,7a-dimethyl-8-(pyrimidin-5-yl)-4,5,5a,5b,6,7,7a,10,10a,10b,11,12-dodecahydrocyclopenta[5,6]naphtho[2,1-c]azepin-3(2*H*)-one; |
| 3-((10*R*,13*S*)-10,13-dimethyl-2,3,4,7,8,9,10,11,12,13,14,15-dodecahydro-1*H*-cyclopenta[a]phenanthren-17-yl)-5-methylpyridine; |
| 3-((10*R*,13*S*)-10,13-dimethyl-2,3,4,7,8,9,10,11,12,13,14,15-dodecahydro-1*H*-cyclopenta[a]phenanthren-17-yl)-5-methoxypyridine; |
| 3-((10*R*,13*S*)-10,13-dimethyl-2,3,4,7,8,9,10,11,12,13,14,15-dodecahydro-1*H*-cyclopenta[a]phenanthren-17-yl)pyridine; |
| (5a*S*,7a*S*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one; |
| (5a*R*,7a*S*)-8-(1*H*-imidazol-1-yl)-5a,7a-dimethyl-1,2,5,5a,5b,6,7,7a,10,10a,10b,11,12,12a-tetradecahydrocyclopenta[5,6]naphtho[2,1-c]azepin-3(4*H*)-one; |
| (5a*R*,7a*S*)-5a,7a-dimethyl-8-(pyrazin-2-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-5a,7a-dimethyl-8-(2*H*-tetrazol-2-yl)-4,5,5a,5b,6,7,7a,10,10a,10b,11,12-dodecahydrocyclopenta[5,6]naphtho[2,1-c]azepin-3(2*H*)-one; |
| (10*R*,13*S*)-10,13-dimethyl-17-(5-methylpyridin-3-yl)-2,3,4,5,6,7,8,9,10,11,12,13,14,15-tetradecahydro-1*H-*cyclopenta[a]phenanthren-6-ol; |
| (10*R*,13*S*)-10,13-dimethyl-17-(5-methylpyridin-3-yl)-2,3,4,5,7,8,9,11,12,13,14,15-dodecahydro-1*H-*cyclopenta[a]phenanthren-6(10*H*)-one; |
| (10*R*,13*S*)-17-(5-methoxypyridin-3-yl)-10,13-dimethyl-2,3,4,5,7,8,9,11,12,13,14,15-dodecahydro-1*H-*cyclopenta[a]phenanthren-6(10*H*)-one; |
| (3*S*,10*R*,13*S*)-17-(1*H*-imidazol-1-yl)-10,13-dimethyl-2,3,4,7,8,9,10,11,12,13-decahydro-1*H-*cyclopenta[a]phenanthren-3-yl acetate; |
| (5a*S*,5b*S*,7a*S*,10a*S*,10b*R*,12a*S*)-5a,7a-dimethyl-8-(1*H*-1,2,3-triazol-1-yl)-3,4,5,5a,5b,6,7,7a,10,10a,10b,11,12,12a-tetradecahydrocyclopenta[5,6]naphtho[1,2-d]azepin-2(1*H*)-one; |
| (5a*R*,5b*S*,7a*S*,10a*S*,10b*R*,12a*R*)-5a,7a-dimethyl-8-(1*H*-1,2,3-triazol-1-yl)-1,2,5,5a,5b,6,7,7a,10,10a,10b,11,12,12a-tetradecahydrocyclopenta[5,6]naphtho[2,1-c]azepin-3(4*H*)-one; |
| (3a*S*,10a*R*,10b*S*,12a*S*)-1-(1*H*-imidazol-1-yl)-10a,12a-dimethyl-3,3a,3b,4,6,7,10,10a,10b,11,12,12a-dodecahydrocyclohepta[a]cyclopenta[f]naphthalen-8(9*H*)-one; |
| (3a*S*,10a*R*,10b*S*,12a*S*)-1-(1*H*-imidazol-1-yl)-10a,12a-dimethyl-3,3a,3b,4,8,9,10,10a,10b,11,12,12a-dodecahydrocyclohepta[a]cyclopenta[f]naphthalen-7(6*H*)-one; |
| (3*S*,10*S*,13*S*)-17-(1*H*-imidazol-1-yl)-10,13-dimethyl-15-oxo-2,3,4,5,6,7,8,9,10,11,12,13,14,15-tetradecahydro-1*H-*cyclopenta[a]phenanthren-3-yl acetate; |
| (10*R*,13*S*)-17-(1*H*-imidazol-1-yl)-10,13-dimethyl-6,7,8,9,10,11,12,13-octahydro-1*H*-cyclopenta[a]phenanthren-3(2*H*)-one; |
| (5a*R*,7a*S*)-8-(1*H*-imidazol-1-yl)-5a,7a-dimethyl-4,5,5a,5b,6,7,7a,10b,11,12-decahydrocyclopenta[5,6]naphtho[2,1-c]azepin-3(2*H*)-one; |
| (3a*S*,3b*S*,5a*S*,8a*S*,8b*R*,10a*S*)-6-(1*H*-imidazol-1-yl)-3a,5a-dimethyl-3,3a,3b,4,5,5a,8,8a,8b,9,10,10a-dodecahydrodicyclopenta[a,f]naphthalen-2(1*H*)-one; |
| (3a*S*,5a*S*)-3a,5a-dimethyl-6-(1*H*-1,2,3-triazol-1-yl)-3,3a,3b,4,5,5a,8,8a,8b,9,10,10a-dodecahydrodicyclopenta[a,f]naphthalen-2(1*H*)-one; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl acetate; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-ol; |
| (3*S*,10*R*,13*S*)-17-(5-methoxypyridin-3-yl)-10,13-dimethyl-2,3,4,7,8,9,10,11,12,13-decahydro-1*H-*cyclopenta[a]phenanthren-3-yl acetate; |
| (3*S*,10*R*,13*S*)-17-(5-methoxypyridin-3-yl)-10,13-dimethyl-2,3,4,7,8,9,10,11,12,13-decahydro-1*H-*cyclopenta[a]phenanthren-3-ol; |
| (10*R*,13*S*)-10,13-dimethyl-17-(pyridin-3-yl)-6,7,8,9,10,11,12,13-octahydro-1*H*-cyclopenta[a]phenanthren-3(2*H*)-one; |
| (10*R*,13*S*)-17-(5-methoxypyridin-3-yl)-10,13-dimethyl-6,7,8,9,10,11,12,13-octahydro-1*H-*cyclopenta[a]phenanthren-3(2*H*)-one; |
| (10*R*,13*S*)-17-(1*H*-imidazol-1-yl)-10,13-dimethyl-2,3,4,5,6,7,8,9,10,11,12,13,14,15-tetradecahydro-1*H-*cyclopenta[a]phenanthren-6-ol; |
| (5a*S*,7a*S*)-5a,7a-dimethyl-8-(1*H*-1,2,3-triazol-1-yl)-1,2,3,4,5,5a,5b,6,7,7a,10,10a,10b,11,12,12a-hexadecahydrocyclopenta[5,6]naphtho[1,2-d]azepine; |
| (5a*R*,7a*S*)-5a,7a-dimethyl-8-(1*H*-1,2,3-triazol-1-yl)-1,2,3,4,5,5a,5b,6,7,7a,10,10a,10b,11,12,12a-hexadecahydrocyclopenta[5,6]naphtho[2,1-c]azepine; |
| (3a*S*,5a*S*)-6-(1*H*-imidazol-1-yl)-3a,5a-dimethyl-1,2,3,3a,3b,4,5,5a,8,8a,8b,9,10,10a-tetradecahydrodicyclopenta[a,f]naphthalen-2-ol; |
| (5*S*,8*R*,9*S*,10*S*,13*S*)-10,13-dimethyl-17-(pyridin-3-yl)-4,5,6,7,8,9,10,11,12,13-decahydro-1*H-*cyclopenta[a]phenanthren-3(2*H*)-one; |
| (5a*S*,5b*S*,7a*S*,10b*R*,12a*S*)-5a,7a-dimethyl-8-(pyridin-3-yl)-3,4,5,5a,5b,6,7,7a,10b,11,12,12a-dodecahydrocyclopenta[5,6]naphtho[1,2-d]azepin-2(1*H*)-one; |
| (5a*R*,5b*S*,7a*S*,10b*R*,12a*R*)-5a,7a-dimethyl-8-(pyridin-3-yl)-1,2,5,5a,5b,6,7,7a,10b,11,12,12a-dodecahydrocyclopenta[5,6]naphtho[2,1-c]azepin-3(4*H*)-one; |
| (5a*R*,7a*S*)-2,5a,7a-trimethyl-8-(pyridin-3-yl)-4,5,5a,5b,6,7,7a,10b,11,12-decahydrocyclopenta[5,6]naphtho[2,1-c]azepin-3(2*H*)-one; |
| (5a*R*,7a*S*)-8-(5-methoxypyridin-3-yl)-2,5a,7a-trimethyl-4,5,5a,5b,6,7,7a,10b,11,12-decahydrocyclopenta[5,6]naphtho[2,1-c]azepin-3(2*H*)-one; |
| (5*S*,8*R*,9*S*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-yl acetate; |
| (5*S*,8*R*,9*S*,10*S*,13*S*)-17-(1*H*-imidazol-1-yl)-10,13-dimethyl-2,3,4,5,6,7,8,9,10,11,12,13-dodecahydro-1*H-*cyclopenta[a]phenanthren-3-yl acetate; |
| (5*S*,8*R*,9*S*,10*S*,13*S*)-17-(1*H*-imidazol-1-yl)-10,13-dimethyl-2,3,4,5,6,7,8,9,10,11,12,13-dodecahydro-1*H-*cyclopenta[a]phenanthren-3-ol; |
| (5*S*,8*R*,9*S*,10*S*,13*S*)-17-(1*H*-imidazol-1-yl)-10,13-dimethyl-4,5,6,7,8,9,10,11,12,13-decahydro-1*H-*cyclopenta[a]phenanthren-3(2*H*)-one; |
| (5a*R*,7a*S*)-2,5a,7a-trimethyl-8-(5-methylpyridin-3-yl)-4,5,5a,5b,6,7,7a,10b,11,12-decahydrocyclopenta[5,6]naphtho[2,1-c]azepin-3(2*H*)-one; |
| (3*S*,8*R*,9*S*,10*S*,13*S*)-10,13-dimethyl-17-(1*H*-1,2,3-triazol-1-yl)-2,3,4,5,6,7,8,9,10,11,12,13-dodecahydro-1*H-*cyclopenta[a]phenanthren-3-ol; |
| (5a*S*,5b*S*,7a*S*,10b*R*)-5a,7a-dimethyl-8-(1*H*-1,2,3-triazol-1-yl)-3,4,5,5a,5b,6,7,7a,10b,11,12,12a-dodecahydrocyclopenta[5,6]naphtho[1,2-d]azepin-2(1*H*)-one; |
| (5a*R*,5b*S*,7a*S*,10b*R*)-5a,7a-dimethyl-8-(1*H*-1,2,3-triazol-1-yl)-1,2,5,5a,5b,6,7,7a,10b,11,12,12a-dodecahydrocyclopenta[5,6]naphtho[2,1-c]azepin-3(4*H*)-one; |
| (3*S*,5*S*,8*R*,9*S*,10*S*,13*S*,14*S*)-10,13-dimethyl-17-(1*H*-1,2,3-triazol-1-yl)-2,3,4,5,6,7,8,9,10,11,12,13,14,15-tetradecahydro-1*H*-cyclopenta[a]phenanthren-3-yl acetate; |
| 1-((5a*R*,5b*S*,7a*S*,10a*S*,10b*R*,12a*R*)-5a,7a-dimethyl-8-(1*H*-1,2,3-triazol-1-yl)-3,4,5,5a,5b,6,7,7a,10,10a,10b,11,12,12a-tetradecahydrocyclopenta[5,6]naphtho[2,1-c]azepin-2(1*H*)-yl)ethanone; |
| (3a*S*,5a*S*)-3a,5a-dimethyl-6-(1*H*-1,2,3-triazol-1-yl)-1,2,3,3a,3b,4,5,5a,8,8a,8b,9,10,10a-tetradecahydrodicyclopenta[a,f]naphthalen-2-ol; |
| (5a*R*,7a*S*)-5a,7a-dimethyl-8-(pyridin-3-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-2,5a,7a-trimethyl-8-(5-methylpyridin-3-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-8-(1*H*-imidazol-1-yl)-2,5a,7a-trimethyl-4,5,5a,5b,6,7,7a,10b,11,12-decahydrocyclopenta[5,6]naphtho[2,1-c]azepin-3(2*H*)-one; |
| (5*S*,8*R*,9*S*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-ol; |
| (5a*R*,7a*S*)-8-(5-ethylpyridin-3-yl)-5a,7a-dimethyl-4,5,5a,5b,6,7,7a,10,10a,10b,11,12-dodecahydrocyclopenta[5,6]naphtho[2,1-c]azepin-3(2*H*)-one; |
| (5aR,7aS)-8-(5-methoxypyridin-3-yl)-2,5a,7a-trimethyl-4,5,5a,5b,6,7,7a,10,10a,10b,11,12-dodecahydrocyclopenta[5,6]naphtho[2,1-c]azepin-3(2*H*)-one; |
| (5a*R*,7a*S*)-8-(5-methoxypyridin-3-yl)-5a,7a-dimethyl-4,5,5a,5b,6,7,7a,10,10a,10b,11,12-dodecahydrocyclopenta[5,6]naphtho[2,1-c]azepin-3(2*H*)-one; |
| (5a*R*,7a*S*)-5a,7a-dimethyl-8-(5-methylpyridin-3-yl)-4,5,5a,5b,6,7,7a,10,10a,10b,11,12-dodecahydrocyclopenta[5,6]naphtho[2,1-c]azepin-3(2*H*)-one; |
| (5a*R*,7a*S*)-2,5a,7a-trimethyl-8-(pyridin-3-yl)-4,5,5a,5b,6,7,7a,10,10a,10b,11,12-dodecahydrocyclopenta[5,6]naphtho[2,1-c]azepin-3(2*H*)-one; |
| (10*R*,13*S*)-10,13-dimethyl-17-(pyridin-3-yl)-2,3,4,5,6,7,8,9,10,11,12,13,14,15-tetradecahydro-1*H-*cyclopenta[a]phenanthren-6-ol; |
| (10*R*,13*S*)-10,13-dimethyl-17-(pyridin-3-yl)-2,3,4,5,7,8,9,11,12,13,14,15-dodecahydro-1*H-*cyclopenta[a]phenanthren-6(10*H*)-one; |
| (5a*R*,7a*S*)-8-(5-methoxypyridin-3-yl)-5a,7a-dimethyl-4,5,5a,5b,6,7,7a,10b,11,12-decahydrocyclopenta[5,6]naphtho[2,1-c]azepin-3(2*H*)-one; |
| (5a*R*,7a*S*)-5a,7a-dimethyl-8-(pyridin-3-yl)-4,5,5a,5b,6,7,7a,10b,11,12-decahydrocyclopenta[5,6]naphtho[2,1-c]azepin-3(2*H*)-one; |
| (10*R*,13*S*)-17-(1*H*-imidazol-1-yl)-10,13-dimethyl-2,3,4,5,7,8,9,11,12,13,14,15-dodecahydro-1*H-*cyclopenta[a]phenanthren-6(10*H*)-one; |
| (10*R*,13*S*)-10,13-dimethyl-17-(pyridin-3-yl)-2,3,5,6,7,8,9,11,12,13,14,15-dodecahydro-1*H-*cyclopenta[a]phenanthren-4(10*H*)-one; |
| (5a*R*,7a*S*)-5a,7a-dimethyl-8-(1*H*-tetrazol-1-yl)-4,5,5a,5b,6,7,7a,10,10a,10b,11,12-dodecahydrocyclopenta[5,6]naphtho[2,1-c]azepin-3(2*H*)-one; |
| (3*S*,8*R*,9*S*,10*S*,13*S*)-10,13-dimethyl-17-(1*H*-1,2,3-triazol-1-yl)-2,3,4,5,6,7,8,9,10,11,12,13-dodecahydro-1*H-*cyclopenta[a]phenanthren-3-yl acetate; |
| (8*R*,9*S*,10*S*,13*S*)-10,13-dimethyl-17-(1*H*-1,2,3-triazol-1-yl)-4,5,6,7,8,9,10,11,12,13-decahydro-1*H-*cyclopenta[a]phenanthren-3(2*H*)-one; |
| (5a*R*,7a*S*)-8-(1*H*-imidazol-1-yl)-5a,7a-dimethyl-4,5,5a,5b,6,7,7a,10,10a,10b,11,12-dodecahydrocyclopenta[5,6]naphtho[2,1-c]azepin-3(2*H*)-one; |
| (4a*R*,6a*S*)-7-(1*H*-imidazol-1-yl)-1,4a,6a-trimethyl-4,4a,4b,5,6,6a,9,9a,9b,10-decahydro-1*H*-indeno[5,4-f]quinolin-2(3*H*)-one; |
| (5a*R*,7a*S*)-1,5a,7a-trimethyl-8-(pyridin-3-yl)-3,4,5,5a,5b,6,7,7a,10,10a,10b,11-dodecahydrocyclopenta[5,6]naphtho[2,1-b]azepin-2(1*H*)-one; |
| (5a*R*,7a*S*)-8-(5-methoxypyridin-3-yl)-1,5a,7a-trimethyl-3,4,5,5a,5b,6,7,7a,10,10a,10b,11-dodecahydrocyclopenta[5,6]naphtho[2,1-b]azepin-2(1*H*)-one; |
| (5a*S*,5b*S*,7a*S*,10b*R*,12a*S*)-8-(1*H*-imidazol-1-yl)-5a,7a-dimethyl-3,4,5,5a,5b,6,7,7a,10b,11,12,12a-dodecahydrocyclopenta[5,6]naphtho[1,2-d]azepin-2(1*H*)-one; |
| (5a*R*,5b*S*,7a*S*,10b*R*,12a*R*)-8-(1*H*-imidazol-1-yl)-5a,7a-dimethyl-1,2,5,5a,5b,6,7,7a,10b,11,12,12a-dodecahydrocyclopenta[5,6]naphtho[2,1-c]azepin-3(4*H*)-one; |
| (5a*R*,7a*S*)-1,5a,7a-trimethyl-8-(5-methylpyridin-3-yl)-3,4,5,5a,5b,6,7,7a,10,10a,10b,11-dodecahydrocyclopenta[5,6]naphtho[2,1-b]azepin-2(1*H*)-one; |
| 1-((3a*R*,5a*S*)-10-methoxy-3a,5a-dimethyl-1,1a,2,3,3a,3b,4,5,5a,8,8a,8b,9,10-tetradecahydrocyclopenta[a]cyclopropa[2,3]cyclopenta[1,2-f]naphthalen-6-yl)-1*H*-imidazole; |
| 3-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)pyridine; |
| 3-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-5-methylpyridine; |
| (3*S*,5*S*,8*R*,9*S*,10*S*,13*S*,14*S*)-10,13-dimethyl-17-(1*H*-1,2,3-triazol-1-yl)-2,3,4,5,6,7,8,9,10,11,12,13,14,15-tetradecahydro-1*H*-cyclopenta[a]phenanthren-3-ol; |
| 1-((5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-1*H*-1,2,3-triazole; |
| (5a*R*,7a*S*)-3,5a,7a-trimethyl-8-(pyrazin-2-yl)-3,4,5,5a,5b,6,7,7a,11,12-decahydrocyclopenta[5,6]naphtho[1,2-d]azepin-2(10b*H*)-one; |
| (3a*R*,5a*S*)-1,3a,5a-trimethyl-6-(pyridin-3-yl)-3,3a,3b,4,5,5a,8,8a,8b,9-decahydroindeno[5,4-e]indol-2(1*H*)-one; |
| (3a*R*,5a*S*)-6-(1*H*-imidazol-1-yl)-1,3a,5a-trimethyl-3,3a,3b,4,5,5a,8,8a,8b,9-decahydroindeno[5,4-e]indol-2(1*H*)-one; |
| 3-((10*R*,13*S*)-3-methoxy-10,13-dimethyl-2,3,4,7,8,9,10,11,12,13,14,15-dodecahydro-1*H-*cyclopenta[a]phenanthren-17-yl)pyridine; |
| 3-((10*R*,13*S*)-3-methoxy-10,13-dimethyl-2,3,4,7,8,9,10,11,12,13,14,15-dodecahydro-1*H-*cyclopenta[a]phenanthren-17-yl)-5-methylpyridine; |
| (3a*R*,5a*S*)-6-(5-methoxypyridin-3-yl)-1,3a,5a-trimethyl-3,3a,3b,4,5,5a,8,8a,8b,9-decahydroindeno[5,4-e]indol-2(1*H*)-one; |
| (3a*R*,5a*S*)-1,3a,5a-trimethyl-6-(5-methylpyridin-3-yl)-3,3a,3b,4,5,5a,8,8a,8b,9-decahydroindeno[5,4-e]indol-2(1*H*)-one; |
| 3-ethyl-5-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H*-cyclopenta[a]phenanthren-17-yl)pyridine; |
| 3-methoxy-5-((3*S*,5*S*,8*R*,9*S,*10*S,*13*S,*14*S*)-3-methoxy-10,13-dimethyl-2,3,4,5,6,7,8,9,10,11,12,13,14,15-tetradecahydro-1*H*-cyclopenta[a]phenanthren-17-yl)pyridine; |
| 3-ethoxy-5-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H*-cyclopenta[a]phenanthren-17-yl)pyridine; |
| 3-((3*S*,5*S*,8*R*,9*S*,10*S*,13*S*,14*S*)-3-methoxy-10,13-dimethyl-2,3,4,5,6,7,8,9,10,11,12,13,14,15-tetradecahydro-1*H-*cyclopenta[a]phenanthren-17-yl)-4-methylpyridine; |
| (10*S*,13*S*)-*N*,10,13-trimethyl-17-(5-methylpyridin-3-yl)-2,3,4,5,6,7,8,9,10,11,12,13,14,15-tetradecahydro-1*H-*cyclopenta[a]phenanthren-3-amine; |
| 1-((3*S*,10*R*,13*S*)-3-methoxy-10,13-dimethyl-2,3,4,7,8,9,10,11,12,13,14,15-dodecahydro-1*H-*cyclopenta[a]phenanthren-17-yl)-1*H*-imidazole; |
| (10*S*,13*S*)-*N*,10,13-trimethyl-17-(pyridin-3-yl)-2,3,4,5,6,7,8,9,10,11,12,13,14,15-tetradecahydro-1*H-*cyclopenta[a]phenanthren-3-amine; |
| (3a*R*,5a*S*)-6-(5-ethoxypyridin-3-yl)-1,3a,5a-trimethyl-3,3a,3b,4,5,5a,8,8a,8b,9-decahydroindeno[5,4-e]indol-2(1*H*)-one; |
| N-((10*S*,13*S*)-10,13-dimethyl-17-(5-methylpyridin-3-yl)-2,3,4,5,6,7,8,9,10,11,12,13,14,15-tetradecahydro-1*H-*cyclopenta[a]phenanthren-3-yl)-*N*-methylacetamide |
| (3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-amine; |
| *N*-((10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)-*N*-methylacetamide; |
| *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)acetamide; |
| (5a*S*,5b*S*,7a*S*,10b*R*)-5a,7a-dimethyl-8-(5-methylpyridin-3-yl)-3,4,5,5a,5b,6,7,7a,10b,11,12,12a-dodecahydrocyclopenta[5,6]naphtho[1,2-d]azepin-2(1*H*)-one; |
| (5a*R*,5b*S*,7a*S*,10b*R*)-5a,7a-dimethyl-8-(5-methylpyridin-3-yl)-1,2,5,5a,5b,6,7,7a,10b,11,12,12a-dodecahydrocyclopenta[5,6]naphtho[2,1-c]azepin-3(4*H*)-one; |
| 3-((3*R*,10*S*,13*S*)-3-azido-10,13-dimethyl-2,3,4,5,6,7,8,9,10,11,12,13,14,15-tetradecahydro-1*H-*cyclopenta[a]phenanthren-17-yl)pyridine; |
| (3*S*,10*R*,13*S*)-10,13-dimethyl-17-(4-methylpyridin-3-yl)-2,3,4,7,8,9,10,11,12,13-decahydro-1*H-*cyclopenta[a]phenanthren-3-ol; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl isobutyrate; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 2-(dimethylamino)acetate; |
| (9a*R*,11a*S*)-5,9a,11a-trimethyl-1-(4-methylpyridin-3-yl)-3b,4,5,8,9,9a,9b,10,11,11a-decahydro-3*H-*cyclopenta[i]phenanthridin-7(3a*H*)-one; |
| *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)isobutyramide; |
| *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)-2,2,2-trifluoroacetamide; |
| (5a*S*,5b*S*,7a*S*,10a*S*,10b*R*,12a*S*)-5a,7a-dimethyl-8-(4-methylpyridin-3-yl)-3,4,5,5a,5b,6,7,7a,10,10a,10b,11,12,12a-tetradecahydrocyclopenta[5,6]naphtho[1,2-d]azepin-2(1*H*)-one; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl pivalate; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)acetamide; |
| *N*-((3*R*,10*S*,13*S*)-10,13-dimethyl-17-(4-methylpyridin-3-yl)-2,3,4,5,6,7,8,9,10,11,12,13,14,15-tetradecahydro-1*H-*cyclopenta[a]phenanthren-3-yl)acetamide; |
| *N*-((3*R*,10*R*,13*S*)-10,13-dimethyl-17-(pyridin-3-yl)-2,3,4,7,8,9,10,11,12,13-decahydro-1*H-*cyclopenta[a]phenanthren-3-yl)acetamide; |
| *N-*((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)acetamide; |
| (3a*R*,5a*S*)-6-(1*H*-imidazol-1-yl)-*N*,3a,5a-trimethyl-1,1a,2,3,3a,3b,4,5,5a,8,8a,8b,9,10-tetradecahydrocyclopenta[a]cyclopropa[2,3]cyclopenta[1,2-f]naphthalen-10-amine; |
| N-((3*R*,10*S*,13*S*)-17-(5-methoxypyridin-3-yl)-10,13-dimethyl-2,3,4,5,6,7,8,9,10,11,12,13,14,15-tetradecahydro-1*H*-cyclopenta[a]phenanthren-3-yl)acetamide; |
| (5a*S*,7a*S*)-5a,7a-dimethyl-8-(4-methylpyridin-3-yl)-3,4,5,5a,5b,6,7,7a,10b,11,12,12a-dodecahydrocyclopenta[5,6]naphtho[1,2-d]azepin-2(1*H*)-one; |
| (5a*R*,7a*S*)-5a,7a-dimethyl-8-(4-methylpyridin-3-yl)-1,2,5,5a,5b,6,7,7a,10b,11,12,12a-dodecahydrocyclopenta[5,6]naphtho[2,1-c]azepin-3(4*H*)-one; |
| (3*S*,10*S*,13*S*)-17-(4-ethylpyridin-3-yl)-10,13-dimethyl-2,3,4,5,6,7,8,9,10,11,12,13-dodecahydro-1*H-*cyclopenta[a]phenanthren-3-ol; |
| (3*S*,10*S*,13*S*)-10,13-dimethyl-17-(4-methylpyridin-3-yl)-2,3,4,5,6,7,8,9,10,11,12,13-dodecahydro-1*H-*cyclopenta[a]phenanthren-3-yl acetate; |
| methyl ((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)carbamate; |
| *N*-((3*R*,10*S*,13*S*)-10,13-dimethyl-17-(pyridin-3-yl)-2,3,4,5,6,7,8,9,10,11,12,13-dodecahydro-1*H-*cyclopenta[a]phenanthren-3-yl)acetamide; |
| *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)cyclopropanecarboxamide; |
| *N*-((3*R*,10*R*,13*S*)-17-(1*H*-imidazol-1-yl)-10,13-dimethyl-2,3,4,7,8,9,10,11,12,13-decahydro-1*H-*cyclopenta[a]phenanthren-3-yl)acetamide; |
| *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)cyclopropanecarboxamide; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclopropanecarboxamide; |
| (5a*S*,7a*S*)-8-(4-ethylpyridin-3-yl)-5a,7a-dimethyl-3,4,5,5a,5b,6,7,7a,10b,11,12,12a-dodecahydrocyclopenta[5,6]naphtho[1,2-d]azepin-2(1*H*)-one; |
| (5a*R*,7a*S*)-8-(4-ethylpyridin-3-yl)-5a,7a-dimethyl-1,2,5,5a,5b,6,7,7a,10b,11,12,12a-dodecahydrocyclopenta[5,6]naphtho[2,1-c]azepin-3(4*H*)-one; |
| methyl((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)carbamate; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 2-methylbenzoate; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)methanesulfonamide; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclobutanecarboxamide; |
| *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)cyclobutanecarboxamide; |
| (3*R*,10*R*,13*S*)-10,13-dimethyl-17-(pyridin-3-yl)-2,3,4,7,8,9,10,11,12,13-decahydro-1*H*-cyclopenta[a]phenanthren-3-ol; |
| 1-((5a*R*,5b*S*,7a*S*,10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)propan-1-one; |
| (5a*R*,5b*S*,7a*S*,10a*S*,10b*R*,12a*R*)-methyl 8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepine;-2(1*H*)-carboxylate; |
| (5a*S*,5b*S*,7a*S*,10a*S*,10b*R*,12a*S*)-8-(4-ethylpyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one; |
| (5a*R*,5b*S*,7a*S*,10a*S*,10b*R*,12a*R*)-8-(4-ethylpyridin-3-yl)-5a,7a-dimethyl-1,2,5,5a,5b,6,7,7a,10,10a,10b,11,12,12a-tetradecahydrocyclopenta[5,6]naphtho[2,1-c]azepin-3(4*H*)-one; |
| (3a*R*,5a*S*)-3a,5a,dimethyl-6-(pyridin-3-yl)-1,1a,2,3,3a,3b,4,5,5a,8b,9,10-dodecahydrocyclopenta[a]cyclopropa[2,3]cyclopenta[1,2-f]naphthalen-10-ol; |
| *N*-((3*R*,10*R*,13*S*)-10,13-dimethyl-17-(5-methylpyridin-3-yl)-2,3,4,7,8,9,10,11,12,13,14,15-dodecahydro-1*H-*cyclopenta[a]phenanthren-3-yl)cyclopropanecarboxamide; |
| *N-*((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)cyclopropanesulfonamide; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 2,6-dimethylbenzoate; |
| 3-((10*R*,13*S*)-10,13-dimethyl-2,7,8,9,10,11,12,13-octahydro-1*H*-cyclopenta[a]phenanthren-17-yl)pyridine; |
| (3*R*,10*R*,13*S*)-10,13-dimethyl-17-(pyridin-3-yl)-2,3,4,7,8,9,10,11,12,13-decahydro-1*H*-cyclopenta[a]phenanthren-3-yl 2,6-dimethylbenzoate; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)thiazole-2-carboxamide; |
| *N-*((3*R*,10*R*,13*S*)-17-(5-methoxypyridin-3-yl)-10,13-dimethyl-2,3,4,7,8,9,10,11,12,13,14,15-dodecahydro-1*H-*cyclopenta[a]phenanthren-3-yl)cyclopropanecarboxamide; |
| (3a*R*,5a*S*)-3a,5a-dimethyl-6-(pyridin-3-yl)-1a,2,3,3a,3b,4,5,5a,8b,9-decahydrocyclopenta[a]cyclopropa[2,3]cyclopenta[1,2-f]naphthalen-10(1*H*)-one; |
| *N*-((3*R*,10*R*,13*S*)-10,13-dimethyl-17-(5-(trifluoromethyl)pyridin-3-yl)-2,3,4,7,8,9,10,11,12,13,14,15-dodecahydro-1*H*-cyclopenta[a]phenanthren-3-yl)acetamide; |
| 1-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)-3-ethylurea; |
| *N*-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)thiazole-2-carboxamide; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 2-(tert-butyl)benzoate; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl ethylcarbamate; |
| *N*-((3*R*,10*R*,13*S*)-17-(1*H*-imidazol-1-yl)-10,13-dimethyl-2,3,4,7,8,9,10,11,12,13-decahydro-1*H-*cyclopenta[a]phenanthren-3-yl)cyclopropanecarboxamide; |
| 1-((10*R*,13*S*)-17-(1*H*-imidazol-1-yl)-10,13-dimethyl-2,3,4,7,8,9,10,11,12,13,14,15-dodecahydro-1*H-*cyclopenta[a]phenanthren-3-yl)-3-ethylurea; |
| 1-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)-3-ethylthiourea; |
| 1-ethyl-3-((10*R*,13*S*)-17-(5-methoxypyridin-3-yl)-10,13-dimethyl-2,3,4,7,8,9,10,11,12,13,14,15-dodecahydro-1*H-*cyclopenta[a]phenanthren-3-yl)urea; |
| *N*-((3a*R*,5a*S*)-3a,5a-dimethyl-6-(pyridin-3-yl)-1,1a,2,3,3a,3b,4,5,5a,8,8a,8b,9,10-tetradecahydrocyclopenta[a]cyclopropa[2,3]cyclopenta[1,2-f]naphthalen-10-yl)thiazol-2-amine; |
| 1-((3*R*,10*S*,13*S*)-17-(1*H*-imidazol-1-yl)-10,13-dimethyl-2,3,4,5,6,7,8,9,10,11,12,13,14,15-tetradecahydro-1*H-*cyclopenta[a]phenanthren-3-yl)-3-ethylurea; |
| (10*R*,13*S*)-10,13-dimethyl-17-(pyridin-3-yl)-6,7,8,9,10,11,12,13-octahydro-1*H*-cyclopenta[a]phenanthren-3(2*H*)-one oxime; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl tert-butylcarbamate; |
| (10*R*,13*S*)-10,13-dimethyl-17-(pyridin-3-yl)-6,7,8,9,10,11,12,13-octahydro-1*H*-cyclopenta[a]phenanthren-3(2*H*)-one O-methyl oxime; |
| (3*S*,9a*S*,11b*R*)-9a,11b-dimethyl-9-(pyridin-3-yl)-1,2,3,4,5a,6,6a,9a,10,11,11a,11b-dodecahydrocyclopenta[1,2]phenanthro[8a,9-b]oxiren-3-ol; |
| (10*R*,13*S*)-10,13-dimethyl-17-(pyrimidin-5-yl)-6,7,8,9,10,11,12,13-octahydro-1*H*-cyclopenta[a]phenanthren-3(2*H*)-one; |
| 3-((3*S*,10*S*,13*S*)-3-hydroxy-10,13-dimethylhexadecahydro-1*H*-cyclopenta[a]phenanthren-17-yl)pyridine; 1-oxide; |
| *N*-((3*R*,10*R*,13*S*)-10,13-dimethyl-17-(pyridin-3-yl)-2,3,4,7,8,9,10,11,12,13,14,15-dodecahydro-1*H-*cyclopenta[a]phenanthren-3-yl)butyramide; |
| (10*R*,13*S*)-10,13-dimethyl-17-(pyridin-3-yl)-6,7,8,9,10,11,12,13,14,15,16,17-dodecahydro-1*H-*cyclopenta[a]phenanthren-3(2*H*)-one; |
| (3*S*,10*S*,13*S*)-3-cyclopropyl-10,13-dimethyl-17-(pyridin-3-yl)-2,3,4,5,6,7,8,9,10,11,12,13,14,15-tetradecahydro-1*H*-cyclopenta[a]phenanthren-3-ol; |
| (3*R*,10*S*,13*S*)-3,10,13-trimethyl-17-(pyridin-3-yl)-2,3,4,5,6,7,8,9,10,11,12,13,14,15-tetradecahydro-1*H-*cyclopenta[a]phenanthren-3-ol; |
| (3*S*,10*S*,13*S*)-3,10,13-trimethyl-17-(pyridin-3-yl)-2,3,4,5,6,7,8,9,10,11,12,13,14,15-tetradecahydro-1*H-*cyclopenta[a]phenanthren-3-ol; |
| (3*R*,10*S*,13*S*)-3-ethyl-10,13-dimethyl-17-(pyridin-3-yl)-2,3,4,5,6,7,8,9,10,11,12,13,14,15-tetradecahydro-1*H-*cyclopenta[a]phenanthren-3-ol; |
| (3*S*,10*S*,13*S*)-3-ethyl-10,13-dimethyl-17-(pyridin-3-yl)-2,3,4,5,6,7,8,9,10,11,12,13,14,15-tetradecahydro-1*H-*cyclopenta[a]phenanthren-3-ol; |
| (10*R*,13*S*)-4,4,10,13-tetramethyl-17-(pyridin-3-yl)-4,7,8,9,10,11,12,13,14,15-decahydro-1*H-*cyclopenta[a]phenanthren-3(2*H*)-one; |
| (10*R*,13*S*)-4,4,10,13-tetramethyl-17-(pyridin-3-yl)-2,3,4,7,8,9,10,11,12,13,14,15-dodecahydro-1*H-*cyclopenta[a]phenanthren-3-ol; |
| (3*S*,10*S*,13*S*)-10,13-dimethyl-17-(pyridin-3-yl)hexadecahydro-1*H*-cyclopenta[a]phenanthren-3-ol; |
| 1-((5a*R*,5b*S*,7a*S*,10a*S*,10b*R*,12a*R*)-8-(1*H*-imidazol-1-yl)-5a,7a-dimethyl-3,4,5,5a,5b,6,7,7a,10,10a,10b,11,12,12a-tetradecahydrocyclopenta[5,6]naphtho[2,1-c]azepin-2(1*H*)-yl)ethanone; |
| (3*S*,10*S*,13*S*)-3,10,13-trimethyl-17-(pyridin-3-yl)-2,3,4,5,6,7,8,9,10,11,12,13-dodecahydro-1*H-*cyclopenta[a]phenanthren-3-ol; |
| (3*R*,10*S*,13*S*)-3,10,13-trimethyl-17-(pyridin-3-yl)-2,3,4,5,6,7,8,9,10,11,12,13-dodecahydro-1*H-*cyclopenta[a]phenanthren-3-ol; |
| (10*R*,13*S*)-4,4,10,13-tetramethyl-17-(pyridin-3-yl)-2,3,4,7,8,9,10,11,12,13-decahydro-1*H-*cyclopenta[a]phenanthren-3-ol; |
| (10*R,*13*S*)-10,13-dimethyl-17-(pyridin-3-yl)-8,9,10,11,12,13-hexahydro-1*H*-cyclopenta[a]phenanthren-3(2*H*)-one; |
| (3*S*,10*S*,13*S*)-3-ethyl-10,13-dimethyl-17-(pyridin-3-yl)-2,3,4,5,6,7,8,9,10,11,12,13-dodecahydro-1*H-*cyclopenta[a]phenanthren-3-ol; |
| (3*R*,10*S*,13*S*)-3-ethyl-10,13-dimethyl-17-(pyridin-3-yl)-2,3,4,5,6,7,8,9,10,11,12,13-dodecahydro-1*H-*cyclopenta[a]phenanthren-3-ol; |
| (3*S*,10*S*,13*S*)-3-ethynyl-10,13-dimethyl-17-(pyridin-3-yl)-2,3,4,5,6,7,8,9,10,11,12,13,14,15-tetradecahydro-1*H-*cyclopenta[a]phenanthren-3-ol; |
| (3*R*,10*S*,13*S*)-3-ethynyl-10,13-dimethyl-17-(pyridin-3-yl)-2,3,4,5,6,7,8,9,10,11,12,13,14,15-tetradecahydro-1*H-*cyclopenta[a]phenanthren-3-ol; |
| (10*R*,13*S*)-10,13-dimethyl-17-(pyrazin-2-yl)-6,7,8,9,10,11,12,13-octahydro-1*H*-cyclopenta[a]phenanthren-3(2*H*)-one; |
| 1-((5a*S*,7a*S*)-8-(1*H*-imidazol-1-yl)-5a,7a-dimethyl-1,2,5,5a,5b,6,7,7a,10,10a,10b,11,12,12a-tetradecahydrocyclopenta[5,6]naphtho[1,2-d]azepin-3(4*H*)-yl)butan-1-one; |
| 1-((5a*R*,7a*S*)-5a,7a-dimethyl-8-(pyridin-3-yl)-3,4,5,5a,5b,6,7,7a,10,10a,10b,11,12,12a-tetradecahydrocyclopenta[5,6]naphtho[2,1-c]azepin-2(1*H*)-yl)propan-1-one; |
| 1-((5a*S*,7a*S*)-5a,7a-dimethyl-8-(pyridin-3-yl)-1,2,5,5a,5b,6,7,7a,10,10a,10b,11,12,12a-tetradecahydrocyclopenta[5,6]naphtho[1,2-d]azepin-3(4*H*)-yl)propan-1-one; |
| (10*R*,13*S*)-6,10,13-trimethyl-17-(pyridin-3-yl)-6,7,8,9,10,11,12,13,14,15-decahydro-1*H-*cyclopenta[a]phenanthren-3(2*H*)-one; |
| (10*R*,13*S*)-10,13-dimethyl-17-(pyridin-3-yl)-6,7,8,9,10,11,12,13-octahydro-3*H*-cyclopenta[a]phenanthren-3-one; |
| (10*R*,13*S*)-4,10,13-trimethyl-17-(pyridin-3-yl)-6,7,8,9,10,11,12,13-octahydro-1*H*-cyclopenta[a]phenanthren-3(2*H*)-one; |
| (10*R*,13*S*)-10,13-dimethyl-6-methylene-17-(pyridin-3-yl)-6,7,8,9,10,11,12,13,14,15-decahydro-3*H-*cyclopenta[a]phenanthren-3-one; |
| (10*R*,13*S*)-4,10,13-trimethyl-17-(pyridin-3-yl)-2,3,6,7,8,9,10,11,12,13-decahydro-1*H*-cyclopenta[a]phenanthren-3-ol; |
| (10*R*,13*S*)-10,13-dimethyl-6-methylene-17-(pyridin-3-yl)-6,7,8,9,10,11,12,13,14,15-decahydro-1*H-*cyclopenta[a]phenanthren-3(2*H*)-one; |
| (10*R*,13*S*)-4-hydroxy-10,13-dimethyl-17-(pyridin-3-yl)-8,9,10,11,12,13-hexahydro-1*H*-cyclopenta[a]phenanthren-3(2*H*)-one; |
| (10*R*,13*S*)-4-hydroxy-10,13-dimethyl-17-(pyridin-3-yl)-8,9,10,11,12,13,14,15-octahydro-1*H-*cyclopenta[a]phenanthren-3(2*H*)-one; |
| (10*R*,13*S*)-10,13-dimethyl-17-(pyridin-3-yl)-1,7,8,9,10,11,12,13,14,15-decahydrospiro[cyclopenta[a]phenanthrene-6,1'-cyclopropan]-3(2*H*)-one; |
| (4a*R*,6a*S*)-4a,6a-dimethyl-7-(pyridin-3-yl)-1a,2,3,4,4a,4b,5,6,6a,9,9a,9b,10,11-tetradecahydro-1*H-*cyclopenta[a]cyclopropa[j]phenanthren-2-ol; |
| (10*R*,13*S*)-6,10,13-trimethyl-17-(pyridin-3-yl)-6,7,8,9,10,11,12,13,14,15-decahydro-1*H-*cyclopenta[a]phenanthren-3(2*H*)-one oxime; |
| (6c*R*,8a*S*)-10,13-dimethyl-17-(pyridin-3-yl)-1,7,8,9,10,11,12,13,14,15-decahydrospiro[cyclopenta[a]phenanthrene-6,1'-cyclopropan]-3(2*H*)-one oxime; |
| (10*R*,13*S*,E)-4,4,10,13-tetramethyl-17-(pyridin-3-yl)-4,7,8,9,10,11,12,13,14,15-decahydro-1*H-*cyclopenta[a]phenanthren-3(2*H*)-one oxime; |
| (5a*R*,7a*S*)-5a,7a,12-trimethyl-8-(pyridin-3-yl)-3,4,5,5a,5b,6,7,7a,10,10a,11,12-dodecahydrocyclopenta[5,6]naphtho[1,2-d]azepin-2(10b*H*)-one; |
| (5a*R*,7a*S*)-5a,7a-dimethyl-8-(pyridin-3-yl)-3,4,5,5a,5b,6,7,7a,10,10a,10b,11-dodecahydro-2*H-*spiro[cyclopenta[5,6]naphtho[1,2-d]azepine;-12,1'-cyclopropan]-2-one; |
| (10*R*,13*S*)-4,10,13-trimethyl-17-(pyridin-3-yl)-6,7,8,9,10,11,12,13,14,15-decahydro-1*H-*cyclopenta[a]phenanthren-3(2*H*)-one; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl pyridin-3-ylcarbamate; |
| (10*R*,13*S*)-6,10,13-trimethyl-17-(pyridin-3-yl)-6,7,8,9,10,11,12,13-octahydro-1*H*-cyclopenta[a]phenanthren-3(2*H*)-one; |
| (3*S*,10*R*,13*S*)-17-(5-fluoropyridin-3-yl)-10,13-dimethyl-2,3,4,7,8,9,10,11,12,13,14,15-dodecahydro-1*H-*cyclopenta[a]phenanthren-3-ol; |
| (10*R*,13*S*)-4,10,13-trimethyl-17-(pyridin-3-yl)-2,3,6,7,8,9,10,11,12,13,14,15-dodecahydro-1*H-*cyclopenta[a]phenanthren-3-ol; |
| (10*R*,13*S*,E)-4,10,13-trimethyl-17-(pyridin-3-yl)-6,7,8,9,10,11,12,13,14,15-decahydro-1*H-*cyclopenta[a]phenanthren-3(2*H*)-one oxime; |
| 5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)nicotinonitrile; |
| (3*S*,5*S*,10*R*,13*S*)-6-fluoro-10,13-dimethyl-17-(pyridin-3-yl)-2,3,4,5,6,7,8,9,10,11,12,13,14,15-tetradecahydro-1*H-*cyclopenta[a]phenanthrene-3,5-diol; |
| (3*S*,5*R*,10*R*,13*S*)-6-fluoro-10,13-dimethyl-17-(pyridin-3-yl)-2,3,4,5,6,7,8,9,10,11,12,13,14,15-tetradecahydro-1*H-*cyclopenta[a]phenanthrene-3,5-diol; |
| (3*S*,10*R*,13*S*)-17-(6-fluoropyridin-3-yl)-10,13-dimethyl-2,3,4,7,8,9,10,11,12,13,14,15-dodecahydro-1*H-*cyclopenta[a]phenanthren-3-ol; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 4-methylnicotinate; |
| (10*R*,13*S*)-10,13-dimethyl-17-(pyridin-3-yl)-7,8,9,11,12,13,14,15-octahydrospiro[cyclopenta[a]phenanthrene-6,1'-cyclopropan]-3(10*H*)-one; |
| (10*R*,13*S*)-6-fluoro-5-hydroxy-10,13-dimethyl-17-(pyridin-3-yl)-4,5,6,7,8,9,10,11,12,13,14,15-dodecahydro-1*H-*cyclopenta[a]phenanthren-3(2*H*)-one; |
| (10*R*,13*S*)-3,10,13-trimethyl-17-(pyridin-3-yl)-2,3,6,7,8,9,10,11,12,13,14,15-dodecahydro-1*H-*cyclopenta[a]phenanthren-3-ol; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 3-hydroxy-2-(hydroxymethyl)-2-methylpropanoate; |
| (6c*R*,8a*S*)-10,13-dimethyl-17-(pyridin-3-yl)-7,8,9,11,12,13,14,15-octahydrospiro[cyclopenta[a]phenanthrene-6,1'-cyclopropan]-3(10*H*)-one oxime; |
| (3a*S*,5b*R*,8*S*)-3a,5b-dimethyl-3-(pyridin-3-yl)-3a,4,5,5a,5b,6,7,8,9,10,10a,11,11a,11b-tetradecahydro-1*H-*cyclopenta[a]cyclopropa[k]phenanthren-8-ol; |
| (10*R*,13*S*)-10,13-dimethyl-17-(pyridin-3-yl)-8,9,10,11,12,13,14,15-octahydro-1*H*-cyclopenta[a]phenanthren-3(2*H*)-one; |
| (10*R*,13*S*)-6-fluoro-10,13-dimethyl-17-(pyridin-3-yl)-6,7,8,9,10,11,12,13,14,15-decahydro-1*H-*cyclopenta[a]phenanthren-3(2*H*)-one oxime; |
| (10*R*,13*S*)-6,10,13-trimethyl-17-(pyridin-3-yl)-6,7,8,9,10,11,12,13,14,15-decahydro-3*H-*cyclopenta[a]phenanthren-3-one; |
| (4a*R*,6a*S*)-4a,6a-dimethyl-7-(pyridin-3-yl)-4,4a,4b,5,6,6a,9,9a,9b,9c,10,10a-dodecahydrocyclopenta[a]cyclopropa[I]phenanthren-2(3*H*)-one; |
| 3-((10*S*,13*S*)-10,13-dimethyl-1,2,4,5,6,7,8,9,10,11,12,13,14,15-tetradecahydrospiro[cyclopenta[a]phenanthrene-3,2'-oxiran]-17-yl)pyridine; |
| (10*R*,13*S*)-3,6,10,13-tetramethyl-17-(pyridin-3-yl)-2,3,6,7,8,9,10,11,12,13,14,15-dodecahydro-1*H-*cyclopenta[a]phenanthren-3-ol; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl 1-methylpiperidine;-4-carboxylate; |
| (3*R*,10*S*,13*S*)-3,6,10,13-tetramethyl-17-(pyridin-3-yl)-2,3,4,5,6,7,8,9,10,11,12,13,14,15-tetradecahydro-1*H-*cyclopenta[a]phenanthren-3-ol; |
| (3*S*,10*S*,13*S*)-3,6,10,13-tetramethyl-17-(pyridin-3-yl)-2,3,4,5,6,7,8,9,10,11,12,13,14,15-tetradecahydro-1*H-*cyclopenta[a]phenanthren-3-ol; |
| (5a*R*,7a*S*)-12-fluoro-5a,7a-dimethyl-8-(pyridin-3-yl)-3,4,5,5a,5b,6,7,7a,10,10a,11,12-dodecahydrocyclopenta[5,6]naphtho[1,2-d]azepin-2(10b*H*)-one; |
| (10*R*,13*S*)-4,10,13-trimethyl-17-(pyridin-3-yl)-4,5,6,7,8,9,10,11,12,13,14,15-dodecahydro-1*H-*cyclopenta[a]phenanthren-3(2*H*)-one; |
| (10*R*,13*S*)-6,10,13-trimethyl-17-(pyridin-3-yl)-6,7,8,9,10,11,12,13,14,15-decahydro-3*H-*cyclopenta[a]phenanthren-3-one oxime; |
| 1-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)-1*H*-imidazole-5-carbonitrile; |
| methyl 1-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)-1*H*-imidazole-5-carboxylate; |
| (10*R*,13*S*)-17-(5-fluoropyridin-3-yl)-10,13-dimethyl-6-methylene-6,7,8,9,10,11,12,13,14,15-decahydro-3*H-*cyclopenta[a]phenanthren-3-one; |
| (10*S*,13*S*)-3-(3-hydroxypropyl)-10,13-dimethyl-17-(pyridin-3-yl)-2,3,4,5,6,7,8,9,10,11,12,13,14,15-tetradecahydro-1*H*-cyclopenta[a]phenanthren-3-ol; |
| (10*R*,13*S*)-4,10,13-trimethyl-17-(pyridin-3-yl)-2,3,4,5,6,7,8,9,10,11,12,13,14,15-tetradecahydro-1*H-*cyclopenta[a]phenanthren-3-ol; |
| (5a*R*,7a*S*)-5a,7a-dimethyl-8-(pyridin-3-yl)-3,4,5,5a,5b,6,7,7a,10,10a,10b,10c,11,11a-tetradecahydro-2*H-*cyclopenta[5,6]cyclopropa[3,4]naphtho[1,2-d]azepin-2-one; |
| 3-fluoro-5-((10*R*,13*S*)-3-fluoro-10,13-dimethyl-2,3,4,7,8,9,10,11,12,13,14,15-dodecahydro-1*H-*cyclopenta[a]phenanthren-17-yl)pyridine; |
| (3*S*,10*R*,13*S*)-17-(5-fluoropyridin-3-yl)-10,13-dimethyl-2,3,4,7,8,9,10,11,12,13-decahydro-1*H-*cyclopenta[a]phenanthren-3-ol; |
| (5a*R*,7a*S*)-1,5a,7a-trimethyl-8-(pyridin-3-yl)-1,2,5,5a,5b,6,7,7a,10,10a,10b,11,12,12a-tetradecahydrocyclopenta[5,6]naphtho[2,1-c]azepin-3(4*H*)-one; |
| (3*S*,10*R*,13*S*)-6-fluoro-17-(5-fluoropyridin-3-yl)-10,13-dimethyl-2,3,4,5,6,7,8,9,10,11,12,13,14,15-tetradecahydro-1*H*-cyclopenta[a]phenanthrene-3,5-diol; |
| (3*R*,10*S*,13*S*)-17-(5-fluoropyridin-3-yl)-3,10,13-trimethyl-2,3,4,5,6,7,8,9,10,11,12,13,14,15-tetradecahydro-1*H-*cyclopenta[a]phenanthren-3-ol; |
| (3*S*,10*S*,13*S*)-17-(5-fluoropyridin-3-yl)-3,10,13-trimethyl-2,3,4,5,6,7,8,9,10,11,12,13,14,15-tetradecahydro-1*H-*cyclopenta[a]phenanthren-3-ol; |
| (10*S*,13*S*)-10,13-dimethyl-17-(pyridin-3-yl)-1,2,4,5,6,7,8,9,10,11,12,13,14,15-tetradecahydro-3'*H-*spiro[cyclopenta[a]phenanthrene-3,2'-furan]-5'(4'*H*)-one; |
| *N*-((3*R,*10*R,*13*S*)-17-(5-fluoropyridin-3-yl)-10,13-dimethyl-2,3,4,7,8,9,10,11,12,13,14,15-dodecahydro-1*H-*cyclopenta[a]phenanthren-3-yl)acetamide; |
| 1-ethyl-3-((3*R*,10*R*,13*S*)-17-(5-fluoropyridin-3-yl)-10,13-dimethyl-2,3,4,7,8,9,10,11,12,13,14,15-dodecahydro-1*H-*cyclopenta[a]phenanthren-3-yl)urea; |
| (10*R*,13*S*)-17-(5-fluoropyridin-3-yl)-10,13-dimethyl-6,7,8,9,10,11,12,13-octahydro-1*H*-cyclopenta[a]phenanthren-3(2*H*)-one; |
| (10*R*,13*S*)-6-fluoro-17-(5-fluoropyridin-3-yl)-5-hydroxy-10,13-dimethyl-4,5,6,7,8,9,10,11,12,13,14,15-dodecahydro-1*H*-cyclopenta[a]phenanthren-3(2*H*)-one; |
| (3*S*,10*R*,13*S*)-17-(2-fluoro-5-methylpyridin-3-yl)-10,13-dimethyl-2,3,4,7,8,9,10,11,12,13,14,15-dodecahydro-1*H-*cyclopenta[a]phenanthren-3-ol; |
| (3*S*,10*R*,13*S*)-17-(6-fluoro-5-methylpyridin-3-yl)-10,13-dimethyl-2,3,4,7,8,9,10,11,12,13,14,15-dodecahydro-1*H-*cyclopenta[a]phenanthren-3-ol; |
| (10*R*,13*S*)-17-(6-fluoropyridin-3-yl)-10,13-dimethyl-6,7,8,9,10,11,12,13-octahydro-1*H*-cyclopenta[a]phenanthren-3(2*H*)-one; |
| (10*R*,13*S*)-6-fluoro-17-(5-fluoropyridin-3-yl)-10,13-dimethyl-6,7,8,9,10,11,12,13,14,15-decahydro-1*H-*cyclopenta[a]phenanthren-3(2*H*)-one; |
| (10*R*,13*S*)-17-(2-fluoro-5-methylpyridin-3-yl)-10,13-dimethyl-6,7,8,9,10,11,12,13,14,15-decahydro-1*H-*cyclopenta[a]phenanthren-3(2*H*)-one; |
| (10*R*,13*S*)-17-(6-fluoropyridin-3-yl)-6,10,13-trimethyl-6,7,8,9,10,11,12,13-octahydro-1*H-*cyclopenta[a]phenanthren-3(2*H*)-one; |
| (4a*R*,6a*S*)-7-(5-fluoropyridin-3-yl)-1,4a,6a-trimethyl-4,4a,4b,5,6,6a,9,9a,9b,10-decahydro-1*H*-indeno[5,4-f]quinolin-2(3*H*)-one; |
| (5a*R*,7a*S*)-8-(5-fluoropyridin-3-yl)-5a,7a-dimethyl-3,4,5,5a,5b,6,7,7a,11,12-decahydrocyclopenta[5,6]naphtho[1,2-d]azepin-2(10b*H*)-one; |
| (3*S*,10*R*,13*S*)-17-(5-(difluoromethyl)pyridin-3-yl)-10,13-dimethyl-2,3,4,7,8,9,10,11,12,13,14,15-dodecahydro-1*H-*cyclopenta[a]phenanthren-3-ol; |
| (5a*R*,7a*S*)-8-(6-fluoropyridin-3-yl)-5a,7a,12-trimethyl-3,4,5,5a,5b,6,7,7a,11,12-decahydrocyclopenta[5,6]naphtho[1,2-d]azepin-2(10b*H*)-one; |
| (3*S*,10*R*,13*S*)-17-(4-fluoropyridin-3-yl)-10,13-dimethyl-2,3,4,7,8,9,10,11,12,13,14,15-dodecahydro-1*H-*cyclopenta[a]phenanthren-3-ol; |
| (3*S*,10*R*,13*S*)-17-(2-fluoropyridin-3-yl)-10,13-dimethyl-2,3,4,7,8,9,10,11,12,13,14,15-dodecahydro-1*H-*cyclopenta[a]phenanthren-3-ol; |
| (10*R*,13*S*)-17-(5-(difluoromethyl)pyridin-3-yl)-10,13-dimethyl-6,7,8,9,10,11,12,13,14,15-decahydro-1*H-*cyclopenta[a]phenanthren-3(2*H*)-one; |
| (4a*R*,6a*S*)-7-(5-fluoropyridin-3-yl)-4a,6a-dimethyl-4,4a,4b,5,6,6a,9,9a,9b,9c,10,10a-dodecahydrocyclopenta[a]cyclopropa[I]phenanthren-2(3*H*)-one; |
| (10*R*,13*S*)-17-(5-fluoropyridin-3-yl)-10,13-dimethyl-8,9,10,11,12,13,14,15-octahydro-1*H-*cyclopenta[a]phenanthren-3(2*H*)-one; |
| (3*S*,10*R*,13*S*)-17-(5-(difluoromethyl)pyridin-3-yl)-10,13-dimethyl-2,3,4,7,8,9,10,11,12,13-decahydro-1*H-*cyclopenta[a]phenanthren-3-ol; |
| (5a*R*,7a*S*)-8-(5-(difluoromethyl)pyridin-3-yl)-5a,7a-dimethyl-3,4,5,5a,5b,6,7,7a,10,10a,11,12-dodecahydrocyclopenta[5,6]naphtho[1,2-d]azepin-2(10b*H*)-one; |
| (3*S*,10*R*,13*S*)-6,10,13-trimethyl-17-(pyridin-3-yl)-2,3,4,5,6,7, 8,9,10,11,12,13,14,15-tetradecahydro-1*H-*cyclopenta[a]phenanthrene-3,5-diol; |
| (10*R*,13*S*)-17-(5-(difluoromethyl)pyridin-3-yl)-10,13-dimethyl-6,7,8,9,10,11,12,13-octahydro-1*H-*cyclopenta[a]phenanthren-3(2*H*)-one; |
| (10*R*,13*S*)-17-(5-fluoropyridin-3-yl)-6,10,13-trimethyl-6,7,8,9,10,11,12,13-octahydro-1*H-*cyclopenta[a]phenanthren-3(2*H*)-one; |
| (5a*R*,7a*S*)-5a,7a-dimethyl-8-(pyridin-3-yl)-1a,2,4,5,5a,5b,6,7,7a,10,10a,10b,11,12-tetradecahydrocyclopenta[5,6]naphtho[2,1-c]cyclopropa[b]azepin-3(1*H*)-one; |
| (4a*R*,6a*S*)-7-(5-fluoropyridin-3-yl)-4a,6a-dimethyl-1a,2,3,4,4a,4b,5,6,6a,9,9a,9b,10,11-tetradecahydro-1*H-*cyclopenta[a]cyclopropa[j]phenanthren-2-ol; |
| (5a*R*,7a*S*)-8-(5-fluoropyridin-3-yl)-5a,7a-dimethyl-3,4,5,5a,5b,5,7,7a,10,10a,10b,10c,11,11a-tetradecahydro-2*H-*cyclopenta[5,6]cyclopropa[3,4]naphtho[1,2-d]azepin-2-one; |
| *N*-((10*R*,13*S*)-4,10,13-trimethyl-17-(pyridin-3-yl)-2,3,4,5,6,7,8,9,10,11,12,13,14,15-tetradecahydro-1*H-*cyclopenta[a]phenanthren-3-yl)acetamide; |
| 1-ethyl-3-((10*R*,13*S*)-4,10,13-trimethyl-17-(pyridin-3-yl)-2,3,4,5,6,7,8,9,10,11,12,13,14,15-tetradecahydro-1*H-*cyclopenta[a]phenanthren-3-yl)urea; |
| (4a*R*,6a*S*)-7-(5-(difluoromethyl)pyridin-3-yl)-1,4a,6a-trimethyl-4,4a,4b,5,6,6a,9,9a,9b,10-decahydro-1*H-*indeno[5,4-f]quinolin-2(3*H*)-one; |
| *N*-((3*R*,10*R*,13*S*)-17-(5-(difluoromethyl)pyridin-3-yl)-10,13-dimethyl-2,3,4,7,8,9,10,11,12,13,14,15-dodecahydro-1*H*-cyclopenta[a]phenanthren-3-yl)acetamide; |
| (10*R*,13*S*)-2,4,10,13-tetramethyl-17-(pyridin-3-yl)-4,5,6,7,8,9,10,11,12,13,14,15-dodecahydro-1*H-*cyclopenta[a]phenanthren-3(2*H*)-one; |
| (5a*R*,7a*S*)-8-(5-fluoropyridin-3-yl)-5a,7a,12-trimethyl-3,4,5,5a,5b,6,7,7a,11,12-decahydrocyclopenta[5,6]naphtho[1,2-d]azepin-2(10b*H*)-one; |
| (10*R*,13*S*)-2,4,10,13-tetramethyl-17-(pyridin-3-yl)-2,3,4,5,6,7,8,9,10,11,12,13,14,15-tetradecahydro-1*H-*cyclopenta[a]phenanthren-3-ol; |
| (4a*R*,6a*S*)-7-(4-fluoropyridin-3-yl)-1,4a,6a-trimethyl-4,4a,4b,5,6,6a,9,9a,9b,10-decahydro-1*H*-indeno[5,4-f]quinolin-2(3*H*)-one; |
| 1,1,1-trifluoro-*N*-(5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)pyridin-3-yl)methanesulfonamide; |
| (3*S*,10*R*,13*S*)-17-(5-aminopyridin-3-yl)-10,13-dimethyl-2,3,4,7,8,9,10,11,12,13,14,15-dodecahydro-1*H-*cyclopenta[a]phenanthren-3-ol; |
| (10*R*,13*S*)-6-cyclopropyl-17-(5-fluoropyridin-3-yl)-10,13-dimethyl-6,7,8,9,10,11,12,13,14,15-decahydro-1*H-*cyclopenta[a]phenanthren-3(2*H*)-one; |
| (5a*R*,7a*S*)-8-(5-(difluoromethyl)pyridin-3-yl)-5a,7a-dimethyl-3,4,5,5a,5b,6,7,7a,11,12-decahydrocyclopenta[5,6]naphtho[1,2-d]azepin-2(10b*H*)-one; |
| (3*S*,10*R*,13*S*)-17-(5-chloropyridin-3-yl)-10,13-dimethyl-2,3,4,7,8,9,10,11,12,13,14,15-dodecahydro-1*H-*cyclopenta[a]phenanthren-3-ol; |
| (10*R*,13*S*)-6-ethyl-17-(5-fluoropyridin-3-yl)-10,13-dimethyl-6,7,8,9,10,11,12,13,14,15-decahydro-1*H-*cyclopenta[a]phenanthren-3(2*H*)-one; |
| (10*R*,13*S*)-6-ethyl-10,13-dimethyl-17-(pyridin-3-yl)-6,7,8,9,10,11,12,13,14,15-decahydro-1*H-*cyclopenta[a]phenanthren-3(2*H*)-one; |
| (10*R*,13*S*)-17-(5-(difluoromethyl)pyridin-3-yl)-6-ethyl-10,13-dimethyl-6,7,8,9,10,11,12,13,14,15-decahydro-1*H-*cyclopenta[a]phenanthren-3(2*H*)-one; |
| (10*R*,13*S*)-17-(5-(difluoromethyl)pyridin-3-yl)-6,10,13-trimethyl-6,7,8,9,10,11,12,13-octahydro-1*H-*cyclopenta[a]phenanthren-3(2*H*)-one; |
| (10*R*,13*S*)-10,13-dimethyl-17-(pyridin-3-yl)-6-(1*H*-1,2,3-triazol-1-yl)-6,7,8,9,10,11,12,13,14,15-decahydro-1*H-*cyclopenta[a]phenanthren-3(2*H*)-one; |
| (10*R*,13*S*)-17-(5-fluoropyridin-3-yl)-4,10,13-trimethyl-2,3,4,5,6,7,8,9,10,11,12,13,14,15-tetradecahydro-1*H-*cyclopenta[a]phenanthren-3-ol; |
| (3*S*,10*R*,13*S*)-17-(5-chloropyridin-3-yl)-10,13-dimethyl-2,3,4,7,8,9,10,11,12,13-decahydro-1*H-*cyclopenta[a]phenanthren-3-ol; |
| (5a*R*,7a*S*)-8-(5-(difluoromethyl)pyridin-3-yl)-5a,7a,12-trimethyl-3,4,5,5a,5b,6,7,7a,11,12-decahydrocyclopenta[5,6]naphtho[1,2-d]azepin-2(10b*H*)-one; |
| (3*S*,4*S*,10*R*,13*S*)-17-(5-(difluoromethyl)pyridin-3-yl)-4,10,13-trimethyl-2,3,4,5,6,7,8,9,10,11,12,13,14,15-tetradecahydro-1*H*-cyclopenta[a]phenanthren-3-ol; |
| (10*R*,13*S*)-17-(5-(difluoromethyl)pyridin-3-yl)-4,10,13-trimethyl-2,3,4,5,6,7,8,9,10,11,12,13,14,15-tetradecahydro-1*H*-cyclopenta[a]phenanthren-3-ol; |
| (3*R*,4*S*,10*R*,13*S*)-17-(5-(difluoromethyl)pyridin-3-yl)-4,10,13-trimethyl-2,3,4,5,6,7,8,9,10,11,12,13,14,15-tetradecahydro-1*H*-cyclopenta[a]phenanthren-3-ol; |
| (10*R*,13*S*)-10,13-dimethyl-17-(pyridin-3-yl)-6-(2*H*-1,2,3-triazol-2-yl)-6,7,8,9,10,11,12,13,14,15-decahydro-1*H-*cyclopenta[a]phenanthren-3(2*H*)-one; |
| 1-((4a*R*,6a*S*)-1,4a,6a-trimethyl-2-oxo-2,3,4,4a,4b,5,6,6a,9,9a,9b,10-dodecahydro-1*H*-indeno[5,4-f]quinolin-7-yl)-1*H*-imidazole-5-carbonitrile; |
| (10*R*,13*S*)-17-(5-fluoropyridin-3-yl)-10,13-dimethyl-6-phenyl-6,7,8,9,10,11,12,13,14,15-decahydro-1*H-*cyclopenta[a]phenanthren-3(2*H*)-one; |
| (10*R*,13*S*)-6-cyclopropyl-10,13-dimethyl-17-(pyridin-3-yl)-6,7,8,9,10,11,12,13,14,15-decahydro-1*H-*cyclopenta[a]phenanthren-3(2*H*)-one; |
| (10*R*,13*S*)-6-(hydroxymethyl)-10,13-dimethyl-17-(pyridin-3-yl)-6,7,8, 9,10,11,12,13-octahydro-1*H-*cyclopenta[a]phenanthren-3(2*H*)-one; |
| (10*R*,13*S*)-10,13-dimethyl-6-phenyl-17-(pyridin-3-yl)-6,7,8,9,10,11,12,13,14,15-decahydro-1*H-*cyclopenta[a]phenanthren-3(2*H*)-one; |
| (3a*S*,5a*S*)-6-(5-fluoropyridin-3-yl)-3a,5a-dimethyl-1,2,3,3a,3b,4,5,5a,8,8a,8b,9,10,10a-tetradecahydrodicyclopenta[a,f]naphthalen-2-ol; |
| (3a*S*,5a*S*)-3a,5a-dimethyl-6-(pyridin-3-yl)-1,2,3,3a,3b,4,5,5a,8,8a,8b,9,10,10a-tetradecahydrodicyclopenta[a,f]naphthalen-2-ol; |
| *N*-(5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)pyridin-3-yl)methanesulfonamide; |
| (3a*S*,5a*S*)-6-(5-chloropyridin-3-yl)-3a,5a-dimethyl-1,2,3,3a,3b,4,5,5a,8,8a,8b,9,10,10a-tetradecahydrodicyclopenta[a,f]naphthalen-2-ol; |
| (3*S*,10*S*,13*S*)-17-(5-chloropyridin-3-yl)-3,10,13-trimethyl-2,3,4,5,6,7,8,9,10,11,12,13,14,15-tetradecahydro-1*H-*cyclopenta[a]phenanthren-3-ol; |
| (3*R*,10*S*,13*S*)-17-(5-chloropyridin-3-yl)-3,10,13-trimethyl-2,3,4,5,6,7,8,9,10,11,12,13,14,15-tetradecahydro-1*H-*cyclopenta[a]phenanthren-3-ol; |
| (10*R*,13*S*)-17-(5-(difluoromethyl)pyridin-3-yl)-6,10,13-trimethyl-6,7,8,9,10,11,12,13,14,15-decahydro-1*H-*cyclopenta[a]phenanthren-3(2*H*)-one; |
| (10*R*,13*S*)-17-(5-fluoropyridin-3-yl)-6,10,13-trimethyl-6,7,8,9,10,11,12,13,14,15-decahydro-1*H-*cyclopenta[a]phenanthren-3(2*H*)-one; |
| (5a*S*,7a*S*)-8-(5-fluoropyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one; |
| (10*R*,13*S*)-6-cyclopropyl-10,13-dimethyl-17-(pyridin-3-yl)-6,7,8,9,10,11,12,13-octahydro-1*H-*cyclopenta[a]phenanthren-3(2*H*)-one; |
| (10*R*,13*S*)-10,13-dimethyl-6-phenyl-17-(pyridin-3-yl)-6,7,8,9,10,11,12,13-octahydro-1*H-*cyclopenta[a]phenanthren-3(2*H*)-one; |
| (10*R*,13*S*)-17-(5-chloropyridin-3-yl)-10,13-dimethyl-6,7,8,9,10,11,12,13-octahydro-1*H-*cyclopenta[a]phenanthren-3(2*H*)-one; |
| (10*R*,13*S*)-17-(5-fluoropyridin-3-yl)-6,10,13-trimethyl-6,7,8,9,10,11,12,13,14,15-decahydro-3*H-*cyclopenta[a]phenanthren-3-one; |
| (10*R*,13*S*)-17-(5-(difluoromethyl)pyridin-3-yl)-6,10,13-trimethyl-6,7,8,9,10,11,12,13,14,15-decahydro-3*H-*cyclopenta[a]phenanthren-3-one; |
| (10*R*,13*S*)-6-ethyl-10,13-dimethyl-17-(pyridin-3-yl)-6,7,8,9,10,11,12,13-octahydro-1*H*-cyclopenta[a]phenanthren-3(2*H*)-one; |
| (10*R*,13*S*)-17-(5-fluoropyridin-3-yl)-10,13-dimethyl-7,8,9,11,12,13,14,15-octahydro-1*H-*cyclopenta[a]phenanthrene-3,6(2*H*,10*H*)-dione; |
| (10*R*,13*S*)-17-(5-fluoropyridin-3-yl)-10,13-dimethyl-4,5,7,8,9,11,12,13,14,15-decahydro-1*H-*cyclopenta[a]phenanthrene-3,6(2*H*,10*H*)-dione; |
| (5a*S*,7a*S*)-8-(5-(difluoromethyl)pyridin-3-yl)-5a,7a-dimethyl-3,4,5,5a,5b,6,7,7a,10,10a,10b,11,12,12a-tetradecahydrocyclopenta[5,6]naphtho[1,2-d]azepin-2(1*H*)-one; |
| (5a*R*,7a*S*)-8-(1*H*-imidazol-1-yl)-5a,7a-dimethyl-2-(methylsulfonyl)-1,2,3,4,5,5a,5b,6,7,7a,10,10a,10b,11,12,12a-hexadecahydrocyclopenta[5,6]naphtho[2,1-c]azepine; |
| (10*R*,13*S*)-17-(5-aminopyridin-3-yl)-6,10,13-trimethyl-6,7,8,9,10,11,12,13-octahydro-1*H-*cyclopenta[a]phenanthren-3(2*H*)-one; |
| (10*R*,13*S*)-17-(5-aminopyridin-3-yl)-10,13-dimethyl-6,7,8,9,10,11,12,13-octahydro-1*H-*cyclopenta[a]phenanthren-3(2*H*)-one; |
| 3-((10*S*,13*S*)-3,3-difluoro-10,13-dimethyl-2,3,4,5,6,7,8,9,10,11,12,13-dodecahydro-1*H-*cyclopenta[a]phenanthren-17-yl)-5-fluoropyridine; |
| 3-((10*S*,13*S*)-3,3-difluoro-10,13-dimethyl-2,3,4,5,6,7,8,9,10,11,12,13-dodecahydro-1*H-*cyclopenta[a]phenanthren-17-yl)pyridine; |
| 3-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)pyridine; |
| 3-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)-5-fluoropyridine; |
| (5a*R*,7a*S*)-8-(5-fluoropyridin-3-yl)-3,5a,7a-trimethyl-3,4,5,5a,5b,6,7,7a,11,12-decahydrocyclopenta[5,6]naphtho[1,2-d]azepin-2(10b*H*)-one; |
| (10*R*,13*S*)-10,13-dimethyl-6-methylene-17-(pyridin-3-yl)-6,7,8,9,10,11,12,13-octahydro-1*H-*cyclopenta[a]phenanthren-3(2*H*)-one; |
| (10*R*,13*S*)-6,10,13-trimethyl-17-(pyridin-3-yl)-6,7,8,9,10,11,12,13-octahydro-3*H*-cyclopenta[a]phenanthren-3-one; |
| 3-((8a*S*,10a*S*)-7,7-difluoro-8a,10a-dimethyl-3,3a,3b,4,5,5a,6,7,8,8a,8b,9,10,10a-tetradecahydrodicyclopenta[a,f]naphthalen-1-yl)pyridine; |
| (3a*S*,5a*S*)-2,3a,5a-trimethyl-6-(pyridin-3-yl)-1,2,3,3a,3b,4,5,5a,8,8a,8b,9,10,10a-tetradecahydrodicyclopenta[a,f]naphthalen-2-ol; |
| (5a*R*,5b*S*,7a*S*,10a*S*,10b*S*,12a*R*)-5a,7a-dimethyl-8-(4-methylpyridin-3-yl)-1,2,5,5a,5b,6,7,7a,10,10a,10b,11,12,12a-tetradecahydrocyclopenta[5,6]naphtho[2,1-c]azepin-3(4*H*)-one; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl propionate; |
| *N*-((3*R*,10*S*,13*S*)-10,13-dimethyl-17-(pyridin-3-yl)-2,3,4,5,6,7,8,9,10,11,12,13,14,15-tetradecahydro-1*H-*cyclopenta[a]phenanthren-3-yl)pivalamide; |
| (3*S*,10*S*,13*S*)-10,13-dimethyl-17-(4-methylpyridin-3-yl)-2,3,4,5,6,7,8,9,10,11,12,13-dodecahydro-1*H-*cyclopenta[a]phenanthren-3-ol; |
| (10*S*,13*S*)-10,13-dimethyl-17-(4-methylpyridin-3-yl)-4,5,6,7,8,9,10,11,12,13-decahydro-1*H-*cyclopenta[a]phenanthren-3(2*H*)-one; |
| (3*S*,10*S*,13*S*)-17-(4-ethylpyridin-3-yl)-10,13-dimethyl-2,3,4,5,6,7,8,9,10,11,12,13-dodecahydro-1*H-*cyclopenta[a]phenanthren-3-yl acetate; |
| (10*S*,13*S*)-17-(4-ethylpyridin-3-yl)-10,13-dimethyl-4,5,6,7,8,9,10,11,12,13-decahydro-1*H-*cyclopenta[a]phenanthren-3(2*H*)-one; |
| (3*S*,10*R*,13*S*)-10,13-dimethyl-17-(pyridin-3-yl)-2,3,4,7,8,9,10,11,12,13-decahydro-1*H*-cyclopenta[a]phenanthren-3-yl benzoate; |
| *N*-(5-((3*S*,10*R*,13*S*)-3-hydroxy-10,13-dimethyl-2,3,4,7,8,9,10,11,12,13,14,15-dodecahydro-1*H-*cyclopenta[a]phenanthren-17-yl)pyridin-3-yl)acetamide; |
| (3*S*,10*R*,13*S*)-17-(5-fluoropyridin-3-yl)-6,10,13-trimethyl-2,3,4,5,6,7,8,9,10,11,12,13,14,15-tetradecahydro-1*H-*cyclopenta[a]phenanthrene-3,5-diol; |
| (3*R*,10*R*,13*S*)-3,10,13-trimethyl-17-(pyridin-3-yl)-2,3,4,7,8,9,10,11,12,13,14,15-dodecahydro-1*H-*cyclopenta[a]phenanthren-3-ol; |
| (3*S*,10*R*,13*S*)-3,10,13-trimethyl-17-(pyridin-3-yl)-2,3,4,7,8,9,10,11,12,13,14,15-dodecahydro-1*H-*cyclopenta[a]phenanthren-3-ol; |
| (3*R*,10*R*,13*S*)-17-(5-fluoropyridin-3-yl)-3,10,13-trimethyl-2,3,4,7,8,9,10,11,12,13,14,15-dodecahydro-1*H-*cyclopenta[a]phenanthren-3-ol; |
| (3*S*,10*R*,13*S*)-17-(5-fluoropyridin-3-yl)-3,10,13-trimethyl-2,3,4,7,8,9,10,11,12,13,14,15-dodecahydro-1*H-*cyclopenta[a]phenanthren-3-ol; |
| 1-((10*S*,13*S*)-3,3-difluoro-10,13-dimethyl-2,3,4,5,6,7,8,9,10,11,12,13,14,15-tetradecahydro-1*H-*cyclopenta[a]phenanthren-17-yl)-1*H*-imidazole; |
| (5a*S*,7a*S*)-8-(1*H*-imidazol-1-yl)-5a,7a-dimethyl-3-(methylsulfonyl)-1,2,3,4,5,5a,5b,6,7,7a,10b,11,12,12a-tetradecahydrocyclopenta[5,6]naphtho[1,2-d]azepine; |
| (10*R*,13*S*)-10,13-dimethyl-6-methylene-17-(pyridin-3-yl)-6,7,8,9,10,11,12,13-octahydro-3*H-*cyclopenta[a]phenanthren-3-one; |
| (3*S*,10*R*,13*S*)-17-(5-fluoropyridin-3-yl)-10,13-dimethyl-2,3,4,7,8,9,10,11,12,13-decahydro-1*H-*cyclopenta[a]phenanthren-3-yl 1-methylpiperidine;-4-carboxylate; |
| (5a*R*,7a*S*)-3,5a,7a,12-tetramethyl-8-(pyridin-3-yl)-3,4,5,5a,5b,6,7,7a,11,12-decahydrocyclopenta[5,6]naphtho[1,2-d]azepin-2(10b*H*)-one; |
| (5a*R*,7a*S*)-5a,7a,12-trimethyl-8-(pyridin-3-yl)-3,4,5,5a,5b,6,7,7a,8,9,10,10a,11,12-tetradecahydrocyclopenta[5,6]naphtho[1,2-d]azepin-2(10b*H*)-one; |
| (5a*R*,7a*S*)-8-(5-fluoropyridin-3-yl)-3,5a,7a,12-tetramethyl-3,4,5,5a,5b,6,7,7a,11,12-decahydrocyclopenta[5,6]naphtho[1,2-d]azepin-2(10b*H*)-one; |
| (5a*S*,7a*S*)-8-(1*H*-imidazol-1-yl)-5a,7a-dimethyl-3-(methylsulfonyl)-1,2,3,4,5,5a,5b,6,7,7a,10,10a,10b,11,12,12a-hexadecahydrocyclopenta[5,6]naphtho[1,2-d]azepine; |
| (10*R*,13*S*)-6,10,13-trimethyl-17-(pyridin-3-yl)-6,7,8,9,10,11,12,13,14,15,16,17-dodecahydro-1*H-*cyclopenta[a]phenanthren-3(2*H*)-one; |
| (10*R*,13*S*)-10,13-dimethyl-17-(pyridin-3-yl)-1,7,8,9,10,11,12,13-octahydrospiro[cyclopenta[a]phenanthrene-6,1'-cyclopropan]-3(2*H*)-one; |
| (10*R*,13*S*)-6,10,13-trimethyl-17-(pyridin-3-yl)-6,7,8,9,10,11,12,13,14,15,16,17-dodecahydro-3*H-*cyclopenta[a]phenanthren-3-one; |
| (10*R*,13*S*)-17-(5-fluoropyridin-3-yl)-10,13-dimethyl-6-methylene-6,7,8,9,10,11,12,13-octahydro-1*H-*cyclopenta[a]phenanthren-3(2*H*)-one; |
| (10*R*,13*S*)-17-(5-fluoropyridin-3-yl)-10,13-dimethyl-1,7,8,9,10,11,12,13-octahydrospiro[cyclopenta[a]phenanthrene-6,1'-cyclopropan]-3(2*H*)-one; |
| (5a*R*,7a*S*)-3,5a,7a-trimethyl-8-(pyridin-3-yl)-3,4,5,5a,5b,6,7,7a,10b,10c,11,11a-dodecahydro-2*H-*cyclopenta[5,6]cyclopropa[3,4]naphtho[1,2-d]azepin-2-one; |
| 1-((5a*R*,7a*S*)-5a,7a-dimethyl-2-(methylsulfonyl)-1,2,3,4,5,5a,5b,6,7,7a,10,10a,10b,11,12,12a-hexadecahydrocyclopenta[5,6]naphtho[2,1-c]azepin-8-yl)-1*H*-imidazole-5-carbonitrile; |
| 1-((5a*S*,7a*S*)-5a,7a-dimethyl-3-(methylsulfonyl)-1,2,3,4,5,5a,5b,6,7,7a,10,10a,10b,11,12,12a-hexadecahydrocydopenta[5,6]naphtho[1,2-d]azepin-8-yl)-1*H*-imidazole-5-carbonitrile; |
| (10*R*,13*S*)-17-(5-fluoropyridin-3-yl)-10,13-dimethyl-7,8,9,11,12,13-hexahydrospiro[cyclopenta[a]phenanthrene-6,1'-cyclopropan]-3(10*H*)-one; |
| (3*S*,10*R*,13*S*)-17-(1*H*-imidazol-1-yl)-10,13-dimethyl-2,3,4,7,8,9,10,11,12,13-decahydro-1*H-*cyclopenta[a]phenanthren-3-ol; |
| (10*R*,13*S*)-10,13-dimethyl-17-(pyridin-3-yl)-6,7,8,9,10,11,12,13,14,15-decahydro-1*H*-cyclopenta[a]phenanthren-3(2*H*)-one; |
| (3*S*,8*R*,9*S*,10*R*,13*S*,14*S*)-10,13-dimethyl-17-(pyridin-3-yl)-2,3,4,7,8,9,10,11,12,13,14,15-dodecahydro-1*H-*cyclopenta[a]phenanthren-3-ol; and |
| (10*R*,13*S*)-6-fluoro-10,13-dimethyl-17-(pyridin-3-yl)-6,7,8,9,10,11,12,13,14,15-decahydro-1*H-*cyclopenta[a]phenanthren-3(2*H*)-one; |
and optionally a pharmaceutical composition comprising the compound and a pharmaceutically acceptable carrier, excipient or binder.

11. A
1) therapeutically effective amount of a compound of any of claims 1-10 or a pharmaceutically acceptable salt or solvate thereof or
2) pharmaceutical composition comprising a therapeutically effective amount of a compound of any of claims 1-10 or a pharmaceutically acceptable salt or solvate thereof and a pharmaceutically acceptable carrier, excipient or binder;
for use in the treatment of cancer;
optionally where the cancer is selected from the group consisting of bladder cancer, brain cancer, breast cancer, cervical cancer, colorectal cancer, endometrial cancer, gastric cancer, glioblastoma, head and neck cancer, Kaposi's sarcoma, kidney cancer, leiomyosarcoma, leukemia, liver cancer, lung cancer, melanoma, multiple myeloma, Non-Hodgkin lymphoma, ovarian cancer, pancreatic cancer, papillary renal cell carcinoma, prostate cancer, renal cancer, squamous cell cancer, and/or thoracic cancer; or optionally where the cancer is prostate cancer.

12. The compound or composition of claim 11 further comprising providing to the subject an additional therapy selected from the group consisting of surgery, an anti-androgenic agent, and radiation, and a combination thereof; optionally where the anti-androgenic agent is optionally selected from the group consisting of flutamide, nicalutamide, bicalutamide, inhibitors of 17α-hydroxylase/C17-20 lyase, luteinizing hormone-releasing hormone agonists, luteinizing hormone-releasing hormone antagonists, and/or 5α-reductase type 1 and/or type 2 and/or combinations thereof.

13. A compound for use in the treatment of an androgen-dependent disorder wherein the compound is a compound according to any one of claims 1-10 or a pharmaceutically acceptable salt or solvate thereof optionally where the androgen-dependent disorder is selected from the group consisting of prostate cancer, benign prostatic hyperplasia, prostatic intraepithelial neoplasia, hirsutism, acne, androgenic alopecia, and polycystic ovary syndrome; optionally where the androgen-dependent disorder is prostate cancer.

14. A compound for use in the treatment of a a disease associated with hypercortisolism wherein the compound is a compound according to any one of claims 1-10 or a pharmaceutically acceptable salt or solvate thereof; optionally where the disease is Cushing's Syndrome.

15. A method of making a compound of claim 1
(a) comprising oxidizing an intermediate of formula 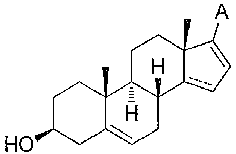 to yield a compound of formula 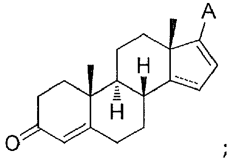 and
optionally treating with hydroxylamine to yield the oxime; or
(b) comprising reacting an intermediate of formula 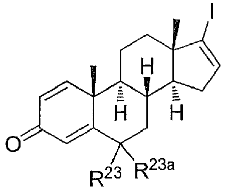 where R²³ and R^{23a} together form C=CH₂ or cycloalkyl, with an intermediate of formula A-B(ethyl)₂ or A-B(OH)₂; to yield the compound of formula 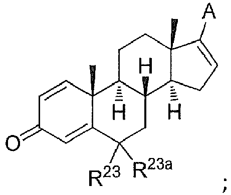 or
(c) comprising oxidizing an intermediate of formula 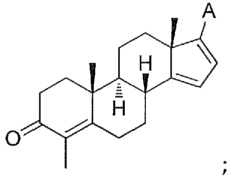 or
treating an intermediate of formula 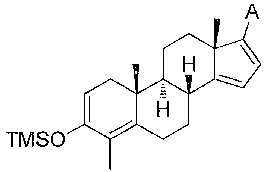 with a catalyst to yield a compound of formula 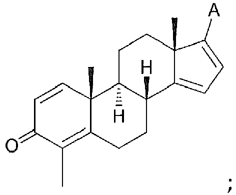 or
(d) comprising reacting an intermediate of formula 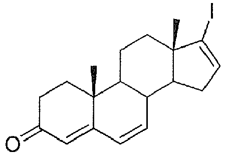 with an intermediate of formula A-B(ethyl)₂ or A-B(OH)₂; to yield the compound of formula 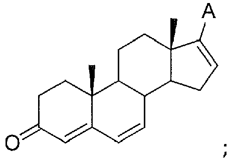 or
(e) comprising reacting an intermediate of formula 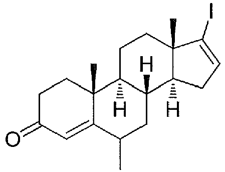 with an intermediate of formula A-B(ethyl)₂ or A-B(OH)₂ to yield a compound of formula 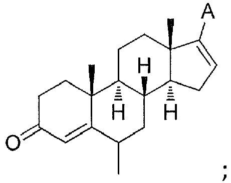 and
optionally oxidizing to yield

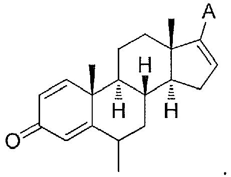

16. (new) A method of making a compound of claim 2 where the compound of Formula XIV is 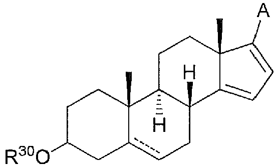
(a) comprising reacting an intermediate of formula 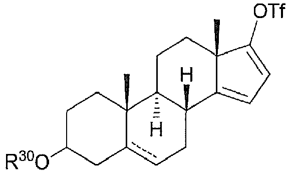 where R³⁰ is acetyl, with an intermediate of formula A-B(ethyl)₂ or A-B(OH)₂; and optionally hydrolyzing to obtain the compound of Formula XIV where R³⁰ is hydrogen; or
(b) comprising reacting an intermediate of formula 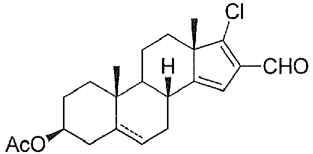 with an intermediate of formula A-H where A-H is a heteroaryl group optionally substituted with 1, 2, 3, or 4 R⁴ groups and where heteroaryl comprises an NH; and removing the formyl group; and optionally hydrolyzing to yield the compound of Formula XIV where R³⁰ is hydrogen; or
(c) comprising reacting an intermediate of formula 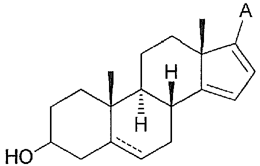 with an intermediate of formula R³⁰X where X is halo or OH and R³⁰ is alkylcarbonyl.
